(19) Europäisches Patentamt
European Patent Office
Office européen des brevets

(11) EP 3 856 737 B1

(12) EUROPEAN PATENT SPECIFICATION

(45) Date of publication and mention of the grant of the patent:
**17.05.2023 Bulletin 2023/20**

(21) Application number: **19782931.0**

(22) Date of filing: **25.09.2019**

(51) International Patent Classification (IPC):
***C07D 405/14*** *(2006.01)* ***C07D 471/04*** *(2006.01)*
***A61P 37/00*** *(2006.01)* ***A61K 31/4375*** *(2006.01)*

(52) Cooperative Patent Classification (CPC):
**C07D 405/14; A61P 37/00; C07D 471/04**

(86) International application number:
**PCT/EP2019/075791**

(87) International publication number:
**WO 2020/064792 (02.04.2020 Gazette 2020/14)**

# (54) HETEROCYCLYL COMPOUNDS FOR THE TREATMENT OF AUTOIMMUNE DISEASE

HETEROCYCLYLVERBINDUNGEN ZUR BEHANDLUNG VON AUTOIMMUNERKRANKUNGEN

COMPOSÉS HÉTÉROCYCLYLES POUR LE TRAITEMENT D'UNE MALADIE AUTO-IMMUNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2018 PCT/CN2018/108015**
**21.08.2019 PCT/CN2019/101852**

(43) Date of publication of application:
**04.08.2021 Bulletin 2021/31**

(73) Proprietor: **F. Hoffmann-La Roche AG**
**4070 Basel (CH)**

(72) Inventors:
- **DEY, Fabian**
  **4070 Basel (CH)**
- **CHEN, Jianguo**
  **Shanghai, 201203 (CN)**
- **LIU, Haixia**
  **Shanghai, 201203 (CN)**
- **LIU, Yafei**
  **Shanghai, 201203 (CN)**
- **SHEN, Hong**
  **Shanghai, 201203 (CN)**
- **ZHANG, Weixing**
  **Shanghai, 201203 (CN)**
- **ZHANG, Zhiwei**
  **Shanghai, 201203 (CN)**
- **ZHU, Wei**
  **Shanghai, 201203 (CN)**

(74) Representative: **Belkacemi, Doria**
**F. Hoffmann-La Roche AG**
**Grenzacherstrasse 124**
**4070 Basel (CH)**

(56) References cited:
**WO-A1-2015/057659 WO-A1-2017/106607**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 856 737 B1

## Description

The present invention relates to organic compounds useful for therapy and/or prophylaxis in a mammal, and in particular to antagonist of TLR7 and/or TLR8 and/or TLR9 for use in treating systemic lupus erythematosus or lupus nephritis.

## FIELD OF THE INVENTION

Autoimmune connective tissue disease (CTD) include prototypical autoimmune syndromes such as Systemic Lupus Erythematosus (SLE), primary Sjögren's syndrome (pSjS), mixed connective tissue disease (MCTD), Dermatomyositis/Polymyositis (DM/PM), Rheumatoid Arthritis (RA), and systemic sclerosis (SSc). With the exception of RA, no really effective and safe therapies are available to patients. SLE represents the prototypical CTD with a prevalence of 20-150 per 100,000 and causes broad inflammation and tissue damage in distinct organs, from commonly observed symptoms in the skin and joints to renal, lung, or heart failure. Traditionally, SLE has been treated with nonspecific anti-inflammatory or immunosuppressive drugs. However, long term usage of immunosuppressive drug, e.g. corticosteroids is only partially effective, and is associated with undesirable toxicity and side effects. Belimumab is the only FDA-approved drug for lupus in the last 50 years, despite its modest and delayed efficacy in only a fraction of SLE patients (Navarra, S. V. et al Lancet 2011, 377, 721.). Other biologics, such as anti-CD20 mAbs, mAbs against or soluble receptors of specific cytokines, have failed in most clinical studies. Thus, novel therapies are required that provide sustained improvement in a greater proportion of patient groups and are safer for chronic use in many autoimmune as well as autoinflammation diseases.

Toll Like Receptors (TLR) are an important family of pattern recognition receptors (PRR) which can initiate broad immune responses in a wide variety of immune cells. As natural host defense sensors, endosomal TLRs 7, 8 and 9 recognize nucleic acids derived from viruses, bacteria; specifically, TLR7/8 and TLR9 recognize single-stranded RNA (ssRNA) and single-stranded CpG-DNA, respectively. However, aberrant nucleic acid sensing of TRL7,8,9 is considered as a key node in a broad of autoimmune and auto-inflammatory diseases (Krieg, A. M. et al. Immunol. Rev. 2007, 220, 251. Jiménez-Dalmaroni, M. J. et al Autoimmun Rev. 2016, 15, 1. Chen, J. Q., et al. Clinical Reviews in Allergy & Immunology 2016, 50, 1.). Anti-RNA and anti-DNA antibodies are well established diagnostic markers of SLE, and these antibodies can deliver both self-RNA and self-DNA to endosomes. While self-RNA complexes can be recognized by TLR7 and TLR8, self-DNA complexes can trigger TLR9 activation. Indeed, defective clearance of self-RNA and self-DNA from blood and/or tissues is evident in SLE (Systemic Lupus Erythematosus) patients. TLR7 and TLR9 have been reported to be upregulated in SLE tissues, and correlate with chronicity and activity of lupus nephritis, respectively. In B cells of SLE patients, TLR7 expression correlates with anti-RNP antibody production, while TLR9 expression with IL-6 and anti-dsDNA antibody levels. Consistently, in lupus mouse models, TLR7 is required for anti-RNA antibodies, and TLR9 is required for anti-nucleosome antibody. On the other hand, overexpression of TLR7 or human TLR8 in mice promotes autoimmunity and autoinflammation. Moreover, activation of TLR8 specifically contributes to inflammatory cytokine secretion of mDC/macrophages, neutrophil NETosis, induction of Th17 cells, and suppression of Treg cells. In addition to the described role of TLR9 in promoting autoantibody production of B cells, activation of TLR9 by self-DNA in pDC also leads to induction of type I IFNs and other inflammatory cytokines. Given these roles of TLR9 in both pDC and B cells, both as key contributors to the pathogenesis of autoimmune diseases, and the extensive presence of self-DNA complexes that could readily activate TLR9 in many patients with autoimmune diseases, it may have extra benefit to further block self-DNA mediated TLR9 pathways on top of inhibition of TLR7 and TLR8 pathways. Taken together, TLR7, 8, and 9 pathways represent new therapeutic targets for the treatment of autoimmune and auto-inflammatory diseases, for which no effective steroid-free and non-cytotoxic oral drugs exist, and inhibition of all these pathways from the very upstream may deliver satisfying therapeutic effects. As such, we invented oral compounds that target and suppress TLR7, TLR8 and TLR9 for the treatment of autoimmune and auto-inflammatory diseases.

## SUMMARY OF THE INVENTION

The present invention relates to novel compounds of formula (I) or (Ia),

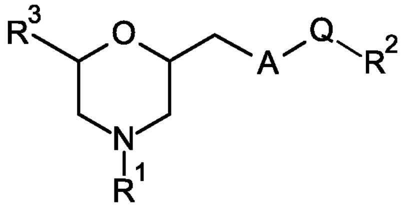
(I) or
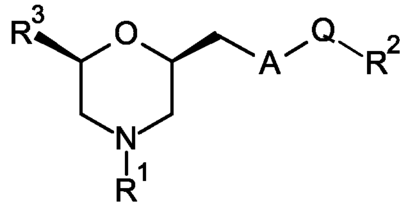
(Ia),

wherein

$R^1$ is

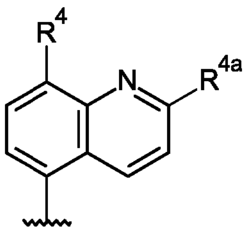, 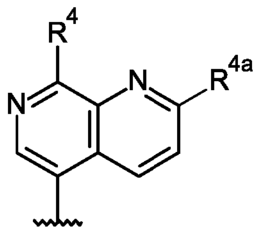, 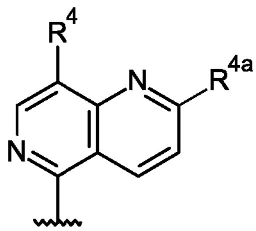, 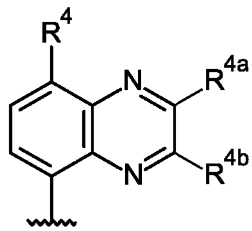,

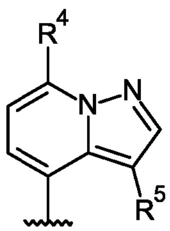, 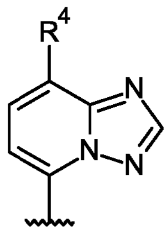, 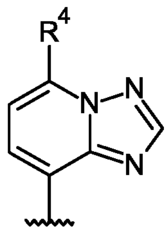, 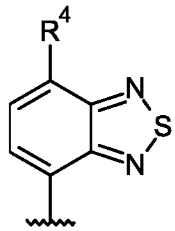, 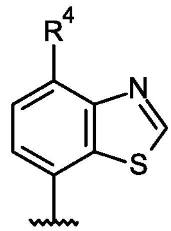,

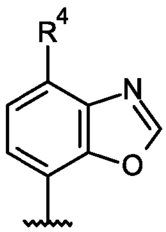, 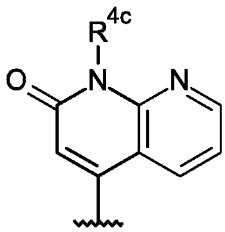 or 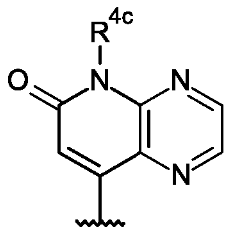;

wherein $R^4$ is $C_{1-6}$alkyl, $C_{1-6}$alkoxy, halo$C_{1-6}$alkyl, halogen, nitro or cyano; $R^{4a}$ is H or deuterium; $R^{4b}$ is H, deuterium or $C_{1-6}$alkyl; $R^{4c}$ is $C_{1-6}$alkyl or $C_{3-7}$cycloalkyl; $R^5$ is H or halogen;
$R^2$ is (($C_{1-6}$alkyl)$_2$amino)$C_{1-6}$alkoxy;

($C_{1-6}$alkoxypyrrolidinyl)amino;
(cyanopyrrolidinyl) amino;
1,4-diazepanyl substituted by hydroxy;
1,6-diazaspiro[3.3]heptanyl;
2,5-diazabicyclo [2.2.1]heptanyl;
2,6-diazaspiro[3.3]heptanyl substituted by $C_{1-6}$alkyl;
3,4,4a,5,7,7a-hexahydro-2H-pyrrolo[3,4-b][1,4]oxazinyl;
3-oxa-7,9-diazabicyclo [3.3.1] nonanyl;
5-oxa-2,8-diazaspiro[3.5]nonanyl;
amino$C_{1-6}$alkyl;
aminooxazepanyl;
azetidinyl substituted once or twice by substituents independently selected from amino and $C_{1-6}$alkyl;
azetidinylamino;
halopyrrolidinylamino;
morpholinyl unsubstituted or substituted by $C_{1-6}$alkyl;
morpholinyl$C_{1-6}$alkyl;
piperazinyl unsubstituted or substituted by $C_{1-6}$alkyl, hydroxy$C_{1-6}$alkyl, pyrrolidinylcarbonyl, (($C_{1-6}$alkyl)$_2$amino)$C_{1-6}$alkylcarbonyl or azetidinylcarbonyl;
piperidinyl unsubstituted or substituted once or twice by substituents independently selected from amino, halogen and $C_{1-6}$alkoxy; or
pyrrolidinyl substituted once, twice or three times by substituents independently selected from amino, halogen, hydroxy, $C_{1-6}$alkyl and $C_{1-6}$alkoxy;

$R^3$ is $C_{1-6}$alkyl;
A is azetidinyl, hydroxyazetidinyl, piperazinyl, pyrrolidinyl, piperidinyl or cyanopiperidinyl;
Q is phenyl unsubstituted or substituted by halogen;

pyrazinyl;
pyridazinyl;
pyridinyl unsubstituted or substituted by $C_{1-6}$alkyl;
pyrimidinyl unsubstituted or substituted once or twice by substituents independently selected from halogen, $C_{1-6}$alkyl, $C_{1-6}$alkoxy and cyano; or
quinazolinyl;

or a pharmaceutically acceptable salt thereof.

Another object of the present invention is related to novel compounds of formula (I) or (Ia), their manufacture, medicaments based on a compound in accordance with the invention and their production as well as the use of compounds of formula (I) or (Ia) as TLR7 and/or TLR8 and/or TLR9 antagonist, and for use in the treatment or prophylaxis of systemic lupus erythematosus or lupus nephritis. The compounds of formula (I) or (Ia) show superior TLR7 and TLR8 and TLR9 antagonism activity. In addition, the compounds of formula (I) or (Ia) also show good cytotoxicity, solubility, hPBMC, human microsome stability and SDPK profiles, as well as low CYP inhibition.

## DETAILED DESCRIPTION OF THE INVENTION

### DEFINITIONS

The term "$C_{1-6}$alkyl" denotes a saturated, linear or branched chain alkyl group containing 1 to 6, particularly 1 to 4 carbon atoms, for example methyl, ethyl, *n*-propyl, isopropyl, *n*-butyl, isobutyl, *tert*-butyl and the like. Particular "$C_{1-6}$alkyl" groups are methyl, ethyl and *n*-propyl.

The term "halogen" and "halo" are used interchangeably herein and denote fluoro, chloro, bromo, or iodo.

The letter "D" shown in the molecular structure denotes "deuterium".

The term "halo$C_{1-6}$alkyl" denotes an alkyl group wherein at least one of the hydrogen atoms of the alkyl group has been replaced by same or different halogen atoms, particularly fluoro atoms. Examples of halo$C_{1-6}$alkyl include monofluoro-, difluoro-or trifluoro-methyl, - ethyl or -propyl, for example 3,3,3-trifluoropropyl, 2-fluoroethyl, 2,2,2-trifluoroethyl, fluoromethyl, difluoromethyl, trifluoromethyl and trifluoroethyl.

The term "halopyrrolidinyl" denotes a pyrrolidinyl substituted once, twice or three times by halogen. Examples of halopyrrolidinyl include, but not limited to, fluoropyrrolidinyl and difluoropyrrolidinyl.

The term "halopiperidinyl" denotes a piperidinyl substituted once, twice or three times by halogen. Examples of halopiperidinyl include, but not limited to, fluoropiperidinyl and difluoropiperidinyl.

The term "LG" denotes a leaving group, which is a molecular fragment that departs with a pair of electrons in heterolytic bond cleavage. Leaving groups can be anions or neutral molecules, but in either case it is crucial that the leaving group be able to stabilize the additional electron density that results from bond heterolysis. Common anionic leaving groups are halides, and sulfonate esters such as OTf, OTs and OMs.

The term "PG" denotes a protecting group, which is introduced into a molecule by chemical modification of a functional group to obtain chemo selectivity in a subsequent chemical reaction. Typical protecting groups are Boc, Cbz and Bn.

The term "*cis*-isomers" and "*trans*-isomers" denote the relative stereochemistry of the molecule or moiety. For example: *tert*-butyl *N*-(cis-5-fluoropiperidin-3-yl)carbamate (

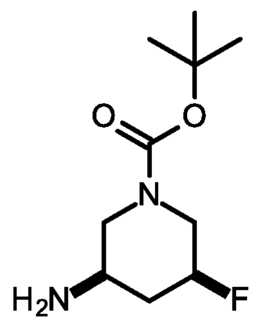

) as the "*cis*-isomers" refers to a mixture of

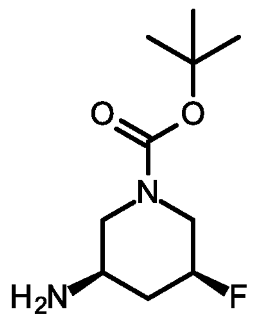

and

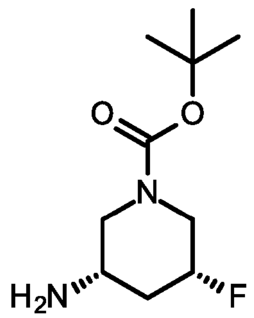

;

similarly, *tert*-butyl *trans*-(4-methoxypyrrolidin-3-yl)carbamate (

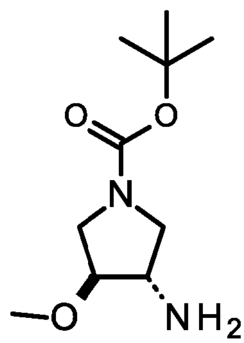

) as the *"trans-isomers"* refers to a mixture of

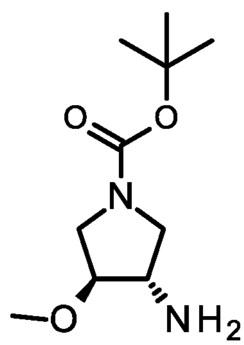

and

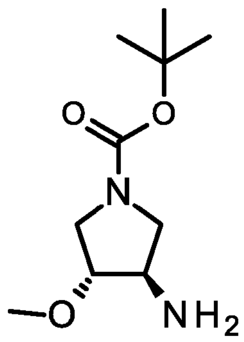

The way of showing relative stereochemistry also applies to the final compound.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

**ANTAGONIST OF TLR7 AND/OR TLR8 AND/OR TLR9**

The present invention relates to (i) a compound of formula (I),

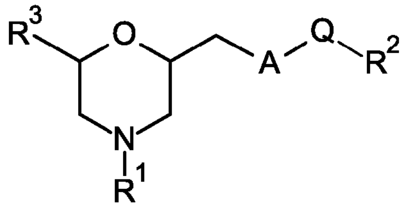

(I),

wherein

$R^1$ is

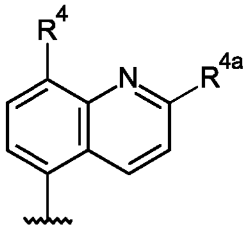 , 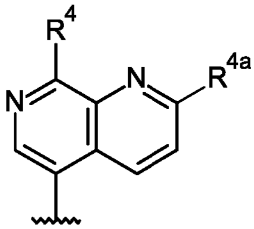 , 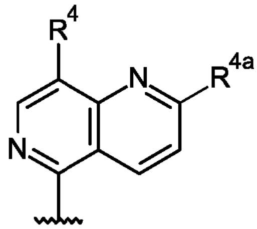 , 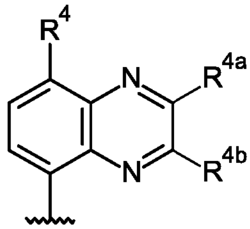 ,

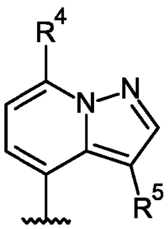 , 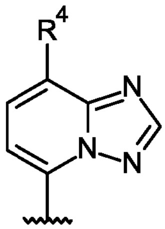 , 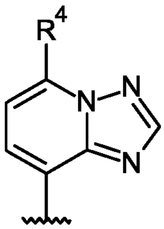 , 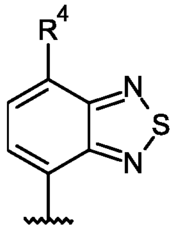 , 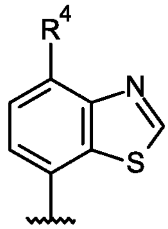 ,

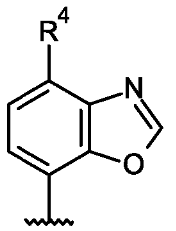 , 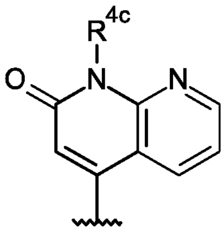 or 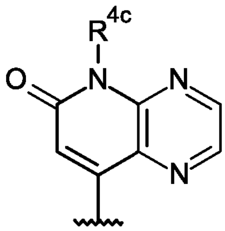 ;

wherein $R^4$ is $C_{1-6}$alkyl, $C_{1-6}$alkoxy, halo$C_{1-6}$alkyl, halogen, nitro or cyano; $R^{4a}$ is H or deuterium; $R^{4b}$ is H, deuterium or $C_{1-6}$alkyl; $R^{4c}$ is $C_{1-6}$alkyl or $C_{3-7}$cycloalkyl; $R^5$ is H or halogen;
$R^2$ is (($C_{1-6}$alkyl)$_2$amino)$C_{1-6}$alkoxy;

($C_{1-6}$alkoxypyrrolidinyl)amino;
(cyanopyrrolidinyl) amino;
1,4-diazepanyl substituted by hydroxy;
1,6-diazaspiro[3.3]heptanyl;
2,5-diazabicyclo [2.2.1]heptanyl;
2,6-diazaspiro[3.3]heptanyl substituted by $C_{1-6}$alkyl;
3,4,4a,5,7,7a-hexahydro-2H-pyrrolo[3,4-b][1,4]oxazinyl;
3-oxa-7,9-diazabicyclo[3.3.1]nonanyl;
5-oxa-2,8-diazaspiro[3.5]nonanyl;
amino$C_{1-6}$alkyl;
aminooxazepanyl;
azetidinyl substituted once or twice by substituents independently selected from amino and $C_{1-6}$alkyl;
azetidinylamino;
halopyrrolidinylamino;
morpholinyl unsubstituted or substituted by $C_{1-6}$alkyl;
morpholinyl$C_{1-6}$alkyl;
piperazinyl unsubstituted or substituted by $C_{1-6}$alkyl, hydroxy$C_{1-6}$alkyl, pyrrolidinylcarbonyl, (($C_{1-6}$alkyl)$_2$amino)$C_{1-6}$alkylcarbonyl or azetidinylcarbonyl;
piperidinyl unsubstituted or substituted once or twice by substituents independently selected from amino, halogen and $C_{1-6}$alkoxy; or
pyrrolidinyl substituted once, twice or three times by substituents independently selected from amino, halogen, hydroxy, $C_{1-6}$alkyl and $C_{1-6}$alkoxy;

$R^3$ is $C_{1-6}$alkyl;
A is azetidinyl, hydroxyazetidinyl, piperazinyl, pyrrolidinyl, piperidinyl or cyanopiperidinyl;
Q is phenyl unsubstituted or substituted by halogen;

pyrazinyl;
pyridazinyl;
pyridinyl unsubstituted or substituted by $C_{1-6}$alkyl;
pyrimidinyl unsubstituted or substituted once or twice by substituents independently selected from halogen, $C_{1-6}$alkyl, $C_{1-6}$alkoxy and cyano; or
quinazolinyl;

or a pharmaceutically acceptable salt thereof.

Another embodiment of present invention is (ii) a compound of formula (Ia),

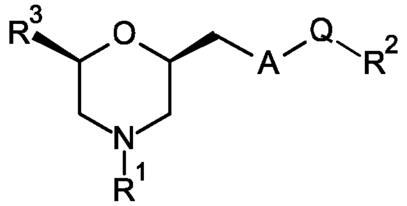

(Ia),

wherein

$R^1$ is

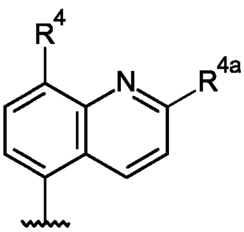 , 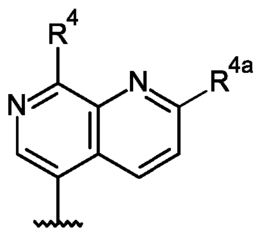 , 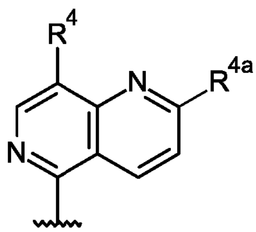 , 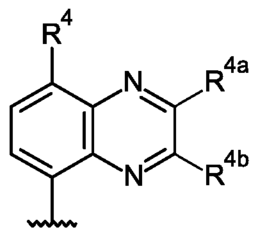 ,

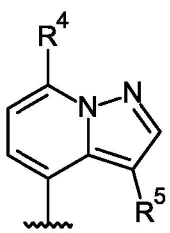 , 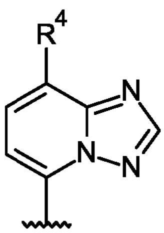 , 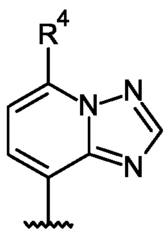 , 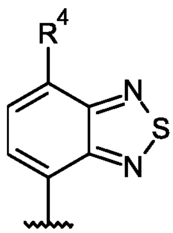 , 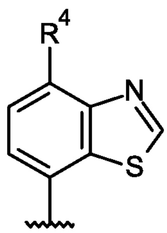 ,

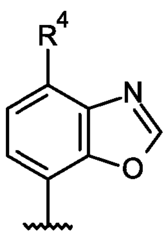 , 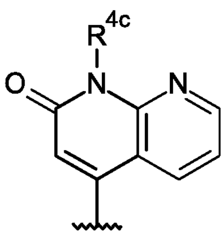 or 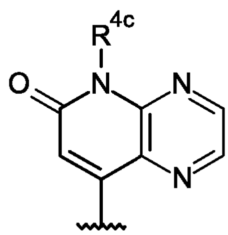 ;

wherein $R^4$ is $C_{1-6}$alkyl, $C_{1-6}$alkoxy, halo$C_{1-6}$alkyl, halogen, nitro or cyano; $R^{4a}$ is H or deuterium; $R^{4b}$ is H, deuterium or $C_{1-6}$alkyl; $R^{4c}$ is $C_{1-6}$alkyl or $C_{3-7}$cycloalkyl; $R^5$ is H or halogen;
$R^2$ is (($C_{1-6}$alkyl)$_2$amino)$C_{1-6}$alkoxy;

($C_{1-6}$alkoxypyrrolidinyl)amino;
(cyanopyrrolidinyl) amino;
1,4-diazepanyl substituted by hydroxy;

1,6-diazaspiro[3.3]heptanyl;
2,5-diazabicyclo [2.2.1]heptanyl;
2,6-diazaspiro[3.3]heptanyl substituted by $C_{1-6}$alkyl;
3,4,4a,5,7,7a-hexahydro-2H-pyrrolo[3,4-b][1,4]oxazinyl;
3-oxa-7,9-diazabicyclo [3.3.1] nonanyl;
5-oxa-2,8-diazaspiro[3.5]nonanyl;
amino$C_{1-6}$alkyl;
aminooxazepanyl;
azetidinyl substituted once or twice by substituents independently selected from amino and $C_{1-6}$alkyl;
azetidinylamino;
halopyrrolidinylamino;
morpholinyl unsubstituted or substituted by $C_{1-6}$alkyl;
morpholinyl$C_{1-6}$alkyl;
piperazinyl unsubstituted or substituted by $C_{1-6}$alkyl, hydroxy$C_{1-6}$alkyl, pyrrolidinylcarbonyl, (($C_{1-6}$alkyl)$_2$amino)$C_{1-6}$alkylcarbonyl or azetidinylcarbonyl;
piperidinyl unsubstituted or substituted once or twice by substituents independently selected from amino, halogen and $C_{1-6}$alkoxy; or
pyrrolidinyl substituted once, twice or three times by substituents independently selected from amino, halogen, hydroxy, $C_{1-6}$alkyl and $C_{1-6}$alkoxy;

$R^3$ is $C_{1-6}$alkyl;
A is azetidinyl, hydroxyazetidinyl, piperazinyl, pyrrolidinyl, piperidinyl or cyanopiperidinyl; Q is phenyl unsubstituted or substituted by halogen;

pyrazinyl;
pyridazinyl;
pyridinyl unsubstituted or substituted by $C_{1-6}$alkyl;
pyrimidinyl unsubstituted or substituted once or twice by substituents independently selected from halogen, $C_{1-6}$alkyl, $C_{1-6}$alkoxy and cyano; or
quinazolinyl;

or a pharmaceutically acceptable salt thereof.

A further embodiment of present invention is (iii) a compound of formula (I) or (Ia) according to (i) or (ii), or a pharmaceutically acceptable salt thereof, wherein

$R^1$ is

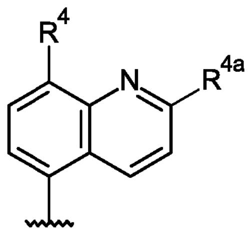 , 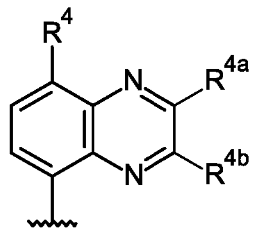 , 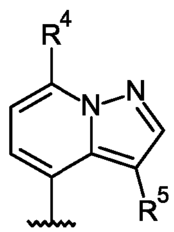 or 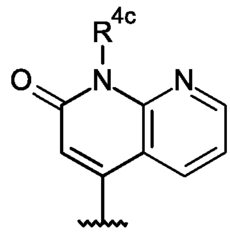 ;

wherein $R^4$ is $C_{1-6}$alkyl, halo$C_{1-6}$alkyl or cyano; $R^{4a}$ is H or deuterium; $R^{4b}$ is H or deuterium; $R^{4c}$ is $C_{1-6}$alkyl; $R^5$ is H or halogen.

A further embodiment of present invention is (iv) a compound of formula (I) or (Ia) according to any one of (i) to (iii), wherein $R^4$ is methyl, trifluoromethyl or cyano; $R^{4a}$ is H or deuterium; $R^{4b}$ is H or deuterium; $R^{4c}$ is methyl; $R^5$ is H or fluoro.

A further embodiment of present invention is (v) a compound of formula (I) or (Ia) according to any one of (i) to (iv), or a pharmaceutically acceptable salt thereof, wherein $R^2$ is ((($C_{1-6}$alkyl)$_2$amino)$C_{1-6}$alkylcarbonyl)piperazinyl; (($C_{1-6}$alkyl)$_2$amino)$C_{1-6}$alkoxy; (azetidinylcarbonyl)piperazinyl; ($C_{1-6}$alkoxypyrrolidinyl)amino; ($C_{1-6}$alkyl)morpholinyl; (cyanopyrrolidinyl)amino; (hydroxy$C_{1-6}$alkyl)piperazinyl; (pyrrolidinylcarbonyl)piperazinyl; 1,6-diazaspiro[3.3]heptanyl;

2,5-diazabicyclo[2.2.1]heptanyl; 3,4,4a,5,7,7a-hexahydro-2H-pyrrolo[3,4-b][1,4]oxazinyl; 3-oxa-7,9-diazabicyclo[3.3.1]nonanyl; 5-oxa-2,8-diazaspiro[3.5]nonanyl; amino($C_{1-6}$alkoxy)piperidinyl; amino($C_{1-6}$alkoxy)pyrrolidinyl; amino($C_{1-6}$alkyl)azetidinyl; amino(hydroxy)($C_{1-6}$alkyl)pyrrolidinyl; aminoazetidinyl; amino$C_{1-6}$alkyl; aminohalopiperidinyl; aminohalopyrrolidinyl; aminooxazepanyl; aminopiperidinyl; azetidinylamino; $C_{1-6}$alkyl-2,6-diazaspiro[3.3]heptanyl; $C_{1-6}$alkylpiperazinyl; halopyrrolidinylamino; hydroxy-1,4-diazepanyl; morpholinyl; morphofinyl$C_{1-6}$alkyl; piperazinyl or piperidinyl.

A further embodiment of present invention is (vi) a compound of formula (I) or (Ia) according to any one of (i) to (v), or a pharmaceutically acceptable salt thereof, wherein $R^2$ is (1-hydroxy-1-methyl-ethyl)piperazin-1-yl; (2-pyrrolidinylcarbonyl)piperazin-1-yl; (4-cyanopyrrolidin-3-yl)amino; (4-fluoropyrrolidin-3-yl)amino; (4-methoxypyrrolidin-3-yl)amino; (dimethylamino)ethoxy; 1,6-diazaspiro[3.3]heptan-6-yl; 2,5-diazabicyclo[2.2.1]heptan-2-yl; 2-methylmorpholin-2-yl; 2-morpholin-2-yl; 3-(hydroxymethyl)piperazin-1-yl; 3,4,4a,5,7,7a-hexahydro-2H-pyrrolo[3,4-b][1,4]oxazin-6-yl; 3-amino-3-methyl-azetidin-1-yl; 3-amino-4-fluoro-1-piperidinyl; 3-amino-4-fluoro-pyrrolidin-1-yl; 3-amino-4-methoxy-1-piperidinyl; 3-amino-4-methoxy-pyrrolidin-1-yl; 3-amino-5-fluoro-1-piperidinyl; 3-aminoazetidin-1-yl; 3-methylpiperazin-1-yl; 3-oxa-7,9-diazabicyclo[3.3.1]nonan-7-yl; 4-(azetidinyl-2-carbonyl)piperazin-1-yl; 4-[2-(dimethylamino)acetyl]piperazin-1-yl; 4-amino-1-piperidinyl; 4-amino-3,3-difluoro-1-piperidinyl; 4-amino-3,3-difluoro-pyrrolidin-1-yl; 4-amino-3-fluoro-1-piperidinyl; 4-amino-3-hydroxy-3-methyl-pyrrolidin-1-yl; 4-amino-3-methoxy-1-piperidinyl; 4-methylpiperazin-1-yl; 4-piperidinyl; 5-amino-3,3-difluoro-1-piperidinyl; 5-oxa-2,8-diazaspiro[3.5]nonan-2-yl; 5-oxa-2,8-diazaspiro[3.5]nonan-8-yl; 6-amino-1,4-oxazepan-4-yl; 6-hydroxy-1,4-diazepan-1-yl; 6-methyl-2,6-diazaspiro[3.3]heptan-2-yl; aminomethyl; azetidin-3-ylamino; morpholin-2-yl; morpholin-2-ylmethyl or piperazin-1-yl.

A further embodiment of present invention is (vii) a compound of formula (I) or (Ia) according to any one of (i) to (vi), or a pharmaceutically acceptable salt thereof, wherein A is

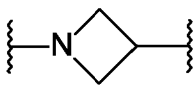,
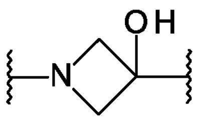,
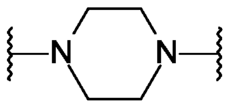,
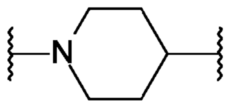,

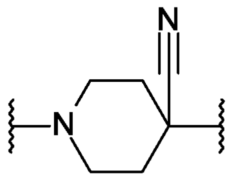 or 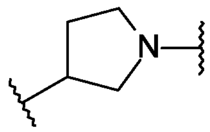.

A further embodiment of present invention is (viii) a compound of formula (I) or (Ia) according to any one of (i) to (vii), or a pharmaceutically acceptable salt thereof, wherein Q is phenyl, fluorophenyl, pyrazinyl, pyridazinyl, pyridinyl, methylpyridinyl, pyrimidinyl, cyanopyrimidinyl, fluoropyrimidinyl, methoxypyrimidinyl, methylpyrimidinyl, dimethylpyrimidinyl or quinazolinyl.

A further embodiment of present invention is (ix) a compound of formula (I) or (Ia) according to any one of (i) to (viii), or a pharmaceutically acceptable salt thereof, wherein $R^3$ is methyl.

A further embodiment of present invention is (x) a compound of formula (I) or (Ia) according to any one of (i) to (ix), or a pharmaceutically acceptable salt thereof, wherein $R^1$ is

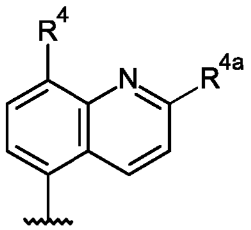, 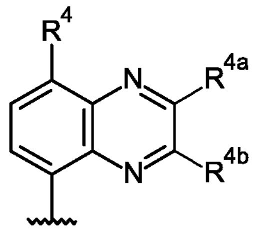 or 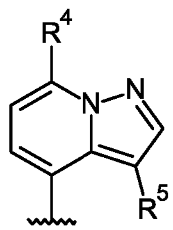;

wherein $R^4$ is $C_{1-6}$alkyl, halo$C_{1-6}$alkyl or cyano; $R^{4a}$ and $R^{4b}$ are simultaneously H or deuterium; $R^5$ is H or halogen.

A further embodiment of present invention is (xi) a compound of formula (I) or (Ia) according to any one of (i) to (x), or a pharmaceutically acceptable salt thereof, wherein $R^4$ is methyl, trifluoromethyl or cyano; $R^{4a}$ and $R^{4b}$ are simultaneously H or deuterium; $R^5$ is H or fluoro.

A further embodiment of present invention is (xii) a compound of formula (I) or (Ia) according to any one of (i) to (xi), or a pharmaceutically acceptable salt thereof, wherein $R^2$ is 3,4,4a,5,7,7a-hexahydro-2H-pyrrolo[3,4-b][1,4]oxazinyl; 5-oxa-2,8-diazaspiro[3.5]nonanyl; amino($C_{1-6}$alkoxy)pyrrolidinyl; amino($C_{1-6}$alkyl)azetidinyl; aminoazetidinyl; aminopiperidinyl; $C_{1-6}$alkyl-2,6-diazaspiro[3.3]heptanyl; halopyrrolidinylamino; morpholinyl; morpholinylCi-ealkyl or piperazinyl.

A further embodiment of present invention is (xiii) a compound of formula (I) or (Ia) according to any one of (i) to (xii), or a pharmaceutically acceptable salt thereof, wherein $R^2$ is (4-fluoropyrrolidin-3-yl)amino; 3,4,4a,5,7,7a-hexahydro-2H-pyrrolo[3,4-b][1,4]oxazin-6-yl; 3-amino-3-methyl-azetidin-1-yl; 3-amino-4-methoxy-pyrrolidin-1-yl; 3-aminoazetidin-1-yl; 4-amino-1-piperidinyl; 5-oxa-2,8-diazaspiro[3.5]nonan-2-yl; 6-methyl-2,6-diazaspiro[3.3]heptan-2-yl; morpholin-2-yl; morpholin-2-ylmethyl or piperazin-1-yl.

A further embodiment of present invention is (xiv) a compound of formula (I) or (Ia) according to any one of (i) to (xiii), or a pharmaceutically acceptable salt thereof, wherein A is

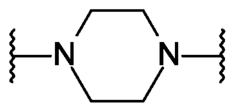

.

A further embodiment of present invention is (xv) a compound of formula (I) or (Ia) according to any one of (i) to (xiv), or a pharmaceutically acceptable salt thereof, wherein Q is phenyl, pyridinyl, pyrimidinyl or $C_{1-6}$alkylpyrimidinyl.

A further embodiment of present invention is (xvi) a compound of formula (I) or (Ia) according to any one of (i) to (xv), or a pharmaceutically acceptable salt thereof, wherein Q is phenyl, pyridinyl, pyrimidinyl or methylpyrimidinyl.

A further embodiment of present invention is (xvii) a compound of formula (I) or (Ia) according to any one of (i) to (xvi), wherein

$R^1$ is

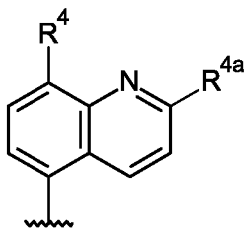 , 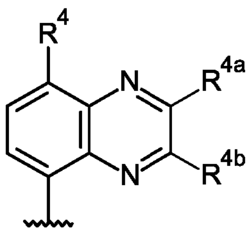 or 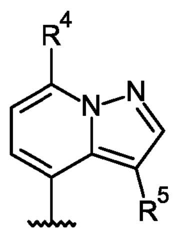 ;

wherein $R^4$ is $C_{1-6}$alkyl, halo$C_{1-6}$alkyl or cyano; $R^{4a}$ and $R^{4b}$ are simultaneously H or deuterium; $R^5$ is H or halogen;
$R^2$ is 3,4,4a,5,7,7a-hexahydro-2H-pyrrolo[3,4-b][1,4]oxazinyl; 5-oxa-2,8-diazaspiro[3.5]nonanyl; amino($C_{1-6}$alkoxy)pyrrolidinyl; amino($C_{1-6}$alkyl)azetidinyl; aminoazetidinyl; aminopiperidinyl; $C_{1-6}$alkyl-2,6-diazaspiro[3.3]heptanyl; halopyrrolidinylamino; morpholinyl; morpholinylCi-ealkyl or piperazinyl;
$R^3$ is $C_{1-6}$alkyl;
A is

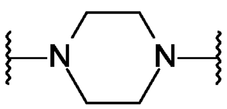

;

Q is phenyl, pyridinyl, pyrimidinyl or $C_{1-6}$alkylpyrimidinyl;
or a pharmaceutically acceptable salt thereof.

A further embodiment of present invention is (xviii) a compound of formula (I) or (Ia) according to any one of (i) to (xvii), wherein

$R^1$ is

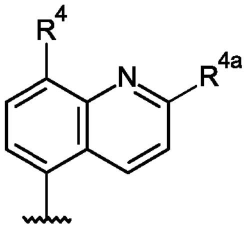, 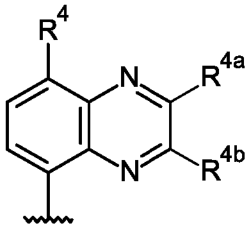 or 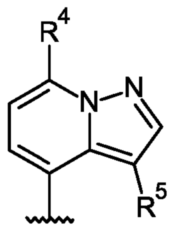;

wherein $R^4$ is methyl, trifluoromethyl or cyano; $R^{4a}$ and $R^{4b}$ are simultaneously H or deuterium; $R^5$ is H or fluoro; $R^2$ is (4-fluoropyrrolidin-3-yl)amino; 3,4,4a,5,7,7a-hexahydro-2H-pyrrolo[3,4-b][1,4]oxazin-6-yl; 3-amino-3-methyl-azetidin-1-yl; 3-amino-4-methoxy-pyrrolidin- 1-yl; 3-aminoazetidin-1-yl; 4-amino-1-piperidinyl; 5-oxa-2,8-diazaspiro[3.5]nonan-2-yl; 6-methyl-2,6-diazaspiro[3.3]heptan-2-yl; morpholin-2-yl; morpholin-2-ylmethyl or piperazin-1-yl;

$R^3$ is methyl;

A is

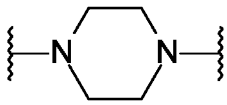;

Q is phenyl, pyridinyl, pyrimidinyl or methylpyrimidinyl;

or a pharmaceutically acceptable salt thereof.

The present invention relates to (i') a compound of formula (I),

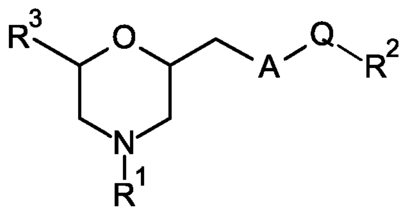 (I),

wherein

$R^1$ is selected from

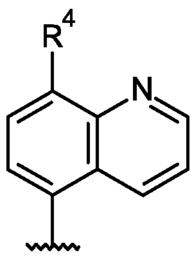, 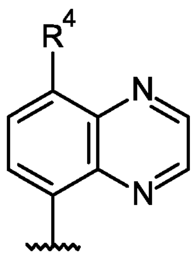, 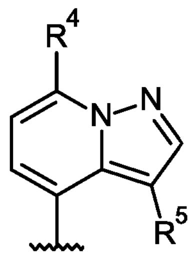, 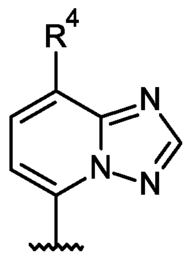

and

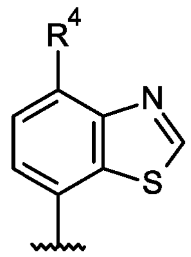

;

wherein $R^4$ is selected from halogen, cyano, $C_{1-6}$alkyl, halo$C_{1-6}$alkyl and $C_{2-6}$alkynyl; $R^5$ is halogen;
$R^2$ is selected from ((($C_{1-6}$alkyl)$_2$amino)$C_{1-6}$alkylcarbonyl)piperazinyl; (($C_{1-6}$alkyl)$_2$amino)$C_{1-6}$alkoxy; (azetidinylcarbonyl)piperazinyl; ($C_{1-6}$alkoxypyrrolidinyl)amino; (cyanopyrrolidinyl)amino; (hydroxy$C_{1-6}$alkyl)piperazinyl; (pyrrolidinylcarbonyl)piperazinyl; 3-oxa-7,9-diazabicyclo[3.3.1]nonanyl; 5-oxa-2,8-diazaspiro[3.5]nonanyl; amino($C_{1-6}$alkoxy)pyrrolidinyl; aminoazetidinyl; amino$C_{1-6}$alkyl; aminohalopiperidinyl; aminohalopyrrolidinyl; aminooxazepanyl; aminopiperidinyl; azetidinylamino; $C_{1-6}$alkylpiperazinyl; halopyrrolidinylamino; hydroxydiazepanyl; morpholinyl; piperazinyl and piperidinyl;
$R^3$ is $C_{1-6}$alkyl;
A is selected from azetidinyl, piperazinyl, pyrrolidinyl, piperidinyl and caynopiperidinyl;
Q is selected from phenyl;

pyrazinyl;
pyridazinyl;
pyridinyl;
pyrimidinyl, said pyrimidinyl being unsubstituted or substituted once or twice by substituents independently selected from halogen, $C_{1-6}$alkyl, $C_{1-6}$alkoxy and cyano; and
quinazolinyl;

or a pharmaceutically acceptable salt thereof.

Another embodiment of present invention is (ii') a compound of formula (Ia),

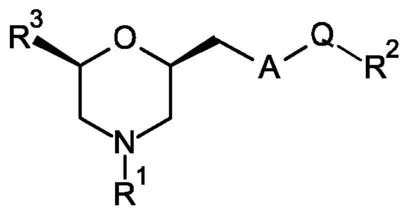

(Ia),

wherein

$R^1$ is selected from

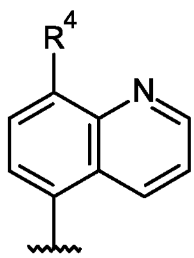

,

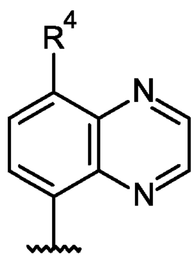

,

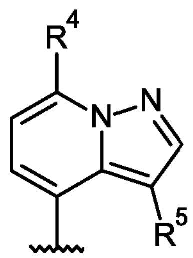

,

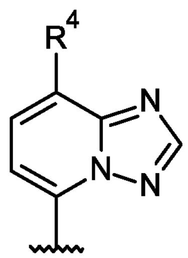

and

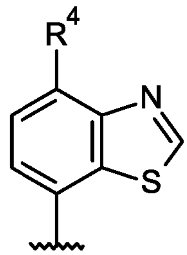

;

wherein $R^4$ is selected from halogen, cyano, $C_{1-6}$alkyl, halo$C_{1-6}$alkyl and $C_{2-6}$alkynyl; $R^5$ is halogen;
$R^2$ is selected from ((($C_{1-6}$alkyl)$_2$amino)$C_{1-6}$alkylcarbonyl)piperazinyl; (($C_{1-6}$alkyl)$_2$amino)$C_{1-6}$alkoxy; (azetidinylcarbonyl)piperazinyl; ($C_{1-6}$alkoxypyrrolidinyl)amino; (cyanopyrrolidinyl)amino; (hydroxy$C_{1-6}$alkyl)piperazinyl; (pyrrolidinylcarbonyl)piperazinyl; 3-oxa-7,9-diazabicyclo[3.3.1]nonanyl; 5-oxa-2,8-diazaspiro[3.5]nonanyl; amino($C_{1-6}$alkoxy)pyrrolidinyl; aminoazetidinyl; amino$C_{1-6}$alkyl; aminohalopiperidinyl; aminohalopyrrolidinyl; aminooxazepanyl; aminopiperidinyl; azetidinylamino; $C_{1-6}$alkylpiperazinyl; halopyrrolidinylamino; hydroxydiazepanyl; morpholinyl; piperazinyl and piperidinyl;
$R^3$ is $C_{1-6}$alkyl;
A is selected from azetidinyl, piperazinyl, pyrrolidinyl, piperidinyl and caynopiperidinyl;
Q is selected from phenyl;

- pyrazinyl;
- pyridazinyl;
- pyridinyl;
- pyrimidinyl, said pyrimidinyl being unsubstituted or substituted once or twice by substituents independently selected from halogen, $C_{1-6}$alkyl, $C_{1-6}$alkoxy and cyano; and
- quinazolinyl;

or a pharmaceutically acceptable salt thereof.

A further embodiment of present invention is (iii') a compound of formula (I) or (Ia) according to (i) or (ii), or a pharmaceutically acceptable salt thereof, wherein $R^1$ is

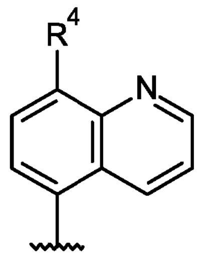

;

wherein $R^4$ is cyano.

A further embodiment of present invention is (iv') a compound of formula (I) or (Ia) according to any one of (i') to (iii'), or a pharmaceutically acceptable salt thereof, wherein A is piperazinyl.

A further embodiment of present invention is (v') a compound of formula (I) or (Ia) according to any one of (i') to (iv'), or a pharmaceutically acceptable salt thereof, wherein Q is pyrimidinyl, said pyrimidinyl being unsubstituted or substituted by $C_{1-6}$alkyl.

A further embodiment of present invention is (vi') a compound of formula (I) or (Ia) according to any one of (i') to (v'), or a pharmaceutically acceptable salt thereof, wherein $R^2$ is selected from amino($C_{1-6}$alkoxy)pyrrolidinyl, aminopiperidinyl and piperazinyl.

A further embodiment of present invention is (vii') a compound of formula (I) or (Ia) according to any one of (i') to (vi'), or a pharmaceutically acceptable salt thereof, wherein $R^2$ is selected from amino(methoxy)pyrrolidinyl, aminopiperidinyl and piperazinyl.

A further embodiment of present invention is (viii') a compound of formula (I) or (Ia) according to any one of (i') to (vii'), wherein

$R^1$ is selected from

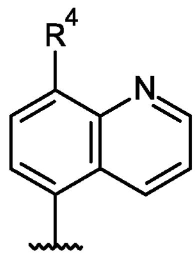

;

wherein $R^4$ is cyano;
$R^2$ is selected from amino($C_{1-6}$alkoxy)pyrrolidinyl, aminopiperidinyl and piperazinyl;
$R^3$ is $C_{1-6}$alkyl;
A is piperazinyl;
Q is pyrimidinyl, said pyrimidinyl being unsubstituted or substituted by $C_{1-6}$alkyl; or a pharmaceutically acceptable salt thereof.

A further embodiment of present invention is (ix') a compound of formula (I) or (Ia) according to any one of (i') to (viii'), wherein

$R^1$ is selected from

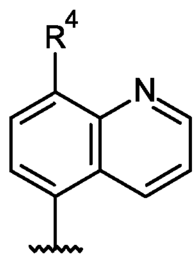

;

wherein $R^4$ is cyano;
$R^2$ is selected from amino(methoxy)pyrrolidinyl, aminopiperidinyl and piperazinyl;
$R^3$ is methyl;
A is piperazinyl;
Q is pyrimidinyl, said pyrimidinyl being unsubstituted or substituted by methyl;
or a pharmaceutically acceptable salt thereof.

Any of the above embodiments may be combined.
Another embodiment of present invention is that (x) a compound of formula (I) or (Ia) selected from the following:

5-[(*2R*,*6S*)-2-methyl-6-[[3-(3-piperazin-1-ylphenyl)pyrrolidin-1-yl]methyl] morpholin-4-yl] quinoline- 8-carbonitrile;
5-[(*2R*,*6S*)-2-methyl-6-[[4-[3-(4-piperidyl)phenyl] -1-piperidyl] methyl] morpholin-4-yl] quinoline- 8-carbonitrile;
5-[(*2R*,*6S*)-2-methyl-6-[[3-(4-piperazin-1-ylphenyl)azetidin-1-yl] methyl] morpholin-4-yl] quinoline- 8-carbonitrile;
5-[(*2R*,*6S*)-2-methyl-6-[[3-(3-piperazin-1-ylphenyl)azetidin-1-yl] methyl] morpholin-4-yl] quino line- 8-carbonitrile;
5-[(*2S*,*6R*)-2-[[4-[4-(aminomethyl)phenyl]piperazin-1-yl]methyl]-6-methyl-morphofin-4-yl] quinoline- 8-carbonitrile;
5-[(*2R*,*6S*)-2-methyl-6-[[4-(5-piperazin-1-ylpyrazin-2-yl)piperazin-1-yl]methyl]morpholin-4-yl]quino line- 8-carbonitrile;
5-[(*2R*,*6S*)-2-methyl-6-[[4-(5-piperazin-1-yl-3-pyridyl)piperazin-1-yllmethyllmorpholin-4-yl] quinoline- 8-carbonitrile;
5-[(*2R*,*6S*)-2-methyl-6-[[4-(5-piperazin-1-yl-2-pyridyl)piperazin-1-yl] methyl] morpho lin-4-yl] quinoline- 8-carbonitrile;
5-[(*2R*,*6S*)-2-methyl-6-[[4-(4-piperazin-1-yl-2-pyridyl)piperazin-1-yl] methyl] morpho lin-4-yl] quinoline- 8-carbonitrile;
5-[(*2R*,*6S*)-2-methyl-6-[[4-(6-piperazin-1-yl-2-pyridyl)piperazin-1-yl] methyl] morpho lin-4-yl] quinoline- 8-carbonitrile;

5-[(2*S*,6*R*)-2-[[4-cyano-4-(4-piperazin-1-ylphenyl)-1-piperidyl]methyl]-6-methyl-morpholin-4-yl]quinoline-8-carbonitrile;
5-[(2*R*,6*S*)-2-methyl-6-[[4-(2-piperazin-1-ylpyrimidin-5-yl)piperazin-1-yl]methyl]morpholin-4-yl]quinoline-8-carbonitrile;
5-[(2*R*,6*S*)-2-methyl-6-[[4-(6-piperazin-1-yl-3-pyridyl)piperazin-1-yl]methyl]morpholin-4-yl] quinoline- 8-carbonitrile;
5-[(2*R*,6*S*)-2-methyl-6-[[4-(4-piperazin-1-ylpyrimidin-2-yl)piperazin-1-yl]methyl]morpholin-4-yl]quinoline-8-carbonitrile;
5-[(2*R*,6*S*)-2-methyl-6-[[4-(2-piperazin-1-ylpyrimidin-4-yl)piperazin-1-yl]methyl]morpholin-4-yl]quinoline-8-carbonitrile;
5-[(2*R*,6*S*)-2-methyl-6-[[4-(6-piperazin-1-ylpyridazin-3-yl)piperazin-1-yl]methyl]morpholin-4-yl]quinoline-8-carbonitrile;
5-[(2*R*,6*S*)-2-methyl-6-[[4-(5-piperazin-1-ylpyrimidin-2-yl)piperazin-1-yl]methyl]morpholin-4-yl]quinoline-8-carbonitrile;
5-[(2*R*,6*S*)-2-methyl-6-[[4-(6-piperazin-1-ylpyrimidin-4-yl)piperazin-1-yl]methyl]morpholin-4-yl]quinoline-8-carbonitrile;
5-[(2*R*,6*S*)-2-methyl-6-[[4-[4-[(2*R*)-morphofin-2-yl]phenyl]piperazin-1-yl]methyl]morpholin-4-yl]quinoline-8-carbonitrile;
5-[(2*R*,6*S*)-2-methyl-6-[[4-[4-[(2*S*)-morpholin-2-yl]phenyllpiperazin- 1-yl]methyl]morpholin-4-yl]quinoline-8-carbonitrile;
5-[(2*R*,6*S*)-2-methyl-6-[[4-[6-(4-methylpiperazin-1-yl)pyrimidin-4-yl]piperazin-1-yl]methyl]morpholin-4-yl]quinoline-8-carbonitrile;
5-[(2*S*,6*R*)-2-[[4-[2-(3-aminoazetidin-1-yl)pyrimidin-4-yl]piperazin-1-yl]methyl]-6-methyl-morpholin-4-yl]quinoline-8-carbonitrile;
5-[(2*S*,6*R*)-2-[[4-[2-(3-aminoazetidin-1-yl)pyrimidin-4-yl]piperazin-1-yl]methyl]-6-methyl-morpholin-4-yl]quinoline-8-carbonitrile;
5-[(2*S*,6*R*)-2-[[4-[2-[[(3*S*,4*R*)-4-fluoropyrrolidin-3-yl]amino]pyrimidin-4-yl]piperazin-1-yl]methyl]-6-methyl-morpholin-4-yl]quinoline-8-carbonitrile;
5-[(2*S*,6*R*)-2-[[4-[2-(6-hydroxy-1,4-diazepan-1-yl)pyrimidin-4-yl]piperazin-1-yl]methyl]-6-methyl-morpholiin-4-yl]quinoline-8-carbonitrile;
5-[(2*S*,6*R*)-2-[[4-[2-*trans*-(3-amino-4-methoxy-pyrrolidin-1-yl)pyrimidin-4-yl]piperazin-1-yl]methyl]-6-methyl-morpholin-4-yl]quinoline-8-carbonitrile;
5-[(2*S*,6*R*)-2-[[4-[2-(4-amino-1-piperidyl)pyrimidin-4-yl]piperazin-1-yl]methyl]-6-methyl-morpholin-4-yl]quinoline-8-carbonitrile;
5-[(2*S*,6*R*)-2-[[4-[2-(azetidin-3-ylamino)pyrimidin-4-yl]piperazin-1-yl] methyl]-6-methyl-morpholin-4-yl]quinoline-8-carbonitrile;
5-[(2*S*,6*R*)-2-[[4-[2-[(3R)-3-(hydroxymethyl)piperazin-1-yl]pyrimidin-4-yl]piperazin-1-yl]methyl]-6-methyl-morpholin-4-yl]quinoline-8-carbonitrile;
5-[(2*S*,6*R*)-2-[[4-[2-[2-(dimethylamino)ethoxy]pyrimidin-4-yl]piperazin-1-yl] methyl]-6-methyl-morpholin-4-yl]quinoline-8-carbonitrile;
5-[(2*R*,6*S*)-2-methyl-6-[[4-[2-[4-[(2S)-pyrrolidine-2-carbonyl]piperazin-1-yl]pyrimidin-4-yl]piperazin-1-yl]methyl]morpholin-4-yl]quinoline-8-carbonitrile;
5-[(2*S*,6*R*)-2-[[4-[2-[4-[2-(dimethylamino)acetyl]piperazin-1-yl]pyrimidin-4-yl]piperazin-1-yl]methyl]-6-methyl-morpholin-4-yl]quinoline-8-carbonitrile;
5-[(2*R*,6*S*)-2-methyl-6-[[4-[6-(4-methylpiperazin-1-yl)-2-pyridyl]piperazin-1-yl]methyl]morpholin-4-yl]quinoline-8-carbonitrile;
5-[(2*S*,6*R*)-2-[[4-[2-[4-(azetidine-2-carbonyl)piperazin-1-yl]pyrimidin-4-yl]piperazin-1-yl]methyl]-6-methyl-morpholin-4-yl]quinoline-8-carbonitrile;
5-[(2S,6*R*)-2-[[4-[6-[[(3*S*,4*S*)-4-fluoropyrrolidin-3-yl]amino]-2-pyridyl]piperazin-1-yl]methyl]-6-methyl-morpholin-4-yl]quinoline-8-carbonitrile;
5-[(2*S*,6*R*)-2-[[4-[6-(4-amino-1-piperidyl)-2-pyridyl]piperazin-1-yl]methyl]-6-methyl-morpholin-4-yl]quinoline-8-carbonitrile;
5-[(2*S*,6*R*)-2-[[4-[6-[[(3*S*,4*R*)-4-fluoropyrrolidin-3-yl]amino]-2-pyridyl]piperazin-1-yl] methyl] -6- methyl- morpholin-4-yl]quinoline-8-carbonitrile;
5-[(2*S*,6*R*)-2-[[4-[6-(4-amino-1-piperidyl)-2-pyridyl]piperazin-1-yl]methyl]-6-methyl-morpholin-4-yl]quinoline-8-carbonitrile;
5-[(2*S*,6*R*)-2-[[4-[6-[[(3*R*,4*R*)-4-methoxypyrrolidin-3-yl]amino]-2-pyridyl]piperazin-1-yl]methyl]-6-methyl-morpholin-4-yl]quinoline-8-carbonitrile;
5-[(2*S*,6*R*)-2-[[4-[6-(3-amino-4-fluoro-pyrrolidin-1-yl)-2-pyridyl]piperazin-1-yl]methyl]-6-methyl-morpholin-4-yl]quinoline-8-carbonitrile;
5-[(2*S*,6*R*)-2-[[4-[6-(6-hydroxy-1,4-diazepan-1-yl)-2-pyridyl]piperazin-1-yl]methyl]-6-methyl-morpholin-4-yl]quinoline-8-carbonitrile;
*5*-[(2*S*,*6R)*-2-[[4-[6-[(*3R,4S*)-3-amino-4-methoxy-pyrrolidin-1-yl]-2-pyridyl]piperazin-1-yl] methyl] -6- methyl- morpholin-4- yl]quinoline-8-carbonitrile;
5-[(2*S*,6*R*)-2-[[4-[6-(3-amino-4-methoxy-pyrrolidin-1-yl)-3-pyridyl]piperazin-1-yl]methyl]-6-methyl-morpholin-4-yl]quinoline-8-carbonitrile;
5-[(*2S*,*6R*)-2-[[4-[6-(3-amino-4-fluoro-pyrrolidin-1-yl)-3-pyridyl]piperazin-1-yl]methyl]-6-methyl-morpholin-4-yl]quinoline-8-carbonitrile;
5-[(*2S*,*6R*)-2-[[4-(5-fluoro-2-piperazin-1-yl-pyrimidin-4-yl)piperazin-1-yl]methyl]-6-methyl-morpholin-4-yl]quinoline-8-carbonitrile;
5-[(2*S*,6*R*)-2-[[4-(5,6-dimethyl-2-piperazin-1-yl-pyrimidin-4-yl)piperazin-1-yl] methyl]-6-methyl-morpholin-4-yl]quinoline-8-carbonitrile;
5-[(*2R*,*6S*)-2-methyl-6-[[4-(6-methyl-2-piperazin-1-yl-pyrimidin-4-yl)piperazin-1-yl]methyl]morpholin-4-yl]quinoline-8-carbonitrile;
5-[(*2S*,*6R*)-2-[[4-(5-methoxy-2-piperazin-1-yl-pyrimidin-4-yl)piperazin-1-yl]methyl]-6-methyl-morpholin-4-yl]quinoline-8-carbonitrile;
5-[(*2R*,*6S*)-2-methyl-6-[[4-(2-piperazin-1-ylquinazolin-4-yl)piperazin-1-yl]methyl]morpholin-4-yl]quinoline-8-carbonitrile;
5-[(2S,6R)-2-[[4-(5-cyano-2-piperazin-1-yl-pyrimidin-4-yl)piperazin-1-yl]methyl]-6-methyl-morpholin-4-yl]quinoline-8-carbonitrile;
5-[(*2S*,*6R*)-2-[[4-(5-cyano-4-piperazin-1-yl-pyrimidin-2-yl)piperazin-1-yl]methyl]-6-methyl-morpholin-4-yl]quinoline-8-carbonitrile;
5-[(*2S*,*6R*)-2-[[4-[2-[(*3R*,*4R*)-3-amino-4-methoxy-pyrrolidin-1-yl]pyrimidin-4-yl]piperazin-1-yl]methyl]-6-methyl-morpholin-4-yl]quinoline-8-carbonitrile;
5-[(*2S*,*6R*)-2-[[4-[2-[(*3S*,*4S*)-3-amino-4-methoxy-pyrrolidin-1-yl]pyrimidin-4-yl]piperazin-1-yl]methyl]-6-methyl-morpholin-4-yl]quinoline-8-carbonitrile;
5-[(2*S*,6*R*)-2-[[4-[2-[(3*S*,4*R*)-3-amino-4-fluoro-pyrrolidin-1-yl]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(3*R*,4*S*)-3-amino-4-fluoro-pyrrolidin-1-yl]-6-methyl-pyrimidin-4-yl]piperazin-1-yl]methyl]-6-methyl-morpholin-4-yl]quinoline-8-carbonitrile;
5-[(*2S*,*6R*)-2-[[4-[2-(6-amino-1,4-oxazepan-4-yl)-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(4-cyanopyrrolidin-3-yl)amino]-6-methyl-pyrimidin-4-yl]piperazin-1-yl]methyl] -6-methyl-morpholin-4-yl]quinoline-8-carbonitrile;
5-[(2*R*,6*S*)-2-methyl-6-[[4-[6-methyl-2-[(1*R*,5*R*)-3-oxa-7,9-diazabicyclo[3.3.1]nonan-7-yl]pyrimidin-4-yl]piperazin-1-yl]methyl]morpholin-4-yl]quinoline-8-carbonitrile;
5-[(2*R*,6*S*)-2-methyl-6-[[4-[6-methyl-2-[trans-3-amino-4-methoxy-pyrrolidin-1-yl]pyrimidin-4-yl]piperazin-1-yl]methyl]morpholin-4-yl]quinoline-8-carbonitrile;
5-[(2*R*,6*S*)-2-methyl-6-[[4-[6-methyl-2-[*cis*-3-amino-4-methoxy-pyrrolidin-1-yl]pyrimidin-4-yl]piperazin-1-yl]methyl]morpholin-4-yl]quinoline-8-carbonitrile;
5-[(2*R*,6*S*)-2-methyl-6-[[4-[6-methyl-2-(5-oxa-2,8-diazaspiro[3.5]nonan-2-yl)pyrimidin-4-yl]piperazin-1-yl]methyl]morpholin-4-quinoline-8-carbonitrile;
5-[(*2R*,*6S*)-2-methyl-6-[[4-(3-morpholin-2-ylphenyl)piperazin-1-yl]methyl]-morpholin-4-yl] quinoline- 8-carbonitrile;
5-[(2*S*,6*R*)-2-[[4-[2-(4-amino-3,3-difluoro-pyrrolidin-1-yl)-6-methyl-pyrimidin-4-yl]piperazin-1-yl]methyl]-6-methyl-morpholin-4-yl]quinoline-8-carbonitrile;
8-[(2*R*,6*S*)-2-methyl-6-[[4-(6-methyl-2-piperazin-1-yl-pyrimidin-4-yl)piperazin-1-yl]methyl]morpholin-4-yl]quinoxaline-5-carbonitrile;
(2*R*,6*S*)-2-methyl-6-[[4-(6-methyl-2-piperazin-1-yl-pyrimidin-4-yl)piperazin-1-yl] methyl] - 4-[8-(trifluoromethyl)quinoxalin-5-yl]morpholine;
5-[(*2S*,*6R*)-2-[[4-[2-(6-hydroxy-1,4-diazepan-1-yl)-6-methyl-pyrimidin-4-yl]piperazin-1-yl]methyl]-6-methyl-morpholin-4-yl]quinoline-8-carbonitrile;
3-Fluoro-4-[(2*R*,6*S*)-2-methyl-6-[[4-(6-methyl-2-piperazin-1-yl-pyrimidin-4-yl)piperazin-1-yl]methyl]morpholin-4-yl]pyrazolo[1,5-a]pyridine-7-carbonitrile;
5-[(2*R*,6*S*)-2-methyl-6-[[4-(4-methyl-6-piperazin-1-yl-pyrimidin-2-yl)piperazin-1-yl]methyl]morpholin-4-yl]quinoline-8-carbonitrile;
5-[(*2S*,*6R*)-2-[[4-[2-*trans*-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;

5-[(2S,6R)-2-[[4-[2-[(3S,4S)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(2S,6R)-2-[[4-[2-[(3R,4R)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(2*S*,6R)-2-[[4-[2[[(3*R*,4*R*)-4-methoxypyrrolidin-3-yl]amino]-6-methyl-pyrimidin-4-yl]piperazin-1-yl]methyl]-6-methyl-morpholin-4-yl]quinoline-8-carbonitrile;
5-[(2*S*,6R)-2-[[4-[2-[[(3*R*,4*S*)-4-fluoropyrrolidin-3-yl]amino]-6-methyl-pyrimidin-4-yl]piperazin-1-yl]methyl]-6-methyl-morpholin-4-yl]quinoline-8-carbonitrile;
5-[(2*S*,6R)-2-[[4-[2-[(*3S*,*4R*)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(2*S*,6R)-2-[[4-[2-[[(3*S*,4*R*)-4-fluoropyrrolidin-3-yl]amino]-6-methyl-pyrimidin-4-yl]piperazin-1-yl]methyl]-6-methyl-morpholin-4-yl]quinoline-8-carbonitrile;
5-[(2*R*,6*S*)-2-methyl-6-[[4-[6-methyl-2-[cis-3-amino-5-fluoro-1-piperidyl]pyrimidin-4-yl]piperazin-1-yl]methyl]morpholin-4-yl]quinoline-8-carbonitrile;
5-[(2R,6S)-2-methyl-6-[[4-[6-methyl-2-[trans-3-amino-5-fluoro-1-piperidyl]pyrimidin-4-yl]piperazin-1-yl]methyl]morpholin-4-yl]quinoline-8-carbonitrile;
5-[(2*S*,6*R*)-2-[[4-[2-[[(3*R*,4*R*)-4-fluoropyrrolidin-3-yl]amino]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(3S,SR)-3-amino-5-fluoro-1-piperidyl]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(3*S*,4*R*)-4-amino-3-fluoro-1-piperidyl]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(3*R*,4*R*)-3-amino-4-fluoro-pyrrolidin-1-yl]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-(5-amino-3,3-difluoro-1-piperidyl)-6-methyl-pyrimidin-4-yl]piperazin-1 -yl] methyl] -6-methyl-morpholin-4-yl]quinoline-8-carbonitrile;
5-[(*2S*,*6R*)-2-[[4-[2-[3-(1-hydroxy-1-methyl-ethyl)piperazin-1-yl]-6-methyl-pyrimidin-4-yl]piperazin-1-yl]methyl]-6-methyl-morpholin-4-yl]quinoline-8-carbonitrile;
5-[(*2S*,*6R*)-2-[[4-[2-[(*3R*,*4R*)-4-amino-3-fluoro-1-piperidyl] -6-methyl-pyrimidin-4-yl]piperazin-1-yl]methyl]-6-methyl-morpholin-4-yl]quinoline-8-carbonitrile;
5-[(*2R*,*6S*)-2-methyl-6-[[4-[6-methyl-2-[(*3S*,*4R*)-3-amino-4-fluoro-1-piperidyl]-pyrimidin-4-yl]piperazin-1-yl]methyl]morpholin-4-yl]quinoline-8-carbonitrile;
5-[(*2R*,*6S*)-2-methyl-6-[[4-[6-methyl-2-[ (3R,4R)-3-amino-4-fluoro-1-piperidyl]pyrimidin-4-yl]piperazin-1-yl]methyl]morphofin-4-yl]quinoline-8-carbonitrile;
5-[(*2S*,*6R*)-2-[[4-[2-(azetidin-3-ylamino)-6-methyl-pyrimidin-4-yl]piperazin-1-yl]methyl]-6-methyl-morpholin-4-yl]quinoline-8-carbonitrile;
5-[(*2S*,*6R*)-2-[[4-[2-(4-amino-3,3-difluoro-1-piperidyl)-6-methyl-pyrimidin-4-yl]piperazin-1-yl]methyl] -6-methyl-morpholin-4-yl]quinoline-8-carbonitrile;
5-[(*2S*,*6R*)-2-[[4-[2-(3-amino azetidin-1-yl)-6-methyl-pyrimidin-4-yl]piperazin-1-yl] methyl]-6-methyl-morpholin-4-yl]quinoline-8-carbonitrile;
5-[(*2S*,*6R*)-2-[[4-[2-[(*3R*,*4S*)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(*2R*,*6S*)-2-methyl-6-[[4-(2-morpholin-2-ylphenyl)piperazin-1-yl]methyl]fin-4-yl] quinoline- 8-carbonitrile;
5-[(*2S*,*6R*)-2-[[4-[2-[(*3S*,*4S*)-4-amino-3-methoxy-1-piperidyl]-6-methyl-pyrimidin-4-yl]piperazin-1-yl]methyl]-6-methyl-morpholin-4-yl]quinoline-8-carbonitrile;
5-[(*2S*,*6R*)-2-[[4-(6-methoxy-2-piperazin-1-yl-pyrimidin-4-yl)piperazin-1-yl]methyl]-6-methyl-morpholin-4-yl]quinoline-8-carbonitrile;
5-[(*2S*,*6R*)-2-[[4-[2-[(*3S*,*4R*)-3-amino-4-methoxy-pyrrolidin-1-yl]pyrimidin-4-yl]piperazin-1-yl]methyl]-6-methyl-morpholin-4-yl]quinoline-8-carbonitrile;
5-[(*2S*,*6R*)-2-[[4-[2-[(*3R*,*4*S)-3-amino-4-methoxy-pyrrolidin-1-yl]pyrimidin-4-yl]piperazin-1-yl]methyl]-6-methyl-morpholin-4-yl]quinoline-8-carbonitrile;
5-[(*2S*,*6R*)-2-[[4-(5-fluoro-4-piperazin-1-yl-pyrimidin-2-yl)piperazin-1-yl]methyl]-6-methyl-morpholin-4-yl]quinoline-8-carbonitrile;
*5-[(2S, 6R)-2-[[4-[2-[(3S, 4R)-3-amino-4-fluoro-pyrrolidin-1-yl]pyrimidin-4-yl]piperazin-1*-yl] methyl] -6- methyl- morpholin-4- yl]quinoline-8-carbonitrile;
5-[(*2S*,*6R*)-2-[[4-[2-[(*3R*,*4R*)-3-amino-4-fluoro-pyrrolidin-1-yl]pyrimidin-4-yl]piperazin-1-yl] methyl] -6- methyl- morpholin-4- yl]quinoline-8-carbonitrile;
5-[(*2S*,*6R*)-2-[[4-[4-[(*3S*,*4S*)-3-amino-4-methoxy-pyrrolidin-1-yl]pyrimidin-2-yl]piperazin-1-yl]methyl]-6-methyl-morpholin-4-yl]quinoline-8-carbonitrile;
*8-[(2S, 6R)-2-[[4-[2-[(3R, 4R)-3-amino-4-methoxy-pyrrolidin-1-yl]pyrimidin-4*-yl]piperazin-1-yl]methyl]-6-methylmorpholin-4-yl]quinoxaline-5-carbonitrile;
8-[(*2S*,*6R*)-2-[[4-[2-[(*3S*,*4S*)-3-amino-4-methoxy-pyrrolidin-1-yl]pyrimidin-4-yl]piperazin-1- yl] methyl] -6- methylmorpholin-4- yl]quinoxaline- 5-carbonitrile;
*5-[(2S, 6R)-2-[[4-[2-[(3R, 4S)-3-amino-4-fluoro-pyrrolidin-1-yl]pyrimidin-4-yl]piperazin-1*-yl] methyl] -6- methyl- morpholin-4- yl]quinoline-8-carbonitrile;
5-[(*2R*,*6S*)-2-methyl-6-[[4-(5-methyl-4-piperazin-1-yl-pyrimidin-2-yl)piperazin-1-yl]methyl]morpholin-4-yl]quinoline-8-carbonitrile;
5-[(*2S*,*6R*)-2-[[4-[2-[(4*aR*,7*aR*)-3,4,4*a*,5,7,7*a*-hexahydro-2*H*-pyrrolo[3,4-b][1,4]oxazin-6-yl]pyrimidin-4-yl]piperazin-1-yl]methyl]-6-methyl-morpholin-4-yl]quinoline-8-carbonitrile;
8-[(*2S*,*6R*)-2-[[4-[4-[(*3S*,*4S*)-3-amino-4-methoxy-pyrrolidin-1-yl]-6-methyl-pyrimidin-2-yl]piperazin-1-yl] methyl] -6-methyl-morphofin-4-yl] quinoxafine-5-carbonitrile;
8-[(*2S*,*6R*)-2-[[4-[4-[(*3S*,*4S*)-3-amino-4-methoxy-pyrrolidin-1-yl]-6-methyl-pyrimidin-2-yl]piperazin-1-yl]methyl]-6-methyl-morpholin-4-yl]-2,3-dideuterio-quinoxaline-5-carbonitrile;
8-[(*2S*,*6R*)-2-[[4-[2-[(*3S*,*4S*)-3-amino-4-methoxy-pyrrolidin-1-yl]-6-methyl-pyrimidin-4-yl]piperazin-1-yl] methyl] -6-methyl-morphofin-4-yl] quinoxafine-5-carbonitrile;
8-[(*2S*,*6R*)-2-[[4-[2-[(*3S*,*4S*)-3-amino-4-fluoro-pyrrolidin-1-yl]pyrimidin-4-yl]piperazin-1-yl]methyl]-6-methyl-morpholin-4-yl]quinoxaline-5-carbonitrile;
8-[(*2S*,*6R*)-2-[[4-[2-[(*3R*,*4S*)-3-amino-4-fluoro-pyrrolidin-1-yl]pyrimidin-4-yl]piperazin-1-yl]methyl]-6-methyl-morpholin-4-yl]quinoxaline-5-carbonitrile;
5-[(*2S*,*6R*)-2-[[4-[2-(3-amino-4-methoxy-1-piperidyl)pyrimidin-4-yl]piperazin-1-yl]methyl]-6-methyl-morpholin-4-yl]quinoline-8-carbonitrile;
5-[(*2S*,*6R*)-2-[[4-[2-[(*3S*,*4S*)-3-amino-4-fluoro-pyrrolidin-1-yl]pyrimidin-4-yl]piperazin-1-yl]methyl]-6-methyl-morpholin-4-yl]quinoline-8-carbonitrile;
8-[(*2S*,*6R*)-2-[[4-[2-[(*3R*,*4S*)-4-amino-3-fluoro-1-piperidyl]pyrimidin-4-yl]piperazin-1-yl]methyl]-6-methyl-morpholin-4-yl]quinoxaline-5-carbonitrile;
8-[(*2S*,*6R*)-2-[[4-[2-(3-aminoazetidin-1-yl)-6-methyl-pyrimidin-4-yl]piperazin-1-yl]methyl]-6-methyl-morpholin-4-yl]quinoxaline-5-carbonitrile;
8-[(*2S*,*6R*)-2-[[4-[2-[(*3R*,*4R*)-4-amino-3-fluoro-1-piperidyl]pyrimidin-4-yl]piperazin-1-yl]methyl]-6-methyl-morpholin-4-yl]quinoxaline-5-carbonitrile;
8-[(*2R*,*6S*)-2-methyl-6-[[4-(4-methyl-6-piperazin-1-yl-pyrimidin-2-yl)piperazin-1-yl]methyl]morpholin-4-yl]quinoxaline-5-carbonitrile;
*8-[(2S, 6R)-2-[[4-[4-[(3R, 4S)-3-amino-4-fluoro-pyrrolidin-1-yl]-6-methyl-pyrimidin-2*-yl]piperazin-1-yl] methyl] -6-methyl-morphofin-4-yl] quinoxafine-5-carbonitrile;
(*2R*,*6S*)-2-methyl-4-(8-methylquinoxalin-5-yl)-6-[[4-(4-piperazin-1-ylpyrimidin-2-yl)piperazin-1-yl]methyl]morpholine;
(*2R*,*6S*)-2-methyl-4-(8-methylquinoxalin-5-yl)-6-[[4-(2-piperazin-1-ylpyrimidin-4-yl)piperazin-1-yl]methyl]morpholine;
8-[(*2S*,*6R*)-2-[[4-[4-(3-aminoazetidin-1-yl)pyrimidin-2-yl]piperazin-1-yl]methyl]-6-methyl-morpholin-4-yl]quinoxaline-5-carbonitrile;
8-[(*2S*,*6R*)-2-[[4-[4-[(*3R*,*4R*)-3-amino-4-fluoro-pyrrolidin-1-yl]pyrimidin-2-yl]piperazin-1-yl] methyl] -6- methyl- morpholin-4- yl]quinoxaline- 5-carbonitrile;
8-[(*2S*,*6R*)-2-[[4-[4-[(*3S*,*4R*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile;
8-[(*2S*,*6R*)-2-[[4-[4-[(*3R*,*4R*)-3-amino-4-methoxy-pyrrolidin-1-yl]-6-methyl-pyrimidin-2-yl]piperazin-1-yl] methyl] -6-methyl-morphofin-4-yl] quinoxafine-5-carbonitrile;
8-[(*2S*,*6R*)-2-[[4-[4-[(*3*R*,4S*)-3-amino-4-methoxy-pyrrolidin-1-yl]-6-methyl-pyrimidin-2-yl]piperazin-1-yl] methyl] -6-methyl-morphofin-4-yl] quinoxafine-5-carbonitrile;
8-[(*2S*,*6R*)-2-[[4-[4-(3-aminoazetidin-1-yl)-6-methyl-pyrimidin-2-yl]piperazin-1-yl] methyl]-6-methyl-morpholin-4-yl]quinoxaline-5-carbonitrile;
8-[(*2S*,*6R*)-2-[[4-[4-(3-aminoazetidin-1-yl)-6-methyl-pyrimidin-2-yl]piperazin-1-yl]methyl]-6-methyl-morpholin-4-yl]-2,3-dideuterio-quinoxaline-5-carbonitrile;
8-[(*2S*,*6R*)-2-[[4-[4-[(*3R*,*4R*)-4-amino-3-hydroxy-3-methyl-pyrrolidin-1-yl]-6-methyl-pyrimidin-2-yl]piperazin-1-yl] methyl]-6-methyl-morphofin-4-yl] quinoxaline-5-carbonitrile;
2-[4-[4-[[(*2S*,*6R*)-6-methyl-4-(8-methylquinoxalin-5-yl)morpholin-2-yl]methyl]piperazin-1-yl]pyrimidin-2-yl]-5-oxa-2,8-diazaspiro[3.5]nonane;
(*2R*,*6S*)-2-methyl-6-[[4-[2-[(3S)-3-methylpiperazin-1-yl]pyrimidin-4-yl]piperazin-1-yl]methyl]-4-(8-methylquinoxalin-5-yl)morpholine;
(*2R*,*6S*)-2-methyl-6-[[4-[2-[(3*R*)-3-methylpiperazin-1-yl]pyrimidin-4-yl]piperazin-1-yl]methyl]-4-(8-methylquinoxalin-5-yl)morpholine;
(*4aR*,*7aR*)-6-[4-[4-[[(*2S*,*6R*)-6-methyl-4-(8-methylquinoxalin-5-yl)morpholin-2-yl]methyl]piperazin-1-yl]pyrimidin-2-yl]-3,4,4*a*,5,7,7*a*-hexahydro-2*H*-pyrrolo[3,4-b][1,4]oxazine;
1-[4-[4-[[(*2S*,*6R*)-6-methyl-4-(8-methylquinoxalin-5-yl)morphofin-2-yl]methyl]piperazin-1-yl]pyrimidin-2-yl] azetidin-3-amine;
5-[(*2S*,*6R*)-2-[[4-[4-(3-amino-3-methyl-azetidin-1-yl)-6-methoxy-pyrimidin-2-yl]piperazin-1-yl]methyl]-6-methyl-morpholin-4-yl]-2-deuterio-quinoline-8-carbonitrile;
5-[(*2S*,*6R*)-2-[[4-[6-(3-amino-3-methyl-azetidin-1-yl)-3-pyridyl]piperazin-1-yl]methyl]-6-methyl-morpholin-4-yl]-2-deuterio-quinoline-8-carbonitrile;
5-[(*2S*,*6R*)-2-[[4-[6-(3-amino-3-methyl-azetidin-1-yl)-4-methyl-3-pyridyl]piperazin-1-yl] methyl]-6-methyl-morphofin-4-yl]-2-deuterio-quinofine-8-carbonitrile;
5-[(*2S*,*6R*)-2-[[4-[4-[(*3S*,*4S*)-3-amino-4-methoxy-pyrrolidin-1-yl]-6-methyl-pyrimidin-2-yl]piperazin-1-yl]methyl]-6-methyl-morpholin-4-yl]-2-deuterio-quinoline-8-carbonitrile;
5-[(*2S*,*6R*)-2-[[4-[4-(3-amino-3-methyl-azetidin-1-yl)-6-methyl-pyrimidin-2-yl]piperazin-1-yl]methyl]-6-methyl-morpholin-4-yl]-2-deuterio-quinoline-8-carbonitrile;
5-[(*2S*,*6R*)-2-[[4-[4-(3-aminoazetidin-1-yl)-6-methyl-pyrimidin-2-yl]piperazin-1-yl]methyl]-6-methyl-morpholin-4-yl]-2-deuterio-quinoline-8-carbonitrile;
5-[(*2S*,*6R*)-2-[[4-[6-(3-amino-3-methyl-azetidin-1-yl)-2-methyl-3-pyridyl]piperazin-1-yl]methyl]-6-methyl-morpholin-4-yl]-2-deuterio-quinoline-8-carbonitrile;
5-[(*2S*,*6R*)-2-[[3-[6-(3-amino-3-methyl-azetidin-1-yl)-3-pyridyl]azetidin-1-yl]methyl]-6-methyl-morpholin-4-yl]-2-deuterio-quinoline-8-carbonitrile;
5-[(*2S*,*6R*)-2-[[4-[5-(3-amino-3-methyl-azetidin-1-yl)-3-methyl-2-pyridyl]piperazin-1-yl]methyl]-6-methyl-morpholin-4-yl]-2-deuterio-quinoline-8-carbonitrile;
5-[(*2S*,*6R*)-2-[[3-[4-[(*3S*,*4S*)-3-amino-4-methoxy-pyrrolidin-1-yl]phenyl]azetidin-1-yl]methyl]-6-methyl-morpholin-4-yl]-2-deuterio-quinoline-8-carbonitrile;
5-[(*2S*,*6R*)-2-[[3-[4-(3-amino-3-methyl-azetidin-1-yl)phenyl]azetidin-1-yl]methyl]-6-methyl-morpholin-4-yl]-2-deuterio-quinoline-8-carbonitrile;
5-[(*2S*,*6R*)-2-[[4-[5-[(*3R*,*4R*)-3-amino-4-methoxy-pyrrolidin-1-yl]-3-methyl-2-pyridyl]piperazin-1-yl]methyl]-6-methyl-morpholin-4-yl]-2-deuterio-quinoline-8-carbonitrile;
5-[(*2S*,*6R*)-2-[[4-[6-[(*3R*,*4R*)-3-amino-4-methoxy-pyrrolidin-1-yl]-3-pyridyl]piperazin-1-yl]methyl]-6-methyl-morpholin-4-yl]-2-deuterio-quinoline-8-carbonitrile;
5-[(*2S*,*6R*)-2-[[4-[4-[(*3R*,*4R*)-3-amino-4-methoxy-pyrrolidin-1-yl]-6-methyl-pyrimidin-2-yl]piperazin-1-yl]methyl]-6-methyl-morpholin-4-yl]-2-deuterio-quinoline-8-carbonitrile;
5-[(2S, 6R)-2-[[3-[6-(3-amino-3-methyl-azetidin-1-yl)-4-methyl-3-pyridyl] azetidin-1-yl]methyl]-6-methyl-morpholin-4-yl]-2-deuterio-quinoline-8-carbonitrile;
5-[(*2S*,*6R*)-2-[[3-[6-[(*3R*,*4R*)-3-amino-4-methoxy-pyrrolidin-1-yl]-4-methyl-3-pyridyl]azetidin-1-yl]methyl]-6-methyl-morpholin-4-yl]-2-deuterio-quinoline-8-carbonitrile;
5-[(*2S*,*6R*)-2-[[4-[5-(3-amino-3-methyl-azetidin-1-yl)-6-methyl-2-pyridyl]piperazin-1-yl]methyl]-6-methyl-morpholin-4-yl]-2-deuterio-quinoline-8-carbonitrile;
4-[(*2S*,*6R*)-2-[[4-[5-(3-amino-3-methyl-azetidin-1-yl)-3-methyl-2-pyridyl]piperazin-1-yl]methyl]-6-methyl-morpholin-4-yl]-3-fluoro-pyrazolo[1,5-a]pyridine-7-carbonitrile;
5-[(*2S*,*6R*)-2-[[3-[6-[(*3R*,*4R*)-3-amino-4-methoxy-pyrrolidin-1-yl]-3-pyridyl]azetidin-1-yl]methyl]-6-methyl-morpholin-4-yl]-2-deuterio-quinoline-8-carbonitrile;
5-[(*2S*,*6R*)-2-[[4-[6-[(*3S*,*4S*)-3-amino-4-fluoro-pyrrolidin-1-yl]-3-pyridyl]piperazin-1-yl]methyl]-6-methyl-morpholin-4-yl]-2-deuterio-quinoline-8-carbonitrile;
2-deuterio-5-[(*2S*,*6R*)-2-[[4-[6-[(1S,4S)-2,5-diazabicyclo[2.2.1]heptan-2-yl]-3-pyridyl]piperazin-1-yl]methyl]-6-methyl-morpholin-4-yl]quinoline-8-carbonitrile;
2-deuterio-5-[(*2R*,*6S*)-2-methyl-6-[[4-[6-(5-oxa-2,8-diazaspiro[3.5]nonan-2-yl)-3-pyridyl]piperazin-1-yl]methyl]morpholin-4-yl]quinoline-8-carbonitrile;
2-deuterio-5-[(*2S*,*6R*)-2-[[4-[6-[(1R,4R)-2,5-diazabicyclo[2.2.1]heptan-2-yl]-3-pyridyl]piperazin-1-yl]methyl]-6-methyl-morpholin-4-yl]quinoline-8-carbonitrile;
4-[(*2S*,*6R*)-2-[[4-[6-(3-amino-3-methyl-azetidin-1-yl)-3-pyridyl]piperazin-1-yl]methyl]-6-methyl-morpholin-4-yl]-3-fluoro-pyrazolo[1,5-a]pyridine-7-carbonitrile;
5-[(*2S*,*6*R)-2-[[4-[6-(3-amino-3-methyl-azetidin-1-yl)-2-pyridyl]piperazin-1-yl]methyl]-6-methyl-morpholin-4-yl]-2-deuterio-quinoline-8-carbonitrile;
5-[(*2S*,*6R*)-2-[[3-[4-(3-amino-3-methyl-azetidin-1-yl)phenyl]-3-hydroxy-azetidin-1-yl] methyl]-6-methyl-morphofin-4-yl]-2-deuterio-quinofine-8-carbonitrile;
4-[(*2S*,*6R*)-2-[[3-[6-(3-amino-3-methyl-azetidin-1-yl)-3-pyridyl]azetidin-1-yl]methyl]-6-methyl-morpholin-4-yl]pyrazolo[1,5-a]pyridine-7-carbonitrile;
4-[(*2S*,*6R*)-2-[[3-[6-(3-amino-3-methyl-azetidin-1-yl)-3-pyridyl]azetidin-1-yl]methyl]-6-methyl-morpholin-4-yl]-3-fluoro-pyrazolo[1,5-a]pyridine-7-carbonitrile;
5-[(*2S*,*6R*)-2-[[3-[6-(3-amino-3-methyl-azetidin-1-yl)-2-pyridyl]azetidin-1-yl]methyl]-6-methyl-morpholin-4-yl]-2-deuterio-quinoline-8-carbonitrile;
4-[(*2S*,*6R*)-2-[[3-[4-(3-amino-3-methyl-azetidin-1-yl)phenyl]azetidin-1-yl]methyl]-6-methyl-morpholin-4-yl]-3-fluoro-pyrazolo[1,5-a]pyridine-7-carbonitrile;
2-deuterio-5-[(*2R*,*6S*)-2-methyl-6-[[4-[6-(6-methyl-2,6-diazaspiro[3.3]heptan-2-yl)-3-pyridyl]piperazin-1-yl]methyl]morpholin-4-yl]quinoline-8-carbonitrile;
2-deuterio-5-[(*2S*,*6R*)-2-[[4-[6-[[(*3S*,*4R*)-4-fluoropyrrolidin-3-yl]amino]-3-pyridyl]piperazin-1-yl]methyl]-6-methyl-morpholin-4-yl]quinoline-8-carbonitrile;
4-[(*2S*,*6R*)-2-[[3-[4-(3-amino-3-methyl-azetidin-1-yl)phenyl]azetidin-1-yl]methyl]-6-methyl-morpholin-4-yl]pyrazolo[1,5-a]pyridine-7-carbonitrile;
4-[(*2S*,*6R*)-2-[[3-[6-[(*3R*,*4R*)-3-amino-4-methoxy-pyrrolidin-1-yl]-3-pyridyl]azetidin-1-yl]methyl]-6-methyl-morpholin-4-yl]-3-fluoro-pyrazolo[1,5-a]pyridine-7-carbonitrile;
4-[(*2S*,*6R*)-2-[[3-[6-[(1S,4S)-2,5-diazabicyclo[2.2.1]heptan-2-yl]-3-pyridyl]azetidin-1-yl]methyl]-6-methyl-morpholin-4-yl]-3-fluoro-pyrazolo[1,5-a]pyridine-7-carbonitrile;
4-[(*2S*,*6R*)-2-[[4-[6-[(*3R*,*4R*)-3-amino-4-methoxy-pyrrolidin-1-yl]-3-pyridyl]piperazin-1-yl]methyl]-6-methyl-morpholin-4-yl]-3-fluoro-pyrazolo[1,5-a]pyridine-7-carbonitrile;
3-fluoro-4-[(*2S*,*6R*)-2-[[4-[6-[[(*3S*,*4R*)-4-fluoropyrrolidin-3-yl]amino]-3-pyridyl]piperazin-1-yl]methyl]-6-methyl-morpholin-4-yl]pyrazolo[1,5-a]pyridine-7-carbonitrile;
4-[(*2S*,*6R*)-2-[[4-[6-[(4*aR*,7*aR*)-3,4,4*a*,5,7,7*a*-hexahydro-2*H*-pyrrolo[3,4-b][1,4]oxazin-6-yl]-3-pyridyl]piperazin-1-yl]methyl]-6-methyl-morpholin-4-yl]-3-fluoro-pyrazolo[1,5-a]pyridine-7-carbonitrile;
3-fluoro-4-[(*2R*,*6S*)-2-methyl-6-[[4-[6-(5-oxa-2,8-diazaspiro[3.5]nonan-2-yl)-3-pyridyl]piperazin-1-yl]methyl]morpholin-4-yl]pyrazolo[1,5-a]pyridine-7-carbonitrile;
3-fluoro-4-[(*2R*,*6S*)-2-methyl-6-[[4-[6-(6-methyl-2,6-diazaspiro[3.3]heptan-2-yl)-3-pyridyl]piperazin-1-yl]methyl]morpholin-4-yl]pyrazolo[1,5-a]pyridine-7-carbonitrile;
1-methyl-4-[(*2R*,*6S*)-2-methyl-6-[[3-(4-piperazin-1-ylphenyl)azetidin-1-yl]methyl]morpholin-4-yl]-1,8-naphthyridin-2-one;
4-[(*2S*,*6R*)-2-[[3-[4-[(*3R*,*4R*)-3-amino-4-methoxy-pyrrolidin-1-yl]phenyl]azetidin-1-yl]methyl]-6-methyl-morpholin-4-yl]-1-methyl-1,8-naphthyridin-2-one;
1-methyl-4-[(*2R*,*6S*)-2-methyl-6-[[3-[4-(5-oxa-2,8-diazaspiro[3.5]nonan-2-yl)phenyl]azetidin-1-yl]methyl]morpholin-4-yl]-1,8-naphthyridin-2-one;
4-[(*2S*,*6R*)-2-[[3-[4-[(*3R*,*4R*)-3-amino-4-fluoro-pyrrolidin-1-yl]phenyl]azetidin-1-yl]methyl]-6-methyl-morpholin-4-yl]-1-methyl-1,8-naphthyridin-2-one;
4-[(*2S*,*6R*)-2-[[4-[6-[(*3R*,*4R*)-4-amino-3-fluoro-1-piperidyl]-3-pyridyl]piperazin-1-yl]methyl]-6-methyl-morpholin-4-yl]-3-fluoro-pyrazolo[1,5-a]pyridine-7-carbonitrile;
4-[(*2S*,*6R*)-2-[[3-[4-(3-amino-3-methyl-azetidin-1-yl)phenyl]azetidin-1-yl]methyl]-6-methyl-morpholin-4-yl]-1-methyl-1,8-naphthyridin-2-one;
3-fluoro-4-[(*2R*,*6S*)-2-methyl-6-[[4-[4-[(*2R*)-morphofin-2-yl]phenyl]piperazin-1-yl] methyl] morpholin-4-yl]pyrazolo[1,5-a]pyridine-7-carbonitrile;
3-fluoro-4-[(*2R*,*6S*)-2-methyl-6-[[4-[6-(5-oxa-2,8-diazaspiro[3.5]nonan-8-yl)-3-pyridyl]piperazin-1-yl]methyl]morpholin-4-yl]pyrazolo[1,5-a]pyridine-7-carbonitrile;
4-[(*2S*,*6R*)-2-[[4-[6-(1,6-diazaspiro[3.3]heptan-6-yl)-3-pyridyl]piperazin-1-yl]methyl]-6-methyl-morpholin-4-yl]-3-fluoro-pyrazolo[1,5-a]pyridine-7-carbonitrile;
1-methyl-4-[(*2R*,*6S*)-2-methyl-6-[[3-[4-(6-methyl-2,6-diazaspiro[3.3]heptan-2-yl)phenyl]azetidin-1-yl]methyl]morpholin-4-yl]-1,8-naphthyridin-2-one;
4-[(2*S*,6*R*)-2-[[3-[4-[(4*aR*,7*aR*)-3,4,4*a*,5,7,7*a*-hexahydro-*2H*-pyrrolo[3,4-*b*][1,4]oxazin-6-yl]phenyl]azetidin-1-yl]methyl] -6-methyl-morpholin-4-yl]- 1-methyl-1,8-naphthyridin-2-one;
3-fluoro-4-[(2*S*,*6R*)-2-[[4-[2-fluoro-4-[(2*S*)-morphofin-2-yl]phenyl]piperazin-1-yl]methyl]-6-methyl-morpholin-4-yl]pyrazolo[1,5-a]pyridine-7-carbonitrile;
3-fluoro-4-[(2*S*,6*R*)-2-[[4-[3-fluoro-4-[(2*R*)-morpholin-2-yllphenyl]piperazin-1-yl]methyl]-6-methyl-morpholin-4-yl]pyrazolo[1,5-a]pyridine-7-carbonitrile;
3-fluoro-4-[(*2R*,*6S*)-2-methyl-6-[[4-[4-(2-methylmorpholin-2-yl)phenyl]piperazin-1-yl]methyl]morpholin-4-yl]pyrazolo[1,5-a]pyridine-7-carbonitrile; and
3-fluoro-4-[(*2R*,*6S*)-2-methyl-6-[[4-[4-[[(*2R*)-morphofin-2-yl]methyl]phenyl]piperazin-1-yl]methyl]morpholin-4-yl]pyrazolo[1,5-a]pyridine-7-carbonitrile;
or a pharmaceutically acceptable salt thereof.

In another embodiment, one or more atoms of formulas or compounds given herein are replaced by its isotope. Examples of isotopes that can be incorporated into compounds of the invention include isotopes of hydrogen, carbon, nitrogen, oxygen, phosphorous, fluorine, chlorine, and iodine, such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{36}Cl$, and $^{125}I$, respectively.

A number of compounds used as reference herein were disclosed in patent US20150105370 showing TLR7 and TLR9 potency data summarized in table 1 (TLR8 data is not available). Compounds in Table 1 are all characterized with an aromatic ring at the terminal position (phenyl or pyridinyl). However, according to the potency data disclosed, only some of the compounds in Table 1 showed good TLR7 potency, and all of which were lack of TLR9 potency. More examples disclosed in US20150105370 with same structural characteristics confirmed such trend, which suggests the terminal aryl/heteroaryl ring is not favorable for TLR9 activity.

Meanwhile, more analogues of the compounds disclosed in US20150105370, such as compound R1, compound R2 which bear some substituents on the terminal aryl ring, were synthesized to confirm the SAR (structure-activity-relationship). But the potency of compound R1 and R2 shown in Table 2 suggested that the substituents on the terminal aryl ring may not necessarily improve the potency of TLR9. Therefore, the skill of the art shall not obtain any incitation from the information disclosed in US20150105370 to further optimize such chemical structures.

Surprisingly, the compounds of this invention significantly improved TLR9 potency (>8 folds compared to ER-888286) while keeping excellent TLR7 and TLR8 potency. In another embodiment, hERG profile and safety ratio were greatly improved as compared with reference compounds from US20150105370 and reference compounds R1 and R2 synthesized herein (see table 3). The compounds of formula (I) or (Ia) also showed good hPBMC, cytotoxicity, solubility, human microsome stability and SDPK profiles, as well as low CYP inhibition.

**Table 1. TLR7 and TLR9 potency of compounds disclosed in US20150105370**

| Compound | Structure | HEK/hTLR7 IC50 (μM) | HEK/hTLR9 IC50 (μM) |
|---|---|---|---|
| ER-887258 | 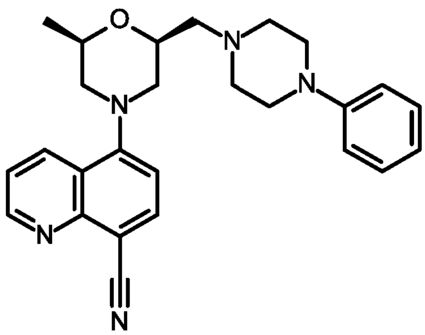 | 0.0852 | >2.0 |
| ER-888285 | 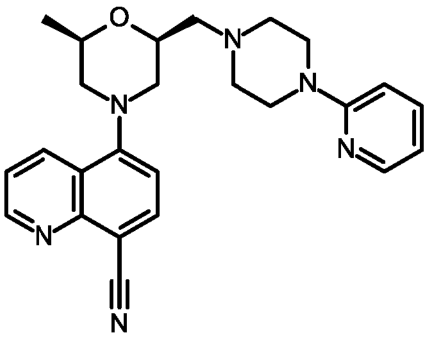 | 0.120 | >2.0 |

(continued)

| Compound | Structure | HEK/hTLR7 IC50 (μM) | HEK/hTLR9 IC50 (μM) |
|---|---|---|---|
| ER-888286 | 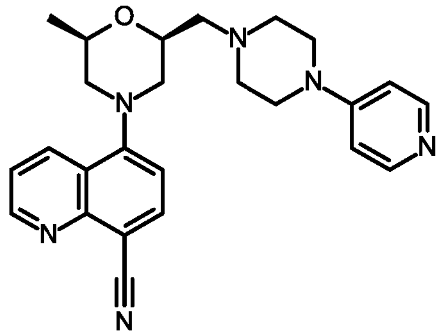 | 1.370 | >2.0 |
| ER-894544 | 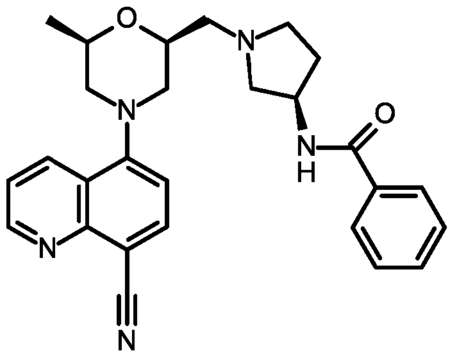 | 0.043 | >6.2 |
| ER-894160 | 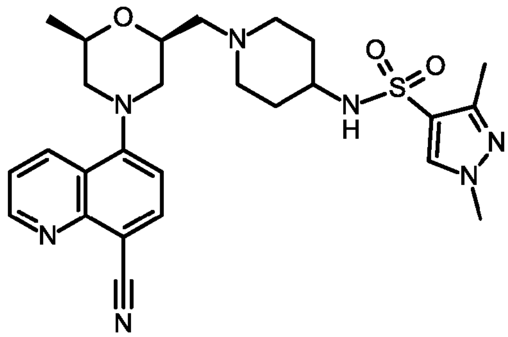 | 0.1990 | >10.0 |
| ER-894155 | 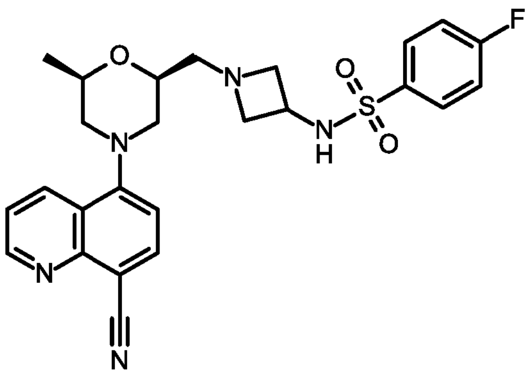 | 0.2820 | >10.0 |

**SYNTHESIS**

The compounds of the present invention can be prepared by any conventional means. Suitable processes for synthesizing these compounds as well as their starting materials are provided in the schemes below and in the examples. All substituents, in particular, $R^1$ to $R^3$, A and Q are as defined above unless otherwise indicated. Furthermore, and

unless explicitly otherwise stated, all reactions, reaction conditions, abbreviations and symbols have the meanings well known to a person of ordinary skill in organic chemistry.

General synthetic routes for preparing the compound of formula (I) or (Ia) are shown below.

## Scheme 1

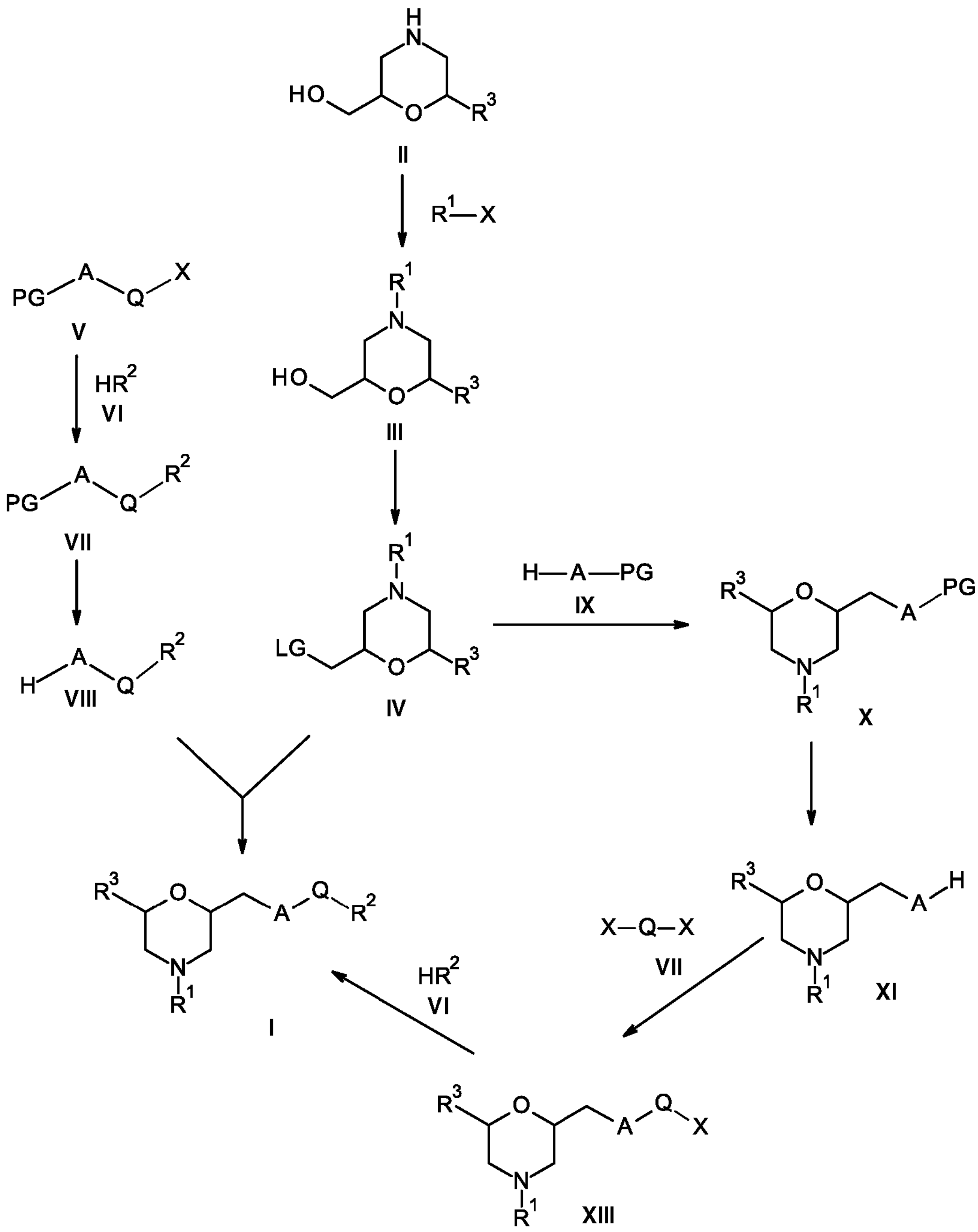


wherein X is halogen; LG is a leaving group, such as OTf, OTs and OMs; PG is a protecting group, such as Boc and Cbz.

The coupling of compound of formula (II) with $R^1$-X can be achieved in the presence of a base, such as DIPEA or $K_2CO_3$, or under Buchwald-Hartwig amination conditions (ref: Acc. Chem. Res. 1998, 31, 805-818; Chem. Rev. 2016, 116, 12564-12649; Topics in Current Chemistry, 2002, 219, 131-209; and references cited therein) with a catalyst, such as RuPhos Pd G2, and a base, such as $Cs_2CO_3$, to provide compound of formula (III). Subsequently the hydroxy group of compound of formula (III) is converted to a leaving group, such as OTf, OTs, and OMs, under basic condition, such as DIPEA, TEA, $K_2CO_3$ and 2,6-dimethylpyridine, with $Tf_2O$, TsCl or MsCl. The coupling of compound of formula (V) with (VI) can be achieved in the presence of a base, such as DIPEA and $K_2CO_3$, or under Buchwald-Hartwig amination

conditions (ref: Acc. Chem. Res. 1998, 31, 805-818; Chem. Rev. 2016,116, 12564-12649; Topics in Current Chemistry, 2002, 219, 131-209; and references cited therein) with a catalyst, such as tBuXPhos Pd G3, RuPhos Pd G2, BrettPhos Pd G3, XPhos Pd G3, $Pd_2(dba)_3$/BINAP and $Pd_2(dba)_3$/XantPhos and a base, such as $Cs_2CO_3$ or t-BuONa, to provide compound of formula (VII). The protecting group of compound of formula (VII) can be removed at high temperature or under acidic condition, such as TFA, or under hydrogenation condition with a catalyst, such as Pd/C and $Pd(OH)_2$/C. Compound of formula (VIII) is further substituted by compound of formula (IV) in the presence of a base, such as $K_2CO_3$, DIPEA and $Cs_2CO_3$, to afford compound of formula (I). On the other hand, compound of formula (IV) can reacted with (IX) in the presence of a base, such as $K_2CO_3$, DIPEA, and $Cs_2CO_3$, to give compound of formula (X). The protecting group of compound of formula (X) can be removed at high temperature or under acidic condition, such as TFA, or under hydrogenation condition with a catalyst, such as Pd/C or $Pd(OH)_2$/C to give compound of formula (XI), which can be further coupled with compound of formula (XII) in the presence of a base, such as DIPEA or $K_2CO_3$, or under Buchwald-Hartwig amination conditions (ref: Acc. Chem. Res. 1998, 31, 805-818; Chem. Rev. 2016, 116, 12564-12649; Topics in Current Chemistry, 2002, 219, 131-209; and references cited therein) with a catalyst, such as tBuXPhos Pd G3 and Ruphos Pd G2, and a base, such as $Cs_2CO_3$, to provide compound of formula (XIII). The coupling of compound of formula (VI) with (XIII) can be achieved in the presence of a base, such as DIPEA and $K_2CO_3$, or under Buchwald-Hartwig amination conditions (ref: Acc. Chem. Res. 1998, 31, 805-818; Chem. Rev. 2016, 116, 12564-12649; Topics in Current Chemistry, 2002, 219, 131-209; and references cited therein) with a catalyst, such as tBuXPhos Pd G3, RuPhos Pd G2, BrettPhos Pd G3, XPhos Pd G3, $Pd_2(dba)_3$/BINAP and $Pd_2(dba)_3$/XantPhos, and a base, such as $Cs_2CO_3$ and t-BuONa, to provide compound of formula (I). In some embodiment, the coupling of compound of formula (VIII) and (IV), or formula (XIII) and (VI) may give a product containing a protecting group, e.g. Boc or Cbz, originated from (VIII) or (VI), which will be removed before affording the final compound of formula (I).

Compounds of this invention can be obtained as mixtures of diastereomers or enantiomers, which can be separated by methods well known in the art, e.g. (chiral) HPLC or SFC. In another embodiment, compound of formula (Ia) can be obtained according to above scheme by using corresponding chiral starting materials.

This invention also relates to a process for the preparation of a compound of formula (I) or (Ia) comprising any of the following steps:

a) the substitution of compound of formula (IV),

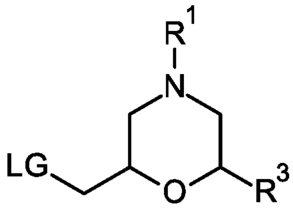

(IV),

with compound of formula (VIII),

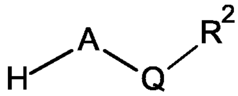

(VIII),

in the presence of a base;

b) the coupling of compound of formula (XIII),

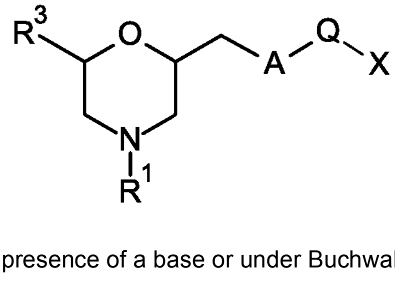

(XIII),

with compound of formula (VI) in the presence of a base or under Buchwald-Hartwig amination condition;

wherein

in step a) and b), the base can be, for example, $K_2CO_3$, DIPEA or $Cs_2CO_3$;
in step b), the Buchwald-Hartwig amination condition includes a catalyst and a base, wherein the catalyst can be, for example, such as tBuXPhos Pd G3, RuPhos Pd G2, BrettPhos Pd G3, XPhos Pd G3, $Pd_2(dba)_3$/BINAP and $Pd_2(dba)_3$/XantPhos; the base can be, for example, $Cs_2CO_3$ and t-BuONa;
A compound of formula (I) or (Ia) when manufactured according to the above process is also an object of the invention.

**INDICATIONS AND METHODS OF TREATMENT**

The present invention provides compounds that can be used as TLR7 and/or TLR8 and/or TLR9 antagonist, which inhibits pathway activation through TLR7 and/or TLR8 and/or TLR9 as well as respective downstream biological events including, but not limited to, innate and adaptive immune responses mediated through the production of all types of cytokines and all forms of auto-antibodies. Accordingly, the compounds of the invention are useful for blocking TLR7 and/or TLR8 and/or TLR9 in all types of cells that express such receptor(s) including, but not limited to, plasmacytoid dendritic cell, B cell, T cell, macrophage, monocyte, neutrophil, keratinocyte, epithelial cell. As such, the compounds can be used as a therapeutic or prophylactic agent for systemic lupus erythematosus and lupus nephritis.
The present invention describes methods for treatment or prophylaxis of systemic lupus erythematosus and lupus nephritis in a patient in need thereof.
Another embodiment includes a compound of formula (I) or (Ia), a stereoisomer, tautomer or pharmaceutically acceptable salt thereof for use in a method of treating or preventing systemic lupus erythematosus and lupus nephritis in a mammal in need of such treatment.

**EXAMPLES**

The invention will be more fully understood by reference to the following examples. They should not, however, be construed as limiting the scope of the invention.

**ABBREVIATIONS**

The invention will be more fully understood by reference to the following examples. They should not, however, be construed as limiting the scope of the invention.
Abbreviations used herein are as follows:

| | |
|---|---|
| ACN: | acetonitrile |
| BINAP: | (2,2'-bis(diphenylphosphino)-1,1'-binaphthyl) |
| BrettPhos Pd G3: | [(2-di-cyclohexylphosphino-3,6-dimethoxy-2',4',6'- triisopropyl-1,1'-biphenyl)-2-(2'-amino-1,1'-biphenyl)]palladium(II) methanesulfonate |
| *t*-BuXPhosPd G3: | [(2-di-*tert*-butylphosphino-2',4',6'-triisopropyl-1,1'-biphenyl)-2-(2'-amino-1,1'-biphenyl)] palladium(II) methanesulfonate |
| DCM: | dichloromethane |
| DIPEA: | *N*,*N*-diisopropylethylamine |
| EtOAc or EA: | ethyl acetate |
| HLM | human liver microsome |
| $IC_{50}$: | half inhibition concentration |
| IPA: | isopropanol |
| LCMS | liquid chromatography-mass spectrometry |
| MS: | mass spectrometry |
| NMP: | *N*-methylpyrrolidin-2-one |
| $Boc_2O$: | di-*tert* butyl dicarbonate |
| $Pd_2(dba)_3$: | tris(dibenzylideneacetone)dipalladium(0) |
| PE: | petroleum ether |
| prep-HPLC: | preparative high performance liquid chromatography |
| prep-TLC: | preparative thin layer chromatography |
| Rf: | retention factor |
| rt: | room temperature |
| RuPhos Pd G2: | chloro(2-dicyclohexylphosphino-2',6'-diisopropoxy-1,1'-biphenyl) [2-(2'-amino-1,1'-biphenyl)]palladium(II)2nd generation |

SFC: supercritical fluid chromatography
TEA: trimethylamine
TFA: trifluoroacetic acid
$Tf_2O$: trifluoromethanesulfonic anhydride
XantPhos: 4,5-bis(diphenylphosphino)-9,9-dimethylxanthene
XPhos: 2-dicyclohexylphosphino-2',4',6'-triisopropylbiphenyl

## GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 μm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge™ Prep-C18 (5 μm, OBDTM 30 × 100 mm) column, SunFire™ Prep-C18 (5 μm, OBD™ 30 × 100 mm) column, Phenomenex Synergi-C18 (10 μm, 25 × 150 mm) or Phenomenex Gemini-C18 (10 μm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 μm, 30 × 250 mm), AS (10 μm, 30 × 250 mm) or AD (10 μm, 30 × 250 mm) using Mettler Toledo Multigram III system SFC, Waters 80Q preparative SFC or Thar 80 preparative SFC, solvent system: $CO_2$ and IPA (0.5% TEA in IPA) or $CO_2$ and MeOH (0.1% $NH_3 \cdot H_2O$ in MeOH), back pressure 100bar, detection UV@ 254 or 220 nm.

LC/MS spectra of compounds were obtained using a LC/MS (Waters™ Alliance 2795-Micromass ZQ, Shimadzu Alliance 2020-Micromass ZQ or Agilent Alliance 6110-Micromass ZQ), LC/MS conditions were as follows (running time 3 or 1.5 mins):

Acidic condition I: A: 0.1% TFA in $H_2O$; B: 0.1% TFA in acetonitrile;
Acidic condition II: A: 0.0375% TFA in $H_2O$; B: 0.01875% TFA in acetonitrile;
Basic condition I: A: 0.1% $NH_3 \cdot H_2O$ in $H_2O$; B: acetonitrile;
Basic condition II: A: 0.025% $NH_3 \cdot H_2O$ in $H_2O$; B: acetonitrile;
Neutral condition: A: $H_2O$; B: acetonitrile.

Mass spectra (MS): generally only ions which indicate the parent mass are reported, and unless otherwise stated the mass ion quoted is the positive mass ion $(MH)^+$.

NMR Spectra were obtained using Bruker Avance 400 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

## PREPARATIVE EXAMPLES

The following examples are intended to illustrate the meaning of the present invention but should by no means represent a limitation within the meaning of the present invention:

### Intermediate A

**[(2*R*,6*R*)-4-(8-cyano-5-quinolyl)-6-methyl-morpholin-2-yl]methyl trifluoromethanesulfonate**

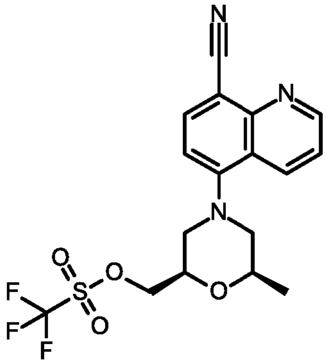

The title compound was prepared according to the following scheme:

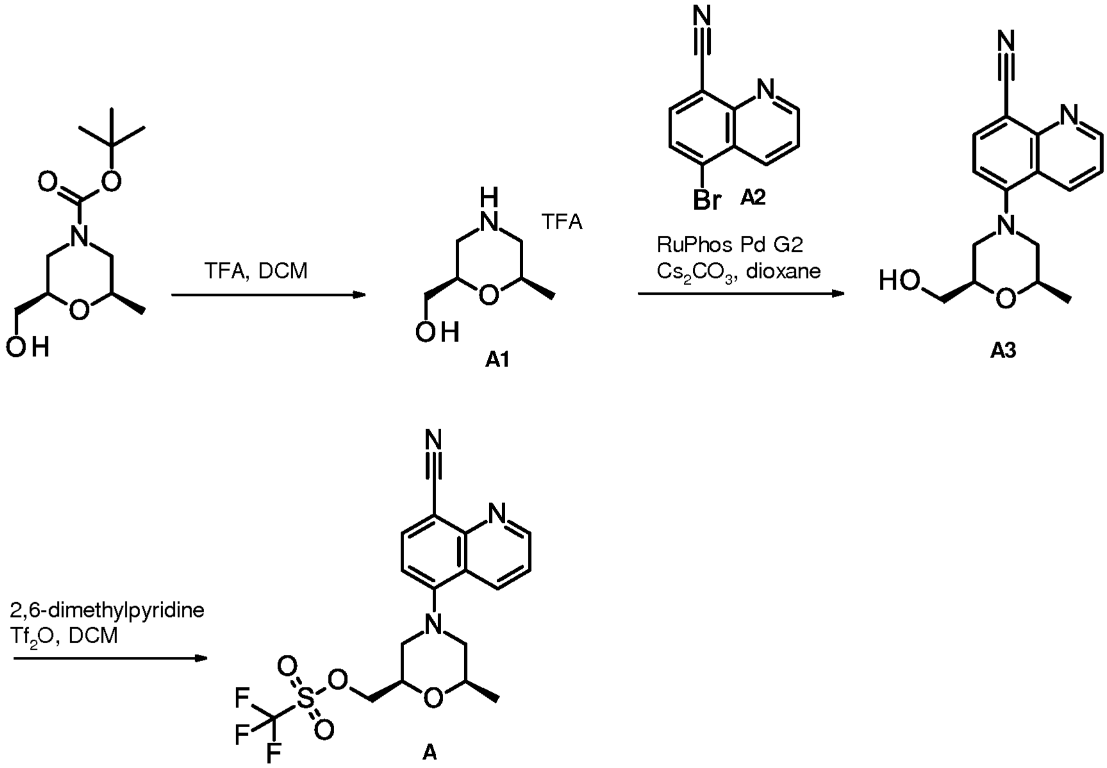


**Step 1: preparation of [(2R,6R)-6-methylmorpholin-2-yl]methanol;2,2,2-trifluoroacetic acid (compound A1)**

To a solution of *tert*-butyl (2*R*,6*R*)-2-(hydroxymethyl)-6-methylmorpholine-4-carboxylate (CAS: 1700609-48-8, Vendor: WuXi Apptec, 1.35 g, 5.84 mmol) in DCM (10 mL) was added TFA (2.66 g, 23.30 mmol). After being stirred at rt for 3 hrs, the reaction mixture was concentrated *in vacuo* to give the crude product compound **A1** (1.43 g) which was used in next step directly. MS: calc'd 132 ($MH^+$), measured 132 ($MH^+$).

**Step 2: preparation of 5-[(2R,6R)-2-(hydroxymethyl)-6-methyl-morpholin-4-yl]quinoline-8-carbonitrile (compound A3)**

The mixture of 5-bromoquinoline-8-carbonitrile (compound **A2,** CAS: 507476-70-2, Vendor: BePharm, 1.50 g, 6.42 mmol), [(2*R*,6*R*)-6-methylmorpholin-2-yl]methanol;2,2,2-trifluoroacetic acid (compound **A1**, 1.43 g, 5.83 mmol), RuPhos Pd G2 (136 mg, 175 μmol) and $Cs_2CO_3$ (5.70 g, 17.50 mmol) in 1,4-dioxane (10 mL) was charged with $N_2$, and heated to 90 °C overnight. After being cooled down, the solid was filtered off and washed with EA (10 mL) twice. The filtrate was concentrated and the residue was purified by silica gel chromatography (EA/PE = 0 to 100%) to afford compound **A3** (709 mg) as a light yellow solid. MS: calc'd 284 ($MH^+$), measured 284 ($MH^+$).

**Step 3: preparation of [(2R,6R)-4-(8-cyano-5-quinolyl)-6-methyl-morpholin-2-yl]methyl trifluoromethanesulfonate (Intermediate A)**

To a flask was added 5-[(2*R*,6*R*)-2-(hydroxymethyl)-6-methyl-morphofin-4-yl]quinoline-8-carbonitrile (compound **A3**, 709 mg, 2.50 mmol), DCM (10 mL) and 2,6-dimethylpyridine (536 mg, 577 μL, 5.00 mmol). Then the reaction mixture was cooled with ice bath and trifluoromethanesulfonic anhydride (1.06 g, 634 μL, 3.75 mmol) was added dropwise. After being stirred for 2 hrs, the mixture was concentrated and purified by silica gel chromatography (EA/PE = 0 to 40%) to give **Intermediate A** (720 mg) as a yellow solid. MS: calc'd 416 ($MH^+$), measured 416 ($MH^+$).

**Intermediate C**

**[(2R, 6R)-4-(8-cyanoquinoxalin-5-yl)-6-methyl-morpholin-2-yl]methyl trifluoromethanesulfonate**

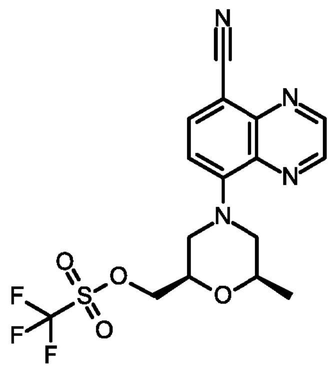

The title compound was prepared in analogy to the preparation of **Intermediate A** by using 8-bromoquinoxaline-5-carbonitrile (Synthesis refers to US 20170174653 A1) instead of 5-bromoquinoline-8-carbonitrile (compound **A2**). **Intermediate** C (825 mg) was obtained as an off-white solid. MS: calc'd 417 ($MH^+$), measured 417 ($MH^+$).

**Intermediate D**

**[(*2R*,*6R*)-4-(7-cyanopyrazolo[1,5-a]pyridin-4-yl)-6-methyl-morpholin-2-yl]methyl trifluoromethanesulfonate**

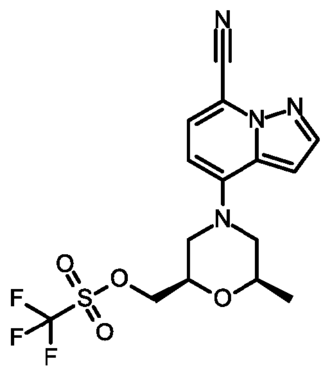

The title compound was prepared in analogy to the preparation of **Intermediate A** by using 4-chloropyrazolo[1,5-a]pyridine-7-carbonitrile (CAS: 1268520-74-6, Vendor: PharmaBlock) instead of 5-bromoquinoline-8-carbonitrile (compound **A2**). **Intermediate D** (166 mg) was obtained as a white solid. MS: calc'd 405 ($MH^+$), measured 405 ($MH^+$).

**Intermediate E**

**[(*2R*,*6R*)-4-(7-cyano-3-fluoro-pyrazolo[1,5-a]pyridin-4-yl)-6-methyl-morpholin-2-yl]methyl trifluoromethanesulfonate**

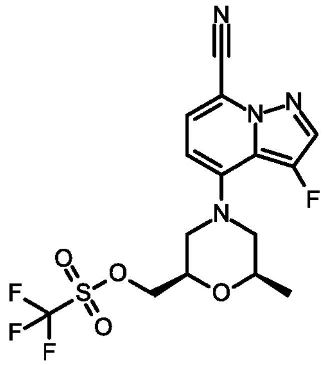

The title compound was prepared according to the following scheme:

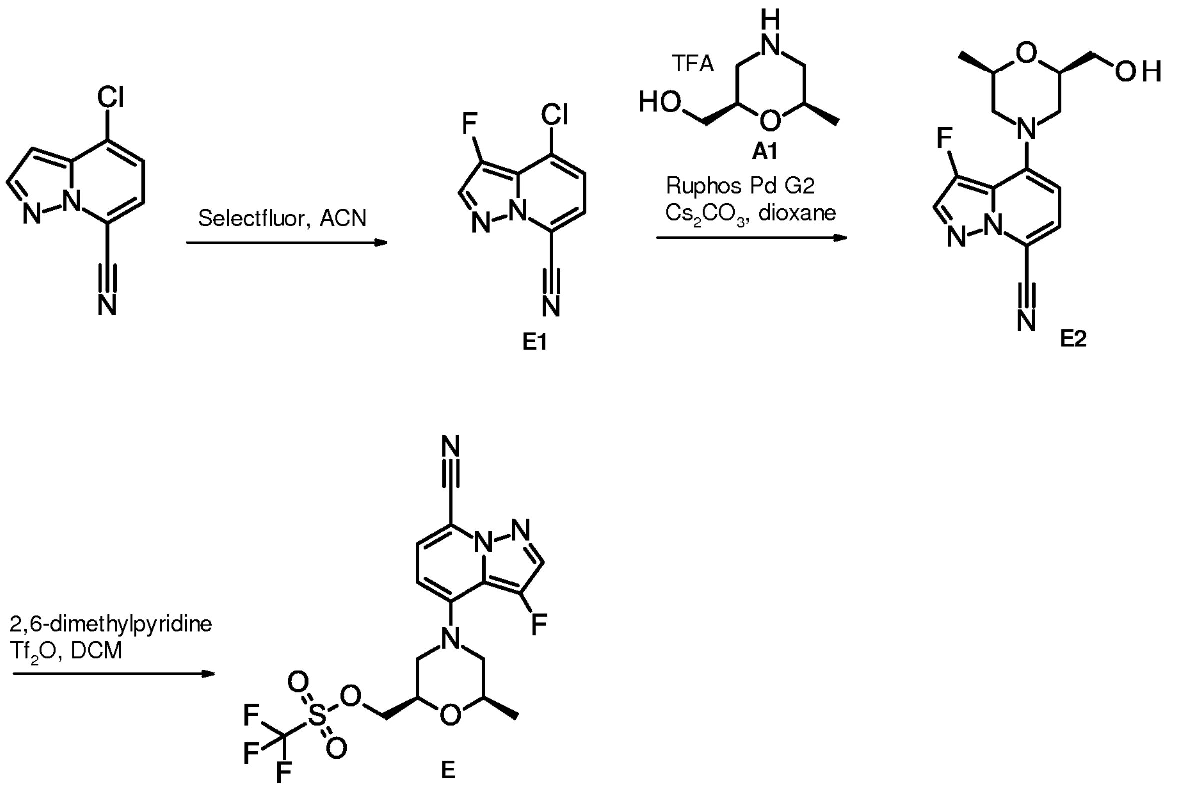

A1
E1
E2
E

**Step 1: preparation of 4-chloro-3-fluoro-pyrazolo[1,5-a]pyridine-7-carbonitrile (compound E1)**

To a solution of 4-chloropyrazolo[1,5-a]pyridine-7-carbonitrile (CAS: 1268520-74-6, Vendor: Pharmablock, 600 mg, 3.38 mmol) in acetonitrile (50 mL) was added SelectFluor (2.39 g, 6.76 mmol). After being stirred at rt for 24 hrs, the mixture was concentrated and diluted with water (30 mL), extracted with DCM (30 mL) twice. The organic layer was washed with sat. $NH_4Cl$ and brine, dried over $Na_2SO_4$, and concentrated to give a crude product which was purified by silica gel chromatography to give compound **E1** (419 mg) as a light yellow powder. MS: calc'd 196 ($MH^+$), measured 196 ($MH^+$). $^1H$ NMR (400MHz, METHANOL-d4) $\delta$ = 8.17 (d, *J*=3.5 Hz, 1H), 7.55 (d, *J*=7.6 Hz, 1H), 7.35 (d, *J*=7.7 Hz, 1H).

**Step 2: preparation of 3-fluoro-4-[(*2R,6R*)-2-(hydroxymethyl)-6-methyl-morpholin-4-yl]pyrazolo[1,5-a]pyridine-7-carbonitrile (compound E2)**

To a solution of 4-chloro-3-fluoropyrazolo[1,5-a]pyridine-7-carbonitrile (compound **E1**, 419 mg, 2.14 mmol), [(2*R*,6*R*)-6-methylmorpholin-2-yl]methanol;2,2,2-trifluoroacetic acid (compound **A1**, 526 mg, 2.14 mmol) and $Cs_2CO_3$ (2.79 g, 8.57 mmol) in 1,4-dioxane (10 mL) was added RuPhos Pd G2 (116 mg, 0.15 mmol) under $N_2$. The reaction mixture was heated at 90 °C for 2 hrs. After being cooled down, the mixture was diluted with EtOAc and filtered through celite. The filtrate was concentrated to give a brown oil which was purified by silica gel chromatography to give compound **E2** (325 mg) as a yellow oil. MS: calc'd 291 ($MH^+$), measured 291 ($MH^+$).

**Step 3: preparation of (*2R,6R*)-4-(7-cyanopyrazolo[1,5-a]pyridin-4-yl)-6-methyl-morpholin-2-yl]methyl trifluoromethanesulfonate (Intermediate E)**

To a solution of 3-fluoro-4-[(2R,6R)-2-(hydroxymethyl)-6-methyl-morpholin-4-yl]pyrazolo[1,5-a]pyridine-7-carbonitrile (compound **E2,** 325 mg, 1.12 mmol) in DCM (3 mL) was added 2,6-dimethylpyridine (240 mg, 258 μL, 2.24 mmol) and $Tf_2O$ (474 mg, 284 μL, 1.68 mmol) at rt. After being stirred for 1.5 hrs, the mixture was diluted with DCM, washed with sat. $NH_4Cl$ and brine. The organic layer was dried over $Na_2SO_4$ and concentrated to give the crude product which was purified by silica gel chromatography to give **Intermediate E** (180 mg) as a yellow solid. MS: calc'd 423 ($MH^+$), measured 423 ($MH^+$).

**Intermediate G**

**[(*2R*,*6R*)-6-methyl-4-(8-methylquinoxalin-5-yl)morpholin-2-yl]methyl trifluoromethanesulfonate**

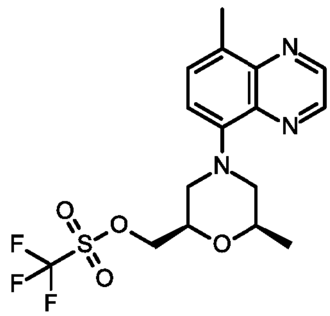

The title compound was prepared in analogy to the preparation of **Intermediate A** by using 5-bromo-8-methylquinoxaline (CAS: 1360599-43-4, Vendor: Bepharm) instead of 5-bromoquinoline-8-carbonitrile (compound **A2**), replacing RuPhos Pd G2 and $Cs_2CO_3$ with $Pd_2(dba)_3$, BINAP and t-BuONa in the Buchwald-Hartwig amination reaction. **Intermediate G** (200 mg) was obtained as a brown solid. MS: calc'd 406 ($MH^+$), measured 406 ($MH^+$).

**Intermediate K**

**[(*2R*,*6R*)-4-(8-cyano-2,3-dideuterio-quinoxalin-5-yl)-6-methyl-morpholin-2-yl]methyl trifluoromethanesulfonate**

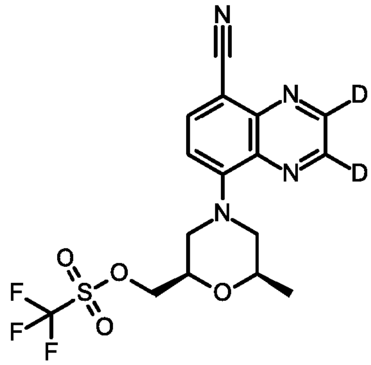

The title compound was prepared in analogy to the preparation of **Intermediate A** by using 8-bromo-2,3-dideuterio-quinoxaline-5-carbonitrile (compound **K8**) instead of 5-bromoquinoline-8-carbonitrile (compound **A2**). **Intermediate K** (200 mg) was obtained as a yellow solid. MS: calc'd 419 ($MH^+$), measured 419 ($MH^+$).

The compound **K8** was prepared according to the following scheme:

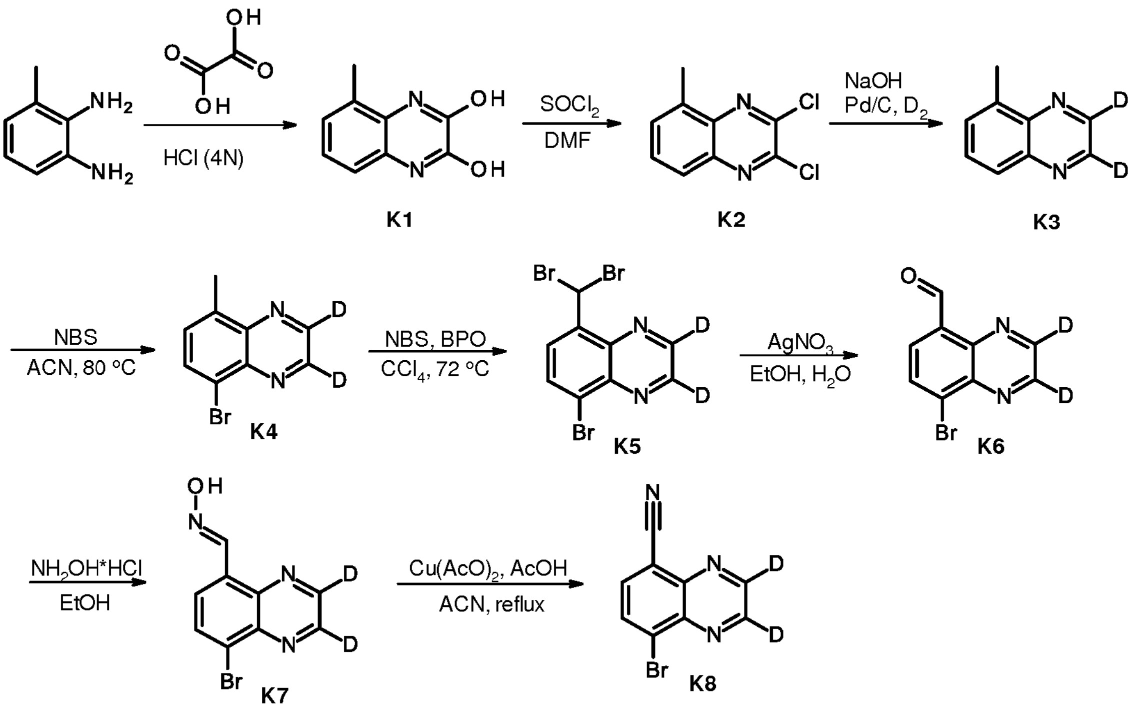

K1 K2 K3 K4 K5 K6 K7 K8

**Step 1: preparation of 5-methylquinoxaline-2,3-diol (compound K1)**

To the solution of 2,3-diaminotoluene (CAS: 2687-25-4, Vendor: Aldrich, 150 g, 1228 mmol) and hydrogenchloride (4N, 3750 mL) was added oxalic acid (221 g, 2456 mmol). The mixture was stirred at 100 °C for 3 hrs. After being cooled down, the mixture was poured into 3000 mL ice-water. A black solid was appeared. The mixture was filtered, and the wet-cake was washed with 500 mL water and dried under vacuum to give compound **K1** (230 g) as a black solid. MS calc'd 177 ($MH^+$), measured 177 ($MH^+$).

**Step 2: preparation of 2,3-dichloro-5-methyl-quinoxaline (compound K2)**

To the solution of 5-methylquinoxaline-2,3-diol (compound **K1**, 220 g, 1249 mmol) in 1,2-dichloroethane (500 mL) was added thionyl chloride (500 mL, 2498 mmol) and DMF (0.97 mL, 12.49 mmol). The final mixture was stirred at 80 °C for 6 hrs. After being cooled down, the mixture was concentrated to give a crude product which was purified by silica gel chromatography (PE/EA = 5/1) to give compound **K2** (152 g) as a yellow solid. MS calc'd 213 ($MH^+$), measured 213 ($MH^+$).

**Step 3: preparation of 2,3-dideuterio-5-methyl-quinoxaline (compound K3)**

To the solution of 2,3-dichloro-5-methyl-quinoxaline (compound **K2,** 46 g, 215.90 mmol) in 1,4-dioxane (760 mL) and deuterium oxide (160 mL) was added 2 N aqueous solution of NaOH in $D_2O$ (215.9 mL, 431.80 mmol) and Pd/C (10 wt.%, 9200 mg). The mixture was stirred at r.t. under deuterium atmosphere for 3 hrs. Then the mixture was filtered and diluted with 800 mL water, extracted with 250 mL EA for three times. The combined organic layer was washed with 300 mL water twice and 200 mL brine once. After dried over $Na_2SO_4$, the organic layer was concentrated to give the crude product which was purified by silica gel chromatography (PE/EA = 10/1) to give compound **K3** (16 g) as a brown oil. MS calc'd 147 ($MH^+$), measured 147 ($MH^+$).

**Step 4: preparation of 5-bromo-2,3-dideuterio-8-methyl-quinoxaline (compound K4)**

A solution of 2,3-dideuterio-5-methyl-quinoxaline (compound **K3,** 10 g, 58.14 mmol), *N*-bromosuccinimide (20697 mg, 116.29 mmol) in ACN (250 mL) was stirred at 80 °C for 18 hrs. After being cooled down, the solution was concentrated to give a crude solid which was washed with 300 mL water. The crude product was purified by silica gel chromatography (PE/EA = 5/1) to give compound **K4** (8500 mg) as a light yellow solid. MS calc'd 225($MH^+$), measured 225($MH^+$).

**Step 5: preparation of 5-bromo-2,3-dideuterio-8-(dibromomethyl)quinoxaline (compound K5)**

A solution of 5-bromo-2,3-dideuterio-8-methyl-quinoxaline (compound **K4,** 8500 mg, 37.76 mmol), *N*-bromosuccinimide (26886 mg, 151.06 mmol), 2,2'-azobis(2-methylpropionitrile) (1240 mg, 7.55 mmol) in carbon tetrachloride (250 mL) was stirred at 80 °C for 18 hrs. After being cooled down, the mixture was concentrated to give an oil which was diluted with 300 mL water. The mixture was stirred for 1 h and filtered. The wet-cake was purified by silica gel chromatography (PE/EA = 10/1) to give compound **K5** (10 g) as a yellow solid. MS calc'd 381 ($MH^+$), measured 381 ($MH^+$).

**Step 6: preparation of 8-bromo-2,3-dideuterio-quinoxaline-5-carbaldehyde (compound K6)**

A solution of silver nitrate (17.8 g, 104.47 mmol) in water (200 mL) was added to the solution of 5-bromo-2,3-dideuterio-8-(dibromomethyl)quinoxaline (compound **K5,** 10000 mg, 26.12 mmol) in ethanol (400 mL). After being stirred at rt for 2 hrs, the mixture was concentrated to about 300 mL and filtered. The filtrate was diluted with 500 mL water and extracted with 200 mL EA for three times. The combined organic layer was washed with 200 mL water twice and 100 mL brine once, dried over $Na_2SO_4$ and concentrated to give the crude product (batch 1). The wet-cake was stirred in 500 mL DCM for 1 h, filtered and the wet-cake was washed with 100 mL DCM twice. The filtrate was concentrated to give the crude product as an oil (batch 2). The two batches were combined to give compound **K6** (5.5 g) as a yellow solid. MS calc'd 239 ($MH^+$), measured 239 ($MH^+$).

**Step 7: preparation of (5*E*)-8-bromo-2,3-dideuterio-quinoxaline-5-carbaldehyde oxime (compound K7)**

A solution of 8-bromo-2,3-dideuterio-quinoxaline-5-carbaldehyde (compound **K6,** 5.5 g, 23.01 mmol), hydroxylamine hydrochloride (2.1 g, 29.91 mmol), sodium acetate (4.15 g, 50.61 mmol) in ethanol (250 mL) was stirred at 70 °C for 16 hrs. After being cooled down, the solution was concentrated to give a residue which was treated with 300 mL water and stirred for 0.5 h. The suspension was filtered, and the wet-cake was treated with 300 mL MeCN. After concentration, the compound **K7** (4.5 g) was obtained as a yellow solid. MS calc'd 254 ($MH^+$), measured 254 ($MH^+$).

**Step 8: preparation of 8-bromo-2,3-dideuterio-quinoxaline-5-carbonitrile (compound K8)**

A solution of (5E)-8-bromo-2,3-dideuterio-quinoxaline-5-carbaldehyde oxime (compound **K7,** 4.5 g, 17.71 mmol), copper(II) acetate (965 mg, 5.31 mmol), acetic acid (1.38 g, 23.02 mmol) in acetonitrile (120 mL) was stirred at 80 °C for 18 hrs. After being cooled down, the solution was concentrated to give an oil which was dissolved in 500 mL DCM. The suspension was stirred for 1h, filtered, and the wet-cake was washed with 100 mL DCM twice. The filtrate was concentrated and purified by silica gel chromatography (PE/EA/DCM = 3/1/1) to give compound **K8** (2.6 g) as a yellow solid. MS calc'd 236 ($MH^+$), measured 236 ($MH^+$).

**Intermediate L**

**[(2*R*,6*R*)-4-(8-cyano-2-deuterio-5-quinolyl)-6-methyl-morpholin-2-yl]methyl trifluoromethanesulfonate**

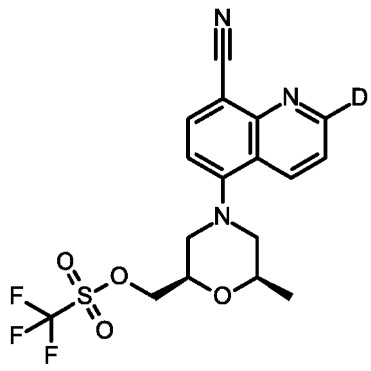

The compound **L** was prepared according to the following scheme:

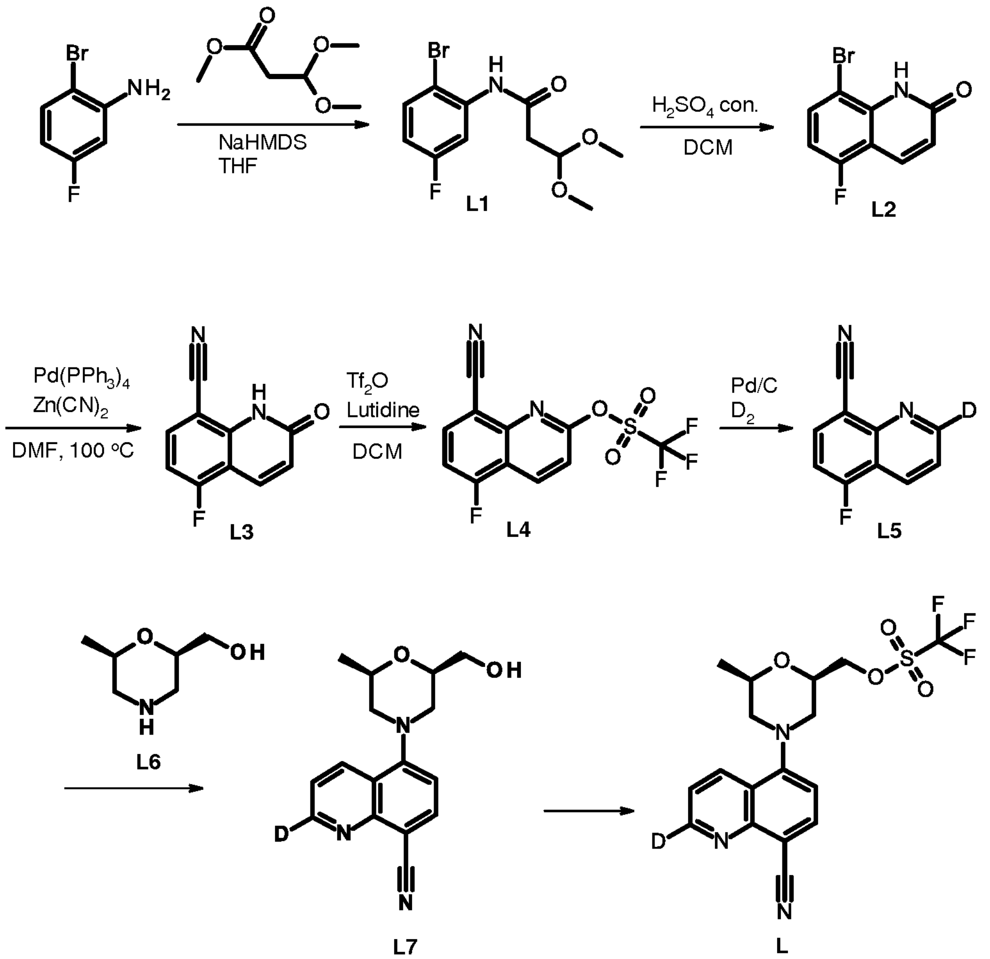


**Step 1: preparation of *N*-(2-bromo-5-fluoro-phenyl)-3,3-dimethoxy-propanamide (compound L1)**

To the solution of 2-bromo-5-fluoroaniline (CAS: 1003-99-2, Vendor: TCI, 50 g, 263.14 mmol) and methyl 3,3-dimethoxypropionate (CAS: 7424-91-1, Vendor: Accela, 45 mL, 315.77 mmol) in THF (150 mL) was added NaHMDS in THF (1M, 394 mL, 394.72 mmol) dropwise at 0 °C. After being stirred at 0 °C for 10 mins, the mixture was warmed

up to 15 °C and stirred for 18 hrs. The reaction was quenched by addition of 100 mL sat. $NH_4Cl$ and concentrated to about 300 mL. The solution was diluted with 500 mL water and extracted with 200 mL EA for three times. The combined organic layer was washed with 200 mL water twice and 100 mL brine, dried over $Na_2SO_4$ and concentrated to give the crude product **L1** (100 g) as a brown oil. MS calc'd 321($MH^+$), measured 321 ($MH^+$).

**Step 2: preparation of 8-bromo-5-fluoro-1*H*-quinolin-2-one (compound L2)**

A solution of *N*-(2-bromo-5-fluoro-phenyl)-3,3-dimethoxy-propanamide (compound **L1**, 100 g, 238.46 mmol) in DCM (500 mL) was added to concentrated sulfuric acid (300 mL) at 0 °C. After being stirred at 15 °C for 2 hrs, the mixture was poured slowly into 2000 mL ice-water, a yellow precipitate was appeared. The mixture was filtered, and the wet-cake was washed with 500 mL water, 200 mL isopropyl alcohol and 300 mL PE. The solid was dried in vacuum to give compound **L2** (50 g) as a yellow solid. MS calc'd 242 ($MH^+$), measured 242 ($MH^+$).

**Step 3: preparation of 5-fluoro-2-oxo-1*H*-quinoline-8-carbonitrile (compound L3)**

A solution of 8-bromo-5-fluoro-1*H*-quinolin-2-one (compound **L2**, 50 g, 206.58 mmol), zinc cyanide (48.50 g, 413.15 mmol), $Pd(PPh_3)_4$ (24.29 g, 21.02 mmol) in DMF (1000 mL) was stirred at 120 °C for 5 hrs. After being cooled down, the reaction mixture was quenched with 300 mL saturated $NH_4Cl$, diluted with 2000 mL water and extracted with 500 mL DCM for three times. The combined organic layer was washed with 500 mL water twice and 200 mL brine once, dried over $Na_2SO_4$ and concentrated to give the crude product which was purified by silica gel chromatography (PE/EA = 3/1) to give compound **L3** (29 g) as a yellow solid. MS calc'd 189 ($MH^+$), measured 189 ($MH^+$).

**Step 4: preparation of (8-cyano-5-fluoro-2-quinolyl) trifluoromethanesulfonate (compound L4)**

To the solution of 5-fluoro-2-oxo-1*H*-quinoline-8-carbonitrile (compound **L3,** 17000 mg, 90.35 mmol), 2,6-dimethylpyridine (38705 mg, 361.39 mmol) in DCM (500 mL) was added trifluoromethanesulfonic anhydride (50975 mg, 180.70 mmol) at 0 °C. After being stirred at 0 °C for 1 h, the mixture was diluted with 500 mL water and extracted with 200 mL DCM for three times. The combined organic layer was washed with 200 mL water twice and 100 mL brine once, dried over $Na_2SO_4$ and concentrated to give the crude product which was purified by silica gel chromatography (PE/EA = 5/1) to give compound **L4** (23000 mg) as a yellow solid. MS calc'd 321 ($MH^+$), measured 321($MH^+$).

**Step 5: preparation of 2-deuterio-5-fluoro-quinoline-8-carbonitrile (compound L5)**

To the solution of (8-cyano-5-fluoro-2-quinolyl) trifluoromethanesulfonate (compound **L4,** 23 g, 71.83 mmol) in THF (230 mL) and deuterium oxide (100 mL) was added potassium carbonate (19.8 g, 1.44 mol) and Pd/C (10 wt.%, 6 g). The mixture was stirred at 40 °C for 5 hrs under deuterium atmosphere. Then the mixture was filtered and the filtrate was concentrated and purified by silica gel chromatography (PE/EA = 5/1) to give compound **L5** (11.4 g) as a light yellow solid. MS calc'd 174 ($MH^+$), measured 174 ($MH^+$).

**Step 6: preparation of 5-((2R,6R)-2-(hydroxymethyl)-6-methylmorpholino)quinoline-8-carbonitrile-2-d (compound L7)**

A mixture of 5-fluoroquinoline-8-carbonitrile-2-d (compound **L5,** 611 mg, 3.53 mmol), ((2*R*,6*R*)-6-methylmorpholin-2-yl)methanol hydrochloride salt (compound **L6**, 710 mg, 4.23 mmol), *N*-ethyl-*N*-isopropylpropan-2-amine (1.37 g, 10.6 mmol) in DMSO (3 mL) was stirred at 120°C for 16 hours. Then the solution was diluted with EA and washed with water and brine. The organic layer was dried and concentrated. The residue was purified by silica gel chromatography (EA/PE from 20% to 80%) to afford 5-((2*R*,6*R*)-2-(hydroxymethyl)-6-methylmorpholino)quinoline-8-carbonitrile-2-d **(compound L7,** 990 mg) as a yellow solid . MS: calc'd 285 ($MH^+$), measured 285 ($MH^+$).

**Step 7: preparation of ((2R,6R)-4-(8-cyanoquinolin-5-yl-2-d)-6-methylmorpholin-2-yl)methyl trifluoromethanesulfonate (Intermediate L)**

To a solution of 5-((2*R*,6*R*)-2-(hydroxymethyl)-6-methylmorphofino) quinoline-8-carbonitrile-2-d **(compound L7,** 3 g) and 2,6-dimethylpyridine (3.39 g, 3.77 ml, 31.7 mmol) in $CH_2Cl_2$ (20 ml) was added trifluoromethanesulfonic anhydride (4.47 g, 2.67 ml, 15.8 mmol) dropwise at 0°C. The mixture was stirred at 0°C for 1h. The mixture was then diluted with DCM and washed with saturated $NH_4Cl$ solution and brine. The organic layer was dried over $Na_2SO_4$ and concentrated. The residue was purified by silica gel chromatography (EA/PE from 20% to 60%) to afford ((2*R*,6*R*)-4-(8-cyanoquinolin-5-yl-2-d)-6-methylmorpholin-2-yl)methyl trifluoromethanesulfonate (Intermediate **L** 4.087 g) as a yellow

solid. MS: calc'd 417 ($MH^+$), measured 417 ($MH^+$).

**Intermediate M**

**[(*2R*,*6R*)-6-methyl-4-(1-methyl-2-oxo-1,8-naphthyridin-4-yl)morpholin-2-yl]methyl trifluoromethanesulfonate**

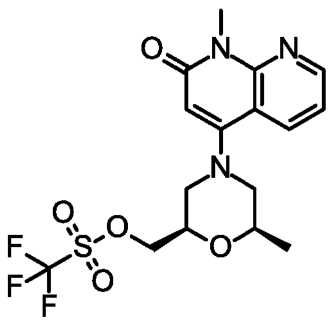

The title compound was prepared in analogy to the preparation of **Intermediate A** by using 4-bromo-1-methyl-1,8-naphthyridin-2-one (compound **M1**) instead of 5-bromoquinoline-8-carbonitrile (compound **A2**). **Intermediate M** (140 mg) was obtained as a yellow solid. MS: calc'd 422 ($MH^+$), measured 422 ($MH^+$).

The compound **M1** was prepared according to the following scheme:

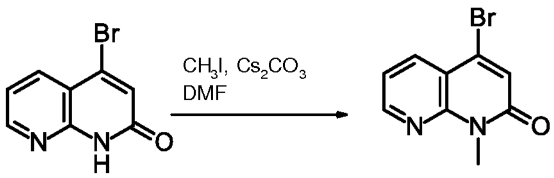


**M1**

**preparation of 4-bromo-1-methyl-1,8-naphthyridin-2-one (compound M1)**

To the solution of 4-bromo-1,8-naphthyridin-2(1H)-one (CAS: 72235-36-0, Vendor: Accela, 370 mg, 1.64 mmol) in DMF (15 mL) was added iodomethane (2.33 g, 16.40 mmol) and $Cs_2CO_3$ (1.07 g, 3.29 mmol). The reaction mixture was stirred at 80 °C overnight. After being cooled down, the reaction was quenched by ice-water (30 mL). The solid was collected by filtration to give crude compound **M1** (419 mg) which was used directly for next step. MS calc'd 239 ($MH^+$), measured 239 ($MH^+$). $^1H$ NMR (400 MHz, METHANOL-d4) $\delta$ = 8.72 (dd, J=1.7, 4.7 Hz, 1H), 8.39 (dd, *J*=1.7, 7.9 Hz, 1H), 7.43 (dd, *J*=4.7, 8.0 Hz, 1H), 7.20 (s, 1H), 3.83 (s, 3H).

**Reference Compound R1**

**5-[(2*S*,6*R*)-2-[[4-(4-methoxyphenyl)piperazin-1-yl]methyl]-6-methyl-morpholin-4-yl] quinoline-8-carbonitrile**

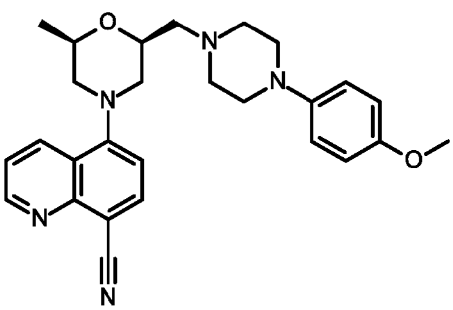

The title compound was prepared according to the following scheme:

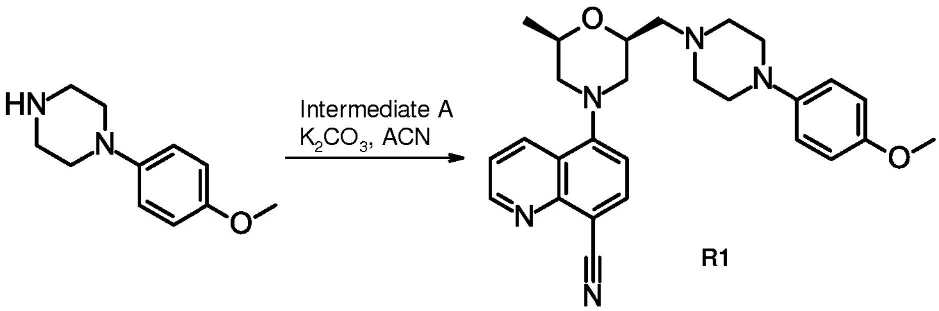


R1

To a flask was added [(2*R*,6*R*)-4-(8-cyano-5-quinolyl)-6-methyl-morphofin-2-yl]methyl trifluoromethanesulfonate (Intermediate **A**, 30 mg, 72 μmol), 1-(4-methoxyphenyl)piperazine (CAS: 38212-30-5, Vendor: Accela, 21 mg, 108 μmol), potassium carbonate (30 mg, 217 μmol) and acetonitrile (4 mL). After being stirred at 85 °C for 2 hrs, the mixture was filtered through celite and purified by prep-HPLC to give compound **R1** (22 mg) as a yellow solid. MS: calc'd 458 ($MH^+$), measured 458 ($MH^+$). $^1H$ NMR (400MHz, METHANOL-$d_4$) $\delta$ = 9.00 (dd, *J*=1.7, 4.2 Hz, 1H), 8.67 (dd, *J*=1.7, 8.6 Hz, 1H), 8.18 (d, *J*=8.1 Hz, 1H), 7.66 (dd, *J*=4.3, 8.6 Hz, 1H), 7.30 (d, *J*=7.9 Hz, 1H), 7.05 - 6.98 (m, 2H), 6.93 - 6.85 (m, 2H), 4.55 - 4.46 (m, 1H), 4.26 - 4.16 (m, 1H), 3.89 - 3.56 (m, 7H), 3.50 - 3.37 (m, 6H), 3.25 - 3.00 (m, 2H), 2.84 - 2.71 (m, 2H), 1.33 (d, *J*=6.2 Hz, 3H).

**Reference Compound R2**

**5-[(2*S*,6*R*)-2-[[4-(4-chlorophenyl)piperazin-1-yl]methyl]-6-methyl-morpholin-4-yl]quinoline-8-carbonitrile**

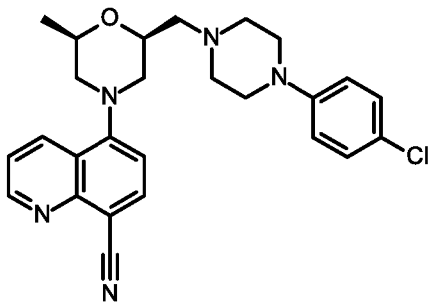

The title compound was prepared in analogy to the preparation of compound **R1** by using 1-(4-cholophenyl)piperazine (CAS: 38212-33-8, Vendor: BePharm) instead of 1-(4-methoxyphenyl)piperazine. Compound **R2** (24 mg) was obtained as a yellow solid. MS: calc'd 462 ($MH^+$), measured 462 ($MH^+$). $^1H$ NMR (400MHz, METHANOL-d4) $\delta$ = 9.00 (dd, 1=1.6, 4.3 Hz, 1H), 8.67 (dd, *J*=1.7, 8.6 Hz, 1H), 8.17 (d, *J*=8.1 Hz, 1H), 7.66 (dd, *J*=4.3, 8.6 Hz, 1H), 7.34 - 7.26 (m, 3H), 7.07 - 6.98 (m, 2H), 4.55 - 4.47 (m, 1H), 4.25 - 4.15 (m, 1H), 3.99 - 3.55 (m, 3H), 3.55 - 3.32 (m, 7H), 3.28 - 3.04 (m, 2H), 2.85 - 2.71 (m, 2H), 1.33 (d, *J*=6.2 Hz, 3H).

**Example 1**

**5-[(*2R,6S*)-2-methyl-6-[[3-(3-piperazin-1-ylphenyl)pyrrolidin-1-yl]methyl]morpholin-4-**yl] **quinoline-8-carbonitrile**

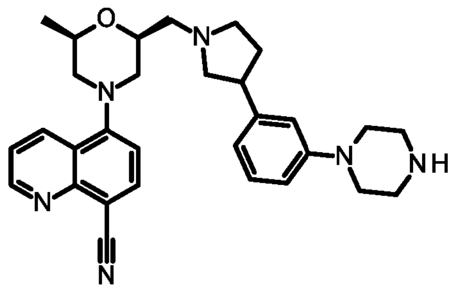

The title compound was prepared according to the following scheme:

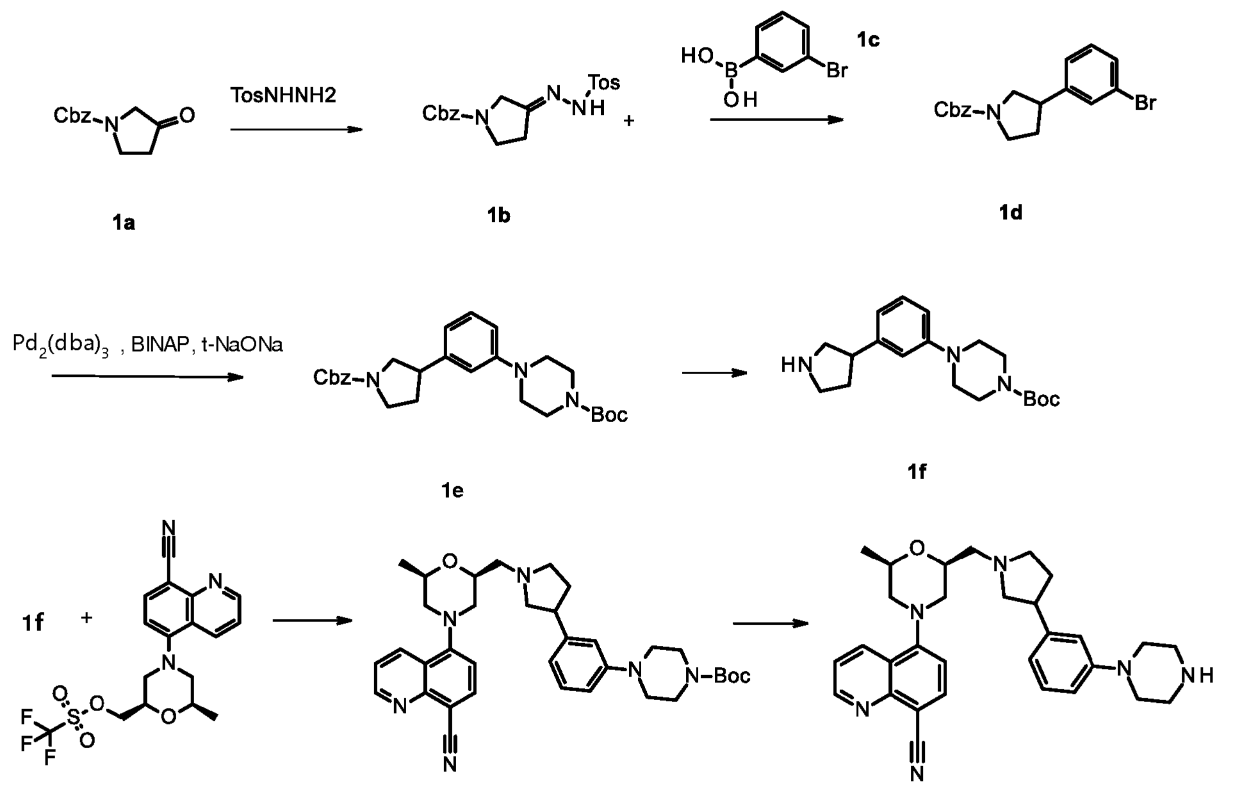


1a 1b 1d

1e 1f

Intermediate A 1g 1

**Step 1: preparation of benzyl (3Z)-3-(p-tolylsulfonylhydrazono)pyrrolidine-1-carboxylate (compound 1b)**

To a solution of 4-methylbenzenesulfonhydrazide (2.8 g, 15.05 mmol) in ethanol (30 mL) was added benzyl 3-oxopyrrolidine-1-carboxylate (compound **1a**, 2.56 mL, 13 mmol). The reaction was stirred at 25°C for 3 hrs. The solid was filtrated, washed with EtOH (50 mL), and dried to give the desired product compound **1b** (4.0 g) as a yellow solid. MS calc'd 388 ($MH^+$); measured 388 ($MH^+$).

**Step 2: preparation of benzyl 3-(3-bromophenyl)pyrrolidine-1-carboxylate (compound 1d)**

To a solution of 3-bromophenylboronic acid (compound **1c**, 777 mg, 4 mmol) in 1,4-dioxane (10 mL) was added benzyl (3Z)-3-(p-tolylsulfonylhydrazono)pyrrolidine-1-carboxylate (compound **1b**, 1.0 g, 2.58 mmol), and cesium carbonate (1.26 g, 3.87 mmol). The reaction mixture was stirred for 16 hours at 115°C under $N_2$, then filtered and the filtrate was concentrated under reduced pressure. The residue was purified by column (PE/EA=3/1, Rf=0.6) to give compound **1d** (300 mg) as yellow solid. MS calc'd 360 ($MH^+$), measured 360 ($MH^+$).

**Step 3: preparation of *tert*-butyl 4-[3-(1-benzyloxycarbonylpyrrolidin-3-yl)phenyl]piperazine-1-carboxylate (compound 1e)**

To a solution of 1-Boc-piperazine (201.6 mg, 1.08 mmol) in toluene (5 mL) was added benzyl 3-(3-bromophenyl)pyrrolidine-1-carboxylate (compound **1d**, 300 mg, 0.83 mmol), sodium *tert*-butoxide (200 mg, 2.08 mmol), (*R*)-binap (103 mg, 0.17 mmol) and bis(dibenzylideneacetone)palladium (48 mg, 0.08 mmol). The reaction was stirred at 110°C for 18 hrs. The mixture was cooled to room temperature and filtered through celite. The filtrate was concentrated under reduced pressure and the residue was purified by silica gel chromatography (PE/EA=3/1, Rf=0.3) to give compound **1e** (200 mg) as a yellow solid. MS calc'd 466.3 ($MH^+$); measured 466.3 ($MH^+$).

**Step 4: preparation of *tert*-butyl 4-isoindolin-5-ylpiperazine-1-carboxylate (compound 1f)**

To a solution of *tert*-butyl 4-[3-(1-benzyloxycarbonylpyrrolidin-3-yl)phenyl]piperazine-1-carboxylate (compound **1e**, 180 mg, 0.41 mmol) and $Pd(OH)_2/C$ (18 mg, 0.41 mmol) in 2-propanol (2 mL) was stirred for 3 hrs at 25°C under $H_2$ balloon atmosphere. The mixture was filtered and the filtrate was concentrated to give compound **1f** (50 mg) as yellow oil. MS calc'd 332.2 ($MH^+$), measured 332.2 ($MH^+$).

**Step 5: preparation of tert-butyl 4-[3-[1-[[(2*S*,6*R*)-4-(8-cyano-5-quinolyl)-6-methyl-morpholin-2-yl]methyl]pyrrolidin-3-yl]phenyl]piperazine-1-carboxylate (compound 1g)**

To a solution of *tert*-butyl 4-(3-pyrrolidin-3-ylphenyl)piperazine-1-carboxylate (compound **1f**, 50 mg, 0.15 mmol) in ACN (1 mL) was added potassium carbonate (52 mg, 0.38 mmol) and [(2*R*,6*R*)-4-(8-cyano-5-quinolyl)-6-methylmorpholin-2-yl]methyl trifluoromethanesulfonate (intermediate **A**, 75 mg, 0.18 mmol). The mixture was stirred for 3 hrs at 55 °C, then filtered and the filtrate was concentrated. The residue was purified by pre-TLC (EA:PE=1:1, Rf =0.5) to get compound **1g** (50 mg) as yellow oil. MS calc'd 597.4 ($MH^+$), measured 597.4 ($MH^+$).

**Step 6: preparation of 5-[(*2R,6S*)-2-methyl-6-[[3-(3-piperazin-1-ylphenyl)pyrrolidin-1-yl]methyl]morpholin-4-yl]quinoline-8-carbonitrile (Example 1)**

To a solution of *tert*-butyl 4-[3-[1-[[(2*S*,6*R*)-4-(8-cyano-5-quinolyl)-6-methyl-morphofin-2-yl]methyl]pyrrolidin-3-yl]phenyl]piperazine-1-carboxylate(compound **1g**, 50 mg, 0.080 mmol) in DCM (2 mL) was added trifluoroacetic acid (1.0 mL, 12.98 mmol) under ice-bath. After being stirred at rt for 3 hrs, the mixture was concentrated *in vacuo* to give a yellow oil which was purified by prep-HPLC to give **Example 1** (24 mg) as a yellow solid. MS calc'd 497.3 ($MH^+$), measured 497.3 ($MH^+$).$^1$H NMR (400 MHz, METHANOL-$d_4$) $\delta$: ppm 9.02 (br s, 1H), 8.64 - 8.73 (m, 1H), 8.19 (d, *J* = 8.0 Hz, 1H), 7.67 (br d, *J* = 4.1 Hz, 1H), 7.28 - 7.39 (m, 2H), 6.94 - 7.08 (m, 3H), 4.43 (br s, 1H), 4.20 (br s, 1H), 3.57 - 4.14 (m, 4H), 3.36 - 3.55 (m, 13H), 2.71 - 2.86 (m, 2H), 2.56 (br s, 1H), 2.17 - 2.38 (m, 1H), 1.34 (br d, *J* = 6.1 Hz, 3 H).

**Example 2**

**5-[(2R, 6S)-2-methyl-6-[[4-[3-(4-piperidyl)phenyl]-1-piperidyl] methyl]morpholin-4-yl] quinoline-8-carbonitrile**

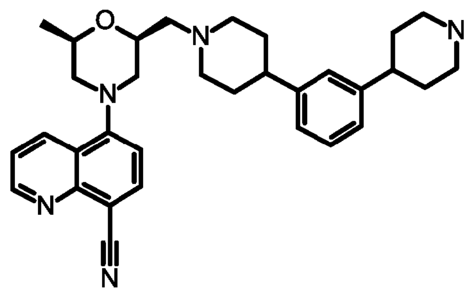

The title compound was prepared in analogy to the preparation of Example **1** by using benzyl 4-oxopiperidine-1-carboxylate and [3-(1-*tert*-butoxycarbonyl-4-piperidyl)phenyl]boronic acid instead of benzyl 3-oxopyrrolidine-1-carboxylate (compound **1a**) and 3-bromophenylboronic acid (compound **1c**). **Example 2** (14 mg) was obtained as a yellow solid. MS: calc'd 510 ($MH^+$), measured 510 ($MH^+$). $^1$H NMR (400 MHz, METHANOL-d4) $\delta$9.01 (dd, *J* = 1.5, 4.1 Hz, 1H),

8.69 (dd, *J* = 1.6, 8.6 Hz, 1H), 8.18 (d, *J* = 7.9 Hz, 1H), 7.67 (dd, *J* = 4.3, 8.5 Hz, 1H), 7.38 - 7.33 (m, 1H), 7.30 (d, *J* = 8.0 Hz, 1H), 7.28 - 7.16 (m, 3H), 4.58 - 4.49 (m, 1H), 4.25 - 4.15 (m, 1H), 3.85 (br d, *J* = 4.9 Hz, 2H), 3.53 (br d, *J* = 12.7 Hz, 2H), 3.45 (br d, *J* = 11.5 Hz, 2H), 3.39 - 3.35 (m, 2H), 3.29 - 3.11 (m, 4H), 3.00 - 2.88 (m, 2H), 2.84 - 2.71 (m, 2H), 2.22 - 1.89 (m, 8H), 1.34 (d, *J* = 6.3 Hz, 3H).

**Example 3**

**5-[(*2R*,*6S*)-2-methyl-6-[[3-(4-piperazin-1-ylphenyl)azetidin-1-yl]methyl]morpholin-4-yl]quinoline-8-carbonitrile**

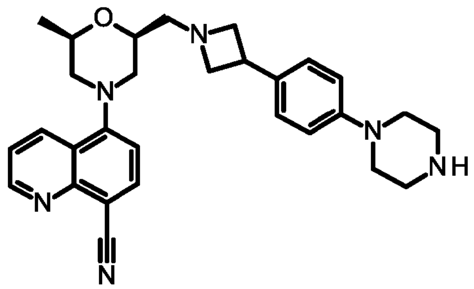

The title compound was prepared in analogy to the preparation of Example **1** by using benzyl 2-(3-oxoazetidin-1-yl)acetate and 4-bromophenylboronic acid instead of benzyl 3-oxopyrrolidine-1-carboxylate (compound **1a**) and 3-bromophenylboronic acid (compound **1c**). **Example 3** (5 mg) was obtained as a yellow solid. MS: calc'd 483 ($MH^+$), measured 483 ($MH^+$). $^1H$ NMR (400 MHz, METHANOL-d4) $\delta$ 9.01 (d, *J* = 4.0 Hz, 1H), 8.68 (d, *J* = 8.8 Hz, 1H), 8.18 (d, *J* = 7.6Hz, 1H), 7.67 (dd, *J* = 4.3, 8.5Hz, 1H), 7.38- 7.36 (m, 2H), 7.29 (d, *J* = 8 Hz, 1H), 7.10 - 7.08 (m, 2H), 4.62 - 4.16 (m, 7H), 3.52 -3.39 (m, 12H), 2.82-2.70 (m, 2H), 1.33-1.31 (d, *J* = 6.0 Hz, 3H).

**Example 4**

**5-[(2R, 6S)-2-methyl-6-[[3-(3-piperazin-1-ylphenyl)azetidin-1-yl]methyl]morpholin-4-yl] quinoline-8-carbonitrile**

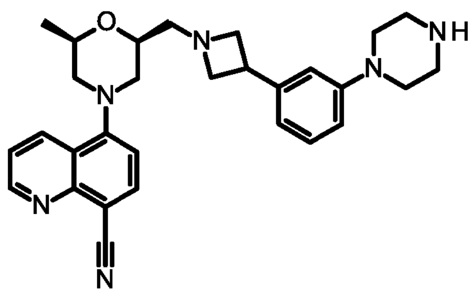

The title compound was prepared in analogy to the preparation of Example **1** by using benzyl 2-(3-oxoazetidin-1-yl)acetate instead of benzyl 3-oxopyrrolidine-1-carboxylate (compound **1a** ). **Example 4** (26 mg) was obtained as a yellow solid. MS: calc'd 483 ($MH^+$), measured 483 ($MH^+$). $^1H$ NMR (400 MHz, METHANOL-d4) $\delta$ 9.01 (dd, *J* = 4.3, 1.5 Hz, 1H), 8.67 (dd, *J*= 8.7, 1.6 Hz, 1H), 8.19 (d, *J* = 8.0 Hz, 1H), 7.67 (dd, *J*= 8.5, 4.3 Hz, 1H), 7.37 (br t, *J* = 8.3 Hz, 1H), 7.30 (d, *J* = 8.0 Hz, 1H), 6.99 - 7.13 (m, 3H), 4.12 - 4.67 (m, 7H), 3.37 - 3.55 (m, 12H), 2.68 - 2.86 (m, 2H), 1.33 (br d, *J* = 6.1 Hz, 3 H).

## Example 5

**5-[(*2S,6R*)-2-[[4-[4-(aminomethyl)phenyl]piperazin-1-yl]methyl]-6-methyl-morpholin-4-yl]quinoline-8-carbonitrile**

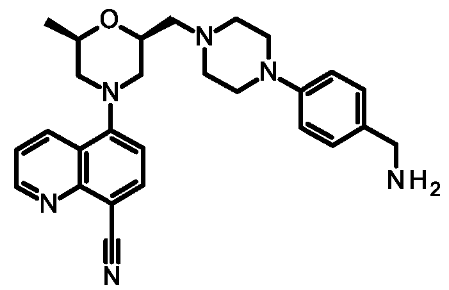

The title compound was prepared according to the following scheme:

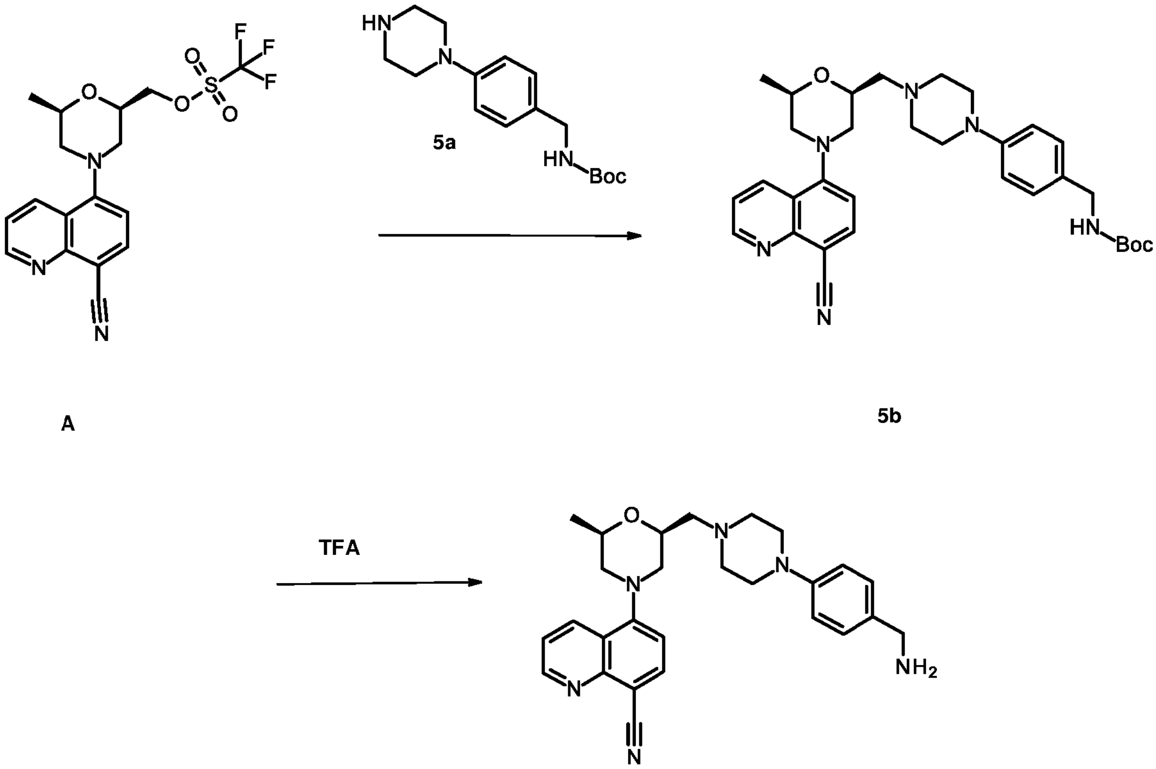


5a

A

5b

5

The solution of [(*2R,6R*)-4-(8-cyano-5-quinolyl)-6-methyl-morphofin-2-yl]methyl trifluoromethanesulfonate(intermediate **A**, 40 mg, 0.1 mmol), tert-butyl 4-(piperazin-1-yl)benzylcarbamate (compound **5a**, 60 mg, 0.2 mmol) and $K_2CO_3$ (44 mg, 0.3 mmol) in ACN (3 mL) was stirred at 50 °C for 2 hrs. The reaction mixture was diluted with EA (20 mL) and washed with $H_2O$ (20 mL). The organic layer was dried and concentrated under vacuum. The residue was purified by TLC (EA/PE=1/1, $R_f$= 0.5) to give compound **5b** (30 mg). MS calc'd 557 ($MH^+$); measured 557 ($MH^+$).

To a solution of *tert*-butyl *N*-[[4-[4-[[(2*S*,6*R*)-4-(8-cyano-5-quinolyl)-6-methyl-morpholin-2-yl]methyl]piperazin-1-yl]phenyl]methyl]carbamate (compound **5b**, 30 mg) in DCM (2 mL) was added TFA (1 mL). After being stirred at 20 °C for 0.5 hour, the mixture was concentrated under vacuum. The residue was purified by prep-HPLC to give **Example 5** (4 mg) as a yellow solid. MS calc'd 457 ($MH^+$); measured 440 ($MH^+$). $^1H$ NMR (400MHz, METHANOL-d4) δppm: 9.01 (d, *J* = 2.8 Hz, 1H), 8.69 (d, *J* = 8.4 Hz, 1H), 8.19 (d, *J* = 7.9 Hz, 1H), 7.68 (dd, *J* = 4.3, 8.5 Hz, 1H), 7.41 (d, *J* = 8.5 Hz,

2H), 7.31 (d, *J* = 8.0 Hz, 1H), 7.13 (d, *J* = 8.7 Hz, 2H), 4.54 (br s, 1H), 4.32 - 4.18 (m, 1H), 4.06 (s, 2H), 3.88 - 3.34 (m, 12H), 2.93 - 2.69 (m, 2H), 1.34 (d, *J* = 6.3 Hz, 3H).

**Example 6**

**5-[(*2R,6S*)-2-methyl-6-[[4-(5-piperazin-1-ylpyrazin-2-yl)piperazin-1-yl]methyl]morpholin-4-yl]quinoline-8-carbonitrile**

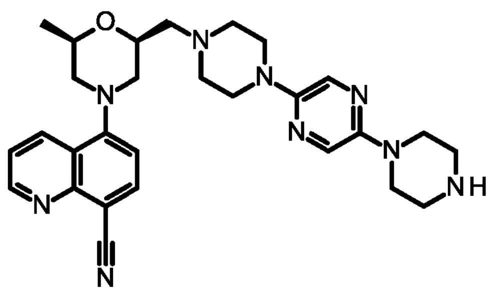

The title compound was prepared according to the following scheme:

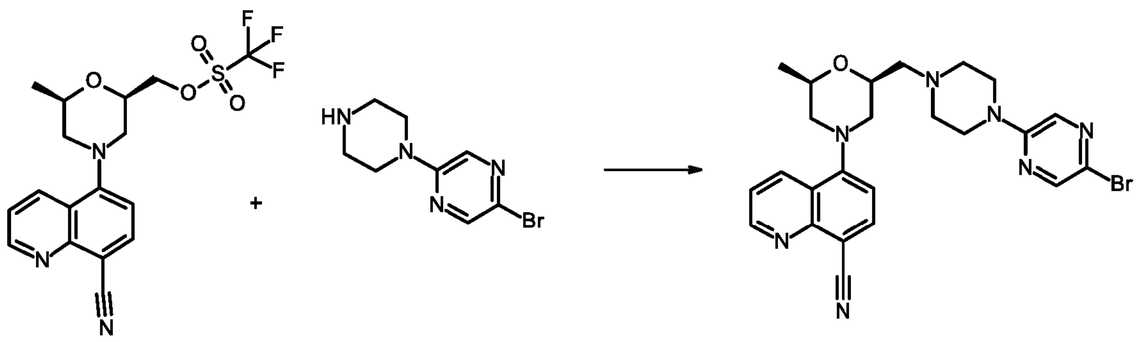

**A** **6a** **6b**

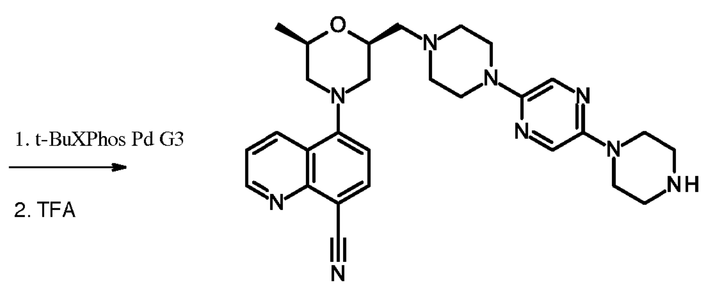


**6**

**Step 1: preparation of 5-[(2*S*,6*R*)-2-[[4-(5-bromopyrazin-2-yl)piperazin-1-yl]methyl]-6-methyl-morpholin-4-yl]quinoline-8-carbonitrile (compound 6b)**

To a tube was added 2-bromo-5-(piperazin-1-yl)pyrazine (compound **6a**, 29 mg, 120 μmol), [(2*R*,6*R*)-4-(8-cyano-5-quinolyl)-6-methyl-morpholin-2-yl]methyl trifluoromethanesulfonate (intermediate **A**, 50 mg, 120 μmol), $K_2CO_3$ (166 mg, 1.2 mmol) and ACN (10 mL), a suspension was formed. The mixture was heated to reflux for 4 hrs, then diluted

with some ACN and filtered through celite. The filtrate was concentrated and diluted with EA. The solution was washed with water. The organic layer was concentrated and the residue was purified by silica gel chromatography to give compound **6b** (45 mg). MS calc'd 509 ($MH^+$); measured 509 ($MH^+$).

**Step 2: preparation of 5-[(2*R*,6*S*)-2-methyl-6-[[4-(5-piperazin-1-ylpyrazin-2-yl)piperazin-1-yl]methyl]morpholin-4-yl]quinoline-8-carbonitrile (Example 6)**

A mixture of 5-[(2*S*,6*R*)-2-[[4-(5-bromopyrazin-2-yl)piperazin-1-yl]methyl]-6-methyl-morpholin-4-yl]quinoline-8-carbonitrile(compound **6b**, 30 mg, 60 μmol), *tert*-butyl piperazine-1-carboxylate (12 mg, 60 μmol), *t*-BuXPhos Pd G3 (5 mg) and sodium *tert*-butoxide (17 mg, 180 μmol) in dioxane (10 mL) was heated at 90 °C overnight under $N_2$ atmosphere. After cooling, the solid was filtered off and washed with EA (20 mL). The filtrate was concentrated to crude intermediate.

The intermediate was dissolved in 5 mL DCM, followed by the addition of 0.5 mL TFA. After being stirred at rt for 3 hrs, the mixture was concentrated *in vacuo* to give a yellow oil which was purified by prep-HPLC. **Example 6** (2 mg) was obtained as a yellow solid. MS: calc'd 514 ($MH^+$), measured 514 ($MH^+$). $^1H$ NMR (400 MHz, METHANOL-d4) $\delta$ 9.02 (dd, *J* = 1.6, 4.3 Hz, 1H), 8.69 (dd, *J*= 1.7, 8.6 Hz, 1H), 8.19 (d, *J*= 7.9 Hz, 1H), 8.08 - 7.99 (m, 2H), 7.68 (dd, *J* = 4.3, 8.6 Hz, 1H), 7.31 (d, *J* = 8.1 Hz, 1H), 4.60 - 4.48 (m, 1H), 4.32 - 4.14 (m, 2H), 3.87 - 3.67 (m, 5H), 3.54 - 3.37 (m, 9H), 3.30 - 3.20 (m, 5H), 2.88 - 2.69 (m, 2H), 1.35 (d, *J*= 6.2 Hz, 3H).

**Example 7**

**5-[(2R, 6S)-2-methyl-6-[[4-(5-piperazin-1-yl-3-pyridyl)piperazin-1-yl] methyl]morpholin-4-yl] quinoline-8-carbonitrile**

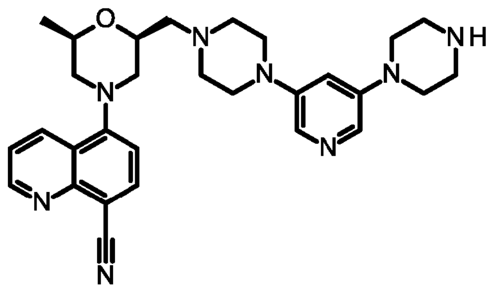

The title compound was prepared in analogy to the preparation of Example **6** by using 1-(5-bromo-3-pyridyl)piperazine instead of 2-bromo-5-(piperazin-1-yl)pyrazine (compound **6a**). **Example 7** (8 mg) was obtained as a yellow solid. MS: calc'd 513 ($MH^+$), measured 513 ($MH^+$). $^1H$ NMR (400 MHz, METHANOL-d4) $\delta$ 9.02 (dd, *J* = 1.6, 4.3 Hz, 1H), 8.69 (dd, *J* = 1.7, 8.6 Hz, 1H), 8.19 (d, *J* = 7.9 Hz, 1H), 8.02 (t, *J* = 2.7 Hz, 2H), 7.67 (dd, *J* = 4.2, 8.6 Hz, 1H), 7.52 (t, *J* = 2.1 Hz, 1H), 7.31 (d, *J*= 8.1 Hz, 1H), 4.65 - 4.49 (m, 1H), 4.27 - 4.14 (m, 1H), 3.94 - 3.57 (m, 11H), 3.50 - 3.39 (m, 8H), 2.86 - 2.67 (m, 3H), 1.34 (d, *J* = 6.2 Hz, 3H).

**Example 8**

**5-[(*2R,6S*)-2-methyl-6-[[4-(5-piperazin-1-yl-2-pyridyl)piperazin-1-yl]methyl]morpholin-4-yl] quinoline-8-carbonitrile**

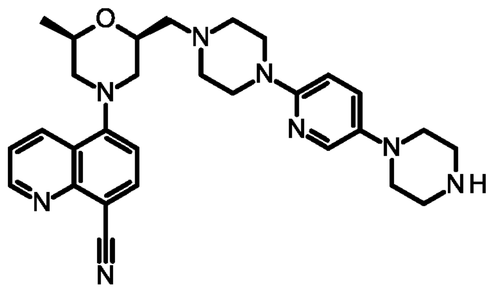

The title compound was prepared in analogy to the preparation of Example **6** by using 1-(5-bromo-2-pyridyl)piperazine instead of 2-bromo-5-(piperazin-1-yl)pyrazine (compound **6a**). **Example 8** (11 mg) was obtained as a yellow solid. MS: calc'd 513 ($MH^+$), measured 513 ($MH^+$). $^1H$ NMR (400 MHz, METHANOL-d4) $\delta$ 9.01 (dd, *J* = 1.7, 4.2 Hz, 1H), 8.68 (dd, *J* = 1.6, 8.6 Hz, 1H), 8.18 (d, *J* = 7.9 Hz, 1H), 7.93 (d, *J* = 2.8 Hz, 1H), 7.72 - 7.61 (m, 2H), 7.30 (d, *J* = 8.1 Hz, 1H), 7.12 (d, *J* = 9.3 Hz, 1H), 4.61 - 4.49 (m, 1H), 4.29 - 4.17 (m, 1H), 3.97 - 3.76 (m, 4H), 3.70 - 3.53 (m, 4H), 3.49 - 3.36 (m, 12H), 2.87 - 2.70 (m, 2H), 1.34 (d, *J* = 6.2 Hz, 3H).

**Example 9**

**5-[(*2R*,*6S*)-2-methyl-6-[[4-(4-piperazin-1-yl-2-pyridyl)piperazin-1-yl]methyl]morpholin-4-yl] quinoline-8-carbonitrile**

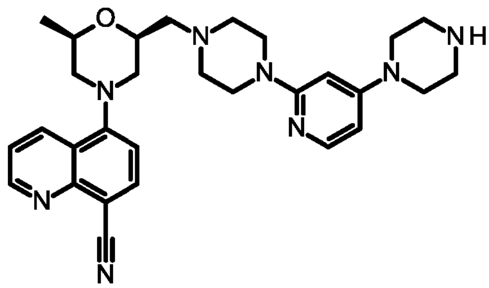

The title compound was prepared in analogy to the preparation of Example **6** by using 1-(4-bromo-2-pyridyl)piperazine instead of 2-bromo-5-(piperazin-1-yl)pyrazine (compound **6a**). **Example 9** (8 mg) was obtained as a yellow solid. MS: calc'd 513 ($MH^+$), measured 513 ($MH^+$). $^1H$ NMR (400 MHz, METHANOL-d4) $\delta$ 9.01 (dd, *J* = 1.7, 4.2 Hz, 1H), 8.68 (dd, *J* = 1.6, 8.6 Hz, 1H), 8.19 (d, *J* = 7.9 Hz, 1H), 7.84 (d, *J* = 7.5 Hz, 1H), 7.67 (dd, *J* = 4.3, 8.6 Hz, 1H), 7.30 (d, *J* = 8.1 Hz, 1H), 6.84 (dd, *J* = 2.4, 7.5 Hz, 1H), 6.46 (d, *J* = 2.3 Hz, 1H), 4.61 - 4.46 (m, 1H), 4.25 - 4.15 (m, 1H), 4.03 - 3.85 (m, 8H), 3.72 - 3.56 (m, 4H), 3.52 - 3.39 (m, 8H), 2.84 - 2.71 (m, 2H), 1.33 (d, *J* = 6.2 Hz, 3H).

**Example 10**

**5-[(2R, 6S)-2-methyl-6-[[4-(6-piperazin-1-yl-2-pyridyl)piperazin-1-yl] methyl]morpholin-4-yl]quinoline-8-carbonitrile**

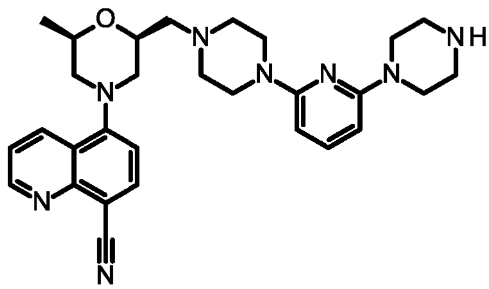

The title compound was prepared in analogy to the preparation of Example **6** by using 1-(6-bromo-2-pyridyl)piperazine instead of 2-bromo-5-(piperazin-1-yl)pyrazine (compound **6a**). **Example 10** (3 mg) was obtained as a yellow solid. MS: calc'd 513 ($MH^+$), measured 513 ($MH^+$). $^1H$ NMR (400 MHz, METHANOL-d4) $\delta$ 9.02 (dd, *J* = 1.7, 4.2 Hz, 1H), 8.69 (dd, *J* = 1.6, 8.6 Hz, 1H), 8.19 (d, *J*= 7.9 Hz, 1H), 7.67 (dd, *J* = 4.3, 8.6 Hz, 1H), 7.53 (t, *J* = 8.1 Hz, 1H), 7.31 (d, *J* = 8.1 Hz, 1H), 6.35 (dd, *J* = 4.6, 8.1 Hz, 2H), 4.60 - 4.45 (m, 2H), 4.31 - 4.15 (m, 2H), 3.87 - 3.74 (m, 5H), 3.56 - 3.38 (m, 12H), 2.88 - 2.69 (m, 3H), 1.34 (d, *J* = 6.4 Hz, 3H).

**Example 11**

**5-[(2S, 6R)-2-[[4-cyano-4-(4-piperazin-1-ylphenyl)-1-piperidyl] methyl]-6-methyl-morpholin-4-yl]quinoline-8-carbonitrile**

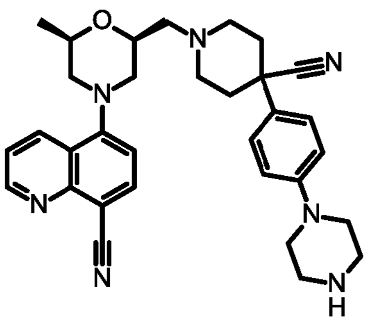

The title compound was prepared in analogy to the preparation of Example **6** by using 4-(4-bromophenyl)piperidine-4-carbonitrile instead of 2-bromo-5-(piperazin-1-yl)pyrazine (compound **6a**). **Example 11** (6 mg) was obtained as a yellow solid. MS: calc'd 536 ($MH^+$), measured 536 ($MH^+$). $^1H$ NMR (400 MHz, METHANOL-d4) $\delta$ 9.01 (dd, *J* = 1.6, 4.3 Hz, 1H), 8.69 (dd, *J* = 1.6, 8.6 Hz, 1H), 8.19 (d, *J* = 7.9 Hz, 1H), 7.67 (dd, *J* = 4.3, 8.6 Hz, 1H), 7.52 (d, *J* = 8.8 Hz, 2H), 7.31 (d, *J* = 7.9 Hz, 1H), 7.15 (d, *J* = 8.9 Hz, 2H), 4.64 - 4.46 (m, 1H), 4.27 - 4.15 (m, 1H), 4.07 - 3.87 (m, 2H), 3.67 - 3.37 (m, 14H), 2.86 - 2.70 (m, 2H), 2.62 - 2.44 (m, 4H), 1.34 (d, *J* = 6.2 Hz, 3H).

**Example 12**

**5-[(*2R,6S*)-2-methyl-6-[[4-(2-piperazin-1-ylpyrimidin-5-yl)piperazin-1-yl]methyl]morpholin-4-yl]quinoline-8-carbonitrile**

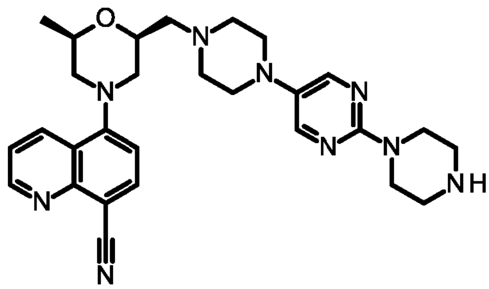

The title compound was prepared in analogy to the preparation of Example **6** by using 2-chloro-5-piperazin-1-yl-pyrimidine instead of 2-bromo-5-(piperazin-1-yl)pyrazine (compound **6a**). **Example 12** (8 mg) was obtained as a yellow solid. MS: calc'd 514 ($MH^+$), measured 514 ($MH^+$). $^1H$ NMR (400 MHz, METHANOL-d4) $\delta$ 8.87 (dd, *J*= 1.5, 4.2 Hz, 1H), 8.55 (dd, *J*= 1.6, 8.6 Hz, 1H), 8.12 (s, 2H), 8.05 (d, *J* = 7.9 Hz, 1H), 7.54 (dd, *J* = 4.3, 8.6 Hz, 1H), 7.15 (d, *J* = 8.1 Hz, 1H), 4.17 - 4.05 (m, 1H), 4.03 - 3.92 (m, 1H), 3.87 - 3.78 (m, 4H), 3.43 - 3.26 (m, 2H), 3.16 - 2.97 (m, 8H), 2.79 - 2.39 (m, 8H), 1.16 (d, *J* = 6.2 Hz, 3H).

**Example 13**

**5-[(*2R,6S*)-2-methyl-6-[[4-(6-piperazin-1-yl-3-pyridyl)piperazin-1-yl]methyl]morpholin-4-yl] quinoline-8-carbonitrile**

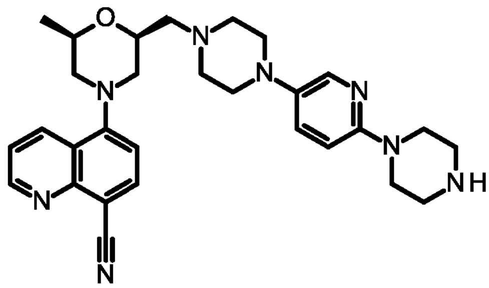

The title compound was prepared in analogy to the preparation of Example **6** by using 1-(6-bromo-3-pyridyl)piperazine instead of 2-bromo-5-(piperazin-1-yl)pyrazine (compound **6a**). **Example 13** (10 mg) was obtained as a yellow solid. MS: calc'd 513 ($MH^+$), measured 513 ($MH^+$). $^1H$ NMR (400 MHz, METHANOL-d4) $\delta$ 9.02 (dd, *J*= 1.6, 4.3 Hz, 1H), 8.69 (dd, *J*= 1.7, 8.6 Hz, 1H), 8.19 (d, *J* = 7.9 Hz, 1H), 7.99 (d, *J* = 2.9 Hz, 1H), 7.68 (dd, *J* = 4.3, 8.6 Hz, 1H), 7.54 (dd, *J* = 2.9, 9.2 Hz, 1H), 7.31 (d, *J* = 7.9 Hz, 1H), 7.00 (d, *J* = 9.3 Hz, 1H), 4.62 - 4.45 (m, 1H), 4.29 - 4.16 (m, 1H), 3.90 - 3.41 (m, 14H), 3.30-3.20 (m, 5H), 2.90 - 2.65 (m, 3H), 1.34 (d, *J* = 6.4 Hz, 3H).

**Example 14**

**5-[(*2R,6S*)-2-methyl-6-[[4-(4-piperazin-1-ylpyrimidin-2-yl)piperazin-1-yl]methyl]morpholin-4-yl]quinoline-8-carbonitrile**

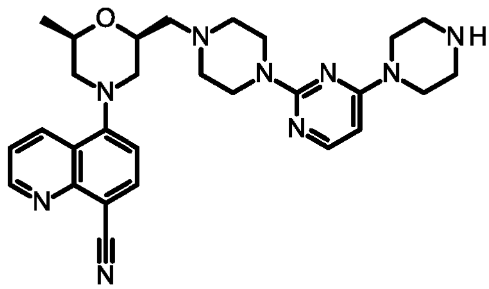

The title compound was prepared in analogy to the preparation of Example **6** by using 4-chloro-2-piperazin-1-yl-pyrimidine instead of 2-bromo-5-(piperazin-1-yl)pyrazine (compound **6a). Example 14** (26 mg) was obtained as a yellow solid. MS: calc'd 514 ($MH^+$), measured 514 ($MH^+$). $^1H$ NMR (400 MHz, METHANOL-d4) $\delta$ 9.02 (dd, *J*= 1.7, 4.3 Hz, 1H), 8.68 (dd, *J*= 1.6, 8.6 Hz, 1H), 8.19 (d, *J* = 7.9 Hz, 1H), 8.01 (d, *J* = 6.8 Hz, 1H), 7.67 (dd, *J* = 4.3, 8.6 Hz, 1H), 7.31 (d, *J* = 8.1 Hz, 1H), 6.53 (d, *J* = 7.0 Hz, 1H), 4.59 - 4.46 (m, 1H), 4.32 - 4.15 (m, 2H), 4.15 - 3.95 (m, 6H), 3.64 - 3.36 (m, 13H), 2.88 - 2.70 (m, 2H), 1.34 (d, *J* = 6.2 Hz, 3H).

**Example 15**

**5-[(*2R*,*6S*)-2-methyl-6-[[4-(2-piperazin-1-ylpyrimidin-4-yl)piperazin-1-yl]methyl]morpholin-4-yl]quinoline-8-carbonitrile**

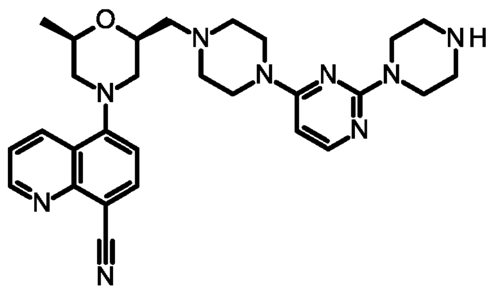

The title compound was prepared in analogy to the preparation of Example **6** by using 2-chloro-4-piperazin-1-yl-pyrimidine instead of 2-bromo-5-(piperazin-1-yl)pyrazine (compound **6a**). **Example 15** (21 mg) was obtained as a yellow solid. MS: calc'd 514 ($MH^+$), measured 514 ($MH^+$). $^1H$ NMR (400 MHz, METHANOL-d4) $\delta$ 9.02 (dd, *J* = 1.6, 4.3 Hz, 1H), 8.68 (dd, *J* = 1.7, 8.6 Hz, 1H), 8.19 (d, *J*= 8.1 Hz, 1H), 8.00 (d, *J*= 6.8 Hz, 1H), 7.67 (dd, *J* = 4.3, 8.6 Hz, 1H), 7.31 (d, *J* = 8.1 Hz, 1H), 6.57 (d, *J* = 7.0 Hz, 1H), 4.61 - 4.46 (m, 1H), 4.28 - 3.97 (m, 10H), 3.64 - 3.38 (m, 11H), 2.88 - 2.69 (m, 2H), 1.34 (d, *J* = 6.2 Hz, 3H).

**Example 16**

**5-[(2*R*,6*S*)-2-methyl-6-[[4-(6-piperazin-1-ylpyridazin-3-yl)piperazin-1-yl]methyl]morpholin-4-yl]quinoline-8-carbonitrile**

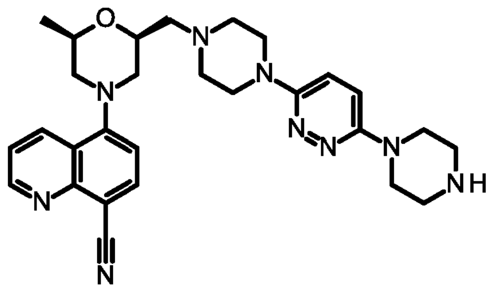

The title compound was prepared in analogy to the preparation of Example **6** by using 3-bromo-6-piperazin-1-yl-pyridazine instead of 2-bromo-5-(piperazin-1-yl)pyrazine (compound **6a**). **Example 16** (2 mg) was obtained as a yellow solid. MS: calc'd 514 ($MH^+$), measured 514 ($MH^+$). $^1H$ NMR (400 MHz, METHANOL-d4) $\delta$ 9.08 - 8.97 (m, 1H), 8.69 (d, *J* = 8.4 Hz, 1H), 8.19 (d, *J* = 8.1 Hz, 1H), 7.74 - 7.61 (m, 3H), 7.31 (d, *J* = 8.1 Hz, 1H), 4.61 - 4.47 (m, 1H), 4.30 - 4.16 (m, 1H), 4.07 - 3.75 (m, 8H), 3.73 - 3.37 (m, 12H), 2.87 - 2.70 (m, 2H), 1.34 (d, *J* = 6.2 Hz, 3H).

**Example 17**

**5-[(*2R,6S*)-2-methyl-6-[[4-(5-piperazin-1-ylpyrimidin-2-yl)piperazin-1-yl]methyl]morpholin-4-yl]quinoline-8-carbonitrile**

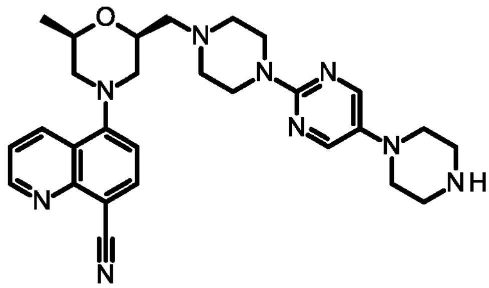

The title compound was prepared in analogy to the preparation of Example **6** by using 5-chloro-2-piperazin-1-yl-pyrimidine instead of 2-bromo-5-(piperazin-1-yl)pyrazine (compound **6a**). **Example 17** (2 mg) was obtained as a yellow solid. MS: calc'd 514 ($MH^+$), measured 514 ($MH^+$). $^1H$ NMR (400 MHz, METHANOL-d4) $\delta$ 9.00 (dd, *J* = 1.6, 4.3 Hz, 1H), 8.69 (dd, *J* = 1.5, 8.6 Hz, 1H), 8.22 - 8.11 (m, 3H), 7.66 (dd, *J* = 4.2, 8.5 Hz, 1H), 7.28 (d, *J* = 7.9 Hz, 1H), 4.29 - 4.04 (m, 2H), 3.75 (t, J = 5.0 Hz, 4H), 3.54 - 3.36 (m, 4H), 3.05 (br d, *J* = 1.8 Hz, 6H), 2.80 - 2.40 (m, 8H), 1.36 - 1.19 (m, 3H).

**Example 18**

**5-[(*2R,6S*)-2-methyl-6-[[4-(6-piperazin-1-ylpyrimidin-4-yl)piperazin-1-yl]methyl]morpholin-4-yl]quinoline-8-carbonitrile**

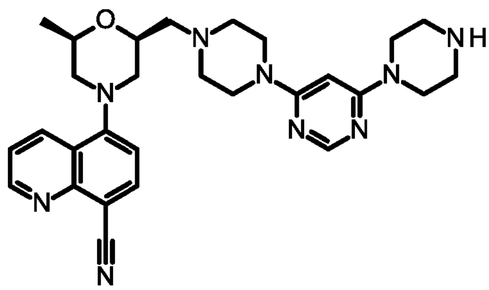

The title compound was prepared in analogy to the preparation of Example **6** by using 4-chloro-6-piperazin-1-yl-pyrimidine instead of 2-bromo-5-(piperazin-1-yl)pyrazine (compound **6a**). **Example 18** (5 mg) was obtained as a yellow solid. MS: calc'd 514 ($MH^+$), measured 514 ($MH^+$). $^1H$ NMR (400 MHz, METHANOL-d4) $\delta$ 9.02 (dd, *J*= 1.6, 4.3 Hz, 1H), 8.69 (dd, *J*= 1.6, 8.6 Hz, 1H), 8.30 - 8.15 (m, 2H), 7.68 (dd, *J* = 4.3, 8.6 Hz, 1H), 7.31 (d, *J* = 8.1 Hz, 1H), 6.18 (s, 1H), 4.61 - 4.46 (m, 1H), 4.27 - 4.15 (m, 1H), 3.99 - 3.87 (m, 5H), 3.64 - 3.37 (m, 10H), 3.31 - 3.20 (m, 5H), 2.90 - 2.66 (m, 2H), 1.35 (d, *J* = 6.2 Hz, 3H).

**Example 19**

**5-[(2*R,6S*)-2-methyl-6-[[4-[4-[(2*R*)-morpholin-2-yl]phenyl]piperazin-1-yl]methyl]morpholin-4-yl]quinoline-8-carbonitrile**

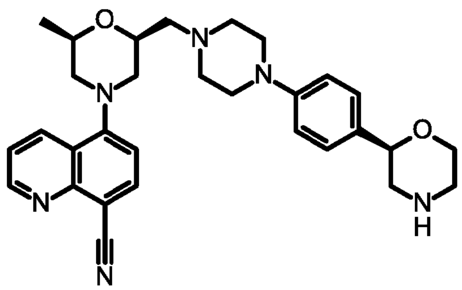

The title compound was prepared according to the following scheme:

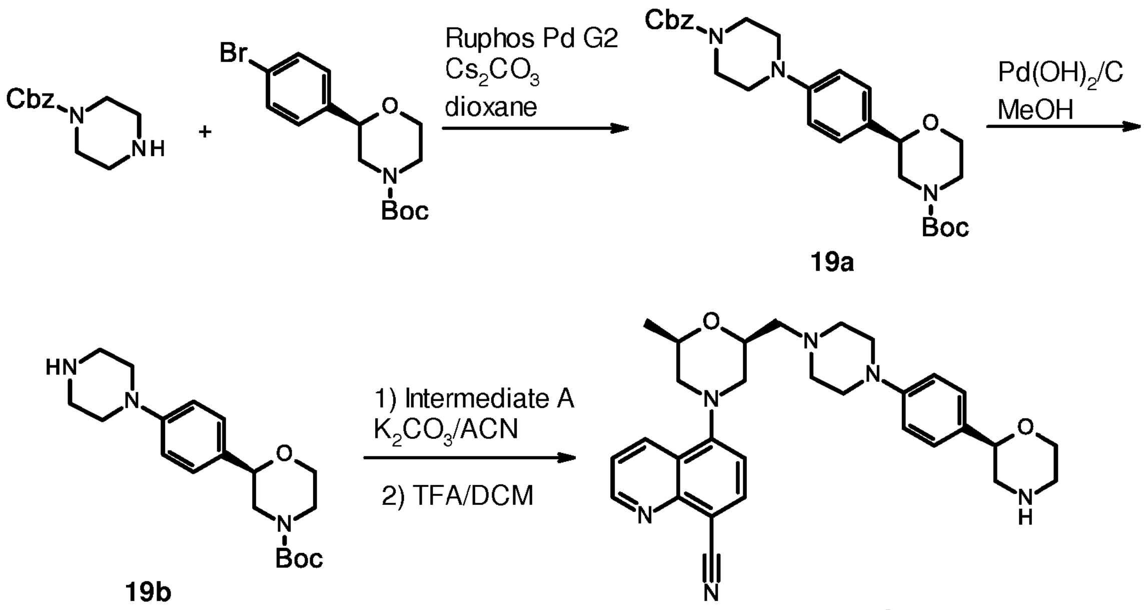


19a

19b

19

**Step 1: preparation of *tert*-butyl (2R)-2-[4-(4-benzyloxycarbonylpiperazin-1-yl)phenyl]morpholine-4-carboxylate (compound 19a)**

To a tube was added benzyl piperazine-1-carboxylate (77.2 mg, 351 µmol), *tert-butyl (R)*-2-(4-bromophenyl)morpholine-4-carboxylate (WO 2012168260, 100 mg, 292 µmol), $Cs_2CO_3$ (190 mg, 584 µmol) and dioxane (4 mL). The suspension was bubbled with $N_2$ for 5 mins and Ruphos Pd G2 (22.7 mg, 29.2 µmol) was added. The mixture was heated at 90 °C and stirred for 2 hrs. The mixture was cooled and filtered. The filtrate was concentrated to give an oil. The crude was purified via silica gel chromatography, the elution was concentrated to give compound **19a** (100 mg) as an oil. MS calc'd 482 ($MH^+$); measured 482 ($MH^+$).

**Step 2: preparation of *tert*-butyl (2R)-2-(4-piperazin-1-ylphenyl)morpholine-4-carboxylate (compound 19b)**

The oil (compound **19a**, 100 mg, 207μmol) was dissolved in MeOH (4 mL) followed by the addition of $Pd(OH)_2$ (20% on carbon with 50% $H_2O$) (10 mg, 71.2 μmol). After being stirred at rt for 2 hrs under $H_2$ atmosphere, the mixture was filtered, and the filtrate was concentrated to give compound **19b** (70 mg) as an oil. MS calc'd 348 ($MH^+$); measured 348 ($MH^+$).

**Step 3: preparation of 5-[(2*R*,6*S*)-2-methyl-6-[[4-[4-[(2*R*)-morpholin-2-yl]phenyl]piperazin-1-yl]methyl]morpholin-4-yl]quinoline-8-carbonitrile (compound 19)**

To a flask was added ((2*R*,6*R*)-4-(8-cyanoquinolin-5-yl)-6-methylmorpholin-2-yl)methyl trifluoromethanesulfonate (Intermediate **A**, 30 mg, 72.2 μmol), *tert*-butyl (2*R*)-2-(4-(piperazin-1-yl)phenyl)morpholine-4-carboxylate (compound **19b**, 32.6 mg, 93.9 μmol), potassium carbonate (29.9 mg, 217 μmol) and ACN (4 mL). The mixture was heated to 85°C for 2 hrs, then filtered through celite. The filtrate was concentrated to give a crude oil, which was dissolved in DCM (2 mL) followed by the addition of TFA (1.48 g, 1 mL, 13 mmol) was added. The mixture was stirred at rt for 1 hour. The mixture was concentrated directly to give an oil which was purified by prep-HPLC. **Example 19** (29.5 mg) was obtained as a light yellow powder. MS: calc'd 513 ($MH^+$), measured 513 ($MH^+$). $^1H$ NMR (400 MHz, METHANOL-$d_4$) $\delta$ = 9.02 (dd, *J*= 1.6, 4.3 Hz, 1H), 8.69 (dd, *J*= 1.6, 8.6 Hz, 1H), 8.20 (d, *J*= 8.1 Hz, 1H), 7.68 (dd, *J*= 4.2, 8.6 Hz, 1H), 7.38 (d, *J* = 8.8 Hz, 2H), 7.31 (d, *J* = 8.1 Hz, 1H), 7.10 (d, *J*= 8.8 Hz, 2H), 4.71 (dd, *J* = 2.3, 11.2 Hz, 1H), 4.57 - 4.49 (m, 1H), 4.27 - 4.17 (m, 2H), 3.99 (dt, *J* = 2.9, 12.5 Hz, 1H), 3.95 - 3.69 (m, 3H), 3.49 - 3.42 (m, 5H), 3.42 - 3.34 (m, 6H), 3.30 - 3.24 (m, 1H), 3.16 - 3.09 (m, 1H), 2.86 - 2.73 (m, 2H), 1.35 (d, *J* = 6.4 Hz, 3H).

**Example 20**

**5-[(2*R*,6*S*)-2-methyl-6-[[4-[4-[(2*S*)-morpholin-2-yl]phenyl]piperazin-1-yl]methyl]morpholin-4-yl]quinoline-8-carbonitrile**

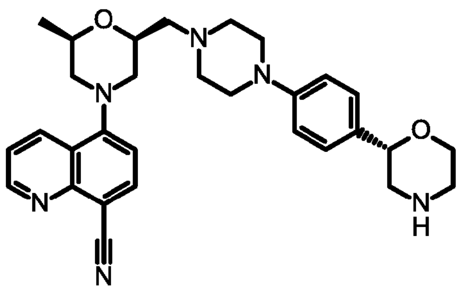

The title compound was prepared in analogy to the preparation of Example **19** by using *tert*-butyl (*S*)-2-(4-bromophenyl)morpholine-4-carboxylate) instead of *tert-butyl* (R)-2-(4-bromophenyl)morpholine-4-carboxylate. **Example 20** (36.7 mg) was obtained as a pale yellow powder. MS: calc'd 513 ($MH^+$), measured 513 ($MH^+$). $^1H$ NMR (400 MHz, METHANOL-d4) $\delta$ = 9.01 (dd, *J* = 1.6, 4.3 Hz, 1H), 8.68 (dd, *J* = 1.6, 8.6 Hz, 1H), 8.18 (d, *J* = 8.1 Hz, 1H), 7.67 (dd, *J* = 4.2, 8.6 Hz, 1H), 7.37 (d, *J* = 8.7 Hz, 2H), 7.30 (d, *J* = 8.1 Hz, 1H), 7.09 (d, *J* = 8.8 Hz, 2H), 4.71 (dd, *J* = 2.3, 11.2 Hz, 1H), 4.58 - 4.50 (m, 1H), 4.27 - 4.17 (m, 2H), 4.04 - 3.95 (m, 1H), 3.94 - 3.56 (m, 4H), 3.49 - 3.42 (m, 5H), 3.41 - 3.34 (m, 4H), 3.32 - 3.18 (m, 2H), 3.12 (dd, *J* = 11.4, 12.7 Hz, 1H), 2.86 - 2.72 (m, 2H), 1.34 (d, *J* = 6.2 Hz, 3H).

**Example 21**

**5-[(2*R*,6*S*)-2-methyl-6-[[4-[6-(4-methylpiperazin-1-yl)pyrimidin-4-yl]piperazin-1-yl]methyl]morpholin-4-yl]quinoline-8-carbonitrile**

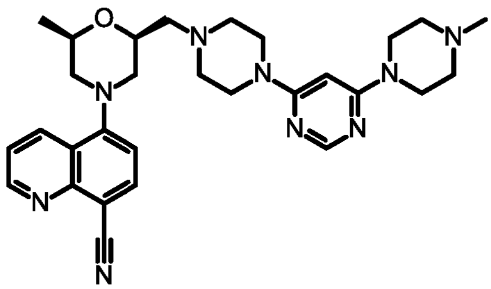

The title compound was prepared in analogy to the preparation of Example **6** by using 4-chloro-6-piperazin-1-yl-pyrimidine and 1-methylpiperazine instead of 2-bromo-5-(piperazin-1-yl)pyrazine (compound **6a**) and tert-butyl piperazine-1-carboxylate. **Example 21** (3 mg) was obtained as a yellow solid. MS: calc'd 528 ($MH^+$), measured 528 ($MH^+$). $^1$H NMR (400 MHz, METHANOL-d4) $\delta$ 8.99 (dd, *J*= 1.7, 4.2 Hz, 1H), 8.66 (dd, *J*= 1.7, 8.6 Hz, 1H), 8.26 (s, 1H), 8.16 (d, *J* = 7.9 Hz, 1H), 7.65 (dd, *J* = 4.3, 8.6 Hz, 1H), 7.28 (d, *J* = 8.1 Hz, 1H), 6.20 (s, 1H), 4.57 - 4.42 (m, 2H), 4.28 - 4.10 (m, 2H), 3.72 - 3.33 (m, 13H), 3.25 - 3.10 (m, 5H), 2.96 (s, 3H), 2.83 - 2.63 (m, 2H), 1.32 (d, *J* = 6.2 Hz, 3H).

**Example 22**

**5-[(*2S*,*6R*)-2-[[4-[2-(3-aminoazetidin-1-yl)pyrimidin-4-yl]piperazin-1-yl]methyl]-6-methyl-morpholin-4-yl]quinoline-8-carbonitrile**

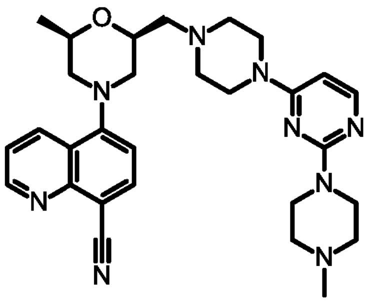

The title compound was prepared according to the following scheme:

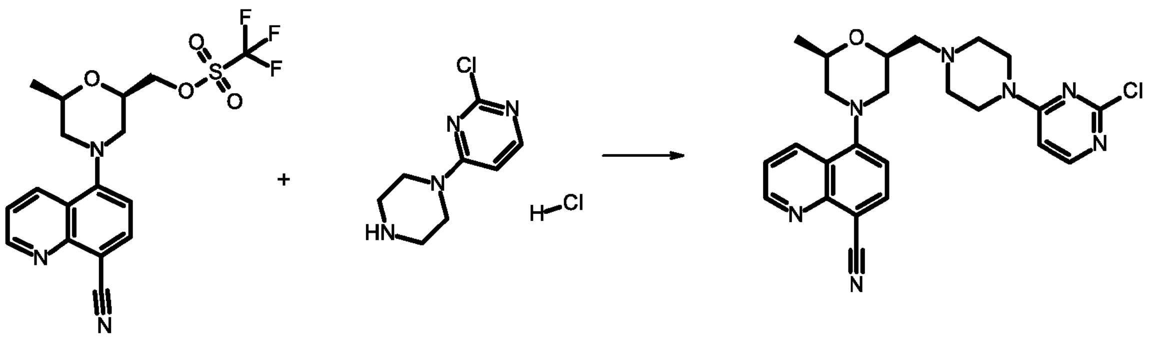

A 22a 22b

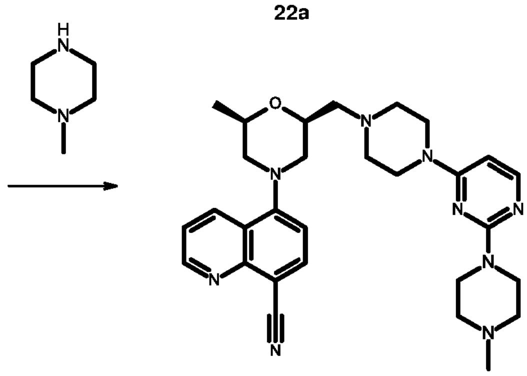

22

**Step 1: 5-((2*S*,6*R*)-2-((4-(2-chloropyrimidin-4-yl)piperazin-1-yl)methyl)-6-methylmorpholino)quinoline-8-carbonitrile (compound 22b)**

A suspension of 2-chloro-4-(piperazin-1-yl)pyrimidine hydrochloride (compound **22a**, 420 mg, 1.78 mmol) and $K_2CO_3$ (710 mg, 5.1 mmol) in ACN (8 mL) was heated to 50°C for 1 h. Then ((2*R*,6*R*)-4-(8-cyanoquinolin-5-yl)-6-methylmorpholin-2-yl)methyl trifluoromethanesulfonate (intermediate **A**, 706 mg, 1.7 mmol) was added. The reaction mixture was heat to 90 °C for 3 hrs. Then the mixture was filtered and the filtrate was concentrated and purified by silica gel chromatography to give compound **22b** (245 mg, 528 $\mu$mol) as yellow solid. MS: calc'd 464 ($MH^+$), measured 464 ($MH^+$).

**Step 2: 5-((2*R*,6*S*)-2-methyl-6-((4-(2-(4-methylpiperazin-1-yl)pyrimidin-4-yl)piperazin-1-yl)methyl)morpholino)quinoline-8-carbonitrile (Example 22)**

A suspension of 5-((2*S*,6*R*)-2-((4-(2-chloropyrimidin-4-yl)piperazin-1-yl)methyl)-6-methylmorpholino)quinoline-8-carbonitrile (compound **22b**, 46 mg, 99.1 $\mu$mol), 1-methylpiperazine (29 mg, 297 $\mu$mol) and $K_2CO_3$ (41 mg, 297 $\mu$mol) in ACN (2 mL) was heated at 110 °C under microwave for 2 hrs. Then the mixture was cooled to room temperature and filtered. The filtrate was concentrated and purified by prep-HPLC to afford **Example 22** (44 mg) as an orange solid. MS: calc'd 528 ($MH^+$), measured 528 ($MH^+$). $^1H$ NMR (METHANOL-$d_4$, 400 MHz) $\delta$ 9.01 (dd, 1H, *J* = 1.6, 4.3 Hz), 8.68 (dd, 1H, *J* = 1.6, 8.6 Hz), 8.18 (d, 1H, *J* = 7.9 Hz), 8.00 (d, 1H, *J* = 7.2 Hz), 7.67 (dd, 1H, *J* = 4.3, 8.6 Hz), 7.30 (d, 1H, *J* = 8.1 Hz), 6.67 (d, 1H, *J* = 7.2 Hz), 4.5-4.6 (m, 1H), 3.9-4.4 (m, 8H), 3.4-3.8 (m, 13H), 3.00 (s, 3H), 2.7-2.9 (m, 2H), 1.33 (d, 3H, *J* = 6.2 Hz).

**Example 23**

**5-[(2*S*,6*R*)-2-[[4-[2-(3-aminoazetidin-1-yl)pyrimidin-4-yl]piperazin-1-yl]methyl]-6-methyl-morpholin-4-yl]quinoline-8-carbonitrile**

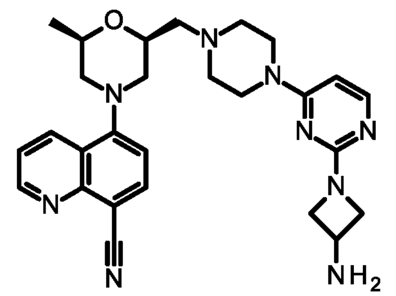

The title compound was prepared according to the following scheme:

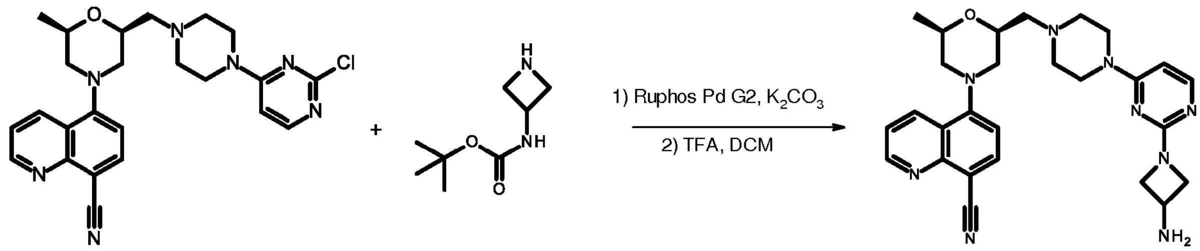


22b 23c 23

A suspension of 5-((2S,6R)-2-((4-(2-chloropyrimidin-4-yl)piperazin-1-yl)methyl)-6-methylmorpholino)quinoline-8-carbonitrile (compound **22b**, 22 mg, 47.4 μmol), *tert*-butyl azetidin-3-ylcarbamate (compound **23c**, 25 mg, 142 μmol), Ruphos Pd G2 (7 mg, 8.62 μmol) and $K_2CO_3$ (20 mg, 142 μmol) in 1,4-Dioxane (2 mL) was heated to 100 °C for 12 hrs under nitrogen atmosphere. The reaction mixture was cooled to room temperature and filtered. The filtrate was concentrated to give an intermediate (MS: calc'd 600 ($MH^+$), measured 600 ($MH^+$)), which was dissolved in 5 mL DCM followed by the addition of 0.5 mL TFA. The reaction mixture was stirred at rt for 2 hours, then concentrated to give a crude product which was purified by prep-HPLC to afford **Example 23** (4 mg) as light yellow solid. MS: calc'd 500 ($MH^+$), measured 500 ($MH^+$).$^1$H NMR (METHANOL-$d_4$, 400 MHz) δ 8.89 (dd, 1H, *J* = 1.6, 4.3 Hz), 8.56 (dd, 1H, *J* = 1.7, 8.6 Hz), 8.07 (d, 1H, *J* = 7.9 Hz), 7.78 (d, 1H, *J* = 7.1 Hz), 7.55 (dd, 1H, *J* = 4.3, 8.6 Hz), 7.18 (d, 1H, *J* = 8.1 Hz), 6.39 (d, 1H, *J* = 7.1 Hz), 4.43 (dd, 2H, *J* = 6.7, 9.8 Hz), 4.30 (br s, 1H), 4.0-4.2 (m, 4H), 3.90 (br s, 3H), 3.3-3.4 (m, 2H), 3.1-3.1 (m, 2H), 2.9-3.0 (m, 2H), 2.6-2.7 (m, 2H), 1.93 (s, 3H), 1.20 (d, 3H, *J* = 6.2 Hz).

**Example 24**

**5-[(2*S*,6*R*)-2-[[4-[2-[[(3*S*,4*R*)-4-fluoropyrrolidin-3-yl]amino]pyrimidin-4-yl]piperazin-1-yl]methyl]-6-methyl-morpholin-4-yl]quinoline-8-carbonitrile**

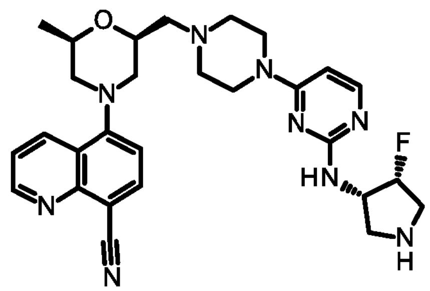

The title compound was prepared in analogy to the preparation of Example **23** by using *tert-butyl* (3S,4R)-3-amino-4-fluoropyrrolidine-1-carboxylate (Pharmablock, PB07374, CAS: 1174020-30-4) instead of *tert*-butyl azetidin-3-ylcarbamate (compound **23c**). **Example 24** (12 mg) was obtained as a light yellow solid. MS: calc'd 532 ($MH^+$), measured 532 ($MH^+$). $^1$H NMR (METHANOL-$d_4$, 400 MHz) δ 9.02 (dd, 1H, *J* = 1.7, 4.2 Hz), 8.68 (dd, 1H, *J* = 1.7, 8.6 Hz), 8.19 (d,

1H, *J* = 7.9 Hz), 7.92 (d, 1H, *J* = 7.5 Hz), 7.67 (dd, 1H, *J* = 4.3, 8.6 Hz), 7.31 (d, 1H, *J* = 8.1 Hz), 6.63 (d, 1H, *J* = 7.3 Hz), 5.3-5.5 (m, 1H), 4.9-5.2 (m, 2H), 4.48 (br s, 1H), 4.0-4.3 (m, 4H), 3.7-4.0 (m, 3H), 3.4-3.5 (m, 6H), 3.28 (br d, 3H, *J* = 6.4 Hz), 2.7-2.8 (m, 2H), 1.33 (d, 3H, *J* = 6.2 Hz).

**Example 25**

**5-[(2*S*,6*R*)-2-[[4-[2-(6-hydroxy-1,4-diazepan-1-yl)pyrimidin-4-yl]piperazin-1-yl]methyl]-6-methyl-morpholin-4-yl]quinoline-8-carbonitrile**

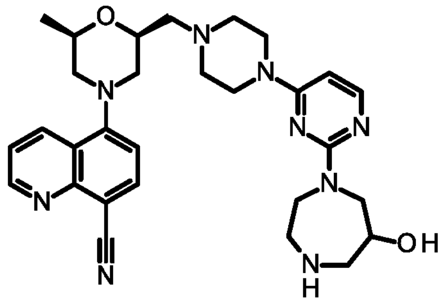

The title compound was prepared in analogy to the preparation of Example **23** by using *tert*-butyl 6-hydroxy-1,4-diazepane-1-carboxylate ( Wuxi Apptec, WX604354, CAS: 956317-40-1) instead of *tert*-butyl azetidin-3-ylcarbamate (compound **23c**). **Example 25** (13 mg) was obtained as a light yellow solid. MS: calc'd 544 ($MH^+$), measured 544 ($MH^+$).$^1$H NMR (METHANOL-$d_4$, 400 MHz) δ 9.02 (dd, 1H, *J* = 1.5, 4.2 Hz), 8.68 (dd, 1H, *J* = 1.6, 8.7 Hz), 8.19 (d, 1H, *J* = 7.9 Hz), 7.93 (d, 1H, *J* = 7.2 Hz), 7.67 (dd, 1H, *J* = 4.2, 8.6 Hz), 7.31 (d, 1H, *J* = 7.9 Hz), 6.67 (d, 1H, *J* = 7.5 Hz), 4.53 (br s, 1H), 4.4-4.5 (m, 1H), 3.9-4.3 (m, 8H), 3.78 (s, 1H), 3.47 (br s, 13H), 2.7-2.9 (m, 2H), 1.34 (d, 3H, *J* = 6.4 Hz).

**Example 26**

**5-[(*2S*,*6R*)-2-[[4-[2-*trans*-(3-amino-4-methoxy-pyrrolidin-1-yl)pyrimidin-4-yl]piperazin-1-yl]methyl]-6-methyl-morpholin-4-yl]quinoline-8-carbonitrile**

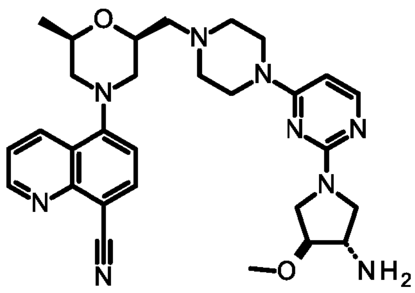

The title compound was prepared in analogy to the preparation of Example **23** by using *tert*-butyl *trans*-(4-methoxypyrrolidin-3-yl)carbamate instead of *tert*-butyl azetidin-3-ylcarbamate (compound **23c**). **Example 26** (4.4 mg) was obtained as a light yellow solid. MS: calc'd 544 ($MH^+$), measured 544 ($MH^+$).$^1$H NMR (METHANOL-$d_4$, 400 MHz) δ 8.88 (dd, 1H, *J* = 1.6, 4.2 Hz), 8.56 (d, 1H, *J* = 10.3 Hz), 8.05 (d, 1H, *J* = 8.1 Hz), 7.70 (d, 1H, *J* = 6.4 Hz), 7.54 (dd, 1H, *J* = 4.2, 8.7 Hz), 7.16 (d, 1H, *J* = 8.1 Hz), 5.97 (d, 1H, *J* = 6.4 Hz), 3.9-4.2 (m, 2H), 3.5-3.8 (m, 7H), 3.3-3.5 (m, 9H), 3.0-3.1 (m, 1H), 2.4-2.7 (m, 8H), 1.16 (d, 3H, *J* = 6.2 Hz).

**Example 27**

**5-[(2*S*,6*R*)-2-[[4-[2-(4-amino-1-piperidyl)pyrimidin-4-yl]piperazin-1-yl]methyl]-6-methyl-morpholin-4-yl]quinoline-8-carbonitrile**

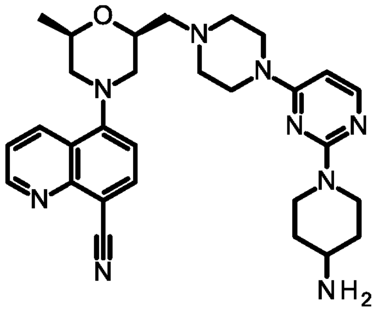

The title compound was prepared in analogy to the preparation of Example **23** by using *tert*-butyl piperidin-4-ylcarbamate instead of *tert*-butyl azetidin-3-ylcarbamate (compound **23c**). **Example 27** (15.5 mg) was obtained as a light yellow solid. MS: calc'd 528 ($MH^+$), measured 528 ($MH^+$).$^1$H NMR (METHANOL-d4, 400 MHz) $\delta$ 9.02 (dd, 1H, *J* = 1.7, 4.3 Hz), 8.68 (dd, 1H, *J* = 1.7, 8.6 Hz), 8.19 (d, 1H, *J* = 8.1 Hz), 7.90 (d, 1H, *J* = 7.5 Hz), 7.67 (dd, 1H, *J* = 4.3, 8.6 Hz), 7.31 (d, 1H, *J* = 8.1 Hz), 6.62 (d, 1H, *J* = 7.5 Hz), 4.5-4.6 (m, 3H), 4.0-4.3 (m, 4H), 3.4-3.6 (m, 8H), 3.4-3.4 (m, 2H), 3.1-3.2 (m, 2H), 2.7-2.9 (m, 2H), 2.1-2.2 (m, 2H), 1.6-1.8 (m, 2H), 1.34 (d, 3H, *J* = 6.4 Hz).

**Example 28**

**5-[(2*S*,6*R*)-2-[[4-[2-(azetidin-3-ylamino)pyrimidin-4-yl]piperazin-1-yl]methyl]-6-methyl-morpholin-4-yl]quinoline-8-carbonitrile**

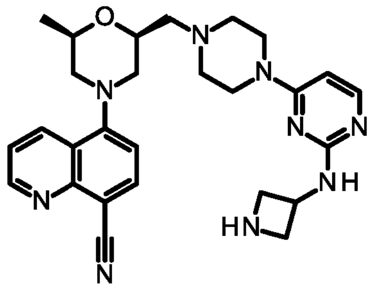

The title compound was prepared in analogy to the preparation of Example **23** by using *tert*-butyl 3-aminoazetidine-1-carboxylate instead of *tert*-butyl azetidin-3-ylcarbamate(compound **23c**). **Example 28** (9.0 mg) was obtained as light yellow solid. MS: calc'd 500 ($MH^+$), measured 500 ($MH^+$).$^1$H NMR (METHANOL-$d_4$, 400 MHz) $\delta$ 9.02 (dd, 1H, *J* = 1.5, 4.2 Hz), 8.68 (d, 1H, *J* = 8.7 Hz), 8.19 (d, 1H, *J* = 7.9 Hz), 7.92 (d, 1H, *J* = 7.3 Hz), 7.67 (dd, 1H, *J* = 4.3, 8.5 Hz), 7.31 (d, 1H, *J* = 8.1 Hz), 6.64 (d, 1H, *J* = 7.5 Hz), 4.6-4.7 (m, 1H), 4.4-4.6 (m, 2H), 4.3-4.4 (m, 2H), 4.2-4.2 (m, 2H), 3.5-3.6 (m, 3H), 3.4-3.5 (m, 2H), 3.4-3.4 (m, 1H), 2.7-2.9 (m, 2H), 2.05 (s, 6H), 1.33 (dd, 3H, *J* = 1.0, 6.2 Hz).

**Example 29**

**5-[(2S,6R)-2-[[4-[2-[(3R)-3-(hydroxymethyl)piperazin-1-yl]pyrimidin-4-yl]piperazin-1-yl]methyl]-6-methyl-morpholin-4-yl]quinoline-8-carbonitrile**

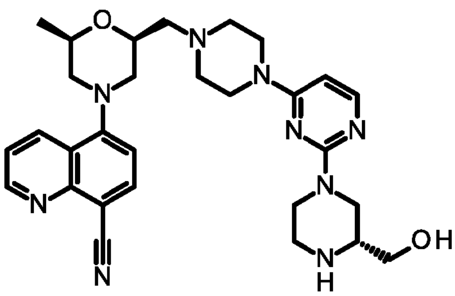

The title compound was prepared in analogy to the preparation of Example **23** by using *tert*-butyl (*R*)-2-(hydroxymethyl)piperazine-1-carboxylate (Bepharm, B32231, CAS: 169448-87-7) instead of *tert*-butyl azetidin-3-ylcarbamate (compound **23c**). **Example 29** (20.9 mg) was obtained as light yellow solid. MS: calc'd 544 ($MH^+$), measured 544 ($MH^+$). $^1H$ NMR (METHANOL-$d_4$, 400 MHz) δ 9.02 (dd, 1H, *J* = 1.7, 4.2 Hz), 8.68 (dd, 1H, *J* = 1.6, 8.7 Hz), 8.2-8.2 (m, 1H), 8.02 (d, 1H, *J* = 6.8 Hz), 7.67 (dd, 1H, *J* = 4.2, 8.6 Hz), 7.31 (d, 1H, *J* = 7.9 Hz), 6.51 (d, 1H, *J* = 6.7 Hz), 4.5-4.7 (m, 4H), 4.20 (br d, 2H, *J* = 10.3 Hz), 4.10 (br s, 3H), 3.7-3.9 (m, 3H), 3.4-3.6 (m, 12H), 2.7-2.9 (m, 2H), 1.34 (d, 3H, *J* = 6.2 Hz).

**Example 30**

**5-[(2S,6R)-2-[[4-[2-[2-(dimethylamino)ethoxy]pyrimidin-4-yl]piperazin-1-yl]methyl]-6-methyl-morpholin-4-yl]quinoline-8-carbonitrile**

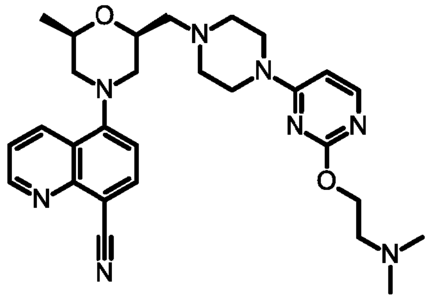

The title compound was prepared in analogy to the preparation of Example **23** by using 2-(dimethylamino)ethan-1-ol instead of *tert*-butyl azetidin-3-ylcarbamate (compound **23c**). **Example 30** (17.8 mg) was obtained as light yellow solid. MS: calc'd 517 ($MH^+$), measured 517 ($MH^+$). $^1H$ NMR (METHANOL-$d_4$, 400 MHz) δ 8.9-8.9 (m, 1H), 8.82 (d, 1H, *J* = 8.7 Hz), 8.0-8.0 (m, 2H), 7.4-7.6 (m, 2H), 7.3-7.4 (m, 2H), 6.97 (d, 1H, *J* = 9.2 Hz), 6.88 (d, 1H, *J* = 8.6 Hz), 4.05 (dd, 1H, *J* = 7.0, 9.5 Hz), 3.8-3.9 (m, 3H), 3.7-3.8 (m, 4H), 3.4-3.6 (m, 7H), 3.1-3.3 (m, 5H), 2.4-2.6 (m, 1H), 2.2-2.3 (m, 1H), 1.3-1.3 (m, 3H).

**Example 31**

**5-[(2*R*,6*S*)-2-methyl-6-[[4-[2-[4-[(2*S*)-pyrrolidine-2-carbonyl]piperazin-1-yl]pyrimidin-4-yl]piperazin-1-yl]methyl]morpholin-4-yl]quinoline-8-carbonitrile**

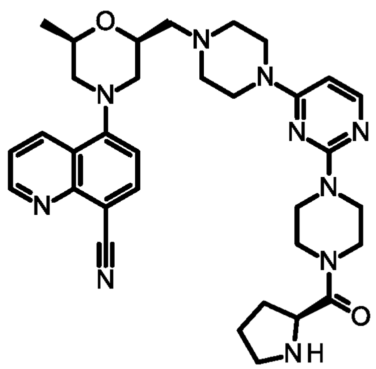

The title compound was prepared according to the following scheme:

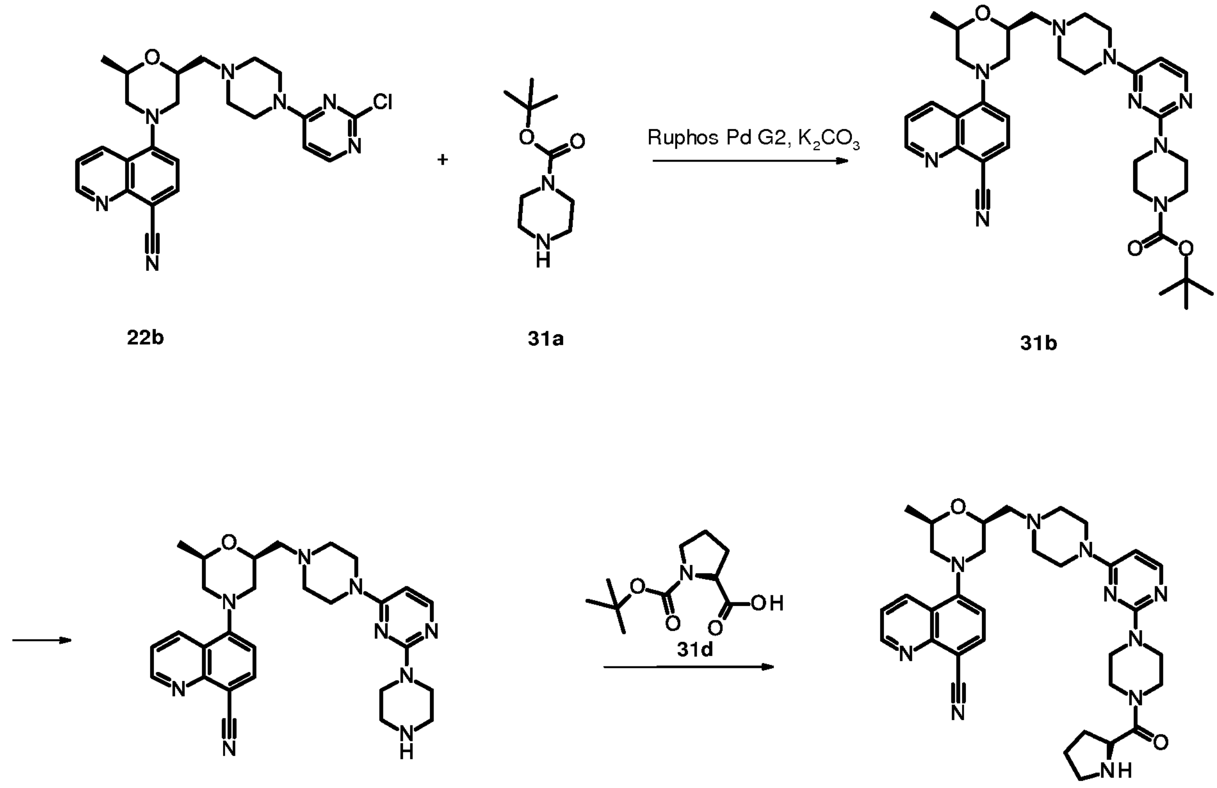


22b 31a 31b

31c 31

**Step 1: tert-butyl 4-(4-(4-(((2S,6R)-4-(8-cyanoquinolin-5-yl)-6-methylmorpholin-2-yl)methyl)piperazin-1-yl)pyrimidin-2-yl)piperazine-1-carboxylate (compound 31b)**

A suspension of 5-((2*S*,6*R*)-2-((4-(2-chloropyrimidin-4-yl)piperazin-1-yl)methyl)-6-methylmorpholino)quinoline-8-carbonitrile (compound **22b**, 92 mg, 198 μmol), *tert*-butyl piperazine-1-carboxylate (compound **31a**, 44 mg, 238 μmol) in ACN (2 mL) was heated at 110°C under microwave for 2 hrs. The reaction mixture was cooled to room temperature and filtered. The filtrate was concentrated to afford Compound **31b** (120 mg) as a yellow solid. MS: calc'd 614 ($MH^+$), measured 614 ($MH^+$).

**Step 2: 5-((2*R*,6*S*)-2-methyl-6-((4-(2-(piperazin-1-yl)pyrimidin-4-yl)piperazin-1-yl)methyl)morpholino)quinoline-8-carbonitrile (compound 31c)**

A solution of *tert*-butyl 4-(4-(4-(((2S,6R)-4-(8-cyanoquinolin-5-yl)-6-methylmorpholin-2-yl)methyl)piperazin-1-yl)pyrimidin-2-yl)piperazine-1-carboxylate (compound **31b**, 120 mg, 196 μmol) in hexafluoroisopropanol (2 mL) was

heated at 110°C under microwave for 2 hrs. The reaction mixture was cooled to room temperature and filtered. The filtrate was concentrated to afford compound **31c** (90 mg) as a yellow solid. MS: calc'd 514 ($MH^+$), measured 514 ($MH^+$).

**Step 3: 5-[(2*R*,6*S*)-2-methyl-6-[[4-[2-[4-[(2*S*)-pyrrolidine-2-carbonyl]piperazin-1-yl]pyrimidin-4-yl]piperazin-1-yl]methyl]morpholin-4-yl]quinoline-8-carbonitrile (Example 31)**

A solution of 5-((2*R*,6*S*)-2-methyl-6-((4-(2-(piperazin-1-yl)pyrimidin-4-yl)piperazin-1-yl)methyl)morpholino)quinoline-8-carbonitrile (compound **31c**, 30 mg, 58.4 μmol), *(tert*-butoxycarbonyl)-D-proline (compound **31d**,14 mg, 64.2 μmol) and $Et_3N$ (18 mg, 24.4 μl, 175 μmol) in N,N-Dimethylformamide (1 mL) was stirred at rt for 2 hrs. The reaction mixture was diluted with EA (10 mL) and washed with water. The organic layer was concentrated and purified by silica gel chromatography to afford *tert*-butyl (*R*)-2-(4-(4-(4-(((2*S*,6*R*)-4-(8-cyanoquinolin-5-yl)-6-methylmorphofin-2-yl)methyl)piperazin-1-yl)pyrimidin-2-yl)piperazine-1-carbonyl)pyrrolidine-1-carboxylate (20 mg) as colorless oil. MS: calc'd 711 ($MH^+$), measured 711 ($MH^+$).

To a solution of tert-butyl (*R*)-2-(4-(4-(4-(((2*S*,6*R*)-4-(8-cyanoquinolin-5-yl)-6-methylmorpholin-2-yl)methyl)piperazin-1 -yl)pyrimidin-2-yl)piperazine-1 -carbonyl)pyrrolidine-1-carboxylate (20 mg, 28.1 μmol) in DCM (2 mL) was added TFA (9.62 mg, 84.4 μmol) at 0 °C. The reaction mixture was stirred at rt for 2 hrs. Then concentrated and purified by prep-HPLC(TFA/ACN) to afford **Example 31** (11.7 mg) as a light yellow solid. MS: calc'd 611 ($MH^+$), measured 611 ($MH^+$).$^1$H NMR (METHANOL-$d_4$, 400 MHz) δ 9.02 (dd, 1H, *J* = 1.7, 4.3 Hz), 8.68 (dd, 1H, *J* = 1.6, 8.7 Hz), 8.19 (d, 1H, *J* = 7.9 Hz), 7.94 (d, 1H, *J* = 7.2 Hz), 7.67 (dd, 1H, *J* = 4.3, 8.6 Hz), 7.31 (d, 1H, *J* = 8.1 Hz), 6.62 (d, 1H, *J* = 7.3 Hz), 4.7-4.8 (m, 1H), 4.52 (br s, 1H), 4.20 (br d, 5H, *J* = 6.2 Hz), 3.7-4.0 (m, 9H), 3.4-3.6 (m, 9H), 2.7-2.9 (m, 2H), 2.5-2.6 (m, 1H), 2.0-2.2 (m, 3H), 1.34 (d, 3H, *J* = 6.2 Hz).

**Example 32**

**5-[(2*S*,6*R*)-2-[[4-[2-[4-[2-(dimethylamino)acetyl]piperazin-1-yl]pyrimidin-4-yl]piperazin-1-yl]methyl]-6-methylmorpholin-4-yl]quinoline-8-carbonitrile**

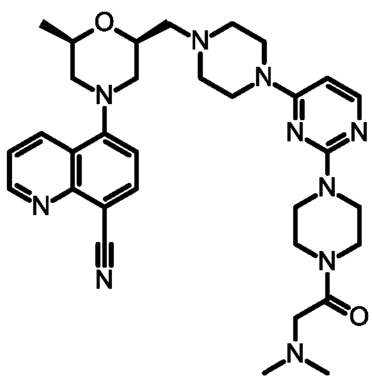

The title compound was prepared in analogy to the preparation of Example **31** by using dimethylglycine instead of (*tert*-butoxycarbonyl)-D-proline (compound **31d). Example 32** (8.8 mg) was obtained as a light yellow solid. MS: calc'd 599 ($MH^+$), measured 599 ($MH^+$).$^1$H NMR (METHANOL-$d_4$, 400 MHz) δ 9.02 (dd, 1H, *J* = 1.6, 4.3 Hz), 8.68 (dd, 1H, *J* = 1.7, 8.6 Hz), 8.19 (d, 1H, *J* = 8.1 Hz), 7.93 (d, 1H, *J* = 7.2 Hz), 7.67 (dd, 1H, *J* = 4.3, 8.6 Hz), 7.30 (d, 1H, *J* = 8.1 Hz), 6.58 (d, 1H, *J* = 7.2 Hz), 4.50 (br s, 1H), 4.34 (s, 2H), 4.0-4.3 (m, 5H), 3.8-3.9 (m, 6H), 3.6-3.7 (m, 2H), 3.4-3.6 (m, 7H), 2.99 (s, 6H), 2.7-2.8 (m, 2H), 1.33 (d, 3H, *J* = 6.2 Hz).

**Example 33**

**5-[(2*R*,6*S*)-2-methyl-6-[[4-[6-(4-methylpiperazin-1-yl)-2-pyridyl]piperazin-1-yl]methyl]morpholin-4-yl]quinoline-8-carbonitrile**

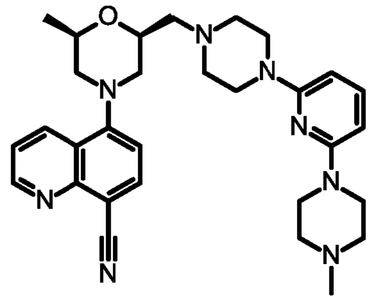

The title compound was prepared according to the following scheme:

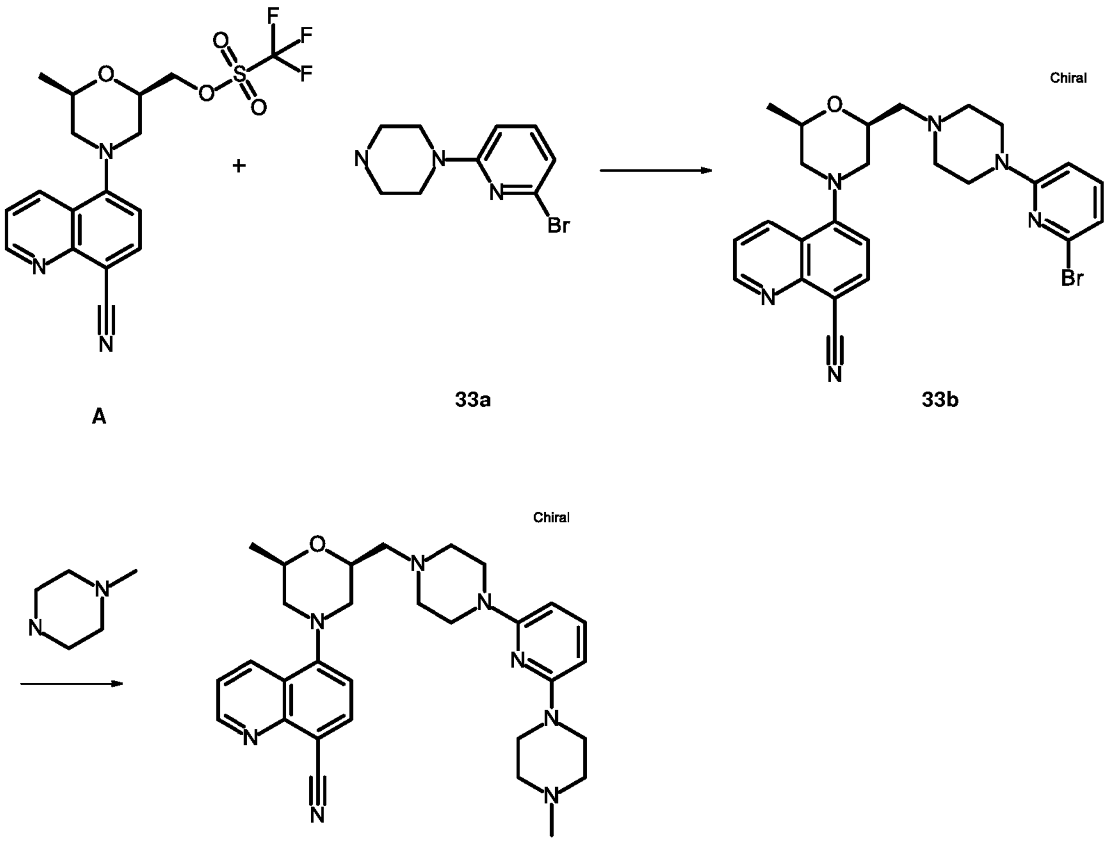


A

33a

33b

33

**Step 1 : 5-((2S,6R)-2-((4-(6-bromopyridin-2-yl)piperazin-1-yl)methyl)-6-methylmorpholino)quinoline-8-carbonitrile(compound 33b)**

A suspension of ((2*R*,6*R*)-4-(8-cyanoquinolin-5-yl)-6-methylmorpholin-2-yl)methyl trifluoromethanesulfonate (intermediate **A**, 831 mg, 2 mmol) and 1-(6-bromopyridin-2-yl)piperazine (compound **33a**, 489 mg, 2.02 mmol) and $K_2CO_3$ (553 mg, 4 mmol) in ACN (8 mL) was heat at 90°C for 3 hrs. Then the mixture was filtered and the filtrate was concentrated and purified by silica gel chromatography to afford Compound **33b** (652.6 mg) as a yellow solid. MS: calc'd 508 ($MH^+$), measured 508 ($MH^+$).

**Step 2 : 5-[(2R,6S)-2-methyl-6-[[4-[6-(4-methylpiperazin-1-yl)-2-pyridyl]piperazin-1-yl]methyl]morpholin-4-yl]quinoline-8-carbonitrile (Example 33)**

A suspension of 5-((2S,6R)-2-((4-(6-bromopyridin-2-yl)piperazin-1-yl)methyl)-6-methylmorpholino)quinoline-8-carbonitrile (compound **33b**, 40 mg, 78.8 μmol), 1-methylpiperazine (10 mg, 94.6 μmol), XPhos Palladacycle Gen. 2 (7 mg, 7.88 μmol) and cesium carbonate (52 mg, 158 μmol) in 1,4-Dioxane (2 mL) was heated at 100°C for 12 hrs under

nitrogen atmosphere. Then the mixture was filtered. The filtrate was concentrated and purified by prep-HPLC to afford **Example 33** (5.8 mg) as a light yellow solid. MS: calc'd 527 ($MH^+$), measured 527 ($MH^+$). $^1H$ NMR (METHANOL-$d_4$, 400 MHz) δ 9.01 (dd, 1H, *J* = 1.6, 4.3 Hz), 8.68 (dd, 1H, *J* = 1.7, 8.6 Hz), 8.18 (d, 1H, *J* = 7.9 Hz), 7.67 (dd, 1H, *J* = 4.3, 8.7 Hz), 7.53 (t, 1H, *J* = 8.1 Hz), 7.30 (d, 1H, *J* = 8.1 Hz), 6.35 (dd, 2H, *J* = 3.6, 8.1 Hz), 4.48 (br d, 4H, *J* = 9.7 Hz), 4.0-4.3 (m, 2H), 3.7-3.9 (m, 2H), 3.5-3.7 (m, 3H), 3.4-3.5 (m, 6H), 3.0-3.3 (m, 5H), 2.97 (s, 3H), 2.7-2.9 (m, 2H), 1.34 (d, 3H, *J* = 6.2 Hz).

**Example 34**

**5-[(2*S*,6*R*)-2-[[4-[2-[4-(azetidine-2-carbonyl)piperazin-1-yl]pyrimidin-4-yl]piperazin-1-yl]methyl]-6-methyl-morpholin-4-yl]quinoline-8-carbonitrile**

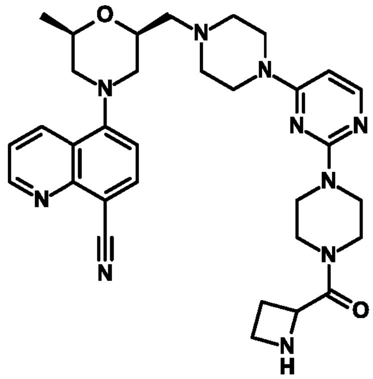

The title compound was prepared in analogy to the preparation of Example **31** by using azetidine-2-carboxylic acid instead of (*tert*-butoxycarbonyl)-D-proline (compound **31d**). **Example 34** (8.8 mg) was obtained as a light yellow solid. MS: calc'd 597 ($MH^+$), measured 597 ($MH^+$). $^1H$ NMR (METHANOL-$d_4$, 400 MHz) δ 8.90 (dd, 1H, *J* = 1.6, 4.3 Hz), 8.56 (dd, 1H, *J* = 1.7, 8.6 Hz), 8.07 (d, 1H, *J* = 7.9 Hz), 7.81 (d, 1H, *J* = 7.2 Hz), 7.55 (dd, 1H, *J* = 4.2, 8.6 Hz), 7.19 (d, 1H, *J* = 8.1 Hz), 6.47 (d, 1H, *J* = 7.2 Hz), 5.36 (t, 1H, *J* = 8.8 Hz), 4.39 (br s, 2H), 4.0-4.1 (m, 6H), 3.6-3.9 (m, 7H), 3.3-3.5 (m, 9H), 2.8-2.9 (m, 1H), 2.6-2.7 (m, 2H), 2.5-2.6 (m, 1H), 1.21 (d, 3H, *J* = 6.2 Hz).

**Example 35**

**5-[(2*S*,6*R*)-2-[[4-[6-[[(3*S*,4*S*)-4-fluoropyrrolidin-3-yl]amino]-2-pyridyl]piperazin-1-yl]methyl]-6-methyl-morpholin-4-yl]quinoline-8-carbonitrile**

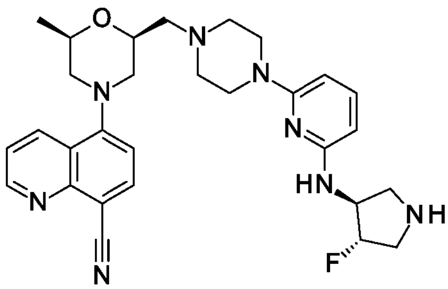

The title compound was prepared according to the following scheme:

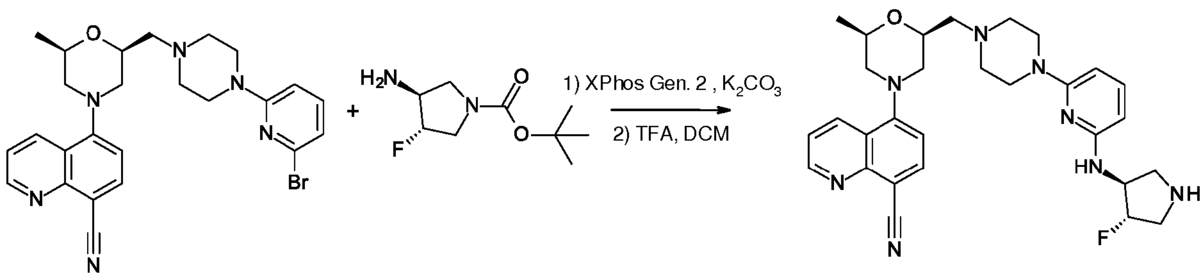


33b 35a 35

A suspension of 5-((2*S*,6*R*)-2-((4-(6-bromopyridin-2-yl)piperazin-1-yl)methyl)-6-methylmorpholino)quinoline-8-carbonitrile (compound **33b**, 40 mg, 78.8 μmol), *tert*-butyl (3S,4S)-3-amino-4-fluoropyrrolidine-1-carboxylate (compound **35a**, 18 mg, 86.7 μmol), cesium carbonate (52 mg, 158 μmol) and XPhos Palladacycle Gen. 2 (6.2 mg, 7.88 μmol) in 1,4-dioxane (2 mL) was heated at 100 °C for 12 hrs under nitrogen atmosphere. The reaction mixture was cooled to room temperature and filtered. The filtrate was concentrated to get crude intermediate. MS: calc'd 631(MH$^+$), measured 631(MH$^+$).

The intermediate was dissolved in 5 mL DCM. Then 0.5 mL TFA was added to the solution. After the mixture was stirred at rt for 3 hrs. The mixture was concentrated *in vacuo* to give a yellow oil which was purified by prep-HPLC. **Example 35** (5.4 mg) was obtained as a yellow oil. MS: calc'd 531 (MH$^+$), measured 531 (MH$^+$). $^1$H NMR (METHANOL-*d*$_4$, 400 MHz) δ 9.0-9.0 (m, 1H), 8.68 (dd, 1H, *J* = 1.7, 8.6 Hz), 8.17 (d, 1H, *J* = 7.9 Hz), 7.66 (dd, 1H, *J* = 4.3, 8.6 Hz), 7.4-7.5 (m, 1H), 7.29 (d, 1H, *J* = 8.1 Hz), 6.24 (d, 1H, *J* = 8.1 Hz), 6.08 (d, 1H, *J* = 7.9 Hz), 5.3-5.5 (m, 1H), 4.69 (br dd, 1H, *J* = 4.1, 13.5 Hz), 4.53 (br dd, 1H, *J* = 2.8, 7.4 Hz), 4.44 (br s, 1H), 4.1-4.3 (m, 1H), 3.43 (br s, 13H), 3.1-3.3 (m, 2H), 2.7-2.9 (m, 2H), 1.34 (d, 3H, *J* = 6.4 Hz).

**Example 36**

**5-[(2*S*,6*R*)-2-[[4-[6-(4-amino-1-piperidyl)-2-pyridyl]piperazin-1-yl]methyl]-6-methyl-morpholin-4-yl]quinoline-8-carbonitrile**

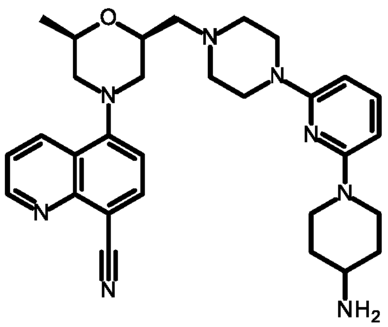

The title compound was prepared in analogy to the preparation of **Example 35** by using Boc-4-aminopiperidine instead of *tert*-butyl (3*S*,4*S*)-3-amino-4-fluoropyrrolidine-1-carboxylate (compound **35a**). **Example 36** (3.3 mg) was obtained as a light yellow solid. MS: calc'd 527 (MH$^+$), measured 527 (MH$^+$). $^1$H NMR (METHANOL-d$_4$, 400 MHz) δ 9.01 (dd, 1H, *J* = 1.6, 4.3 Hz), 8.68 (dd, 1H, *J* = 1.6, 8.6 Hz), 8.18 (d, 1H, *J* = 7.9 Hz), 7.67 (dd, 1H, *J* = 4.2, 8.6 Hz), 7.4-7.5 (m, 1H), 7.30 (d, 1H, *J* = 8.1 Hz), 6.30 (d, 1H, *J* = 8.2 Hz), 6.24 (d, 1H, *J* = 8.1 Hz), 4.3-4.6 (m, 4H), 4.2-4.3 (m, 1H), 3.4-4.0 (m, 10H), 3.1-3.3 (m, 2H), 2.9-3.0 (m, 2H), 2.7-2.9 (m, 2H), 2.0-2.1 (m, 2H), 1.62 (dq, 2H, *J* = 4.2, 12.2 Hz), 1.34 (d, 3H, *J* = 6.2 Hz).

**Example 37**

**5-[(*2S,6R*)-2-[[4-[6-[[(*3S,4R*)-4-fluoropyrrolidin-3-yl]amino]-2-pyridyl]piperazin-1-yl]methyl]-6-methyl-morpholin-4-yl]quinoline-8-carbonitrile**

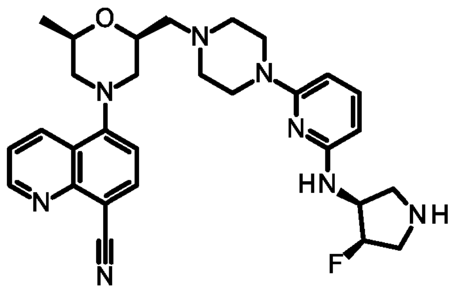

The title compound was prepared in analogy to the preparation of **Example 35** by using *tert-butyl* (3*S*,4*R*)-3-amino-4-fluoropyrrolidine-1-carboxylate (CAS: 1174020-30-4) instead of *tert-butyl* (3*S*,4*S*)-3-amino-4-fluoropyrrolidine-1-carboxylate (compound **35a**). **Example 37** (11.3 mg) was obtained as a light yellow solid. MS: calc'd 531 ($MH^+$), measured 531 ($MH^+$).$^1$H NMR (METHANOL-$d_4$, 400 MHz) δ 9.00 (br d, 1H, *J* = 2.8 Hz), 8.67 (br d, 1H, *J* = 8.6 Hz), 8.17 (dd, 1H, *J* = 3.7, 8.0 Hz), 7.66 (ddd, 1H, *J* = 2.1, 4.2, 8.6 Hz), 7.38 (t, 1H, *J* = 4.0 Hz), 7.29 (dd, 1H, *J* = 3.3, 8.1 Hz), 6.19 (d, 1H, *J* = 8.1 Hz), 6.12 (d, 1H, *J* = 8.1 Hz), 5.41 (t, 1H, *J* = *3.1* Hz), 5.28 (br s, 1H), 4.9-5.0 (m, 2H), 4.8-4.9 (m, 1H), 4.5-4.6 (m, 1H), 4.39 (br s, 1H), 4.2-4.3 (m, 1H), 3.7-3.9 (m, 4H), 3.3-3.5 (m, 6H), 3.19 (t, 2H, *J* = 11.2 Hz), 2.7-2.9 (m, 2H), 1.34 (d, 3H, *J* = 6.2 Hz).

**Example 38**

**5-[(2*S*,6*R*)-2-[[4-[6-(4-amino-1-piperidyl)-2-pyridyl]piperazin-1-yl]methyl]-6-methyl-morpholin-4-yl]quinoline-8-carbonitrile**

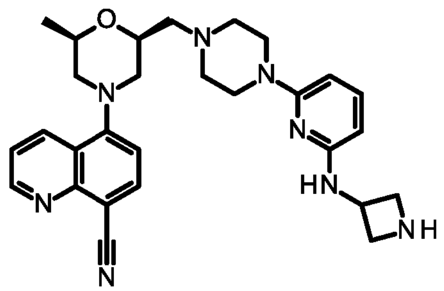

The title compound was prepared in analogy to the preparation of **Example 35** by using 1-BOC-3-(amino)azetidine instead of *tert*-butyl (3*S*,4*S*)-3-amino-4-fluoropyrrolidine-1-carboxylate (compound **35a**). **Example 38** (8.7 mg) was obtained as a light yellow solid. MS: calc'd 499 ($MH^+$), measured 499 ($MH^+$). $^1$H NMR (METHANOL-$d_4$, 400 MHz) δ 9.01 (dd, 1H, *J* = 1.7, 4.2 Hz), 8.68 (dd, 1H, *J* = 1.6, 8.6 Hz), 8.18 (d, 1H, *J* = 7.9 Hz), 7.67 (dd, 1H, *J* = 4.2, 8.6 Hz), 7.47 (t, 1H, *J* = 4.0 Hz), 7.30 (d, 1H, *J* = 8.1 Hz), 6.28 (d, 1H, *J* = 8.2 Hz), 5.93 (d, 1H, *J* = 7.9 Hz), 4.4-4.7 (m, 3H), 4.1-4.4 (m, 5H), 3.99 (dd, 2H, *J* = 4.3, 9.4 Hz), 3.4-3.9 (m, 9H), 2.7-2.9 (m, 2H), 1.34 (d, 3H, *J* = 6.2 Hz).

**Example 39**

**5-[(*2S,6R*)-2-[[4-[6-[[(*3R,4R*)-4-methoxypyrrolidin-3-yl]amino]-2-pyridyl]piperazin-1-yl]methyl]-6-methyl-morpholin-4-yl]quinoline-8-carbonitrile**

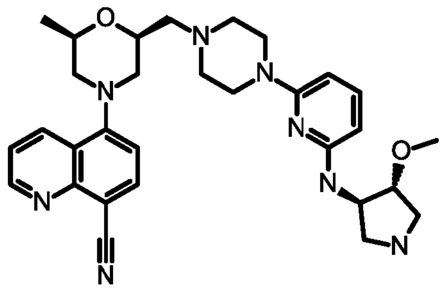

The title compound was prepared in analogy to the preparation of **Example 35** by using *tert-butyl* (3*R*,4*R*)-3-amino-4-methoxypyrrolidine-1-carboxylate (PharmaBlock, PBXA3109, CAS: 1400562-12-0) instead of tert-butyl (3*S*,4*S*)-3-amino-4-fluoropyrrolidine-1-carboxylate (compound **35a**). **Example 39** (16.2 mg) was obtained as a light yellow solid. MS: calc'd 543 ($MH^+$), measured 543 ($MH^+$).$^1$H NMR (METHANOL-$d_4$, 400 MHz) δ 8.88 (dd, 1H, *J* = 1.7, 4.2 Hz), 8.55 (dd, 1H, *J* = 1.7, 8.6 Hz), 8.05 (d, 1H, *J* = 7.9 Hz), 7.54 (dd, 1H, *J* = 4.2, 8.6 Hz), 7.3-7.3 (m, 1H), 7.17 (d, 1H, *J* = 8.1 Hz), 6.1-6.1 (m, 1H), 5.95 (d, 1H, *J* = 8.1 Hz), 4.4-4.5 (m, 2H), 4.2-4.3 (m, 1H), 3.9-4.2 (m, 3H), 3.5-3.8 (m, 4H), 3.2-3.5 (m, 13H), 2.6-2.7 (m, 2H), 1.22 (d, 3H, *J* = 6.2 Hz).

**Example 40**

**5-[(2*S*,6*R*)-2-[[4-[6-(3-amino-4-fluoro-pyrrolidin-1-yl)-2-pyridyl]piperazin-1-yl] methyl]-6-methyl-morpholin-4-yl]quinoline-8-carbonitrile**

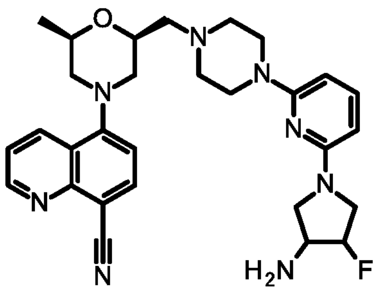

The title compound was prepared in analogy to the preparation of **Example 35** by using tert-butyl *N*-(4-fluoro-pyrrolidin-3-yl)carbamate instead of *tert*-butyl (3*S*,4*S*)-3-amino-4-fluoropyrrolidine-1-carboxylate (compound **35a**). **Example 40** (14.8 mg) was obtained as a light yellow solid. MS: calc'd 531 ($MH^+$), measured 531 ($MH^+$). $^1$H NMR (METHANOL-$d_4$, 400 MHz) δ 8.90 (dd, 1H, *J* = 1.7, 4.2 Hz), 8.57 (dd, 1H, *J* = 1.6, 8.6 Hz), 8.07 (d, 1H, *J* = 7.9 Hz), 7.55 (dd, 1H, *J* = 4.2, 8.6 Hz), 7.3-7.4 (m, 1H), 7.19 (d, 1H, *J* = 7.9 Hz), 6.15 (d, 1H, *J* = 8.1 Hz), 5.91 (d, 1H, *J* = 7.9 Hz), 5.2-5.4 (m, 1H), 4.2-4.5 (m, 3H), 4.0-4.2 (m, 3H), 3.7-3.9 (m, 3H), 3.5-3.7 (m, 4H), 3.2-3.4 (m, 6H), 2.66 (ddd, 2H, *J* = 10.3, 12.0, 18.8 Hz), 1.22 (d, 3H, *J* = 6.2 Hz).

**Example 41**

**5-[(2*S*,6*R*)-2-[[4-[6-(6-hydroxy-1,4-diazepan-1-yl)-2-pyridyl]piperazin-1-yl]methyl]-6-methyl-morpholin-4-yl]quinoline-8-carbonitrile**

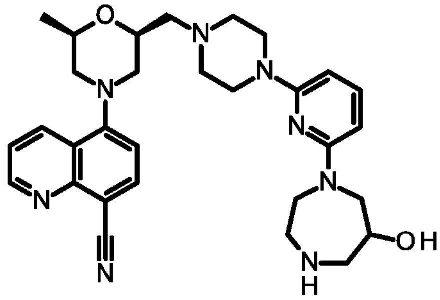

The title compound was prepared in analogy to the preparation of **Example 35** by using *tert*-butyl 6-hydroxy-1,4-diazepane-1-carboxylate (Wuxi Apptec, WX604354, CAS: 956317-40-1) instead of *tert*-butyl (3S,4S)-3-amino-4-fluoropyrrolidine-1-carboxylate (compound **35a**). **Example 41** (0.9 mg) was obtained as a light yellow solid. MS: calc'd 543 ($MH^+$), measured 543 ($MH^+$). $^1H$ NMR (METHANOL-$d_4$, 400 MHz) $\delta$ 8.9-9.1 (m, 1H), 8.68 (dd, 1H, *J* = 1.5, 8.7 Hz), 8.18 (d, 1H, *J* = 7.9 Hz), 7.67 (dd, 1H, *J* = 4.2, 8.6 Hz), 7.4-7.5 (m, 1H), 7.30 (d, 1H, *J* = 7.9 Hz), 6.26 (d, 1H, *J* = 8.3 Hz), 6.2-6.2 (m, 1H), 6.22 (s, 1H), 4.5-4.6 (m, 1H), 4.40 (q, 2H, *J* = 4.2 Hz), 4.1-4.3 (m, 2H), 3.8-4.1 (m, 4H), 3.74 (br dd, 3H, *J* = 4.2, 14.9 Hz), 3.5-3.6 (m, 2H), 3.4-3.5 (m, 5H), 3.1-3.3 (m, 3H), 2.7-2.9 (m, 2H), 1.34 (d, 3H, *J* = 6.2 Hz).

**Example 42**

**5-[(2S, 6R)-2-[[4-[6-[(3R,4S)-3-amino-4-methoxy-pyrrolidin-1-yl]-2-pyridyl]piperazin-1-yl]methyl]-6-methyl-morpholin-4-yl]quinoline-8-carbonitrile**

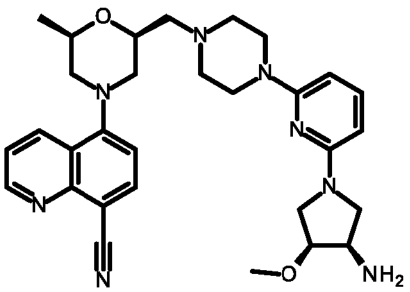

The title compound was prepared in analogy to the preparation of **Example 35** by using *tert*-butyl *N*-((3*R*,4*S*)-4-methoxypyrrolidin-3-yl)carbamate (PharmaBlock, PBZ4729, CAS: 1932508-77-4) instead of *tert-butyl* (3*S*,4*S*)-3-amino-4-fluoropyrrolidine-1-carboxylate (compound **35a**). **Example 42** (20.6 mg) was obtained as a light yellow solid. MS: calc'd 543 ($MH^+$), measured 543 ($MH^+$). $^1H$ NMR (METHANOL-$d_4$, 400 MHz) $\delta$ 9.01 (dd, 1H, *J* = 1.6, 4.3 Hz), 8.68 (dd, 1H, *J* = 1.7, 8.6 Hz), 8.19 (d, 1H, *J* = 7.9 Hz), 7.67 (dd, 1H, *J* = 4.2, 8.6 Hz), 7.46 (quin, 1H, *J* = 4.0 Hz), 7.30 (d, 1H, *J* = 8.1 Hz), 6.22 (d, 1H, *J* = 8.1 Hz), 5.98 (d, 1H, *J* = 8.1 Hz), 4.3-4.6 (m, 3H), 4.1-4.3 (m, 3H), 3.6-4.1 (m, 7H), 3.4-3.6 (m, 9H), 3.2-3.3 (m, 1H), 2.78 (ddd, 2H, *J* = 10.3, 12.0, 19.3 Hz), 1.34 (d, 3H, *J* = 6.2 Hz).

**Example 43**

**5-[(*2S*,*6R*)-2-[[4-[6-(3-amino-4-methoxy-pyrrolidin-1-yl)-3-pyridyl]piperazin-1-yl]methyl]-6-methyl-morpholin-4-yl]quinoline-8-carbonitrile**

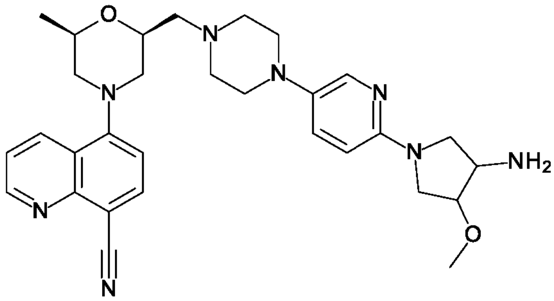

The title compound was prepared in analogy to the preparation of **Example 33** by using 1-(6-bromopyridin-3-yl)piperazine and *tert*-butyl N-(4-methoxypyrrolidin-3-yl)carbamate instead of 1-(6-bromopyridin-2-yl)piperazine (compound **33a**) and 1-methylpiperazine. **Example 43** (4.1 mg) was obtained as a light yellow solid. MS: calc'd 543 ($MH^+$), measured 543 ($MH^+$). $^1H$ NMR (METHANOL-$d_4$, 400 MHz) δ 9.01 (dd, 1H, *J* = 1.6, 4.3 Hz), 8.69 (dd, 1H, *J* = 1.7, 8.6 Hz), 8.19 (d, 1H, *J* = 7.9 Hz), 7.93 (dd, 1H, *J* = 2.8, 9.7 Hz), 7.6-7.8 (m, 2H), 7.31 (d, 1H, *J* = 8.1 Hz), 7.03 (d, 1H, *J* = 9.5 Hz), 4.5-4.6 (m, 1H), 4.3-4.4 (m, 1H), 4.1-4.3 (m, 2H), 4.0-4.1 (m, 1H), 3.8-3.9 (m, 1H), 3.7-3.8 (m, 2H), 3.6-3.7 (m, 4H), 3.53 (s, 3H), 3.3-3.5 (m, 7H), 3.0-3.2 (m, 1H), 2.7-2.9 (m, 2H), 1.34 (d, 3H, *J* = 6.2 Hz).

## Example 44

**5-[(*2S,6R*)-2-[[4-[6-(3-amino-4-fluoro-pyrrolidin-1-yl)-3-pyridyl]piperazin-1-yl]methyl]-6-methyl-morpholin-4-yl]quinoline-8-carbonitrile**

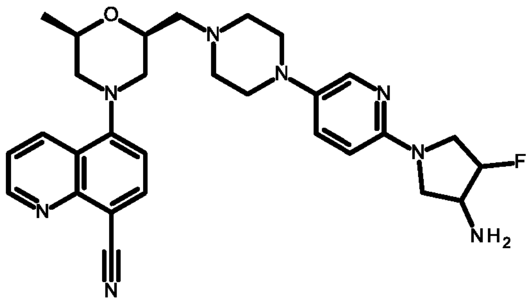

The title compound was prepared in analogy to the preparation of **Example 33** by using 1-(6-bromopyridin-3-yl)piperazine and *tert*-butyl N-(4-fluoropyrrolidin-3-yl)carbamate and instead of 1-(6-bromopyridin-2-yl)piperazine (compound **33a**) and 1-methylpiperazine. **Example 44** (2.0 mg) was obtained as a light yellow solid. MS: calc'd 531 ($MH^+$), measured 531 ($MH^+$). $^1H$ NMR (METHANOL-$d_4$, 400 MHz) δ 8.88 (d, 1H, *J* = 4.5 Hz), 8.56 (d, 1H, *J* = 7.0 Hz), 8.05 (d, 1H, *J* = 7.9 Hz), 7.67 (d, 1H, *J* = 2.8 Hz), 7.5-7.5 (m, 1H), 7.5-7.5 (m, 1H), 7.53 (s, 1H), 7.31 (dd, 1H, *J* = 2.8, 9.1 Hz), 7.1-7.2 (m, 1H), 6.44 (d, 1H, *J* = 8.9 Hz), 4.1-4.1 (m, 1H), 3.9-4.0 (m, 1H), 3.6-3.8 (m, 2H), 3.5-3.6 (m, 3H), 3.3-3.4 (m, 4H), 2.97 (s, 3H), 2.8-2.8 (m, 1H), 2.7-2.7 (m, 1H), 2.5-2.7 (m, 6H), 1.16 (d, 3H, *J* = 6.2 Hz).

## Example 45

**5-[(*2S,6R*)-2-[[4-(5-fluoro-2-piperazin-1-yl-pyrimidin-4-yl)piperazin-1-yl]methyl]-6-methyl-morpholin-4-yl]quinoline-8-carbonitrile**

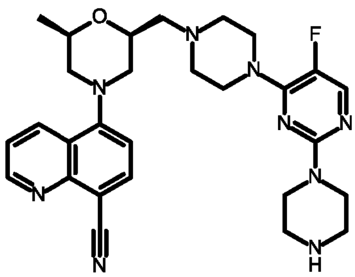

The title compound was prepared according to the following scheme:

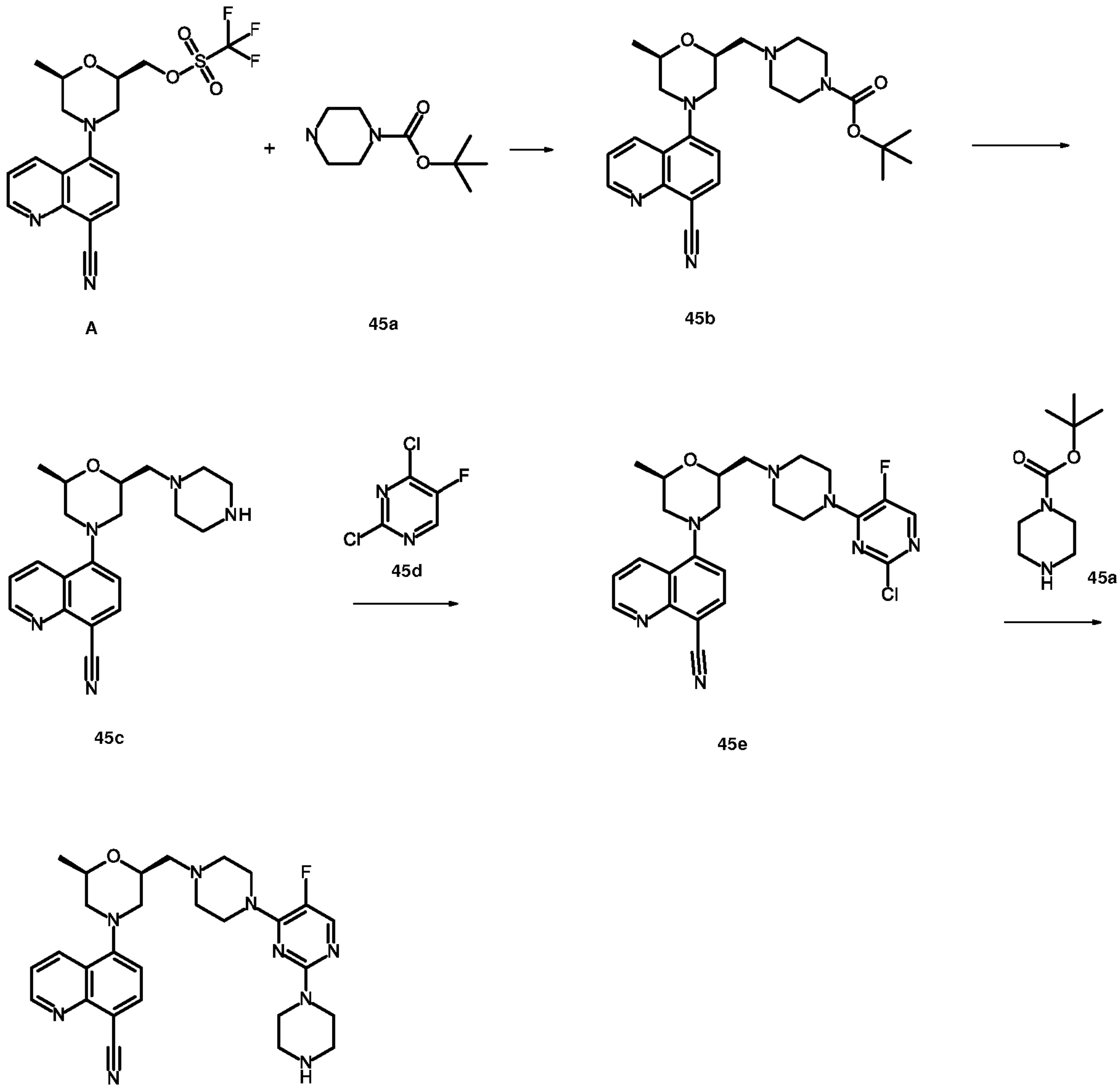

A

45a

45b

45d

45a

45c

45e

45

**Step 1: tert-butyl 4-(((2*S*,6*R*)-4-(8-cyanoquinolin-5-yl)-6-methylmorpholin-2-yl)methyl)piperazine-1-carboxylate (compound 45b)**

A suspension of ((*2R,6R*)-4-(8-cyanoquinolin-5-yl)-6-methylmorpholin-2-yl)methyl trifluoromethanesulfonate (Intermediate **A**, 800 mg, 1.93 mmol), *tert*-butyl piperazine-1-carboxylate (compound **45a**, 430 mg, 2.31 mmol) and $K_2CO_3$ (532 mg, 3.85 mmol) in ACN (8 mL) was heated at 90°C for 3 hrs. Then the mixture was filtered and the filtrate was concentrated and purified by silica gel chromatography to afford *tert*-butyl 4-(((2*S*,6*R*)-4-(8-cyanoquinolin-5-yl)-6-methylmorpholin-2-yl)methyl)piperazine-1-carboxylate (compound **45b**, 800 mg) as yellow solid. MS: calc'd 452 ($MH^+$), measured 452 ($MH^+$).

**Step 2 : 5-((2*R*,6*S*)-2-methyl-6-(piperazin-1-ylmethyl)morpholino)quinoline-8-carbonitrile 2,2,2-trifluoroacetate (compound 45c)**

A solution of *tert*-butyl 4-(((2S,6R)-4-(8-cyanoquinolin-5-yl)-6-methylmorpholin-2-yl)methyl)piperazine-1-carboxylate (compound **45b**, 800 mg, 1.77 mmol) and added TFA (0.5mL) in DCM (6 mL) was stirred at rt for 2 hrs. Then the mixture was concentrated to afford crude 5-((2*R*,6*S*)-2-methyl-6-(piperazin-1-ylmethyl)morpholino)quinoline-8-carbonitrile 2,2,2-trifluoroacetate (compound **45c**, 800 mg) as yellow oil. MS: calc'd 352 ($MH^+$), measured 352 ($MH^+$).

**Step 3 : 5-((2*S*,6*R*)-2-((4-(2-chloro-5-fluoropyrimidin-4-yl)piperazin-1-yl)methyl)-6-methylmorpholino)quinoline-8-carbonitrile (compound 45e)**

A solution of 2,4-dichloro-5-fluoropyrimidine (compound **45d**, 63 mg, 376 μmol) and 5-((2*R*,6*S*)-2-methyl-6-(piperazin-1-ylmethyl)morpholino)quinoline-8-carbonitrile 2,2,2-trifluoroacetate (compound **45c**, 250 mg, 376 μmol) in ethanol (6 mL) was stirred at room temperature for 12 hrs. Then the mixture was concentrated and purified by silica gel Chromatography to afford 5-((2*S*,6*R*)-2-((4-(2-chloro-5-fluoropyrimidin-4-yl)piperazin-1-yl)methyl)-6-methylmorpholino)quinoline-8-carbonitrile (compound **45e,** 90 mg). MS: calc'd 482 ($MH^+$), measured 482 ($MH^+$).

**Step 4 : 5-[(2*S*,6*R*)-2-[[4-(5-fluoro-2-piperazin-1-yl-pyrimidin-4-yl)piperazin-1-yl]methyl]-6-methyl-morpholin-4-yl]quinoline-8-carbonitrile (compound 45)**

A suspension of 5-((2S,6R)-2-((4-(2-chloro-5-fluoropyrimidin-4-yl)piperazin-1-yl)methyl)-6-methylmorpholino)quinoline-8-carbonitrile (compound **45e,** 90 mg, 187 μmol), *tert*-butyl piperazine-1-carboxylate (compound **45a**, 37 mg, 196 μmol), Ruphos Pd G2 (15 mg, 18.7 μmol) and $k_2CO_3$ (52 mg, 373 μmol) in dioxane (10 mL) was heated at 100°C for 12 hrs under nitrogen atmosphere. The reaction mixture was cooled to room temperature and filtered. The filtrate was concentrated. Then the residue was purified by silica gel to get crude intermediate (*tert*-butyl 4-(4-(4-(((2*S*,6*R*)-4-(8-cyanoquinolin-5-yl)-6-methylmorpholin-2-yl)methyl)piperazin-1-yl)-5-fluoropyrimidin-2-yl)piperazine-1-carboxylate) (60 mg). MS: calc'd 632 ($MH^+$), measured 632 ($MH^+$).

To a solution of crude intermediate *tert*-butyl 4-(4-(4-(((2*S*,6*R*)-4-(8-cyanoquinolin-5-yl)-6-methylmorpholin-2-yl)methyl)piperazin-1-yl)-5-fluoropyrimidin-2-yl)piperazine-1-carboxylate (60 mg, 95 μmol) in DCM (2 mL) was added TFA (49 mg, 427 μmol) at 0 °C. The reaction mixture was stirred at rt for 2 hrs. Then the mixture was concentrated and purified by prep-HPLC to afford **Example 45** (27.1 mg) as a light yellow solid. MS: calc'd 532 ($MH^+$), measured 532 ($MH^+$). $^1H$ NMR (METHANOL-$d_4$, 400 MHz) δ 9.0-9.0 (m, 1H), 8.6-8.7 (m, 1H), 8.1-8.2 (m, 1H), 8.0-8.1 (m, 1H), 7.6-7.7 (m, 1H), 7.2-7.3 (m, 1H), 4.5-4.6 (m, 1H), 4.3-4.3 (m, 1H), 4.1-4.3 (m, 1H), 4.1-4.1 (m, 1H), 4.0-4.0 (m, 4H), 3.7-3.7 (m, 1H), 3.5-3.7 (m, 3H), 3.4-3.5 (m, 4H), 3.3-3.3 (m, 6H), 2.7-2.8 (m, 2H), 1.3-1.4 (m, 3H).

**Example 46**

**5-[(2*S*,6*R*)-2-[[4-(5,6-dimethyl-2-piperazin-1-yl-pyrimidin-4-yl)piperazin-1-yl]methyl]-6-methyl-morpholin-4-yl]quinoline-8-carbonitrile**

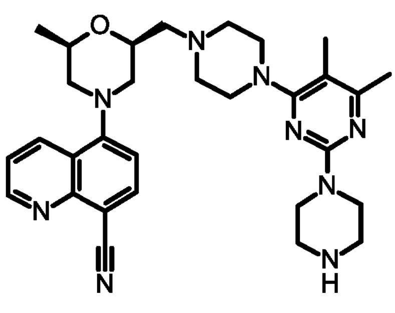

The title compound was prepared in analogy to the preparation of **Example 45** by using 2,4-dichloro-5,6-dimethylpyrimidine instead of 2,4-dichloro-5-fluoropyrimidine (compound **45d). Example 46** (18.1 mg) was obtained as

a light yellow solid. MS: calc'd 542 (MH+), measured 542 (MH+). $^1$H NMR (METHANOL-$d_4$, 400 MHz) δ 8.9-9.0 (m, 1H), 8.6-8.7 (m, 1H), 8.1-8.2 (m, 1H), 7.6-7.7 (m, 1H), 7.2-7.3 (m, 1H), 4.5-4.6 (m, 1H), 4.11 (br d, 6H, *J* = 4.3 Hz), 3.8-4.1 (m, 2H), 3.5-3.8 (m, 4H), 3.3-3.5 (m, 9H), 2.7-2.8 (m, 2H), 2.4-2.5 (m, 3H), 2.2-2.2 (m, 3H), 1.3-1.4 (m, 3H).

**Example 47**

**5-[(2*R*,6*S*)-2-methyl-6-[[4-(6-methyl-2-piperazin-1-yl-pyrimidin-4-yl)piperazin-1-yl]methyl]morpholin-4-yl]quinoline-8-carbonitrile**

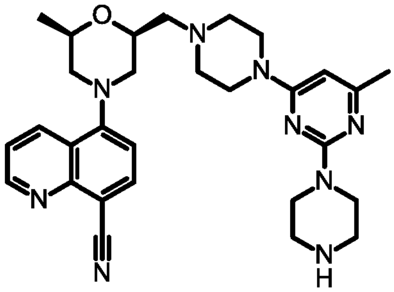

The title compound was prepared in analogy to the preparation of **Example 45** by using 2,4-dichloro-6-methylpyrimidine instead of 2,4-dichloro-5-fluoropyrimidine(compound **45d**). **Example 47** (196.3 mg) was obtained as a light yellow solid. MS: calc'd 528 (MH+), measured 528 (MH+). $^1$H NMR (METHANOL-$d_4$, 400 MHz) δ 9.00 (dd, 1H, *J* = 1.6, 4.3 Hz), 8.66 (dd, 1H, *J* = 1.7, 8.6 Hz), 8.17 (d, 1H, *J* = 7.9 Hz), 7.65 (dd, 1H, *J* = 4.3, 8.6 Hz), 7.28 (d, 1H, *J* = 8.1 Hz), 6.55 (s, 1H), 4.5-4.6 (m, 1H), 3.9-4.4 (m, 9H), 3.58 (br d, 4H, *J* = 1.6 Hz), 3.3-3.5 (m, 8H), 2.7-2.8 (m, 2H), 2.43 (s, 3H), 1.31 (d, 3H, *J* = 6.2 Hz).

**Example 48**

**5-[(2*S*,6*R*)-2-[[4-(5-methoxy-2-piperazin-1-yl-pyrimidin-4-yl)piperazin-1-yl]methyl]-6-methyl-morpholin-4-yl]quinoline-8-carbonitrile**

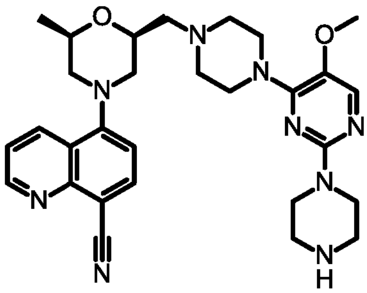

The title compound was prepared in analogy to the preparation of **Example 45** by using 2,4-dichloro-5-methoxypyrimidine instead of 2,4-dichloro-5-fluoropyrimidine(compound **45d**). **Example 48** (54.3 mg) was obtained as a light yellow solid. MS: calc'd 544 (MH+), measured 544 (MH+). $^1$H NMR (METHANOL-$d_4$, 400 MHz) δ 9.01 (dd, 1H, *J* = 1.6, 4.3 Hz), 8.68 (dd, 1H, *J* = 1.7, 8.6 Hz), 8.18 (d, 1H, *J* = 8.1 Hz), 7.75 (s, 1H), 7.67 (dd, 1H, *J* = 4.3, 8.6 Hz), 7.30 (d, 1H, *J* = 8.1 Hz), 4.5-4.6 (m, 1H), 4.2-4.3 (m, 2H), 4.0-4.0 (m, 4H), 3.88 (s, 3H), 3.4-3.7 (m, 8H), 3.3-3.4 (m, 7H), 2.7-2.8 (m, 2H), 1.33 (d, 3H, *J* = 6.2 Hz).

**Example 49**

**5-[(2*R*,6*S*)-2-methyl-6-[[4-(2-piperazin-1-ylquinazolin-4-yl)piperazin-1-yl]methyl]morpholin-4-yl]quinoline-8-carbonitrile**

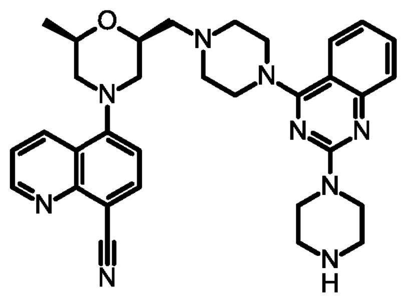

The title compound was prepared in analogy to the preparation of **Example 45** by using 2,4-dichloroquinazoline instead of 2,4-dichloro-5-fluoropyrimidine(compound **45d**). **Example 49** (10.3 mg) was obtained as a light yellow solid. MS: calc'd 564 ($MH^+$), measured 564 ($MH^+$). $^1H$ NMR (METHANOL-$d_4$, 400 MHz) $\delta$ 9.02 (dd, 1H, *J* = 1.7, 4.3 Hz), 8.69 (dd, 1H, *J* = 1.6, 8.6 Hz), 8.20 (d, 1H, *J* = 8.1 Hz), 8.01 (d, 1H, *J* = 8.2 Hz), 7.8-7.8 (m, 1H), 7.6-7.7 (m, 2H), 7.4-7.5 (m, 1H), 7.31 (d, 1H, *J* = 8.1 Hz), 4.5-4.6 (m, 1H), 4.1-4.3 (m, 8H), 3.6-3.7 (m, 4H), 3.4-3.5 (m, 9H), 2.7-2.9 (m, 2H), 1.34 (d, 3H, *J* = 6.4 Hz).

**Example 50**

**5-[(2S,6R)-2-[[4-(5-cyano-2-piperazin-1-yl-pyrimidin-4-yl)piperazin-1-yl]methyl]-6-methyl-morpholin-4-yl]quinoline-8-carbonitrile**

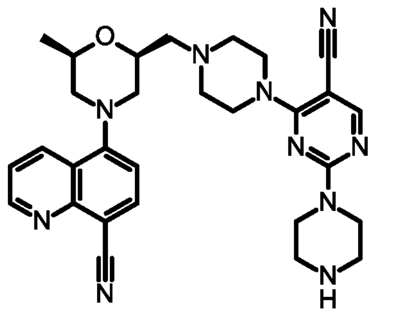

The title compound was prepared according to the following scheme:

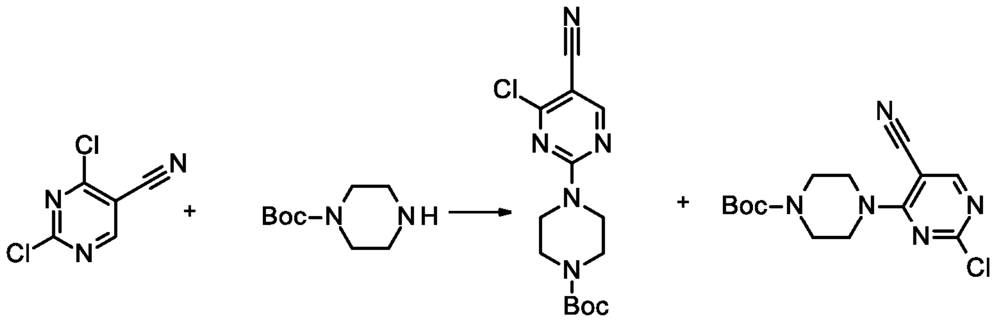

50a 50b 50c 50d

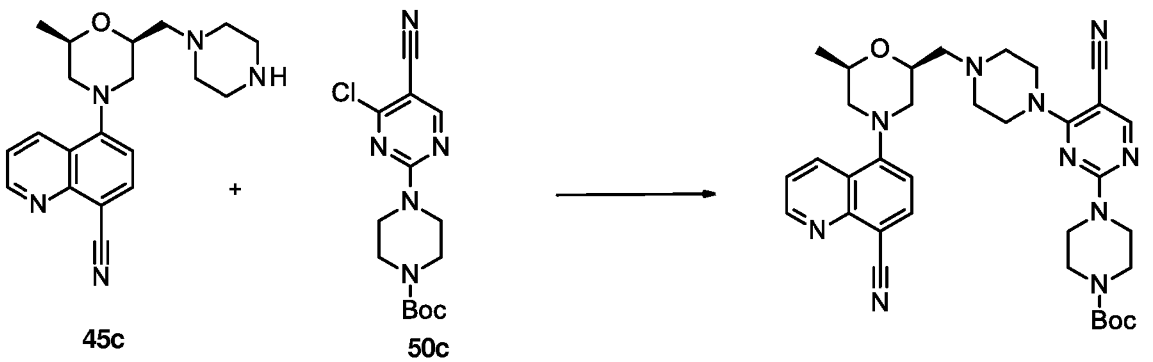

45c 50c 50e

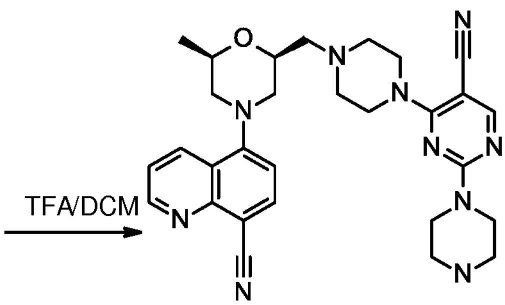


50

**Step 1 : *tert*-butyl 4-(4-chloro-5-cyanopyrimidin-2-yl)piperazine-1-carboxylate (compound 50c) and tert-butyl 4-(2-chloro-5-cyanopyrimidin-4-yl)piperazine-1-carboxylate (compound 50d)**

A solution of 2,4-dichloropyrimidine-5-carbonitrile (174 mg, 1 mmol) and *tert*-butyl piperazine-1-carboxylate (186 mg, 1 mmol) in ethanol (6 mL) was stirred at rt for 12 hrs. Then the mixture was concentrated and purified by silica gel to get *tert*-butyl 4-(4-chloro-5-cyanopyrimidin-2-yl)piperazine-1-carboxylate (compound **50c,** 100 mg) and *tert*-butyl 4-(2-chloro-5-cyanopyrimidin-4-yl)piperazine-1-carboxylate (compound **50d**, 120 mg).

Compound **50c**, MS: calc'd 324 ($MH^+$), measured 324 ($MH^+$). $^1H$ NMR (CHLOROFORM-d, 400 MHz) δ 8.35 (s, 1H), 3.7-3.9 (m, 4H), 3.4-3.5 (m, 4H), 1.42 (s, 9H).

Compound **50d**, MS: calc'd 324 ($MH^+$), measured 324 ($MH^+$). $^1H$ NMR (CHLOROFORM-d, 400 MHz) δ 8.33 (s, 1H), 3.9-4.0 (m, 4H), 3.5-3.6 (m, 4H), 1.42 (s, 9H).

**Step 2 : *tert*-butyl 4-(5-cyano-4-(4-(((2S,6R)-4-(8-cyanoquinolin-5-yl)-6-methylmorpholin-2-yl)methyl)piperazin-1-yl)pyrimidin-2-yl)piperazine-1-carboxylate (compound 50e)**

A solution of 5-((2*R*,6*S*)-2-methyl-6-(piperazin-1-ylmethyl)morpholino)quinoline-8-carbonitrile 2,2,2-trifluoroacetate (compound **45c**, 200 mg, 301 μmol), *tert*-butyl 4-(4-chloro-5-cyanopyrimidin-2-yl)piperazine-1-carboxylate (compound **50c**, 100 mg, 309 μmol) and $K_2CO_3$ (83 mg, 602 μmol) in ACN (6 mL) was heated at 90°C for 2 hrs. Then the reaction mixture was cooled to room temperature, diluted with EtOAc (10 mL) and washed with water (5 mL). The organic layer was dried and concentrated to afford *tert*-butyl 4-(5-cyano-4-(4-(((2*S*,6*R*)-4-(8-cyanoquinolin-5-yl)-6-methylmorpholin-2-yl)methyl)piperazin-1-yl)pyrimidin-2-yl)piperazine-1-carboxylate (compound **50e**, 140 mg) which can be used for next step without purification. MS: calc'd 639 ($MH^+$), measured 639 ($MH^+$).

**Step 3 : 5-[(2*S*,6*R*)-2-[[4-(5-cyano-2-piperazin-1-yl-pyrimidin-4-yl)piperazin-1-yl]methyl]-6-methyl-morpholin-4-yl]quinoline-8-carbonitrile (Example 50)**

To a solution of *tert*-butyl 4-(5-cyano-4-(4-(((2S,6R)-4-(8-cyanoquinolin-5-yl)-6-methylmorpholin-2-yl)methyl)piperazin-1-yl)pyrimidin-2-yl)piperazine-1-carboxylate (compound **50e,** 140 mg, 219 μmol) in DCM (2 mL) was added TFA (75 mg, 658 μmol) at 0°C. The reaction mixture was stirred at rt for 2 hrs. Then the mixture was concentrated and purified by prep-HPLC to afford **Example 50** (88.8 mg) as a light yellow solid. MS: calc'd 539 ($MH^+$), measured 539 ($MH^+$). $^1H$ NMR (METHANOL-$d_4$, 400 MHz) δ 9.02 (dd, 1H, *J* = 1.7, 4.3 Hz), 8.67 (d, 1H, *J* = 1.6 Hz), 8.45 (s, 1H), 8.19 (d, 1H, *J* = 7.9 Hz), 7.67 (dd, 1H, *J* = 4.3, 8.6 Hz), 7.30 (d, 1H, *J* = 8.1 Hz), 4.5-4.6 (m, 1H), 4.2-4.3 (m, 2H), 4.1-4.2 (m, 5H), 3.5-3.7 (m, 4H), 3.4-3.5 (m, 5H), 3.3-3.3 (m, 5H), 2.78 (ddd, 2H, *J* = 10.1, 12.0, 18.5 Hz), 1.34 (d, 3H, *J* = 6.2 Hz).

**Example 51**

**5-[(2*S*,6*R*)-2-[[4-(5-cyano-4-piperazin-1-yl-pyrimidin-2-yl)piperazin-1-yl]methyl]-6-methyl-morpholin-4-yl]quinoline-8-carbonitrile**

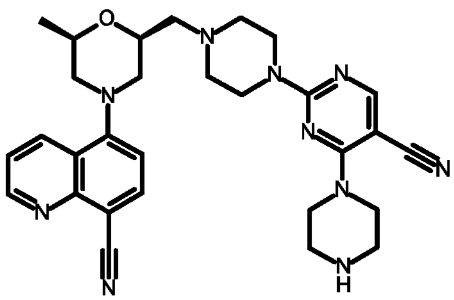

The title compound was prepared in analogy to the preparation of **Example 50** by using *tert*-butyl 4-(2-chloro-5-cyanopyrimidin-4-yl)piperazine-1-carboxylate(compound **50d**) instead of *tert*-butyl 4-(4-chloro-5-cyanopyrimidin-2-yl)piperazine-1-carboxylate (compound **50c**). **Example 51** (68 mg) was obtained as a light yellow solid. MS: calc'd 539 ($MH^+$), measured 539 ($MH^+$). $^1H$ NMR (METHANOL-$d_4$, 400 MHz) δ 9.02 (dd, 1H, *J* = 1.7, 4.2 Hz), 8.68 (dd, 1H, *J* = 1.7, 8.6 Hz), 8.45 (s, 1H), 8.19 (d, 1H, *J* = 8.1 Hz), 7.67 (dd, 1H, *J* = 4.2, 8.6 Hz), 7.31 (d, 1H, *J* = 8.1 Hz), 4.5-4.6 (m, 1H), 4.1-4.3 (m, 6H), 3.3-3.9 (m, 15H), 2.7-2.8 (m, 2H), 1.34 (d, 3H, *J* = 6.2 Hz).

**Example 52**

**5-[(*2S,6R*)-2-[[4-[2-[(*3R,4R*)-3-amino-4-methoxy-pyrrolidin-1-yl]pyrimidin-4-yl]piperazin-1-yl]methyl]-6-methyl-morpholin-4-yl]quinoline-8-carbonitrile**

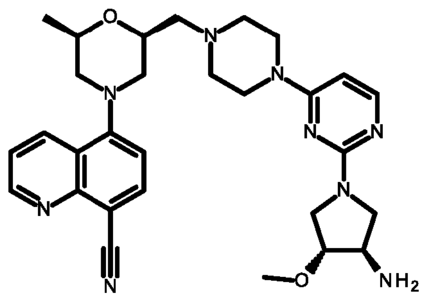

The title compound was prepared in analogy to the preparation of Example **23** by using *tert*-butyl *N*-[(3*R*,4*R*)-4-methoxypyrrolidin-3-yl]carbamate ( PharmaBlock, PBZ4728, CAS: 1932066-52-8) instead of *tert*-butyl azetidin-3-ylcarbamate (compound **23c**). **Example 52** (33.8 mg) was obtained as a light yellow solid. MS: calc'd 544 ($MH^+$), measured 544 ($MH^+$). $^1H$ NMR (METHANOL-$d_4$, 400 MHz) δ 9.01 (br d, 1H, *J* = 3.3 Hz), 8.67 (br d, 1H, *J* = 7.8 Hz), 8.18 (d, 1H, *J* = 7.9 Hz), 7.91 (d, 1H, *J* = 7.3 Hz), 7.66 (dd, 1H, *J* = 4.2, 8.6 Hz), 7.29 (d, 1H, *J* = 7.9 Hz), 6.67 (d, 1H, *J* = 7.5 Hz), 4.5-4.6 (m, 1H), 4.1-4.3 (m, 6H), 4.04 (br s, 4H), 3.5-3.7 (m, 4H), 3.50 (s, 3H), 3.4-3.5 (m, 5H), 2.7-2.9 (m, 2H), 1.33 (d, 3H, *J* = 6.1 Hz).

## Example 53

**5-[(*2S,6R*)-2-[[4-[2-[(*3S,4S*)-3-amino-4-methoxy-pyrrolidin-1-yl]pyrimidin-4-yl]piperazin-1-yl]methyl]-6-methyl-morpholin-4-yl]quinoline-8-carbonitrile**

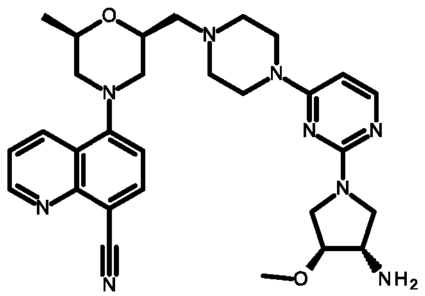

The title compound was prepared in analogy to the preparation of Example **23** by using *tert*-butyl *N*-[(3*S*,4*S*)-4-methoxypyrrolidin-3-yl]carbamate ( PharmaBlock, PBZ4724, CAS: 1627185-88-9) instead of *tert*-butyl azetidin-3-ylcarbamate (compound **23c**). **Example 53** (33.6 mg) was obtained as a light yellow solid. MS: calc'd 544 ($MH^+$), measured 544 ($MH^+$). $^1H$ NMR (METHANOL-$d_4$, 400 MHz) δ 9.01 (dd, 1H, *J* = 1.2, 4.2 Hz), 8.68 (dd, 1H, *J* = 1.2, 8.4 Hz), 8.18 (d, 1H, *J* = 7.9 Hz), 7.91 (d, 1H, *J* = 7.5 Hz), 7.67 (dd, 1H, *J* = 4.2, 8.6 Hz), 7.30 (d, 1H, *J* = 8.1 Hz), 6.67 (d, 1H, *J* = 7.6 Hz), 4.55 (br s, 1H), 4.1-4.4 (m, 6H), 4.0-4.1 (m, 4H), 3.5-3.7 (m, 4H), 3.50 (s, 3H), 3.4-3.5 (m, 5H), 2.7-2.9 (m, 2H), 1.33 (d, 3H, *J* = 6.2 Hz).

## Example 54

**5-[(*2S,6R*)-2-[[4-[2-[(*3S,4R*)-3-amino-4-fluoro-pyrrolidin-1-yl]-6-methyl-pyrimidin-4-yl]piperazin-1-yl]methyl]-6-methyl-morpholin-4-yl]quinoline-8-carbonitrile**

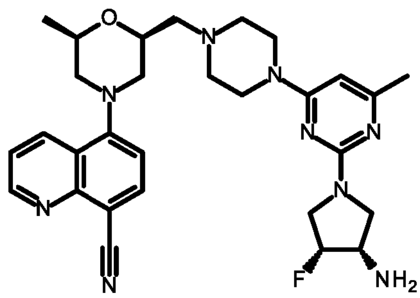

The title compound was prepared in analogy to the preparation of **Example 45** by using tert-butyl N-((3*S*,4*R*)-4-fluoropyrrolidin-3-yl)carbamate ( PharmaBlock, PB09204, CAS: 1033718-91-0) and 2,4-dichloro-6-methylpyrimidine instead of 2,4-dichloro-5-fluoropyrimidine (compound **45d**) and *tert*-butyl piperazine-1-carboxylate (compound **45a**). **Example 54** (69 mg) was obtained as an orange solid. MS: calc'd 546 ($MH^+$), measured 546 ($MH^+$). $^1H$ NMR (METHANOL-$d_4$, 400 MHz) δ 8.98 (dd, 1H, *J* = 1.6, 4.3 Hz), 8.65 (dd, 1H, *J* = 1.6, 8.6 Hz), 8.15 (d, 1H, *J* = 8.1 Hz), 7.64 (dd, 1H, *J* = 4.3, 8.6 Hz), 7.27 (d, 1H, *J* = 8.1 Hz), 6.55 (s, 1H), 5.4-5.6 (m, 1H), 4.5-4.6 (m, 1H), 3.9-4.4 (m, 9H), 3.7-3.8 (m, 1H), 3.5-3.7 (m, 4H), 3.4-3.5 (m, 4H), 2.7-2.8 (m, 2H), 2.44 (s, 3H), 1.31 (d, 3H, *J*= 6.2 Hz).

## Example 55

**5-[(2*S*,6*R*)-2-[[4-[2-[(3*R*,4*S*)-3-amino-4-fluoro-pyrrolidin-1-yl]-6-methyl-pyrimidin-4-yl]piperazin-1-yl]methyl]-6-methyl-morpholin-4-yl]quinoline-8-carbonitrile**

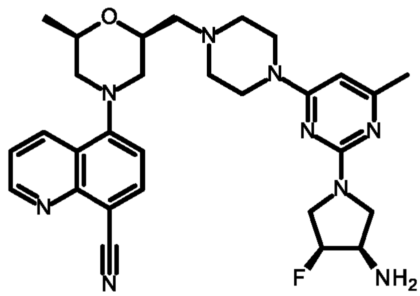

The title compound was prepared in analogy to the preparation of **Example 45** by using *tert*-butyl *N*-((3*R*,4*S*)-4-fluoropyrrolidin-3-yl)carbamate and 2,4-dichloro-6-methylpyrimidine instead of 2,4-dichloro-5-fluoropyrimidine (compound **45d**) and *tert3*-butyl piperazine-1-carboxylate (compound **45a**). **Example 55** (66 mg) was obtained as an orange solid. MS: calc'd 546 ($MH^+$), measured 546 ($MH^+$). $^1H$ NMR (METHANOL-$d_4$, 400 MHz) δ 8.98 (dd, 1H, *J* = 1.5, 4.3 Hz), 8.65 (dd, 1H, *J* = 1.5, 8.6 Hz), 8.15 (d, 1H, *J* = 7.9 Hz), 7.64 (dd, 1H, *J* = 4.3, 8.6 Hz), 7.27 (d, 1H, *J* = 8.1 Hz), 6.55 (s, 1H), 5.4-5.6 (m, 1H), 4.5-4.6 (m, 1H), 3.9-4.4 (m, 9H), 3.7-3.8 (m, 1H), 3.5-3.7 (m, 4H), 3.43 (br d, 4H, *J* = 3.1 Hz), 2.7-2.8 (m, 2H), 2.44 (s, 3H), 1.31 (d, 3H, *J* = 6.2 Hz).

## Example 56

**5-[(2S, 6R)-2-[[4-[2-(6-amino-1,4-oxazepan-4-yl)-6-methyl-pyrimidin-4-yl]piperazin-1-yl]methyl]-6-methyl-morpholin-4-yl]quinoline-8-carbonitrile**

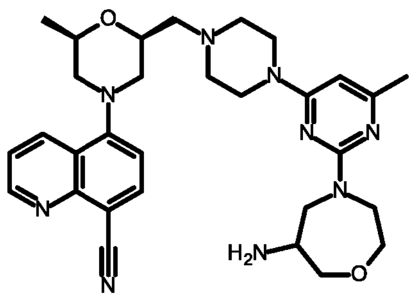

The title compound was prepared in analogy to the preparation of **Example 45** by using *tert*-butyl N-(1,4-oxazepan-6-yl)carbamate (CAS: 1782916-90-8) and 2,4-dichloro-6-methylpyrimidine instead of 2,4-dichloro-5-fluoropyrimidine (compound **45d**) and *tert*-butyl piperazine-1-carboxylate (compound **45a**). **Example 56** (25.3 mg) was obtained as an orange solid. MS: calc'd 558 ($MH^+$), measured 558 ($MH^+$). $^1H$ NMR (METHANOL-$d_4$, 400 MHz) $\delta$ 8.99 (dd, 1H, $J$ = 1.6, 4.3 Hz), 8.65 (dd, 1H, $J$ = 1.7, 8.6 Hz), 8.16 (d, 1H, $J$ = 8.1 Hz), 7.64 (dd, 1H, $J$ = 4.3, 8.6 Hz), 7.28 (d, 1H, $J$ = 8.1 Hz), 6.54 (s, 1H), 4.4-4.6 (m, 2H), 3.7-4.3 (m, 13H), 3.59 (br d, 4H, $J$ = 3.4 Hz), 3.4-3.5 (m, 4H), 2.7-2.8 (m, 2H), 2.44 (s, 3H), 1.31 (d, 3H, $J$ = 6.2 Hz).

**Example 57**

**5-[ (2*S*,6*R*)-2-[[4-[2-[(4-cyanopyrrolidin-3-yl)amino]-6-methyl-pyrimidin-4-yl]piperazin-1-yl]methyl]-6-methyl-morpholin-4-yl]quinoline-8-carbonitrile**

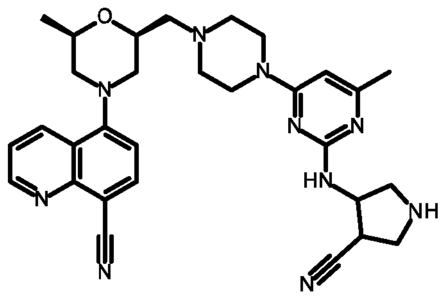

The title compound was prepared in analogy to the preparation of **Example 45** by using *tert*-butyl 3-amino-4-cyanopyrrolidine-1-carboxylate and 2,4-dichloro-6-methylpyrimidine instead of 2,4-dichloro-5-fluoropyrimidine (compound **45d**) and *tert*-butyl piperazine-1-carboxylate (compound **45a**). **Example 57** (53.7 mg) was obtained as an orange solid. MS: calc'd 558 ($MH^+$), measured 558 ($MH^+$). $^1H$ NMR (METHANOL-$d_4$, 400 MHz) $\delta$ 8.99 (dd, 1H, $J$ = 1.5, 4.2 Hz), 8.65 (dd, 1H, $J$ = 1.5, 8.6 Hz), 8.16 (d, 1H, $J$ = 8.1 Hz), 7.64 (dd, 1H, $J$ = 4.3, 8.6 Hz), 7.28 (d, 1H, $J$ = 8.1 Hz), 6.54 (d, 1H, $J$ = 5.3 Hz), 5.2-5.3 (m, 1H), 4.5-4.5 (m, 1H), 3.9-4.4 (m, 5H), 3.7-3.9 (m, 4H), 3.4-3.7 (m, 9H), 2.7-2.8 (m, 2H), 2.4-2.4 (m, 3H), 1.31 (d, 3H, $J$ = 6.2 Hz).

**Example 58**

**5-[(2*R*,6*S*)-2-methyl-6-[[4-[6-methyl-2-[(1R,SR)-3-oxa-7,9-diazabicyclo[3.3.1]nonan-7-yl]pyrimidin-4-yl]piperazin-1-yl]methyl]morpholin-4-yl]quinoline-8-carbonitrile**

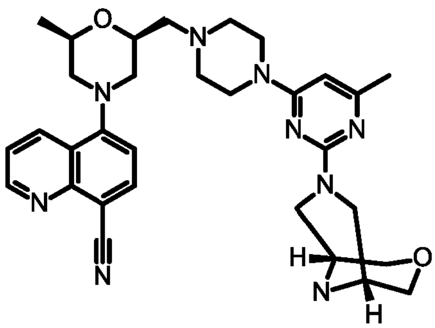

The title compound was prepared in analogy to the preparation of **Example 45** by using *tert*-butyl (*1R,5R*)-3-oxa-7,9-diazabicyclo[3.3.1]nonane-9-carboxylate (PharmaBlock, PB07078, CAS: 1251010-45-3) and 2,4-dichloro-6-methylpyrimidine instead of 2,4-dichloro-5-fluoropyrimidine (compound **45d**) and *tert*-butyl piperazine-1-carboxylate (compound **45a**). **Example 58** (9.9 mg) was obtained as an orange solid. MS: calc'd 570 ($MH^+$), measured 570 ($MH^+$). $^1H$ NMR (METHANOL-$d_4$, 400 MHz) δ 8.99 (dd, 1H, *J* = 1.6, 4.3 Hz), 8.66 (dd, 1H, *J* = 1.5, 8.6 Hz), 8.16 (d, 1H, *J* = 8.1 Hz), 7.65 (dd, 1H, *J* = 4.2, 8.6 Hz), 7.28 (d, 1H, *J* = 7.9 Hz), 6.54 (s, 1H), 4.9-5.1 (m, 2H), 4.5-4.6 (m, 1H), 4.0-4.3 (m, 8H), 3.7-3.9 (m, 4H), 3.58 (br d, 4H, *J* = 1.1 Hz), 3.4-3.5 (m, 4H), 2.7-2.8 (m, 2H), 2.44 (s, 3H), 1.3-1.3 (m, 3H).

**Example 59**

**5-[(2*R*,6*S*)-2-methyl-6-[[4-[6-methyl-2-[trans-3-amino-4-methoxy-pyrrolidin-1-yl]pyrimidin-4-yl]piperazin-1-yl]methyl]morpholin-4-yl]quinoline-8-carbonitrile**

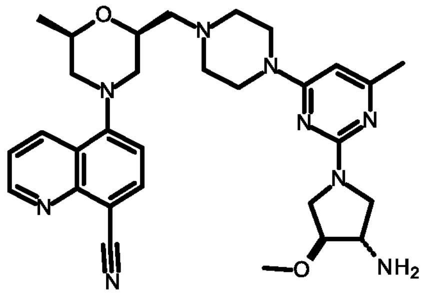

The title compound was prepared in analogy to the preparation of **Example 45** by using *tert*-butyl *N*-(*trans*-4-methoxypyrrolidin-3-yl)carbamate and 2,4-dichloro-6-methylpyrimidine instead of 2,4-dichloro-5-fluoropyrimidine (compound **45d**) and *tert*-butyl piperazine-1-carboxylate (compound **45a**). **Example 59** (4.5 mg) was obtained as an orange solid. MS: calc'd 558 ($MH^+$), measured 558 ($MH^+$). $^1H$ NMR (METHANOL-$d_4$, 400 MHz) δ 9.00 (dd, 1H, *J* = 1.6, 4.3 Hz), 8.66 (dd, 1H, *J* = 1.6, 8.7 Hz), 8.17 (d, 1H, *J* = 7.9 Hz), 7.65 (dd, 1H, *J* = 4.3, 8.6 Hz), 7.28 (d, 1H, *J* = 8.1 Hz), 6.51 (s, 1H), 4.4-4.5 (m, 1H), 4.0-4.4 (m, 9H), 3.6-4.0 (m, 3H), 3.4-3.6 (m, 10H), 2.7-2.8 (m, 2H), 2.42 (s, 3H), 1.31 (d, 3H, *J* = 6.2 Hz).

**Example 60**

**5-[(2*R*,6*S*)-2-methyl-6-[[4-[6-methyl-2-[cis-3-amino-4-methoxy-pyrrolidin-1-yl]pyrimidin-4-yl]piperazin-1-yl]methyl]morpholin-4-yl]quinoline-8-carbonitrile**

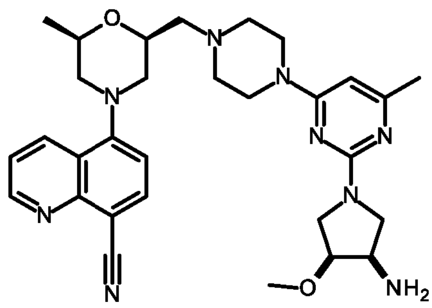

The title compound was prepared in analogy to the preparation of **Example 45** by using *tert*-butyl *N*-(cis-4-methoxypyrrolidin-3-yl)carbamate and 2,4-dichloro-6-methylpyrimidine instead of 2,4-dichloro-5-fluoropyrimidine (compound **45d**) and *tert*-butyl piperazine-1-carboxylate (compound **45a**). **Example 59** (6.2 mg) was obtained as an orange solid. MS: calc'd 558 ($MH^+$), measured 558 ($MH^+$). $^1H$ NMR (METHANOL-$d_4$, 400 MHz) $\delta$ 8.99 (dd, 1H, *J* = 1.6, 4.3 Hz), 8.66 (dd, 1H, *J* = 1.7, 8.6 Hz), 8.16 (d, 1H, *J* = 7.9 Hz), 7.65 (dd, 1H, *J* = 4.3, 8.6 Hz), 7.28 (d, 1H, *J* = 8.1 Hz), 6.53 (s, 1H), 4.5-4.6 (m, 1H), 4.0-4.4 (m, 8H), 3.7-4.0 (m, 3H), 3.5-3.7 (m, 4H), 3.51 (s, 3H), 3.4-3.5 (m, 4H), 2.7-2.8 (m, 2H), 2.43 (s, 3H), 1.31 (d, 3H, *J* = 6.2 Hz).

**Example 61**

**5-[(2*R*,6*S*)-2-methyl-6-[[4-[6-methyl-2-(5-oxa-2,8-diazaspiro[3.5]nonan-2-yl)pyrimidin-4-yl]piperazin-1-yl]methyl]morpholin-4-yl]quinoline-8-carbonitrile**

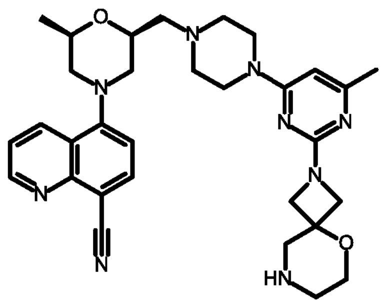

The title compound was prepared in analogy to the preparation of **Example 45** by using *tert*-butyl 5-oxa-2,8-diazaspiro[3.5]nonane-8-carboxylate and 2,4-dichloro-6-methylpyrimidine instead of 2,4-dichloro-5-fluoropyrimidine (compound **45d**) and tert-butyl piperazine-1-carboxylate (compound **45a**). **Example 61** (55 mg) was obtained as an orange solid. MS: calc'd 570 ($MH^+$), measured 570 ($MH^+$). $^1H$ NMR (METHANOL-$d_4$, 400 MHz) $\delta$ 8.98 (dd, 1H, *J* = 1.6, 4.3 Hz), 8.64 (dd, 1H, *J* = 1.5, 8.6 Hz), 8.14 (d, 1H, *J* = 8.1 Hz), 7.64 (dd, 1H, *J* = 4.2, 8.6 Hz), 7.26 (d, 1H, *J* = 8.1 Hz), 6.49 (s, 1H), 4.5-4.6 (m, 1H), 4.3-4.4 (m, 2H), 4.1-4.3 (m, 6H), 4.0-4.0 (m, 2H), 3.56 (s, 6H), 3.41 (br s, 4H), 3.3-3.4 (m, 1H), 3.3-3.3 (m, 2H), 2.7-2.8 (m, 2H), 2.39 (s, 3H), 1.31 (d, 3H, *J* = 6.2 Hz).

**Example 62**

**5-[(2*R*,6*S*)-2-methyl-6-[[4-(3-morpholin-2-ylphenyl)piperazin-1-yl]methyl]-morpholin-4-**yl]**quinoline-8-carbonitrile**

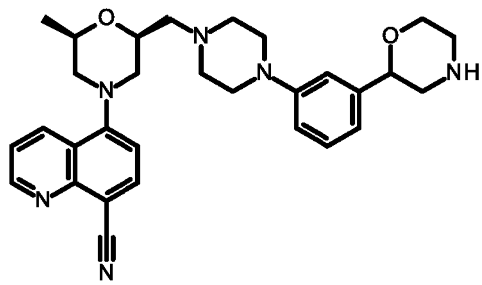

The title compound was prepared according to the following scheme:

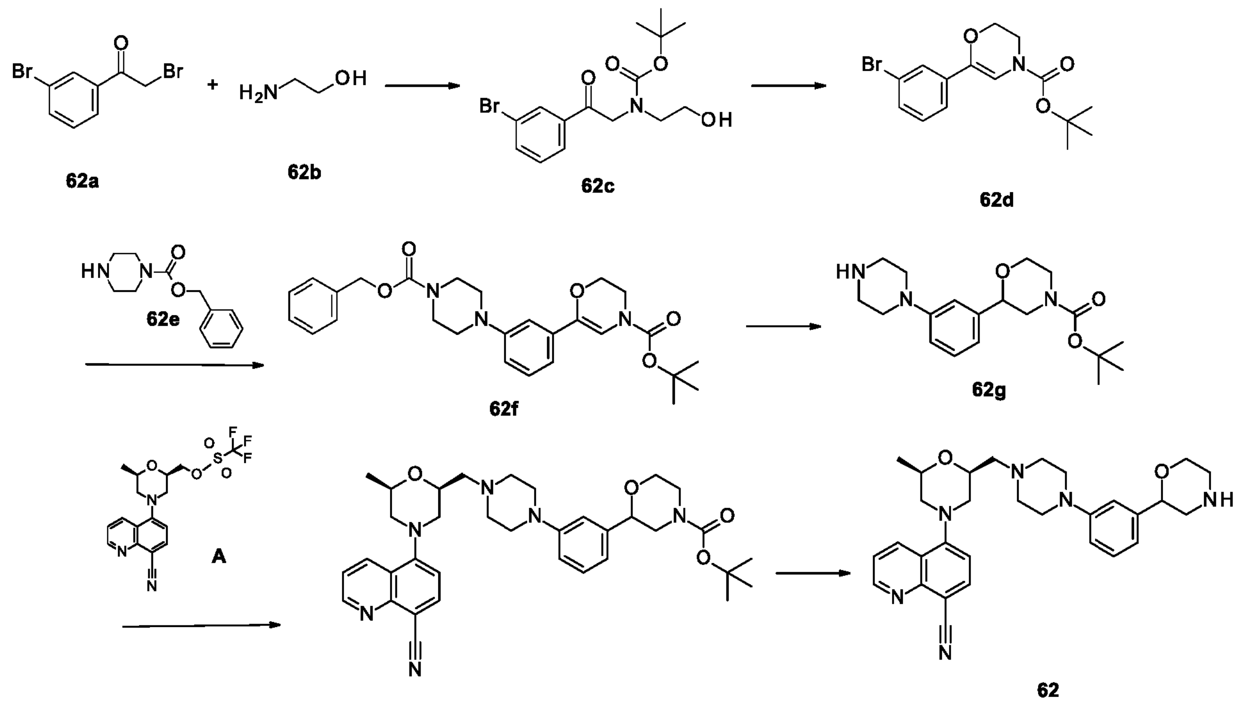

62a 62b 62c 62d 62e 62f 62g A 62h 62

**Step 1: preparation of *tert*-butyl *N*-[2-(3-bromophenyl)-2-oxo-ethyl]-*N*-(2-hydroxyethyl)carbamate(compound 62c)**

To the solution of 2-aminoethan-1-ol (compound **62b**, 1.424 mL, 23.8 mmol) in EtOH (60 mL) was added 2-bromo-1-(3-bromophenyl)ethan-1-one (compound **62a**, 3.3 g, 11.9 mmol) at - 10 °C. Then *N*,*N*-Diisopropylethylamine (6.2 mL) was added to the mixture slowly at the same temperature. The mixture was stirred for 40 minutes at 20°C. Then $Et_3N$ and $(Boc)_2O$ were added to the mixture and the mixture was stirred for 30 minutes at 20°C. The mixture was concentrated and the crude was dissolved in DCM (100 mL). The mixture was washed with saturated $NH_4Cl$ aqueous solution (80 mL) and $H_2O$ (80 mL). The organic layer was dried and concentrated *in vacuo.* The residue was purified by column chromatography to give compound **62c** (1.01 g). MS calc'd 358, 360 ($MH^+$); measured 358, 360 ($MH^+$).

**Step 2: preparation of tert-butyl 6-(3-bromophenyl)-2,3-dihydro-1,4-oxazine-4-carboxylate (compound 62d)**

To the solution of *tert*-butyl *N*-[2-(3-bromophenyl)-2-oxo-ethyl]-*N*-(2-hydroxyethyl)-carbamate (compound **62c**, 358 mg, 1 mmol) and $Et_3SiH$ (160 μL, 2 mmol) in dry DCM (10 mL) was added $InBr_3$ at 0°C. Then the mixture was stirred for 16 h at 20°C. The mixture was diluted with DCM (15 mL) and washed with $H_2O$ (10 mL). The organic layer was dried and concentrated. The residue was purified by column chromatography to give compound **62d** (270 mg). MS calc'd 341 ($MH^+$); measured 341 ($MH^+$).

**Step 3: preparation of *tert*-butyl 6-[3-(4-benzyloxycarbonylpiperazin-1-yl)phenyl]-2,3-dihydro-1,4-oxazine-4-carboxylate (compound 62f)**

The solution of tert-butyl 6-(3-bromophenyl)-2,3-dihydro-1,4-oxazine-4-carboxylate (compound **62d,** 135 mg, 0.397 mmol), benzyl piperazine-1-carboxylate (compound **63e,** 131 mg, 0.595 mmol), *t*BuXPhos Pd G3 (31.5 mg, 0.040 mmol) and *t*BuONa (76.3 mg, 0.794 mmol) in dry dioxane (3 mL) was stirred at 90°C for 2 hrs. The mixture was diluted with EA (20 mL) and filtered through $Na_2SO_4$. The filtrate was washed with saturated $NH_4Cl$ aqueous solution (10 mL) and brine (10 mL). The organic layer was dried and concentrated under vacuum. The residue was purified by column chromatography to give compound **62f** (96 mg). MS calc'd 480 ($MH^+$); measured 480 ($MH^+$).

**Step 4: preparation of *tert*-butyl 2-(3-piperazin-1-ylphenyl)morpholine-4-carboxylate (compound 62g)**

The solution of *tert*-butyl 6-[3-(4-benzyloxycarbonylpiperazin-1-yl)phenyl]-2,3-dihydro-1,4-oxazine-4-carboxylate (compound **62f**, 96 mg, 0.2 mmol), $Pd(OH)_2$ (20% on carbon with 50% $H_2O$) (11.2 mg, 0.04 mmol) in MeOH (3.5 mL) was stirred at 20°C under $H_2$ for 20 hours. The mixture was filtered and the filtrate was concentrated to give compound **62g** (69 mg). MS calc'd 348 ($MH^+$); measured 348 ($MH^+$).

**Step 5: preparation of *tert*-butyl 2-[3-[4-[[(*2S*,*6R*)-4-(8-cyano-5-quinolyl)-6-methyl-morpholin-2-yl]methyl]piperazin-1-yl]phenyl]morpholine-4-carboxylate (compound 62h)**

To a tube was added *tert*-butyl 2-(3-(piperazin-1-yl)phenyl)morpholine-4-carboxylate (compound **62g**, 69 mg, 200 μmol), [(2*R*,6*R*)-4-(8-cyano-5-quinolyl)-6-methyl-morpholin-2-yl]methyl trifluoromethanesulfonate (intermediate **A**, 100 mg, 240 μmol), $K_2CO_3$ (55 mg, 400 μmol) and ACN(6 mL), The mixture was stirred for 2 hrs at 80°C. The mixture was diluted with some ACN and filtered through celite. The filtrate was concentrated to give a yellow oil which was purified by prep-HPLC to give compound **62h** (35 mg) as yellow solid. MS calc'd 613 ($MH^+$); measured 613 ($MH^+$).

**Step 6: preparation of 5-[(*2R*,*6S*)-2-methyl-6-[[4-(3-morpholin-2-ylphenyl)piperazin-1-yl]methyl]morpholin-4-yl]quinoline-8-carbonitrile (Example 62)**

To a solution of *tert*-butyl 2-[3-[4-[[(2*S*,6*R*)-4-(8-cyano-5-quinolyl)-6-methyl-morpholin-2-yl]methyl]piperazin-1-yl]phenyl]morpholine-4-carboxylate (compound **62h**, 7 mg, 0.011 mmol) in EA (3 mL) was added HCl in EA (1 M) (1.0 mL, 1 mmol). After being stirred at rt overnight, the mixture was concentrated *in vacuo* to give **Example 62** (7 mg) as a brown solid. MS calc'd 513 ($MH^+$), measured 513 ($MH^+$).**$^1$H NMR (400 MHz, METHANOL-*$d_4$*)** δ: ppm 9.01 (d, *J* = 3.8 Hz, 1H), 8.71 (br d, *J* = 8.3 Hz, 1H), 8.19 (d, *J* = 7.9 Hz, 1H), 7.68 (dd, *J* = 4.3, 8.4 Hz, 1H), 7.38 - 7.28 (m, 2H), 7.13 (s, 1H), 7.06 (br d, *J* = 8.1 Hz, 1H), 6.98 (d, *J* = 7.6 Hz, 1H), 4.78 - 4.74 (m, 1H), 4.60 - 4.50 (m, 1H), 4.29 - 4.16 (m, 2H), 4.06 - 3.96 (m, 1H), 3.95 - 3.76 (m, 4H), 3.50 - 3.36 (m, 9H), 3.26 - 3.19 (m, 2H), 3.13 - 3.06 (m, 1H), 2.84 - 2.71 (m, 2H), 1.33 (d, *J*= 6.0 Hz, 3 H).

**Example 63**

**5-[(2*S*,6*R*)-2-[[4-[2-(4-amino-3,3-difluoro-pyrrolidin-1-yl)-6-methyl-pyrimidin-4-yl]piperazin-1-yl]methyl]-6-methyl-morpholin-4-yl]quinoline-8-carbonitrile**

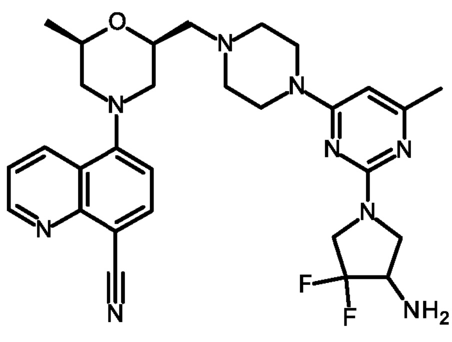

The title compound was prepared in analogy to the preparation of **Example 45** by using *tert*-butyl *N*-(4,4-difluoropyrrolidin-3-yl)carbamate (CAS: 1434141-95-3) and 2,4-dichloro-6-methylpyrimidine instead of 2,4-dichloro-5-fluoropyrimidine (compound **45d**) and *tert*-butyl piperazine-1-carboxylate (compound **45a**). **Example 63** (17.8 mg) was obtained as an orange solid. MS: calc'd 564 (MH+), measured 564 (MH+). $^1$H NMR (METHANOL-$d_4$, 400 MHz) δ 8.99 (dd, 1H, *J* = 1.7, 4.2 Hz), 8.66 (dd, 1H, *J* = 1.6, 8.6 Hz), 8.16 (d, 1H, *J* = 7.9 Hz), 7.65 (dd, 1H, *J* = 4.3, 8.7 Hz), 7.28 (d, 1H, *J* = 8.1 Hz), 6.56 (s, 1H), 4.4-4.6 (m, 2H), 4.1-4.4 (m, 7H), 3.8-4.0 (m, 2H), 3.5-3.7 (m, 4H), 3.4-3.5 (m, 4H), 2.7-2.8 (m, 2H), 2.43 (s, 3H), 1.31 (d, 3H, *J* = 6.2 Hz).

**Example 64**

**8-[(2*R*,6*S*)-2-methyl-6-[[4-(6-methyl-2-piperazin-1-yl-pyrimidin-4-yl)piperazin-1-yl]methyl]morpholin-4-yl]quinoxaline-5-carbonitrile**

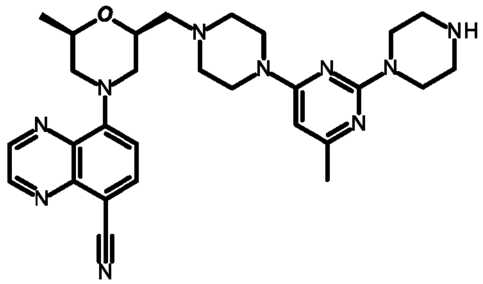

The title compound was prepared in analogy to the preparation of **Example 45** by using 2,4-dichloro-6-methylpyrimidine and ((2*R*,6*R*)-4-(8-cyanoquinoxalin-5-yl)-6-methylmorpholin-2-yl)methyl trifluoromethanesulfonate (Compound **64a)** instead of 2,4-dichloro-5-fluoropyrimidine (compound **45d**) and ((2*R*,6*R*)-4-(8-cyanoquinolin-5-yl)-6-methylmorpholin-2-yl)methyl trifluoromethanesulfonate (Intermediate **A**). **Example 64** (18.8 mg) was obtained as an orange solid. MS: calc'd 529 (MH+), measured 529 (MH+). $^1$H NMR (METHANOL-$d_4$, 400 MHz) δ 8.97 (d, 1H, *J* = 1.7 Hz), 8.89 (d, 1H, *J* = 1.7 Hz), 8.15 (d, 1H, *J* = 8.4 Hz), 7.26 (d, 1H, *J* = 8.3 Hz), 6.47 (s, 1H), 4.4-4.5 (m, 1H), 4.2-4.4 (m, 1H), 4.0-4.2 (m, 9H), 3.4-3.6 (m, 4H), 3.3-3.4 (m, 7H), 2.8-2.9 (m, 2H), 2.39 (s, 3H), 1.32 (d, 3H, *J* = 6.1 Hz).

**Preparation of ((2*R*,6*R*)-4-(8-cyanoquinoxalin-5-yl)-6-methylmorpholin-2-yl)methyl trifluoromethanesulfonate (Compound 64a)**

Compound 64a was prepared in analogy to the preparation of intermediate A by using 8-bromoquinoxaline-5-carbonitrile instead of 5-bromoquinoline-8-carbonitrile (compound **A2**)

**Example 65**

**(2*R*,6*S*)-2-methyl-6-[[4-(6-methyl-2-piperazin-1-yl-pyrimidin-4-yl)piperazin-1-yl]methyl]-4-[8-(trifluoromethyl)quinoxalin-5-yl]morpholine**

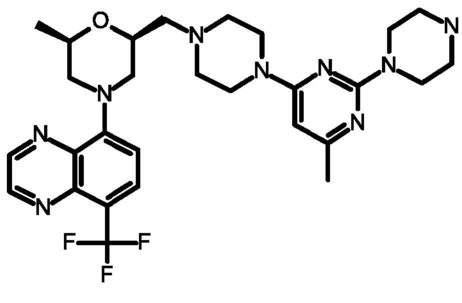

The title compound was prepared in analogy to the preparation of **Example 45** by using 2,4-dichloro-6-methylpyrimidine and ((2*R*,6*R*)-4-(8-(trifluoromethyl)quinoxafin-5-yl)-6-methylmorpholin-2-yl)methyl trifluoromethanesulfonate (Compound **65a)** instead of 2,4-dichloro-5-fluoropyrimidine (compound **45d**) and ((2*R*,6*R*)-4-(8-cyanoquinolin-5-yl)-6-methylmorpholin-2-yl)methyl trifluoromethanesulfonate (Intermediate **A**). **Example 65** (17.6 mg) was obtained as an orange solid. MS: calc'd 572 ($MH^+$), measured 572 ($MH^+$). $^1H$ NMR (METHANOL-$d_4$, 400 MHz) δ 8.95 (d, 1H, *J* = 1.8 Hz), 8.88 (d, 1H, *J* = 1.7 Hz), 8.1-8.1 (m, 1H), 7.27 (d, 1H, *J* = 8.4 Hz), 6.44 (s, 1H), 4.4-4.5 (m, 1H), 4.0-4.2 (m, 10H), 3.4-3.6 (m, 4H), 3.3-3.4 (m, 7H), 2.7-2.8 (m, 2H), 2.38 (s, 3H), 1.33 (d, 3H, *J* = 6.2 Hz).

**Preparation of ((2R,6R)-4-(8-(trifluoromethyl)quinoxalin-5-yl)-6-methylmorpholin-2-yl)methyl trifluoromethanesulfonate (Compound 65a)**

Compound 65a was prepared in analogy to the preparation of intermediate A by using 5-bromo-8-(trifluoromethyl)quinoxaline instead of 5-bromoquinoline-8-carbonitrile (compound **A2**)

**Example 66**

**5-[(2S, 6R)-2-[[4-[2-(6-hydroxy-1,4-diazepan-1-yl)-6-methyl-pyrimidin-4-yl]piperazin-1-yl]methyl]-6-methyl-morpholin-4-yl]quinoline-8-carbonitrile**

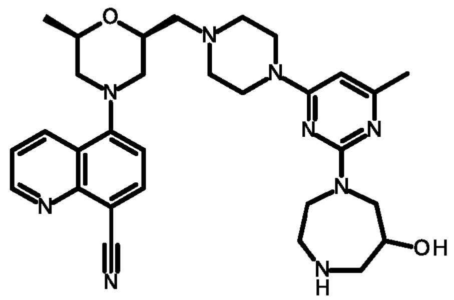

The title compound was prepared in analogy to the preparation of **Example 45** by using *tert*-butyl 6-hydroxy-1,4-diazepane-1-carboxylate (Wuxi Apptec, WX604354, CAS: 956317-40-1) and 2,4-dichloro-6-methylpyrimidine instead of 2,4-dichloro-5-fluoropyrimidine (compound **45d**) and *tert*-butyl piperazine-1-carboxylate (compound **45a**). **Example 66** (24.1 mg) was obtained as an orange solid. MS: calc'd 558 ($MH^+$), measured 558 ($MH^+$). $^1H$ NMR (METHANOL-d4, 400 MHz) δ 9.0-9.1 (m, 1H), 8.68 (br d, 1H, *J* = 8.4 Hz), 8.19 (dd, 1H, *J* = 1.7, 7.8 Hz), 7.6-7.8 (m, 1H), 7.31 (br d, 1H, *J* = 8.2 Hz), 6.53 (s, 1H), 4.5-4.6 (m, 2H), 4.4-4.5 (m, 1H), 4.3-4.4 (m, 2H), 4.1-4.2 (m, 4H), 4.0-4.1 (m, 1H), 3.8-3.9 (m, 2H), 3.6-3.7 (m, 1H), 3.4-3.6 (m, 10H), 2.7-2.9 (m, 2H), 2.43 (s, 3H), 1.33 (br d, 3H, *J* = 6.1 Hz).

**Example 67**

**3-Fluoro-4-[(2*R*,6*S*)-2-methyl-6-[[4-(6-methyl-2-piperazin-1-yl-pyrimidin-4-yl)piperazin-1-yl]methyl]morpholin-4-yl]pyrazolo[1,5-a]pyridine-7-carbonitrile**

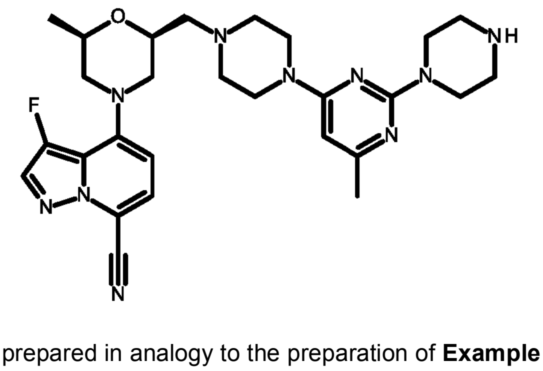

The title compound was prepared in analogy to the preparation of **Example 45** by using 2,4-dichloro-6-methylpyrimidine and (*2R,6R*)-4-(7-cyano-3-fluoro-pyrazolo[1,5-a]pyridin-4-yl)-6-methyl-morpholin-2-yl]methyl trifluoromethanesulfonate (Compound **67a**) instead of 2,4-dichloro-5-fluoropyrimidine (compound **45d**) and ((*2R,6R*)-4-(8-cyanoquinolin-5-yl)-6-methylmorpholin-2-yl)methyl trifluoromethanesulfonate (Intermediate **A**). **Example 67** (28.6 mg) was obtained as an orange solid. MS: calc'd 535 ($MH^+$), measured 535 ($MH^+$). $^1H$ NMR (METHANOL-$d_4$, 400 MHz) δ 8.03 (br d, 1H, *J* = 2.1 Hz), 7.46 (dd, 1H, *J* = 2.1, 7.9 Hz), 6.57 (dd, 1H, *J* = 1.9, 7.9 Hz), 6.48 (s, 1H), 4.3-4.4 (m, 1H), 4.09 (br s, 8H), 3.6-3.7 (m, 3H), 3.4-3.5 (m, 4H), 3.38 (br d, 6H, *J* = 3.5 Hz), 2.7-2.8 (m, 2H), 2.41 (d, 3H, *J* = 1.7 Hz), 1.32 (dd, 3H, *J* = 2.0, 6.2 Hz).

**Preparation of (2*R*,6*R*)-4-(7-cyano-3-fluoro-pyrazolo[1,5-a]pyridin-4-yl)-6-methyl-morpholin-2-yl]methyl trifluoromethanesulfonate (Compound 67a)**

Compound 67a was prepared in analogy to the preparation of intermediate **A** by using 4-bromo-3-fluoro-pyrazolo[1,5-a]pyridine-7-carbonitrile instead of 5-bromoquinoline-8-carbonitrile (compound **A2**)

**Example 68**

**5-[(2*R*,6*S*)-2-methyl-6-[[4-(4-methyl-6-piperazin-1-yl-pyrimidin-2-yl)piperazin-1-yl]methyl]morpholin-4-yl]quinoline-8-carbonitrile**

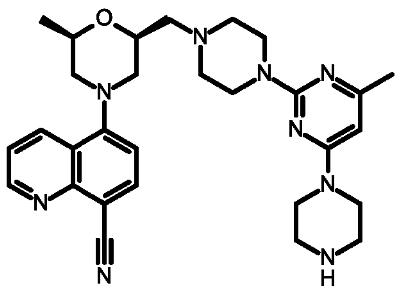

The title compound was prepared in analogy to the preparation of **Example 45** by using 2,4-dichloro-6-methylpyrimidine instead of 2,4-dichloro-5-fluoropyrimidine (compound **45d**). **Example 68** (5.2 mg) was obtained as an orange solid. MS: calc'd 558 ($MH^+$), measured 558 ($MH^+$). $^1H$ NMR (METHANOL-d4, 400 MHz) δ 9.0-9.1 (m, 1H), 8.7-8.7 (m, 1H), 8.19 (d, 1H, *J* = 7.9 Hz), 7.67 (dd, 1H, *J* = 4.2, 8.6 Hz), 7.31 (d, 1H, *J* = 7.9 Hz), 6.50 (s, 1H), 4.5-4.6 (m, 2H), 4.2-4.2 (m, 4H), 4.09 (br s, 4H), 3.57 (br d, 4H, *J* = 2.0 Hz), 3.4-3.5 (m, 8H), 2.7-2.9 (m, 2H), 2.42 (s, 3H), 1.33 (br d, 3H, *J* = 6.2 Hz).

**Example 69A and 69B**

**5-[(2S,6R)-2-[[4-[2-[(3S,4S)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile** and **5-[(2S,6R)-2-[[4-[2-[(3R,4R)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile**

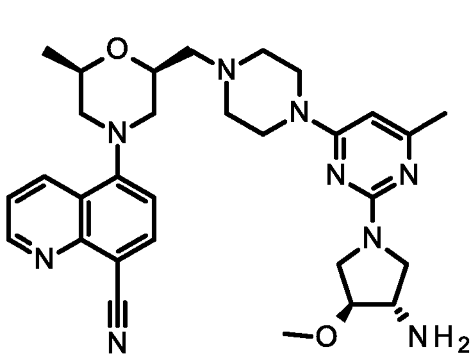

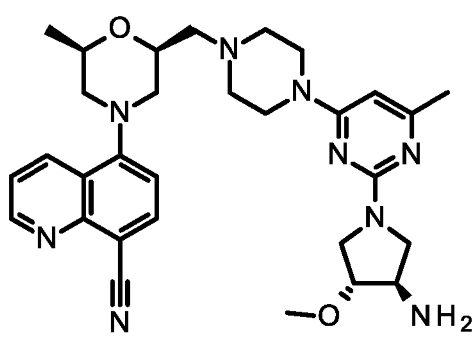

The title compound was prepared according to the following scheme:

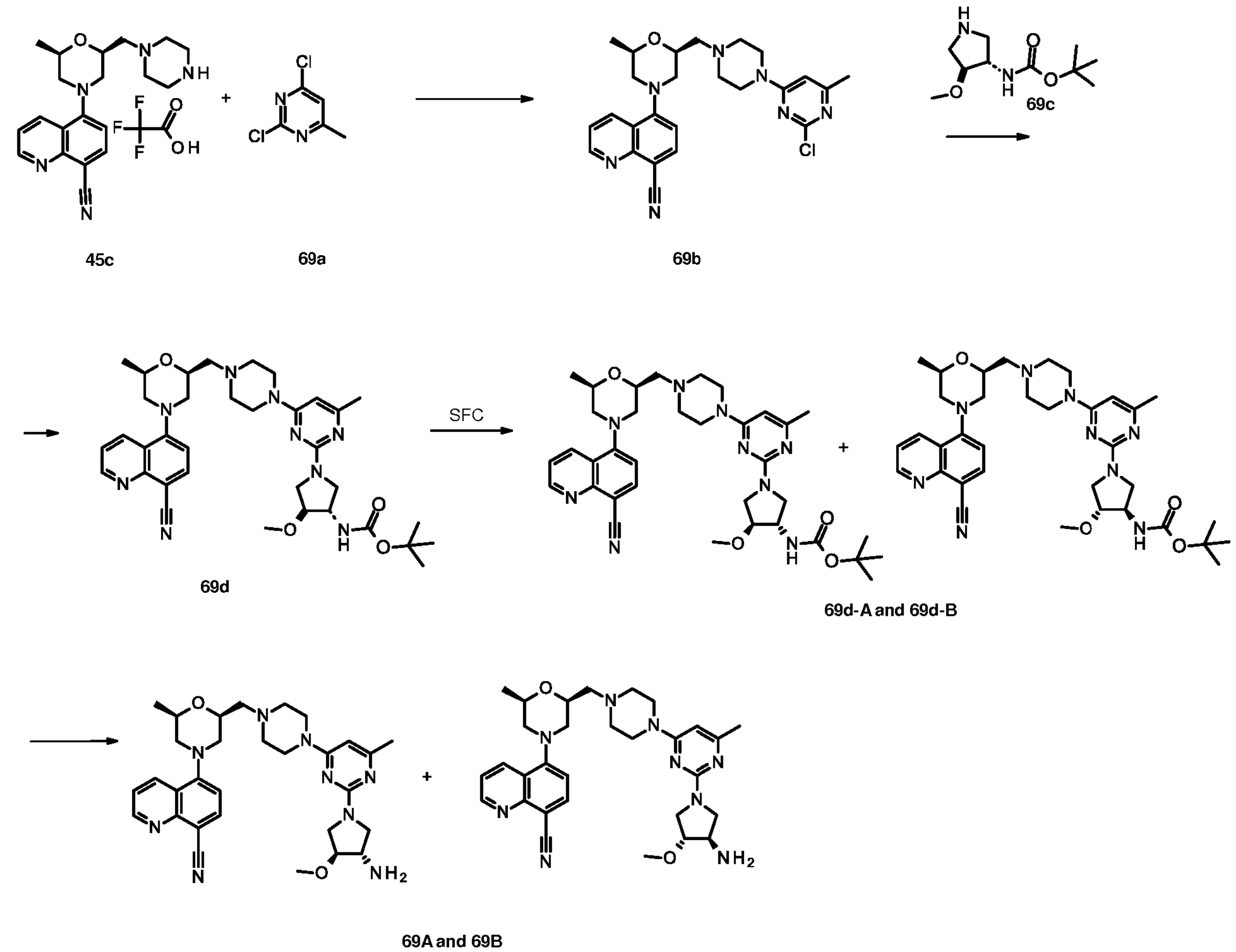


45c 69a 69b 69c

69d 69d-A and 69d-B

69A and 69B

**Step 1 : 5-((*2S,6R*)-2-((4-(2-chloro-6-methylpyrimidin-4-yl)piperazin-1-yl)methyl)-6-methylmorpholino)quinoline-8-carbonitrile (compound 69b)**

A solution of 2,4-dichloro-6-methylpyrimidine (compound **69a**, 350 mg, 2.15 mmol) and 5-((2*R*,6*S*)-2-methyl-6-(piperazin-1-ylmethyl)morpholino)quinoline-8-carbonitrile 2,2,2-trifluoroacetate (compound **45c**, 2 g, 2.15 mmol) in ethanol (10 mL) was stirred at room temperature for 12 hrs. Then reaction mixture was concentrated and purified by silica gel Chromatography to afford 5-((*2S,6R*)-2-((4-(2-chloro-6-methylpyrimidin-4-yl)piperazin-1-yl)methyl)-6-methylmorpholino)quinoline-8-carbonitrile (compound **69b,** 1 g) as a light yellow solid. MS: calc'd 478 ($MH^+$), measured 478 ($MH^+$).

**Step 2 : 5-((*2S,6R*)-2-((4-(2-chloro-6-methylpyrimidin-4-yl)piperazin-1-yl)methyl)-6-methylmorpholino)quinoline-8-carbonitrile (compound 69d)**

A suspension of 5-((*2S,6R*)-2-((4-(2-chloro-6-methylpyrimidin-4-yl)piperazin-1-yl)methyl)-6-methylmorpholino)quinoline-8-carbonitrile (compound **69b,** 240 mg, 502 μmol), *tert*-butyl *N*-(trans-4-methoxypyrrolidin-3-yl)carbamate (compound **69c,** 119 mg, 552 μmol), Ruphos Pd G2 (39 mg, 50.2 μmol) and $K_2CO_3$ (139 mg, 1 mmol) in 1,4-dioxane (4 mL) was heated to 100 °C for 12 hrs under nitrogen atmosphere. The mixture was cooled to rt and then diluted with water (5 mL) and the mixture was extracted with EtOAC (10 mL) three times. The combined organic layer was washed with brine and concentrated. The resulting residue was purified by prep-HPLC to afford *tert*-butyl (*trans*-1-(4-(4-(((2S,6R)-4-(8-cyanoquinolin-5-yl)-6-methylmorpholin-2-yl)methyl)piperazin-1-yl)-6-methylpyrimidin-2-yl)-4-methoxypyrrolidin-3-yl)carbamate product (160 mg). Then it was purified by SFC-HPLC (40% $CO_2$/0.5%$NH_3$ in methanol as eluent on Daicel AD-H Column) to afford *tert*-butyl ((*3S,4R*)-1-(4-(4-(((*2S,6R*)-4-(8-cyanoquinolin-5-yl)-6-methylmorpholin-2-yl)methyl)piperazin-1-yl)-6-methylpyrimidin-2-yl)-4-methoxypyrrolidin-3-yl)carbamate and *tert*-butyl((*3R,4S*)-1-(4-(4-(((*2S,6R*)-4-(8-cyanoquinolin-5-yl)-6-methylmorpholin-2-yl)methyl)piperazin-1-yl)-6-methylpyrimidin-2-yl)-4-methoxypyrrolidin-3-yl)carbamate (compound **69d-A,** faster eluted, 76 mg and compound **69d-B,** slower eluted, 77 mg). MS: calc'd 658 ($MH^+$), measured 658 ($MH^+$).

**Step 3 : preparation of Example 69A and 69B**

To a solution of *tert*-butyl ((3S,4S)-1-(4-(4-(((2S,6R)-4-(8-cyanoquinolin-5-yl)-6-methylmorpholin-2-yl)methyl)piperazin-1-yl)-6-methylpyrimidin-2-yl)-4-methoxypyrrolidin-3-yl)carbamate or *tert*-butyl ((*3R,4R*)-1-(4-(4-(((2S,6R)-4-(8-cyanoquinolin-5-yl)-6-methylmorpholin-2-yl)methyl)piperazin-1-yl)-6-methylpyrimidin-2-yl)-4-methoxypyrrolidin-3-yl)carbamate (compound **69d-A,** 76 mg or compound **69d-B,** 77 mg) in DCM (2 mL) was added hydrogen chloride (4 M in dioxane, 0.5 mL) at 0°C. The reaction mixture was stirred at rt for 2 hrs. Then the mixture was concentrated and purified by prep-HPLC to afford **Example 69A** and **Example 69B: 5-[(2S,6R)-2-[[4-[2-[(3S,4S)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile** and **5-[(2S,6R)-2-[[4-[2-[(3R,4R)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile.**

**Example 69A** (44.2 mg) was obtained as a light yellow solid. MS: calc'd 558 ($MH^+$), measured 558 ($MH^+$). $^1H$ NMR (METHANOL-$d_4$, 400 MHz) δ 9.04 (dd, 1H, *J* = 1.7, 4.3 Hz), 8.77 (dd, 1H, *J* = 1.7, 8.6 Hz), 8.22 (d, 1H, *J* = 7.9 Hz), 7.72 (dd, 1H, *J* = 4.3, 8.6 Hz), 7.33 (d, 1H, *J* = 8.1 Hz), 6.59 (s, 1H), 5.0-5.2 (m, 1H), 4.6-4.7 (m, 1H), 4.3-4.5 (m, 1H), 4.2-4.3 (m, 2H), 3.8-4.2 (m, 8H), 3.6-3.7 (m, 1H), 3.52 (s, 9H), 2.7-2.9 (m, 2H), 2.48 (s, 3H), 1.35 (d, 3H, *J* = 6.2 Hz).

**Example 69B** (48 mg) was obtained as a light yellow solid. MS: calc'd 558 ($MH^+$), measured 558 ($MH^+$). $^1H$ NMR (METHANOL-$d_4$, 400 MHz) δ 9.05 (dd, 1H, *J* = 1.6, 4.4 Hz), 8.80 (dd, 1H, *J* = 1.5, 8.6 Hz), 8.23 (d, 1H, *J* = 7.9 Hz), 7.75 (dd, 1H, *J* = 4.4, 8.6 Hz), 7.35 (d, 1H, *J* = 8.1 Hz), 6.59 (s, 1H), 5.0-5.2 (m, 1H), 4.61 (br t, 1H, *J* = 9.7 Hz), 4.3-4.5 (m, 1H), 4.2-4.3 (m, 2H), 4.0-4.2 (m, 3H), 3.7-4.0 (m, 5H), 3.4-3.5 (m, 9H), 2.81 (ddd, 2H, *J* = 10.0, 12.1, 17.5 Hz), 2.48 (s, 3H), 1.35 (d, 4H, *J* = 6.2 Hz).

**Example 70**

**5-[(2*S*,6*R*)-2-[[4-[2-[[(3*R*,4*R*)-4-methoxypyrrolidin-3-yl]amino]-6-methyl-pyrimidin-4-yl]piperazin-1-yl]methyl]-6-methyl-morpholin-4-yl]quinoline-8-carbonitrile**

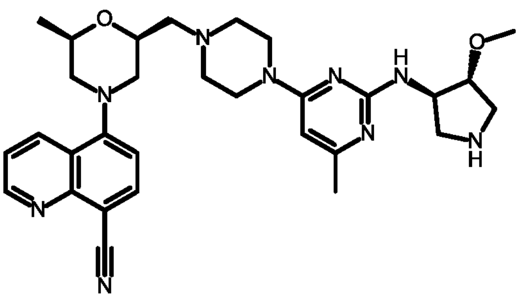

The title compound was prepared in analogy to the preparation of **Example 47** by using *tert*-butyl (3R,4R)-3-amino-4-methoxy-pyrrolidine-1-carboxylate (CAS: 1400562-12-0) and 2,4-dichloro-6-methylpyrimidine instead of 2,4-dichloro-5-fluoropyrimidine (compound **45d**) and *tert*-butyl piperazine-1-carboxylate (compound **45a**). **Example 70** (19.5 mg) was obtained as an orange solid. MS: calc'd 558 ($MH^+$), measured 558 ($MH^+$). $^1H$ NMR (METHANOL-$d_4$, 400 MHz) δ 8.8-8.9 (m, 1H), 8.55 (dd, 1H, *J* = 1.2, 8.6 Hz), 8.05 (td, 1H, *J* = 2.1, 7.9 Hz), 7.54 (br dd, 1H, *J* = 4.3, 8.6 Hz), 7.1-7.2 (m, 1H), 6.29 (s, 1H), 4.6-4.6 (m, 1H), 4.1-4.3 (m, 1H), 4.0-4.1 (m, 1H), 3.8-3.9 (m, 4H), 3.6-3.7 (m, 1H), 3.4-3.4 (m, 1H), 3.4-3.4 (m, 3H), 3.3-3.3 (m, 1H), 3.3-3.3 (m, 1H), 3.2-3.2 (m, 5H), 2.8-2.9 (m, 4H), 2.5-2.7 (m, 2H), 2.23 (s, 3H), 1.17 (d, 3H, *J* = 6.2 Hz).

**Example 71**

**5-[(2S,6R)-2-[[4-[2-[[(3R,4S)-4-fluoropyrrolidin-3-yl]amino]-6-methyl-pyrimidin-4-yl]piperazin-1-yl]methyl]-6-methyl-morpholin-4-yl]quinoline-8-carbonitrile**

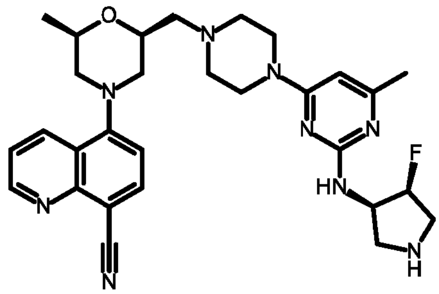

The title compound was prepared in analogy to the preparation of **Example 45** by using *tert*-butyl (*3R*,*4S*)-3-amino-4-fluoro-pyrrolidine-1-carboxylate (Pharmablock, PB07375, CAS: 1009075-48-2) and 2,4-dichloro-6-methylpyrimidine instead of 2,4-dichloro-5-fluoropyrimidine (compound **45d**) and *tert*-butyl piperazine-1-carboxylate (compound **45a**). **Example 71** (10.7 mg) was obtained as an orange solid. MS: calc'd 546 ($MH^+$), measured 546 ($MH^+$). $^1H$ NMR (METHANOL-$d_4$, 400 MHz) δ 8.99 (dd, 1H, *J* = 1.6, 4.3 Hz), 8.66 (dd, 1H, *J* = 1.7, 8.6 Hz), 8.17 (d, 1H, *J* = 8.1 Hz), 7.65 (dd, 1H, *J* = 4.2, 8.6 Hz), 7.28 (d, 1H, *J* = 8.1 Hz), 6.49 (s, 1H), 5.3-5.5 (m, 1H), 5.0-5.1 (m, 3H), 4.4-4.5 (m, 1H), 4.0-4.2 (m, 4H), 3.79 (s, 3H), 3.3-3.5 (m, 6H), 3.2-3.3 (m, 2H), 2.7-2.8 (m, 2H), 2.38 (s, 3H), 1.30 (d, 3H, *J* = 6.2 Hz).

## Example 72

**5-[(2*S*,6*R*)-2-[[4-[2-[(*3S*,*4R*)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile**

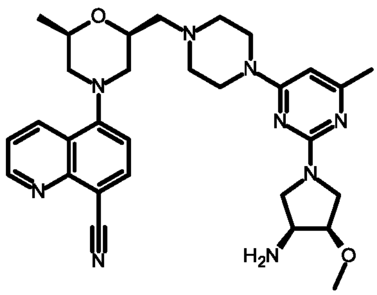

The title compound was prepared in analogy to the preparation of **Example 45** by using *tert*-butyl *N*-[(*3S*,*4R*)-4-methoxypyrrolidin-3-yl]carbamate (PharmaBlock, PBZ4730, CAS: 1931911-57-7) and 2,4-dichloro-6-methylpyrimidine instead of 2,4-dichloro-5-fluoropyrimidine (compound **45d**) and *tert*-butyl piperazine-1-carboxylate (compound **45a**). **Example 72** (31.3 mg) was obtained as an orange solid. MS: calc'd 558 ($MH^+$), measured 558 ($MH^+$). $^1H$ NMR (METHANOL-$d_4$, 400 MHz) δ 8.99 (dd, 1H, *J* = 1.7, 4.2 Hz), 8.66 (dd, 1H, *J* = 1.6, 8.6 Hz), 8.16 (d, 1H, *J* = 8.1 Hz), 7.65 (dd, 1H, *J* = 4.3, 8.6 Hz), 7.28 (d, 1H, *J* = 8.1 Hz), 6.53 (s, 1H), 4.5-4.6 (m, 1H), 3.9-4.4 (m, 9H), 3.7-3.8 (m, 2H), 3.5-3.7 (m, 4H), 3.51 (s, 3H), 3.4-3.5 (m, 4H), 2.7-2.8 (m, 2H), 2.43 (s, 3H), 1.31 (d, 3H, *J* = 6.2 Hz).

## Example 73

**5-[(2*S*,6*R*)-2-[[4-[2-[[(3*S*,4*R*)-4-fluoropyrrolidin-3-yl]amino]-6-methyl-pyrimidin-4-yl]piperazin-1-yl]methyl]-6-methyl-morpholin-4-yl]quinoline-8-carbonitrile**

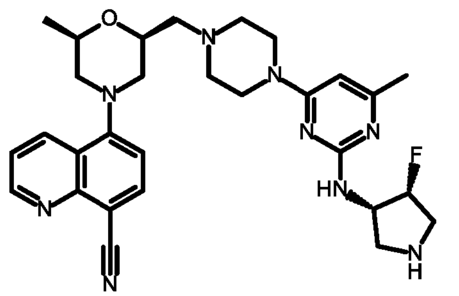

The title compound was prepared in analogy to the preparation of **Example 45** by using *tert*-butyl (*3S,4R*)-3-amino-4-fluoro-pyrrolidine-1-carboxylate and 2,4-dichloro-6-methylpyrimidine instead of 2,4-dichloro-5-fluoropyrimidine (compound **45d**) and *tert*-butyl piperazine-1-carboxylate (compound **45a**). **Example 73** (9.5 mg) was obtained as an orange solid. MS: calc'd 546 ($MH^+$), measured 546 (MH+).$^1$H NMR (METHANOL-$d_4$, 400 MHz) $\delta$ 8.99 (dd, 1H, *J* = 1.7, 4.2 Hz), 8.65 (dd, 1H, *J* = 1.7, 8.6 Hz), 8.16 (d, 1H, *J* = 8.1 Hz), 7.65 (dd, 1H, *J* = 4.3, 8.6 Hz), 7.28 (d, 1H, *J* = 8.1 Hz), 6.49 (s, 1H), 5.3-5.5 (m, 1H), 4.4-4.5 (m, 1H), 4.0-4.2 (m, 4H), 3.6-3.9 (m, 4H), 3.3-3.5 (m, 8H), 3.23 (br d, 2H, *J* = 5.6 Hz), 2.7-2.9 (m, 2H), 2.38 (s, 3H), 1.30 (d, 3H, *J* = 6.2 Hz).

**Example 74**

**5-[(2*R*,6*S*)-2-methyl-6-[[4-[6-methyl-2-[*cis*-3-amino-5-fluoro-1-piperidyl]pyrimidin-4-yl]piperazin-1-yl]methyl]morpholin-4-yl]quinoline-8-carbonitrile**

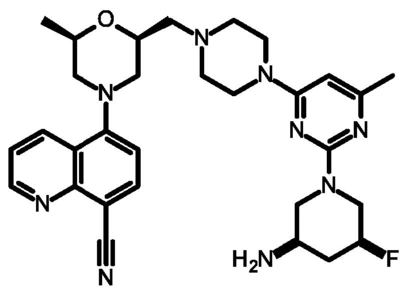

The title compound was prepared in analogy to the preparation of **Example 45** by using *tert*-butyl *N*-(*cis*-5-fluoropiperidin-3-yl)carbamate (CAS:1363378-08-8) and 2,4-dichloro-6-methylpyrimidine instead of 2,4-dichloro-5-fluoropyrimidine (compound **45d**) and *tert*-butyl piperazine-1-carboxylate (compound **45a**). **Example 74** (21.5 mg) was obtained as a light yellow solid. MS: calc'd 560 ($MH^+$), measured 560 (MH+).$^1$H NMR (METHANOL-$d_4$, 400 MHz) $\delta$ 8.99 (dd, 1H, *J* = 1.6, 4.3 Hz), 8.66 (dd, 1H, *J* = 1.6, 8.6 Hz), 8.16 (d, 1H, *J* = 7.9 Hz), 7.64 (dd, 1H, *J* = 4.3, 8.6 Hz), 7.28 (d, 1H, *J* = 8.1 Hz), 6.55 (s, 1H), 5.0-5.2 (m, 1H), 4.8-4.8 (m, 2H), 4.5-4.7 (m, 2H), 4.1-4.3 (m, 4H), 3.5-3.8 (m, 7H), 3.4-3.5 (m, 4H), 2.7-2.8 (m, 2H), 2.44 (s, 3H), 2.3-2.4 (m, 2H), 1.31 (d, 3H, *J* = 6.4 Hz).

**Example 75**

**5-[(2*R*,6*S*)-2-methyl-6-[[4-[6-methyl-2-[*trans*-3-amino-5-fluoro-1-piperidyl]pyrimidin-4-yl]piperazin-1-yl]methyl]morpholin-4-yl]quinoline-8-carbonitrile**

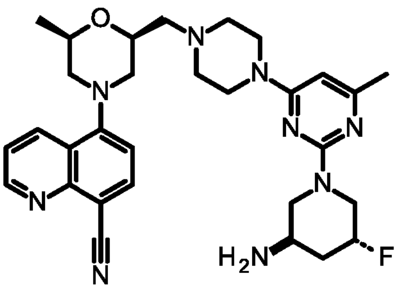

The title compound was prepared in analogy to the preparation of **Example 45** by using *tert*-butyl *N*-(trans-5-fluoropiperidin-3-yl)carbamate and 2,4-dichloro-6-methylpyrimidine instead of 2,4-dichloro-5-fluoropyrimidine (compound **45d**) and *tert*-butyl piperazine-1-carboxylate (compound **45a**). **Example 75** (9.8 mg) was obtained as a light yellow solid. MS: calc'd 560 ($MH^+$), measured 560 (MH+).$^1$H NMR (METHANOL-$d_4$, 400 MHz) δ 8.99 (dd, 1H, *J* = 1.5, 4.2 Hz), 8.66 (dd, 1H, *J* = 1.5, 8.6 Hz), 8.16 (d, 1H, *J* = 8.1 Hz), 7.65 (dd, 1H, *J* = 4.3, 8.6 Hz), 7.28 (d, 1H, *J* = 8.1 Hz), 6.54 (s, 1H), 5.0-5.2 (m, 2H), 4.6-4.7 (m, 1H), 4.5-4.6 (m, 1H), 4.1-4.4 (m, 4H), 3.41 (s, 11H), 3.2-3.3 (m, 1H), 2.7-2.8 (m, 2H), 2.5-2.6 (m, 1H), 2.43 (s, 3H), 1.9-2.1 (m, 1H), 1.31 (d, 3H, *J* = 6.2 Hz).

## Example 76

**5-[(2*S*,6*R*)-2-[[4-[2-[[(3*R*,4*R*)-4-fluoropyrrolidin-3-yl]amino]-6-methyl-pyrimidin-4-yl]piperazin-1-yl]methyl]-6-methyl-morpholin-4-yl]quinoline-8-carbonitrile**

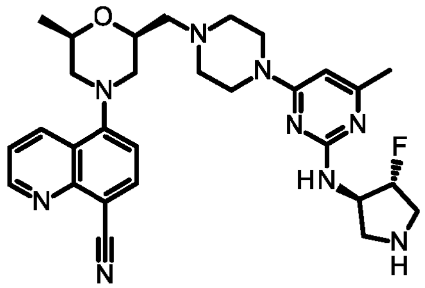

The title compound was prepared in analogy to the preparation of **Example 45** by using *tert*-butyl *(3R,4R)*-3-amino-4-fluoropyrrolidine-1-carboxylate (CAS: 1441392-27-3) and 2,4-dichloro-6-methylpyrimidine instead of 2,4-dichloro-5-fluoropyrimidine (compound **45d**) and *tert*-butyl piperazine-1-carboxylate (compound **45a**). **Example 76** (14.6 mg) was obtained as a light yellow solid. MS: calc'd 546 ($MH^+$), measured 546 (MH+).$^1$H NMR (METHANOL-$d_4$, 400 MHz) δ 8.99 (dd, 1H, *J* = 1.7, 4.2 Hz), 8.65 (dd, 1H, *J* = 1.7, 8.6 Hz), 8.15 (d, 1H, *J* = 7.9 Hz), 7.64 (dd, 1H, *J* = 4.3, 8.6 Hz), 7.27 (d, 1H, *J* = 8.1 Hz), 6.53 (s, 1H), 5.3-5.5 (m, 1H), 4.92 (br s, 1H), 4.5-4.6 (m, 1H), 4.0-4.4 (m, 4H), 3.9-3.9 (m, 1H), 3.5-3.8 (m, 7H), 3.4-3.5 (m, 4H), 3.2-3.3 (m, 1H), 2.7-2.9 (m, 2H), 2.40 (s, 3H), 1.3-1.3 (m, 3H).

## Example 77

**5-[(2*S*,6*R*)-2-[[4-[2-[(3*S*,S*R*)-3-amino-5-fluoro-1-piperidyl]-6-methyl-pyrimidin-4-yl]piperazin-1-yl]methyl]-6-methyl-morpholin-4-yl]quinoline-8-carbonitrile**

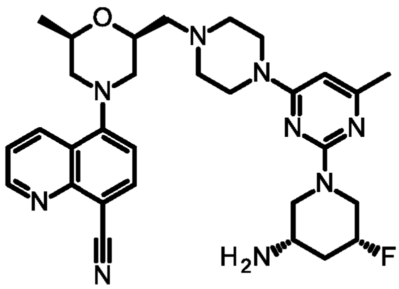

The title compound was prepared in analogy to the preparation of **Example 45** by using *tert*-butyl *N*-[(*3S,5R*)-5-fluoropiperidin-3-yl]carbamate (PharmaBlock, PBZS8178, CAS:1405128-38-2) and 2,4-dichloro-6-methylpyrimidine instead of 2,4-dichloro-5-fluoropyrimidine (compound **45d**) and *tert*-butyl piperazine-1-carboxylate (compound **45a**). **Example 77** (12.8 mg) was obtained as a light yellow solid. MS: calc'd 560 ($MH^+$), measured 560 (MH+).$^1$H NMR (METHANOL-$d_4$, 400 MHz) $\delta$ 8.99 (dd, 1H, *J* = 1.7, 4.2 Hz), 8.66 (dd, 1H, *J* = 1.2, 8.6 Hz), 8.16 (d, 1H, *J* = 8.1 Hz), 7.65 (dd, 1H, *J* = 4.3, 8.7 Hz), 7.28 (d, 1H, *J* =7.9 Hz), 6.57 (d, 1H, *J* = 19.6 Hz), 5.0-5.2 (m, 1H), 4.9-4.9 (m, 1H), 4.7-4.8 (m, 1H), 4.5-4.6 (m, 2H), 4.1-4.4 (m, 4H), 3.5-3.8 (m, 7H), 3.4-3.5 (m, 4H), 2.7-2.8 (m, 2H), 2.4-2.4 (m, 3H), 2.3-2.4 (m, 2H), 1.31 (d, 3H, *J* = 6.2 Hz).

### Example 78

**5-[(2*S*,6*R*)-2-[[4-[2-[(3*S*,4*R*)-4-amino-3-fluoro-1-piperidyl]-6-methyl-pyrimidin-4-yl]piperazin-1-yl]methyl]-6-methyl-morpholin-4-yl]quinoline-8-carbonitrile**

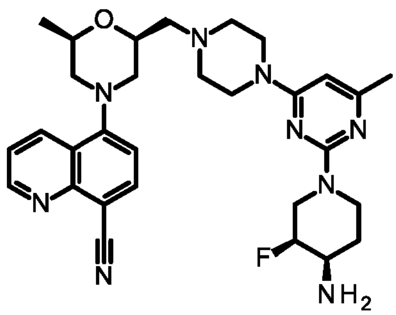

The title compound was prepared in analogy to the preparation of **Example 45** by using *tert*-butyl *N*-[(*3S*,*4R*)-3-fluoropiperidin-4-yl]carbamate and 2,4-dichloro-6-methylpyrimidine instead of 2,4-dichloro-5-fluoropyrimidine (compound **45d**) and *tert*-butyl piperazine-1-carboxylate (compound **45a**). **Example 78** (17.4 mg) was obtained as a light yellow solid. MS: calc'd 560 ($MH^+$), measured 560 (MH+).$^1$H NMR (METHANOL-$d_4$, 400 MHz) $\delta$ 8.99 (dd, 1H, *J* = 1.5, 4.2 Hz), 8.66 (dd, 1H, *J* = 1.6, 8.6 Hz), 8.16 (d, 1H, *J* = 7.9 Hz), 7.65 (dd, 1H, *J* = 4.3, 8.7 Hz), 7.28 (d, 1H, *J* = 8.1 Hz), 6.52 (s, 1H), 4.9-5.0 (m, 1H), 4.7-4.8 (m, 1H), 4.5-4.7 (m, 3H), 4.1-4.3 (m, 4H), 3.5-3.7 (m, 5H), 3.42 (br d, 4H, *J* = 11.9 Hz), 3.2-3.3 (m, 2H), 2.7-2.8 (m, 2H), 2.41 (s, 3H), 2.2-2.3 (m, 1H), 1.82 (br s, 1H), 1.31 (d, 3H, *J* = 6.2 Hz).

### Example 79

**5-[(2*S*, 6*R*)-2-[[4-[2-[(3*R*,4*R*)-3-amino-4-fluoro-pyrrolidin-1-yl]-6-methyl-pyrimidin-4-yl]piperazin-1-yl]methyl]-6-methyl-morpholin-4-yl]quinoline-8-carbonitrile**

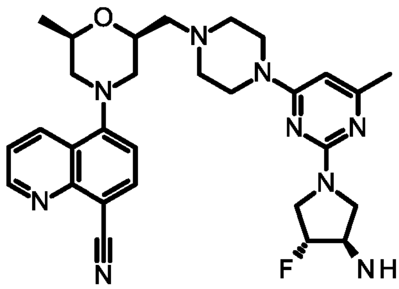

The title compound was prepared in analogy to the preparation of **Example 45** by using *tert*-butyl *N*-[(3*R*,4*R*)-4-fluoropyrrolidin-3-yl)]carbamate hydrochloride (CAS: 2097061-04-4) and 2,4-dichloro-6-methylpyrimidine instead of 2,4-dichloro-5-fluoropyrimidine (compound **45d**) and *tert*-butyl piperazine-1-carboxylate (compound **45a**). **Example 79** (39.1 mg) was obtained as a light yellow solid. MS: calc'd 546 ($MH^+$), measured 546 (MH+).$^1$H NMR (METHANOL-$d_4$, 400 MHz) $\delta$ 9.01 (dd, 1H, *J* = 1.6, 4.3 Hz), 8.68 (dd, 1H, *J* = 1.6, 8.6 Hz), 8.18 (d, 1H, *J* = 8.1 Hz), 7.67 (dd, 1H, *J* = 4.3, 8.6 Hz), 7.30 (d, 1H, *J* = 8.1 Hz), 6.57 (s, 1H), 5.4-5.6 (m, 1H), 4.5-4.6 (m, 1H), 4.0-4.3 (m, 10H), 3.5-3.6 (m, 4H), 3.4-3.5 (m, 4H), 2.7-2.9 (m, 2H), 2.45 (s, 3H), 1.33 (d, 3H, *J* = 6.2 Hz).

**Example 80**

**5-[(2S,6R)-2-[[4-[2-(5-amino-3,3-difluoro-1-piperidyl)-6-methyl-pyrimidin-4-yl]piperazin-1-yl]methyl]-6-methyl-morpholin-4-yl]quinoline-8-carbonitrile**

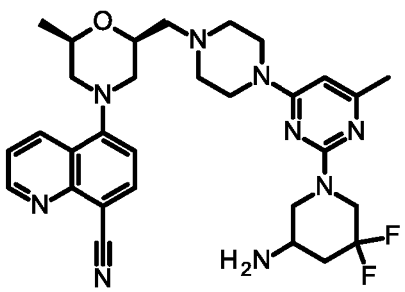

The title compound was prepared in analogy to the preparation of **Example 45** by using *tert*-butyl N-(5,5-difluoropiperidin-3-yl)carbamate and 2,4-dichloro-6-methylpyrimidine instead of 2,4-dichloro-5-fluoropyrimidine (compound **45d**) and *tert*-butyl piperazine-1-carboxylate (compound **45a**). **Example 80** (14.3 mg) was obtained as a light yellow solid. MS: calc'd 578 ($MH^+$), measured 578 (MH+).$^1$H NMR (METHANOL-$d_4$, 400 MHz) $\delta$ 9.00 (dd, 1H, *J* = 1.7, 4.2 Hz), 8.66 (dd, 1H, *J* = 1.6, 8.6 Hz), 8.17 (d, 1H, *J* = 8.1 Hz), 7.65 (dd, 1H, *J* = 4.2, 8.6 Hz), 7.29 (d, 1H, *J* = 8.1 Hz), 6.39 (s, 1H), 4.4-4.6 (m, 3H), 4.3-4.4 (m, 1H), 4.1-4.2 (m, 2H), 4.0-4.1 (m, 3H), 3.88 (br dd, 1H, *J* = 6.2, 7.9 Hz), 3.66 (br d, 1H, *J* = 2.7 Hz), 3.39 (br d, 8H, *J* = 7.2 Hz), 2.7-2.8 (m, 2H), 2.5-2.7 (m, 1H), 2.3-2.4 (m, 4H), 1.32 (d, 3H, *J* = 6.2 Hz).

**Example 81**

**5-[(2*S*,6*R*)-2-[[4-[2-[3-(1-hydroxy-1-methyl-ethyl)piperazin-1-yl]-6-methyl-pyrimidin-4-yl]piperazin-1-yl]methyl]-6-methyl-morpholin-4-yl]quinoline-8-carbonitrile**

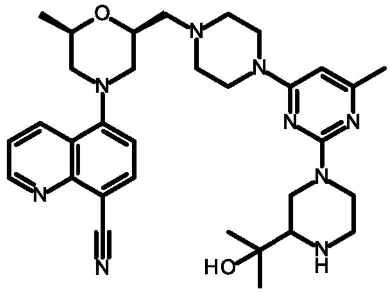

The title compound was prepared in analogy to the preparation of **Example 45** by using 2-(piperazin-2-yl)propan-2-ol and 2,4-dichloro-6-methylpyrimidine instead of 2,4-dichloro-5-fluoropyrimidine (compound **45d**) and *tert*-butyl piperazine-1-carboxylate (compound **45a**). **Example 81** (15.2 mg) was obtained as a light yellow solid. MS: calc'd 586 ($MH^+$), measured 586 (MH+).$^1$H NMR (METHANOL-$d_4$, 400 MHz) δ 8.99 (dd, 1H, *J* = 1.7, 4.2 Hz), 8.65 (dd, 1H, *J* = 1.7, 8.6 Hz), 8.15 (d, 1H, *J* = 7.9 Hz), 7.64 (dd, 1H, *J* = 4.3, 8.6 Hz), 7.27 (d, 1H, *J* = 8.1 Hz), 6.53 (s, 1H), 5.3-5.5 (m, 1H), 4.92 (br s, 1H), 4.5-4.6 (m, 1H), 4.0-4.4 (m, 4H), 3.9-3.9 (m, 1H), 3.5-3.8 (m, 7H), 3.4-3.5 (m, 4H), 3.2-3.3 (m, 1H), 2.7-2.9 (m, 2H), 2.40 (s, 3H), 1.3-1.3 (m, 3H).

**Example 82**

**5-[(*2S*,*6R*)-2-[[4-[2-[(*3R*,*4R*)-4-amino-3-fluoro-1-piperidyl]-6-methyl-pyrimidin-4-yl]piperazin-1-yl]methyl]-6-methyl-morpholin-4-yl]quinoline-8-carbonitrile**

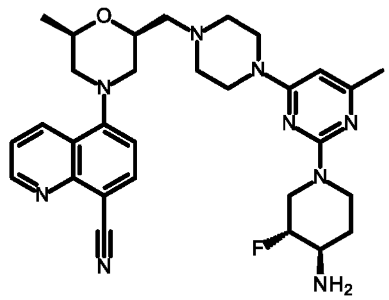

The title compound was prepared in analogy to the preparation of **Example 45** by using *tert*-butyl *N*-[(*3R,4R*)-3-fluoropiperidin-4-yl]carbamate (PharmaBlock, PB08067, CAS: 1523530-29-1) and 2,4-dichloro-6-methylpyrimidine instead of 2,4-dichloro-5-fluoropyrimidine (compound **45d**) and *tert*-butyl piperazine-1-carboxylate (compound **45a**). **Example 82** (9.5 mg) was obtained as a light yellow solid. MS: calc'd 560 ($MH^+$), measured 560 (MH+).$^1$H NMR (METHANOL-$d_4$, 400 MHz) δ 9.0-9.0 (m, 1H), 8.68 (dd, 1H, *J* = 1.2, 8.6 Hz), 8.18 (d, 1H, *J* = 8.1 Hz), 7.67 (dd, 1H, *J* = 4.2, 8.5 Hz), 7.30 (d, 1H, *J* = 8.1 Hz), 6.54 (s, 1H), 5.0-5.2 (m, 3H), 4.7-4.8 (m, 2H), 4.5-4.6 (m, 1H), 4.19 (br d, 4H, *J* = 6.6 Hz), 3.7-3.8 (m, 1H), 3.5-3.6 (m, 4H), 3.4-3.5 (m, 5H), 2.7-2.9 (m, 2H), 2.44 (s, 3H), 2.0-2.1 (m, 2H), 1.33 (d, 3H, *J* = 6.2 Hz).

**Example 83**

**5-[(*2R*,*6S*)-2-methyl-6-[[4-[6-methyl-2-[(*3S*,*4R*)-3-amino-4-fluoro-1-piperidyl]-pyrimidin-4-yl]piperazin-1-yl]methyl]morpholin-4-yl]quinoline-8-carbonitrile**

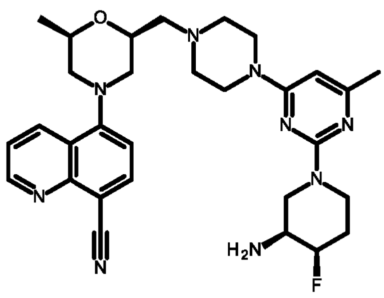

The title compound was prepared in analogy to the preparation of **Example 45** by using *tert*-butyl *N*-[*cis*-4-fluoropiperidin-3-yl]carbamate and 2,4-dichloro-6-methylpyrimidine instead of 2,4-dichloro-5-fluoropyrimidine (compound **45d**) and *tert*-butyl piperazine-1-carboxylate (compound **45a**). **Example 83** (7.2 mg) was obtained as a light yellow solid. MS: calc'd 560 ($MH^+$), measured 560 (MH+).$^1$H NMR (METHANOL-d4, 400 MHz) $\delta$ 9.01 (dd, 1H, *J* = 1.7, 4.2 Hz), 8.68 (dd, 1H, *J* = 1.5, 8.6 Hz), 8.18 (d, 1H, *J* = 8.1 Hz), 7.67 (dd, 1H, *J* = 4.3, 8.6 Hz), 7.30 (d, 1H, *J* = 7.9 Hz), 6.53 (s, 1H), 5.1-5.3 (m, 1H), 4.72 (br d, 1H, *J* = 9.4 Hz), 4.5-4.6 (m, 1H), 4.0-4.4 (m, 6H), 3.5-3.8 (m, 7H), 3.4-3.5 (m, 4H), 2.7-2.9 (m, 2H), 2.4-2.5 (m, 3H), 2.2-2.3 (m, 1H), 2.0-2.2 (m, 1H), 1.34 (d, 3H, *J* = 6.2 Hz).

## Example 84

**5-[(*2R*,*6S*)-2-methyl-6-[[4-[6-methyl-2-[(*3R*,*4R*)-3-amino-4-fluoro-1-piperidyl]pyrimidin-4-yl]piperazin-1-yl]methyl]morpholin-4-yl]quinoline-8-carbonitrile**

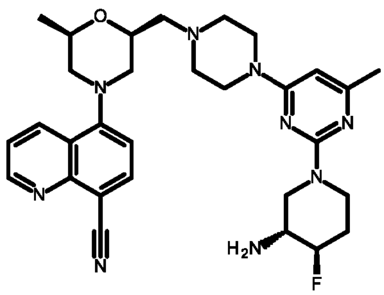

The title compound was prepared in analogy to the preparation of **Example 45** by using *tert*-butyl *N*-[*trans*-4-fluoropiperidin-3-yl]carbamate (CAS: 1052713-46-8) and 2,4-dichloro-6-methylpyrimidine instead of 2,4-dichloro-5-fluoropyrimidine (compound 45d) and *tert*-butyl piperazine-1-carboxylate (compound **45a**). **Example 84** (21.8 mg) was obtained as a light yellow solid. MS: calc'd 560 ($MH^+$), measured 560 ($MH^+$). $^1$H NMR (METHANOL-d4, 400 MHz) $\delta$ 9.01 (dd, 1H, *J* = 1.6, 4.3 Hz), 8.68 (dd, 1H, *J* = 1.5, 8.6 Hz), 8.18 (d, 1H, *J* = 8.1 Hz), 7.67 (dd, 1H, *J* = 4.3, 8.6 Hz), 7.30 (d, 1H, *J* = 8.1 Hz), 6.53 (s, 1H), 4.9-5.0 (m, 1H), 4.7-4.8 (m, 1H), 4.5-4.6 (m, 1H), 4.4-4.5 (m, 1H), 3.9-4.3 (m, 5H), 3.5-3.7 (m, 5H), 3.4-3.5 (m, 6H), 2.7-2.9 (m, 2H), 2.44 (s, 3H), 2.3-2.4 (m, 1H), 1.8-2.0 (m, 1H), 1.34 (d, 3H, *J* = 6.2 Hz).

## Example 85

**5-[(*2S,6R*)-2-[[4-[2-(azetidin-3-ylamino)-6-methyl-pyrimidin-4-yl]piperazin-1-yl]methyl]-6-methyl-morpholin-4-yl]quinoline-8-carbonitrile**

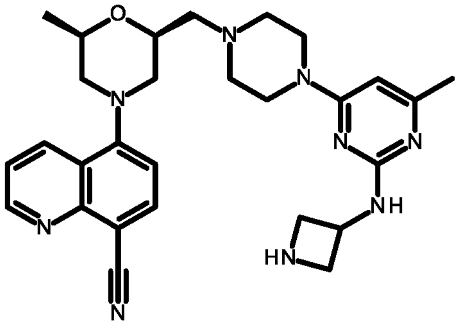

The title compound was prepared in analogy to the preparation of **Example 45** by using 1-BOC-3-(amino)azetidine and 2,4-dichloro-6-methylpyrimidine instead of 2,4-dichloro-5-fluoropyrimidine (compound **45d**) and tert-butyl piperazine-1-carboxylate (compound **45a**). **Example 85** (19.2 mg) was obtained as a light yellow solid. MS: calc'd 514 ($MH^+$), measured 514 ($MH^+$). $^1H$ NMR (METHANOL-$d_4$, 400 MHz) δ 9.01 (dd, 1H, *J* = 1.6, 4.2 Hz), 8.67 (dd, 1H, *J* = 1.6, 8.6 Hz), 8.17 (d, 1H, *J* = 7.9 Hz), 7.66 (dd, 1H, *J* = 4.3, 8.6 Hz), 7.29 (d, 1H, *J = 8.1* Hz), 6.52 (s, 1H), 5.08 (quin, 1H, *J* = 7.6 Hz), 4.5-4.6 (m, 1H), 4.4-4.5 (m, 2H), 3.8-4.4 (m, 7H), 3.60 (br s, 4H), 3.4-3.5 (m, 4H), 2.7-2.8 (m, 2H), 2.41 (s, 3H), 1.33 (d, 3H, *J* = 6.2 Hz).

**Example 86**

**5-[(*2S,6R*)-2-[[4-[2-(4-amino-3,3-difluoro-1-piperidyl)-6-methyl-pyrimidin-4-yl]piperazin-1-yl]methyl]-6-methyl-morpholin-4-yl]quinoline-8-carbonitrile**

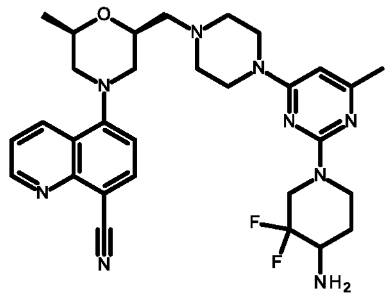

The title compound was prepared in analogy to the preparation of **Example 45** by using 4-(Boc-amino)-3,3-difluoropiperidine (CAS: 1263180-22-8) and 2,4-dichloro-6-methylpyrimidine instead of 2,4-dichloro-5-fluoropyrimidine (compound **45d**) and tert-butyl piperazine-1-carboxylate (compound **45a**). **Example 86** (8.3 mg) was obtained as a light yellow solid. MS: calc'd 578 ($MH^+$), measured 578 ($MH^+$). $^1H$ NMR (METHANOL-$d_4$, 400 MHz) δ 9.01 (br d, 1H, *J* = 3.2 Hz), 8.6-8.7 (m, 1H), 8.19 (d, 1H, *J* = 8.1 Hz), 7.67 (dd, 1H, *J* = 4.1, 8.5 Hz), 7.30 (d, 1H, *J* = 8.1 Hz), 6.51 (s, 1H), 5.1-5.2 (m, 1H), 4.7-4.8 (m, 1H), 4.53 (br s, 1H), 4.0-4.3 (m, 6H), 3.5-3.7 (m, 5H), 3.4-3.5 (m, 5H), 2.78 (td, 2H, *J* = 11.1, 19.1 Hz), 2.42 (s, 3H), 2.3-2.3 (m, 1H), 1.9-2.0 (m, 1H), 1.33 (d, 3H, *J* = 6.1 Hz).

**Example 87**

**5-[(*2S,6R*)-2-[[4-[2-(3-aminoazetidin-1-yl)-6-methyl-pyrimidin-4-yl]piperazin-1-yl]methyl]-6-methyl-morpholin-4-yl]quinoline-8-carbonitrile**

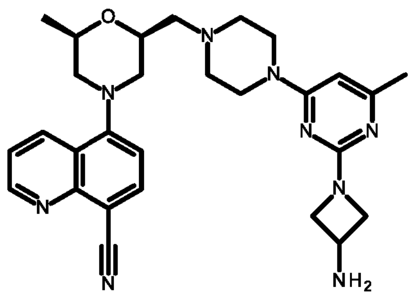

The title compound was prepared in analogy to the preparation of **Example 45** by using 3-*N*-boc-amino-azetidine and 2,4-dichloro-6-methylpyrimidine instead of 2,4-dichloro-5-fluoropyrimidine (compound **45d**) and tert-butyl piperazine-1-carboxylate (compound **45a**). **Example 87** (31 mg) was obtained as a light yellow solid. MS: calc'd 514 ($MH^+$), measured 514 ($MH^+$). $^1H$ NMR (METHANOL-$d_4$, 400 MHz) δ 9.00 (dd, 1H, *J* = 1.3, 4.2 Hz), 8.67 (dd, 1H, *J* = 1.2, 8.6 Hz), 8.17 (d, 1H, *J* = 8.1 Hz), 7.66 (dd, 1H, *J* = 4.3, 8.6 Hz), 7.29 (d, 1H, *J* = 8.1 Hz), 6.53 (s, 1H), 4.64 (br dd, 2H, *J* = 7.2, 10.0 Hz), 4.54 (br t, 1H, *J* = 9.2 Hz), 4.34 (s, 4H), 4.0-4.2 (m, 4H), 3.5-3.7 (m, 4H), 3.4-3.5 (m, 4H), 2.7-2.9 (m, 2H), 2.41 (s, 3H), 1.33 (d, 3H, *J* = 6.2 Hz).

**Example 88**

**5-[(*2S*,*6R*)-2-[[4-[2-[(*3R*,*4S*)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile**

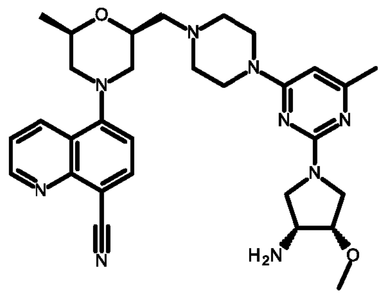

The title compound was prepared in analogy to the preparation of **Example 45** by using *tert*-butyl *N*-[(*3R,4S*)-4-methoxypyrrolidin-3-yl]carbamate (CAS: 1932508-77-4) and 2,4-dichloro-6-methylpyrimidine instead of 2,4-dichloro-5-fluoropyrimidine (compound **45d**) and tert-butyl piperazine-1-carboxylate (compound **45a**). **Example 88** (106.9 mg) was obtained as a light yellow solid. MS: calc'd 558 ($MH^+$), measured 558 ($MH^+$). $^1H$ NMR (METHANOL-$d_4$, 400 MHz) δ 9.01 (dd, 1H, *J* = 1.5, 4.2 Hz), 8.67 (dd, 1H, *J* = 1.4, 8.6 Hz), 8.18 (d, 1H, *J* = 8.1 Hz), 7.67 (dd, 1H, *J* = 4.3, 8.6 Hz), 7.30 (d, 1H, *J* = 8.1 Hz), 6.55 (s, 1H), 4.5-4.6 (m, 1H), 4.13 (br s, 8H), 3.9-4.0 (m, 1H), 3.7-3.9 (m, 2H), 3.6-3.7 (m, 4H), 3.53 (s, 3H), 3.43 (br s, 4H), 2.7-2.9 (m, 2H), 2.45 (s, 3H), 1.33 (d, 3H, *J* = 6.2 Hz).

**Example 89**

**5-[(*2R*,*6S*)-2-methyl-6-[[4-(2-morpholin-2-ylphenyl)piperazin-1-yl]methyl]morpholin-4-yl]quinoline-8-carbonitrile**

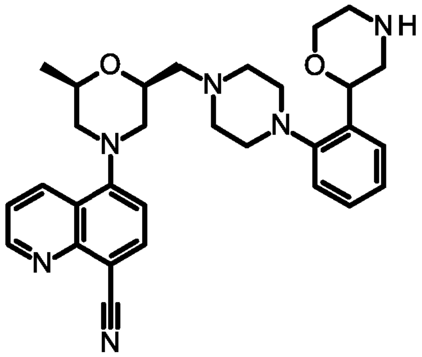

The title compound was prepared in analogy to the preparation of Example **62** by using 2-bromo-1-(2-bromophenyl)ethanone instead of 2-bromo-1-(3-bromophenyl)ethanone (compound **62a**). **Example 89** (14 mg) was obtained as a yellow solid. MS: calc'd 513 ($MH^+$), measured 513 ($MH^+$). $^1H$ NMR (METHANOL-$d_4$, 400 MHz) $\delta$ 9.1-9.2 (m, 2H), 8.37 (d, 1 H, *J* = 8.1 Hz), 7.98 (ddd, 1 H, *J* = 2.1, 5.0, 8.3 Hz), 7.57 (dd, 1 H, *J* = 1.2, 7.7 Hz), 7.47 (d, 1 H, *J* = 8.2 Hz), 7.4-7.4 (m, 1H), 7.4-7.4 (m, 1H), 7.3-7.3 (m, 1H), 5.4-5.5 (m, 1H), 4.6-4.7 (m, 1H), 4.2-4.3 (m, 3H), 3.9-4.0 (m, 1H), 3.7-3.9 (m, 4H), 3.3-3.6 (m, 9H), 3.2-3.3 (m, 1H), 3.1-3.2 (m, 1H), 2.8-3.0 (m, 2H), 1.36 (dd, 3 H, *J* = 1.5, 6.2 Hz).

**Example 90**

**5-[(*2S,6R*)-2-[[4-[2-[(*3S,4S*)-4-amino-3-methoxy-1-piperidyl]-6-methyl-pyrimidin-4-yl]piperazin-1-yl]methyl]-6-methyl-morpholin-4-yl]quinoline-8-carbonitrile**

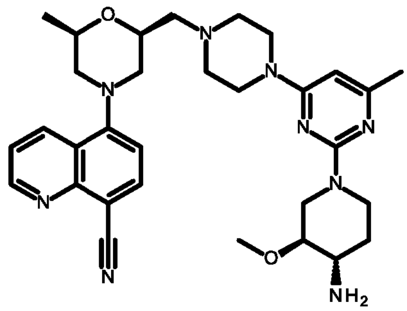

The title compound was prepared in analogy to the preparation of **Example 45** by using *tert*-butyl *N*-[(*3R*,*4R*)-3-methoxypiperidin-4-yl]carbamate hemioxalate (PharmaBlock, PB07428-1, CAS:907544-19-8) and 2,4-dichloro-6-methylpyrimidine instead of 2,4-dichloro-5-fluoropyrimidine (compound **45d**) and *tert*-butyl piperazine-1-carboxylate (compound **45a**). **Example 90** (7 mg) was obtained as a light yellow solid. MS: calc'd 572 ($MH^+$), measured 572 ($MH^+$). $^1H$ NMR (METHANOL-$d_4$, 400 MHz) $\delta$ 8.90 (dd, 1H, *J*=1.7, 4.2 Hz), 8.56 (dd, 1H, *J*=1.7, 8.6 Hz), 8.07 (d, 1H, *J*=8.1 Hz), 7.55 (dd, 1H, *J*=4.3, 8.6 Hz), 7.18 (d, 1H, *J*=8.1 Hz), 6.39 (s, 1H), 4.8-4.9 (m, 1H), 4.8-4.8 (m, 1H), 4.5-4.6 (m, 1H), 4.3-4.4 (m, 1H), 3.9-4.2 (m, 4H), 3.46 (s, 3H), 3.3-3.4 (m, 6H), 3.2-3.3 (m, 3H), 3.1-3.2 (m, 1H), 3.0-3.1 (m, 1H), 2.7-2.9 (m, 1H), 2.6-2.7 (m, 2H), 2.31 (s, 3H), 2.0-2.1 (m, 1H), 1.6-1.7 (m, 1H), 1.21 (d, 3H, J=6.2 Hz).

**Example 91**

**5-[(2S, 6R)-2-[[4-(6-methoxy-2-piperazin-1-yl-pyrimidin-4-yl)piperazin-1-yl]methyl]-6-methyl-morpholin-4-yl]quinoline-8-carbonitrile**

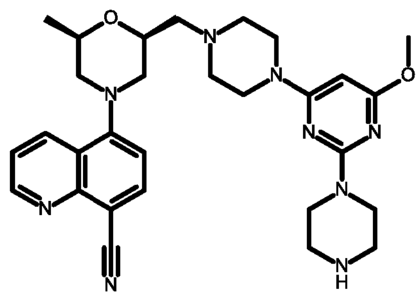

The title compound was prepared in analogy to the preparation of **Example 45** by using 2,4-dichloro-6-methoxypyrimidine instead of 2,4-dichloro-5-fluoropyrimidine (compound **45d**). **Example 91** (58 mg) was obtained as a light yellow solid. MS: calc'd 544 ($MH^+$), measured 544 ($MH^+$). $^1H$ NMR (METHANOL-$d_4$, 400 MHz) δ 9.00 (dd, 1H, *J*=1.6, 4.3 Hz), 8.67 (dd, 1H, *J*=1.7, 8.6 Hz), 8.17 (d, 1H, *J*=7.9 Hz), 7.66 (dd, 1H, *J*=4.3, 8.6 Hz), 7.29 (d, 1H, *J*=8.1 Hz), 4.3-4.8 (m, 3H), 4.1-4.2 (m, 1H), 3.9-4.1 (m, 5H), 3.89 (s, 3H), 3.5-3.8 (m, 3H), 3.4-3.5 (m, 5H), 3.3-3.4 (m, 2H), 3.1-3.3 (m, 4H), 2.77 (ddd, 2H, *J*=10.3, 12.0, 18.6 Hz), 1.33 (d, 3H, *J*=6.2 Hz).

**Example 92**

**5-[(*2S*,*6R*)-2-[[4-[2-[(*3S*,*4R*)-3-amino-4-methoxy-pyrrolidin-1-yl]pyrimidin-4-yl]piperazin-1-yl]methyl]-6-methyl-morpholin-4-yl]quinoline-8-carbonitrile**

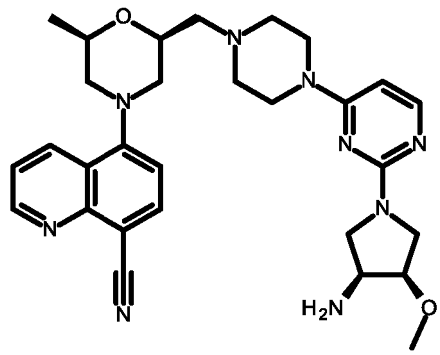

The title compound was prepared in analogy to the preparation of Example **23** by using *tert*-butyl *N*-[(*3S*,*4R*)-4-methoxypyrrolidin-3-yl]carbamate instead of *tert*-butyl azetidin-3-ylcarbamate (compound **23c**). **Example 92** (13 mg) was obtained as a light yellow solid. MS: calc'd 544 ($MH^+$), measured 544 ($MH^+$). $^1H$ NMR (METHANOL-$d_4$, 400 MHz) δ 9.02 (dd, 1H, *J*=1.6, 4.3 Hz), 8.68 (dd, 1H, *J*=1.7, 8.6 Hz), 8.19 (d, 1H, *J*=8.1 Hz), 7.91 (d, 1H, *J*=7.5 Hz), 7.67 (dd, 1H, *J*=4.3, 8.6 Hz), 7.31 (d, 1H, *J*=8.1 Hz), 6.63 (d, 1H, *J*=7.5 Hz), 4.4-4.5 (m, 1H), 3.9-4.4 (m, 9H), 3.8-3.8 (m, 1H), 3.6-3.8 (m, 1H), 3.53 (s, 3H), 3.4-3.5 (m, 6H), 3.37 (br s, 2H), 2.7-2.8 (m, 2H), 1.33 (d, 3H, *J*=6.4 Hz).

**Example 93**

**5-[*(2S,6R)*-2-[[4-[2-[(*3R*,*4S*)-3-amino-4-methoxy-pyrrolidin-1-yl]pyrimidin-4-yl]piperazin-1-yl]methyl]-6-methyl-morpholin-4-yl]quinoline-8-carbonitrile**

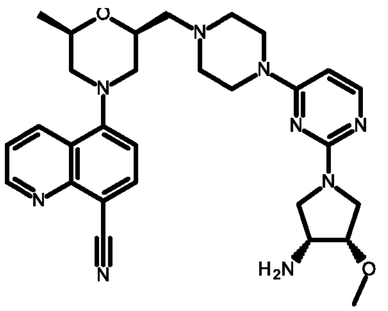

The title compound was prepared in analogy to the preparation of Example **23** by using *tert*-butyl *N*-[(*3R*,*4S*)-4-methoxypyrrolidin-3-yl]carbamate instead of *tert*-butyl azetidin-3-ylcarbamate (compound **23c**). **Example 93** (10 mg) was obtained as a light yellow solid. MS: calc'd 544 ($MH^+$), measured 544 ($MH^+$). $^1H$ NMR (METHANOL-$d_4$, 400 MHz) δ 9.01 (dd, 1H, *J*=1.6, 4.3 Hz), 8.68 (dd, 1H, *J*=1.7, 8.6 Hz), 8.18 (d, 1H, *J*=8.1 Hz), 7.91 (d, 1H, *J*=7.5 Hz), 7.67 (dd, 1H, *J*=4.2, 8.6 Hz), 7.30 (d, 1H, *J*=8.1 Hz), 6.67 (d, 1H, *J*=7.6 Hz), 4.5-4.6 (m, 1H), 3.9-4.5 (m, 9H), 3.8-3.9 (m, 2H), 3.5-3.7 (m, 4H), 3.5-3.5 (m, 3H), 3.4-3.5 (m, 4H), 2.7-2.8 (m, 2H), 1.33 (d, 3H, *J*=6.2 Hz).

**Example 94**

**5-[(*2S,6R*)-2-[[4-(5-fluoro-4-piperazin-1-yl-pyrimidin-2-yl)piperazin-1-yl]methyl]-6-methyl-morpholin-4-yl]quinoline-8-carbonitrile**

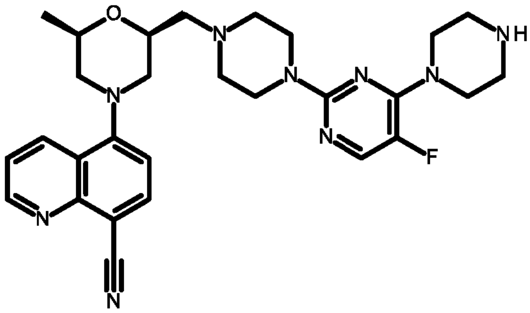

The title compound was prepared according to the following scheme:

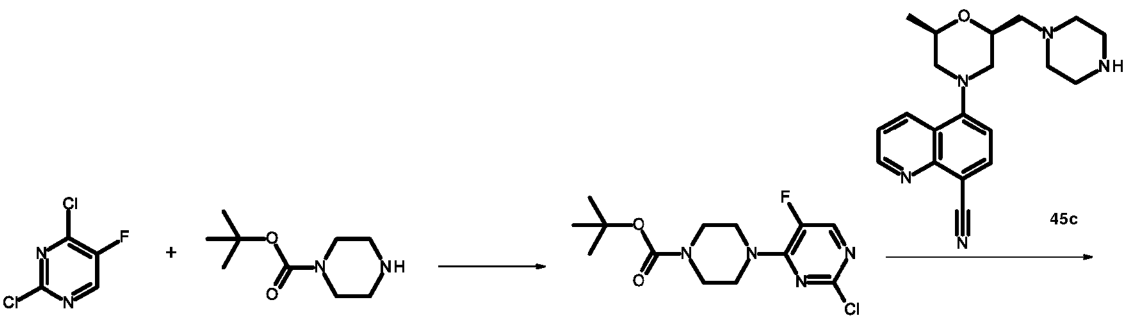

45c

94a

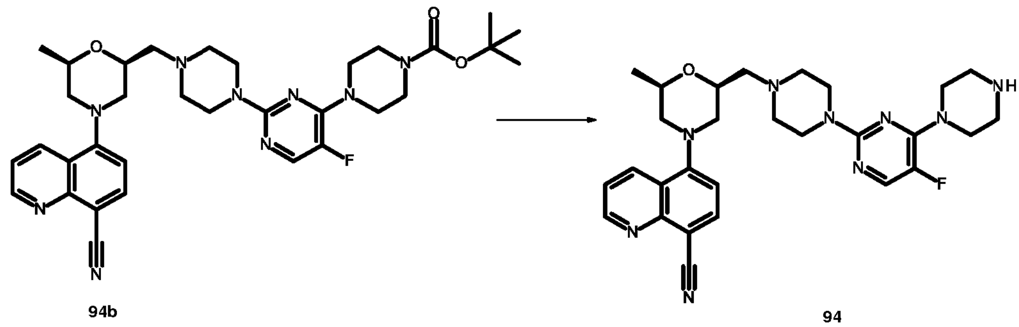

94b

94

**Step 1: preparation of *tert*-butyl 4-(2-chloro-5-fluoropyrimidin-4-yl)piperazine-1-carboxylate (compound 94a)**

A solution of 2,4-dichloro-5-fluoropyrimidine (CAS: 2927-71-1, 334 mg, 2 mmol) and *tert*-butyl piperazine-1-carboxylate (373 mg, 2 mmol) in ethanol (6 mL) was stirred at room temperature for 12 hrs. Then the mixture was concentrated and purified by silica gel chromatography to afford compound **94a** (500 mg) as a white solid. MS: calc'd 317 ($MH^+$), measured 317 ($MH^+$).

**Step 2: preparation of *tert*-butyl 4-(2-(4-(((*2S,6R*)-4-(8-cyanoquinolin-5-yl)-6-methylmorpholin-2-yl)methyl)piperazin-1-yl)-5-fluoropyrimidin-4-yl)piperazine-1-carboxylate (compound 94b)**

A suspension of 5-((*2R,6S*)-2-methyl-6-(piperazin-1-ylmethyl)morphofino)quinoline-8-carbonitrile (compound **45c,** 80 mg, 228 μmol), *tert*-butyl 4-(2-chloro-5-fluoropyrimidin-4-yl)piperazine-1-carboxylate (compound **94a,** 72 mg, 228 μmol), Ruphos Pd G2 (17.7 mg, 22.8 μmol) and $K_2CO_3$ (62 mg, 455 μmol) in 1,4-Dioxane (4 ml) was heated at 100°C for 12 hrs under nitrogen atmoshpere. The mixture was then cooled to room temperature and diluted with water (5 mL) and extracted with EtOAC (10 mL ) three times. The combined organic layer was washed with brine and dried over $Na_2SO_4$. The solution was concentrated and the residue was purified by silica gel chromatography (EA/PE from 20% to 80%) to afford compound **94b** (35 mg) as a pale yellow solid. MS: calc'd 632 ($MH^+$), measured 632 ($MH^+$).

**Step 3: preparation of 5-[(*2S,6R*)-2-[[4-(5-fluoro-4-piperazin-1-yl-pyrimidin-2-yl)piperazin-1-yl]methyl]-6-methyl-morpholin-4-yl]quinoline-8-carbonitrile (Example 94)**

To a solution of *tert*-butyl 4-(2-(4-(((*2S,6R*)-4-(8-cyanoquinolin-5-yl)-6-methylmorpholin-2-yl)methyl)piperazin-1-yl)-5-fluoropyrimidin-4-yl)piperazine-1-carboxylate (compound **94b**, 35 mg, 55.4 μmol) in DCM (2 ml) was added 2,2,2-trifluoroacetic acid (740 mg, 0.5 ml, 6.49 mmol) at 0 °C. The reaction mixture was stirred at r.t. for 2 hrs. Then the mixture was concentrated and purified by prep-HPLC to get 5-[(*2S,6R*)-2-[[4-(5-fluoro-4-piperazin-1-yl-pyrimidin-2-yl)piperazin-1-yl]methyl]-6-methyl-morpholin-4-yl]quinoline-8-carbonitrile (**Example 94,** 10 mg) as an orange solid. MS calc'd 532 ($MH^+$), measured 532 ($MH^+$). $^1H$ NMR (METHANOL-$d_4$, 400 MHz) δ 9.01 (dd, 1H, *J*=1.7, 4.2 Hz), 8.68 (dd, 1H, *J*=1.6, 8.6 Hz), 8.18 (d, 1H, *J*=8.1 Hz), 8.02 (d, 1H, *J*=6.2 Hz), 7.67 (dd, 1H, *J*=4.2, 8.6 Hz), 7.30 (d, 1H, *J*=8.1 Hz), 4.53 (dtd, 1H, *J*=2.7, 5.0, 10.0 Hz), 4.2-4.3 (m, 1H), 4.0-4.1 (m, 4H), 3.4-3.9 (m, 9H), 3.3-3.4 (m, 5H), 3.2-3.3 (m, 1H), 2.78 (ddd, 2H, *J*= 10.3, 12.1, 18.9 Hz), 1.34 (d, 3H, *J*=6.2 Hz).

**Example 95**

**5-[*(2S,6R)*-2-[[4-[2-[(*3S*,*4R*)-3-amino-4-fluoro-pyrrolidin-1-yl]pyrimidin-4-yl]piperazin-1-yl]methyl]-6-methyl-morpholin-4-yl]quinoline-8-carbonitrile**

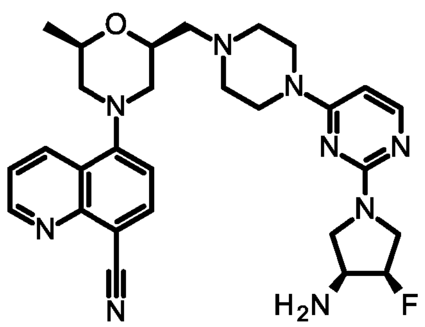

The title compound was prepared in analogy to the preparation of Example **23** by using *tert*-butyl *N*-[(*3S*,*4R*)-4-fluoropyrrolidin-3-yl]carbamate instead of *tert*-butyl azetidin-3-ylcarbamate (compound **23c**). **Example 95** (14 mg) was obtained as a light yellow solid. MS: calc'd 532 ($MH^+$), measured 532 ($MH^+$). $^1H$ NMR (METHANOL-$d_4$, 400 MHz) δ 9.02 (dd, 1H, *J*=1.7, 4.2 Hz), 8.68 (dd, 1H, *J*=1.7, 8.6 Hz), 8.19 (d, 1H, *J*=7.9 Hz), 7.94 (d, 1H, *J*=7.5 Hz), 7.67 (dd, 1H, *J*=4.2, 8.6 Hz), 7.30 (d, 1H, *J*=8.1 Hz), 6.67 (d, 1H, *J*=7.5 Hz), 5.4-5.7 (m, 1H), 4.5-4.6 (m, 1H), 3.9-4.4 (m, 9H), 3.72 (br t, 1H, 7=10.5 Hz), 3.5-3.7 (m, 4H), 3.43 (br d, 4H, *J*=6.2 Hz), 2.7-2.9 (m, 2H), 1.34 (d, 3H, *J*=6.4 Hz).

**Example 96**

**5-[*(2S,6R)*-2-[[4-[2-[(*3R*,*4R*)-3-amino-4-fluoro-pyrrolidin-1-yl]pyrimidin-4-yl]piperazin-1-yl]methyl]-6-methyl-morpholin-4-yl]quinoline-8-carbonitrile**

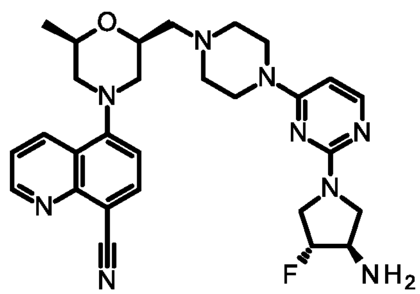

The title compound was prepared in analogy to the preparation of Example **23** by using *tert*-butyl *N*-[(*3R,4R*)-4-fluoropyrrolidin-3-yl]carbamate instead of *tert*-butyl azetidin-3-ylcarbamate (compound **23c**). **Example 96** (14 mg) was obtained as a light yellow solid. MS: calc'd 532 ($MH^+$), measured 532 ($MH^+$). $^1H$ NMR (METHANOL-$d_4$, 400 MHz) δ 8.99 (dd, 1H, *J*=1.6, 4.3 Hz), 8.65 (dd, 1H, *J*=1.6, 8.6 Hz), 8.16 (d, 1H, *J*=7.9 Hz), 7.91 (d, 1H, *J*=7.5 Hz), 7.64 (dd, 1H, *J*=4.2, 8.6 Hz), 7.28 (d, 1H, *J*=8.1 Hz), 6.66 (d, 1H, *J*=7.6 Hz), 5.4-5.6 (m, 1H), 4.5-4.6 (m, 1H), 3.8-4.4 (m, 10H), 3.5-3.7 (m, 4H), 3.4-3.5 (m, 4H), 2.7-2.8 (m, 2H), 1.31 (d, 3H, *J*=6.2 Hz).

**Example 97**

**5-[*(2S,6R)*-2-[[4-[4-[(*3S*,*4S*)-3-amino-4-methoxy-pyrrolidin-1-yl]pyrimidin-2-yl]piperazin-1-yl]methyl]-6-methyl-morpholin-4-yl]quinoline-8-carbonitrile**

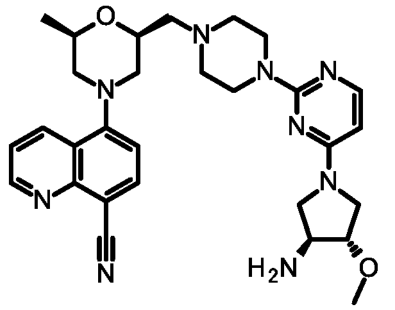

The title compound was prepared according to the following scheme:

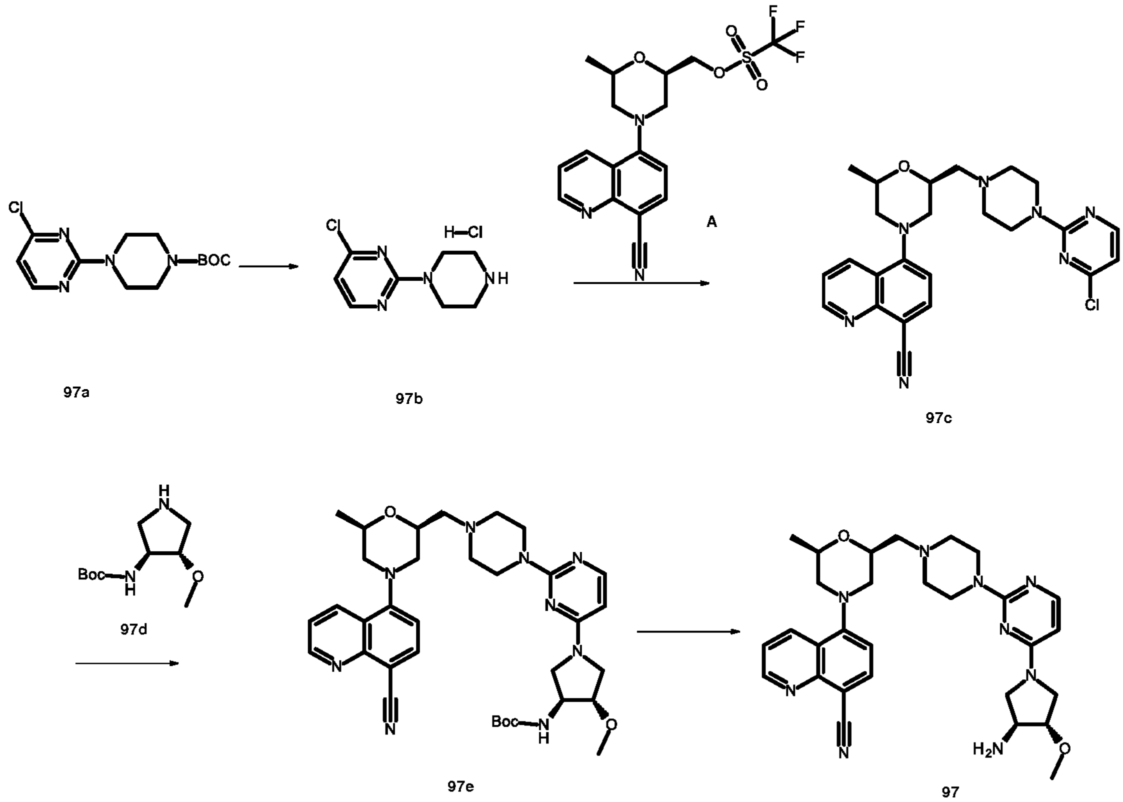


97a 97b 97c 97d 97e 97

**Step 1: preparation of 4-chloro-2-(piperazin-1-yl)pyrimidine hydrochloride (compound 97b)**

A solution of *tert*-butyl 4-(4-chloropyrimidin-2-yl)piperazine-1-carboxylate (compound **97a**, 600 mg, 2.01 mmol) in DCM (4 mL) was added a solution of hydrogen chloride in dioxane (2 mL, 4M). After the mixture was stirred at room temperature for 12 hrs, it was concentrated to afford compound **97b** (383 mg). MS: calc'd 199 ($MH^+$), measured 199 ($MH^+$).

**Step 2: preparation of 5-((*2S,6R*)-2-((4-(4-chloropyrimidin-2-yl)piperazin-1-yl)methyl)-6-methylmorpholino)quinoline-8-carbonitrile (compound 97c)**

A suspension of 4-chloro-2-(piperazin-1-yl)pyrimidine hydrochloride (compound **97b**, 383 mg, 1.63 mmol), ((*2R,6R*)-4-(8-cyanoquinolin-5-yl)-6-methylmorpholin-2-yl)methyl trifluoromethanesulfonate (Intermediate **A**, 582 mg, 1.4 mmol) and $K_2CO_3$ (581 mg, 4.2 mmol) in MeCN (8 ml) was stirred at room temperature for 3 hrs. The mixture was cooled to room temperature and then diluted with water (5 mL) and extracted with EtOAc (10 mL ) three times. The combined organic layer was washed with brine (5 mL), dried over $Na_2SO_4$, and concentrated. Then the residue was purified by silica gel chromatography (EA/PE from 20% to 80%) to afford compound **97c** (492 mg) as yellow solid. MS: calc'd 464 ($MH^+$), measured 464 ($MH^+$).

**Step 3: preparation of *tert*-butyl ((*3S,4S*)-1-(2-(4-(((*2S,6R*)-4-(8-cyanoquinolin-5-yl)-6-methylmorpholin-2-yl)methyl)piperazin-1-yl)pyrimidin-4-yl)-4-methoxypyrrolidin-3-yl)carbamate (compound 97e)**

A suspension of 5-((*2S,6R*)-2-((4-(4-chloropyrimidin-2-yl)piperazin-1-yl)methyl)-6-methylmorpholino)quinoline-8-carbonitrile (compound **97c**, 46 mg, 99.1 μmol), *tert*-butyl *N*-[(*3S,4S*)-4-methoxypyrrolidin-3-yl]carbamate (compound **97d**, 23.6 mg, 109 μmol) and $K_2CO_3$ (41.1 mg, 297 μmol) in MeCN (8 ml) was stirred at 120 °C for 12 hrs. The mixture was cooled to room temperature and then diluted with water (5 mL) and extracted with EtOAc (10 mL) three times. The combined organic layer was washed with brine (5 mL), dried over $Na_2SO_4$ and concentrated. Then the residue was purified by silica gel chromatography (EA/PE from 20% to 80%) to afford compound **97e** (60 mg) as a yellow solid. MS: calc'd 644 ($MH^+$), measured 644 ($MH^+$).

**Step 4: preparation of 5-[*(2S,6R)*-2-[[4-[4-[(*3S,4S*)-3-amino-4-methoxy-pyrrolidin-1-yl]pyrimidin-2-yl]piperazin-1-yl]methyl]-6-methyl-morpholin-4-yl]quinoline-8-carbonitrile (Example 97)**

To a solution of *tert*-butyl ((*3S,4S*)-1-(2-(4-(((*2S,6R*)-4-(8-cyanoquinolin-5-yl)-6-methylmorpholin-2-yl)methyl)piperazin-1-yl)pyrimidin-4-yl)-4-methoxypyrrolidin-3-yl)carbamate (compound **97e** , 60 mg, 93.2 μmol) in DCM (4 ml) was added 2,2,2-trifluoroacetic acid (740 mg, 0.5 ml, 6.49 mmol) at 0 °C. The reaction mixture was stirred at r.t. for 2 hrs. Then the mixture was concentrated and purified by prep-HPLCto afford 5-[*(2S,6R)*-2-[[4-[2-[(*3R,4R)*-3-amino-4-fluoro-pyrrolidin-1-yl]pyrimidin-4-yl]piperazin-1-yl] methyl]-6-methyl-morpholin-4-yl]quinoline-8-carbonitrile (**Example 97,** 54 mg) as a light yellow solid. MS calc'd 544 ($MH^+$), measured 544 ($MH^+$). $^1H$ NMR (METHANOL-$d_4$, 400 MHz) δ 9.01 (dd, 1H, *J*=1.7, 4.2 Hz), 8.67 (dd, 1H, *J*=1.6, 8.6 Hz), 8.18 (d, 1H, *J*=7.9 Hz), 7.91 (d, 1H, *J*=7.2 Hz), 7.66 (dd, 1H, *J*=4.3, 8.6 Hz), 7.30 (d, 1H, *J*=8.1 Hz), 6.39 (d, 1H, *J*=7.2 Hz), 4.5-4.6 (m, 1H), 4.0-4.3 (m, 9H), 3.7-3.9 (m, 2H), 3.5-3.7 (m, 4H), 3.50 (s, 3H), 3.4-3.5 (m, 4H), 2.7-2.8 (m, 2H), 1.33 (d, 3H, *J*=6.2 Hz).

**Example 98**

**8-[*(2S,6R)*-2-[[4-[2-[(*3R,4R*)-3-amino-4-methoxy-pyrrolidin-1-yl]pyrimidin-4-yl]piperazin-1-yl]methyl]-6-methyl-morpholin-4-yl]quinoxaline-5-carbonitrile**

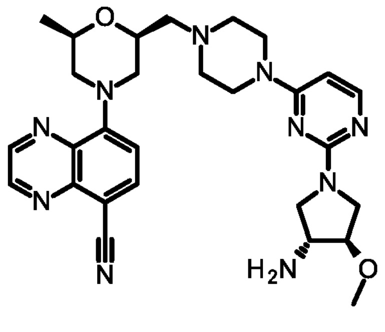

The title compound was prepared in analogy to the preparation of Example **23** by using ((*2R,6R*)-4-(8-cyanoquinoxalin-5-yl)-6-methylmorpholin-2-yl)methyl trifluoromethanesulfonate (Intermediate **C**) and *tert*-butyl *N-[(3R,4R)*-4-methoxypyrrolidin-3-yl]carbamate instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 98** (31 mg) was obtained as a light yellow solid. MS: calc'd 545 ($MH^+$), measured 545 ($MH^+$). $^1H$ NMR (METHANOL-$d_4$, 400 MHz) δ 8.99 (d, 1H, *J*=1.7 Hz), 8.91 (d, 1H, *J*=1.7 Hz), 8.17 (d, 1H, *J*=8.3 Hz), 7.91 (d, 1H, *J*=7.5 Hz), 7.28 (d, 1H, *J*=8.3 Hz), 6.65 (d, 1H, *J*=7.5 Hz), 4.4-4.5 (m, 1H), 4.0-4.4 (m, 11H), 3.7-4.0 (m, 2H), 3.55 (br d, 4H, *J*=1.6 Hz), 3.5-3.5 (m, 3H), 3.4-3.5 (m, 2H), 2.8-2.9 (m, 2H), 1.34 (d, 3H, *J*=6.1 Hz).

**Example 99**

**8-[(*2S,6R*)-2-[[4-[2-[(*3S,4S*)-3-amino-4-methoxy-pyrrolidin-1-yl]pyrimidin-4-yl]piperazin-1-yl]methyl]-6-methyl-morpholin-4-yl]quinoxaline-5-carbonitrile**

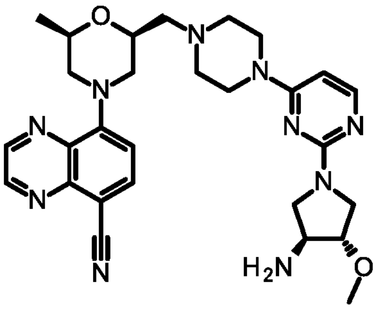

The title compound was prepared in analogy to the preparation of Example **23** by using ((*2R,6R*)-4-(8-cyanoquinoxalin-5-yl)-6-methylmorpholin-2-yl)methyl trifluoromethanesulfonate (Intermediate **C**) and *tert*-butyl *N-[(3S,4S)*-4-methoxypyrrolidin-3-yl]carbamate instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 99** (18 mg) was obtained as a light yellow solid. MS: calc'd 545 ($MH^+$), measured 545 ($MH^+$). $^1H$ NMR (METHANOL-$d_4$, 400 MHz) $\delta$ 8.99 (d, 1H, *J*=1.8 Hz), 8.91 (d, 1H, *J*=1.8 Hz), 8.17 (d, 1H, *J*=8.3 Hz), 7.91 (d, 1H, *J*=7.5 Hz), 7.28 (d, 1H, *J*=8.3 Hz), 6.65 (d, 1H, *J*=7.6 Hz), 4.4-4.5 (m, 1H), 4.1-4.4 (m, 8H), 4.0-4.1 (m, 3H), 3.8-4.0 (m, 1H), 3.7-3.8 (m, 1H), 3.5-3.7 (m, 4H), 3.50 (s, 3H), 3.4-3.4 (m, 2H), 2.8-2.9 (m, 2H), 1.35 (d, 3H, *J*=6.1 Hz).

**Example 101**

**5-[(*2S,6R*)-2-[[4-[2-[(*3R,4S*)-3-amino-4-fluoro-pyrrolidin-1-yl]pyrimidin-4-yl]piperazin-1-yl]methyl]-6-methylmorpholin-4-yl]quinoline-8-carbonitrile**

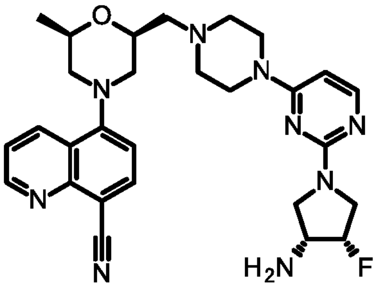

The title compound was prepared in analogy to the preparation of Example **23** by using *tert*-butyl *N-[(3R,4S)*-4-fluoropyrrolidin-3-yl]carbamate instead of *tert*-butyl azetidin-3-ylcarbamate (compound **23c**). **Example 101** (9 mg) was obtained as a light yellow solid. MS: calc'd 532 ($MH^+$), measured 532 ($MH^+$). $^1H$ NMR (METHANOL-$d_4$, 400 MHz) $\delta$ 8.88 (dd, 1H, *J*=1.7, 4.2 Hz), 8.55 (dd, 1H, *J*=1.7, 8.6 Hz), 8.05 (d, 1H, *J*=8.1 Hz), 7.82 (d, 1H, *J*=7.5 Hz), 7.54 (dd, 1H, *J*=4.3, 8.6 Hz), 7.17 (d, 1H, *J*=7.9 Hz), 6.56 (d, 1H, *J*=7.5 Hz), 5.3-5.5 (m, 1H), 4.4-4.5 (m, 1H), 3.8-4.3 (m, 9H), 3.61 (br t, 1H, *J*=10.3 Hz), 3.4-3.6 (m, 4H), 3.31 (br s, 4H), 2.6-2.7 (m, 2H), 1.21 (d, 3H, *J*=6.2 Hz).

**Example 102**

**5-[(*2R,6S*)-2-methyl-6-[[4-(5-methyl-4-piperazin-1-yl-pyrimidin-2-yl)piperazin-1-yl]methyl]morpholin-4-yl]quinoline-8-carbonitrile**

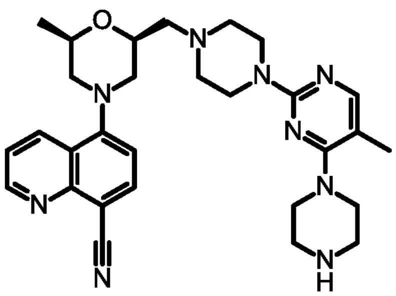

The title compound was prepared according to the following scheme:

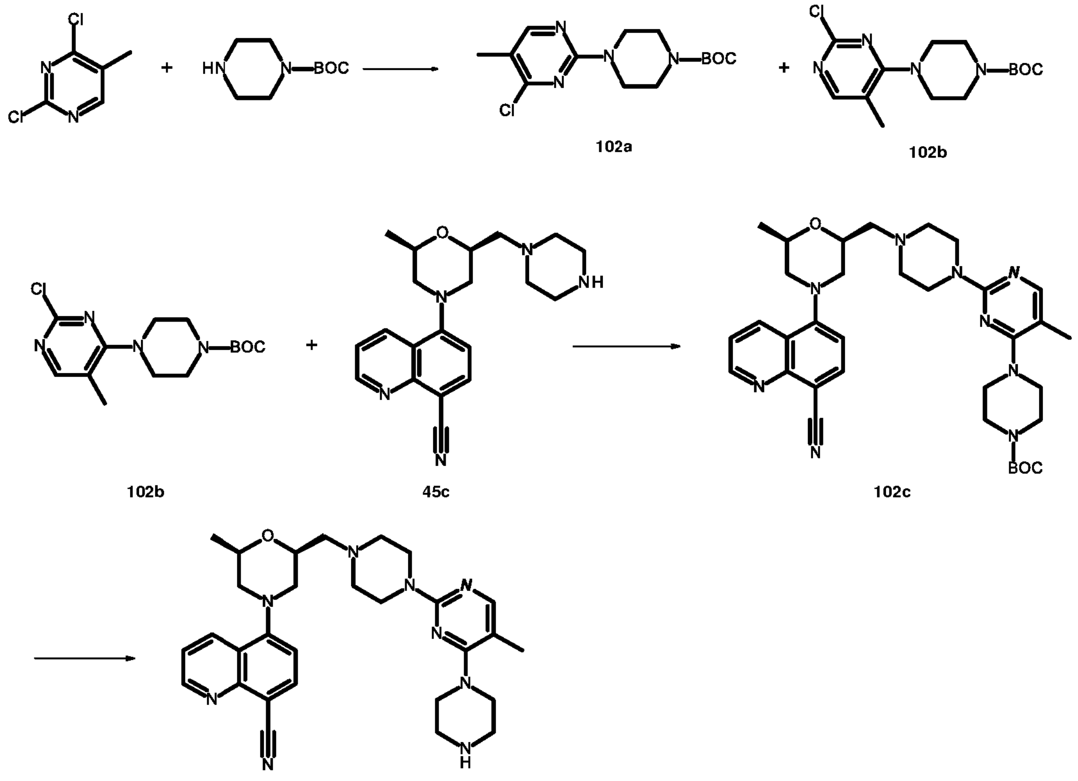

102a 102b

102b 45c 102c

102

**Step 1: preparation of *tert*-butyl 4-(4-chloro-5-methylpyrimidin-2-yl)piperazine-1-carboxylate (compound 102a) and *tert*-butyl 4-(2-chloro-5-methylpyrimidin-4-yl)piperazine-1-carboxylate (compound 102b)**

A solution of 2,4-dichloro-5-methylpyrimidine (326 mg, 2 mmol) and 2,4-dichloro-5-methylpyrimidine (326 mg, 2 mmol) in ethanol (6 mL) was stirred at room temperature for 12 hrs. Then the mixture was concentrated and purified by silica gel chromatography (EA/PE from 20% to 80%) to afford compound **102a** (100 mg) as a white solid. MS: calc'd 313 ($MH^+$), measured 313 ($MH^+$).

**Step 2: preparation of *tert*-butyl 4-(2-(4-(((*2S,6R*)-4-(8-cyanoquinolin-5-yl)-6-methylmorpholin-2-yl)methyl)piperazin-1-yl)-5-methylpyrimidin-4-yl)piperazine-1-carboxylate (compound 102c)**

A suspension of 5-((*2R,6S*)-2-methyl-6-(piperazin-1-ylmethyl)morphofino)quinoline-8-carbonitrile (compound **45c**, 35 mg, 99.6 μmol), *tert*-butyl 4-(2-chloro-5-methylpyrimidin-4-yl)piperazine-1-carboxylate (compound **102b**, 31 mg, 99.6 μmol), Ruphos Pd G2 (7 mg, 9.96 μmol) and $k_2CO_3$ (27 mg, 199 μmol) in 1,4-Dioxane (4 mL) was heated to 100°C for 12 hrs under nitrogen atmosphere. The mixture was cooled to room temperature and then diluted with water (5 mL) and extracted with EtOAC (10 mL ) three times. The combined organic layer was washed with brine, dried over $Na_2SO_4$, and concentrated. The resulting residue was purified by silica gel chromatography (EA/PE from 20% to 80%) to afford *tert*-butyl 4-(2-(4-(((*2S,6R*)-4-(8-cyanoquinolin-5-yl)-6-methylmorpholin-2-yl)methyl)piperazin-1-yl)-5-methylpyrimidin-4-yl)piperazine-1-carboxylate (compound **102c**, 19 mg) as a pale yellow solid. MS: calc'd 628 ($MH^+$), measured 628 ($MH^+$).

**Step 3: preparation of 5-[*(2S,6R)*-2-[[4-(5-fluoro-4-piperazin-1-yl-pyrimidin-2-yl)piperazin-1-yl]methyl]-6-methyl-morpholin-4-yl]quinoline-8-carbonitrile (Example 102)**

To a solution of *tert*-butyl 4-(2-(4-(((*2S,6R*)-4-(8-cyanoquinofin-5-yl)-6-methylmorphofin-2-yl)methyl)piperazin-1-yl)-5-methylpyrimidin-4-yl)piperazine-1-carboxylate (compound **102c**, 19 mg, 30.3 μmol) in DCM (4 mL) was added 2,2,2-trifluoroacetic acid (0.5 mL) at 0 °C. The reaction mixture was stirred at r.t. for 2 hrs. Then the mixture was concentrated and purified by prep-HPLC(TFA/MeCN) to afford **Example 102** (27 mg) as a light yellow solid. MS calc'd 528 ($MH^+$), measured 528 ($MH^+$). $^1H$ NMR (METHANOL-$d_4$, 400 MHz) δ 8.99 (dd, 1H, *J*=1.7, 4.2 Hz), 8.66 (dd, 1H, *J*=1.7, 8.6 Hz), 8.16 (d, 1H, *J*=8.1 Hz), 7.88 (d, 1H, *J*=0.9 Hz), 7.65 (dd, 1H, *J*=4.2, 8.6 Hz), 7.28 (d, 1H, *J*=8.1 Hz), 4.5-4.6 (m, 1H), 4.1-4.3 (m, 4H), 4.0-4.0 (m, 4H), 3.5-3.7 (m, 4H), 3.4-3.5 (m, 8H), 2.7-2.8 (m, 2H), 2.28 (s, 3H), 1.33 (d, 3H, *J*=6.2 Hz).

**Example 103**

**5-[*(2S,6R)*-2-[[4-[2-[(4*aR*,7*aR*)-3,4,4*a*,5,7,7*a*-hexahydro-2*H*-pyrrolo[3,4-b][1,4]oxazin-6-yl]pyrimidin-4-yl]piperazin-1-yl]methyl]-6-methyl-morpholin-4-yl]quinoline-8-carbonitrile**

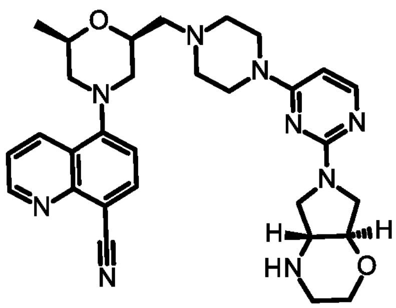

The title compound was prepared in analogy to the preparation of Example **23** by using *tert*-butyl (4*aR*,*7aR*)-*3*,*4a*,5,6,7,7a-hexahydro-2*H*-pyrrolo[3,4-b][1,4]oxazine-4-carboxylate (PharmaBlock, PBXA8123, CAS: 1932337-68-2) instead of *tert*-butyl azetidin-3-ylcarbamate (compound **23c**). **Example 101** (2 mg) was obtained as a light yellow solid. MS: calc'd 556 ($MH^+$), measured 556 ($MH^+$). $^1H$ NMR (METHANOL-$d_4$, 400 MHz) δ 9.0-9.0 (m, 1H), 8.6-8.7 (m, 1H), 8.17 (d, 1H, *J*=8.1 Hz), 7.90 (d, 1H, *J*=7.1 Hz), 7.65 (dd, 1H, *J*=4.4, 8.6 Hz), 7.28 (d, 1H, *J*=8.1 Hz), 6.6-6.7 (m, 1H), 4.5-4.5 (m, 2H), 3.9-4.3 (m, 10H), 3.4-3.6 (m, 12H), 2.7-2.8 (m, 2H), 1.31 (d, 3H, *J*=6.2 Hz).

**Example 104**

**8-[*(2S,6R)*-2-[[4-[4-[(*3S,4S*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile**

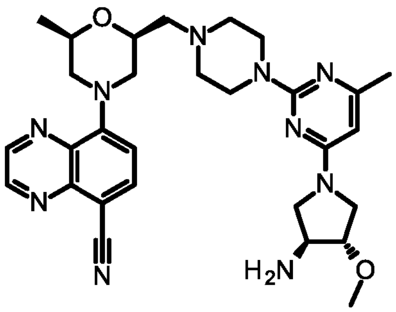

The title compound was prepared according to the following scheme:

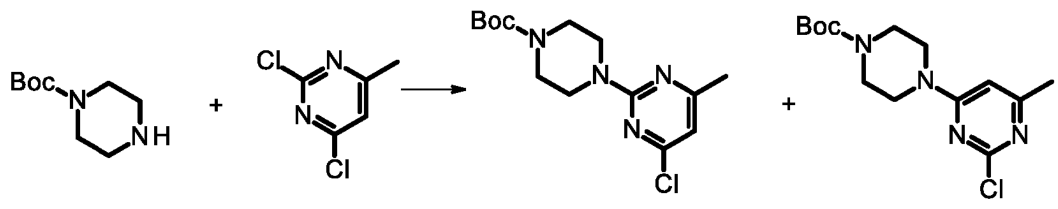

104a 104b

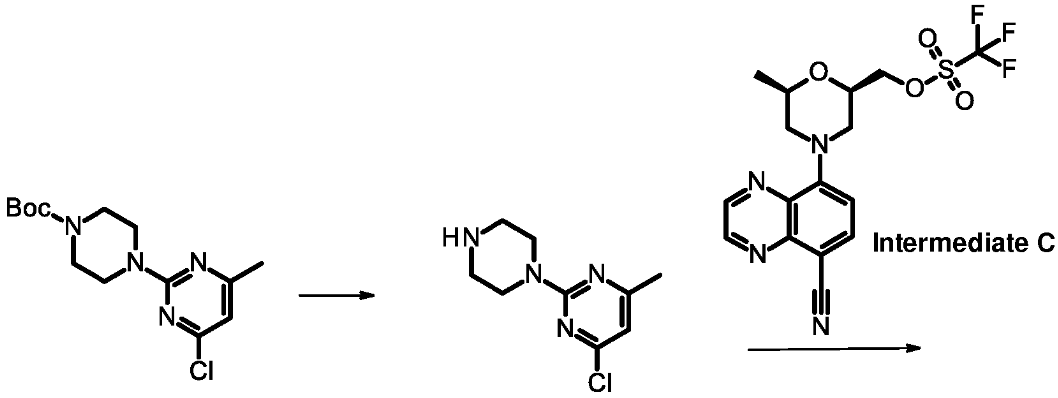


104a 104c

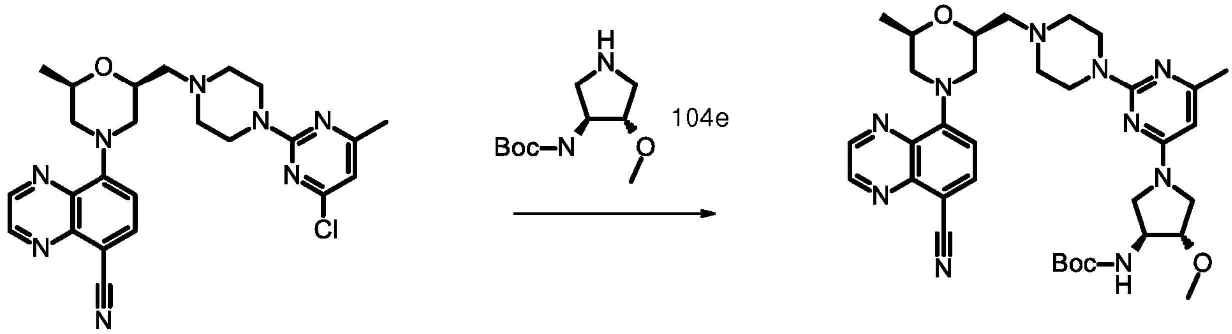

104e

104d 104f

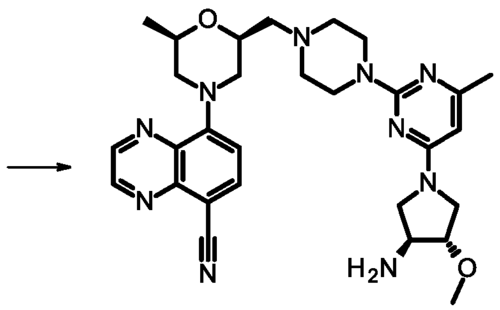

104

**Step 1: preparation of *tert*-butyl 4-(4-chloro-6-methylpyrimidin-2-yl)piperazine-1-carboxylate (compound 104a) and *tert*-butyl 4-(2-chloro-6-methylpyrimidin-4-yl)piperazine-1-carboxylate (compound 104b)**

A solution of 2,4-dichloro-6-methylpyrimidine (3.26 g, 20 mmol), $Et_3N$ (2.79 mL, 20 mmol) and *tert*-butyl piperazine-1-carboxylate (4.10 g, 22 mmol) in ethanol (40 mL) was stirred at room temperature for 12 hrs. Then the mixture was concentrated and diluted with DCM (20 mL) and washed with water (10 mL) three times, brine (10 mL) and dried over $Na_2SO_4$. The resulting residue was purified by silica gel chromatography (PE/EtOAc from 10% to 60%) to afford *tert*-butyl 4-(4-chloro-6-methylpyrimidin-2-yl)piperazine-1-carboxylate (compound **104a,** 1 g) and *tert*-butyl 4-(2-chloro-6-methylpyrimidin-4-yl)piperazine-1-carboxylate (compound **104b,** 4.35 g). MS: calc'd 313 ($MH^+$), measured 313 ($MH^+$).

**Step 2: preparation of 4-chloro-6-methyl-2-(piperazin-1-yl)pyrimidine (compound 104c)**

A solution of *tert*-butyl 4-(4-chloro-6-methylpyrimidin-2-yl)piperazine-1-carboxylate (compound **104a,** 1 g) in DCM (4 mL) was added hydrogen chloride/dioxane (4 mL, 4M in dioxane). After the reaction mixture was stirred at room temperature for 12 hrs, it was concentrated to afford 4-chloro-6-methyl-2-(piperazin-1-yl)pyrimidine (compound **104c,** 756 mg). MS: calc'd 213 ($MH^+$), measured 213 ($MH^+$).

**Step 3: preparation of 8-[(*2S,6R*)-2-[[4-(4-chloro-6-methyl-pyrimidin-2-yl)piperazin-1-yl]methyl]-6-methyl-morpholin-4-yl]quinoxaline-5-carbonitrile (compound 104d)**

A suspension of 4-chloro-6-methyl-2-(piperazin-1-yl)pyrimidine (compound **104c,** 479 mg, 1.92 mmol), [(*2R,6R*)-4-(8-cyanoquinoxalin-5-yl)-6-methyl-morphofin-2-yl]methyl trifluoromethanesulfonate (intermediate C, 800 mg, 1.92 mmol) and $K_2CO_3$ (797 mg, 5.76 mmol) in MeCN (8 ml) was stirred at room temperature for 3 hrs. The mixture was cooled to room temperature and then diluted with water (5 mL) and extracted with EtOAc (10 mL) three times. The combined organic layer was washed with brine (5 mL), dried over $Na_2SO_4$, and concentrated. Then the residue was purified by silica gel chromatography (PE/EtOAc 20% to 100%) to afford 8-[(*2S*,*6R*)-2-[[4-(4-chloro-6-methyl-pyrimidin-2-yl)piperazin-1-yl]methyl]-6-methyl-morpholin-4-yl]quinoxaline-5-carbonitrile (compound **104d,** 560 mg) as a yellow solid. MS: calc'd 479 ($MH^+$), measured 479 ($MH^+$).

**Step 4: preparation of *tert*-butyl ((*3S,4S*)-1-(2-(4-(((2S,6R)-4-(8-cyanoquinoxalin-5-yl)-6-methylmorpholin-2-yl)methyl)piperazin-1-yl)-6-methylpyrimidin-4-yl)-4-methoxypyrrolidin-3-yl)carbamate (compound 104f)**

A suspension of 8-[(*2S,6R*)-2-[[4-(4-chloro-6-methyl-pyrimidin-2-yl)piperazin-1-yl]methyl]-6-methyl-morpholin-4-yl]quinoxaline-5-carbonitrile (compound **104d,** 21 mg, 43.8 μmol), *tert*-butyl *N*-[(*3S,4S*)-4-methoxypyrrolidin-3-yl]carbamate (compound **104e**, 10.4 mg, 48.2 μmol) and $K_2CO_3$ (18.2 mg, 132 μmol) in MeCN (8 mL) was stirred at 120 °C for 12 hrs. The mixture was cooled to room temperature and then diluted with water (5 mL) and extracted with EtOAc (10 mL ) three times. The combined organic layer was washed with brine (5 mL), dried over $Na_2SO_4$ and concentrated. Then the residue was purified by silica gel chromatography (EA/PE from 20% to 80%) to afford compound *tert*-butyl ((*3S,4S*)-1-(2-(4-(((*2S,6R)*-4-(8-cyanoquinoxalin-5-yl)-6-methylmorpholin-2-yl)methyl)piperazin-1-yl)-6-methylpyrimidin-4-yl)-4-methoxypyrrolidin-3-yl)carbamate (compound **104f,** 20 mg) as a yellow solid. MS: calc'd 659 ($MH^+$), measured 659($MH^+$).

**Step 5: preparation of 8-[*(2S,6R)*-2-[[4-[4-[(*3S*,*4S*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile (compound 104)**

To a solution of *tert*-butyl ((*3S,4S*)-1-(2-(4-(((*2S,6R*)-4-(8-cyanoquinoxalin-5-yl)-6-methylmorpholin-2-yl)methyl)piperazin-1-yl)-6-methylpyrimidin-4-yl)-4-methoxypyrrolidin-3-yl)carbamate (compound **104f,** 20 mg, 30.4 μmol) in DCM (4 mL) was added HCl (0.2 mL, 4 M in dioxane) at 0 °C. The reaction mixture was stirred at rt for 2 hrs. Then the mixture was concentrated to afford **Example 104** (13.1 mg) as a light yellow solid. MS calc'd 559 ($MH^+$), measured 559 ($MH^+$). $^1H$ NMR (METHANOL-$d_4$, 400 MHz) δ 8.98 (d, 1H, *J*=1.8 Hz), 8.90 (d, 1H, *J*=1.7 Hz), 8.16 (d, 1H, *J*=8.3 Hz), 7.27 (d, 1H, *J*=8.3 Hz), 6.31 (s, 1H), 4.6-4.8 (m, 1H), 4.5-4.5 (m, 1H), 4.3-4.3 (m, 1H), 4.15 (br d, 8H, *J*=2.0 Hz), 3.6-3.9 (m, 6H), 3.5-3.5 (m, 1H), 3.5-3.5 (m, 3H), 3.4-3.5 (m, 2H), 2.8-2.9 (m, 2H), 2.46 (s, 3H), 1.34 (d, 3H, *J*=6.1 Hz).

**Example 105**

**8-[(*2S,6R*)-2-[[4-[4-[(*3S,4S*)-3-amino-4-methoxy-pyrrolidin-1-yl]-6-methyl-pyrimidin-2-yl]piperazin-1-yl]methyl]-6-methyl-morpholin-4-yl]-2,3-dideuterio-quinoxaline-5-carbonitrile**

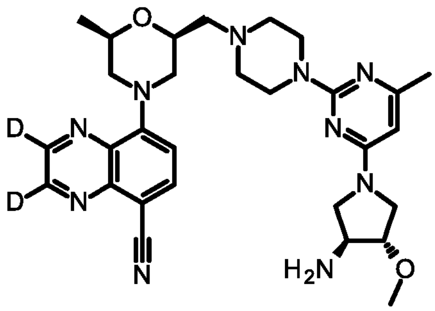

The title compound was prepared in analogy to the preparation of Example **104** by using [(*2R,6R*)-4-(8-cyano-2,3-dideuterio-quinoxalin-5-yl)-6-methyl-morpholin-2-yl]methyl trifluoromethanesulfonate (Intermediate **K**) instead of [(*2R,6R*)-4-(8-cyanoquinoxalin-5-yl)-6-methyl-morpholin-2-yl]methyl trifluoromethanesulfonate (Intermediate **C**). **Example 105** ( 21 mg) was obtained as a light yellow solid. MS: calc'd 561 ($MH^+$), measured 561 ($MH^+$). $^1H$ NMR (METHANOL-d4, 400 MHz) δ 8.1-8.2 (m, 1H), 7.26 (d, 1H, *J*=8.3 Hz), 6.24 (s, 1H), 4.4-4.5 (m, 1H), 4.29 (br d, 1H, *J*=12.1 Hz), 4.1-4.2 (m, 6H), 3.9-4.1 (m, 4H), 3.7-3.8 (m, 1H), 3.5-3.6 (m, 4H), 3.5-3.5 (m, 3H), 3.4-3.4 (m, 2H), 2.8-2.9 (m, 2H), 2.41 (s, 3H), 1.4-1.4 (m, 1H), 1.3-1.3 (m, 3H).

**Example 106**

**8-[*(2S,6R)*-2-[[4-[2-[(*3S,4S*)-3-amino-4-methoxy-pyrrolidin-1-yl]-6-methyl-pyrimidin-4-yl]piperazin-1-yl]methyl]-6-methyl-morpholin-4-yl]quinoxaline-5-carbonitrile**

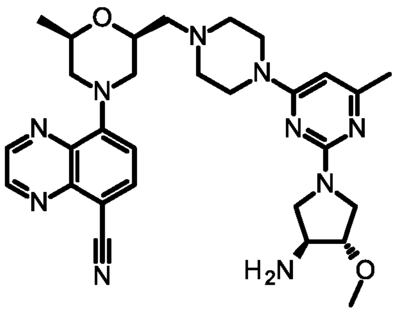

The title compound was prepared in analogy to the preparation of **Example 45** by using 2,4-dichloro-6-methyl-pyrimidine and ((*2R,6R*)-4-(8-cyanoquinoxalin-5-yl)-6-methylmorpholin-2-yl)methyl trifluoromethanesulfonate (Intermediate **C**) and *tert*-butyl *N-[(3S,4S*)-4-methoxypyrrolidin-3-yl]carbamate instead of 2,4-dichloro-5-fluoropyrimidine (compound **45d**) and ((*2R,6R*)-4-(8-cyanoquinolin-5-yl)-6-methylmorpholin-2-yl)methyl trifluoromethanesulfonate (Intermediate **A**) and *tert*-butyl piperazine-1-carboxylate (compound **45a**). **Example 106** (41 mg) was obtained as a light yellow solid. MS: calc'd 559 ($MH^+$), measured 559 ($MH^+$). $^1H$ NMR (METHANOL-$d_4$, 400 MHz) δ 8.98 (d, 1H, *J*=1.8 Hz), 8.89 (d, 1H, *J*=1.7 Hz), 8.16 (d, 1H, *J*=8.3 Hz), 7.27 (d, 1H, *J*=8.6 Hz), 6.54 (s, 1H), 4.4-4.5 (m, 1H), 4.0-4.3 (m, 10H), 3.6-3.9 (m, 6H), 3.5-3.6 (m, 2H), 3.5-3.5 (m, 3H), 3.5-3.5 (m, 1H), 2.8-2.9 (m, 2H), 2.44 (s, 3H), 1.33 (d, 3H, *J*=5.9 Hz).

**Example 107**

**8-[*(2S,6R)*-2-[[4-[2-[(*3S,4S*)-3-amino-4-fluoro-pyrrolidin-1-yl]pyrimidin-4-yl]piperazin-1-yl]methyl]-6-methyl-morpholin-4-yl]quinoxaline-5-carbonitrile**

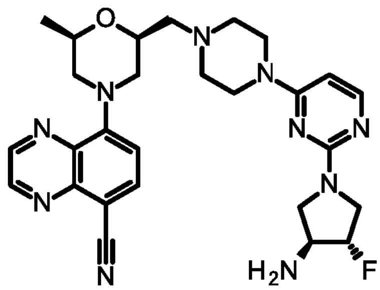

The title compound was prepared in analogy to the preparation of Example **23** by using ((*2R,6R*)-4-(8-cyanoquinoxalin-5-yl)-6-methylmorpholin-2-yl)methyl trifluoromethanesulfonate (Intermediate **C**) and *tert*-butyl *N-[(3S,4S)*-4-fluoroprrolidin-3-yl]carbamate instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 107** (50 mg) was obtained as a light yellow solid. MS: calc'd 533 ($MH^+$), measured 533 ($MH^+$). $^1H$ NMR (METHANOL-$d_4$, 400 MHz) $\delta$ 9.00 (d, 1H, *J*=1.7 Hz), 8.18 (d, 1H, *J*=8.3 Hz), 7.94 (d, 1H, *J*=7.5 Hz), 7.30 (d, 1H, *J*=8.4 Hz), 6.74 (d, 1H, *J*=7.6 Hz), 5.5-5.7 (m, 1H), 5.16 (br s, 1H), 4.4-4.6 (m, 2H), 4.1-4.4 (m, 7H), 3.6-4.1 (m, 6H), 3.4-3.6 (m, 4H), 2.8-2.9 (m, 2H), 1.36 (d, 3H, *J*=6.1 Hz).

**Example 108**

**8-[*(2S,6R)*-2-[[4-[2-[(*3R,4S*)-3-amino-4-fluoro-pyrrolidin-1-yl]pyrimidin-4-yl]piperazin-1-yl]methyl]-6-methyl-morpholin-4-yl]quinoxaline-5-carbonitrile**

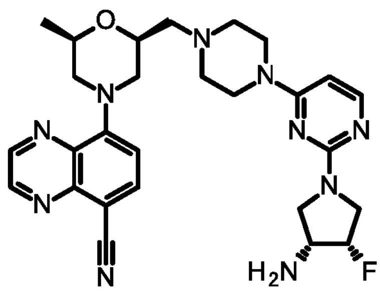

The title compound was prepared in analogy to the preparation of Example **23** by using ((*2R,6R*)-4-(8-cyanoquinoxalin-5-yl)-6-methylmorpholin-2-yl)methyl trifluoromethanesulfonate (Intermediate **C**) and *tert*-butyl *N-[(3R,4S)*-4-fluoroprrolidin-3-yl]carbamate instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 108** (27 mg) was obtained as a light yellow solid. MS: calc'd 533 ($MH^+$), measured 533 ($MH^+$). $^1H$ NMR (METHANOL-$d_4$, 400 MHz) $\delta$ 8.84 (d, 1H, *J*=1.7 Hz), 8.78 (d, 1H, *J*=1.7 Hz), 8.01 (d, 1H, *J*=8.3 Hz), 7.71 (d, 1H, *J*=6.2 Hz), 7.10 (d, 1H, *J*=8.4 Hz), 5.97 (d, 1H, *J*=6.2 Hz), 4.8-5.0 (m, 1H), 4.21 (br d, 1H, *J*=12.2 Hz), 4.0-4.1 (m, 2H), 3.8-4.0 (m, 2H), 3.6-3.8 (m, 6H), 3.3-3.5 (m, 1H), 3.0-3.1 (m, 1H), 2.6-2.7 (m, 4H), 2.49 (br d, 4H, *J*=7.0 Hz), 1.16 (d, 3H, *J*=6.2 Hz).

**Example 109**

**5-[*(2S,6R)*-2-[[4-[2-(3-amino-4-methoxy-1-piperidyl)pyrimidin-4-yl]piperazin-1-yl]methyl]-6-methyl-morpholin-4-yl]quinoline-8-carbonitrile**

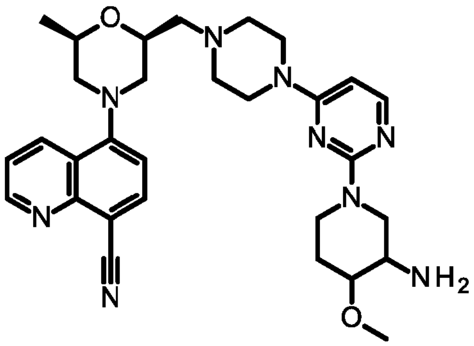

The title compound was prepared in analogy to the preparation of Example **23** by using *tert*-butyl *N*-(4-methoxy-3-piperidyl)carbamate (PharmaBlock, PBCS1406262, CAS: 1262407-41-9) instead of *tert*-butyl azetidin-3-ylcarbamate (compound **23c**). **Example 109** (28 mg) was obtained as a light yellow solid. MS: calc'd 558 ($MH^+$), measured 558 ($MH^+$). $^1H$ NMR (METHANOL-$d_4$, 400 MHz) $\delta$ 9.02 (dd, 1H, *J*=1.7, 4.2 Hz), 8.68 (dd, 1H, *J*=1.6, 8.6 Hz), 8.19 (d, 1H, *J*=7.9 Hz), 7.93 (d, 1H, *J*=7.3 Hz), 7.67 (dd, 1H, *J*=4.3, 8.6 Hz), 7.31 (d, 1H, *J*=8.1 Hz), 6.64 (d, 1H, *J*=7.3 Hz), 4.5-4.6 (m, 1H), 4.41 (br dd, 1H, *J*=3.4, 13.2 Hz), 4.1-4.3 (m, 4H), 3.84 (br s, 3H), 3.5-3.7 (m, 6H), 3.5-3.5 (m, 3H), 3.4-3.5 (m, 5H), 2.7-2.9 (m, 2H), 2.1-2.2 (m, 1H), 1.9-2.0 (m, 1H), 1.34 (d, 3H, *J*=6.2 Hz).

**Example 110**

**5-[(*2S,6R*)-2-[[4-[2-[(*3S,4S*)-3-amino-4-fluoro-pyrrolidin-1-yl]pyrimidin-4-yl]piperazin-1-yl]methyl]-6-methyl-morpholin-4-yl]quinoline-8-carbonitrile**

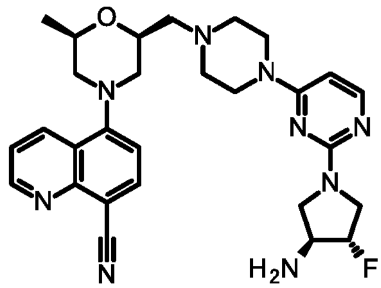

The title compound was prepared in analogy to the preparation of Example **23** by using *tert*-butyl *N*-[(*3S,4S*)-4-fluoropyrrolidin-3-yl]carbamate instead of *tert*-butyl azetidin-3-ylcarbamate (compound **23c**). **Example 110** (14 mg) was obtained as a light yellow solid. MS: calc'd 532 ($MH^+$), measured 532 ($MH^+$). $^1H$ NMR (METHANOL-$d_4$, 400 MHz) $\delta$ 8.98 (dd, 1H, *J*=1.6, 4.2 Hz), 8.66 (dd, 1H, *J*=1.6, 8.6 Hz), 8.15 (d, 1H, *J*=7.9 Hz), 7.81 (d, 1H, *J*=6.2 Hz), 7.64 (dd, 1H, *J*=4.3, 8.6 Hz), 7.25 (d, 1H, *J*=7.9 Hz), 6.07 (d, 1H, *J*=6.4 Hz), 4.1-4.2 (m, 1H), 4.1-4.1 (m, 1H), 3.6-3.9 (m, 8H), 3.4-3.6 (m, 6H), 2.6-2.7 (m, 6H), 1.25 (d, 3H, *J*=6.2 Hz).

**Example 111**

**8-[*(2S,6R)*-2-[[4-[2-[(*3R,4S*)-4-amino-3-fluoro-1-piperidyl]pyrimidin-4-yl]piperazin-1-yl]methyl]-6-methyl-morpholin-4-yl]quinoxaline-5-carbonitrile**

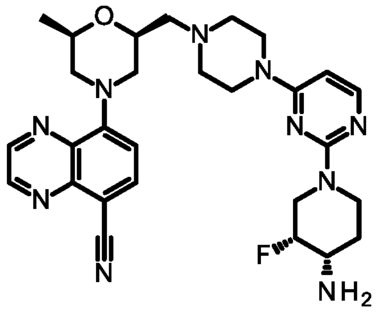

The title compound was prepared in analogy to the preparation of Example **23** by using ((*2R,6R*)-4-(8-cyanoquinoxalin-5-yl)-6-methylmorpholin-2-yl)methyl trifluoromethanesulfonate (Intermediate **C**) and *tert*-butyl *N-[(3R,4S)*-3-fluoro-4-piperidyl]carbamate (PharmaBlock, PBZS1007, CAS:1630815-57-4) instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 111** (21 mg) was obtained as a light yellow solid. MS: calc'd 547 ($MH^+$), measured 547 ($MH^+$). $^1H$ NMR (METHANOL-$d_4$, 400 MHz) δ 8.99 (d, 1H, *J*=1.8 Hz), 8.92 (d, 1H, *J*=1.7 Hz), 8.17 (d, 1H, *J*=8.3 Hz), 7.92 (d, 1H, *J*=7.5 Hz),7.29 (d, 1H, *J*=8.3 Hz), 6.72 (d, 1H, *J*=7.6 Hz), 5.1-5.3 (m, 2H), 4.9-5.0 (m, 1H), 4.6-4.7 (m, 1H), 4.4-4.6 (m, 2H), 4.3-4.3 (m, 1H), 4.1-4.2 (m, 2H), 3.54 (s, 11H), 2.8-3.0 (m, 2H), 2.0-2.2 (m, 2H), 1.3-1.4 (m, 3H).

**Example 112**

**8-[(*2S,6R*)-2-[[4-[2-(3-aminoazetidin-1-yl)-6-methyl-pyrimidin-4-yl]piperazin-1-yl]methyl]-6-methyl-morpholin-4-yl]quinoxaline-5-carbonitrile**

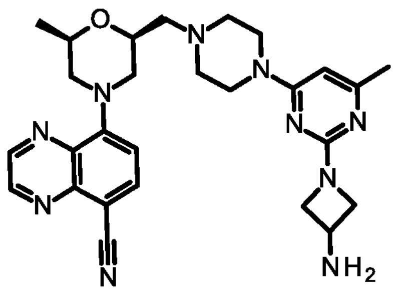

The title compound was prepared in analogy to the preparation of **Example 45** by using 2,4-dichloro-6-methylpyrimidine and ((*2R,6R*)-4-(8-cyanoquinoxalin-5-yl)-6-methylmorpholin-2-yl)methyl trifluoromethanesulfonate (Intermediate **C**) and *tert*-butyl *N*-(azetidin-3-yl)carbamate instead of 2,4-dichloro-5-fluoropyrimidine (compound **45d**) and ((*2R,6R*)-4-(8-cyanoquinolin-5-yl)-6-methylmorpholin-2-yl)methyl trifluoromethanesulfonate (Intermediate **A**) and *tert*-butyl piperazine-1-carboxylate (compound **45a**). **Example 112** (20 mg) was obtained as a light yellow solid. MS: calc'd 515 ($MH^+$), measured 515 ($MH^+$). $^1H$ NMR (METHANOL-$d_4$, 400 MHz) δ 8.99 (d, 1H, *J*=1.7 Hz), 8.92 (d, 1H, *J*=1.8 Hz), 8.17 (d, 1H, *J*=8.3 Hz), 7.29 (d, 1H, *J*=8.4 Hz), 6.56 (s, 1H), 4.68 (dd, 2H, *J*=7.6, 10.3 Hz), 4.5-4.6 (m, 1H), 4.3-4.4 (m, 4H), 4.1-4.2 (m, 2H), 3.4-4.0 (m, 10H), 2.8-2.9 (m, 2H), 2.44 (s, 3H), 1.36 (d, 3H, *J*=6.2 Hz).

**Example 113**

**8-[*(2S,6R)*-2-[[4-[2-[(*3R,4R*)-4-amino-3-fluoro-1-piperidyl]pyrimidin-4-yl]piperazin-1-yl]methyl]-6-methyl-morpholin-4-yl]quinoxaline-5-carbonitrile**

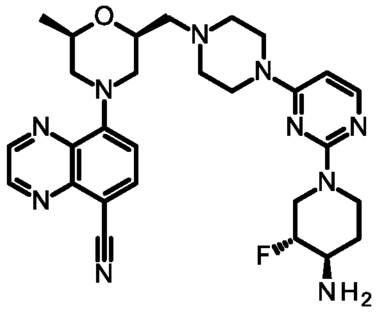

The title compound was prepared in analogy to the preparation of Example **23** by using ((*2R,6R*)-4-(8-cyanoquinoxalin-5-yl)-6-methylmorpholin-2-yl)methyl trifluoromethanesulfonate (Intermediate **C**) and *tert*-butyl *N*-[(*3R,4R*)-3-fluoro-4-piperidyl]carbamate (PharmaBlock, PB08067, CAS:1523530-29-1) instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 113** (17 mg) was obtained as a light yellow solid. MS: calc'd 547 ($MH^+$), measured 547 ($MH^+$). $^1H$ NMR (METHANOL-$d_4$, 400 MHz) δ 8.87 (d, 1H, *J*=1.8 Hz), 8.79 (d, 1H, *J*=1.7 Hz), 8.05 (d, 1H, *J*=8.3 Hz), 7.83 (d, 1H, *J*=7.3 Hz), 7.16 (d, 1H, *J*=8.4 Hz), 6.54 (d, 1H, *J*=7.3 Hz), 4.66 (dt, 1H, *J*=5.0, 9.7 Hz), 4.54 (dt, 1H, *J*=5.3, 9.8 Hz), 4.3-4.5 (m, 2H), 4.2-4.2 (m, 1H), 4.0-4.2 (m, 5H), 3.4-3.6 (m, 5H), 3.3-3.4 (m, 3H), 3.1-3.2 (m, 2H), 2.7-2.8 (m, 2H), 2.1-2.2 (m, 1H), 1.6-1.8 (m, 1H), 1.23 (d, 3H, *J*=6.1 Hz).

**Example 114**

**8-[(*2R,6S*)-2-methyl-6-[[4-(4-methyl-6-piperazin-1-yl-pyrimidin-2-yl)piperazin-1-yl]methyl]morpholin-4-yl]quinoxaline-5-carbonitrile**

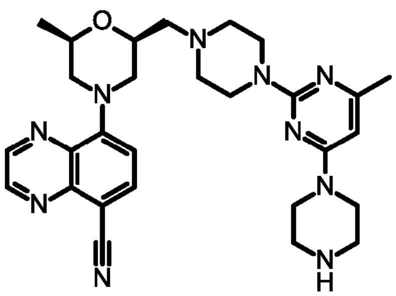

The title compound was prepared in analogy to the preparation of Example **104** by using *tert*-butyl piperazine-1-carboxylate instead of *tert*-butyl *N-[(3S,4S)*-4-methoxypyrrolidin-3-yl]carbamate (compound **104e**). **Example 114** (13 mg) was obtained as a light yellow solid. MS: calc'd 529 ($MH^+$), measured 529 ($MH^+$). $^1H$ NMR (METHANOL-$d_4$, 400 MHz) δ 8.98 (d, 1H, *J*=1.8 Hz), 8.90 (d, 1H, *J*=1.7 Hz), 8.16 (d, 1H, *J*=8.3 Hz), 7.27 (d, 1H, *J*=8.3 Hz), 6.31 (s, 1H), 4.6-4.8 (m, 1H), 4.5-4.5 (m, 1H), 4.3-4.3 (m, 1H), 4.15 (br d, 8H, *J*=2.0 Hz), 3.6-3.9 (m, 6H), 3.5-3.5 (m, 1H), 3.5-3.5 (m, 3H), 3.4-3.5 (m, 2H), 2.8-2.9 (m, 2H), 2.46 (s, 3H), 1.34 (d, 3H, *J*=6.1 Hz).

**Example 115**

**8-[*(2S,6R)*-2-[[4-[4-[(*3R,4S*)-3-amino-4-fluoro-pyrrolidin-1-yl]-6-methyl-pyrimidin-2-yl]piperazin-1-yl]methyl]-6-methyl-morpholin-4-yl]quinoxaline-5-carbonitrile**

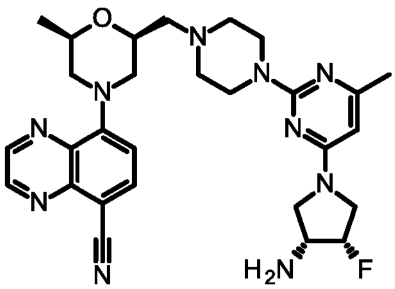

The title compound was prepared in analogy to the preparation of Example **104** by using *tert*-butyl *N*-[(*3R,4S*)-4-fluoropyrrolidin-3-yl]carbamate instead of *tert*-butyl *N-((3S,4S)-4*-methoxypyrrolidin-3-yl]carbamate (compound **104e**). **Example 115** (22 mg) was obtained as a light yellow solid. MS: calc'd 547 ($MH^+$), measured 547 ($MH^+$). $^1H$ NMR (METHANOL-$d_4$, 400 MHz) $\delta$ 9.00 (d, 1H, *J*=1.8 Hz), 8.92 (d, 1H, *J*=1.7 Hz), 8.18 (d, 1H, *J*=8.3 Hz), 7.30 (d, 1H, *J*=8.3 Hz), 6.3-6.4 (m, 1H), 5.5-5.7 (m, 1H), 4.4-4.6 (m, 2H), 4.19 (br d, 9H, *J*=11.7 Hz), 3.6-3.8 (m, 4H), 3.4-3.6 (m, 4H), 2.8-2.9 (m, 2H), 2.48 (s, 3H), 1.36 (d, 3H, *J*=6.1 Hz).

**Example 116**

**(2R, 6S)-2-methyl-4-(8-methylquinoxalin-5-yl)-6-[[4-(4-piperazin-1-ylpyrimidin-2-yl)piperazin-1 -yl] methyl] morpholine**

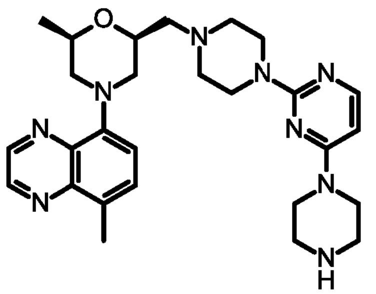

The title compound was prepared in analogy to the preparation of Example **6** by using [(*2R,6R*)-6-methyl-4-(8-methylquinoxalin-5-yl)morpholin-2-yl] methyl trifluoromethanesulfonate (Intermediate **G**) and 4-chloro-2-piperazin-1-yl-pyrimidine and instead of [(*2R,6R*)-4-(8-cyano-5-quinolyl)-6-methyl-morpholin-2-yl] methyl trifluoromethanesulfonate (Intermediate **A**) and 2-bromo-5-(piperazin-1-yl)pyrazine (compound **6a**). **Example 116** (16 mg) was obtained as a light yellow solid. MS: calc'd 504 ($MH^+$), measured 504 ($MH^+$). $^1H$ NMR (METHANOL-$d_4$, 400 MHz) $\delta$ 8.87 (d, 1H, *J*=1.7 Hz), 8.81 (d, 1H, *J*=1.7 Hz), 7.84 (d, 1H, *J*=7.5 Hz), 7.6-7.6 (m, 1H), 7.40 (d, 1H, *J*=7.8 Hz), 6.62 (d, 1H, *J*=7.5 Hz), 4.5-4.6 (m, 2H), 3.9-4.3 (m, 6H), 3.5-3.9 (m, 6H), 3.3-3.5 (m, 8H), 2.8-2.9 (m, 2H), 2.64 (s, 3H), 1.25 (d, 3H, *J*=6.2 Hz).

**Example 117**

**[(*2R,6S*)-2-methyl-4-(8-methylquinoxalin-5-yl)-6-[[4-(2-piperazin-1-ylpyrimidin-4-yl)piperazin-1-yl]methyl]morpholine**

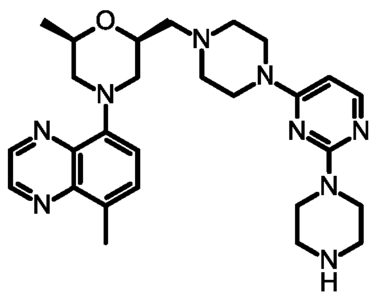

The title compound was prepared in analogy to the preparation of Example **23** by using [(*2R*,*6R*)-6-methy1-4-(8-methylquinoxalin-5-yl)morpholin-2-yl]methyl trifluoromethanesulfonate (Intermediate **G**) and piperazine-1-carboxylate instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 117** (9 mg) was obtained as a light yellow solid. MS: calc'd 504 ($MH^+$), measured 504 ($MH^+$). $^1H$ NMR (METHANOL-$d_4$, 400 MHz) δ 8.96 (d, 1H, *J*=1.6 Hz), 8.90 (d, 1H, *J*=1.6 Hz), 7.96 (d, 1H, *J*=7.5 Hz), 7.7-7.7 (m, 1H), 7.43 (d, 1H, *J*=7.8 Hz), 6.75 (d, 1H, *J*=7.5 Hz), 4.6-4.7 (m, 2H), 4.2-4.3 (m, 1H), 4.11 (br s, 5H), 3.7-3.9 (m, 4H), 3.4-3.6 (m, 9H), 2.8-2.9 (m, 2H), 2.75 (s, 3H), 1.3-1.4 (m, 4H).

**Example 118**

**8-[*(2S,6R)*-2-[[4-[4-(3-aminoazetidin-1-yl)pyrimidin-2-yl]piperazin-1-yl]methyl]-6-methyl-morpholin-4-yl]quinoxaline-5-carbonitrile**

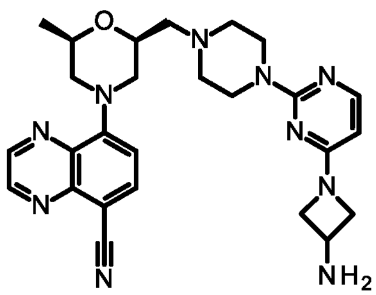

The title compound was prepared in analogy to the preparation of Example **97** by using ((*2R,6R*)-4-(8-cyanoquinoxalin-5-yl)-6-methylmorpholin-2-yl)methyl trifluoromethanesulfonate (Intermediate **C**) and *tert*-butyl *N*-(azetidin-3-yl)carbamate instead of ((*2R,6R*)-4-(8-cyanoquinolin-5-yl)-6-methylmorpholin-2-yl)methyl trifluoromethanesulfonate (Intermediate **A**) and *tert*-butyl *N*-[(*3S,4S*)-4-methoxypyrrolidin-3-yl]carbamate (compound **97d**). **Example 118** (30 mg) was obtained as a light yellow solid. MS: calc'd 501 ($MH^+$), measured 501 ($MH^+$). $^1H$ NMR (METHANOL-$d_4$, 400 MHz) δ 8.98 (d, 1H, *J*=1.8 Hz), 8.90 (d, 1H, *J*=1.8 Hz), 8.15 (d, 1H, *J*=8.3 Hz), 7.87 (d, 1H, *J*=7.1 Hz), 7.26 (d, 1H, *J*=8.3 Hz), 6.22 (d, 1H, *J*=7.1 Hz), 4.6-4.7 (m, 2H), 4.4-4.5 (m, 1H), 4.3-4.4 (m, 4H), 4.0-4.2 (m, 6H), 3.5-3.7 (m, 4H), 3.4-3.5 (m, 2H), 2.8-2.9 (m, 2H), 1.34 (d, 3H, *J*=6.1 Hz).

**Example 119**

**8-[*(2S,6R)*-2-[[4-[4-[(*3R*,*4R*)-3-amino-4-fluoro-pyrrolidin-1-yl]pyrimidin-2-yl]piperazin-1-yl]methyl]-6-methyl-morpholin-4-yl]quinoxaline-5-carbonitrile**

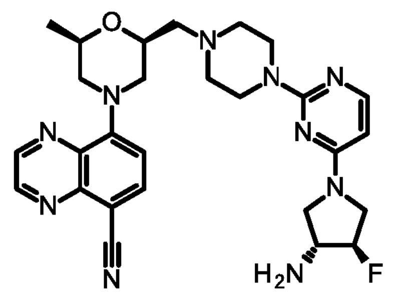

The title compound was prepared in analogy to the preparation of Example **97** by using ((*2R,6R*)-4-(8-cyanoquinoxalin-5-yl)-6-methylmorpholin-2-yl)methyl trifluoromethanesulfonate (Intermediate **C**) and *tert*-butyl *N*-[(*3R*,*4R*)-4-fluoropyrrolidin-3-yl]carbamate instead of ((*2R,6R*)-4-(8-cyanoquinolin-5-yl)-6-methylmorpholin-2-yl)methyl trifluoromethanesulfonate (Intermediate **A**) and tert-butyl *N*-[(*3S,4S*)-4-methoxypyrrolidin-3-yl]carbamate (compound **97d**). **Example 119** (29 mg) was obtained as a light yellow solid. MS: calc'd 533 ($MH^+$), measured 533 ($MH^+$). $^1H$ NMR (METHANOL-$d_4$, 400 MHz) δ 9.00 (d, 1H, *J*=1.8 Hz), 8.91 (d, 1H, *J*=1.7 Hz), 8.18 (d, 1H, *J*=8.3 Hz), 7.97 (d, 1H, *J*=6.8 Hz), 7.28 (d, 1H, *J*=8.3 Hz), 6.31 (d, 1H, *J*=6.8 Hz), 5.4-5.6 (m, 1H), 4.4-4.5 (m, 1H), 4.2-4.3 (m, 3H), 3.9-4.2 (m, 9H), 3.5-3.6 (m, 4H), 3.4-3.4 (m, 2H), 2.8-2.9 (m, 2H), 1.35 (d, 3H, *J*=6.1 Hz).

### Example 120

**8-[*(2S,6R)*-2-[[4-[4-[(*3S*,*4R*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile**

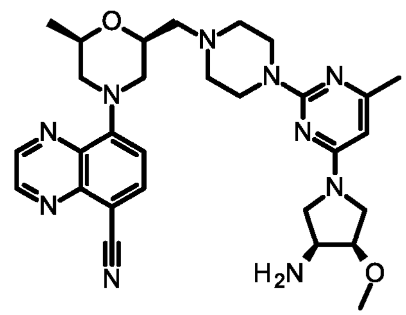

The title compound was prepared in analogy to the preparation of Example **104** by using *tert*-butyl *N-[(3S,4R)*-4-methoxypyrrolidin-3-yl]carbamate instead of *tert*-butyl *N-[(3S,4S)-4-*methoxypyrrolidin-3-yl]carbamate (compound **104e**). **Example 120** (18 mg) was obtained as a light yellow solid. MS: calc'd 559 ($MH^+$), measured 559 ($MH^+$). $^1H$ NMR (METHANOL-$d_4$, 400 MHz) δ 8.99 (d, 1H, *J*=1.8 Hz), 8.91 (d, 1H, 7=1.7 Hz), 8.17 (d, 1H, *J*=8.3 Hz), 7.28 (d, 1H, *J*=8.3 Hz), 6.28 (br s, 1H), 4.4-4.5 (m, 1H), 4.31 (br d, 2H, *J*=11.9 Hz), 4.0-4.3 (m, 8H), 3.8-4.0 (m, 1H), 3.79 (dd, 1H, *J*=4.3, 13.1 Hz), 3.60 (br s, 5H), 3.52 (s, 3H), 3.4-3.5 (m, 2H), 2.86 (dt, 2H, *J*=10.8, 12.8 Hz), 2.44 (s, 3H), 1.34 (d, 3H, *J*=6.1 Hz).

### Example 121

**8-[*(2S,6R)*-2-[[4-[4-[(*3R*,*4R*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile**

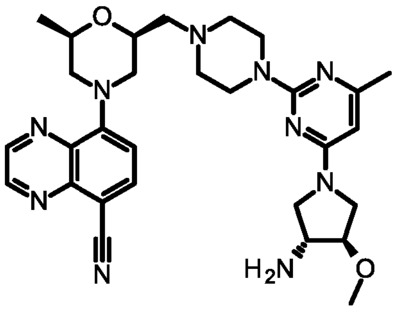

The title compound was prepared in analogy to the preparation of Example **104** by using *tert*-butyl *N-[(3R,4R)*-4-methoxypyrrolidin-3-yl]carbamate instead of *tert*-butyl *N-((3S,4S)-4*-methoxypyrrolidin-3-yl]carbamate (compound 104e). **Example 121** (19 mg) was obtained as a light yellow solid. MS: calc'd 559 ($MH^+$), measured 559 ($MH^+$). $^1H$ NMR (METHANOL-$d_4$, 400 MHz) δ 9.00 (d, 1H, *J*=1.7 Hz), 8.91 (d, 1H, *J*=1.7 Hz), 8.17 (d, 1H, *J*=8.3 Hz), 7.28 (d, 1H, *J*=8.3 Hz), 6.27 (s, 1H), 4.4-4.5 (m, 1H), 4.31 (br d, 1H, *J*=12.2 Hz), 4.1-4.3 (m, 6H), 4.0-4.1 (m, 4H), 3.7-3.9 (m, 2H), 3.5-3.7 (m, 4H), 3.50 (s, 3H), 3.4-3.4 (m, 2H), 2.8-2.9 (m, 2H), 2.43 (s, 3H), 1.35 (d, 3H, *J*=6.2 Hz).

**Example 122**

**8-[(*2S,6R*)-2-[[4-[4-[(*3R,4S*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile**

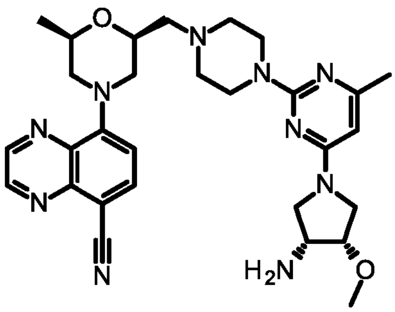

The title compound was prepared in analogy to the preparation of Example **104** by using *tert*-butyl *N-[(3R,4S)*-4-methoxypyrrolidin-3-yl]carbamate instead of *tert*-butyl *N-[(3S,4S)-4*-methoxypyrrolidin-3-yl]carbamate (compound **104e**). **Example 122** (19 mg) was obtained as a light yellow solid. MS: calc'd 559 ($MH^+$), measured 559 ($MH^+$). $^1H$ NMR (METHANOL-$d_4$, 400 MHz) δ 8.98 (d, 1H, *J*=1.5 Hz), 8.9-8.9 (m, 1H), 8.15 (dd, 1H, *J*=2.2, 8.3 Hz), 7.26 (d, 1H, *J*=8.3 Hz), 6.29 (br s, 1H), 4.48 (br t, 1H, *J*=9.8 Hz), 4.31 (br d, 2H, *J*=12.0 Hz), 4.0-4.3 (m, 8H), 3.91 (br d, 1H, *J*=11.4 Hz), 3.7-3.8 (m, 2H), 3.6-3.7 (m, 4H), 3.52 (s, 3H), 3.4-3.5 (m, 2H), 2.8-2.9 (m, 2H), 2.44 (s, 3H), 1.34 (d, 3H, *J*=6.2 Hz).

**Example 123**

**8-[(*2S,6R*)-2-[[4-[4-(3-aminoazetidin-1-yl)-6-methyl-pyrimidin-2-yl]piperazin-1-yl]methyl]-6-methyl-morpholin-4-yl]quinoxaline-5-carbonitrile**

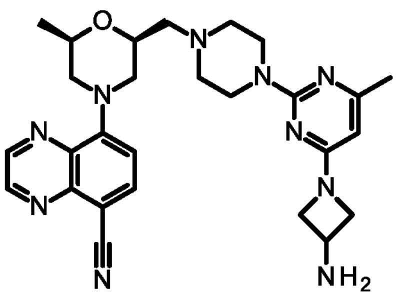

The title compound was prepared in analogy to the preparation of Example **104** by using *tert*-butyl *N*-(azetidin-3-yl)carbamate instead of *tert*-butyl *N-[(3S,4S)*-4-methoxypyrrolidin-3-yl]carbamate (compound **104e**). **Example 123** (15 mg) was obtained as a light yellow solid. MS: calc'd 515 ($MH^+$), measured 515 ($MH^+$). $^1H$ NMR (METHANOL-$d_4$, 400 MHz) $\delta$ 9.00 (d, 1H, *J*=1.7 Hz), 8.91 (d, 1H, *J*=1.8 Hz), 8.17 (d, 1H, *J*=8.2 Hz), 7.28 (d, 1H, *J*=8.3 Hz), 6.07 (s, 1H), 4.6-4.6 (m, 2H), 4.4-4.5 (m, 1H), 4.3-4.4 (m, 4H), 3.9-4.2 (m, 6H), 3.5-3.6 (m, 4H), 3.4-3.4 (m, 2H), 2.8-2.9 (m, 2H), 2.39 (s, 3H), 1.34 (d, 3H, *J*=6.1 Hz).

**Example 124**

**8-[*(2S,6R)*-2-[[4-[4-(3-aminoazetidin-1-yl)-6-methyl-pyrimidin-2-yl]piperazin-1-yl]methyl]-6-methyl-morpholin-4-yl]-2,3-dideuterio-quinoxaline-5-carbonitrile**

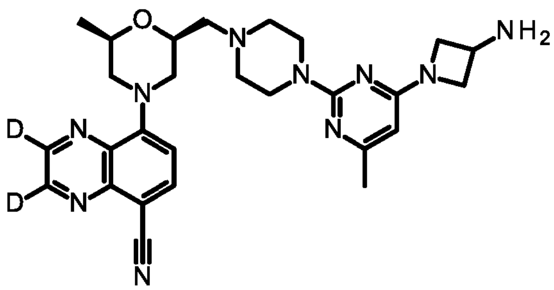

The title compound was prepared in analogy to the preparation of Example **104** by using [(*2R,6R*)-4-(8-cyano-2,3-dideuterio-quinoxalin-5-yl)-6-methyl-morpholin-2-yl]methyl trifluoromethanesulfonate (Intermediate **K**) and *tert*-butyl *N*-(azetidin-3-yl)carbamate instead of ((*2R,6R*)-4-(8-cyanoquinoxalin-5-yl)-6-methylmorpholin-2-yl)methyl trifluoromethanesulfonate (Intermediate **C**) and *tert*-butyl *N-[(3S,4S)*-4-methoxypyrrolidin-3-yl]carbamate (compound **104e**). **Example 124** (18 mg) was obtained as a light yellow solid. MS: calc'd 517 ($MH^+$), measured 517 ($MH^+$). $^1H$ NMR (METHANOL-$d_4$, 400 MHz) $\delta$ 8.12 (d, 1H, *J*=8.4 Hz), 7.21 (d, 1H, *J*=8.4 Hz), 5.57 (s, 1H), 4.5-4.6 (m, 1H), 4.1-4.4 (m, 5H), 3.8-4.1 (m, 2H), 3.6-3.8 (m, 6H), 2.5-2.8 (m, 6H), 2.17 (s, 3H), 1.2-1.4 (m, 4H).

**Example 125**

**8-[*(2S,6R)*-2-[[4-[4-[(*3R,4R)*-4-amino-3-hydroxy-3-methyl-pyrrolidin-1-yl]-6-methyl-pyrimidin-2-yl]piperazin-1-yl]methyl]-6-methyl-morpholin-4-yl]quinoxaline-5-carbonitrile**

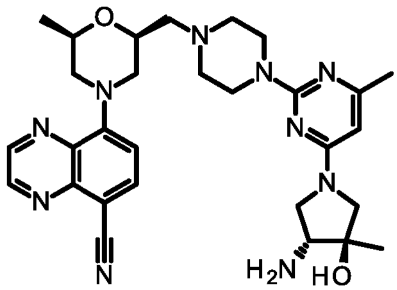

The title compound was prepared in analogy to the preparation of Example **104** by using *tert*-butyl *N*-[(*3R,4R*)-4-hydroxy-4-methyl-pyrrolidin-3-yl]carbamate instead of *tert*-butyl *N-[(3S,4S)*-4-methoxypyrrolidin-3-yl]carbamate (compound **104e**). **Example 125** (11 mg) was obtained as a light yellow solid. MS: calc'd 559 ($MH^+$), measured 559 ($MH^+$). $^1H$ NMR (METHANOL-$d_4$, 400 MHz) $\delta$ 8.99 (d, 1H, *J*=1.8 Hz), 8.91 (d, 1H, *J*=1.8 Hz), 8.17 (d, 1H, *J*=8.3 Hz), 7.28 (d, 1H, *J*=8.4 Hz), 6.2-6.3 (m, 1H), 4.4-4.5 (m, 1H), 4.3-4.3 (m, 1H), 4.1-4.2 (m, 6H), 3.6-3.8 (m, 3H), 3.5-3.6 (m, 4H), 3.4-3.5 (m, 3H), 2.8-2.9 (m, 2H), 2.4-2.4 (m, 3H), 1.53 (s, 2H), 1.4-1.4 (m, 2H), 1.3-1.4 (m, 3H).

**Example 126**

**2-[4-[4-[[*(2S,6R)*-6-methyl-4-(8-methylquinoxalin-5-yl)morpholin-2-yl]methyl]piperazin-1-yl]pyrimidin-2-yl]-5-oxa-2,8-diazaspiro[3.5]nonane**

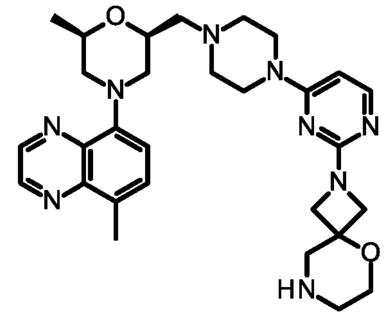

The title compound was prepared in analogy to the preparation of Example **23** by using [(2*R,6R*)-6-methyl-4-(8-methylquinoxalin-5-yl)morpholin-2-yl]methyl trifluoromethanesulfonate (Intermediate **G**) and *tert*-butyl 5-oxa-2,8-diazaspiro[3.5]nonane-8-carboxylate (PharmaBlock, PBN20111065, CAS: 1251005-61-4) instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 126** (11 mg) was obtained as a light yellow solid. MS: calc'd 546 ($MH^+$), measured 546 ($MH^+$). $^1H$ NMR (METHANOL-$d_4$, 400 MHz) $\delta$ 8.89 (d, 1H, *J*=1.7 Hz), 8.83 (d, 1H, *J*=1.8 Hz), 7.89 (d, 1H, *J*=7.5 Hz), 7.6-7.6 (m, 1H), 7.23 (d, 1H, *J*=7.9 Hz), 6.62 (d, 1H, *J*=7.5 Hz), 4.4-4.5 (m, 1H), 4.37 (d, 2H, *J*=10.3 Hz), 4.2-4.3 (m, 3H), 4.1-4.2 (m, 3H), 3.9-4.0 (m, 3H), 3.78 (br d, 1H, *J*=11.2 Hz), 3.69 (br d, 1H, *J*=11.5 Hz), 3.5-3.6 (m, 6H), 3.4-3.4 (m, 2H), 3.3-3.3 (m, 2H), 2.69 (s, 3H), 2.63 (dd, 2H, *J*=10.5, 11.1 Hz), 1.31 (d, 3H, *J*=6.4 Hz).

**Example 127**

**(*2R,6S*)-2-methyl-6-[[4-[2-[(3S)-3-methylpiperazin-1-yl]pyrimidin-4-yl]piperazin-1-yl]methyl]-4-(8-methylquinoxalin-5-yl)morpholine**

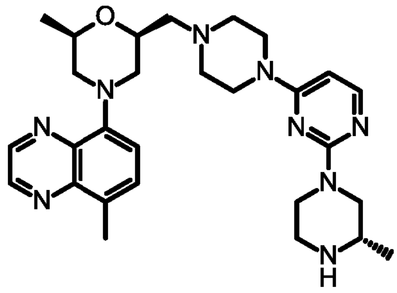

The title compound was prepared in analogy to the preparation of Example **23** by using [(*2R*,*6R*)-6-methyl-4-(8-methylquinoxalin-5-yl)morphofin-2-yl]methyl trifluoromethanesulfonate (Intermediate **G**) and *tert*-butyl (2S)-2-methylpiperazine-1-carboxylate (Bidepharm, BD12408, CAS:169447-70-5) instead of ((*2R,6R*)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 127** (9 mg) was obtained as a light yellow solid. MS: calc'd 518 ($MH^+$), measured 518 ($MH^+$). $^1H$ NMR (METHANOL-$d_4$, 400 MHz) δ 8.89 (d, 1H, *J*=1.7 Hz), 8.83 (d, 1H, *J*=1.7 Hz), 7.98 (d, 1H, *J*=7.1 Hz), 7.6-7.6 (m, 1H), 7.23 (d, 1H, *J*=7.8 Hz), 6.57 (d, 1H, *J*=7.1 Hz), 4.5-4.7 (m, 3H), 4.0-4.3 (m, 4H), 3.78 (br d, 1H, *J*= 11.4 Hz), 3.69 (br d, 1H, *J*=11.6 Hz), 3.3-3.7 (m, 10H), 3.2-3.3 (m, 2H), 2.69 (s, 3H), 2.6-2.7 (m, 2H), 1.41 (d, 3H, *J*=6.6 Hz), 1.31 (d, 3H, *J*=6.2 Hz).

**Example 128**

**(*2R,6S*)-2-methyl-6-[[4-[2-[(3*R*)-3-methylpiperazin-1-yl]pyrimidin-4-yl]piperazin-1-yl]methyl]-4-(8-methylquinoxalin-5-yl)morpholine**

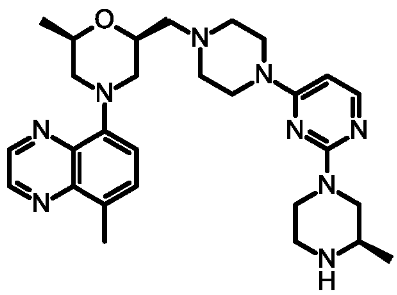

The title compound was prepared in analogy to the preparation of Example **23** by using [(*2R*,*6R*)-6-methyl-4-(8-methylquinoxalin-5-yl)morpholin-2-yl]methyl trifluoromethanesulfonate (Intermediate **G**) and *tert*-butyl (2*R*)-2-methylpiperazine-1-carboxylate (Accela ChemBio Inc , SY007943, CAS:170033-47-3) instead of ((2R,6R)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 128** (12 mg) was obtained as a light yellow solid. MS: calc'd 518 ($MH^+$), measured 518 ($MH^+$). $^1H$ NMR (METHANOL-$d_4$, 400 MHz) δ 8.89 (d, 1H, *J*=1.8 Hz), 8.83 (d, 1H, *J*=1.8 Hz), 7.97 (d, 1H, *J*=7.2 Hz), 7.6-7.6 (m, 1H), 7.23 (d, 1H, *J*=7.8 Hz), 6.62 (d, 1H, *J*=7.2 Hz), 4.5-4.6 (m, 3H), 3.9-4.4 (m, 5H), 3.78 (br d, 1H, *J*=11.4 Hz), 3.69 (br d, 1H, *J*=11.5 Hz), 3.4-3.7 (m, 9H), 3.2-3.3 (m, 2H), 2.69 (s, 3H), 2.6-2.7 (m, 2H), 1.41 (d, 3H, *J*=6.5 Hz), 1.31 (d, 3H, *J*=6.2 Hz).

**Example 129**

**(*4aR,7aR*)-6-[4-[4-[[*(2S,6R)*-6-methyl-4-(8-methylquinoxalin-5-yl)morpholin-2-yl]methyl]piperazin-1-yl]pyrimidin-2-yl]-3,4,4*a*,*5*,*7*,*7a*-hexahydro-2*H*-pyrrolo[3,4-b][1,4]oxazine**

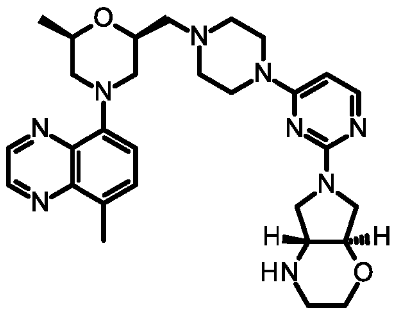

The title compound was prepared in analogy to the preparation of Example **23** by using [(*2R*,*6R*)-6-methyl-4-(8-methylquinoxalin-5-yl)morpholin-2-yl]methyl trifluoromethanesulfonate (Intermediate **G**) and *tert*-butyl (4*aR*,*7aR*)-*3*,*4a*,5,6,7,7a-hexahydro-2*H*-pyrrolo[3,4-b][1,4]oxazine-4-carboxylate (Pharmablock, PBXA8123, CAS: 1932337-68-2) instead of ((2R,6R)-4-(8-cyanoquinolin-5-yl)-6-methylmorpholin-2-yl)methyl trifluoromethanesulfonate (Intermediate **A**) and *tert*-butyl azetidin-3-ylcarbamate (compound **23c**). **Example 129** (10 mg) was obtained as a light yellow solid. MS: calc'd 546 ($MH^+$), measured 546 ($MH^+$). $^1H$ NMR (METHANOL-$d_4$, 400 MHz) δ 8.89 (d, 1H, *J*=1.7 Hz), 8.83 (d, 1H, *J*=1.8 Hz), 7.91 (d, 1H, *J*=7.5 Hz), 7.6-7.6 (m, 1H), 7.23 (d, 1H, *J*=7.8 Hz), 6.67 (d, 1H, *J*=7.6 Hz), 4.5-4.5 (m, 1H), 4.27 (br dd, 2H, *J*=3.7, 13.2 Hz), 4.1-4.2 (m, 5H), 4.01 (dt, 2H, *J*=2.5, 12.7 Hz), 3.78 (br d, 1H, *J*=11.4 Hz), 3.4-3.7 (m, 13H), 2.69 (s, 3H), 2.63 (t, 2H, *J*=10.8 Hz), 1.31 (d, 3H, *J*=6.2 Hz).

**Example 130**

**1-[4-[4-[[*(2S,6R)*-6-methyl-4-(8-methylquinoxalin-5-yl)morpholin-2-yl]methyl]piperazin-1-yl]pyrimidin-2-yl]azetidin-3-amine**

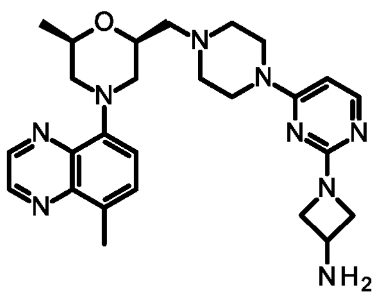

The title compound was prepared in analogy to the preparation of Example **23** by using [(*2R*,*6R*)-6-methyl-4-(8-methylquinoxalin-5-yl)morpholin-2-yl]methyl trifluoromethanesulfonate (Intermediate **G**) instead of ((*2R,6R*)-4-(8-cyanoquinolin-5-yl)-6-methylmorpholin-2-yl)methyl trifluoromethanesulfonate (Intermediate **A**). **Example 130** (10 mg) was obtained as a light yellow solid. MS: calc'd 490 ($MH^+$), measured 490 ($MH^+$). $^1H$ NMR (METHANOL-$d_4$, 400 MHz) δ 8.89 (d, 1H, *J*=1.8 Hz), 8.83 (d, 1H, *J*=1.8 Hz), 7.90 (d, 1H, *J*=7.6 Hz), 7.60 (d, 1H, *J*=7.8 Hz), 7.23 (d, 1H, *J*=7.8 Hz), 6.63 (d, 1H, *J*=7.5 Hz), 4.6-4.7 (m, 2H), 4.5-4.5 (m, 1H), 4.3-4.4 (m, 4H), 4.0-4.3 (m, 4H), 3.8-3.8 (m, 1H), 3.7-3.7 (m, 1H), 3.5-3.7 (m, 4H), 3.4-3.4 (m, 2H), 2.69 (s, 3H), 2.63 (t, 2H, *J*=10.8 Hz), 1.30 (d, 3H, *J*=6.2 Hz).

**Example 131**

**5-[*(2S,6R)*-2-[[4-[4-(3-amino-3-methyl-azetidin-1-yl)-6-methoxy-pyrimidin-2-yl]piperazin-1-yl]methyl]-6-methylmorpholin-4-yl]-2-deuterio-quinoline-8-carbonitrile**

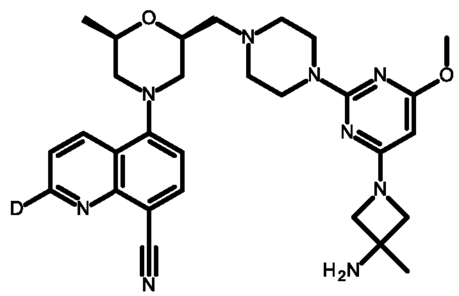

The title compound was prepared in analogy to the preparation of Example **104** by using [(2*R*,6*R*)-4-(8-cyano-2-deuterio-5-quinolyl)-6-methyl-morpholin-2-yl]methyl trifluoromethanesulfonate (intermediate **L**) and 2,4-dichloro-6-methoxy-pyrimidine and *tert*-butyl *N*-(3-methylazetidin-3-yl)carbamate instead of [(*2R,6R*)-4-(8-cyanoquinoxafin-5-yl)-6-methyl-morpholin-2-yl]methyl trifluoromethanesulfonate (intermediate *C*) and 2,4-dichloro-6-methylpyrimidine and *tert*-butyl *N-[(3S,4S)*-4-methoxypyrrolidin-3-yl]carbamate (compound **104e**). **Example 131** (29 mg) was obtained as a light yellow solid. MS: calc'd 545 ($MH^+$), measured 545 ($MH^+$). $^1H$ NMR (METHANOL-$d_4$, 400 MHz) δ 8.66 (d, 1H, *J*=8.7 Hz), 8.16 (d, 1H, *J*=7.9 Hz), 7.65 (d, 1H, *J*=8.7 Hz), 7.28 (d, 1H, *J*=8.1 Hz), 4.5-4.5 (m, 1H), 4.2-4.2 (m, 1H), 4.1-4.1 (m, 2H), 4.0-4.0 (m, 2H), 3.85 (s, 3H), 3.5-3.8 (m, 2H), 3.4-3.5 (m, 5H), 3.3-3.3 (m, 5H), 3.2-3.3 (m, 1H), 2.7-2.8 (m, 2H), 1.66 (s, 3H), 1.32 (d, 3H, *J*=6.2 Hz).

**Example 132**

**5-[*(2S,6R*)-2-[[4-[6-(3-amino-3-methyl-azetidin-1-yl)-3-pyridyl]piperazin-1-yl]methyl]-6-methyl-morpholin-4-yl]-2-deuterio-quinoline-8-carbonitrile**

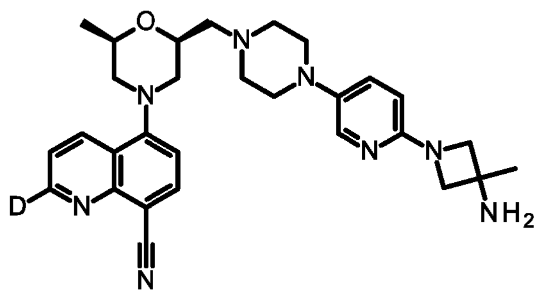

The title compound was prepared according to the following scheme:

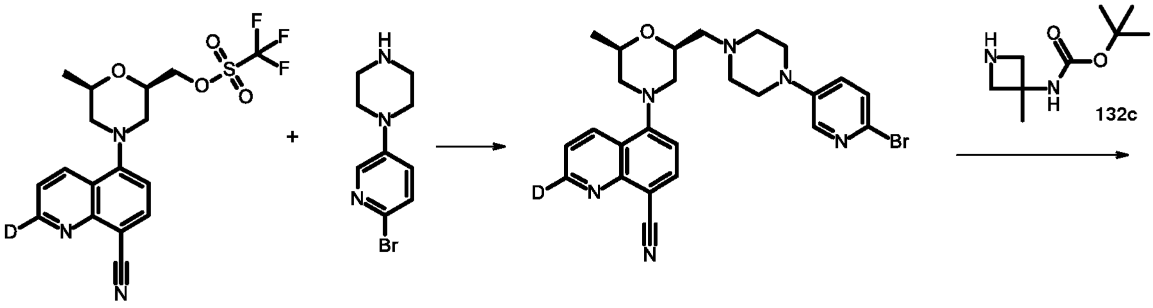

132c

Intermediate L 132a 132b

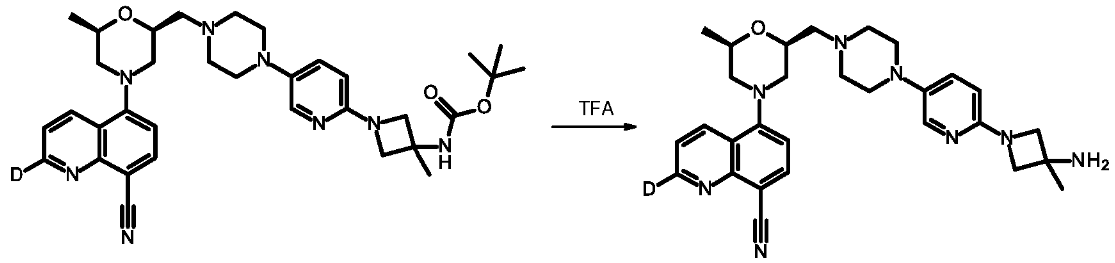


132d 132

**Step 1: 2-deuterio-5-[*(2S,6R)*-2-[[4-(6-bromo-3-pyridyl)piperazin-1-yl]methyl]-6-methyl-morpholin-4-yl]quinoline-8-carbonitrile (compound 132b)**

A solution of 1-(6-bromopyridin-3-yl)piperazine (compound **132a**, 1.5 g, 6.2 mmol) and ((*2R,6R*)-4-(8-cyanoquinofin-5-yl-2-d)-6-methylmorphofin-2-yl)methyl trifluoromethanesulfonate (Intermediate **L**, 2.58 g, 6.2 mmol) and DIPEA (2.4g, 3.25mL) in $CH_2Cl_2$ (20 mL) was stirred at room temperature for 2 hrs. The mixture was then diluted with DCM and washed with brine. The organic layer was dried over $Na_2SO_4$ and concentrated. The residue was purified by silica gel chromatography (EA/PE from 0% to 100%) to get 2-deuterio-5-[*(2S,6R)*-2-[[4-(6-bromo-3-pyridyl)piperazin-1-yl]methyl]-6-methyl-morpholin-4-yl]quinoline-8-carbonitrile (compound **132b**, 2.96 g) as a yellow solid .MS: calc'd 508 ($MH^+$), measured 508 ($MH^+$).

**Step 2 : *tert*-butyl N-[3-methyl-1-[5-[4-[[*(2S,6R)*-4-(8-cyano-2-deuterio-5-quinolyl)-6-methyl-morpholin-2-yl]methyl]piperazin-1-yl]-2-pyridyl]azetidin-3-yl]carbamate (compound 132d)**

To a solution of 2-deuterio-5-[*(2S,6R)*-2-[[4-(6-bromo-3-pyridyl)piperazin-1-yl]methyl]-6-methyl-morpholin-4-yl]quinoline-8-carbonitrile (compound **132b**, 2.4 g, 4.72 mmol) in dioxane (60 mL) was added RuPhos-Pd-G2 (367 mg, 472 μmol), *tert*-butyl (3-methylazetidin-3-yl)carbamate (compound **132c**, 1.32 g, 7.08 mmol) and $Cs_2CO_3$ (4.61 g, 14.2 mmol). The reaction mixture was stirred for 16 hours at 120 °C under $N_2$. Then the mixture was filtered and the filtrate was concentrated under reduced pressure to afford crude product, which was purified by silica gel chromatography (MeOH/DCM from 2% to 12%) to afford *tert*-butyl N-[3-methyl-1-[5-[4-[[*(2S,6R)*-4-(8-cyano-2-deuterio-5-quinolyl)-6-methyl-morpholin-2-yl]methyl]piperazin-1-yl]-2-pyridyl]azetidin-3-yl]carbamate (compound **132d**, 2.4 g) as a light yellow solid. MS: calc'd 614 ($MH^+$), measured 614 ($MH^+$).

**Step 3: 5-[*(2S,6R)*-2-[[4-[6-(3-amino-3-methyl-azetidin-1-yl)-3-pyridyl]piperazin-1-yl]methyl]-6-methyl-morpholin-4-yl]-2-deuterio-quinoline-8-carbonitrile (Example 132)**

To a solution of *tert*-butyl *N*-[3-methyl-1-[5-[4-[[*(2S,6R)*-4-(8-cyano-2-deuterio-5-quinolyl)-6-methyl-morpholin-2-yl]methyl]piperazin-l-yl]-2-pyridyl]azetidin-3-yl]carbamate (compound **132d**, 360 mg) in DCM (10 mL) was added TFA (2 mL) at 0 °C. The reaction mixture was stirred at r.t. for 2 hrs. Then the mixture was concentrated to crude product, which was purified by prep-HPLC to afford **Example 132** (102 mg) as a yellow solid. MS calc'd 514 ($MH^+$), measured 514 ($MH^+$). $^1H$ NMR (METHANOL-$d_4$, 400 MHz) δ 8.68 (d, 1H, *J*=8.6 Hz), 8.18 (d, 1H, *J*=8.1 Hz), 7.90 (dd, 1H, *J*=2.7, 9.4 Hz), 7.6-7.7 (m, 2H), 7.30 (d, 1H, *J*=8.1 Hz), 6.86 (d, 1H, *J*=9.4 Hz), 4.5-4.6 (m, 1H), 4.3-4.4 (m, 2H), 4.2-4.3 (m, 3H), 3.5-3.8 (m, 5H), 3.4-3.5 (m, 6H), 2.7-2.9 (m, 2H), 1.73 (s, 3H), 1.4-1.5 (m, 1H), 1.33 (d, 3H, *J*=6.2 Hz).

**Example 133**

**5-[*(2S,6R)*-2-[[4-[6-(3-amino-3-methyl-azetidin-1-yl)-4-methyl-3-pyridyl]piperazin-1-yl]methyl]-6-methyl-morpholin-4-yl]-2-deuterio-quinoline-8-carbonitrile**

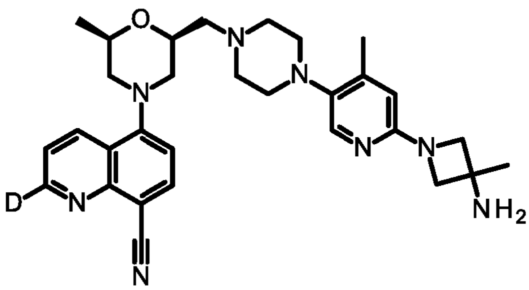

The title compound was prepared in analogy to the preparation of Example **132** by using 1-(6-bromo-4-methyl-3-pyridyl)piperazine instead of 1-(6-bromopyridin-3-yl)piperazine (compound **132a**). **Example 133** (3 mg) was obtained as a light yellow solid. MS: calc'd 528 ($MH^+$), measured 528 (MH+).$^1$H NMR (METHANOL-$d_4$, 400 MHz) δ 8.67 (d, 1H, *J*=8.6 Hz), 8.17 (d, 1H, *J*=8.1 Hz), 7.75 (s, 1H), 7.65 (d, 1H, *J*=8.6 Hz), 7.29 (d, 1H, *J*=8.1 Hz), 6.81 (s, 1H), 4.52 (dt, 1H, *J*=2.9, 8.2 Hz), 4.40 (d, 2H, *J*=9.9 Hz), 4.3-4.3 (m, 2H), 4.1-4.2 (m, 1H), 3.7-4.0 (m, 2H), 3.4-3.5 (m, 6H), 3.1-3.3 (m, 4H), 2.7-2.8 (m, 2H), 2.47 (s, 3H), 1.72 (s, 3H), 1.32 (d, 3H, *J*=6.2 Hz).

**Example 134**

**5-[*(2S,6R)*-2-[[4-[4-[(*3S,4S*)-3-amino-4-methoxy-pyrrolidin-1-yl]-6-methyl-pyrimidin-2-yl]piperazin-1-yl]methyl]-6-methyl-morpholin-4-yl]-2-deuterio-quinoline-8-carbonitrile**

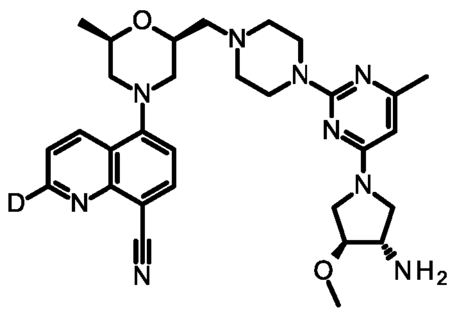

The title compound was prepared in analogy to the preparation of Example **104** by using [(*2R,6R*)-4-(8-cyano-2-deuterio-5-quinolyl)-6-methyl-morpholin-2-yl]methyl trifluoromethanesulfonate (intermediate **L)** instead of [*(2R,6R)*-4-(8-cyanoquinoxalin-5-yl)-6-methyl-morpholin-2-yl]methyl trifluoromethanesulfonate (intermediate **C)**. **Example 134** (100 mg) was obtained as a light yellow solid. MS: calc'd 559 ($MH^+$), measured 559 ($MH^+$). $^1$H NMR (METHANOL-$d_4$, 400 MHz) δ 8.67 (d, 1H, *J*=8.6 Hz), 8.15 (d, 1H, *J*=7.9 Hz), 7.64 (d, 1H, *J*=8.7 Hz), 7.25 (d, 1H, *J*=8.1 Hz), 5.70 (s, 1H), 4.2-4.3 (m, 1H), 4.0-4.1 (m, 1H), 3.7-3.8 (m, 6H), 3.6-3.7 (m, 1H), 3.4-3.5 (m, 3H), 3.40 (s, 3H), 3.4-3.4 (m, 1H), 2.5-2.7 (m, 9H), 2.19 (s, 3H), 1.26 (d, 3H, *J*=6.4 Hz).

**Example 135**

**5-[(*2S,6R*)-2-[[4-[4-(3-amino-3-methyl-azetidin-1-yl)-6-methyl-pyrimidin-2-yl]piperazin-1-yl]methyl]-6-methyl-morpholin-4-yl]-2-deuterio-quinoline-8-carbonitrile**

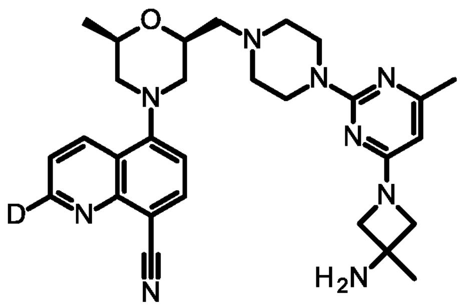

The title compound was prepared in analogy to the preparation of Example **104** by using [(2*R*,6*R*)-4-(8-cyano-2-deuterio-5-quinolyl)-6-methyl-morpholin-2-yl]methyl trifluoromethanesulfonate (intermediate **L**) and *tert*-butyl *N*-(3-methylazetidin-3-yl)carbamate instead of [(*2R,6R*)-4-(8-cyanoquinoxalin-5-yl)-6-methyl-morpholin-2-yl]methyl trifluoromethanesulfonate (intermediate **C)** and *tert*-butyl *N-[(3S,4S)*-4-methoxypyrrolidin-3-yl]carbamate (compound **104e**). **Example 135** (80 mg) was obtained as a light yellow solid. MS: calc'd 529 ($MH^+$), measured 529 ($MH^+$). $^1H$ NMR (METHANOL-$d_4$, 400 MHz) δ 8.66 (d, 1H, *J*=8.6 Hz), 8.15 (d, 1H, *J*=7.9 Hz), 7.64 (d, 1H, *J*=8.6 Hz), 7.25 (d, 1H, *J*=8.1 Hz), 5.57 (s, 1H), 4.2-4.2 (m, 1H), 4.0-4.1 (m, 1H), 3.8-3.9 (m, 2H), 3.7-3.8 (m, 6H), 3.46 (br d, 1H, *J*=12.5 Hz), 3.38 (br d, 1H, *J*=11.9 Hz), 2.5-2.7 (m, 8H), 2.16 (s, 3H), 1.46 (s, 3H), 1.25 (d, 3H, *J*=6.2 Hz).

**Example 136**

**5-[*(2S,6R)*-2-[[4-[4-(3-aminoazetidin-1-yl)-6-methyl-pyrimidin-2-yl]piperazin-1-yl]methyl]-6-methyl-morpholin-4-yl]-2-deuterio-quinoline-8-carbonitrile**

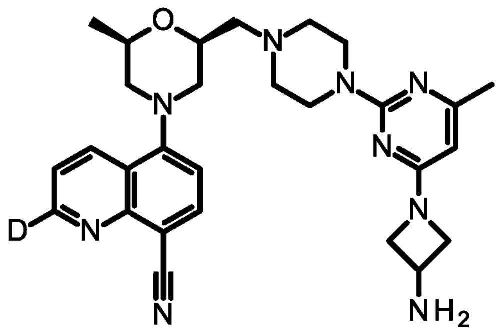

The title compound was prepared in analogy to the preparation of Example **104** by using [(2*R*,6*R*)-4-(8-cyano-2-deuterio-5-quinolyl)-6-methyl-morpholin-2-yl]methyl trifluoromethanesulfonate (intermediate **L**) and *tert*-butyl *N*-(azetidin-3-yl)carbamate instead of [(*2R,6R*)-4-(8-cyanoquinoxafin-5-yl)-6-methyl-morphofin-2-yl]methyl trifluoromethanesulfonate (intermediate **C)** and *tert*-butyl *N-[(3S,4S)*-4-methoxypyrrolidin-3-yl]carbamate (compound **104e**). **Example 136** (73 mg) was obtained as a light yellow solid. MS: calc'd 515 ($MH^+$), measured 515 ($MH^+$). $^1H$ NMR (METHANOL-$d_4$, 400 MHz) δ 8.66 (d, 1H, *J*=8.6 Hz), 8.15 (d, 1H, *J*=7.9 Hz), 7.64 (d, 1H, *J*=8.7 Hz), 7.25 (d, 1H, *J*=8.1 Hz), 5.56 (s, 1H), 4.2-4.2 (m, 3H), 4.0-4.1 (m, 1H), 3.88 (tt, 1H, *J*=5.3, 7.3 Hz), 3.74 (t, 4H, *J*=5.0 Hz), 3.67 (dd, 2H, *J*=5.3, 9.2 Hz), 3.4-3.5 (m, 1H), 3.4-3.4 (m, 1H), 2.6-2.7 (m, 4H), 2.5-2.6 (m, 4H), 2.16 (s, 3H), 1.25 (d, 3H, *J*=6.4 Hz).

**Example 137**

5-**[(*2S,6R)*-2-[[4-[6-(3-amino-3-methyl-azetidin-1-yl)-2-methyl-3-pyridyl]piperazin-1-yl]methyl]-6-methyl-morpholin-4-yl]-2-deuterio-quinoline-8-carbonitrile**

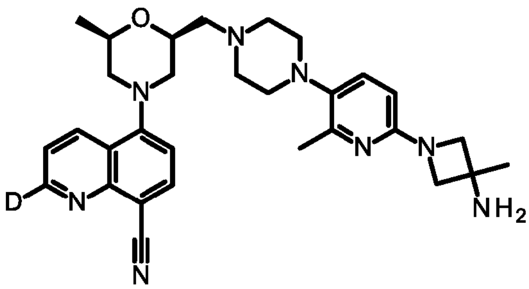

The title compound was prepared in analogy to the preparation of Example **132** by using 1-(6-bromo-2-methyl-3-pyridyl)piperazine instead of 1-(6-bromopyridin-3-yl)piperazine (compound **132a**)**. Example 132** (33 mg) was obtained as a light yellow solid. MS: calc'd 528 ($MH^+$), measured 528 ($MH^+$). $^1H$ NMR (METHANOL-$d_4$, 400 MHz) $\delta$ 8.66 (d, 1H, *J*=8.6 Hz), 8.15 (d, 1H, *J*=7.9 Hz), 7.97 (d, 1H, *J*=9.3 Hz), 7.64 (d, 1H, *J*=8.7 Hz), 7.28 (d, 1H, *J*=8.1 Hz), 6.73 (d, 1H, *J*=9.2 Hz), 4.5-4.6 (m, 1H), 4.43 (d, 2H, *J*=9.9 Hz), 4.3-4.4 (m, 2H), 4.1-4.2 (m, 1H), 3.7-3.9 (m, 2H), 3.4-3.5 (m, 6H), 3.1-3.3 (m, 4H), 2.7-2.8 (m, 2H), 2.57 (s, 3H), 1.72 (s, 3H), 1.31 (d, 3H, *J*=6.4 Hz).

**Example 138**

**5-[*(2S,6R)*-2-[[3-[6-(3-amino-3-methyl-azetidin-1-yl)-3-pyridyl]azetidin-1-yl]methyl]-6-methyl-morpholin-4-yl]-2-deuterio-quinoline-8-carbonitrile**

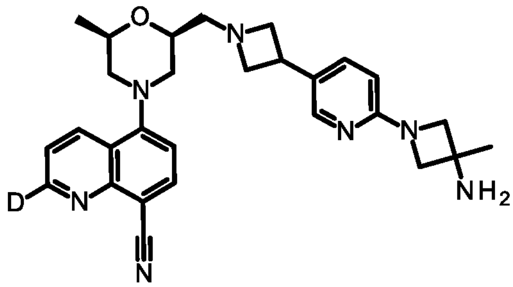

The title compound was prepared according to the following scheme:

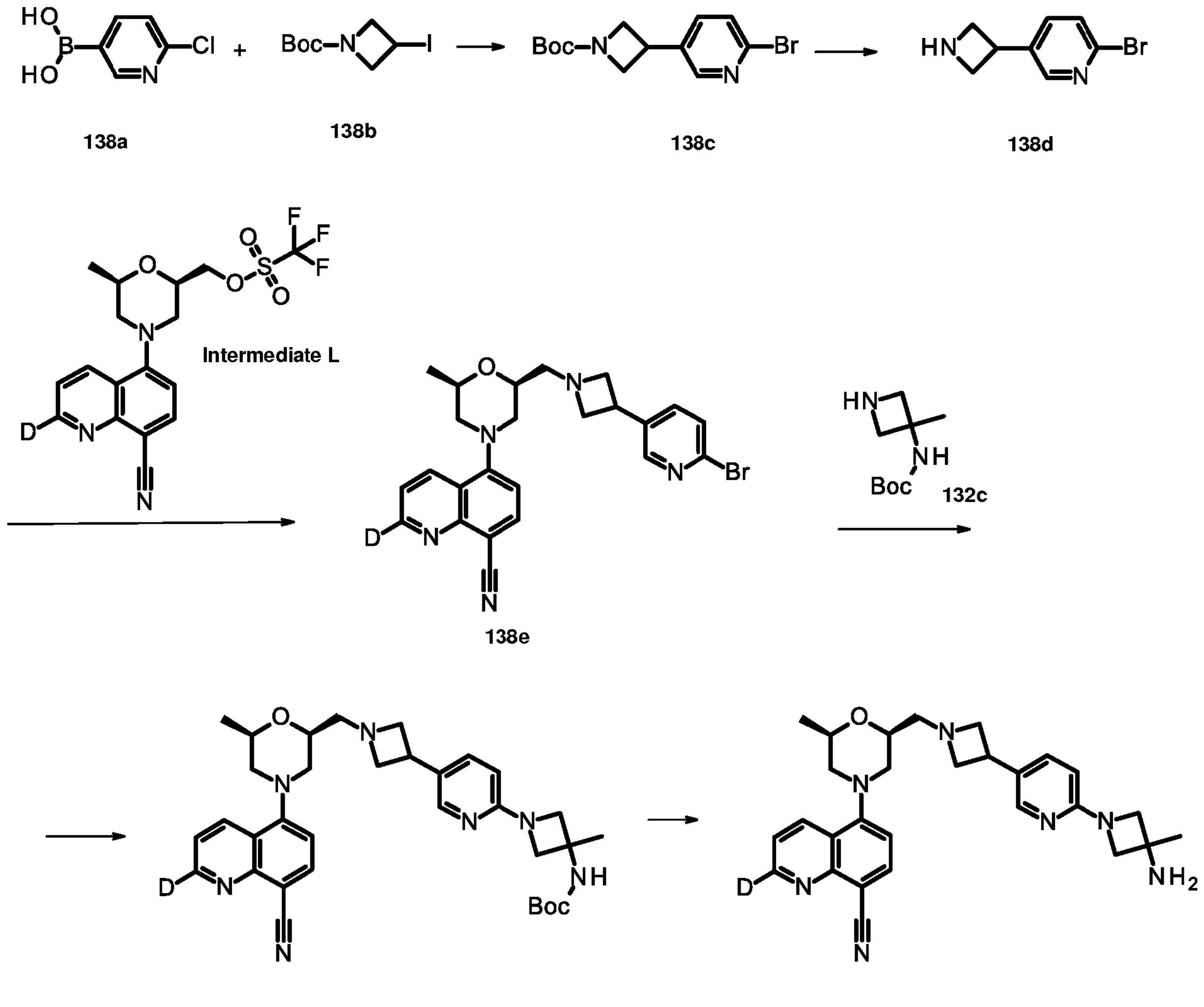

138a 138b 138c 138d

Intermediate L

138e

132c

138f 138

**Step 1: preparation of *tert*-butyl 3-(6-chloropyridin-3-yl)azetidine-1-carboxylate (compound 138c)**

To a stirred suspension of (6-chloropyridin-3-yl)boronic acid (compound **138a,** 1.4 g, 8.9 mmol) in 2-propanol (4 mL) was added a solution of *tert*-butyl 3-iodoazetidine-1-carboxylate (compound **138b,** 1.4 g, 4.95 mmol) in 2-propanol (7 mL) at room temperature. To the mixture was added rac-(1*R*,2*R*)-2-aminocyclohexan-1-ol hydrochloride (45 mg, 297 μmol), nickel(II) iodide (92.7 mg, 297 μmol) and sodium bis(trimethylsilyl)amide in THF (4.95 mL, 9.89 mmol) under argon. After the resulting mixture was stirred for 10 mins at r.t., the mixture was heated at 80°C under microwave for 1 hour. The reaction mixture was poured to a solution of water and ethyl acetate. The aqueous layer was extracted twice with ethyl acetate. The combined organic layer was dried over $MgSO_4$ and concentrated. The residue was purified by silica gel chromatography (PE/EtOAc from 10% to 60%) to afford *tert*-butyl 3-(6-chloropyridin-3-yl)azetidine-1-carboxylate (compound **138c**, 842 mg) as a colorless solid. MS: calc'd 269 ($MH^+$), measured 269 ($MH^+$).

**Step 2: preparation of 5-(azetidin-3-yl)-2-chloropyridine 2,2,2-trifluoroacetate (compound 138d)**

To a solution of *tert*-butyl 3-(6-chloropyridin-3-yl)azetidine-1-carboxylate (compound **138c,** 842 mg, 2.69 mmol) in DCM (4 mL) was added 2,2,2-trifluoroacetic acid (4 mL) at 0 °C. The reaction mixture was stirred at r.t. for 2 hrs. Then it was concentrated to get crude 5-(azetidin-3-yl)-2-chloropyridine 2,2,2-trifluoroacetate (compound **138d,** 750 mg) as a light yellow oil which was used in next step without purification. MS: calc'd 169($MH^+$), measured 169 ($MH^+$).

**Step 3: preparation of 5-((*2S,6R*)-2-((3-(6-chloropyridin-3-yl)azetidin-1-yl)methyl)-6-methylmorpholino)quinoline-8-carbonitrile-2-d (compound 138e)**

A solution of 5-(azetidin-3-yl)-2-chloropyridine 2,2,2-trifluoroacetate (compound **138d,** 377 mg, 1.15 mmol) and

[(*2R,6R*)-4-(8-cyano-2-deuterio-5-quinolyl)-6-methyl-morpholin-2-yl]methyl trifluoromethanesulfonate (Intermediate **L,** 400 mg, 961 μmol) and TEA (1 mL) in $CH_2Cl_2$ (20 mL) was stirred at r.t. for 2 hrs. Then the mixture was concentrated and purified by silica gel chromatography (PE/EtOAC from 40% to 100%) to afford 5-(*(2S,6R)*-2-((3-(6-chloropyridin-3-yl)azetidin-1-yl)methyl)-6-methylmorpholino)quinoline-8-carbonitrile-2-d (compound **138e,** 320 mg) as a yellow solid.MS: calc'd 435 ($MH^+$), measured 435 ($MH^+$).

**Step 4: preparation of *tert*-butyl (1-(5-(1-(((*2S,6R)*-4-(8-cyanoquinolin-5-yl-2-d)-6-methylmorpholin-2-yl)methyl)azetidin-3-yl)pyridin-2-yl)-3-methylazetidin-3-yl)carbamate (compound 138f)**

To a solution of 5-(*(2S,6R)*-2-((3-(6-chloropyridin-3-yl)azetidin-1-yl)methyl)-6-methylmorpholino)quinoline-8-carbonitrile-2-d (compound **138e,** 80 mg, 184 μmol) in dioxane (4 mL) was added *tert*-butyl (3-methylazetidin-3-yl)carbamate (compound **132c,** 41.1 mg, 221 μmol), RuPhos-Pd-G2 (14.3 mg, 18.4 μmol) and $Cs_2CO_3$ (120 mg, 368 μmol). The reaction mixture was stirred for 16 hours at 120°C under $N_2$. Then the mixture was filtered and the filtrate was concentrated and the crude oil was purified by silica gel chromatography (MeOH/DCM from 2% to 12%) to afford *tert*-butyl (1-(5-(1-(((*2S,6R)*-4-(8-cyanoquinofin-5-yl-2-d)-6-methylmorpholin-2-yl)methyl)azetidin-3-yl)pyridin-2-yl)-3-methylazetidin-3-yl)carbamate (compound **138f,** 79 mg) . MS: calc'd 585 ($MH^+$), measured 585 ($MH^+$).

**Step 5: preparation of 5-[*(2S,6R)*-2-[[3-[6-(3-amino-3-methyl-azetidin-1-yl)-3-pyridyl]azetidin-1-yl]methyl]-6-methyl-morpholin-4-yl]-2-deuterio-quinoline-8-carbonitrile (Example 138)**

To a solution of *tert*-butyl (1-(5-(1-(((*2S,6R)*-4-(8-cyanoquinofin-5-yl-2-d)-6-methylmorpholin-2-yl)methyl)azetidin-3-yl)pyridin-2-yl)-3-methylazetidin-3-yl)carbamate (compound **138f,** 79 mg, 135 μmol) in DCM (2 mL) was added 2,2,2-trifluoroacetic acid (740 mg, 0.5 ml, 6.49 mmol) at 0 °C. The reaction mixture was stirred at rt for 2 hrs. Then the mixture was concentrated and the residue was purified by prep-HPLC to afford 5-[*(2S,6R)*-2-[[3-[6-(3-amino-3-methyl-azetidin-1-yl)-3-pyridyl]azetidin-1-yl]methyl]-6-methyl-morpholin-4-yl]-2-deuterio-quinoline-8-carbonitrile (**Example 138,** 22 mg) as a yellow solid. MS calc'd 485 ($MH^+$), measured 485 ($MH^+$). $^1H$ NMR (METHANOL-$d_4$, 400 MHz) δ 8.64 (d, 1H, *J*=8.7 Hz), 8.14 (d, 1H, *J*=8.1 Hz), 7.91 (d, 1H, *J*=2.2 Hz), 7.63 (br d, 2H, *J*=8.6 Hz), 7.24 (d, 1H, *J*=8.1 Hz), 6.43 (d, 1H, *J*=8.6 Hz), 4.0-4.1 (m, 2H), 3.8-3.9 (m, 2H), 3.7-3.8 (m, 4H), 3.6-3.7 (m, 1H), 3.3-3.4 (m, 2H), 3.2-3.3 (m, 2H), 2.6-2.8 (m, 4H), 1.49 (s, 3H), 1.24 (d, 3H, *J*=6.2 Hz).

**Example 139**

**5-[*(2S,6R)*-2-[[4-[5-(3-amino-3-methyl-azetidin-1-yl)-3-methyl-2-pyridyl]piperazin-1-yl]methyl]-6-methyl-morpholin-4-yl]-2-deuterio-quinoline-8-carbonitrile**

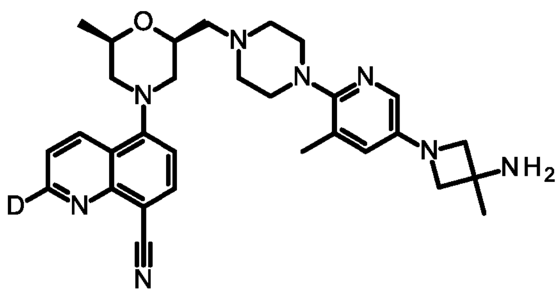

The title compound was prepared in analogy to the preparation of Example **132** by using 1-(5-bromo-3-methyl-2-pyridyl)piperazine instead of 1-(6-bromopyridin-3-yl)piperazine (compound **132a**). **Example 139** (8 mg) was obtained as a light yellow solid. MS: calc'd 528 ($MH^+$), measured 528 ($MH^+$). $^1H$ NMR (METHANOL-$d_4$, 400 MHz) δ 8.69 (d, 1H, *J*=8.6 Hz), 8.19 (d, 1H, *J*=8.1 Hz), 7.68 (d, 1H, *J*=8.6 Hz), 7.50 (d, 1H, *J*=2.8 Hz), 7.31 (d, 1H, *J*=8.1 Hz), 6.96 (d, 1H, *J*=2.4 Hz), 4.4-4.6 (m, 1H), 4.2-4.3 (m, 1H), 4.0-4.1 (m, 2H), 3.9-4.0 (m, 2H), 3.7-3.9 (m, 2H), 3.2-3.6 (m, 10H), 2.7-2.9 (m, 2H), 2.35 (s, 3H), 1.69 (s, 3H), 1.34 (d, 3H, *J*=6.2 Hz).

**Example 140**

**5-[*(2S,6R)*-2-[[3-[4-[(*3S,4S*)-3-amino-4-methoxy-pyrrolidin-1-yl]phenyl]azetidin-1-yl]methyl]-6-methyl-morpholin-4-yl]-2-deuterio-quinoline-8-carbonitrile**

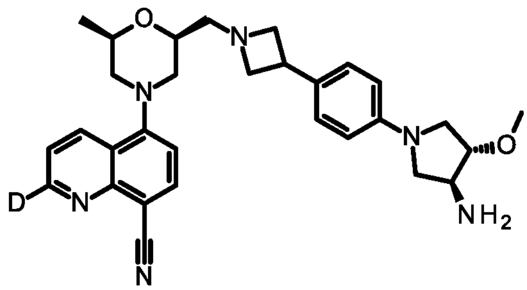

The title compound was prepared in analogy to the preparation of Example **132** by using 3-(4-bromophenyl)azetidine and *tert*-butyl *N*-[(*3S,4S*)-4-methoxypyrrolidin-3-yl]carbamate instead of 1-(6-bromopyridin-3-yl)piperazine (compound **132a)** and *tert*-butyl (3-methylazetidin-3-yl)carbamate (compound **132c). Example 140** (1 mg) was obtained as a light yellow solid. MS: calc'd 514 ($MH^+$), measured 514 ($MH^+$). $^1H$ NMR (METHANOL-$d_4$, 400 MHz) δ 8.68 (d, 1H, *J*=8.7 Hz), 8.19 (d, 1H, *J*=8.1 Hz), 7.6-7.8 (m, 1H), 7.2-7.4 (m, 3H), 6.73 (d, 2H, *J*=8.8 Hz), 4.4-4.7 (m, 3H), 4.1-4.4 (m, 6H), 3.6-3.9 (m, 3H), 3.4-3.5 (m, 7H), 2.6-2.9 (m, 3H), 1.2-1.4 (m, 3H).

**Example 141**

**5-[*(2S,6R)*-2-[[3-[4-(3-amino-3-methyl-azetidin-1-yl)phenyl]azetidin-1-yl]methyl]-6-methyl-morpholin-4-yl]-2-deuterio-quinoline-8-carbonitrile**

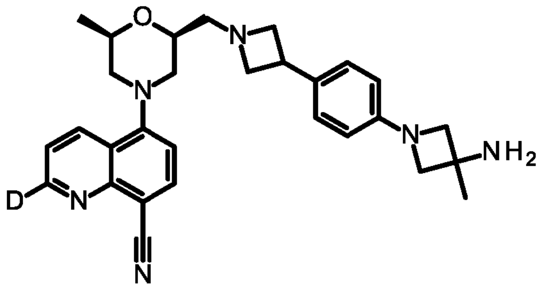

The title compound was prepared in analogy to the preparation of Example **132** by using 3-(4-bromophenyl)azetidine instead of 1-(6-bromopyridin-3-yl)piperazine (compound **132a**). **Example 141** (13 mg) was obtained as a light yellow solid. MS: calc'd 484 ($MH^+$), measured 484 ($MH^+$). $^1H$ NMR (METHANOL-$d_4$, 400 MHz) δ 8.56 (d, 1H, *J*=8.7 Hz), 8.06 (d, 1H, *J*=7.9 Hz), 7.55 (d, 1H, *J*=8.6 Hz), 7.18 (t, 3H, *J*=7.7 Hz), 6.48 (br d, 2H, *J*=8.3 Hz), 4.3-4.6 (m, 3H), 4.0-4.3 (m, 4H), 3.8-3.9 (m, 2H), 3.7-3.8 (m, 2H), 3.3-3.5 (m, 4H), 2.5-2.8 (m, 2H), 1.57 (s, 3H), 1.20 (d, 3H, *J*=6.1 Hz).

**Example 142**

**5-[*(2S,6R)*-2-[[4-[5-[(*3R,4R*)-3-amino-4-methoxy-pyrrolidin-1-yl]-3-methyl-2-pyridyl]piperazin-1-yl]methyl]-6-methyl-morpholin-4-yl]-2-deuterio-quinoline-8-carbonitrile**

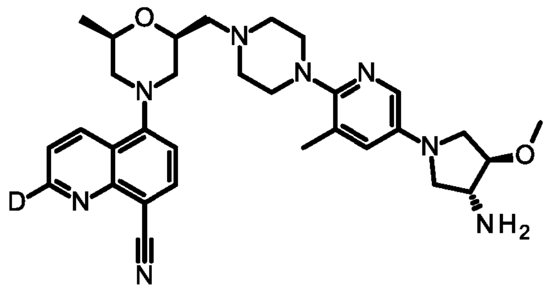

The title compound was prepared in analogy to the preparation of Example **132** by using 1-(5-bromo-3-methyl-2-pyridyl)piperazine and *tert*-butyl *N*-[(*3R,4R*)-4-methoxypyrrolidin-3-yl]carbamate instead of 1-(6-bromopyridin-3-yl)piperazine (compound **132a**) and *tert*-butyl (3-methylazetidin-3-yl)carbamate (compound **132c**). **Example 142** (8 mg) was obtained as a light yellow solid. MS: calc'd 558 ($MH^+$), measured 558 ($MH^+$). $^1H$ NMR (METHANOL-$d_4$, 400 MHz) δ 8.69 (d, 1H, *J*=8.7 Hz), 8.20 (d, 1H, *J*=7.9 Hz), 7.68 (d, 1H, *J*=8.7 Hz), 7.61 (d, 1H, *J*=2.8 Hz), 7.32 (d, 1H, *J*=8.1 Hz), 7.13 (d, 1H, *J*=2.7 Hz), 4.63 (br d, 1H, *J*=7.5 Hz), 4.1-4.3 (m, 2H), 3.8-4.0 (m, 3H), 3.6-3.7 (m, 2H), 3.2-3.5 (m, 15H), 2.6-3.0 (m, 2H), 2.38 (s, 3H), 1.35 (d, 3H, *J*=6.2 Hz).

**Example 143**

**5-[*(2S,6R)*-2-[[4-[6-[(*3R,4R*)-3-amino-4-methoxy-pyrrolidin-1-yl]-3-pyridyl]piperazin-1-yl]methyl]-6-methyl-morpholin-4-yl]-2-deuterio-quinoline-8-carbonitrile**

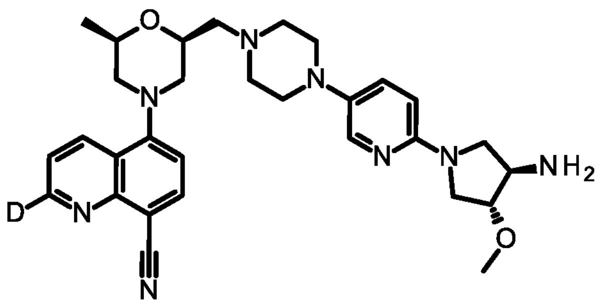

The title compound was prepared in analogy to the preparation of Example **132** by using *tert*-butyl *N*-[(*3R,4R*)-4-methoxypyrrolidin-3-yl]carbamate instead of *tert*-butyl (3-methylazetidin-3-yl)carbamate (compound **132c**). **Example 143** (52 mg) was obtained as a light yellow solid. MS: calc'd 544 ($MH^+$), measured 544 ($MH^+$). $^1H$ NMR (METHANOL-$d_4$, 400 MHz) δ 8.64 (d, 1H, *J*=8.7 Hz), 8.13 (d, 1H, *J*=8.1 Hz), 8.06 (dd, 1H, *J*=2.8, 9.8 Hz), 7.63 (d, 1H, *J*=8.7 Hz), 7.57 (d, 1H, *J*=2.7 Hz), 7.25 (d, 1H, *J*=8.1 Hz), 7.16 (d, 1H, *J*=9.8 Hz), 4.5-4.6 (m, 1H), 4.3-4.3 (m, 1H), 4.1-4.2 (m, 1H), 4.0-4.1 (m, 3H), 3.5-3.9 (m, 7H), 3.49 (s, 4H), 3.3-3.5 (m, 5H), 3.2-3.3 (m, 1H), 2.7-2.8 (m, 2H), 1.31 (d, 3H, *J*=6.4 Hz).

**Example 144**

**5-[*(2S,6R)*-2-[[4-[4-[(*3R,4R*)-3-amino-4-methoxy-pyrrolidin-1-yl]-6-methyl-pyrimidin-2-yl]piperazin-1-yl]methyl]-6-methyl-morpholin-4-yl]-2-deuterio-quinoline-8-carbonitrile**

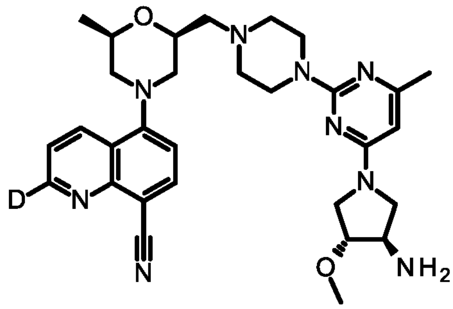

The title compound was prepared in analogy to the preparation of Example **104** by using [(*2R,6R*)-4-(8-cyano-2-deuterio-5-quinolyl)-6-methyl-morphofin-2-yl]methyl trifluoromethanesulfonate (intermediate **L**) and *tert*-butyl *N*-[(*3R,4R*)-4-methoxypyrrolidin-3-yl]carbamate instead of [(*2R,6R*)-4-(8-cyanoquinoxalin-5-yl)-6-methyl-morpholin-2-yl]methyl trifluoromethanesulfonate (intermediate **C)** and *tert*-butyl *N-[(3S,4S)*-4-methoxypyrrolidin-3-yl]carbamate (compound **104e**). **Example 144** (43 mg) was obtained as a light yellow solid. MS: calc'd 559 ($MH^+$), measured 559 ($MH^+$). $^1H$ NMR (METHANOL-$d_4$, 400 MHz) δ 8.55 (d, 1H, *J*=8.6 Hz), 8.03 (d, 1H, *J*=8.1 Hz), 7.53 (d, 1H, *J*=8.6 Hz), 7.13 (d, 1H, *J*=8.1 Hz), 5.59 (s, 1H), 4.1-4.1 (m, 1H), 3.9-4.0 (m, 1H), 3.6-3.7 (m, 6H), 3.4-3.6 (m, 2H), 3.3-3.4 (m, 3H), 3.3-3.3 (m, 3H), 3.3-3.3 (m, 1H), 2.5-2.6 (m, 4H), 2.4-2.5 (m, 4H), 2.09 (s, 3H), 1.16 (d, 3H, *J*=6.2 Hz).

**Example 145**

**5-[(*2S,6R*)-2-[[3-[6-(3-amino-3-methyl-azetidin-1-yl)-4-methyl-3-pyridyl]azetidin-1-yl]methyl]-6-methyl-morpholin-4-yl]-2-deuterio-quinoline-8-carbonitrile**

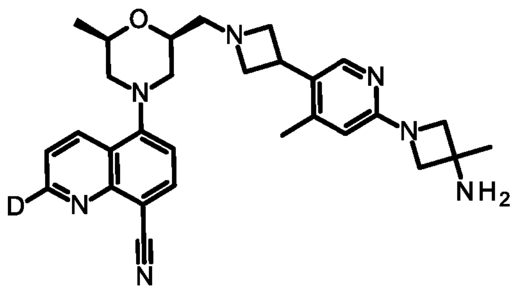

The title compound was prepared in analogy to the preparation of Example **138** by using (6-fluoro-4-methylpyridin-3-yl)boronic acid instead of (6-chloropyridin-3-yl)boronic acid (compound **138a**). **Example 145** (1 mg) was obtained as a light yellow solid. MS: calc'd 499 ($MH^+$), measured 499 ($MH^+$). $^1H$ NMR (METHANOL-$d_4$, 400 MHz) δ 8.65 (d, 1H, *J*=8.6 Hz), 8.15 (d, 1H, *J*=7.9 Hz), 7.81 (s, 1H), 7.64 (d, 1H, *J*=8.6 Hz), 7.24 (d, 1H, *J*=7.9 Hz), 6.27 (s, 1H), 4.0-4.1 (m, 2H), 3.8-4.0 (m, 7H), 3.3-3.4 (m, 4H), 2.6-2.8 (m, 4H), 2.16 (s, 3H), 1.51 (s, 3H), 1.2-1.3 (m, 3H).

**Example 146**

**5-[*(2S,6R)*-2-[[3-[6-[(*3R,4R*)-3-amino-4-methoxy-pyrrolidin-1-yl]-4-methyl-3-pyridyl]azetidin-1-yl]methyl]-6-methyl-morpholin-4-yl]-2-deuterio-quinoline-8-carbonitrile**

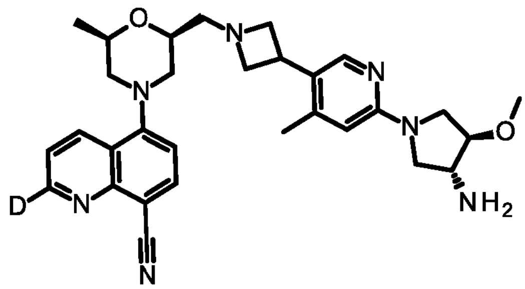

The title compound was prepared in analogy to the preparation of Example **138** by using (6-fluoro-4-methylpyridin-3-yl)boronic acid and *tert*-butyl ((*3R,4R*)-4-methoxypyrrolidin-3-yl)carbamate instead of (6-chloropyridin-3-yl)boronic acid ((compound **138a** ) and *tert*-butyl (3-methylazetidin-3-yl)carbamate (compound **132c**). **Example 146** (1 mg) was obtained as a light yellow solid. MS: calc'd 529 ($MH^+$), measured 529 ($MH^+$). $^1H$ NMR (METHANOL-$d_4$, 400 MHz) $\delta$ 8.65 (d, 1H, *J*=8.6 Hz), 8.15 (d, 1H, *J*=7.9 Hz), 7.81 (s, 1H), 7.64 (d, 1H, *J*=8.6 Hz), 7.25 (d, 1H, *J*=8.1 Hz), 6.3-6.4 (m, 1H), 4.0-4.1 (m, 2H), 3.9-4.0 (m, 2H), 3.7-3.8 (m, 3H), 3.6-3.7 (m, 1H), 3.5-3.6 (m, 1H), 3.3-3.4 (m, 7H), 3.2-3.3 (m, 2H), 2.6-2.8 (m, 4H), 2.17 (s, 3H), 1.25 (d, 3H, *J*=6.2 Hz).

## Example 147

**5-[*(2S,6R)*-2-[[4-[5-(3-amino-3-methyl-azetidin-1-yl)-6-methyl-2-pyridyl]piperazin-1-yl]methyl]-6-methyl-morpholin-4-yl]-2-deuterio-quinoline-8-carbonitrile**

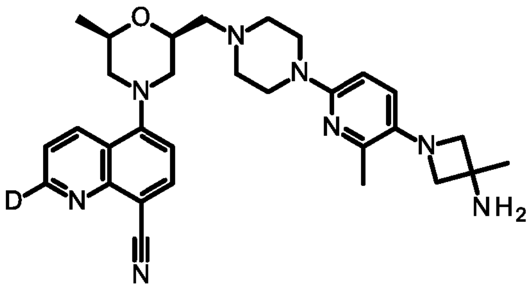

The title compound was prepared in analogy to the preparation of Example **132** by using 1-(5-bromo-6-methyl-2-pyridyl)piperazine instead of 1-(6-bromopyridin-3-yl)piperazine (compound **132a**). **Example 147** (4 mg) was obtained as a light yellow solid. MS: calc'd 528 ($MH^+$), measured 528 ($MH^+$). $^1H$ NMR (METHANOL-$d_4$, 400 MHz) $\delta$ 8.57 (d, 1H, *J*=8.7 Hz), 8.08 (d, 1H, *J*=7.9 Hz), 7.56 (d, 1H, *J*=8.7 Hz), 7.19 (d, 1H, *J*=7.9 Hz), 6.96 (d, 1H, *J*=8.8 Hz), 6.67 (d, 1H, *J*=8.8 Hz), 4.3-4.5 (m, 1H), 4.0-4.2 (m, 2H), 3.7-3.9 (m, 4H), 3.3-3.5 (m, 9H), 2.8-3.0 (m, 1H), 2.5-2.8 (m, 3H), 2.26 (s, 3H), 1.56 (s, 3H), 1.22 (d, 3H, *J*=6.4 Hz)

## Example 148

**4-[*(2S,6R)*-2-[[4-[5-(3-amino-3-methyl-azetidin-1-yl)-3-methyl-2-pyridyl]piperazin-1-yl]methyl]-6-methyl-morpholin-4-yl]-3-fluoro-pyrazolo[1,5-a]pyridine-7-carbonitrile**

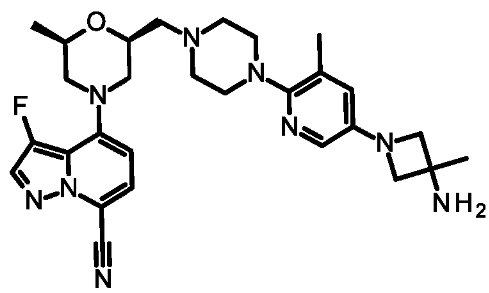

The title compound was prepared in analogy to the preparation of Example **132** by using [(2*R*,6*R*)-4-(7-cyano-3-fluoro-pyrazolo[1,5-a]pyridin-4-yl)-6-methyl-morphofin-2-yl]methyl trifluoromethanesulfonate (intermediate **E**) and 1-(5-bromo-3-methyl-2-pyridyl)piperazine instead of [(2*R*,6*R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a**). **Example 148** (17 mg) was obtained as a light yellow solid. MS: calc'd 534 ($MH^+$), measured 534 ($MH^+$). $^1H$ NMR (METHANOL-$d_4$, 400 MHz) δ 8.00 (d, 1H, *J*=3.5 Hz), 7.47 (d, 1H, *J*=2.8 Hz), 7.43 (d, 1H, J=7.9 Hz), 7.06 (d, 1H, *J*=2.4 Hz), 6.55 (d, 1H, *J*=7.9 Hz), 4.3-4.4 (m, 1H), 4.0-4.1 (m, 3H), 3.95 (d, 2H, *J*=8.4 Hz), 3.5-3.7 (m, 5H), 3.3-3.5 (m, 7H), 2.7-2.8 (m, 2H), 2.34 (s, 3H), 1.68 (s, 3H), 1.3-1.3 (m, 3H).

**Example 149**

**5-[*(2S,6R)*-2-[[3-[6-[(*3R,4R*)-3-amino-4-methoxy-pyrrolidin-1-yl]-3-pyridyl]azetidin-1-yl]methyl]-6-methyl-morpholin-4-yl]-2-deuterio-quinoline-8-carbonitrile**

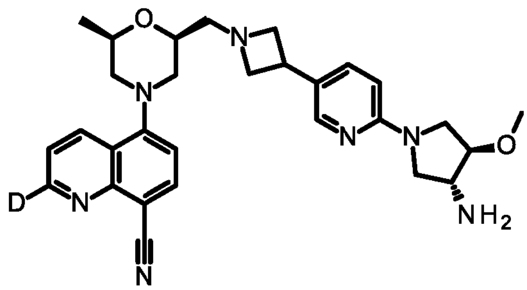

The title compound was prepared in analogy to the preparation of Example **138** by using *tert*-butyl ((*3R,4R*)-4-methoxypyrrolidin-3-yl)carbamate instead of *tert*-butyl (3-methylazetidin-3-yl)carbamate (compound **132c**). **Example 149** (4 mg) was obtained as a light yellow solid. MS: calc'd 515 ($MH^+$), measured 515 ($MH^+$). $^1H$ NMR (METHANOL-$d_4$, 400 MHz) δ 8.65 (d, 1H, *J*=8.6 Hz), 8.3-8.6 (m, 1H), 8.16 (d, 1H, *J*=7.9 Hz), 8.05 (d, 1H, *J*=2.1 Hz), 7.73 (br d, 1H, *J*=2.3 Hz), 7.64 (d, 1H, *J*=8.6 Hz), 7.27 (d, 1H, *J*=8.1 Hz), 6.63 (d, 1H, *J*=8.7 Hz), 4.3-4.4 (m, 2H), 4.2-4.3 (m, 1H), 4.0-4.2 (m, 5H), 3.8-3.9 (m, 3H), 3.5-3.6 (m, 1H), 3.5-3.5 (m, 1H), 3.46 (s, 3H), 3.41 (br d, 2H, *J*=12.2 Hz), 3.2-3.3 (m, 1H), 2.6-2.8 (m, 2H), 1.28 (d, 3H, *J*=6.2 Hz).

**Example 150**

**5-[*(2S,6R)*-2-[[4-[6-[(*3S,4S*)-3-amino-4-fluoro-pyrrolidin-1-yl]-3-pyridyl]piperazin-1-yl]methyl]-6-methyl-morpholin-4-yl]-2-deuterio-quinoline-8-carbonitrile**

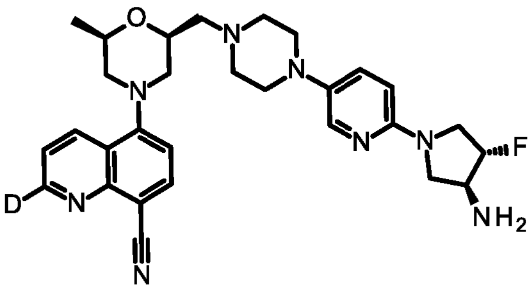

The title compound was prepared in analogy to the preparation of Example **132** by using *tert*-butyl *N*-[(*3S,4S*)-4-fluoropyrrolidin-3-yl]carbamate instead of *tert*-butyl (3-methylazetidin-3-yl)carbamate (compound **132c**). **Example 150** (4 mg) was obtained as a light yellow solid. MS: calc'd 532 ($MH^+$), measured 532 ($MH^+$). $^1H$ NMR (METHANOL-$d_4$, 400 MHz) δ 8.66 (d, 1H, *J*=8.6 Hz), 8.3-8.4 (m, 1H), 8.15 (d, 1H, *J*=7.9 Hz), 7.82 (d, 1H, *J*=2.8 Hz), 7.64 (d, 1H, *J*=8.6 Hz), 7.45 (dd, 1H, *J*=2.9, 9.1 Hz), 7.26 (d, 1H, *J*=8.1 Hz), 6.60 (d, 1H, *J*=9.0 Hz), 5.1-5.3 (m, 1H), 4.2-4.4 (m, 1H), 4.1-4.2 (m, 1H), 3.9-4.0 (m, 1H), 3.8-3.9 (m, 1H), 3.6-3.7 (m, 1H), 3.5-3.6 (m, 1H), 3.4-3.5 (m, 2H), 3.1-3.2 (m, 4H), 2.9-3.1 (m, 4H), 2.6-2.9 (m, 4H), 1.27 (d, 3H, *J*=6.2 Hz).

**Example 151**

**2-deuterio-5-[*(2S,6R)*-2-[[4-[6-[(*1S,4S*)-2,5-diazabicyclo[2.2.1]heptan-2-yl]-3-pyridyl]piperazin-1-yl]methyl]-6-methyl-morpholin-4-yl]quinoline-8-carbonitrile**

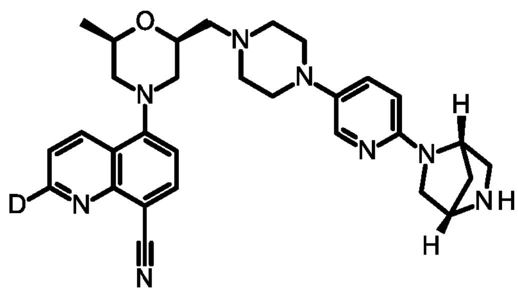

The title compound was prepared in analogy to the preparation of Example **132** by using *tert*-butyl (1*S*,*4S*)-2,5-diazabicyclo[2.2.1]heptane-2-carboxylate (Bidepharm, BD30269, CAS: 113451-59-5) instead of *tert*-butyl (3-methylazetidin-3-yl)carbamate (compound **132c** ). **Example 151** (5 mg) was obtained as a light yellow solid. MS: calc'd 526 ($MH^+$), measured 526 ($MH^+$). $^1H$ NMR (METHANOL-$d_4$, 400 MHz) δ 8.67 (d, 1H, *J*=8.7 Hz), 8.17 (d, 1H, *J*=8.1 Hz), 7.77 (d, 1H, *J*=2.7 Hz), 7.72 (dd, 1H, *J*=2.9, 9.4 Hz), 7.66 (d, 1H, *J*=8.6 Hz), 7.29 (d, 1H, *J*=8.1 Hz), 6.89 (d, 1H, *J*=9.4 Hz), 4.97 (s, 1H), 4.58 (s, 1H), 4.5-4.5 (m, 1H), 4.1-4.2 (m, 1H), 3.7-3.8 (m, 3H), 3.5-3.7 (m, 4H), 3.4-3.5 (m, 8H), 3.3-3.4 (m, 1H), 2.7-2.8 (m, 2H), 2.31 (br d, 1H, *J*=10.9 Hz), 2.11 (br d, 1H, *J*=11.5 Hz), 1.3-1.3 (m, 3H).

**Example 152**

**2-deuterio-5-[(*2R,6S*)-2-methyl-6-[[4-[6-(5-oxa-2,8-diazaspiro[3.5]nonan-2-yl)-3-pyridyl]piperazin-1-yl]methyl]morpholin-4-yl]quinoline-8-carbonitrile**

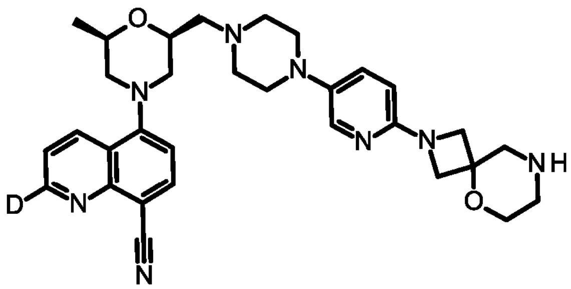

The title compound was prepared in analogy to the preparation of Example **132** by using *tert*-butyl 5-oxa-2,8-diazaspiro[3.5]nonane-8-carboxylate instead of *tert*-butyl (3-methylazetidin-3-yl)carbamate (compound **132c). Example 152** (10 mg) was obtained as a light yellow solid. MS: calc'd 556 ($MH^+$), measured 556 ($MH^+$). $^1H$ NMR (METHANOL-$d_4$, 400 MHz) $\delta$ 8.65 (d, 1H, *J*=8.6 Hz), 8.14 (d, 1H, *J*=7.9 Hz), 7.99 (dd, 1H, *J*=2.7, 9.7 Hz), 7.64 (d, 1H, *J*=8.6 Hz), 7.56 (d, 1H, *J*=2.6 Hz), 7.27 (d, 1H, *J*=8.1 Hz), 6.92 (d, 1H, *J*=9.7 Hz), 4.5-4.7 (m, 2H), 4.39 (d, 2H, *J*=9.8 Hz), 4.27 (d, 2H, *J*=9.7 Hz), 4.18 (ddd, 1H, *J*=2.1, 6.3, 10.0 Hz), 4.0-4.1 (m, 2H), 3.4-3.7 (m, 13H), 3.3-3.3 (m, 2H), 2.75 (ddd, 2H, *J*= 10.4, 11.9, 19.4 Hz), 1.31 (d, 3H, *J*=6.2 Hz).

**Example 153**

**2-deuterio-5-[*(2S,6R)*-2-[[4-[6-[(1R,4R)-2,5-diazabicyclo[2.2.1]heptan-2-yl]-3-pyridyl]piperazin-1-yl]methyl]-6-methyl-morpholin-4-yl]quinoline-8-carbonitrile**

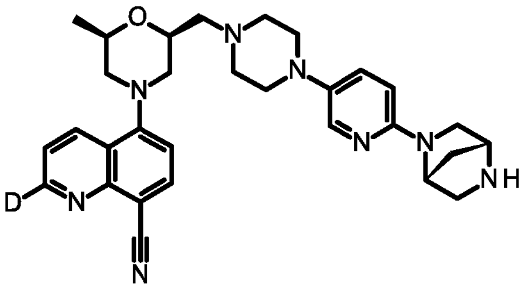

The title compound was prepared in analogy to the preparation of Example **132** by using *tert*-butyl (1*R,4R*)-2,5-diazabicyclo[2.2.1]heptane-2-carboxylate (Pharmablock, PBN20120578, CAS: 134003-84-2) instead of *tert*-butyl (3-methylazetidin-3-yl)carbamate (compound **132c). Example 153** (3 mg) was obtained as a light yellow solid. MS: calc'd 526 ($MH^+$), measured 526 ($MH^+$). $^1H$ NMR (METHANOL-$d_4$, 400 MHz) $\delta$ 8.7-8.7 (m, 1H), 8.2-8.2 (m, 1H), 7.8-7.8 (m, 1H), 7.6-7.7 (m, 2H), 7.29 (d, 1H, *J*=8.1 Hz), 6.87 (d, 1H, *J*=9.4 Hz), 4.9-5.0 (m, 1H), 4.5-4.6 (m, 2H), 4.1-4.3 (m, 1H), 3.8-3.8 (m, 1H), 3.7-3.9 (m, 2H), 3.5-3.7 (m, 4H), 3.4-3.5 (m, 8H), 2.9-3.1 (m, 1H), 2.7-2.8 (m, 3H), 2.3-2.3 (m, 1H), 2.1-2.1 (m, 1H), 1.3-1.3 (m, 3H).

**Example 154**

**4-[*(2S,6R)*-2-[[4-[6-(3-amino-3-methyl-azetidin-1-yl)-3-pyridyl]piperazin-1-yl]methyl]-6-methyl-morpholin-4-yl]-3-fluoro-pyrazolo[1,5-a]pyridine-7-carbonitrile**

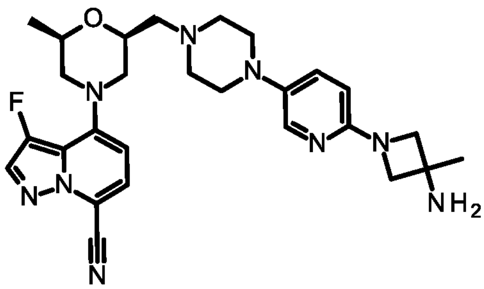

The title compound was prepared in analogy to the preparation of Example **132** by using [(*2R,6R*)-4-(7-cyano-3-fluoro-pyrazolo[1,5-a]pyridin-4-yl)-6-methyl-morphofin-2-yl]methyl trifluoromethanesulfonate (intermediate **E**) instead of [(*2R,6R*)-4-(8-cyano-2-deuterio-5-quinolyl)-6-methyl-morpholin-2-yl]methyl trifluoromethanesulfonate (intermediate **L**). **Example 154** (19 mg) was obtained as a light yellow solid. MS: calc'd 520 ($MH^+$), measured 520 ($MH^+$). $^1H$ NMR (METHANOL-$d_4$, 400 MHz) δ 8.01 (d, 1H, *J*=3.7 Hz), 7.97 (dd, 1H, *J*=2.7, 9.5 Hz), 7.63 (d, 1H, *J*=2.6 Hz), 7.45 (d, 1H, *J*=7.9 Hz), 6.92 (d, 1H, *J*=9.5 Hz), 6.56 (d, 1H, *J*=7.9 Hz), 4.3-4.4 (m, 5H), 4.0-4.1 (m, 1H), 3.5-3.9 (m, 7H), 3.4-3.5 (m, 5H), 2.7-2.8 (m, 2H), 1.74 (s, 3H), 1.32 (d, 3H, *J*=6.2 Hz).

**Example 155**

**5-[*(2S,6R)*-2-[[4-[6-(3-amino-3-methyl-azetidin-1-yl)-2-pyridyl]piperazin-1-yl]methyl]-6-methyl-morpholin-4-yl]-2-deuterio-quinoline-8-carbonitrile**

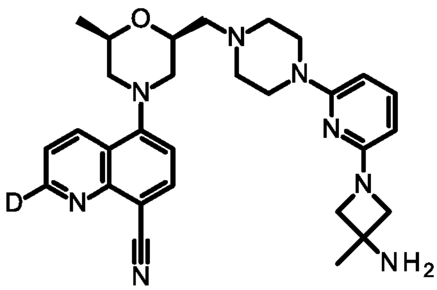

The title compound was prepared in analogy to the preparation of Example **132** by using 1-(6-bromo-2-pyridyl)piperazine instead of 1-(6-bromopyridin-3-yl)piperazine (compound **132a**). **Example 155** (5 mg) was obtained as a light yellow solid. MS: calc'd 514 ($MH^+$), measured 514 ($MH^+$). $^1H$ NMR (METHANOL-$d_4$, 400 MHz) δ 8.68 (d, 1H, *J*=8.7 Hz), 8.17 (d, 1H, *J*=8.1 Hz), 7.66 (d, 1H, *J*=8.6 Hz), 7.36 (t, 1H, *J*=8.0 Hz), 7.27 (d, 1H, *J*=8.1 Hz), 6.11 (d, 1H, *J*=8.1 Hz), 5.79 (d, 1H, *J*=7.8 Hz), 4.2-4.3 (m, 1H), 4.0-4.1 (m, 1H), 3.8-3.9 (m, 6H), 3.4-3.6 (m, 6H), 2.5-2.8 (m, 6H), 1.56 (s, 3H), 1.2-1.4 (m, 3H).

**Example 156**

**5-[*(2S,6R)*-2-[[3-[4-(3-amino-3-methyl-azetidin-1-yl)phenyl]-3-hydroxy-azetidin-1-yl]methyl]-6-methyl-morpholin-4-yl]-2-deuterio-quinoline-8-carbonitrile**

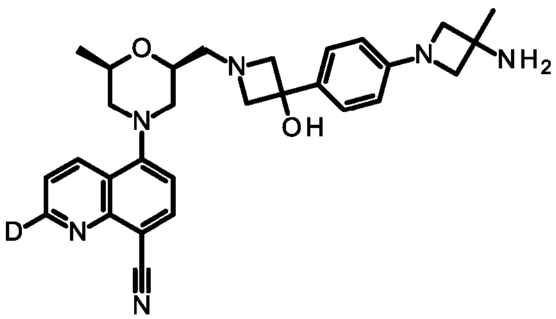

The title compound was prepared in analogy to the preparation of Example **132** by using 3-(4-bromophenyl)azetidin-3-ol instead of 1-(6-bromopyridin-3-yl)piperazine (compound **132a**). **Example 156** (7 mg) was obtained as a light yellow solid. MS: calc'd 500 ($MH^+$), measured 500 ($MH^+$). $^1H$ NMR (METHANOL-$d_4$, 400 MHz) δ 8.66 (d, 1H, *J*=8.7 Hz), 8.16 (d, 1H, *J*=8.1 Hz), 7.64 (d, 1H, *J*=8.7 Hz), 7.4-7.5 (m, 2H), 7.26 (d, 1H, *J*=8.1 Hz), 6.4-6.6 (m, 2H), 4.0-4.1 (m, 2H), 3.7-3.9 (m, 4H), 3.6-3.7 (m, 2H), 3.4-3.6 (m, 4H), 2.6-2.9 (m, 3H), 1.55 (s, 3H), 1.26 (d, 4H, *J*=6.2 Hz).

**Example 157**

**4-[*(2S,6R)*-2-[[3-[6-(3-amino-3-methyl-azetidin-1-yl)-3-pyridyl]azetidin-1-yl]methyl]-6-methyl-morpholin-4-yl]pyrazolo[1,5-a]pyridine-7-carbonitrile**

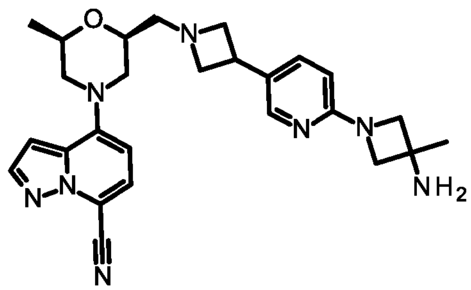

The title compound was prepared in analogy to the preparation of Example 138 by using (*2R,6R*)-4-(7-cyanopyrazolo[1,5-a]pyridin-4-yl)-6-methyl-morpholin-2-yl]methyl trifluoromethanesulfonate(intermediate **D**) instead of [(*2R,6R*)-4-(8-cyano-2-deuterio-5-quinolyl)-6-methyl-morpholin-2-yl]methyl trifluoromethanesulfonate (intermediate **L**). **Example 157** (13 mg) was obtained as a light yellow solid. MS: calc'd 473 ($MH^+$), measured 473 ($MH^+$). $^1H$ NMR (METHANOL-$d_4$, 400 MHz) δ 8.02 (d, 1H, *J*=2.3 Hz), 7.91 (d, 1H, *J*=2.2 Hz), 7.63 (dd, 1H, *J*=2.4, 8.6 Hz), 7.46 (d, 1H, *J*=8.1 Hz), 6.86 (d, 1H, *J*=2.4 Hz), 6.58 (d, 1H, *J*=8.1 Hz), 6.44 (d, 1H, *J*=8.7 Hz), 3.7-3.9 (m, 10H), 3.6-3.7 (m, 1H), 3.25 (t, 2H, *J*=7.5 Hz), 2.6-2.7 (m, 4H), 1.49 (s, 3H), 1.24 (d, 3H, *J*=6.2 Hz).

**Example 158**

**4-[*(2S,6R)*-2-[[3-[6-(3-amino-3-methyl-azetidin-1-yl)-3-pyridyl]azetidin-1-yl]methyl]-6-methyl-morpholin-4-yl]-3-fluoro-pyrazolo[1,5-a]pyridine-7-carbonitrile**

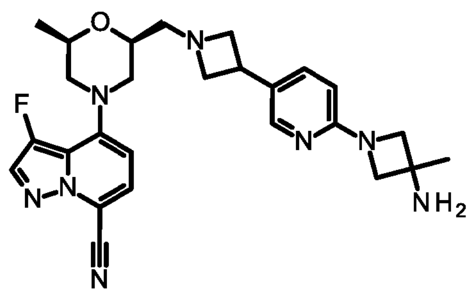

The title compound was prepared in analogy to the preparation of **Example 138** by using [(*2R,6R*)-4-(7-cyano-3-fluoro-pyrazolo[1,5-a]pyridin-4-yl)-6-methyl-morpholin-2-yllmethyl trifluoromethanesulfonate (intermediate **E**) instead of [(*2R,6R*)-4-(8-cyano-2-deuterio-5-quinolyl)-6-methyl-morpholin-2-yl]methyl trifluoromethanesulfonate (intermediate **L**). **Example 158** (23 mg) was obtained as a light yellow solid. MS: calc'd 491 ($MH^+$), measured 491 ($MH^+$). $^1H$ NMR (METHANOL-$d_4$, 400 MHz) $\delta$ 7.99 (d, 1H, *J*=3.7 Hz), 7.91 (d, 1H, *J*=2.1 Hz), 7.63 (dd, 1H, *J*=2.4, 8.6 Hz), 7.41 (d, 1H, *J*=7.9 Hz), 6.50 (d, 1H, *J*=8.1 Hz), 6.44 (d, 1H, *J*=8.6 Hz), 3.7-3.9 (m, 8H), 3.5-3.7 (m, 3H), 3.23 (dt, 2H, *J*=3.6, 7.5 Hz), 2.69 (s, 2H), 2.6-2.7 (m, 2H), 1.49 (s, 3H), 1.23 (d, 3H, *J*=6.2 Hz).

**Example 159**

**5-[*(2S,6R)*-2-[[3-[6-(3-amino-3-methyl-azetidin-1-yl)-2-pyridyl]azetidin-1-yl]methyl]-6-methyl-morpholin-4-yl]-2-deuterio-quinoline-8-carbonitrile**

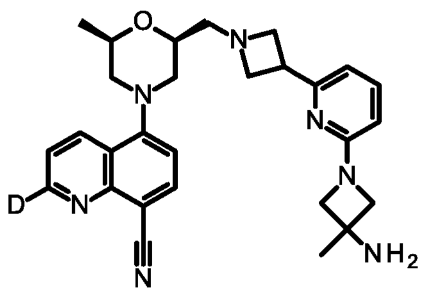

The title compound was prepared in analogy to the preparation of Example **132** by using 2-(azetidin-3-yl)-6-bromo-pyridine instead of 1-(6-bromopyridin-3-yl)piperazine (compound **132a**). **Example 159** (2 mg) was obtained as a light yellow solid. MS: calc'd 485 ($MH^+$), measured 485 ($MH^+$). $^1H$ NMR (METHANOL-$d_4$, 400 MHz) $\delta$ 8.64 (d, 1H, *J*=8.6 Hz), 8.15 (d, 1H, *J*=7.9 Hz), 7.64 (d, 1H, *J*=8.6 Hz), 7.44 (dd, 1H, *J*=7.4, 8.1 Hz), 7.24 (d, 1H, *J*=8.1 Hz), 6.58 (d, 1H, *J*=7.3 Hz), 6.26 (d, 1H, *J*=8.3 Hz), 4.5-4.7 (m, 1H), 4.0-4.2 (m, 2H), 3.7-4.0 (m, 6H), 3.4-3.6 (m, 6H), 2.57 (br dd, 4H, *J*=2.6, 3.5 Hz), 1.49 (s, 3H).

**Example 160**

**4-[*(2S,6R)*-2-[[3-[4-(3-amino-3-methyl-azetidin-1-yl)phenyl]azetidin-1-yl]methyl]-6-methyl-morpholin-4-yl]-3-fluoro-pyrazolo[1,5-a]pyridine-7-carbonitrile**

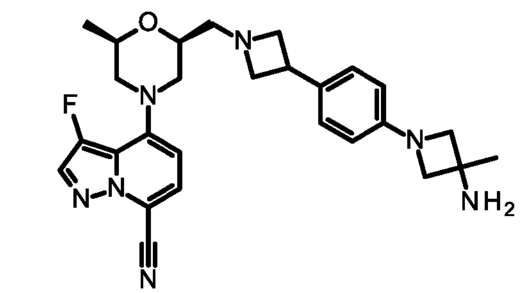

The title compound was prepared in analogy to the preparation of Example **132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and 3-(4-bromophenyl)azetidine instead of [(*2R,6R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a**). **Example 160** (4 mg) was obtained as a light yellow solid. MS: calc'd 490 ($MH^+$), measured 490 ($MH^+$). $^1H$ NMR (METHANOL-$d_4$, 400 MHz) δ 7.9-8.1 (m, 1H), 7.44 (d, 1H, *J*=7.9 Hz), 7.28 (br d, 2H, *J*=8.2 Hz), 6.4-6.7 (m, 3H), 4.3-4.7 (m, 2H), 4.0-4.3 (m, 4H), 3.8-4.0 (m, 4H), 3.4-3.7 (m, 5H), 2.6-2.8 (m, 2H), 1.66 (s, 3H), 1.29 (d, 3H, *J*=6.4 Hz).

**Example 161**

**2-deuterio-5-[(*2R,6S*)-2-methyl-6-[[4-[6-(6-methyl-2,6-diazaspiro[3.3]heptan-2-yl)-3-pyridyl]piperazin-1-yl]methyl]morpholin-4-yl]quinoline-8-carbonitrile**

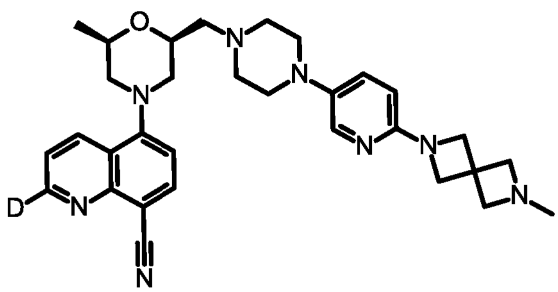

The title compound was prepared in analogy to the preparation of Example **132** by using 2-methyl-2,6-diazaspiro[3.3]heptane instead of *tert*-butyl (3-methylazetidin-3-yl)carbamate (compound **132c** ). **Example 161** (2 mg) was obtained as a light yellow solid. MS: calc'd 540 ($MH^+$), measured 540 ($MH^+$). $^1H$ NMR (METHANOL-$d_4$, 400 MHz) δ 8.66 (d, 1H, *J*=8.7 Hz), 8.15 (d, 1H, *J*=8.1 Hz), 7.72 (d, 1H, *J*=2.4 Hz), 7.63 (d, 1H, *J*=8.6 Hz), 7.38 (dd, 1H, *J*=2.9, 9.0 Hz), 7.25 (d, 1H, *J*=8.1 Hz), 6.42 (d, 1H, *J*=8.7 Hz), 4.1-4.2 (m, 1H), 4.0-4.1 (m, 1H), 4.0-4.0 (m, 3H), 3.5-3.5 (m, 1H), 3.4-3.4 (m, 4H), 3.4-3.4 (m, 1H), 3.4-3.4 (m, 1H), 3.06 (t, 3H, *J*=5.0 Hz), 2.8-2.9 (m, 2H), 2.7-2.7 (m, 3H), 2.6-2.7 (m, 2H), 2.5-2.6 (m, 1H), 2.3-2.3 (m, 4H), 1.25 (d, 3H, *J*=6.2 Hz).

**Example 162**

**2-deuterio-5-[*(2S,6R)*-2-[[4-[6-[[(*3S,4R*)-4-fluoropyrrolidin-3-yl]amino]-3-pyridyl]piperazin-1-yl]methyl]-6-methyl-morpholin-4-yl]quinoline-8-carbonitrile**

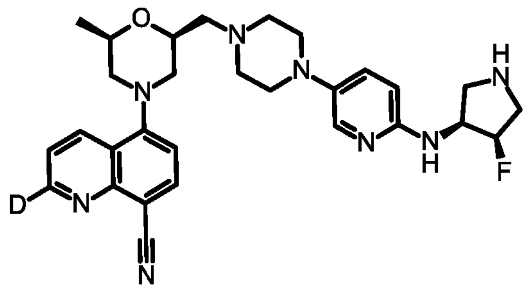

The title compound was prepared in analogy to the preparation of Example **132** by using *tert-butyl* (*3S,4R*)-3-amino-4-fluoro-pyrrolidine-1-carboxylate instead of *tert*-butyl (3-methylazetidin-3-yl)carbamate (compound **132c). Example 162** (6 mg) was obtained as a light yellow solid. MS: calc'd 532 ($MH^+$), measured 532 ($MH^+$). $^1H$ NMR (METHANOL-$d_4$, 400 MHz) δ 8.66 (d, 1H, *J*=8.6 Hz), 8.15 (d, 1H, *J*=8.1 Hz), 7.68 (d, 1H, *J*=2.4 Hz), 7.64 (d, 1H, *J*=8.7 Hz), 7.31 (dd, 1H, *J*=2.9, 9.1 Hz), 7.25 (d, 1H, *J*=8.1 Hz), 6.62 (d, 1H, *J*=9.0 Hz), 5.0-5.2 (m, 1H), 4.2-4.4 (m, 1H), 4.2-4.2 (m, 1H), 4.0-4.1 (m, 1H), 3.3-3.5 (m, 3H), 3.2-3.3 (m, 1H), 3.1-3.2 (m, 1H), 3.0-3.1 (m, 4H), 2.8-2.9 (m, 2H), 2.5-2.8 (m, 7H), 1.25 (d, 3H, *J*=6.2 Hz).

**Example 163**

**4-[*(2S,6R)*-2-[[3-[4-(3-amino-3-methyl-azetidin-1-yl)phenyl]azetidin-1-yl]methyl]-6-methyl-morpholin-4-yl]pyrazolo[1,5-a]pyridine-7-carbonitrile**

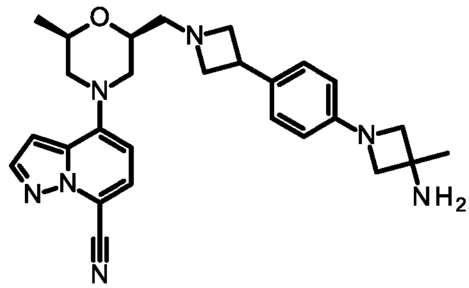

The title compound was prepared in analogy to the preparation of Example **132** by using [(*2R,6R*)-4-(7-cyanopyrazolo[1,5-a]pyridin-4-yl)-6-methyl-morpholin-2-yl]methyl trifluoromethanesulfonate(intermediate **D**) and 3-(4-bromophenyl)azetidine instead of [(*2R,6R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a**). **Example 163** (12 mg) was obtained as a light yellow solid. MS: calc'd 472 ($MH^+$), measured 472 ($MH^+$). $^1H$ NMR (METHANOL-$d_4$, 400 MHz) δ 8.04 (d, 1H, *J*=2.3 Hz), 7.49 (d, 1H, *J*=7.9 Hz), 7.29 (br d, 2H, *J*=8.3 Hz), 6.90 (d, 1H, *J*=2.4 Hz), 6.5-6.7 (m, 3H), 4.64 (s, 3H), 4.0-4.3 (m, 4H), 3.97 (d, 3H, *J*=8.3 Hz), 3.8-3.9 (m, 2H), 3.7-3.8 (m, 2H), 3.4-3.6 (m, 1H), 2.6-2.8 (m, 2H), 1.66 (s, 3H), 1.31 (br d, 3H, *J*=6.2 Hz)

**Example 164**

**4-[*(2S,6R)*-2-[[3-[6-[(*3R,4R*)-3-amino-4-methoxy-pyrrolidin-1-yl]-3-pyridyl]azetidin-1-yl]methyl]-6-methyl-morpholin-4-yl]-3-fluoro-pyrazolo[1,5-a]pyridine-7-carbonitrile**

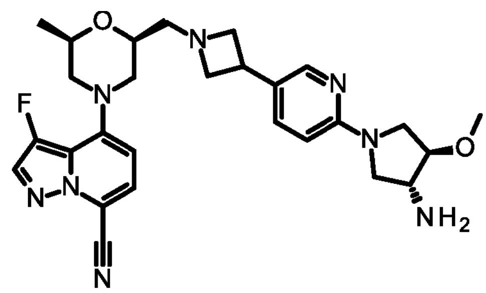

The title compound was prepared in analogy to the preparation of **Example 138** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and *tert*-butyl ((*3R,4R*)-4-methoxypyrrolidin-3-yl)carbamate instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c**). **Example 164** (5 mg) was obtained as a light yellow solid. MS: calc'd 521 ($MH^+$), measured 521($MH^+$). $^1H$ NMR (METHANOL-$d_4$, 400 MHz) δ 8.01 (d, 1H, *J*=3.7 Hz), 7.95 (d, 1H, *J*=2.2 Hz), 7.64 (dd, 1H, *J*=2.4, 8.7 Hz), 7.44 (d, 1H, *J*=7.9 Hz), 6.5-6.6 (m, 2H), 3.8-3.9 (m, 6H), 3.6-3.7 (m, 2H), 3.5-3.6 (m, 4H), 3.43 (s, 3H), 3.2-3.3 (m, 3H), 2.7-2.8 (m, 2H), 2.6-2.7 (m, 2H), 1.25 (d, 3H, *J*=6.2 Hz).

**Example 165**

**4-[*(2S,6R)*-2-[[3-[6-[(1S,4S)-2,5-diazabicyclo[2.2.1]heptan-2-yl]-3-pyridyl]azetidin-1-yl]methyl]-6-methyl-morpholin-4-yl]-3-fluoro-pyrazolo[1,5-a]pyridine-7-carbonitrile**

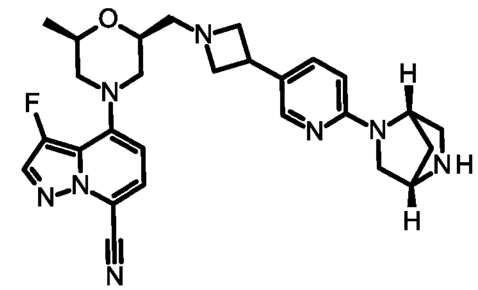

The title compound was prepared in analogy to the preparation of Example **138** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and(1*S,4S*)-2-Boc-2,5-diazabicyclo[2.2.1]heptane (Bidepharm, BD30269, CAS: 113451-59-5) instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c**). **Example 165** (10 mg) was obtained as a light yellow solid. MS: calc'd 503 ($MH^+$), measured 503 ($MH^+$). $^1H$ NMR (METHANOL-$d_4$, 400 MHz) δ 8.11 (d, 1H, *J*=2.0 Hz), 8.07 (dd, 1H, *J*=2.1, 9.1 Hz), 8.02 (d, 1H, *J*=3.5 Hz), 7.45 (d, 1H, *J*=7.9 Hz), 7.06 (br d, 1H, *J*=8.9 Hz), 6.56 (d, 1H, *J*=7.9 Hz), 5.11 (s, 1H), 4.66 (s, 1H), 4.57 (br t, 2H, *J*=9.2 Hz), 4.3-4.4 (m, 1H), 4.2-4.3 (m, 1H), 4.1-4.2 (m, 1H), 3.9-4.0 (m, 1H), 3.8-3.9 (m, 1H), 3.7-3.8 (m, 1H), 3.5-3.7 (m, 6H), 3.23 (d, 1H, *J*=7.3 Hz), 2.79 (br t, 1H, *J*=11.2 Hz), 2.7-2.7 (m, 1H), 2.36 (br d, 1H, *J*= 11.5 Hz), 2.19 (br d, 1H, *J*=11.5 Hz), 1.30 (d, 3H, *J*=6.2 Hz).

**Example 166**

**4-[*(2S,6R)*-2-[[4-[6-[(*3R,4R*)-3-amino-4-methoxy-pyrrolidin-1-yl]-3-pyridyl]piperazin-1-yl]methyl]-6-methyl-morpholin-4-yl]-3-fluoro-pyrazolo[1,5-a]pyridine-7-carbonitrile**

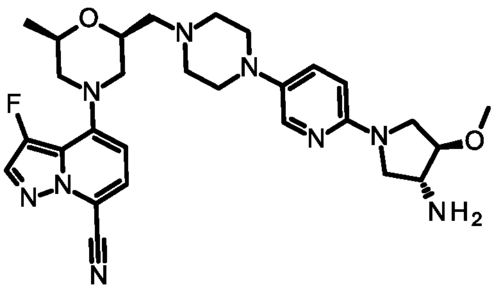

The title compound was prepared in analogy to the preparation of Example **132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and *tert*-butyl *N*-[*(3R,4R*)-4-methoxypyrrolidin-3-yl]carbamate instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c**). **Example 166** (10 mg) was obtained as a light yellow solid. MS: calc'd 550 ($MH^+$), measured 550 ($MH^+$). $^1H$ NMR (METHANOL-$d_4$, 400 MHz) δ 7.89 (d, 1H, *J*=3.7 Hz), 7.65 (d, 1H, *J*=2.7 Hz), 7.3-7.3 (m, 2H), 6.4-6.4 (m, 2H), 3.9-4.0 (m, 1H), 3.80 (ddd, 1H, *J*=2.3, 6.4, 10.1 Hz), 3.6-3.7 (m, 2H), 3.5-3.6 (m, 2H), 3.4-3.5 (m, 2H), 3.3-3.3 (m, 4H), 3.15 (dd, 1H, *J*=3.2, 10.1 Hz), 2.95 (t, 4H, *J*=4.9 Hz), 2.6-2.7 (m, 2H), 2.5-2.6 (m, 5H), 2.4-2.5 (m, 1H), 1.15 (d, 3H, *J*=6.4 Hz).

**Example 167**

**3-fluoro-4-[*(2S,6R)*-2-[[4-[6-[[(*3S,4R*)-4-fluoropyrrolidin-3-yl]amino]-3-pyridyl]piperazin-1-yl]methyl]-6-methyl-morpholin-4-yl]pyrazolo[1,5-a]pyridine-7-carbonitrile**

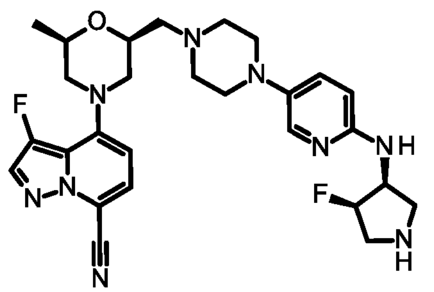

The title compound was prepared in analogy to the preparation of Example **132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and *tert*-butyl (*3S,4R*)-3-amino-4-fluoro-pyrrolidine-1-carboxylate instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c** ). **Example 167** (32 mg) was obtained as a light yellow solid. MS: calc'd 538 ($MH^+$), measured 538 ($MH^+$). $^1H$ NMR (METHANOL-$d_4$, 400 MHz) δ 8.10 (dd, 1H, *J*=2.8, 9.8 Hz), 8.04 (d, 1H, *J*=3.7 Hz), 7.56 (d, 1H, *J*=2.7 Hz), 7.47 (d, 1H, *J*=7.9 Hz), 7.29 (d, 1H, *J*=9.8 Hz), 6.60 (d, 1H, *J*=8.1 Hz), 5.4-5.6 (m, 1H), 4.4-4.4 (m, 1H), 4.0-4.1 (m, 1H), 3.7-4.0 (m, 7H), 3.6-3.7 (m, 3H), 3.4-3.5 (m, 5H), 3.37 (s, 1H), 3.2-3.3 (m, 1H), 2.7-2.9 (m, 2H), 1.34 (d, 3H, *J*=6.2 Hz).

**Example 168**

**4-[*(2S,6R)*-2-[[4-[6-[(4*aR,7aR*)-3,4,4*a*,5,7,7*a*-hexahydro-2*H*-pyrrolo[3,4-b][1,4]oxazin-6-yl]-3-pyridyl]piperazin-1-yl]methyl]-6-methyl-morpholin-4-yl]-3-fluoro-pyrazolo[1,5-a]pyridine-7-carbonitrile**

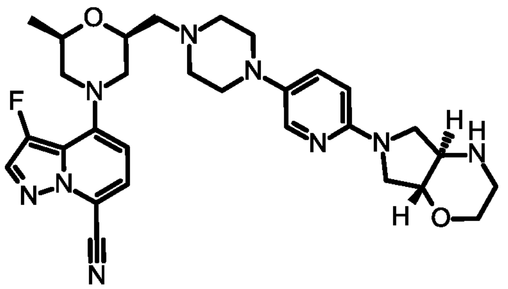

The title compound was prepared in analogy to the preparation of Example **132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and *tert*-butyl (4*aR*,7*aR*)-3,4*a*,5,6,7,7a-hexahydro-2*H*-pyrrolo[3,4-*b*][1,4]oxazine-4-carboxylate instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c** ). **Example 168** (12 mg) was obtained as a light yellow solid. MS: calc'd 562 ($MH^+$), measured 562 ($MH^+$). $^1H$ NMR (METHANOL-$d_4$, 400 MHz) $\delta$ 7.89 (d, 1H, *J*=3.7 Hz), 7.65 (d, 1H, *J*=2.8 Hz), 7.3-7.3 (m, 2H), 6.41 (d, 2H, *J*=8.3 Hz), 3.9-4.0 (m, 1H), 3.9-3.9 (m, 1H), 3.8-3.8 (m, 1H), 3.5-3.7 (m, 5H), 3.4-3.5 (m, 1H), 2.9-3.1 (m, 10H), 2.7-2.7 (m, 2H), 2.6-2.6 (m, 2H), 2.5-2.6 (m, 3H), 1.15 (d, 3H, *J*=6.2 Hz).

**Example 169**

**3-fluoro-4-[(*2R,6S*)-2-methyl-6-[[4-[6-(5-oxa-2,8-diazaspiro[3.5]nonan-2-yl)-3-pyridyl]piperazin-1-yl]methyl]morpholin-4-yl]pyrazolo[1,5-a]pyridine-7-carbonitrile**

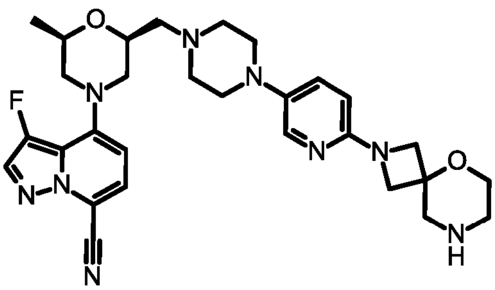

The title compound was prepared in analogy to the preparation of Example **132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and *tert*-butyl 5-oxa-2,8-diazaspiro[3.5]nonane-8-carboxylate instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c** ). **Example 169** (14 mg) was obtained as a light yellow solid. MS: calc'd 562 ($MH^+$), measured 562 ($MH^+$). $^1H$ NMR (METHANOL-$d_4$, 400 MHz) $\delta$ 7.88 (d, 1H, *J*=3.4 Hz), 7.64 (d, 1H, *J*=2.6 Hz), 7.2-7.4 (m, 2H), 6.38 (dd, 2H, *J*=8.6, 11.2 Hz), 3.9-4.0 (m, 1H), 3.8-3.9 (m, 3H), 3.6-3.7 (m, 2H), 3.5-3.6 (m, 3H), 3.41 (br d, 1H, *J*=11.9 Hz), 3.21 (br s, 2H), 2.97 (br t, 3H, *J*=4.5 Hz), 2.92 (s, 2H), 2.6-2.8 (m, 4H), 2.4-2.6 (m, 5H), 1.15 (d, 3H, *J*=6.2 Hz).

**Example 170**

**3-fluoro-4-[(*2R,6S*)-2-methyl-6-[[4-[6-(6-methyl-2,6-diazaspiro[3.3]heptan-2-yl)-3-pyridyl]piperazin-1-yl]methyl]morpholin-4-yl]pyrazolo[1,5-a]pyridine-7-carbonitrile**

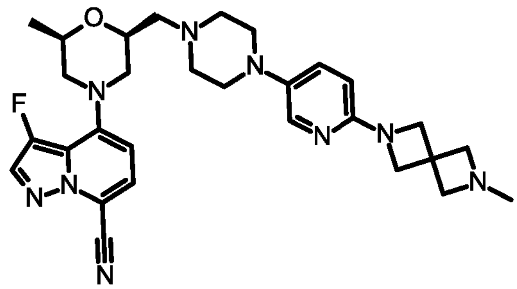

The title compound was prepared in analogy to the preparation of Example **132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and 2-methyl-2,6-diazaspiro[3.3]heptane instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c ). Example 170** (28 mg) was obtained as a light yellow solid. MS: calc'd 546 ($MH^+$), measured 546 ($MH^+$). $^1H$ NMR (METHANOL-$d_4$, 400 MHz) δ 8.01 (d, 1H, *J*=3.7 Hz), 7.74 (d, 1H, *J*=2.6 Hz), 7.44 (d, 1H, *J*=7.9 Hz), 7.40 (dd, 1H, *J*=2.8, 9.0 Hz), 6.53 (d, 1H, J=8.1 Hz), 6.45 (d, 1H, *J*=8.9 Hz), 4.0-4.1 (m, 1H), 4.0-4.0 (m, 4H), 3.92 (ddd, 1H, *J*=2.3, 6.2, 10.1 Hz), 3.67 (br d, 1H, *J*=12.2 Hz), 3.54 (br d, 1H, *J*=12.0 Hz), 3.50 (s, 4H), 3.08 (t, 4H, *J*=4.9 Hz), 2.8-2.8 (m, 2H), 2.6-2.7 (m, 5H), 2.5-2.6 (m, 1H), 2.37 (s, 3H), 1.27 (d, 3H, *J*=6.2 Hz).

**Example 171**

**1-methyl-4-[(*2R,6S*)-2-methyl-6-[[3-(4-piperazin-1-ylphenyl)azetidin-1-yl]methyl]morpholin-4-yl]-1,8-naphthyridin-2-one**

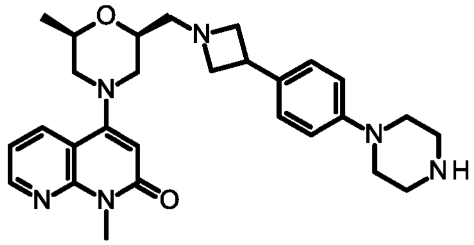

The title compound was prepared in analogy to the preparation of Example **132** by using [(*2R,6R*)-6-methyl-4-(1-methyl-2-oxo-1,8-naphthyridin-4-yl)morpholin-2-yl]methyl trifluoromethanesulfonate (intermediate **M**) and 3-(4-bromophenyl)azetidine and *tert*-butyl piperazine-1-carboxylate instead of [(*2R,6R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a**) and *tert*-butyl (3-methylazetidin-3-yl)carbamate (compound **132c**). **Example 171** (10 mg) was obtained as a light yellow solid. MS: calc'd 489 ($MH^+$), measured 489 ($MH^+$). $^1H$ NMR (METHANOL-$d_4$, 400 MHz) δ 8.65 (dd, 1H, *J*=1.7, 4.6 Hz), 8.26 (dd, 1H, *J*=1.8, 8.0 Hz), 7.3-7.4 (m, 3H), 7.07 (br d, 2H, *J*=8.6 Hz), 6.18 (s, 1H), 4.4-4.7 (m, 3H), 3.9-4.3 (m, 6H), 3.77 (s, 3H), 3.2-3.6 (m, 9H), 2.5-2.7 (m, 3H), 1.28 (d, 3H, *J*=6.1 Hz).

**Example 172**

**4-[*(2S,6R)*-2-[[3-[4-[(*3R,4R*)-3-amino-4-methoxy-pyrrolidin-1-yl]phenyl]azetidin-1-yl]methyl]-6-methyl-morpholin-4-yl]-1-methyl-1,8-naphthyridin-2-one**

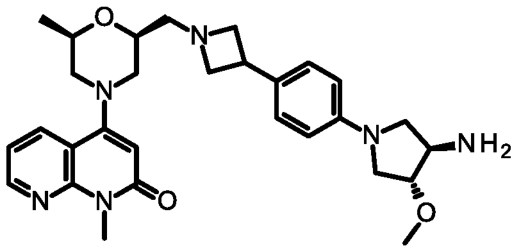

The title compound was prepared in analogy to the preparation of Example **132** by using [(*2R,6R*)-6-methyl-4-(1-methyl-2-oxo-1,8-naphthyridin-4-yl)morpholin-2-yl]methyl trifluoromethanesulfonate (intermediate **M**) and 3-(4-bromophenyl)azetidine and *tert*-butyl *N*-[(*3R,4R*)-4-methoxypyrrolidin-3-yl]carbamate (CAS: 1932066-52-8) instead of [(*2R,6R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a**) and *tert*-butyl (3-methylazetidin-3-yl)carbamate (compound **132c**). **Example 172** (10 mg) was obtained as a light yellow solid. MS: calc'd 519 ($MH^+$), measured 519 ($MH^+$). $^1H$ NMR (METHANOL-$d_4$, 400 MHz) $\delta$ 8.65 (dd, 1H, *J*=1.8, 4.6 Hz), 8.26 (dd, 1H, *J*=1.7, 8.1 Hz), 7.2-7.4 (m, 3H), 6.6-6.8 (m, 2H), 6.18 (s, 1H), 4.0-4.7 (m, 5H), 3.8-3.9 (m, 3H), 3.77 (s, 3H), 3.5-3.6 (m, 4H), 3.4-3.5 (m, 7H), 2.48 (br dd, 2H, *J*=8.5, 11.6 Hz), 1.28 (d, 4H, *J*=6.1 Hz).

## Example 173

**1-methyl-4-[(*2R,6S*)-2-methyl-6-[[3-[4-(5-oxa-2,8-diazaspiro[3.5]nonan-2-yl)phenyl]azetidin-1-yl]methyl]morpholin-4-yl]-1,8-naphthyridin-2-one**

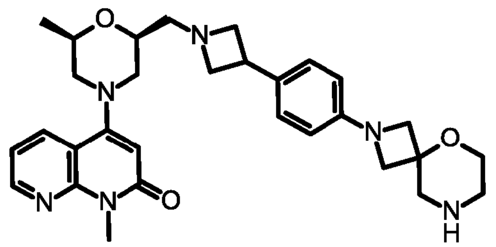

The title compound was prepared in analogy to the preparation of Example **132** by using [(*2R,6R*)-6-methyl-4-(1-methyl-2-oxo-1,8-naphthyridin-4-yl)morpholin-2-yl]methyl trifluoromethanesulfonate (intermediate **M**) and 3-(4-bromophenyl)azetidine and *tert*-butyl 5-oxa-2,8-diazaspiro[3.5]nonane-8-carboxylate instead of [(*2R,6R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a)** and *tert*-butyl (3-methylazetidin-3-yl)carbamate (compound **132c**). **Example 173** (15 mg) was obtained as a light yellow solid. MS: calc'd 531 ($MH^+$), measured 531 ($MH^+$). $^1H$ NMR (METHANOL-$d_4$, 400 MHz) $\delta$ 8.65 (dd, 1H, *J*=1.7, 4.6 Hz), 8.26 (dd, 1H, *J*=1.8, 8.0 Hz), 7.2-7.4 (m, 3H), 6.5-6.7 (m, 2H), 6.18 (s, 1H), 4.68 (s, 3H), 4.1-4.3 (m, 3H), 3.9-4.1 (m, 4H), 3.6-3.8 (m, 7H), 3.3-3.5 (m, 7H), 2.5-2.8 (m, 2H), 1.28 (d, 3H, *J*=6.1 Hz).

## Example 174

**4-[*(2S,6R)*-2-[[3-[4-[(*3R,4R*)-3-amino-4-fluoro-pyrrolidin-1-yl]phenyl]azetidin-1-yl]methyl]-6-methyl-morpholin-4-yl]-1-methyl-1,8-naphthyridin-2-one**

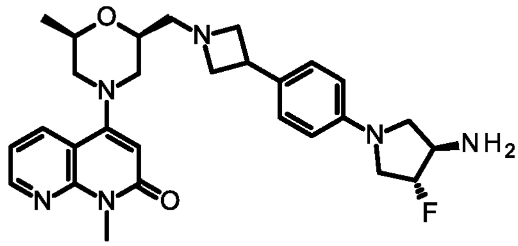

The title compound was prepared in analogy to the preparation of Example **132** by using [(*2R,6R*)-6-methyl-4-(1-methyl-2-oxo-1,8-naphthyridin-4-yl)morpholin-2-yl]methyl trifluoromethanesulfonate (intermediate **M**) and 3-(4-bromophenyl)azetidine and *tert*-butyl *N*-[(*3R,4R*)-4-fluoropyrrolidin-3-yl]carbamate (cas:2097061-04-4) instead of [(*2R,6R*)-4-(8-cyano-2-deuterio-5-quinolyl)-6-methyl-morpholin-2-yl] methyl trifluoromethanesulfonate (intermediate **L**) and 1-(6-bromopyridin-3-yl)piperazine (compound **132a**) and *tert*-butyl (3-methylazetidin-3-yl)carbamate (compound **132c**). **Example 174** (9 mg) was obtained as a light yellow solid. MS: calc'd 507 ($MH^+$), measured 507 ($MH^+$). $^1H$ NMR (METHANOL-$d_4$, 400 MHz) δ 8.65 (dd, 1H, *J*=1.7, 4.6 Hz), 8.26 (dd, 1H, *J*=1.7, 7.9 Hz), 7.32 (dd, 3H, *J*=4.5, 8.0 Hz), 6.73 (br d, 2H, *J*=8.4 Hz), 6.18 (s, 1H), 5.3-5.5 (m, 1H), 4.74 (br d, 3H, *J*=2.6 Hz), 4.0-4.3 (m, 4H), 3.8-4.0 (m, 1H), 3.7-3.8 (m, 4H), 3.4-3.7 (m, 7H), 2.4-2.8 (m, 2H), 1.28 (br d, 3H, *J*=6.2 Hz).

**Example 175**

**4-[*(2S,6R)*-2-[[4-[6-[(*3R,4R*)-4-amino-3-fluoro-1-piperidyl]-3-pyridyl]piperazin-1-yl]methyl]-6-methyl-morpholin-4-yl]-3-fluoro-pyrazolo[1,5-a]pyridine-7-carbonitrile**

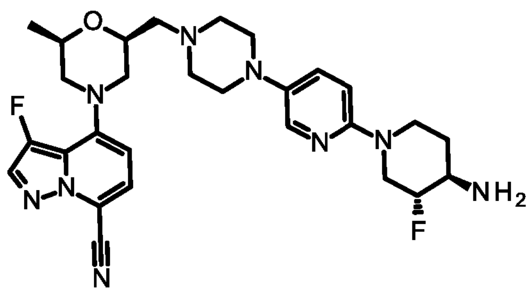

The title compound was prepared in analogy to the preparation of **Example 132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and *tert*-butyl ((*3R,4R*)-3-fluoropiperidin-4-yl)carbamate (CAS: 1523530-29-1) instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound 132c). **Example 175** (3 mg) was obtained as a light yellow solid. MS: calc'd 552 ($MH^+$), measured 552 ($MH^+$). $^1H$ NMR (METHANOL-$d_4$, 400 MHz) δ 7.98 (d, 1H, *J*=3.5 Hz), 7.83 (d, 1H, *J*=2.8 Hz), 7.41 (d, 1H, *J*=7.9 Hz), 7.36 (dd, 1H, *J*=3.1, 9.2 Hz), 6.85 (d, 1H, *J*=9.2 Hz), 6.51 (d, 1H, *J*=8.1 Hz), 4.2-4.4 (m, 2H), 3.9-4.1 (m, 2H), 3.9-3.9 (m, 1H), 3.6-3.7 (m, 1H), 3.5-3.6 (m, 1H), 3.0-3.1 (m, 4H), 2.9-3.0 (m, 1H), 2.7-2.9 (m, 4H), 2.5-2.7 (m, 6H), 1.9-2.0 (m, 1H), 1.50 (dq, 1H, *J*=4.0, 12.3 Hz), 1.24 (d, 3H, *J*=6.2 Hz).

**Example 176**

**4-[*(2S,6R)*-2-[[3-[4-(3-amino-3-methyl-azetidin-1-yl)phenyl]azetidin-1-yl]methyl]-6-methyl-morpholin-4-yl]-1-methyl-1,8-naphthyridin-2-one**

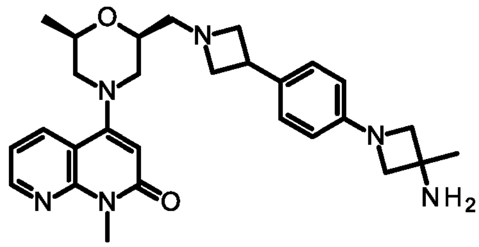

The title compound was prepared in analogy to the preparation of Example **132** by using [(*2R,6R*)-6-methyl-4-(1-methyl-2-oxo-1,8-naphthyridin-4-yl)morpholin-2-yl]methyl trifluoromethanesulfonate (intermediate **M**) and 3-(4-bromophenyl)azetidine instead of [(*2R,6R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a**). **Example 176** (6 mg) was obtained as light yellow solid. MS: calc'd 489 ($MH^+$), measured 489 ($MH^+$). $^1H$ NMR (METHANOL-$d_4$, 400 MHz) δ 8.65 (dd, 1H, *J*=1.8, 4.6 Hz), 8.26 (dd, 1H, *J*=1.7, 8.1 Hz), 7.2-7.4 (m, 3H), 6.5-6.7 (m, 2H), 6.18 (s, 1H), 4.4-4.7 (m, 3H), 4.0-4.4 (m, 5H), 3.8-4.0 (m, 4H), 3.77 (s, 3H), 3.4-3.5 (m, 3H), 2.5-2.8 (m, 2H), 1.66 (s, 3H), 1.28 (d, 3H, *J*=6.1 Hz).

**Example 177**

**3-fluoro-4-[(*2R,6S*)-2-methyl-6-[[4-[4-[(*2R*)-morpholin-2-yl]phenyl]piperazin-1-yl]methyl]morpholin-4-yl]pyrazolo[1,5-a]pyridine-7-carbonitrile**

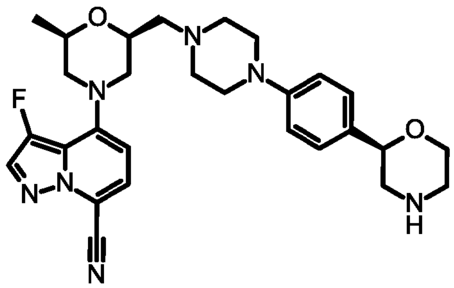

The title compound was prepared according to the following scheme:

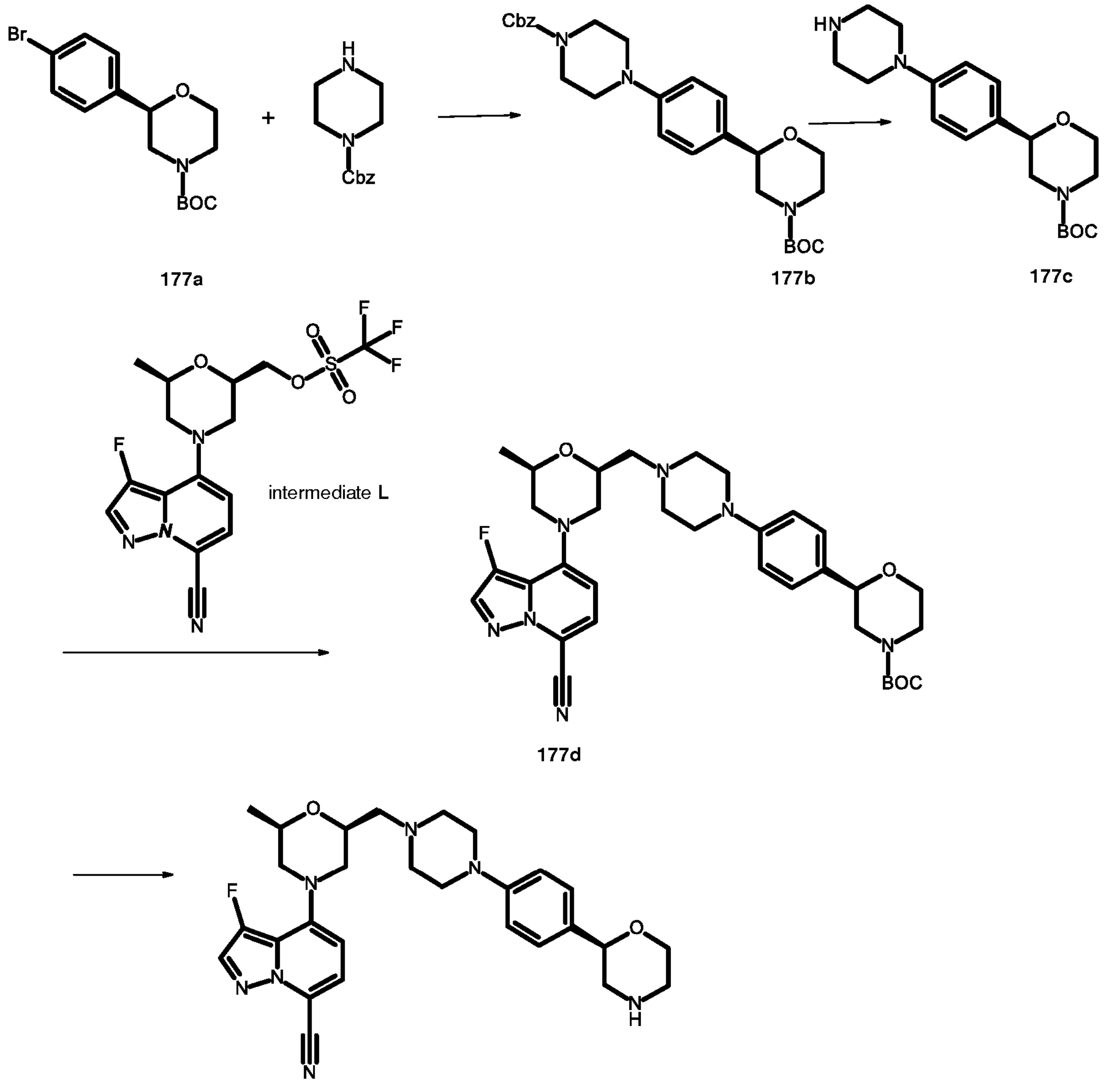


177a 177b 177c

177d

177

**Step 1: *tert*-butyl (*R*)-2-(4-(4-((benzyloxy)carbonyl)piperazin-1-yl)phenyl)-morpholine-4-carboxylate (compound 177b)**

To a solution of *tert*-butyl (*R*)-2-(4-bromophenyl)morpholine-4-carboxylate (**compound 177a,** CAS: 1312566-00-9, 100 mg, 292 μmol, synthesis refers to WO2012126922A1) in dioxane (2 mL) was added RuPhos-Pd-G2 (22.7 mg, 29.2 μmol), benzyl piperazine-1-carboxylate (84 mg, 380 μmol) and $Cs_2CO_3$ (286 mg, 877 μmol). After the reaction mixture was stirred for 16 hours at 120°C under $N_2$, it was filtered and the filtrate was concentrated under reduced pressure. The crude product was purified by silica gel chromatography (EtOAc/PE from 0% to 40%) to afford *tert*-butyl (R)-2-(4-(4-((benzyloxy)carbonyl)piperazin-1-yl)phenyl)morpholine-4-carboxylate (**compound 177b,** 100 mg) as a white solid. MS: calc'd 482 ($MH^+$), measured 482 ($MH^+$).

**Step 2 : *tert*-butyl (*R*)-2-(4-(piperazin-1-yl)phenyl)morpholine-4-carboxylate (compound 177c)**

The solution of *tert*-butyl (*R*)-2-(4-(4-((benzyloxy)carbonyl)piperazin-1-yl)phenyl)morpholine-4-carboxylate (**compound 177b**, 100 mg, 208 μmol) in MeOH (10 ml) was added PdOH/C (10mg). Then the reaction mixture was charged with $H_2$ balloon and stirred at r.t. for 2 hours. The mixture was filtered and concentrated to afford crude product *tert*-butyl (*R*)-2-(4-(piperazin-1-yl)phenyl)morpholine-4-carboxylate (**compound 177c,** 70 mg), which was used for next step without further purification. MS: calc'd 348 ($MH^+$), measured 348 ($MH^+$).

**Step 3 : *tert*-butyl (R)-2-(4-(4-(((*2S,6R*)-4-(7-cyano-3-fluoropyrazolo[1,5-a]pyridin-4-yl)-6-methylmorpholin-2-yl)methyl)piperazin-1-yl)phenyl)morpholine-4-carboxylate (compound 177d)**

A solution of *tert*-butyl (*R*)-2-(4-(piperazin-1-yl)phenyl)morpholine-4-carboxylate (**compound 177c,** 70 mg, 201 μmol), $Et_3N$ (62 mg, 84.2 μL, 604 μmol) and ((2*R*,6*R*)-4-(7-cyano-3-fluoropyrazolo[1,5-a]pyridin-4-yl)-6-methylmorpholin-2-yl)methyl trifluoromethanesulfonate (intermediate **E,** 85 mg, 201 μmol) in acetonitrile (10 mL) was stirred at room temperature for 2 hrs. Then the mixture was concentrated and purified by silica gel Chromatography (MeOH/DCM from 2% to 10%) to afford *tert*-butyl (*R*)-2-(4-(4-(((2*S*,6*R*)-4-(7-cyano-3-fluoropyrazolo[1,5-a]pyridin-4-yl)-6-methylmorpholin-2-yl)methyl)piperazin-1-yl)phenyl)morpholine-4-carboxylate **(compound 177d,** 80 mg) as a yellow solid. MS: calc'd 620 ($MH^+$), measured 620 ($MH^+$).

**Step 4: *tert*-butyl (*R*)-2-(4-(4-(((*2S,6R*)-4-(7-cyano-3-fluoropyrazolo[1,5-a]pyridin-4-yl)-6-methylmorpholin-2-yl)methyl)piperazin-1-yl)phenyl)morpholine-4-carboxylate (Example 177)**

To a solution of *tert*-butyl (*R*)-2-(4-(4-(((2*S*,6*R*)-4-(7-cyano-3-fluoropyrazolo[1,5-a]pyridin-4-yl)-6-methylmorpholin-2-yl)methyl)piperazin-1-yl)phenyl)morpholine-4-carboxylate (**compound 177d,** 80 mg) in DCM (2 mL) was added 2,2,2-trifluoroacetic acid (0.5 mL) at 0 °C. The reaction mixture was stirred at rt for 2 hrs. Then the mixture was concentrated and purified by prep-HPLC to afford 3-fluoro-4-[(*2R,6S*)-2-methyl-6-[[4-[4-[(2*R*)-morpholin-2-yl]phenyl]piperazin-1-yl]methyl]morpholin-4-yl]pyrazolo[1,5-a]pyridine-7-carbonitrile (**Example 177,** 22 mg) as a light yellow solid. MS calc'd 520 ($MH^+$), measured 520 ($MH^+$). $^1H$ NMR (METHANOL-$d_4$, 400 MHz) δ 7.98 (d, 1H, *J*=3.5 Hz), 7.4-7.5 (m, 1H), 7.1-7.3 (m, 2H), 6.94 (br d, 2H, *J*=8.7 Hz), 6.50 (d, 1H, *J*=7.9 Hz), 4.3-4.4 (m, 1H), 3.9-4.1 (m, 3H), 3.7-3.8 (m, 1H), 3.6-3.7 (m, 1H), 3.5-3.5 (m, 1H), 3.1-3.2 (m, 4H), 2.5-2.9 (m, 12H), 1.2-1.3 (m, 3H).

**Example 178**

**3-fluoro-4-[(2*R,6S*)-2-methyl-6-[[4-[6-(5-oxa-2,8-diazaspiro[3.5]nonan-8-yl)-3-pyridyl]piperazin-1-yl]methyl]morpholin-4-yl]pyrazolo[1,5-a]pyridine-7-carbonitrile**

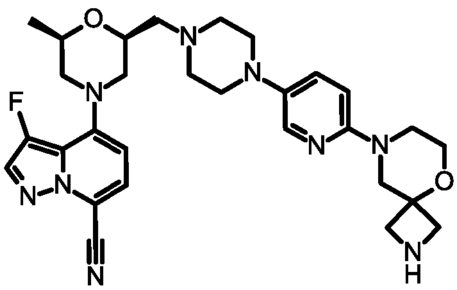

The title compound was prepared in analogy to the preparation of Example **132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and *tert*-butyl 5-oxa-2,8-diazaspiro[3.5]nonane-2-carboxylate (Pharmablock, PBN20111063, CAS: 1251011-05-8) instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c**). **Example 178** (42 mg) was obtained as a light yellow solid. MS: calc'd 562 ($MH^+$), measured 562 ($MH^+$). $^1H$ NMR (METHANOL-$d_4$, 400 MHz) δ 7.99 (d, 1H, *J*=3.7 Hz), 7.87 (dd, 1H, *J*=3.0, 9.6 Hz), 7.78 (d, 1H, *J*=2.9 Hz), 7.43 (d, 1H, *J*=7.9 Hz), 7.26 (d, 1H, *J*=9.5 Hz), 6.54 (d, 1H, *J*=8.1 Hz), 4.3-4.4 (m, 1H), 4.13 (q, 4H, *J*=11.6 Hz), 4.0-4.1 (m, 1H), 3.9-3.9 (m, 2H), 3.81 (s, 2H), 3.5-3.8 (m, 6H), 3.3-3.5 (m, 7H), 3.1-3.3 (m, 1H), 2.7-2.8 (m, 2H), 1.30 (d, 3H, *J*=6.2 Hz).

**Example 179**

**4-[(*2S,6R*)-2-[[4-[6-(1,6-diazaspiro[3.3]heptan-6-yl)-3-pyridyl]piperazin-1-yl]methyl]-6-methyl-morpholin-4-yl]-3-fluoro-pyrazolo[1,5-a]pyridine-7-carbonitrile**

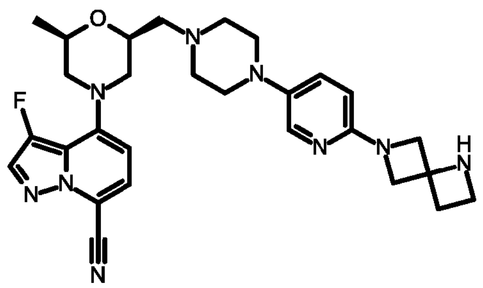

The title compound was prepared in analogy to the preparation of Example **132** by using [(*2R,6R*)-4-(7-cyano-3-fluoro-pyrazolo[1,5-a]pyridin-4-yl)-6-methyl-morpholin-2-yl]methyl trifluoromethanesulfonate (intermediate **E**) and *tert*-butyl 1,6-diazaspiro[3.3]heptane-1-carboxylate(Pharmablock, PBN2011926-02, CAS:1394319-56-2) instead of [(*2R,6R*)-4-(8-cyano-2-deuterio-5-quinolyl)-6-methyl-morpholin-2-yl]methyl trifluoromethanesulfonate (intermediate **L**) and *tert*-butyl (3-methylazetidin-3-yl)carbamate (compound **132c**). **Example 179** (19 mg) was obtained as a light yellow solid. MS: calc'd 532 ($MH^+$), measured 532 ($MH^+$). $^1H$ NMR (METHANOL-$d_4$, 400 MHz) $\delta$ 7.99 (d, 1H, *J*=3.7 Hz), 7.92 (dd, 1H, *J*=2.8, 9.5 Hz), 7.62 (d, 1H, *J*=2.6 Hz), 7.43 (d, 1H, *J*=7.9 Hz), 6.8-6.9 (m, 1H), 6.54 (d, 1H, *J*=7.9 Hz), 4.73 (dd, 2H, *J*=1.4, 10.6 Hz), 4.54 (dd, 2H, *J*=1.3, 10.7 Hz), 4.3-4.4 (m, 1H), 4.0-4.1 (m, 3H), 3.5-3.8 (m, 8H), 3.4-3.5 (m, 4H), 2.9-2.9 (m, 2H), 2.7-2.8 (m, 2H), 1.30 (d, 3H, *J*=6.2 Hz).

**Example 180**

**1-methyl-4-[(2*R,6S*)-2-methyl-6-[[3-[4-(6-methyl-2,6-diazaspiro[3.3]heptan-2-yl)phenyl]azetidin-1-yl]methyl]morpholin-4-yl]-1,8-naphthyridin-2-one**

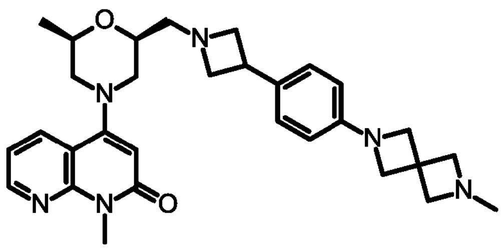

The title compound was prepared in analogy to the preparation of Example **132** by using [(*2R,6R*)-6-methyl-4-(1-methyl-2-oxo-1,8-naphthyridin-4-yl)morpholin-2-yl]methyl trifluoromethanesulfonate (intermediate **M**) and 3-(4-bromophenyl)azetidine and 2-methyl-2,6-diazaspiro[3.3]heptane instead of [(*2R,6R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a**) and *tert*-butyl (3-methylazetidin-3-yl)carbamate (compound **132c**). **Example 180** ( 18 mg) was obtained as a light yellow solid. MS: calc'd 515 ($MH^+$), measured 515 ($MH^+$). $^1H$ NMR (METHANOL-$d_4$, 400 MHz) $\delta$ 8.64 (dd, 1H, *J*=1.7, 4.6 Hz), 8.25 (dd, 1H, *J*=1.6, 7.9 Hz), 7.32 (dd, 1H, *J*=4.6, 7.9 Hz), 7.26 (br d, 2H, *J*=8.3 Hz), 6.53 (br d, 2H, *J*=8.3 Hz), 6.17 (s, 1H), 4.4-4.6 (m, 4H), 4.1-4.3 (m, 5H), 4.0-4.1 (m, 5H), 3.76 (s, 3H), 3.3-3.7 (m, 5H), 2.94 (s, 3H), 2.5-2.7 (m, 2H), 1.28 (br d, 3H, *J*=6.1 Hz).

**Example 182**

**4-[(2*S*,6*R*)-2-[[3-[4-[(4*aR*,7*aR*)-3,4,4*a*,5,7,7*a*-hexahydro-2*H*-pyrrolo[3,4-*b*][1,4]oxazin-6-yl]phenyl]azetidin-1-yl]methyl]-6-methyl-morpholin-4-yl]-1-methyl-1,8-naphthyridin-2-one**

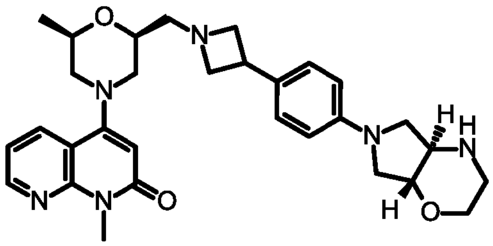

The title compound was prepared in analogy to the preparation of Example **132** by using [(*2R,6R*)-6-methyl-4-(1-methyl-2-oxo-1,8-naphthyridin-4-yl)morpholin-2-yl]methyl trifluoromethanesulfonate (intermediate **M**) and 3-(4-bromophenyl)azetidine and *tert*-butyl rac-(4*aR,7aR*)-3,4a,5,6,7,7a-hexahydro-2*H*-pyrrolo[3,4-*b*][1,4]oxazine-4-carboxylate instead of [(*2R,6R*)-4-(8-cyano-2-deuterio-5-quinolyl)-6-methyl-morpholin-2-yl]methyl trifluoromethanesulfonate (intermediate **L**) and 1-(6-bromopyridin-3-yl)piperazine (compound **132a**) and *tert*-butyl (3-methylazetidin-3-yl)carbamate (compound **132c**). **Example 182** (8 mg) was obtained as a white solid. MS: calc'd 531 ($MH^+$), measured 531 ($MH^+$). $^1H$ NMR (METHANOL-$d_4$, 400 MHz) $\delta$ 8.63 (dd, 1H, *J*=1.7, 4.6 Hz), 8.24 (dd, 1H, *J*=1.7, 7.9 Hz), 7.31 (dd, 1H, *J*=4.6, 8.1 Hz), 7.13 (d, 2H, *J*=8.6 Hz), 6.49 (d, 2H, *J*=8.6 Hz), 6.14 (s, 1H), 3.9-4.0 (m, 3H), 3.7-3.9 (m, 6H), 3.6-3.7 (m, 2H), 3.50 (dt, 2H, *J*=4.5, 7.5 Hz), 3.3-3.4 (m, 2H), 3.23 (dt, 2H, *J*=4.6, 7.8 Hz), 3.13 (t, 1H, *J*=8.8 Hz), 2.9-3.1 (m, 4H), 2.7-2.7 (m, 2H), 2.5-2.6 (m, 2H), 1.22 (d, 3H, *J*=6.2 Hz).

**Example 183**

**3-fluoro-4-[(2*S,6R*)-2-[[4-[2-fluoro-4-[(2*S*)-morpholin-2-yl]phenyl]piperazin-1-yl]methyl]-6-methyl-morpholin-4-yl]pyrazolo[1,5-a]pyridine-7-carbonitrile**

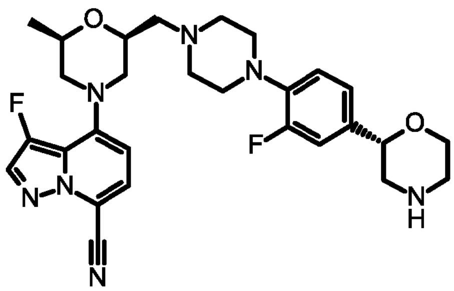

The title compound was prepared in analogy to the preparation of Example **177** by using *tert*-butyl (2*S*)-2-(4-bromo-3-fluoro-phenyl)morpholine-4-carboxylate (CAS: 1447831-94-8, synthesis refers to WO2012126922A1) instead of *tert*-butyl (*R*)-2-(4-bromophenyl)morpholine-4-carboxylate (compound **177a**). **Example 183** (18 mg) was obtained as a white solid. MS: calc'd 538 ($MH^+$), measured 538 ($MH^+$). $^1H$ NMR (METHANOL-$d_4$, 400 MHz) $\delta$ 7.98 (d, 1H, *J*=3.7 Hz), 7.41 (d, 1H, *J*=8.1 Hz), 7.30 (t, 1H, *J*=8.6 Hz), 6.75 (dd, 1H, *J*=2.4, 8.7 Hz), 6.64 (dd, 1H, *J*=2.3, 13.9 Hz), 6.50 (d, 1H, *J*=7.9 Hz), 4.67 (dd, 1H, *J*=2.3, 10.5 Hz), 4.0-4.1 (m, 1H), 3.96 (dd, 1H, *J*=2.6, 11.6 Hz), 3.9-3.9 (m, 1H), 3.75 (dt, 1H, *J*=3.0, 11.5 Hz), 3.65 (br d, 1H, *J*=12.3 Hz), 3.52 (br d, 1H, *J*=12.1 Hz), 3.21 (t, 4H, *J*=5.1 Hz), 2.8-3.0 (m, 3H), 2.7-2.8 (m, 3H), 2.6-2.7 (m, 4H), 2.6-2.6 (m, 1H), 2.5-2.6 (m, 1H), 1.24 (d, 3H, *J*=6.2 Hz).

**Example 184**

**3-fluoro-4-[(2*S*,6*R*)-2-[[4-[3-fluoro-4-1(2*R*)-morpholin-2-yl]phenyl]piperazin-1-yl]methyl]-6-methyl-morpholin-4-yl]pyrazolo[1,5-a]pyridine-7-carbonitrile**

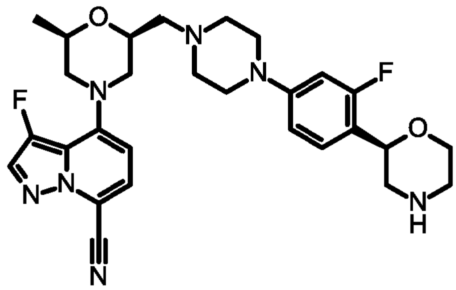

The title compound was prepared in analogy to the preparation of Example **177** by using *tert*-butyl (2*R*)-2-(4-bromo-2-fluoro-phenyl)morpholine-4-carboxylate (CAS: 14400997-81-0, synthesis refers to WO2012126922A1) instead of *tert*-butyl (*R*)-2-(4-bromophenyl)morpholine-4-carboxylate (compound **177a**). **Example 184** ( 64 mg) was obtained as a white solid. MS: calc'd 538 ($MH^+$), measured 538 ($MH^+$). $^1H$ NMR (METHANOL-$d_4$, 400 MHz) $\delta$ 7.99 (d, 1H, *J*=3.7 Hz), 7.4-7.4 (m, 1H), 7.0-7.1 (m, 3H), 6.5-6.5 (m, 1H), 4.40 (dd, 1H, *J*=2.3, 10.5 Hz), 3.9-4.1 (m, 3H), 3.73 (dt, 1H, *J*=3.4, 11.3 Hz), 3.65 (br d, 1H, *J*= 12.2 Hz), 3.52 (br d, 1H, *J*=12.0 Hz), 3.10 (t, 4H, *J*=4.6 Hz), 2.9-3.0 (m, 1H), 2.7-2.9 (m, 4H), 2.6-2.7 (m, 6H), 2.5-2.6 (m, 1H), 1.25 (d, 3H, *J*=6.4 Hz).

**Example 185**

**3-fluoro-4-[(2*R*,6*S*)-2-methyl-6-[[4-[4-(2-methylmorpholin-2-yl)phenyl]piperazin-1-yl]methyl]morpholin-4-yl]pyrazolo[1,5-a]pyridine-7-carbonitrile**

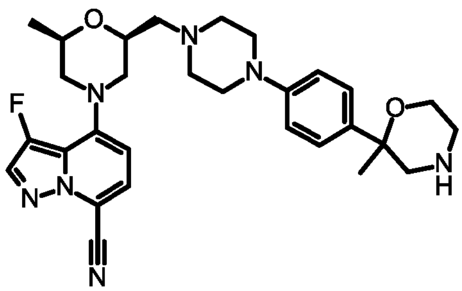

The title compound was prepared in analogy to the preparation of Example **177** by using *tert*-butyl 2-(4-bromophenyl)-2-methyl-morpholine-4-carboxylate (CAS: 179821-59-1, synthesis refers to WO 9618615 A1) instead of *tert*-butyl (*R*)-2-(4-bromophenyl)morpholine-4-carboxylate (compound **177a**). **Example 185** ( 35 mg) was obtained as a white solid. MS: calc'd 534 ($MH^+$), measured 534 ($MH^+$). $^1H$ NMR (METHANOL-$d_4$, 400 MHz) $\delta$ 7.99 (d, 1H, *J*=3.5 Hz), 7.41 (d, 1H, *J*=7.9 Hz), 7.31 (d, 2H, *J*=8.8 Hz), 7.00 (d, 2H, *J*=8.9 Hz), 6.51 (d, 1H, *J*=7.9 Hz), 4.0-4.1 (m, 1H), 3.9-3.9 (m, 1H), 3.6-3.7 (m, 3H), 3.5-3.5 (m, 1H), 3.39 (d, 1H, *J*=13.6 Hz), 3.20 (t, 4H, *J*=5.0 Hz), 2.7-2.9 (m, 4H), 2.6-2.7 (m, 4H), 2.5-2.6 (m, 3H), 1.3 (s, 3H), 1.25 (d, 3H, *J*=6.4 Hz).

**Example 186**

**3-fluoro-4-[(2*R*,6*S*)-2-methyl-6-[[4-[4-[[(2*R*)-morpholin-2-yl]methyl]phenyl]piperazin-1-yl]methyl]morpholin-4-yl]pyrazolo[1,5-a]pyridine-7-carbonitrile**

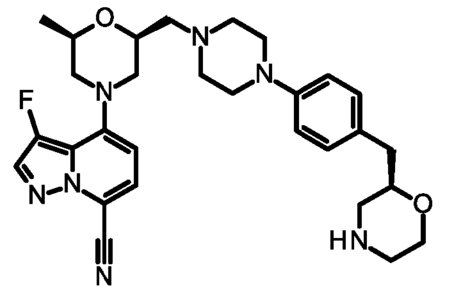

The title compound was prepared in analogy to the preparation of Example **177** by using *tert*-butyl (2*R*)-2-[(4-bromophenyl)methyl]morpholine-4-carboxylate (compound **186a**) instead of *tert*-butyl (*R*)-2-(4-bromophenyl)morpholine-4-carboxylate (compound **177a**). **Example 186** ( 42 mg) was obtained as a white solid. MS: calc'd 534 ($MH^+$), measured 534 ($MH^+$). $^1H$ NMR (METHANOL-$d_4$, 400 MHz) δ 7.99 (d, 1H, *J*=3.7 Hz), 7.42 (d, 1H, *J*=7.9 Hz), 7.09 (d, 2H, *J*=8.6 Hz), 6.90 (d, 2H, *J*=8.7 Hz), 6.52 (s, 1H), 4.0-4.1 (m, 1H), 3.9-3.9 (m, 1H), 3.81 (br d, 1H, *J*=11.0 Hz), 3.5-3.7 (m, 4H), 3.16 (t, 4H, *J*=5.0 Hz), 2.7-2.8 (m, 5H), 2.5-2.7 (m, 9H), 1.25 (d, 3H, *J*=6.2 Hz).

**Preparation of *tert*-butyl (2*R*)-2-[(4-bromophenyl)methyl]morpholine-4-carboxylate (compound 186a)**

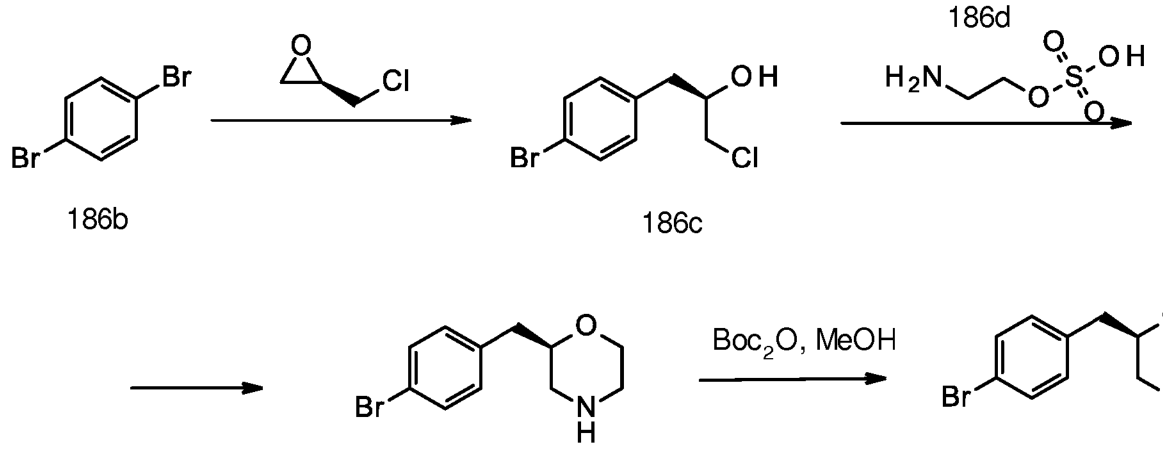


186b

186c

186e

186a

**Step 1: preparation of (2*R*)-1-(4-bromophenyl)-3-chloro-propan-2-ol (compound 186c)**

Magnesium (587 mg, 24.2 mmol) was placed in a three-necked flask, fitted with Ar-stream. 10 mL of THF was added. 1,4-dibromobenzene (compound **186b,** 6 g, 25.4 mmol) dissolved in 20 mL of THF was slowly dropped into the flask. The reaction mixture was heated under reflux for 2 hours until all the magnesium disappeared. The solution was cooled down to 0 °C and (*R*)-2-(chloromethyl)oxirane (2.35 g, 1.99 mL, 25.4 mmol) dissolved in 10mL THF was added dropwise in 10 minutes. After the reaction mixture was stirred for 1 hour, saturated $NH_4Cl$ was added and the solution was extracted with EtOAC twice. The combined organic layer was dried over $MgSO_4$ and evaporated. Then the residue was purified by silica gel chromatography (EA/PE from 20% to 80%) to afford compound **186c** (3.9g). MS: calc'd 249 ($MH^+$), measured 249 ($MH^+$).

**Step 2: preparation of (2*R*)-2-[(4-bromophenyl)methyl]morpholine (compound 186e)**

(2*R*)-1-(4-bromophenyl)-3-chloro-propan-2-ol (compound **186c,** 3.34 g, 13.4 mmol) was dissolved in methanol (6.7 mL). NaOH (3.21 g, 80.3 mmol) dissolved in water (3.5 mL) was added to form a suspension. After the reaction mixture was stirred at r.t. for another hour, 2-aminoethyl hydrogen sulfate (compound **186d,** 7.56 g, 53.5 mmol) was added. The reaction mixture was heated at 45 ° C for 2 hours and diluted with toluene (23 mL), then NaOH (3.37 g, 84.3

mmol) was added. After being stirred at 65 °C overnight, the reaction mixture was cooled down and diluted with water, extracted with EtOAc. The organic layer was washed with brine, dried over $MgSO_4$ and concentrated. The residue was purified by silica gel chromatography (EA/PE from 20% to 80%) to afford compound **186e** (1.1g). MS: calc'd 256 ($MH^+$), measured 256 ($MH^+$).

**Step 3: preparation of *tert*-butyl (2*R*)-2-[(4-bromophenyl)methyl]morpholine-4-carboxylate (compound 186a )**

(2*R*)-2-[(4-bromophenyl)methyl] morpholine (compound **186e,** 1.042 g, 4.07 mmol) was dissolved in DCM (10 mL). Then di-*tert*-butyl dicarbonate (1.07 g, 4.88 mmol) was added and the reaction mixture was stirred at rt for 1 hour. After the reaction mixture was concentrated, the residue was purified by silica gel chromatography (EA/PE from 20% to 80%) to afford compound **186a** (1.4 g). MS: calc'd 356 ($MH^+$), measured 356 ($MH^+$).

**Example 187**

The following tests were carried out in order to determine the activity of the compounds of formula (I) and (Ia) in HEK293-Blue-hTLR-7/8/9 cells assay.

**HEK293-Blue-hTLR-7 cells assay:**

A stable HEK293-Blue-hTLR-7 cell line was purchased from InvivoGen (Cat.#: hkb-htlr7, San Diego, California, USA). These cells were originally designed for studying the stimulation of human TLR7 by monitoring the activation of NF-xB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR7 cells with TLR7 ligands. Therefore the reporter expression was declined by TLR7 antagonist under the stimulation of a ligand, such as R848 (Resiquimod), for incubation of 20 hrs. The cell culture supernatant SEAP reporter activity was determined using QUANTI-Blue™ kit (Cat.#: rep-qbl, Invivogen, San Diego, Ca, USA) at a wavelength of 640 nm, a detection medium that turns purple or blue in the presence of alkaline phosphatase.

HEK293-Blue-hTLR7 cells were incubated at a density of 250,000~450,000 cells/mL in a volume of 170 ,uL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 μL test compound in a serial dilution in the presence of final DMSO at 1% and 10 μL of 20uM R848 in above DMEM, perform incubation under 37 °C in a $CO_2$ incubator for 20 hrs. Then 20 μL of the supernatant from each well was incubated with 180 μL Quanti-blue substrate solution at 37 °C for 2 hrs and the absorbance was read at 620-655 nm using a spectrophotometer. The signaling pathway that TLR7 activation leads to downstream NF-κB activation has been widely accepted, and therefore similar reporter assay was modified for evaluating TLR7 antagonist.

**HEK293-Blue-hTLR-8 cells assay:**

A stable HEK293-Blue-hTLR-8 cell line was purchased from InvivoGen (Cat.#: hkb-htlr8, San Diego, California, USA). These cells were originally designed for studying the stimulation of human TLR8 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR8 cells with TLR8 ligands. Therefore the reporter expression was declined by TLR8 antagonist under the stimulation of a ligand, such as R848, for incubation of 20 hrs. The cell culture supernatant SEAP reporter activity was determined using QUANTI-Blue™ kit (Cat.#: rep-qbl, Invivogen, San Diego, Ca, USA) at a wavelength of 640 nm, a detection medium that turns purple or blue in the presence of alkaline phosphatase.

HEK293-Blue-hTLR8 cells were incubated at a density of 250,000~450,000 cells/mL in a volume of 170 μL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 μL test compound in a serial dilution in the presence of final DMSO at 1% and 10 μL of 60uM R848 in above DMEM, perform incubation under 37 °C in a $CO_2$ incubator for 20 hrs. Then 20 μL of the supernatant from each well was incubated with 180 μL Quanti-blue substrate solution at 37°C for 2 hrs and the absorbance was read at 620-655 nm using a spectrophotometer. The signaling pathway that TLR8 activation leads to downstream NF-κB activation has been widely accepted, and therefore similar reporter assay was modified for evaluating TLR8 antagonist.

**HEK293-Blue-hTLR-9 cells assay:**

A stable HEK293-Blue-hTLR-9 cell line was purchased from InvivoGen (Cat.#: hkb-htlr9, San Diego, California,

USA). These cells were originally designed for studying the stimulation of human TLR9 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR9 cells with TLR9 ligands. Therefore the reporter expression was declined by TLR9 antagonist under the stimulation of a ligand, such as ODN2006 (Cat.#: tlrl-2006-1, Invivogen, San Diego, California, USA), for incubation of 20 hrs. The cell culture supernatant SEAP reporter activity was determined using QUANTI-Blue™ kit (Cat.#: rep-qb1, Invivogen, San Diego, California, USA) at a wavelength of 640 nm, a detection medium that turns purple or blue in the presence of alkaline phosphatase.

HEK293-Blue-hTLR9 cells were incubated at a density of 250,000~450,000 cells/mL in a volume of 170 μL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 μL test compound in a serial dilution in the presence of final DMSO at 1% and 10 μL of 20uM ODN2006 in above DMEM, perform incubation under 37 °C in a $CO_2$ incubator for 20 hrs. Then 20 μL of the supernatant from each well was incubated with 180 μL Quanti-blue substrate solution at 37 °C for 2 h and the absorbance was read at 620-655 nm using a spectrophotometer. The signaling pathway that TLR9 activation leads to downstream NF-κB activation has been widely accepted, and therefore similar reporter assay was modified for evaluating TLR9 antagonist.

The compounds of formula (I) or (Ia) have human TLR7 and/or TLR8 inhibitory activities ($IC_{50}$ value) <0.5 μM. Moreover, some compounds also have human TLR9 inhibitory activity <0.5μM. Activity data of the compounds of the present invention were shown in Table 2.

**Table 2. The activity of the compounds of present invention in HEK293-Blue-hTLR-7/8/9 cells assays**

| Example No | HEK/hTLR7 $IC_{50}$ (μM) | HEK/hTLR8 $IC_{50}$ (μM) | HEK/hTLR9 $IC_{50}$ (μM) |
|---|---|---|---|
| **R1 (reference compound)** | 0.095 | 0.142 | 6.623 |
| **R2 (reference compound)** | 0.210 | 0.342 | 7.034 |
| 1 | 0.084 | 0.040 | 0.136 |
| 2 | 0.093 | 0.026 | 0.074 |
| 3 | 0.011 | 0.009 | 0.052 |
| 4 | 0.016 | 0.017 | 0.068 |
| 5 | 0.054 | 0.033 | 0.198 |
| 6 | 0.051 | 0.007 | 0.120 |
| 7 | 0.034 | 0.009 | 0.051 |
| 8 | 0.050 | 0.010 | 0.093 |
| 9 | 0.019 | 0.003 | 0.032 |
| 10 | 0.033 | 0.010 | 0.105 |
| 11 | 0.023 | 0.013 | 0.092 |
| 12 | 0.012 | 0.003 | 0.032 |
| 13 | 0.018 | 0.005 | 0.142 |
| 14 | 0.006 | 0.003 | 0.032 |
| 15 | 0.007 | 0.003 | 0.036 |
| 17 | 0.031 | 0.006 | 0.186 |
| 18 | 0.011 | 0.006 | 0.052 |
| 19 | 0.025 | 0.012 | 0.091 |
| 20 | 0.020 | 0.010 | 0.096 |
| 21 | 0.016 | 0.010 | 0.219 |
| 22 | 0.010 | 0.003 | 0.045 |
| 23 | 0.050 | 0.018 | 0.232 |

(continued)

| Example No | HEK/hTLR7 $IC_{50}$ (μM) | HEK/hTLR8 $IC_{50}$ (μM) | HEK/hTLR9 $IC_{50}$ (μM) |
|---|---|---|---|
| 24 | 0.038 | 0.011 | 0.032 |
| 25 | 0.026 | 0.007 | 0.033 |
| 26 | 0.008 | 0.003 | 0.032 |
| 27 | 0.015 | 0.003 | 0.043 |
| 28 | 0.011 | 0.004 | 0.065 |
| 29 | 0.016 | 0.010 | 0.051 |
| 30 | 0.016 | 0.006 | 0.250 |
| 31 | 0.018 | 0.005 | 0.046 |
| 32 | 0.006 | 0.003 | 0.073 |
| 33 | 0.024 | 0.007 | 0.116 |
| 34 | 0.027 | 0.004 | 0.071 |
| 35 | 0.065 | 0.033 | 0.147 |
| 36 | 0.028 | 0.011 | 0.118 |
| 37 | 0.070 | 0.030 | 0.234 |
| 38 | 0.016 | 0.011 | 0.097 |
| 39 | 0.051 | 0.023 | 0.104 |
| 40 | 0.035 | 0.017 | 0.134 |
| 41 | 0.081 | 0.032 | 0.150 |
| 42 | 0.023 | 0.013 | 0.147 |
| 43 | 0.022 | 0.011 | 0.119 |
| 44 | 0.028 | 0.010 | 0.164 |
| 45 | 0.015 | 0.004 | 0.188 |
| 46 | 0.018 | 0.009 | 0.157 |
| 47 | 0.013 | 0.003 | 0.067 |
| 48 | 0.009 | 0.004 | 0.121 |
| 49 | 0.029 | 0.010 | 0.146 |
| 50 | 0.016 | 0.003 | 0.126 |
| 51 | 0.028 | 0.003 | 0.121 |
| 52 | 0.015 | 0.003 | 0.032 |
| 53 | 0.008 | 0.003 | 0.032 |
| 54 | 0.009 | 0.003 | 0.034 |
| 55 | 0.014 | 0.003 | 0.032 |
| 56 | 0.056 | 0.006 | 0.034 |
| 57 | 0.027 | 0.004 | 0.038 |
| 58 | 0.030 | 0.004 | 0.045 |
| 59 | 0.011 | 0.003 | 0.032 |
| 60 | 0.011 | 0.003 | 0.032 |
| 61 | 0.008 | 0.003 | 0.032 |

(continued)

| Example No | HEK/hTLR7 $IC_{50}$ ($\mu$M) | HEK/hTLR8 $IC_{50}$ ($\mu$M) | HEK/hTLR9 $IC_{50}$ ($\mu$M) |
|---|---|---|---|
| 62 | 0.018 | 0.014 | 0.092 |
| 63 | 0.054 | 0.013 | 0.246 |
| 64 | 0.005 | 0.002 | 0.035 |
| 65 | 0.015 | 0.009 | 0.129 |
| 66 | 0.017 | 0.008 | 0.032 |
| 67 | 0.024 | 0.023 | 0.081 |
| 68 | 0.009 | 0.002 | 0.095 |
| 69A | 0.011 | 0.002 | 0.054 |
| 69B | 0.007 | 0.002 | 0.070 |
| 70 | 0.070 | 0.012 | 0.080 |
| 71 | 0.045 | 0.015 | 0.066 |
| 72 | 0.006 | 0.003 | 0.046 |
| 73 | 0.049 | 0.017 | 0.069 |
| 74 | 0.024 | 0.003 | 0.072 |
| 75 | 0.025 | 0.003 | 0.057 |
| 76 | 0.020 | 0.003 | 0.050 |
| 77 | 0.026 | 0.002 | 0.056 |
| 78 | 0.020 | 0.001 | 0.065 |
| 79 | 0.021 | 0.002 | 0.063 |
| 80 | 0.107 | 0.006 | 0.120 |
| 81 | 0.022 | 0.002 | 0.046 |
| 82 | 0.029 | 0.001 | 0.056 |
| 83 | 0.032 | 0.003 | 0.096 |
| 84 | 0.010 | 0.007 | 0.104 |
| 86 | 0.022 | 0.006 | 0.189 |
| 87 | 0.008 | 0.002 | 0.075 |
| 88 | 0.021 | 0.002 | 0.071 |
| 89 | 0.006 | 0.029 | 0.099 |
| 90 | 0.021 | 0.002 | 0.054 |
| 91 | 0.013 | 0.008 | 0.102 |
| 92 | 0.005 | 0.002 | 0.042 |
| 93 | 0.012 | 0.003 | 0.031 |
| 94 | 0.013 | 0.006 | 0.051 |
| 95 | 0.007 | 0.003 | 0.031 |
| 96 | 0.018 | 0.004 | 0.109 |
| 97 | 0.027 | 0.001 | 0.034 |
| 98 | 0.006 | 0.001 | 0.049 |
| 99 | 0.011 | 0.001 | 0.063 |

(continued)

| Example No | HEK/hTLR7 $IC_{50}$ (μM) | HEK/hTLR8 $IC_{50}$ (μM) | HEK/hTLR9 $IC_{50}$ (μM) |
|---|---|---|---|
| 101 | 0.011 | 0.004 | 0.031 |
| 102 | 0.018 | 0.002 | 0.080 |
| 103 | 0.010 | 0.001 | 0.100 |
| 104 | 0.022 | 0.001 | 0.070 |
| 105 | 0.013 | 0.000 | 0.069 |
| 106 | 0.009 | 0.001 | 0.046 |
| 107 | 0.008 | 0.001 | 0.080 |
| 108 | 0.005 | 0.001 | 0.068 |
| 109 | 0.016 | 0.002 | 0.046 |
| 110 | 0.014 | 0.002 | 0.068 |
| 111 | 0.003 | 0.001 | 0.037 |
| 112 | 0.004 | 0.001 | 0.065 |
| 113 | 0.008 | 0.001 | 0.098 |
| 114 | 0.003 | 0.001 | 0.062 |
| 115 | 0.004 | 0.001 | 0.038 |
| 116 | 0.021 | 0.005 | 0.089 |
| 117 | 0.013 | 0.007 | 0.109 |
| 118 | 0.004 | 0.001 | 0.051 |
| 119 | 0.016 | 0.001 | 0.094 |
| 120 | 0.005 | 0.001 | 0.031 |
| 121 | 0.008 | 0.001 | 0.044 |
| 122 | 0.005 | 0.001 | 0.031 |
| 123 | 0.003 | 0.001 | 0.031 |
| 124 | 0.005 | 0.001 | 0.031 |
| 125 | 0.047 | 0.002 | 0.083 |
| 126 | 0.021 | 0.006 | 0.146 |
| 127 | 0.029 | 0.009 | 0.149 |
| 128 | 0.030 | 0.012 | 0.182 |
| 129 | 0.020 | 0.007 | 0.171 |
| 130 | 0.016 | 0.008 | 0.155 |
| 131 | 0.032 | 0.004 | 0.113 |
| 132 | 0.023 | 0.016 | 0.140 |
| 133 | 0.041 | 0.017 | 0.068 |
| 134 | 0.025 | 0.001 | 0.048 |
| 135 | 0.016 | 0.001 | 0.056 |
| 136 | 0.007 | 0.001 | 0.033 |
| 137 | 0.019 | 0.011 | 0.066 |
| 138 | 0.005 | 0.019 | 0.040 |

(continued)

| Example No | HEK/hTLR7 $IC_{50}$ ($\mu$M) | HEK/hTLR8 $IC_{50}$ ($\mu$M) | HEK/hTLR9 $IC_{50}$ ($\mu$M) |
|---|---|---|---|
| 139 | 0.020 | 0.013 | 0.078 |
| 140 | 0.022 | 0.025 | 0.162 |
| 141 | 0.004 | 0.009 | 0.044 |
| 142 | 0.020 | 0.017 | 0.064 |
| 143 | 0.021 | 0.008 | 0.047 |
| 144 | 0.011 | 0.001 | 0.031 |
| 145 | 0.014 | 0.014 | 0.031 |
| 146 | 0.013 | 0.014 | 0.031 |
| 147 | 0.053 | 0.011 | 0.181 |
| 148 | 0.056 | 0.049 | 0.075 |
| 149 | 0.016 | 0.012 | 0.068 |
| 150 | 0.035 | 0.007 | 0.175 |
| 151 | 0.035 | 0.023 | 0.096 |
| 152 | 0.015 | 0.017 | 0.099 |
| 153 | 0.032 | 0.019 | 0.113 |
| 154 | 0.032 | 0.050 | 0.112 |
| 155 | 0.018 | 0.008 | 0.071 |
| 156 | 0.021 | 0.024 | 0.046 |
| 157 | 0.008 | 0.118 | 0.031 |
| 158 | 0.003 | 0.037 | 0.041 |
| 159 | 0.030 | 0.052 | 0.100 |
| 160 | 0.011 | 0.068 | 0.163 |
| 161 | 0.008 | 0.008 | 0.059 |
| 162 | 0.011 | 0.011 | 0.036 |
| 163 | 0.022 | 0.068 | 0.101 |
| 164 | 0.010 | 0.030 | 0.086 |
| 165 | 0.007 | 0.092 | 0.108 |
| 166 | 0.021 | 0.031 | 0.046 |
| 167 | 0.014 | 0.034 | 0.046 |
| 168 | 0.020 | 0.057 | 0.116 |
| 169 | 0.009 | 0.030 | 0.078 |
| 170 | 0.006 | 0.013 | 0.070 |
| 171 | 0.038 | 0.129 | 0.035 |
| 172 | 0.041 | 0.093 | 0.057 |
| 173 | 0.029 | 0.074 | 0.06 |
| 174 | 0.047 | 0.159 | 0.086 |
| 175 | 0.010 | 0.012 | 0.066 |
| 176 | 0.036 | 0.323 | 0.101 |

(continued)

| Example No | HEK/hTLR7 $IC_{50}$ (μM) | HEK/hTLR8 $IC_{50}$ (μM) | HEK/hTLR9 $IC_{50}$ (μM) |
|---|---|---|---|
| 177 | 0.016 | 0.027 | 0.098 |
| 178 | 0.038 | 0.091 | 0.116 |
| 179 | 0.030 | 0.054 | 0.157 |
| 180 | 0.053 | 0.077 | 0.041 |
| 182 | 0.026 | 0.051 | 0.039 |
| 183 | 0.028 | 0.013 | 0.12 |
| 184 | 0.023 | 0.014 | 0.124 |
| 185 | 0.039 | 0.033 | 0.186 |
| 186 | 0.018 | 0.023 | 0.124 |

**Example 188**

**hERG channel inhibition assay:**

The hERG channel inhibition assay is a highly sensitive measurement that identifies compounds exhibiting hERG inhibition related to cardiotoxicity in vivo. The hERG $K^+$ channels were cloned in humans and stably expressed in a CHO (Chinese hamster ovary) cell line. $CHO_{hERG}$ cells were used for patch-clamp (voltage-clamp, whole-cell) experiments. Cells were stimulated by a voltage pattern to activate hERG channels and conduct $I_{KhERG}$ currents (rapid delayed outward rectifier potassium current of the hERG channel). After the cells were stabilized for a few minutes, the amplitude and kinetics of $I_{KhERG}$ were recorded at a stimulation frequency of 0.1 Hz, (6 bpm). Thereafter, the test compound was added to the preparation at increasing concentrations. For each concentration, an attempt was made to reach a steady-state effect, usually, this was achieved within 3-10 min at which time the next highest concentration was applied. The amplitude and kinetics of $I_{KhERG}$ are recorded in each concentration of the drug which were compared to the control values (taken as 100%). (references: Redfern WS, Carlsson L, Davis AS, Lynch WG, MacKenzie I, Palethorpe S, Siegl PK, Strang I, Sullivan AT, Wallis R, Camm AJ, Hammond TG. 2003; Relationships between preclinical cardiac electrophysiology, clinical QT interval prolongation and torsade de pointes for a broad range of drugs: evidence for a provisional safety margin in drug development. Cardiovasc. Res. 58:32-45, Sanguinetti MC, Tristani-Firouzi M. 2006; hERG potassium channels and cardiac arrhythmia. Nature 440:463-469, Webster R, Leishman D, Walker D. 2002; Towards a drug concentration effect relationship for QT prolongation and torsades de pointes. Curr. Opin. Drug Discov. Devel. 5:116-26).

Results of hERO are given in Table 3. A safety ratio (hERG $IC_{20}$ /$EC_{50}$) > 30 suggests a sufficient window to differentiate the pharmacology by inhibiting TLR7/8/9 pathways from the potential hERG related cardiotoxicity. According to the calculation of hERG $IC_{20}$ / TLR7/8/9 $IC_{50}$ below which serves as early selectivity index to assess hERG liability, obviously reference compounds ER-887258, ER-888285, ER-888286, R1 and R2 have much narrower safety window compared to the compounds of this invention.

**Table 3. hERG and safety ratio results**

| Example No | hERG $IC_{20}$ (μM) | hERG $IC_{50}$ (μM) | hERG $IC_{20}$/ TLR7 $IC_{50}$ | hERG $IC_{20}$/ TLR8 $IC_{50}$ | hERG $IC_{20}$/ TLR9 $IC_{50}$ |
|---|---|---|---|---|---|
| **ER-887258** | 0.687 | 2.784 | 8.1 | N.A. | 0.3 |
| **ER-888285** | 1.006 | 3.105 | 8.4 | N.A. | 0.5 |
| **ER-888286** | 0.348 | 1.297 | 0.3 | N.A. | 0.2 |
| **R1** | 0.879 | 2.745 | 9.3 | 6.2 | 0.1 |
| **R2** | 0.280 | 0.770 | 1.3 | 0.8 | 0.04 |
| **7** | >10 | >20 | >294 | >1111 | >196 |
| **15** | >10 | >20 | >1429 | >3333 | >278 |

(continued)

| Example No | hERG $IC_{20}$ (μM) | hERG $IC_{50}$ (μM) | hERG $IC_{20}$/ TLR7 $IC_{50}$ | hERG $IC_{20}$/ TLR8 $IC_{50}$ | hERG $IC_{20}$/ TLR9 $IC_{50}$ |
|---|---|---|---|---|---|
| **38** | 5.5 | >20 | 344 | 500 | 57 |
| **98** | >10 | >20 | >1666 | >10000 | >204 |
| **99** | 9 | >20 | 818 | 9000 | 142 |
| **104** | >10 | >20 | >454 | >10000 | >142 |
| **114** | >10 | >20 | >3333 | >10000 | >161 |
| **118** | >10 | >20 | >2500 | >10000 | >196 |
| **124** | >10 | >20 | >2000 | >10000 | >322 |
| **132** | >10 | >20 | >434 | >625 | >71 |
| **143** | >10 | >20 | >476 | >1250 | >212 |
| **144** | >10 | >20 | >909 | >10000 | >322 |
| **154** | >10 | >20 | >312 | >200 | >89 |
| **166** | 8 | >20 | 380 | 258 | 173 |
| **167** | >10 | >20 | >714 | >294 | >217 |
| **168** | 8.6 | >20 | 430 | 150 | 74 |

**Example 189**

The compounds would be desirable to have minimal DDI liabilities. Therefore, the effects of compounds of formula (I) of (Ia) on major CYP isoforms, e.g. CYP2C9, CYP2D6 and CYP3A4, are determined.

**CYP inhibition assay**

This is a high throughput screening assay used for assessment of reversible inhibition of CYP2C9, CYP2D6, and CYP3A4 activity of test compounds in human liver microsome (HLM) in early discovery stage.

**Table 4. Chemicals and materials used in the CYP inhibition assay**

| **Substances** | **Description** | **Source** | **Cat. No.** | **Final Concentration in incubation** |
|---|---|---|---|---|
| Human Liver Microsomes | | BD-Gentest | 452117 | 0.2 mg/mL |
| Diclofenac | CYP2C9 substrate | Sigma | D-6899 | 5 μM |
| 4'-Hydroxydiclofenac | CYP2C9 product | | | |
| 4'-OH-Diclofenac-13C6 | CYP2C9 internal standard | Becton Dickinson | 451006 | |
| Dextromethorphan | CYP2D6 substrate | Sigma | D-2531 | 5 μM |
| Dextrorphan | CYP2D6 product | | | |
| Dextrorphan-D3 | CYP2D6 internal standard | Promochem | CERD-041 | |
| Midazolam | CYP3A4 substrate | Roche | | 5 μM |
| 1'-Hydroxymidazolam | CYP3A4 product | | | |
| 1'-OH Midazolam-D4 | CYP3A4 internal standard | Roche | | |
| Sulfaphenazole | CYP2C9 inhibitor | | | 2 μM |

(continued)

| Substances | Description | Source | Cat. No. | Final Concentration in incubation |
|---|---|---|---|---|
| Quinidine | CYP2D6 inhibitor | | | 0.5 μM |
| Ketoconazole | CYP3A4 inhibitor | | | 0.5 μM |

Procedure

10 mM DMSO stock solutions of test compounds were diluted in DMSO to generate 2 mM intermediate stock solution. 250 nL of intermediate stock solution were transferred in duplicate into 3 separate 384 well microtitre plates (assay-ready plates). A mixture of HLM and each substrate was made up. 45 μL of HLM substrate mix was then transferred to each well of an assay ready plate and mixed. The negative (solvent) and positive controls (standard inhibitor for each CYP) were included in each assay ready plate. The assay ready plate was warmed to 37 °C in an incubator over 10 minutes. 5 μL pre-warmed NADPH regenerating system was added to each incubation well to start the reaction. Final incubation volume was 50 μL. The assay plate then was placed back in the 37 °C incubator. After incubation (10 minutes for CYP2D6) for 5 minutes, incubates were quenched by addition of 50 μL 100% acetonitrile containing internal standards (400 ng/mL 13C6-4'-OH-Diclofenac, 20 ng/mL D3-Dextrorphan and 20 ng/mL D4-1'OH-Midazolam). The supernatants were collected for RapidFire/MS/MS analysis.

RapidFire online solid phase extraction/sample injection system (Agilent) coupled with API4000 triple quadrupole mass spectrometer (AB Sciex) were used for sample analysis. The mobile phase composed of acetonitrile and water supplemented with 0.1% formic acid. A C4 solid phase extraction cartridge is used for sample separation. MS detection is achieved in positive ion MRM mode.

Data analysis

Peak areas for substrate, metabolite and internal standard are determined using the RapidFire integrator software (version 3.6.12009.12296). Peak area ratios (PAR) of metabolite and internal standard (stable-labelled metabolite) are then calculated. The measurement window for each experiment is then defined:

PAR (0% activity) = average PAR for all incubations containing concentrated inhibitor;
Par (100% activity) = average PAR for all incubations containing no inhibitor (DMSO controls);

$$\begin{aligned}&\%\ \text{Activity (test inhibitor)}\\&= [\text{PAR(test inhibitor)-PAR}(0\%\ \text{activity})]/[\text{PAR}(100\%\ \text{activity})\text{-PAR}(0\%\ \text{activity})];\end{aligned}$$

$$\%\ \text{Inhibition (test inhibitor)} = 100\text{-}\%\ \text{Activity (test inhibitor)}.$$

The compounds of present invention were found to have low CYP inhibition for CYP2D6 determined in the assays described above.

**Table 5. CYP inhibition of the compounds of this invention for CYP2D6**

| Example No | CYP (%) 2C9/2D6/3A4 |
|---|---|
| **ER-888286** | 29.5 / 52.5 / 5.5 |
| **1** | -7 / 5 / 14 |
| **2** | 2 / 20 / 18 |
| **3** | -18 / 13.5 / 4.5 |
| **4** | 5.5 / 11 / 0.5 |
| **7** | 2 / 7.5 / 15.5 |
| **8** | 1 / 6 / -3.5 |

(continued)

| Example No | CYP (%) 2C9/2D6/3A4 |
|---|---|
| **9** | -7.5 / 2 / 7.5 |
| **11** | 0.5 / 0 / 11 |
| **14** | 0 / 16.5 / -2 |
| **15** | 9 / -2.5 / 11.5 |
| **19** | 11.5 / 0.5 / 12.5 |
| **21** | -3 / 2.5 / -1.5 |
| **22** | 20 / 2 / -16 |
| **24** | 3.5 / 6 / 4 |
| **25** | -5.5 / 5.5 / -4 |
| **29** | -2 / 8.5 / -5.5 |
| **38** | 20 / 20.5 / 20 |
| **39** | 23 / -4 / 23.5 |
| **43** | 2.5 /-11 / 16 |
| **45** | -3.5 / 5 / -5 |
| **52** | 3.5 / 10.5 / 2 |
| **53** | -4.5 / 21.5 / 7.5 |
| **104** | -1 / 15 / -12 |
| **106** | 17 / 3 / 20.5 |
| **107** | 19.5 / 16 / 34.5 |
| **110** | 22 / 13.5 / 44 |
| **112** | 0.5 / -2 / -5 |
| **114** | 7.5 / 32 / 16 |
| **116** | -8.5 / 10 / 8 |
| **117** | 8.5 / 4.5 / 10 |
| **119** | -12.5 / -8.5 / 23.5 |
| **124** | -2 / 9.5 / 16 |
| **132** | -1 / -2.5 / 7.5 |
| **134** | 44.5 / -4 / 47.5 |
| **143** | -9.5 / -30 / 18 |
| **152** | -1 / 12.5 / 35 |
| **154** | 2.5 / -10.5 / 10.5 |
| **158** | -5.5 / -7.5 / -0.5 |
| **160** | -1.5 / 17 / 4.5 |
| **162** | -2.5 / 1 / 42.5 |
| **163** | -4.5 / 6.5 / 0.5 |
| **164** | -1 / 26 / 30.5 |
| **165** | -8.5 / 17.5 / 9 |
| **166** | -3.5 / 20 / 35.5 |

(continued)

| Example No | CYP (%) 2C9/2D6/3A4 |
|---|---|
| **167** | -1 / 11.5 / 44 |
| **168** | -8 / 1.5 / 11 |
| **170** | -1 / 0 / -7 |
| ND: not detected; percentage inhibition <0: not or weak inhibitor | |

## Claims

**1.** A compound of formula (I),

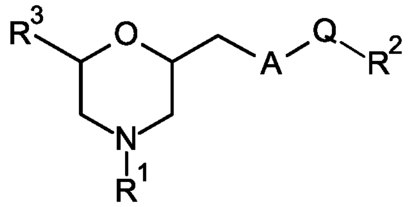 (I),

wherein

$R^1$ is

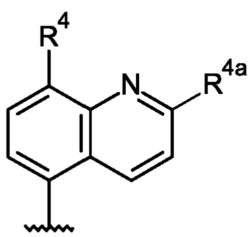, 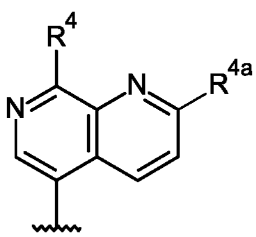, 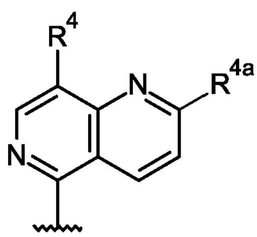, 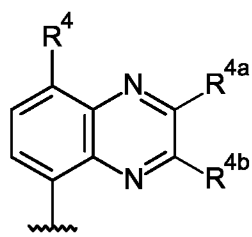,

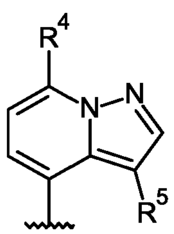, 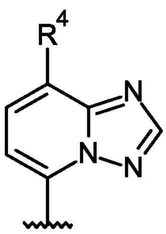, 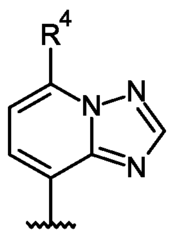, 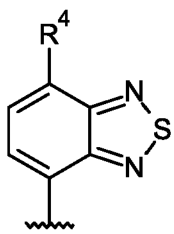, 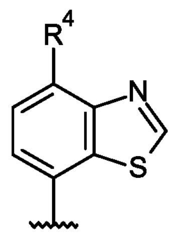,

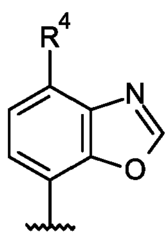, 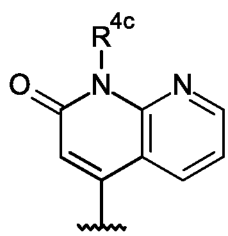 or 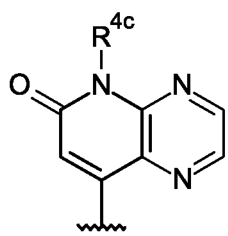;

wherein $R^4$ is $C_{1-6}$alkyl, $C_{1-6}$alkoxy, halo$C_{1-6}$alkyl, halogen, nitro or cyano; $R^{4a}$ is H or deuterium; $R^{4b}$ is H,

deuterium or $C_{1-6}$alkyl; $R^{4c}$ is $C_{1-6}$alkyl or $C_{3-7}$cycloalkyl; $R^5$ is H or halogen;
$R^2$ is (($C_{1-6}$alkyl)zamino)$C_{1-6}$alkoxy;

($C_{1-6}$alkoxypyrrolidinyl)amino;
(cyanopyrrolidinyl)amino;
1,4-diazepanyl substituted by hydroxy;
1,6-diazaspiro[3.3]heptanyl;
2,5-diazabicyclo[2.2.1]heptanyl;
2,6-diazaspiro[3.3]heptanyl substituted by $C_{1-6}$alkyl;
3,4,4a,5,7,7a-hexahydro-2H-pyrrolo[3,4-b][1,4]oxazinyl;
3-oxa-7,9-diazabicyclo[3.3.1]nonanyl;
5-oxa-2,8-diazaspiro[3.5]nonanyl;
amino$C_{1-6}$alkyl;
aminooxazepanyl;
azetidinyl substituted once or twice by substituents independently selected from amino and $C_{1-6}$alkyl;
azetidinylamino;
halopyrrolidinylamino;
morpholinyl unsubstituted or substituted by $C_{1-6}$alkyl;
morpholinyl$C_{1-6}$alkyl;
piperazinyl unsubstituted or substituted by $C_{1-6}$alkyl, hydroxy$C_{1-6}$alkyl, pyrrolidinylcarbonyl, (($C_{1-6}$alkyl)zamino)$C_{1-6}$alkylcarbonyl or azetidinylcarbonyl;
piperidinyl unsubstituted or substituted once or twice by substituents independently selected from amino, halogen and $C_{1-6}$alkoxy; or
pyrrolidinyl substituted once, twice or three times by substituents independently selected from amino, halogen, hydroxy, $C_{1-6}$alkyl and $C_{1-6}$alkoxy;

$R^3$ is $C_{1-6}$alkyl;
A is azetidinyl, hydroxyazetidinyl, piperazinyl, pyrrolidinyl, piperidinyl or cyanopiperidinyl;
Q is phenyl unsubstituted or substituted by halogen;

pyrazinyl;
pyridazinyl;
pyridinyl unsubstituted or substituted by $C_{1-6}$alkyl;
pyrimidinyl unsubstituted or substituted once or twice by substituents independently selected from halogen, $C_{1-6}$alkyl, $C_{1-6}$alkoxy and cyano; or
quinazolinyl;

or a pharmaceutically acceptable salt thereof.

**2.** A compound according to claim 1 of formula (Ia),

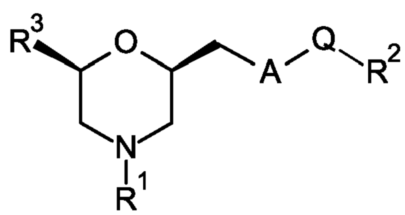

(Ia),

wherein

$R^1$ is

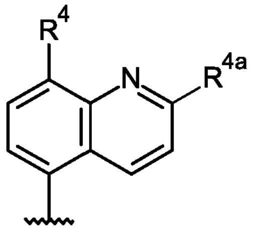, 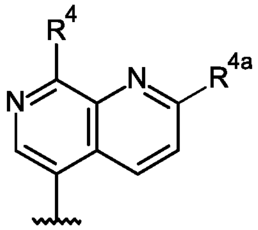, 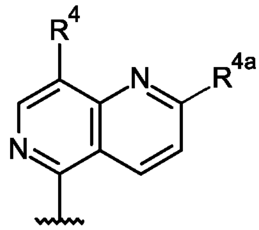, 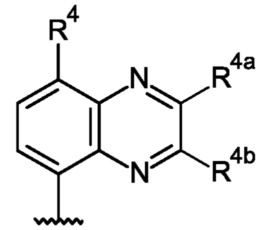,

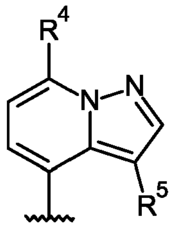, 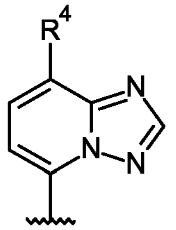, 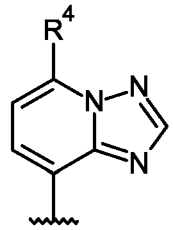, 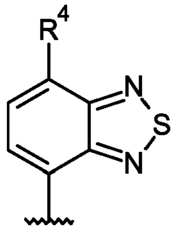, 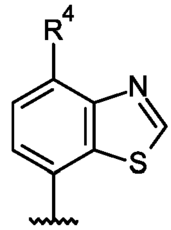,

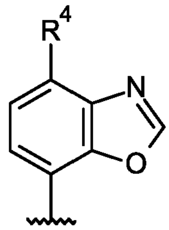, 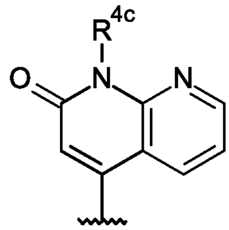 or 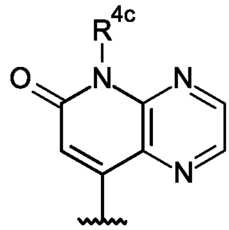;

wherein $R^4$ is $C_{1-6}$alkyl, $C_{1-6}$alkoxy, halo$C_{1-6}$alkyl, halogen, nitro or cyano; $R^{4a}$ is H or deuterium; $R^{4b}$ is H, deuterium or $C_{1-6}$alkyl; $R^{4c}$ is $C_{1-6}$alkyl or $C_{3-7}$cycloalkyl; $R^5$ is H or halogen;
$R^2$ is (($C_{1-6}$alkyl)zamino)$C_{1-6}$alkoxy;

($C_{1-6}$alkoxypyrrolidinyl)amino;
(cyanopyrrolidinyl)amino;
1,4-diazepanyl substituted by hydroxy;
1,6-diazaspiro[3.3]heptanyl;
2,5-diazabicyclo[2.2.1]heptanyl;
2,6-diazaspiro[3.3]heptanyl substituted by $C_{1-6}$alkyl;
3,4,4a,5,7,7a-hexahydro-2H-pyrrolo[3,4-b][1,4]oxazinyl;
3-oxa-7,9-diazabicyclo[3.3.1]nonanyl;
5-oxa-2,8-diazaspiro[3.5]nonanyl;
amino$C_{1-6}$alkyl;
aminooxazepanyl;
azetidinyl substituted once or twice by substituents independently selected from amino and $C_{1-6}$alkyl;
azetidinylamino;
halopyrrolidinylamino;
morpholinyl unsubstituted or substituted by $C_{1-6}$alkyl;
morpholinyl$C_{1-6}$alkyl;
piperazinyl unsubstituted or substituted by $C_{1-6}$alkyl, hydroxy$C_{1-6}$alkyl, pyrrolidinylcarbonyl, (($C_{1-6}$alkyl)zamino)$C_{1-6}$alkylcarbonyl or azetidinylcarbonyl;
piperidinyl unsubstituted or substituted once or twice by substituents independently selected from amino, halogen and $C_{1-6}$alkoxy; or
pyrrolidinyl substituted once, twice or three times by substituents independently selected from amino, halogen, hydroxy, $C_{1-6}$alkyl and $C_{1-6}$alkoxy;

$R^3$ is $C_{1-6}$alkyl;

A is azetidinyl, hydroxyazetidinyl, piperazinyl, pyrrolidinyl, piperidinyl or cyanopiperidinyl;
Q is phenyl unsubstituted or substituted by halogen;

pyrazinyl;
pyridazinyl;
pyridinyl unsubstituted or substituted by $C_{1-6}$alkyl;
pyrimidinyl unsubstituted or substituted once or twice by substituents independently selected from halogen, $C_{1-6}$alkyl, $C_{1-6}$alkoxy and cyano; or
quinazolinyl;

or a pharmaceutically acceptable salt thereof.

**3.** A compound according to claim 1 or 2, or a pharmaceutically acceptable salt thereof, wherein $R^1$ is

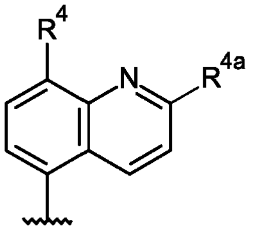 , 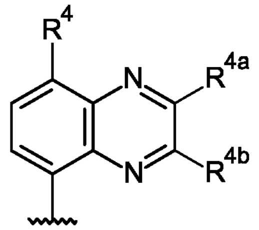 , 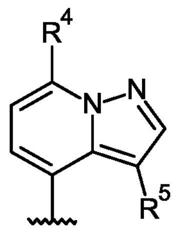 or 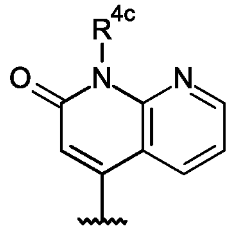 ;

wherein $R^4$ is $C_{1-6}$alkyl, halo$C_{1-6}$alkyl or cyano; $R^{4a}$ is H or deuterium; $R^{4b}$ is H or deuterium; $R^{4c}$ is $C_{1-6}$alkyl; $R^5$ is H or halogen.

**4.** A compound according to claim 3, or a pharmaceutically acceptable salt thereof, wherein $R^4$ is methyl, trifluoromethyl or cyano; $R^{4a}$ is H or deuterium; $R^{4b}$ is H or deuterium; $R^{4c}$ is methyl; $R^5$ is H or fluoro.

**5.** A compound according to claim 3, or a pharmaceutically acceptable salt thereof, wherein $R^2$ is (((${C_{1-6}}$alkyl)$_2$amino)$C_{1-6}$alkylcarbonyl)piperazinyl; (($C_{1-6}$alkyl)$_2$amino)$C_{1-6}$alkoxy; (azetidinylcarbonyl)piperazinyl; ($C_{1-6}$alkoxypyrrolidinyl)amino; ($C_{1-6}$alkyl)morpholinyl; (cyanopyrrolidinyl)amino; (hydroxy$C_{1-6}$alkyl)piperazinyl; (pyrrolidinylcarbonyl)piperazinyl; 1,6-diazaspiro[3.3]heptanyl; 2,5-diazabicyclo[2.2.1]heptanyl; 3,4,4a,5,7,7a-hexahydro-2H-pyrrolo[3,4-b][1,4]oxazinyl; 3-oxa-7,9-diazabicyclo[3.3.1]nonanyl; 5-oxa-2,8-diazaspiro[3.5]nonanyl; amino($C_{1-6}$alkoxy)piperidinyl; amino($C_{1-6}$alkoxy)pyrrolidinyl; amino($C_{1-6}$alkyl)azetidinyl; amino(hydroxy)($C_{1-6}$alkyl)pyrrolidinyl; aminoazetidinyl; amino$C_{1-6}$alkyl; aminohalopiperidinyl; aminohalopyrrolidinyl; aminooxazepanyl; aminopiperidinyl; azetidinylamino; $C_{1-6}$alkyl-2,6-diazaspiro[3.3]heptanyl; $C_{1-6}$alkylpiperazinyl; halopyrrolidinylamino; hydroxy-1,4-diazepanyl; morpholinyl; morpholinyl$C_{1-6}$alkyl; piperazinyl or piperidinyl.

**6.** A compound according to claim 5, or a pharmaceutically acceptable salt thereof, wherein $R^2$ is (1-hydroxy-1-methylethyl)piperazin-1-yl; (2-pyrrolidinylcarbonyl)piperazin-1-yl; (4-cyanopyrrolidin-3-yl)amino; (4-fluoropyrrolidin-3-yl)amino; (4-methoxypyrrolidin-3-yl)amino; (dimethylamino)ethoxy; 1,6-diazaspiro[3.3]heptan-6-yl; 2,5-diazabicyclo[2.2.1]heptan-2-yl; 2-methylmorpholin-2-yl; 2-morpholin-2-yl; 3-(hydroxymethyl)piperazin-1-yl; 3,4,4a,5,7,7a-hexahydro-2H-pyrrolo[3,4-b][1,4]oxazin-6-yl; 3-amino-3-methyl-azetidin-1-yl; 3-amino-4-fluoro-1-piperidinyl; 3-amino-4-fluoro-pyrrolidin-1-yl; 3-amino-4-methoxy-1-piperidinyl; 3-amino-4-methoxy-pyrrolidin-1-yl; 3-amino-5-fluoro-1-piperidinyl; 3-aminoazetidin-1-yl; 3-methylpiperazin-1-yl; 3-oxa-7,9-diazabicyclo[3.3.1]nonan-7-yl; 4-(azetidinyl-2-carbonyl)piperazin-1-yl; 4-[2-(dimethylamino)acetyl]piperazin-1-yl; 4-amino-1-piperidinyl; 4-amino-3,3-difluoro-1-piperidinyl; 4-amino-3,3-difluoro-pyrrolidin-1-yl; 4-amino-3-fluoro-1-piperidinyl; 4-amino-3-hydroxy-3-methyl-pyrrolidin-1-yl; 4-amino-3-methoxy-1-piperidinyl; 4-methylpiperazin-1-yl; 4-piperidinyl; 5-amino-3,3-difluoro-1-piperidinyl; 5-oxa-2,8-diazaspiro[3.5]nonan-2-yl; 5-oxa-2,8-diazaspiro[3.5]nonan-8-yl; 6-amino-1,4-oxazepan-4-yl; 6-hydroxy-1,4-diazepan-1-yl; 6-methyl-2,6-diazaspiro[3.3]heptan-2-yl; aminomethyl; azetidin-3-ylamino; morpholin-2-yl; morpholin-2-ylmethyl or piperazin-1-yl.

**7.** A compound according to claim 6, or a pharmaceutically acceptable salt thereof, wherein A is

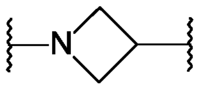 , 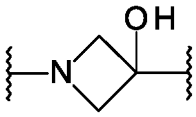 , 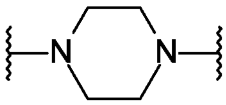 , 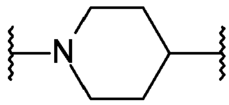 ,

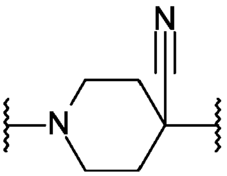 or 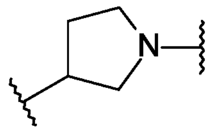 .

8. A compound according to claim 7, or a pharmaceutically acceptable salt thereof, wherein Q is phenyl, fluorophenyl, pyrazinyl, pyridazinyl, pyridinyl, methylpyridinyl, pyrimidinyl, cyanopyrimidinyl, fluoropyrimidinyl, methoxypyrimidinyl, methylpyrimidinyl, dimethylpyrimidinyl or quinazolinyl.

9. A compound according to claim 8, or a pharmaceutically acceptable salt thereof, wherein $R^3$ is methyl.

10. A compound according to claim 1 or 2, or a pharmaceutically acceptable salt thereof, wherein $R^1$ is

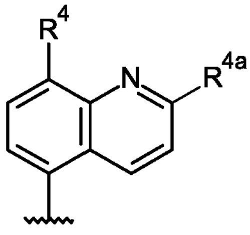 , 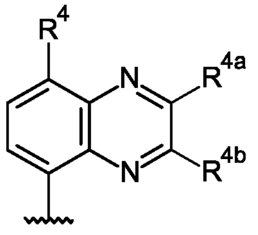 or 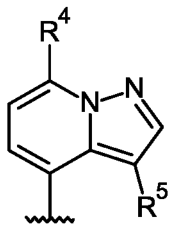 ;

wherein $R^4$ is $C_{1-6}$alkyl, halo$C_{1-6}$alkyl or cyano; $R^{4a}$ and $R^{4b}$ are simultaneously H or deuterium; $R^5$ is H or halogen.

11. A compound according to claim 10, or a pharmaceutically acceptable salt thereof, wherein $R^4$ is methyl, trifluoromethyl or cyano; $R^{4a}$ and $R^{4b}$ are simultaneously H or deuterium; $R^5$ is H or fluoro.

12. A compound according to claim 10, or a pharmaceutically acceptable salt thereof, wherein $R^2$ is 3,4,4a,5,7,7a-hexahydro-2H-pyrrolo[3,4-b][1,4]oxazinyl; 5-oxa-2,8-diazaspiro[3.5]nonanyl; amino($C_{1-6}$alkoxy)pyrrolidinyl; amino($C_{1-6}$alkyl)azetidinyl; aminoazetidinyl; aminopiperidinyl; $C_{1-6}$alkyl-2,6-diazaspiro[3.3]heptanyl; halopyrrolidinylamino; morpholinyl; morpholinyl$C_{1-6}$alkyl or piperazinyl.

13. A compound according to claim 12, or a pharmaceutically acceptable salt thereof, wherein $R^2$ is (4-fluoropyrrolidin-3-yl)amino; 3,4,4a,5,7,7a-hexahydro-2H-pyrrolo[3,4-b][1,4]oxazin-6-yl; 3-amino-3-methyl-azetidin-1-yl; 3-amino-4-methoxy-pyrrolidin-1-yl; 3-aminoazetidin-1-yl; 4-amino-1-piperidinyl; 5-oxa-2,8-diazaspiro[3.5]nonan-2-yl; 6-methyl-2,6-diazaspiro[3.3]heptan-2-yl; morpholin-2-yl; morpholin-2-ylmethyl or piperazin-1-yl.

14. A compound according to claim 12, or a pharmaceutically acceptable salt thereof, wherein A is

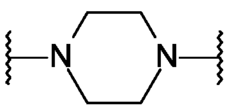 .

15. A compound according to claim 14, or a pharmaceutically acceptable salt thereof, wherein Q is phenyl, pyridinyl, pyrimidinyl or $C_{1-6}$alkylpyrimidinyl.

16. A compound according to claim 15, or a pharmaceutically acceptable salt thereof, wherein Q is phenyl, pyridinyl,

pyrimidinyl or methylpyrimidinyl.

**17.** A compound according to claim 3, wherein

$R^1$ is

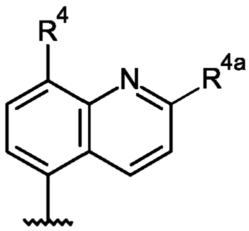
,
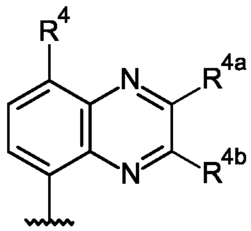
or
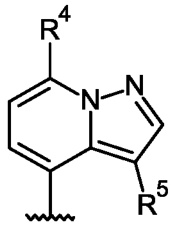
;

wherein $R^4$ is $C_{1-6}$alkyl, halo$C_{1-6}$alkyl or cyano; $R^{4a}$ and $R^{4b}$ are simultaneously H or deuterium; $R^5$ is H or halogen;
$R^2$ is 3,4,4a,5,7,7a-hexahydro-2H-pyrrolo[3,4-b][1,4]oxazinyl; 5-oxa-2,8-diazaspiro[3.5]nonanyl; amino($C_{1-6}$alkoxy)pyrrolidinyl; amino($C_{1-6}$alkyl)azetidinyl; aminoazetidinyl; aminopiperidinyl; $C_{1-6}$alkyl-2,6-diazaspiro[3.3]heptanyl; halopyrrolidinylamino; morpholinyl; morpholinyl$C_{1-6}$alkyl or piperazinyl;
$R^3$ is $C_{1-6}$alkyl;
A is

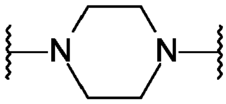
;

Q is phenyl, pyridinyl, pyrimidinyl or $C_{1-6}$alkylpyrimidinyl;
or a pharmaceutically acceptable salt thereof.

**18.** A compound according to claim 17, wherein

$R^1$ is

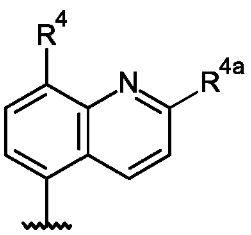
,
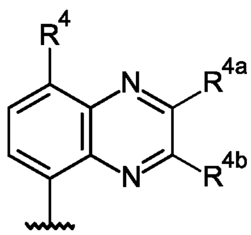
or
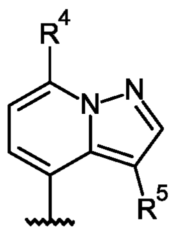
;

wherein $R^4$ is methyl, trifluoromethyl or cyano; $R^{4a}$ and $R^{4b}$ are simultaneously H or deuterium; $R^5$ is H or fluoro;
$R^2$ is (4-fluoropyrrolidin-3-yl)amino; 3,4,4a,5,7,7a-hexahydro-2H-pyrrolo[3,4-b][1,4]oxazin-6-yl; 3-amino-3-methyl-azetidin-1-yl; 3-amino-4-methoxy-pyrrolidin-1-yl; 3-aminoazetidin-1-yl; 4-amino-1-piperidinyl; 5-oxa-2,8-diazaspiro[3.5]nonan-2-yl; 6-methyl-2,6-diazaspiro[3.3]heptan-2-yl; morpholin-2-yl; morpholin-2-ylmethyl or piperazin-1-yl;
$R^3$ is methyl;
A is

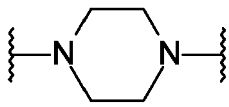
;

Q is phenyl, pyridinyl, pyrimidinyl or methylpyrimidinyl;
or a pharmaceutically acceptable salt thereof.

**19.** A compound according to claim 1 selected from:

5-[(*2R*,*6S*)-2-methyl-6-[[3-(3-piperazin-1-ylphenyl)pyrrolidin-1-yl]methyl]morpholin-4-yl]quinoline-8-carbonitrile;
5-[(*2R*,*6S*)-2-methyl-6-[[4-[3-(4-piperidyl)phenyl]-1-piperidyl]methyl]morpholin-4-yl]quinoline-8-carbonitrile;
5-[(*2R*,*6S*)-2-methyl-6-[[3-(4-piperazin-1-ylphenyl)azetidin-1-yl]methyl]morpholin-4-yl]quinoline-8-carbonitrile;
5-[(*2R*,*6S*)-2-methyl-6-[[3-(3-piperazin-1-ylphenyl)azetidin-1-yl]methyl]morpholin-4-yl]quinoline-8-carbonitrile;
5-[(*2S*, *6R*)-2-[[4-[4-(aminomethyl)phenyl]piperazin-1-yl]methyl]-6-methyl-morpholin-4-yl]quinoline-8-carbonitrile;
5-[(*2R*,*6S*)-2-methyl-6-[[4-(5-piperazin-1-ylpyrazin-2-yl)piperazin-1-yl]methyl]morpholin-4-yl]quinoline-8-carbonitrile;
5-[(*2R*,*6S*)-2-methyl-6-[[4-(5-piperazin-1-yl-3-pyridyl)piperazin-1-yl]methyl]morpholin-4-yl]quinoline-8-carbonitrile;
5-[(*2R*,*6S*)-2-methyl-6-[[4-(5-piperazin-1-yl-2-pyridyl)piperazin-1-yl]methyl]morpholin-4-yl]quinoline-8-carbonitrile;
5-[(*2R*,*6S*)-2-methyl-6-[[4-(4-piperazin-1-yl-2-pyridyl)piperazin-1-yl]methyl]morpholin-4-yl]quinoline-8-carbonitrile;
5-[(*2R*,*6S*)-2-methyl-6-[[4-(6-piperazin-1-yl-2-pyridyl)piperazin-1-yl]methyl]morpholin-4-yl]quinoline-8-carbonitrile;
5-[(*2S*,*6R*)-2-[[4-cyano-4-(4-piperazin-1-ylphenyl)-1-piperidyl]methyl]-6-methyl-morpholin-4-yl]quinoline-8-carbonitrile;
5-[(*2R*,*6S*)-2-methyl-6-[[4-(2-piperazin-1-ylpyrimidin-5-yl)piperazin-1-yl]methyl]morpholin-4-yl]quinoline-8-carbonitrile;
5-[(*2R*,*6S*)-2-methyl-6-[[4-(6-piperazin-1-yl-3-pyridyl)piperazin-1-yl]methyl]morpholin-4-yl]quinoline-8-carbonitrile;
5-[(*2R*,*6S*)-2-methyl-6-[[4-(4-piperazin-1-ylpyrimidin-2-yl)piperazin-1-yl]methyl]morpholin-4-yl]quinoline-8-carbonitrile;
5-[(*2R*,*6S*)-2-methyl-6-[[4-(2-piperazin-1-ylpyrimidin-4-yl)piperazin-1-yl]methyl]morpholin-4-yl]quinoline-8-carbonitrile;
5-[(*2R*,*6S*)-2-methyl-6-[[4-(6-piperazin-1-ylpyridazin-3-yl)piperazin-1-yl]methyl]morpholin-4-yl]quinoline-8-carbonitrile;
5-[(*2R*,*6S*)-2-methyl-6-[[4-(5-piperazin-1-ylpyrimidin-2-yl)piperazin-1-yl]methyl]morpholin-4-yl]quinoline-8-carbonitrile;
5-[(*2R*,*6S*)-2-methyl-6-[[4-(6-piperazin-1-ylpyrimidin-4-yl)piperazin-1-yl]methyl]morpholin-4-yl]quinoline-8-carbonitrile;
5-[(*2R*,*6S*)-2-methyl-6-[[4-[4-[(2*R*)-morpholin-2-yl]phenyl]piperazin-1-yl]methyl]morpholin-4-yl]quinoline-8-carbonitrile;
5-[(*2R*,*6S*)-2-methyl-6-[[4-[4-[(2*S*)-morpholin-2-yl]phenyl]piperazin-1-yl]methyl]morpholin-4-yl]quinoline-8-carbonitrile;
5-[(*2R*,*6S*)-2-methyl-6-[[4-[6-(4-methylpiperazin-1-yl)pyrimidin-4-yl]piperazin-1-yl]methyl]morpholin-4-yl]quinoline-8-carbonitrile;
5-[(*2S*,*6R*)-2-[[4-[2-(3-aminoazetidin-1-yl)pyrimidin-4-yl]piperazin-1-yl]methyl]-6-methyl-morpholin-4-yl] quinoline-8-carbonitrile;
5-[(*2S*,*6R*)-2-[[4-[2-(3-aminoazetidin-1-yl)pyrimidin-4-yl]piperazin-1-yl]methyl]-6-methyl-morpholin-4-yl] quinoline-8-carbonitrile;
5-[(2*S*,6*R*)-2-[[4-[2-[[(3*S*,4*R*)-4-fluoropyrrolidin-3-yl]amino]pyrimidin-4-yl]piperazin-1-yl]methyl]-6-methyl-morpholin-4-yl]quinoline-8-carbonitrile;
5-[(2*S*,6*R*)-2-[[4-[2-(6-hydroxy-1,4-diazepan-1-yl)pyrimidin-4-yl]piperazin-1-yl]methyl]-6-methyl-morpholin-4-yl]quinoline-8-carbonitrile;

5-[(*2S*,*6R*)-2-[[4-[2-*trans-(*3-amino-4-methoxy-pyrrolidin-1-yl)pyrimidin-4-yl]piperazin-1-yl]methyl]-6-methyl-morpholin-4-yl]quinoline-8-carbonitrile;

5-[(*2S*,*6R*)-2-[[4-[2-(4-amino-1-piperidyl)pyrimidin-4-yl]piperazin-1-yl]methyl]-6-methyl-morpholin-4-yl] quinoline-8-carbonitrile;

5-[(*2S*,*6R*)-2-[[4-[2-(azetidin-3-ylamino)pyrimidin-4-yl]piperazin-1-yl]methyl]-6-methyl-morpholin-4-yl] quinoline-8-carbonitrile;

5-[(2*S*,6*R*)-2-[[4-[2-[(3R)-3-(hydroxymethyl)piperazin-1-yl]pyrimidin-4-yl]piperazin-1-yl]methyl]-6-methyl-morpholin-4-yl]quinoline-8-carbonitrile;

5-[(2*S*,6*R*)-2-[[4-[2-[2-(dimethylamino)ethoxy]pyrimidin-4-yl]piperazin-1-yl]methyl]-6-methyl-morpholin-4-yl]quinoline-8-carbonitrile;

5-[(2*R*,6*S*)-2-methyl-6-[[4-[2-[4-[(2*S*)-pyrrolidine-2-carbonyl]piperazin-1-yl]pyrimidin-4-yl]piperazin-1-yl]methyl]morpholin-4-yl]quinoline-8-carbonitrile;

5-[(2*S*,6*R*)-2-[[4-[2-[4-[2-(dimethylamino)acetyl]piperazin-1-yl]pyrimidin-4-yl]piperazin-1-yl]methyl]-6-methyl-morpholin-4-yl]quinoline-8-carbonitrile;

5-[(2*R*,6*S*)-2-methyl-6-[[4-[6-(4-methylpiperazin-1-yl)-2-pyridyl]piperazin-1-yl]methyl]morpholin-4-yl]quinoline-8-carbonitrile;

5-[(*2S*,*6R*)-2-[[4-[2-[4-(azetidine-2-carbonyl)piperazin-1-yl]pyrimidin-4-yl]piperazin-1-yl]methyl]-6-methyl-morpholin-4-yl]quinoline-8-carbonitrile;

5-[(2*S*,6*R*)-2-[[4-[6-[[(3*S*,4*S*)-4-fluoropyrrolidin-3-yl]amino]-2-pyridyl]piperazin-1-yl]methyl]-6-methyl-morpholin-4-yl]quinoline-8-carbonitrile;

5-[(2*S*,6*R*)-2-[[4-[6-(4-amino-1-piperidyl)-2-pyridyl]piperazin-1-yl]methyl]-6-methyl-morpholin-4-yl] quinoline-8-carbonitrile;

5-[(*2S*,*6R*)-2-[[4-[6-[[(*3S,4R*)-4-fluoropyrrolidin-3-yl]amino]-2-pyridyl]piperazin-1-yl]methyl]-6-methyl-morpholin-4-yl]quinoline-8-carbonitrile;

5-[(2*S*,6*R*)-2-[[4-[6-(4-amino-1-piperidyl)-2-pyridyl]piperazin-1-yl]methyl]-6-methyl-morpholin-4-yl] quinoline-8-carbonitrile;

5-[(*2S*,*6R*)-2-[[4-[6-[[(*3R*,*4R*)-4-methoxypyrrolidin-3-yl]amino]-2-pyridyl]piperazin-1-yl]methyl]-6-methyl-morpholin-4-yl]quinoline-8-carbonitrile;

5-[(*2S*,*6R*)-2-[[4-[6-(3-amino-4-fluoro-pyrrolidin-1-yl)-2-pyridyl]piperazin-1-yl]methyl]-6-methyl-morpholin-4-yl]quinoline-8-carbonitrile;

5-[(*2S*,*6R*)-2-[[4-[6-(6-hydroxy-1,4-diazepan-1-yl)-2-pyridyl]piperazin-1-yl]methyl]-6-methyl-morpholin-4-yl]quinoline-8-carbonitrile;

5-[(*2S*,*6R*)-2-[[4-[6-[(*3R*,*4S*)-3-amino-4-methoxy-pyrrolidin-1-yl]-2-pyridyl]piperazin-1-yl]methyl]-6-methyl-morpholin-4-yl]quinoline-8-carbonitrile;

5-[(*2S*,*6R*)-2-[[4-[6-(3-amino-4-methoxy-pyrrolidin-1-yl)-3-pyridyl]piperazin-1-yl]methyl]-6-methyl-morpholin-4-yl]quinoline-8-carbonitrile;

5-[(*2S*,*6R*)-2-[[4-[6-(3-amino-4-fluoro-pyrrolidin-1-yl)-3-pyridyl]piperazin-1-yl]methyl]-6-methyl-morpholin-4-yl]quinoline-8-carbonitrile;

5-[(*2S*,*6R*)-2-[[4-(5-fluoro-2-piperazin-1-yl-pyrimidin-4-yl)piperazin-1-yl]methyl]-6-methyl-morpholin-4-yl]quinoline-8-carbonitrile;

5-[(*2S*,*6R*)-2-[[4-(5,6-dimethyl-2-piperazin-1-yl-pyrimidin-4-yl)piperazin-1-yl]methyl]-6-methyl-morpholin-4-yl]quinoline-8-carbonitrile;

5-[(*2R*,*6S*)-2-methyl-6-[[4-(6-methyl-2-piperazin-1-yl-pyrimidin-4-yl)piperazin-1-yl]methyl]morpholin-4-yl]quinoline-8-carbonitrile;

5-[(*2S*,*6R*)-2-[[4-(5-methoxy-2-piperazin-1-yl-pyrimidin-4-yl)piperazin-1-yl]methyl]-6-methyl-morpholin-4-yl]quinoline-8-carbonitrile;

5-[(*2R*,*6S*)-2-methyl-6-[[4-(2-piperazin-1-ylquinazolin-4-yl)piperazin-1-yl]methyl]morpholin-4-yl]quinoline-8-carbonitrile;

5-[(2S,6R)-2-[[4-(5-cyano-2-piperazin-1-yl-pyrimidin-4-yl)piperazin-1-yl]methyl]-6-methyl-morpholin-4-yl]quinoline-8-carbonitrile;

5-[(*2S*,*6R*)-2-[[4-(5-cyano-4-piperazin-1-yl-pyrimidin-2-yl)piperazin-1-yl]methyl]-6-methyl-morpholin-4-yl]quinoline-8-carbonitrile;

5-[(*2S*,*6R*)-2-[[4-[2-[(*3R*,*4R*)-3-amino-4-methoxy-pyrrolidin-1-yl]pyrimidin-4-yl]piperazin-1-yl]methyl]-6-methyl-morpholin-4-yl]quinoline-8-carbonitrile;

5-[(*2S*,*6R*)-2-[[4-[2-[(*3S*,*4S*)-3-amino-4-methoxy-pyrrolidin-1-yl]pyrimidin-4-yl]piperazin-1-yl]methyl]-6-methyl-morpholin-4-yl]quinoline-8-carbonitrile;

*5-[(2S, 6R)-2-[[4-[2-[(3S, 4R)-3-amino-4-fluoro-pyrrolidin-1-yl]-6-methyl-pyrimidin-4-*yl]piperazin-1-yl]methyl]-6-methyl-morpholin-4-yl]quinoline-8-carbonitrile;

5-[(2*S*,6*R*)-2-[[4-[2-[(3*R*,4*S*)-3-amino-4-fluoro-pyrrolidin-1-yl]-6-methyl-pyrimidin-4-yl]piperazin-1-yl]methyl]-6-methyl-morpholin-4-yl]quinoline-8-carbonitrile;
5-[(*2S*,*6R*)-2-[[4-[2-(6-amino-1,4-oxazepan-4-yl)-6-methyl-pyrimidin-4-yl]piperazin-1-yl]methyl]-6-methyl-morpholin-4-yl]quinoline-8-carbonitrile;
5-[(*2S*,*6R*)-2-[[4-[2-[(4-cyanopyrrolidin-3-yl)amino]-6-methyl-pyrimidin-4-yl]piperazin-1-yl]methyl]-6-methyl-morpholin-4-yl]quinoline-8-carbonitrile;
5-[(2*R*,6*S*)-2-methyl-6-[[4-[6-methyl-2-[(1*R*,5*R*)-3-oxa-7,9-diazabicyclo[3.3.1]nonan-7-yl]pyrimidin-4-yl]piperazin-1-yl]methyl]morpholin-4-yl]quinoline-8-carbonitrile;
5-[(2*R*,6*S*)-2-methyl-6-[[4-[6-methyl-2-[trans-3-amino-4-methoxy-pyrrolidin-1-yl]pyrimidin-4-yl]piperazin-1-yl]methyl]morpholin-4-yl]quinoline-8-carbonitrile;
5-[(2*R*,6S)-2-methyl-6-[[4-[6-methyl-2-[cis-3-amino-4-methoxy-pyrrolidin-1-yl]pyrimidin-4-yl]piperazin-1-yl]methyl]morpholin-4-yl]quinoline-8-carbonitrile;
5-[(*2R*,*6S*)-2-methyl-6-[[4-[6-methyl-2-(5-oxa-2,8-diazaspiro[3.5]nonan-2-yl)pyrimidin-4-yl]piperazin-1-yl]methyl]morpholin-4-yl]quinoline-8-carbonitrile;
5-[(*2R*,*6S*)-2-methyl-6-[[4-(3-morpholin-2-ylphenyl)piperazin-1-yl]methyl]-morpholin-4-yl]quinoline-8-carbonitrile;
5-[(2*S*,6*R*)-2-[[4-[2-(4-amino-3,3-difluoro-pyrrolidin-1-yl)-6-methyl-pyrimidin-4-yl]piperazin-1-yl]methyl]-6-methyl-morpholin-4-yl]quinoline-8-carbonitrile;
8-[(2*R*,6*S*)-2-methyl-6-[[4-(6-methyl-2-piperazin-1-yl-pyrimidin-4-yl)piperazin-1-yl]methyl]morpholin-4-yl]quinoxaline-5-carbonitrile;
(*2R*,*6S*)-2-methyl-6-[[4-(6-methyl-2-piperazin-1-yl-pyrimidin-4-yl)piperazin-1-yl]methyl]-4-[8-(trifluoromethyl)quinoxalin-5-yl]morpholine;
5-[(*2S*,*6R*)-2-[[4-[2-(6-hydroxy-1,4-diazepan-1-yl)-6-methyl-pyrimidin-4-yl]piperazin-1-yl]methyl]-6-methyl-morpholin-4-yl]quinoline-8-carbonitrile;
3-Fluoro-4-[(2*R*,6*S*)-2-methyl-6-[[4-(6-methyl-2-piperazin-1-yl-pyrimidin-4-yl)piperazin-1-yl]methyl]morpholin-4-yl]pyrazolo[1,5-a]pyridine-7-carbonitrile;
5-[(2*R*,6*S*)-2-methyl-6-[[4-(4-methyl-6-piperazin-1-yl-pyrimidin-2-yl)piperazin-1-yl]methyl]morpholin-4-yl]quinoline-8-carbonitrile;
5-[(*2S*,*6R*)-2-[[4-[2-*trans*-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(2S,6R)-2-[[4-[2-[(3 S,4S)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(2S,6R)-2-[[4-[2-[(3R,4R)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[[(3*R*,4*R*)-4-methoxypyrrolidin-3-yl]amino]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[[(3*R*,4*S)*-4-fluoropyrrolidin-3-yl]amino]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(*3S*,*4R*)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[[(3*S*,4*R*)-4-fluoropyrrolidin-3-yl]amino]-6-methyl-pyrimidin-4-yl]piperazin-1-yl]methyl]-6-methyl-morpholin-4-yl]quinoline-8-carbonitrile;
5-[(2*R*,6*S*)-2-methyl-6-[[4-[6-methyl-2-[*cis*-3-amino-5-fluoro-1-piperidyl]pyrimidin-4-yl]piperazin-1-yl]methyl]morpholin-4-yl]quinoline-8-carbonitrile;
5-[(2*R*,6*S*)-2-methyl-6-[[4-[6-methyl-2-[*trans*-3-amino-5-fluoro-1-piperidyl]pyrimidin-4-yl]piperazin-1-yl]methyl]morpholin-4-yl]quinoline-8-carbonitrile;
5-[(2*S*,6*R*)-2-[[4-[2-[[(3*R*,4*R*)-4-fluoropyrrolidin-3-yl]amino]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(3*S*,5*R*)-3-amino-5-fluoro-1-piperidyl]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(3*S*,4*R*)-4-amino-3-fluoro-1-piperidyl]-6-methyl-pyrimidin-4-yl]piperazin-1-yl]methyl]-6-methyl-morpholin-4-yl]quinoline-8-carbonitrile;
5-[(2*S*,6*R*)-2-[[4-[2-[(3*R*,4*R*)-3-amino-4-fluoro-pyrrolidin-1-yl]-6-methyl-pyrimidin-4-yl]piperazin-1-yl]methyl]-6-methyl-morpholin-4-yl]quinoline-8-carbonitrile;
5-[(2S,6R)-2-[[4-[2-(5-amino-3,3-difluoro-1-piperidyl)-6-methyl-pyrimidin-4-yl]piperazin-1-yl]methyl]-6-methyl-morpholin-4-yl]quinoline-8-carbonitrile;
5-[*(2S,6R)*-2-[[4-[2-[3-(1-hydroxy-1-methyl-ethyl)piperazin-1-yl]-6-methyl-pyrimidin-4-yl]piperazin-1-yl]methyl]-6-methyl-morpholin-4-yl]quinoline-8-carbonitrile;

5-[(*2S*,*6R*)-2-[[4-[2-[(*3R*,*4R*)-4-amino-3-fluoro-1-piperidyl]-6-methyl-pyrimidin-4-yl]piperazin-1-yl]methyl]-6-methyl-morpholin-4-yl]quinoline-8-carbonitrile;
5-[(*2R*,*6S*)-2-methyl-6-[[4-[6-methyl-2-[(*3S*,*4R*)-3-amino-4-fluoro-1-piperidyl]-pyrimidin-4-yl]piperazin-1-yl]methyl]morpholin-4-yl]quinoline-8-carbonitrile;
5-[(*2R*,*6S*)-2-methyl-6-[[4-[6-methyl-2-[(*3R*,*4R*)-3-amino-4-fluoro-1-piperidyl]pyrimidin-4-yl]piperazin-1-yl]methyl]morpholin-4-yl]quinoline-8-carbonitrile;
5-[(*2S*,*6R*)-2-[[4-[2-(azetidin-3-ylamino)-6-methyl-pyrimidin-4-yl]piperazin-1-yl]methyl]-6-methyl-morpholin-4-yl]quinoline-8-carbonitrile;
5-[(*2S*,*6R*)-2-[[4-[2-(4-amino-3,3-difluoro-1-piperidyl)-6-methyl-pyrimidin-4-yl]piperazin-1-yl]methyl]-6-methyl-morpholin-4-yl]quinoline-8-carbonitrile;
5-[(*2S*,*6R*)-2-[[4-[2-(3-aminoazetidin-1-yl)-6-methyl-pyrimidin-4-yl]piperazin-1-yl]methyl]-6-methyl-morpholin-4-yl]quinoline-8-carbonitrile;
5-[(*2S*,*6R*)-2-[[4-[2-[(*3R*,*4S*)-3-amino-4-methoxy-pyrrolidin-1-yl]-6-methyl-pyrimidin-4-yl]piperazin-1-yl]methyl]-6-methyl-morpholin-4-yl]quinoline-8-carbonitrile;
5-[(*2R*,*6S*)-2-methyl-6-[[4-(2-morpholin-2-ylphenyl)piperazin-1-yl]methyl]morpholin-4-yl]quinoline-8-carbonitrile;
5-[*(2S*,*6R*)-2-[[4-[2-[(*3S*,*4S*)-4-amino-3-methoxy-1-piperidyl]-6-methyl-pyrimidin-4-yl]piperazin-1-yl]methyl]-6-methyl-morpholin-4-yl]quinoline-8-carbonitrile;
5-[*(2S*,*6R)*-2-[[4-(6-methoxy-2-piperazin-1-yl-pyrimidin-4-yl)piperazin-1-yl]methyl]-6-methyl-morpholin-4-yl]quinoline-8-carbonitrile;
5-[*(2S*,*6R)*-2-[[4-[2-[(*3S*,*4R*)-3-amino-4-methoxy-pyrrolidin-1-yl]pyrimidin-4-yl]piperazin-1-yl]methyl]-6-methyl-morpholin-4-yl]quinoline-8-carbonitrile;
5-[*(2S*,*6R)*-2-[[4-[2-[(*3R*,*4S*)-3-amino-4-methoxy-pyrrolidin-1-yl]pyrimidin-4-yl]piperazin-1-yl]methyl]-6-methyl-morpholin-4-yl]quinoline-8-carbonitrile;
5-[*(2S*,*6R)*-2-[[4-(5-fluoro-4-piperazin-1-yl-pyrimidin-2-yl)piperazin-1-yl]methyl]-6-methyl-morpholin-4-yl]quinoline-8-carbonitrile;
5-[*(2S*,*6R)*-2-[[4-[2-[(*3S*,*4R*)-3-amino-4-fluoro-pyrrolidin-1-yl]pyrimidin-4-yl]piperazin-1-yl]methyl]-6-methyl-morpholin-4-yl]quinoline-8-carbonitrile;
5-[*(2S*,*6R)*-2-[[4-[2-[*(3R*,*4R*)-3-amino-4-fluoro-pyrrolidin-1-yl]pyrimidin-4-yl]piperazin-1-yl]methyl]-6-methyl-morpholin-4-yl]quinoline-8-carbonitrile;
5-[*(2S*,*6R)*-2-[[4-[4-[(*3S*,*4S*)-3-amino-4-methoxy-pyrrolidin-1-yl]pyrimidin-2-yl]piperazin-1-yl]methyl]-6-methyl-morpholin-4-yl]quinoline-8-carbonitrile;
8-[*(2S*,*6R)*-2-[[4-[2-[*(3R*,*4R*)-3-amino-4-methoxy-pyrrolidin-1-yl]pyrimidin-4-yl]piperazin-1-yl]methyl]-6-methyl-morpholin-4-yl]quinoxaline-5-carbonitrile;
8-[*(2S*,*6R)*-2-[[4-[2-[(*3S*,*4S*)-3-amino-4-methoxy-pyrrolidin-1-yl]pyrimidin-4-yl]piperazin-1-yl]methyl]-6-methyl-morpholin-4-yl]quinoxaline-5-carbonitrile;
5-[*(2S*,*6R)*-2-[[4-[2-[(*3R*,*4S*)-3-amino-4-fluoro-pyrrolidin-1-yl]pyrimidin-4-yl]piperazin-1-yl]methyl]-6-methyl-morpholin-4-yl]quinoline-8-carbonitrile;
5-[(*2R*,*6S*)-2-methyl-6-[[4-(5-methyl-4-piperazin-1-yl-pyrimidin-2-yl)piperazin-1-yl]methyl]morpholin-4-yl]quinoline-8-carbonitrile;
5-[*(2S*,*6R)*-2-[[4-[2-[(4*aR*,7*aR*)-3,4,4a,5,7,7a-hexahydro-2*H*-pyrrolo[3,4-b][1,4]oxazin-6-yl]pyrimidin-4-yl]piperazin-1-yl]methyl]-6-methyl-morpholin-4-yl]quinoline-8-carbonitrile;
8-[*(2S*,*6R)*-2-[[4-[4-[(*3S*,*4S*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile;
8-[*(2S*,*6R)*-2-[[4-[4-[(*3S*,*4S*)-3-amino-4-methoxy-pyrrolidin-1-yl]-6-methyl-pyrimidin-2-yl]piperazin-1-yl]methyl]-6-methyl-morpholin-4-yl]-2,3-dideuterio-quinoxaline-5-carbonitrile;
8-[*(2S*,*6R)*-2-[[4-[2-[(*3S*,*4S*)-3-amino-4-methoxy-pyrrolidin-1-yl]-6-methyl-pyrimidin-4-yl]piperazin-1-yl]methyl]-6-methyl-morpholin-4-yl]quinoxaline-5-carbonitrile;
8-[*(2S*,*6R)*-2-[[4-[2-[(*3S*,*4S*)-3-amino-4-fluoro-pyrrolidin-1-yl]pyrimidin-4-yl]piperazin-1-yl]methyl]-6-methyl-morpholin-4-yl]quinoxaline-5-carbonitrile;
8-[*(2S*,*6R)*-2-[[4-[2-[(*3R*,*4S*)-3-amino-4-fluoro-pyrrolidin-1-yl]pyrimidin-4-yl]piperazin-1-yl]methyl]-6-methyl-morpholin-4-yl]quinoxaline-5-carbonitrile;
5-[*(2S*,*6R)*-2-[[4-[2-(3-amino-4-methoxy-1-piperidyl)pyrimidin-4-yl]piperazin-1-yl]methyl]-6-methyl-morpholin-4-yl]quinoline-8-carbonitrile;
5-[*(2S*,*6R)*-2-[[4-[2-[(*3S*,*4S*)-3-amino-4-fluoro-pyrrolidin-1-yl]pyrimidin-4-yl]piperazin-1-yl]methyl]-6-methyl-morpholin-4-yl]quinoline-8-carbonitrile;
8-[*(2S*,*6R)*-2-[[4-[2-[*(3R*,*4S*)-4-amino-3-fluoro-1 -piperidyl]pyrimidin-4-yl]piperazin-1 - yl]methyl]-6-methyl-morpholin-4-yl]quinoxaline-5-carbonitrile;

8-[*(2S,6R)*-2-[[4-[2-(3-aminoazetidin-1-yl)-6-methyl-pyrimidin-4-yl]piperazin-1-yl]methyl]-6-methyl-morpholin-4-yl]quinoxaline-5-carbonitrile;
8-[*(2S,6R)*-2-[[4-[2-[(*3R*,*4R*)-4-amino-3-fluoro-1-piperidyl]pyrimidin-4-yl]piperazin-1-yl]methyl]-6-methyl-morpholin-4-yl]quinoxaline-5-carbonitrile;
8-[(*2R*,*6S*)-2-methyl-6-[[4-(4-methyl-6-piperazin-1-yl-pyrimidin-2-yl)piperazin-1-yl]methyl]morpholin-4-yl]quinoxaline-5-carbonitrile;
8-[*(2S,6R)*-2-[[4-[4-[(*3R*,*4S*)-3-amino-4-fluoro-pyrrolidin-1-yl]-6-methyl-pyrimidin-2-yl]piperazin-1-yl]methyl]-6-methyl-morpholin-4-yl]quinoxaline-5-carbonitrile;
(*2R*,*6S*)-2-methyl-4-(8-methyl quinoxalin-5-yl)-6-[[4-(4-piperazin-1 -ylpyrimidin-2-yl)piperazin-1-yl]methyl]morpholine;
(*2R*,*6S*)-2-methyl-4-(8-methyl quinoxalin-5-yl)-6-[[4-(2-piperazin-1 -ylpyrimidin-4-yl)piperazin-1-yl]methyl]morpholine;
8-[*(2S,6R)*-2-[[4-[4-(3-aminoazetidin-1-yl)pyrimidin-2-yl]piperazin-1-yl]methyl]-6-methyl-morpholin-4-yl] quinoxaline-5-carbonitrile;
8-[*(2S,6R)*-2-[[4-[4-[*(3R,4R)*-3-amino-4-fluoro-pyrrolidin-1-yl]pyrimidin-2-yl]piperazin-1-yl]methyl]-6-methyl-morpholin-4-yl]quinoxaline-5-carbonitrile;
8-[*(2S,6R)*-2-[[4-[4-[(*3S*,*4R*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile;
8-[*(2S,6R)*-2-[[4-[4-[(*3R*,*4R*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile;
8-[*(2S,6R)*-2-[[4-[4-[(*3R*,*4S*)-3-amino-4-methoxy-pyrrolidin-1-yl]-6-methyl-pyrimidin-2-yl]piperazin-1-yl]methyl]-6-methyl-morpholin-4-yl]quinoxaline-5-carbonitrile;
8-[*(2S,6R)*-2-[[4-[4-(3-aminoazetidin-1-yl)-6-methyl-pyrimidin-2-yl]piperazin-1-yl]methyl]-6-methyl-morpholin-4-yl]quinoxaline-5-carbonitrile;
8-[*(2S,6R)*-2-[[4-[4-(3-aminoazetidin-1-yl)-6-methyl-pyrimidin-2-yl]piperazin-1-yl]methyl]-6-methyl-morpholin-4-yl]-2,3-dideuterio-quinoxaline-5-carbonitrile;
8-[(*2S*,*6R*)-2-[[4-[4-[(3*R*,*4R*)-4-amino-3-hydroxy-3-methyl-pyrrolidin-1-yl]-6-methyl-pyrimidin-2-yl]piperazin-1-yl]methyl]-6-methyl-morpholin-4-yl]quinoxaline-5-carbonitrile;
2-[4-[4-[[*(2S,6R)*-6-methyl-4-(8-methylquinoxalin-5-yl)morpholin-2-yl]methyl]piperazin-1-yl]pyrimidin-2-yl]-5-oxa-2,8-diazaspiro[3.5]nonane;
(*2R*,*6S*)-2-methyl-6-[[4-[2-[(3S)-3-methylpiperazin-1-yl]pyrimidin-4-yl]piperazin-1-yl]methyl]-4-(8-methylquinoxalin-5-yl)morpholine;
(*2R*,*6S*)-2-methyl-6-[[4-[2-[(3*R*)-3-methylpiperazin-1-yl]pyrimidin-4-yl]piperazin-1-yl]methyl]-4-(8-methylquinoxalin-5-yl)morpholine;
(*4aR*,*7aR*)-6-[4-[4-[[*(2S,6R)*-6-methyl-4-(8-methylquinoxalin-5-yl)morpholin-2-yl]methyl]piperazin-1-yl]pyrimidin-2-yl]-3,4,4*a*,*5*,*7*,*7a*-hexahydro-*2H*-pyrrolo[3,4-b][1,4]oxazine;
1-[4-[4-[[*(2S,6R)*-6-methyl-4-(8-methylquinoxalin-5-yl)morpholin-2-yl]methyl]piperazin-1-yl]pyrimidin-2-yl] azetidin-3-amine;
5-[*(2S,6R)*-2-[[4-[4-(3-amino-3-methyl-azetidin-1-yl)-6-methoxy-pyrimidin-2-yl]piperazin-1-yl]methyl]-6-methyl-morpholin-4-yl]-2-deuterio-quinoline-8-carbonitrile;
5-[*(2S,6R)*-2-[[4-[6-(3-amino-3-methyl-azetidin-1-yl)-3-pyridyl]piperazin-1-yl]methyl]-6-methyl-morpholin-4-yl]-2-deuterio-quinoline-8-carbonitrile;
5-[*(2S,6R)*-2-[[4-[6-(3-amino-3-methyl-azetidin-1-yl)-4-methyl-3-pyridyl]piperazin-1-yl]methyl]-6-methyl-morpholin-4-yl]-2-deuterio-quinoline-8-carbonitrile;
5-[*(2S,6R)*-2-[[4-[4-[(*3S*,*4S*)-3-amino-4-methoxy-pyrrolidin-1-yl]-6-methyl-pyrimidin-2-yl]piperazin-1-yl]methyl]-6-methyl-morpholin-4-yl]-2-deuterio-quinoline-8-carbonitrile;
5-[*(2S,6R)*-2-[[4-[4-(3-amino-3-methyl-azetidin-1-yl)-6-methyl-pyrimidin-2-yl]piperazin-1-yl]methyl]-6-methyl-morpholin-4-yl]-2-deuterio-quinoline-8-carbonitrile;
5-[*(2S,6R)*-2-[[4-[4-(3-aminoazetidin-1-yl)-6-methyl-pyrimidin-2-yl]piperazin-1-yl]methyl]-6-methyl-morpholin-4-yl]-2-deuterio-quinoline-8-carbonitrile;
5-[*(2S,6R)*-2-[[4-[6-(3-amino-3-methyl-azetidin-1-yl)-2-methyl-3-pyridyl]piperazin-1-yl]methyl]-6-methyl-morpholin-4-yl]-2-deuterio-quinoline-8-carbonitrile;
5-[*(2S,6R)*-2-[[3-[6-(3-amino-3-methyl-azetidin-1-yl)-3-pyridyl]azetidin-1-yl]methyl]-6-methyl-morpholin-4-yl]-2-deuterio-quinoline-8-carbonitrile;
5-[(2S, 6R)-2-[[4-[5-(3-amino-3-methyl-azetidin-1-yl)-3-methyl-2-pyridyl]piperazin-1-yl]methyl]-6-methyl-morpholin-4-yl]-2-deuterio-quinoline-8-carbonitrile;
5-[*(2S,6R)*-2-[[3-[4-[(*3S*,*4S*)-3-amino-4-methoxy-pyrrolidin-1-yl]phenyl]azetidin-1-yl]methyl]-6-methyl-morpholin-4-yl]-2-deuterio-quinoline-8-carbonitrile;

5-[*(2S,6R)*-2-[[3-[4-(3-amino-3-methyl-azetidin-1-yl)phenyl]azetidin-1-yl]methyl]-6-methyl-morpholin-4-yl]-2-deuterio-quinoline-8-carbonitrile;
*5-[(2S, 6R)-2-[[4-[5-[(3R, 4R)-3-amino-4-methoxy-pyrrolidin-1-yl]-3-methyl-2*-pyridyl]piperazin-1-yl]methyl]-6-methyl-morpholin-4-yl]-2-deuterio-quinoline-8-carbonitrile;
5-[(*2S*,*6R*)-2-[[4-[6-[(*3R*,*4R*)-3-amino-4-methoxy-pyrrolidin-1-yl]-3-pyridyl]piperazin-1-yl]methyl]-6-methyl-morpholin-4-yl]-2-deuterio-quinoline-8-carbonitrile;
5-[*(2S*,*6R*)-2-[[4-[4-[(*3R*,*4R*)-3-amino-4-methoxy-pyrrolidin-1-yl]-6-methyl-pyrimidin-2-yl]piperazin-1-yl]methyl]-6-methyl-morpholin-4-yl]-2-deuterio-quinoline-8-carbonitrile;
5-[*(2S*,*6R*)-2-[[3-[6-(3-amino-3-methyl-azetidin-1-yl)-4-methyl-3-pyridyl]azetidin-1-yl]methyl]-6-methyl-morpholin-4-yl]-2-deuterio-quinoline-8-carbonitrile;
5-[*(2S*,*6R*)-2-[[3-[6-[(*3R*,*4R*)-3-amino-4-methoxy-pyrrolidin-1-yl]-4-methyl-3-pyridyl]azetidin-1-yl]methyl]-6-methyl-morpholin-4-yl]-2-deuterio-quinoline-8-carbonitrile;
5-[*(2S*,*6R)*-2-[[4-[5-(3-amino-3-methyl-azetidin-1-yl)-6-methyl-2-pyridyl]piperazin-1-yl]methyl]-6-methyl-morpholin-4-yl]-2-deuterio-quinoline-8-carbonitrile;
4-[*(2S*,*6R)*-2-[[4-[5-(3-amino-3-methyl-azetidin-1-yl)-3-methyl-2-pyridyl]piperazin-1-yl]methyl]-6-methyl-morpholin-4-yl]-3-fluoro-pyrazolo[1,5-a]pyridine-7-carbonitrile;
5-[*(2S*,*6R)*-2-[[3-[6-[(*3R*,*4R*)-3-amino-4-methoxy-pyrrolidin-1-yl]-3-pyridyl]azetidin-1-yl]methyl]-6-methyl-morpholin-4-yl]-2-deuterio-quinoline-8-carbonitrile;
5-[*(2S*,*6R)*-2-[[4-[6-[(*3S*,*4S*)-3-amino-4-fluoro-pyrrolidin-1-yl]-3-pyridyl]piperazin-1-yl]methyl]-6-methyl-morpholin-4-yl]-2-deuterio-quinoline-8-carbonitrile;
2-deuterio-5-[*(2S*,*6R)*-2-[[4-[6-[(1S,4S)-2,5-diazabicyclo[2.2.1]heptan-2-yl]-3-pyridyl]piperazin-1-yl]methyl]-6-methyl-morpholin-4-yl]quinoline-8-carbonitrile;
2-deuterio-5-[(*2R*,*6S*)-2-methyl-6-[[4-[6-(5-oxa-2,8-diazaspiro[3.5]nonan-2-yl)-3-pyridyl]piperazin-1-yl]methyl]morpholin-4-yl]quinoline-8-carbonitrile;
2-deuterio-5-[*(2S*,*6R)*-2-[[4-[6-[(1R,4R)-2,5-diazabicyclo[2.2.1]heptan-2-yl]-3-pyridyl]piperazin-1-yl]methyl]-6-methyl-morpholin-4-yl]quinoline-8-carbonitrile;
4-[*(2S*,*6R)*-2-[[4-[6-(3-amino-3-methyl-azetidin-1-yl)-3-pyridyl]piperazin-1-yl]methyl]-6-methyl-morpholin-4-yl]-3-fluoro-pyrazolo[1,5-a]pyridine-7-carbonitrile;
5-[*(2S*,*6R)*-2-[[4-[6-(3-amino-3-methyl-azetidin-1-yl)-2-pyridyl]piperazin-1-yl]methyl]-6-methyl-morpholin-4-yl]-2-deuterio-quinoline-8-carbonitrile;
5-[(*2S*,*6R*)-2-[[3-[4-(3-amino-3-methyl-azetidin-1-yl)phenyl]-3-hydroxy-azetidin-1-yl]methyl]-6-methyl-morpholin-4-yl]-2-deuterio-quinoline-8-carbonitrile;
4-[*(2S*,*6R)*-2-[[3-[6-(3-amino-3-methyl-azetidin-1-yl)-3-pyridyl]azetidin-1-yl]methyl]-6-methyl-morpholin-4-yl]pyrazolo[1,5-a]pyridine-7-carbonitrile;
4-[(*2S*,*6R*)-2-[[3-[6-(3-amino-3-methyl-azetidin-1-yl)-3-pyridyl]azetidin-1-yl]methyl]-6-methyl-morpholin-4-yl]-3-fluoro-pyrazolo[1,5-a]pyridine-7-carbonitrile;
5-[(*2S*,*6R*)-2-[[3-[6-(3-amino-3-methyl-azetidin-1-yl)-2-pyridyl]azetidin-1-yl]methyl]-6-methyl-morpholin-4-yl]-2-deuterio-quinoline-8-carbonitrile;
4-[(*2S*,*6R*)-2-[[3-[4-(3-amino-3-methyl-azetidin-1-yl)phenyl]azetidin-1-yl]methyl]-6-methyl-morpholin-4-yl]-3-fluoro-pyrazolo[1,5-a]pyridine-7-carbonitrile;
2-deuterio-5-[(*2R*,*6S*)-2-methyl-6-[[4-[6-(6-methyl-2,6-diazaspiro[3.3]heptan-2-yl)-3-pyridyl]piperazin-1-yl]methyl]morpholin-4-yl]quinoline-8-carbonitrile;
2-deuterio-5-[(*2S*,*6R*)-2-[[4-[6-[[(*3S*,*4R*)-4-fluoropyrrolidin-3-yl]amino]-3-pyridyl]piperazin-1-yl]methyl]-6-methyl-morpholin-4-yl]quinoline-8-carbonitrile;
4-[(*2S*,*6R*)-2-[[3-[4-(3-amino-3-methyl-azetidin-1-yl)phenyl]azetidin-1-yl]methyl]-6-methyl-morpholin-4-yl]pyrazolo[1,5-a]pyridine-7-carbonitrile;
4-[*(2S*,*6R)*-2-[[3-[6-[(*3R*,*4R*)-3-amino-4-methoxy-pyrrolidin-1-yl]-3-pyridyl]azetidin-1-yl]methyl]-6-methyl-morpholin-4-yl]-3-fluoro-pyrazolo[1,5-a]pyridine-7-carbonitrile;
4-[*(2S*,*6R)*-2-[[3-[6-[(1S,4S)-2,5-diazabicyclo[2.2.1]heptan-2-yl]-3-pyridyl]azetidin-1-yl]methyl]-6-methyl-morpholin-4-yl]-3-fluoro-pyrazolo[1,5-a]pyridine-7-carbonitrile;
4-[*(2S*,*6R)*-2-[[4-[6-[(*3R*,*4R*)-3-amino-4-methoxy-pyrrolidin-1-yl]-3-pyridyl]piperazin-1-yl]methyl]-6-methyl-morpholin-4-yl]-3-fluoro-pyrazolo[1,5-a]pyridine-7-carbonitrile;
3-fluoro-4-[(*2S*,*6R*)-2-[[4-[6-[[(*3S*,*4R*)-4-fluoropyrrolidin-3-yl]amino]-3-pyridyl]piperazin-1-yl]methyl]-6-methyl-morpholin-4-yl]pyrazolo[1,5-a]pyridine-7-carbonitrile;
4-[*(2S*,*6R)*-2-[[4-[6-[(*4aR*,*7aR*)-3,4,4*a*,5,7,7*a*-hexahydro-2*H*-pyrrolo[3,4-b][1,4]oxazin-6-yl]-3-pyridyl]piperazin-1-yl]methyl]-6-methyl-morpholin-4-yl]-3-fluoro-pyrazolo[1,5-a]pyridine-7-carbonitrile;
3-fluoro-4-[(*2R*,*6S*)-2-methyl-6-[[4-[6-(5-oxa-2,8-diazaspiro[3.5]nonan-2-yl)-3-pyridyl]piperazin-1-yl]methyl]morpholin-4-yl]pyrazolo[1,5-a]pyridine-7-carbonitrile;

3-fluoro-4-[(*2R*,*6S*)-2-methyl-6-[[4-[6-(6-methyl-2,6-diazaspiro[3.3]heptan-2-yl)-3-pyridyl]piperazin-1-yl]methyl]morpholin-4-yl]pyrazolo[1,5-a]pyridine-7-carbonitrile;
1-methyl-4-[(*2R*,*6S*)-2-methyl-6-[[3-(4-piperazin-1-ylphenyl)azetidin-1-yl]methyl]morpholin-4-yl]-1,8-naphthyridin-2-one;
4-[*(2S,6R)*-2-[[3-[4-[*(3R,4R)*-3-amino-4-methoxy-pyrrolidin-1-yl]phenyl]azetidin-1-yl]methyl]-6-methyl-morpholin-4-yl]-1-methyl-1, 8-naphthyridin-2-one;
1-methyl-4-[(*2R*,*6S*)-2-methyl-6-[[3-[4-(5-oxa-2,8-diazaspiro[3.5]nonan-2-yl)phenyl]azetidin-1-yl]methyl]morpholin-4-yl]-1,8-naphthyridin-2-one;
4-[*(2S,6R)*-2-[[3-[4-[(*3R*,*4R*)-3-amino-4-fluoro-pyrrolidin-1-yl]phenyl]azetidin-1-yl]methyl]-6-methyl-morpholin-4-yl]-1-methyl-1, 8-naphthyridin-2-one;
4-[*(2S,6R)*-2-[[4-[6-[(*3R*,*4R*)-4-amino-3-fluoro-1-piperidyl]-3-pyridyl]piperazin-1-yl]methyl]-6-methyl-morpholin-4-yl]-3-fluoro-pyrazolo[1,5-a]pyridine-7-carbonitrile;
4-[*(2S,6R)*-2-[[3-[4-(3-amino-3-methyl-azetidin-1-yl)phenyl]azetidin-1-yl]methyl]-6-methyl-morpholin-4-yl]-1-methyl-1,8-naphthyridin-2-one;
3-fluoro-4-[(*2R*,*6S*)-2-methyl-6-[[4-[4-[(*2R*)-morpholin-2-yl]phenyl]piperazin-1-yl]methyl]morpholin-4-yl]pyrazolo[1,5-a]pyridine-7-carbonitrile;
3-fluoro-4-[(*2R*,*6S*)-2-methyl-6-[[4-[6-(5-oxa-2,8-diazaspiro[3.5]nonan-8-yl)-3-pyridyl]piperazin-1-yl]methyl]morpholin-4-yl]pyrazolo[1,5-a]pyridine-7-carbonitrile;
4-[(2*S*,6*R*)-2-[[4-[6-(1,6-diazaspiro[3.3]heptan-6-yl)-3-pyridyl]piperazin-1-yl]methyl]-6-methyl-morpholin-4-yl]-3-fluoro-pyrazolo[1,5-a]pyridine-7-carbonitrile;
1-methyl-4-[(2*R*,*6S*)-2-methyl-6-[[3-[4-(6-methyl-2,6-diazaspiro[3.3]heptan-2-yl)phenyl]azetidin-1-yl]methyl]morpholin-4-yl]-1,8-naphthyridin-2-one;
4-[(2*S*,*6R*)-2-[[3-[4-[(4*aR*,*7aR*)-3,4,4*a*,5,7,7*a*-hexahydro-2*H*-pyrrolo[3,4-*b*][1,4]oxazin-6-yl]phenyl]azetidin-1-yl]methyl]-6-methyl-morpholin-4-yl]-1-methyl-1,8-naphthyridin-2-one;
3-fluoro-4-[(2*S*,*6R*)-2-[[4-[2-fluoro-4-[(2*S*)-morpholin-2-yl]phenyl]piperazin-1-yl]methyl]-6-methyl-morpholin-4-yl]pyrazolo[1,5-a]pyridine-7-carbonitrile;
3-fluoro-4-[(2*S*,*6R*)-2-[[4-[3-fluoro-4-[(2*R*)-morpholin-2-yl]phenyl]piperazin-1-yl]methyl]-6-methyl-morpholin-4-yl]pyrazolo[1,5-a]pyridine-7-carbonitrile;
3-fluoro-4-[(*2R*,*6S*)-2-methyl-6-[[4-[4-(2-methylmorpholin-2-yl)phenyl]piperazin-1-yl]methyl]morpholin-4-yl]pyrazolo[1,5-a]pyridine-7-carbonitrile; and
3-fluoro-4-[(2*R*,6*S*)-2-methyl-6-[[4-[4-[[(2*R*)-morpholin-2-yl]methyl]phenyl]piperazin-1-yl]methyl]morpholin-4-yl]pyrazolo[1,5-a]pyridine-7-carbonitrile;
or a pharmaceutically acceptable salt thereof.

**20.** A process for the preparation of a compound according to any one of claims 1 to 19 comprising any of the following steps:

a) the substitution of compound of formula (IV),

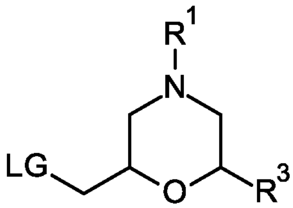

(IV),

with compound of formula (VIII),

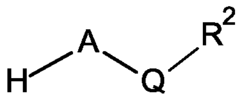

(VIII),

in the presence of a base;
b) the coupling of compound of formula (XIII),

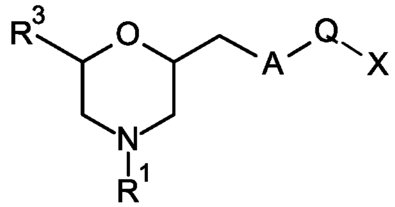

(XIII),

with compound of formula (VI) in the presence of a base or under Buchwald-Hartwig amination condition;

wherein the base in step a) and b) is selected from $K_2CO_3$, DIPEA and $Cs_2CO_3$; the Buchwald-Hartwig amination condition in step b) includes a catalyst and a base, wherein the catalyst is selected from tBuXPhos Pd G3, RuPhos Pd G2, BrettPhos Pd G3, XPhos Pd G3, $Pd_2(dba)_3$/BINAP and $Pd_2(dba)_3$/XantPhos; the base is $Cs_2CO_3$ or *t*-BuONa; LG is a leaving group; $R^1$, $R^2$, $R^3$, A, Q and X are defined as in any one of claims 1 to 18.

**21.** A compound or pharmaceutically acceptable salt according to any one of claims 1 to 19 for use as therapeutically active substance.

**22.** A pharmaceutical composition comprising a compound in accordance with any one of claims 1 to 19 and a therapeutically inert carrier.

**23.** A compound or pharmaceutically acceptable salt, enantiomer or diastereomer according to any one of claims 1 to 19 for use in the treatment or prophylaxis of systemic lupus erythematosus or lupus nephritis.

## Patentansprüche

**1.** Verbindung der Formel (I)

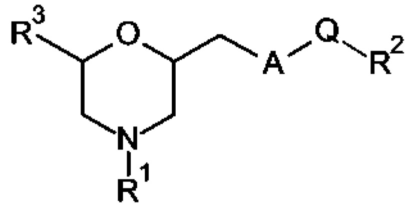

(I),

wobei

R1

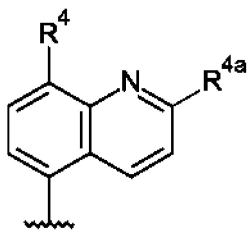,
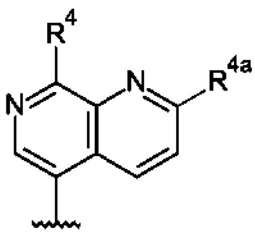,
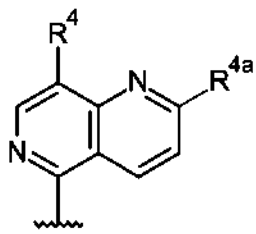,
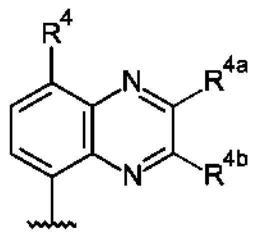,

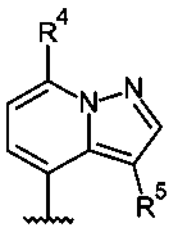, 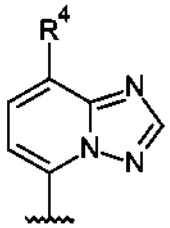, 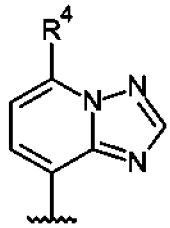, 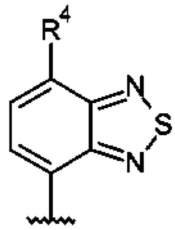, 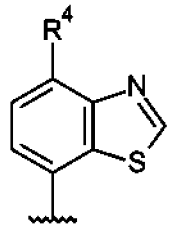,

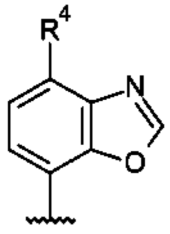, 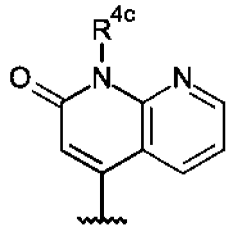 oder 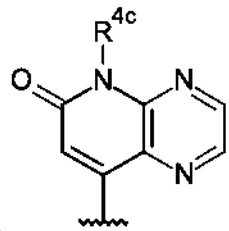

ist; wobei $R^4$ $C_{1-6}$-Alkyl, $C_{1-6}$-Alkoxy, Halogen-$C_{1-6}$-alkyl, Halogen, Nitro oder Cyano ist; $R^{4a}$ H oder Deuterium ist; $R^{4b}$ H, Deuterium oder $C_{1-6}$-Alkyl ist; $R^{4c}$ $C_{1-6}$-Alkyl oder $C_{3-7}$-Cycloalkyl ist; $R^5$ H oder Halogen ist;
$R^2$ (($C_{1-6}$-Alkyl)$_2$-amino)$C_{1-6}$-alkoxy;

($C_{1-6}$-Alkoxypyrrolidinyl)amino;
(Cyanopyrrolidinyl)amino;
mit Hydroxy substituiertes 1,4-Diazepanyl;
1,6-Diazaspiro[3.3]heptanyl;
2,5-Diazabicyclo[2.2.1]heptanyl;
mit $C_{1-6}$-Alkyl substituiertes 2,6-Diazaspiro[3.3]heptanyl;
3,4,4a,5,7,7a-Hexahydro-2H-pyrrolo[3,4-b][1,4]oxazinyl;
3-Oxa-7,9-diazabicyclo[3.3.1]nonanyl;
5-Oxa-2,8-diazaspiro[3.5]nonanyl,
Amino-$C_{1-6}$-alkyl;
Aminooxazepanyl;
Azetidinyl, das einmal oder zweimal mit Substituenten substituiert ist, die unabhängig voneinander aus Amino und $C_{1-6}$-Alkyl ausgewählt sind;
Azetidinylamino;
Halogenpyrrolidinylamino;
Morpholinyl, das unsubstituiert oder mit $C_{1-6}$-Alkyl substituiert ist;
Morpholinyl-$C_{1-6}$-alkyl;
Piperazinyl, das unsubstituiert oder mit $C_{1-6}$-Alkyl, Hydroxy-$C_{1-6}$-alkyl, Pyrrolidinylcarbonyl, (($C_{1-6}$-Alkyl)$_2$-amino)$C_{1-6}$-alkylcarbonyl oder Azetidinylcarbonyl substituiert ist;
Piperidinyl, das unsubstituiert oder einmal oder zweimal mit Substituenten substituiert ist, die unabhängig voneinander aus Amino, Halogen und $C_{1-6}$-Alkoxy ausgewählt sind; oder
Pyrrolidinyl, das einmal, zweimal oder dreimal mit Substituenten substituiert ist, die unabhängig voneinander aus Amino, Halogen, Hydroxy, $C_{1-6}$-Alkyl und $C_{1-6}$-Alkoxy ausgewählt sind, ist;

$R^3$ $C_{1-6}$-Alkyl ist;
A Azetidinyl, Hydroxyazetidinyl, Piperazinyl, Pyrrolidinyl, Piperidinyl oder Cyanopiperidinyl ist;
Q Phenyl, das unsubstituiert oder mit Halogen substituiert ist;

Pyrazinyl;
Pyridazinyl;
Pyridinyl, das unsubstituiert oder mit $C_{1-6}$-Alkyl substituiert ist;
Pyrimidinyl, das unsubstituiert oder einmal oder zweimal mit Substituenten substituiert ist, die unabhängig voneinander aus Halogen, $C_{1-6}$-Alkyl, $C_{1-6}$-Alkoxy und Cyano ausgewählt sind; oder

Chinazolinyl ist;

oder ein pharmazeutisch unbedenkliches Salz davon.

**2.** Verbindung nach Anspruch 1 der Formel (Ia)

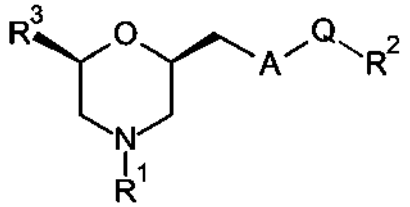

(Ia),

wobei

R1

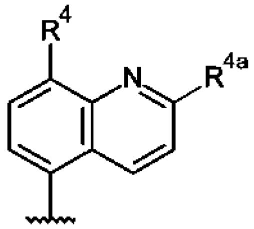
,
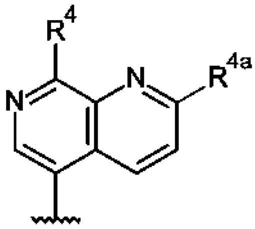
,
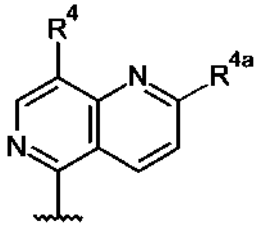
,
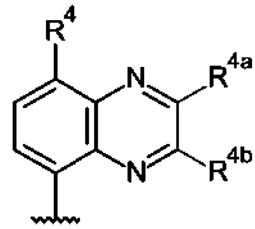
,

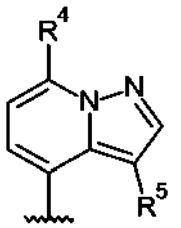
,
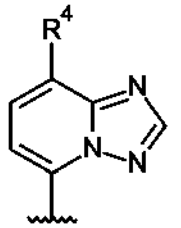
,
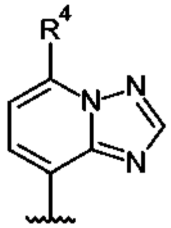
,
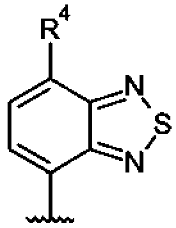
,
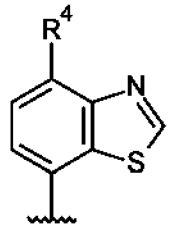
,

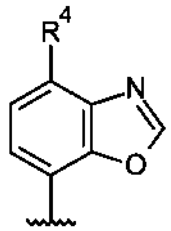
,
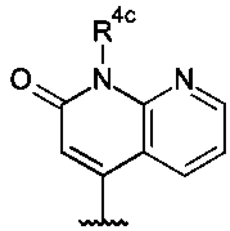
oder
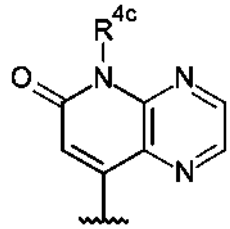

ist; wobei $R^4$ $C_{1-6}$-Alkyl, $C_{1-6}$-Alkoxy, Halogen-$C_{1-6}$-alkyl, Halogen, Nitro oder Cyano ist; $R^{4a}$ H oder Deuterium ist; $R^{4b}$ H, Deuterium oder $C_{1-6}$-Alkyl ist; $R^{4c}$ $C_{1-6}$-Alkyl oder $C_{3-7}$-Cycloalkyl ist; $R^5$ H oder Halogen ist;
$R^2$ (($C_{1-6}$-Alkyl)$_2$-amino)$C_{1-6}$-alkoxy;

($C_{1-6}$-Alkoxypyrrolidinyl)amino;
(Cyanopyrrolidinyl)amino;
mit Hydroxy substituiertes 1,4-Diazepanyl;
1,6-Diazaspiro[3.3]heptanyl;
2,5-Diazabicyclo[2.2.1]heptanyl;
mit $C_{1-6}$-Alkyl substituiertes 2,6-Diazaspiro[3.3]heptanyl;
3,4,4a,5,7,7a-Hexahydro-2H-pyrrolo[3,4-b][1,4]oxazinyl;
3-Oxa-7,9-diazabicyclo[3.3.1]nonanyl;
5-Oxa-2,8-diazaspiro[3.5]nonanyl,

Amino-$C_{1-6}$-alkyl;
Aminooxazepanyl;
Azetidinyl, das einmal oder zweimal mit Substituenten substituiert ist, die unabhängig voneinander aus Amino und $C_{1-6}$-Alkyl ausgewählt sind;
Azetidinylamino;
Halogenpyrrolidinylamino;
Morpholinyl, das unsubstituiert oder mit $C_{1-6}$-Alkyl substituiert ist;
Morpholinyl-$C_{1-6}$-alkyl;
Piperazinyl, das unsubstituiert oder mit $C_{1-6}$-Alkyl, Hydroxy-$C_{1-6}$-alkyl, Pyrrolidinylcarbonyl, (($C_{1-6}$-Alkyl)$_2$-amino)$C_{1-6}$-alkylcarbonyl oder Azetidinylcarbonyl substituiert ist;
Piperidinyl, das unsubstituiert oder einmal oder zweimal mit Substituenten substituiert ist, die unabhängig voneinander aus Amino, Halogen und $C_{1-6}$-Alkoxy ausgewählt sind; oder
Pyrrolidinyl, das einmal, zweimal oder dreimal mit Substituenten substituiert ist, die unabhängig voneinander aus Amino, Halogen, Hydroxy, $C_{1-6}$-Alkyl und $C_{1-6}$-Alkoxy ausgewählt sind, ist;

$R^3$ $C_{1-6}$-Alkyl ist;
A Azetidinyl, Hydroxyazetidinyl, Piperazinyl, Pyrrolidinyl, Piperidinyl oder Cyanopiperidinyl ist;
Q Phenyl, das unsubstituiert oder mit Halogen substituiert ist;

Pyrazinyl;
Pyridazinyl;
Pyridinyl, das unsubstituiert oder mit $C_{1-6}$-Alkyl substituiert ist;
Pyrimidinyl, das unsubstituiert oder einmal oder zweimal mit Substituenten substituiert ist, die unabhängig voneinander aus Halogen, $C_{1-6}$-Alkyl, $C_{1-6}$-Alkoxy und Cyano ausgewählt sind; oder
Chinazolinyl ist;

oder ein pharmazeutisch unbedenkliches Salz davon.

**3.** Verbindung nach Anspruch 1 oder 2 oder ein pharmazeutisch unbedenkliches Salz davon, wobei R1

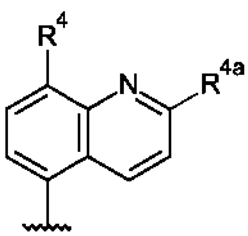 , 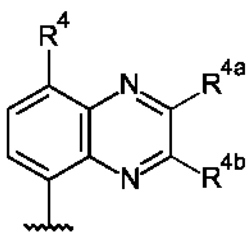 , 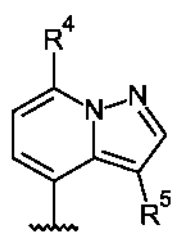 oder 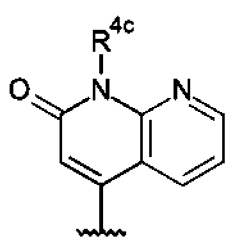

ist; wobei $R^4$ $C_{1-6}$-Alkyl, Halogen-$C_{1-6}$-alkyl oder Cyano ist; $R^{4a}$ H oder Deuterium ist; $R^{4b}$ H oder Deuterium ist; $R^{4c}$ $C_{1-6}$-Alkyl ist; $R^5$ H oder Halogen ist.

**4.** Verbindung nach Anspruch 3 oder ein pharmazeutisch unbedenkliches Salz davon, wobei $R^4$ Methyl, Trifluormethyl oder Cyano ist; $R^{4a}$ H oder Deuterium ist; $R^{4b}$ H oder Deuterium ist; $R^{4c}$ Methyl ist; $R^5$ H oder Fluor ist.

**5.** Verbindung nach Anspruch 3 oder ein pharmazeutisch unbedenkliches Salz davon, wobei $R^2$ ((($C_{1-6}$-Alkyl)$_2$-amino)$C_{1-6}$-alkylcarbonyl)piperazinyl; (($C_{1-6}$-Alkyl)$_2$-amino)$C_{1-6}$-alkoxy; (Azetidinylcarbonyl)piperazinyl; ($C_{1-6}$-Alkoxypyrrolidinyl)amino; ($C_{1-6}$-Alkyl)morpholinyl; (Cyanopyrrolidinyl)amino; (Hydroxy-$C_{1-6}$-alkyl)piperazinyl; (Pyrrolidinylcarbonyl)piperazinyl; 1,6-Diazaspiro[3.3]heptanyl; 2,5-Diazabicyclo[2.2.1]heptanyl; 3,4,4a,5,7,7a-Hexahydro-2H-pyrrolo[3,4-b][1,4]oxazinyl, 3-Oxa-7,9-diazabicyclo[3.3.1]nonanyl; 5-Oxa-2,8-diazaspiro[3.5]nonanyl; Amino($C_{1-6}$-alkoxy)piperidinyl; Amino($C_{1-6}$-alkoxy)pyrrolidinyl; Amino($C_{1-6}$-alkyl)azetidinyl, Amino(hydroxy)($C_{1-6}$-alkyl)pyrrolidinyl; Aminoazetidinyl; Amino-$C_{1-6}$-alkyl; Aminohalogenpiperidinyl; Aminohalogenpyrrolidinyl; Aminooxazepanyl; Aminopiperidinyl; Azetidinylamino; $C_{1-6}$-Alkyl-2,6-diazaspiro[3.3]heptanyl; $C_{1-6}$-Alkylpiperazinyl; Halogenpyrrolidinylamino; Hydroxy-1,4-diazepanyl; Morpholinyl; Morpholinyl-$C_{1-6}$-alkyl; Piperazinyl oder Piperidinyl ist.

**6.** Verbindung nach Anspruch 5 oder ein pharmazeutisch unbedenkliches Salz davon, wobei $R^2$ (1-Hydroxy-1-methy-

lethyl)piperazin-1-yl; (2-Pyrrolidinylcarbonyl)piperazin-1-yl; (4-Cyanopyrrolidin-3-yl)amino; (4-Fluorpyrrolidin-3-yl)amino; (4-Methoxypyrrolidin-3-yl)amino; (Dimethylamino)ethoxy; 1,6-Diazaspiro[3.3]heptan-6-yl; 2,5-Diazabicyclo[2.2.1]heptan-2-yl; 2-Methylmorpholin-2-yl; 2-Morpholin-2-yl; 3-(Hydroxymethyl)piperazin-1-yl; 3,4,4a,5,7,7a-Hexahydro-2H-pyrrolo[3,4-b][1,4]oxazin-6-yl, 3-Amino-3-methylazetidin-1-yl; 3-Amino-4-fluor-1-piperidinyl; 3-Amino-4-fluorpyrrolidin-1-yl; 3-Amino-4-methoxy-1-piperidinyl; 3-Amino-4-methoxypyrrolidin-1-yl; 3-Amino-5-fluor-1-piperidinyl; 3-Aminoazetidin-1-yl; 3-Methylpiperazin-1-yl; 3-Oxa-7,9-diazabicyclo[3.3.1]nonan-7-yl; 4-(Azetidinyl-2-carbonyl)piperazin-1-yl; 4-[2-(Dimethylamino)acetyl]piperazin-1-yl; 4-Amino-1-piperidinyl; 4-Amino-3,3-difluor-1-piperidinyl; 4-Amino-3,3-difluorpyrrolidin-1-yl; 4-Amino-3-fluor-1-piperidinyl; 4-Amino-3-hydroxy-3-methylpyrrolidin-1-yl; 4-Amino-3-methoxy-1-piperidinyl; 4-Methylpiperazin-1-yl; 4-Piperidinyl; 5-Amino-3,3-difluor-1-piperidinyl; 5-Oxa-2,8-diazaspiro[3.5]nonan-2-yl; 5-Oxa-2,8-diazaspiro[3.5]nonan-8-yl; 6-Amino-1,4-oxazepan-4-yl; 6-Hydroxy-1,4-diazepan-1-yl; 6-Methyl-2,6-diazaspiro[3.3]heptan-2-yl; Aminomethyl; Azetidin-3-ylamino; Morpholin-2-yl; Morpholin-2-ylmethyl oder Piperazin-1-yl ist.

**7.** Verbindung nach Anspruch 6 oder ein pharmazeutisch unbedenkliches Salz davon, wobei A

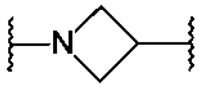,
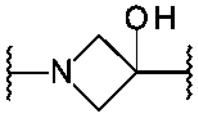,
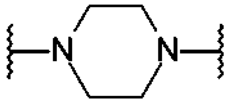,
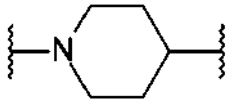,

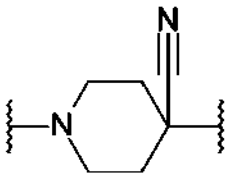
oder
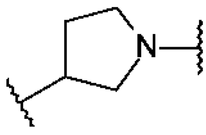

ist.

**8.** Verbindung nach Anspruch 7 oder ein pharmazeutisch unbedenkliches Salz davon, wobei Q Phenyl, Fluorphenyl, Pyrazinyl, Pyridazinyl, Pyridinyl, Methylpyridinyl, Pyrimidinyl, Cyanopyrimidinyl, Fluorpyrimidinyl, Methoxypyrimidinyl, Methylpyrimidinyl, Dimethylpyrimidinyl oder Chinazolinyl ist.

**9.** Verbindung nach Anspruch 8 oder ein pharmazeutisch unbedenkliches Salz davon, wobei $R^3$ Methyl ist.

**10.** Verbindung nach Anspruch 1 oder 2 oder ein pharmazeutisch unbedenkliches Salz davon, wobei
R1

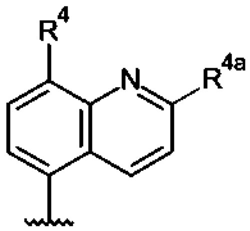,
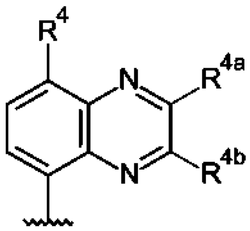
oder
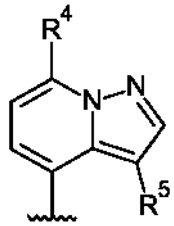

ist; wobei $R^4$ $C_{1-6}$-Alkyl, Halogen-$C_{1-6}$-alkyl oder Cyano ist; $R^{4a}$ und $R^{4b}$ gleichzeitig H oder Deuterium sind; $R^5$ H oder Halogen ist.

**11.** Verbindung nach Anspruch 10 oder ein pharmazeutisch unbedenkliches Salz davon, wobei $R^4$ Methyl, Trifluormethyl oder Cyano ist; $R^{4a}$ und $R^{4b}$ gleichzeitig H oder Deuterium sind; $R^5$ H oder Fluor ist.

**12.** Verbindung nach Anspruch 10 oder ein pharmazeutisch unbedenkliches Salz davon, wobei $R^2$ 3,4,4a,5,7,7a-Hexahydro-2H-pyrrolo[3,4-b][1,4]oxazinyl; 5-Oxa-2,8-diazaspiro[3.5]nonanyl; Amino-($C_{1-6}$-alkoxy)pyrrolidinyl; Ami-

no-($C_{1-6}$-alkyl)azetidinyl, Aminoazetidinyl; Aminopiperidinyl; $C_1$-$_6$-Alkyl-2,6-diazaspiro[3.3]heptanyl; Halogenpyrrolidinylamino; Morpholinyl; Morpholinyl-$C_{1-6}$-alkyl oder Piperazinyl ist.

**13.** Verbindung nach Anspruch 12 oder ein pharmazeutisch unbedenkliches Salz davon, wobei $R^2$ (4-Fluorpyrrolidin-3-yl)amino; 3,4,4a,5,7,7a-Hexahydro-2H-pyrrolo[3,4-b][1,4]oxazin-6-yl; 3-Amino-3-methylazetidin-1-yl; 3-Amino-4-methoxypyrrolidin-1-yl; 3-Aminoazetidin-1-yl; 4-Amino-1-piperidinyl; 5-Oxa-2,8-diazaspiro[3.5]nonan-2-yl; 6-Methyl-2,6-diazaspiro[3.3]heptan-2-yl; Morpholin-2-yl; Morpholin-2-ylmethyl oder Piperazin-1-yl ist.

**14.** Verbindung nach Anspruch 12 oder ein pharmazeutisch unbedenkliches Salz davon, wobei A

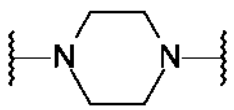

ist.

**15.** Verbindung nach Anspruch 14 oder ein pharmazeutisch unbedenkliches Salz davon, wobei Q Phenyl, Pyridinyl, Pyrimidinyl oder $C_{1-6}$-Alkylpyrimidinyl ist.

**16.** Verbindung nach Anspruch 15 oder ein pharmazeutisch unbedenkliches Salz davon, wobei Q Phenyl, Pyridinyl, Pyrimidinyl oder Methylpyrimidinyl ist.

**17.** Verbindung nach Anspruch 3, wobei

R1

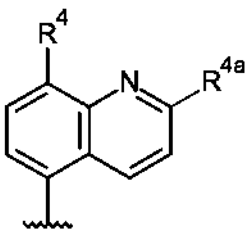 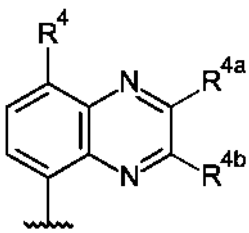 , oder 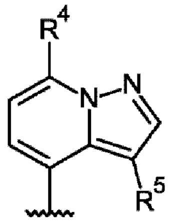

ist; wobei $R^4$ $C_{1-6}$-Alkyl, Halogen-$C_{1-6}$-alkyl oder Cyano ist; $R^{4a}$ und $R^{4b}$ gleichzeitig H oder Deuterium sind; $R^5$ H oder Halogen ist;
$R^2$ 3,4,4a,5,7,7a-Hexahydro-2H-pyrrolo[3,4-b][1,4]oxazinyl; 5-Oxa-2,8-diazaspiro[3.5]nonanyl; Amino($C_{1-6}$-alkoxy)pyrrolidinyl; Amino-($C_{1-6}$-alkyl)azetidinyl; Aminoazetidinyl; Aminopiperidinyl; $C_{1-6}$-Akyl-2,6-diazaspiro[3.3]heptanyl; Halogenpyrrolidinylamino; Morpholinyl; Morpholinyl-$C_{1-6}$-alkyl oder Piperazinyl ist;
$R^3$ $C_{1-6}$-Alkyl ist;
A

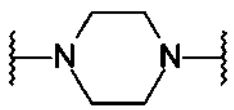

ist;
Q Phenyl, Pyridinyl, Pyrimidinyl oder $C_{1-6}$-Alkylpyrimidinyl ist;
oder ein pharmazeutisch unbedenkliches Salz davon.

**18.** Verbindung nach Anspruch 17, wobei

R1

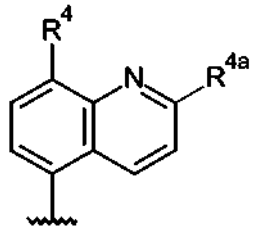
,
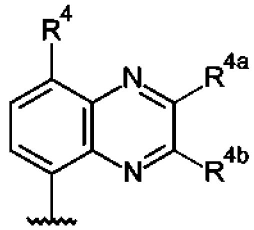
oder
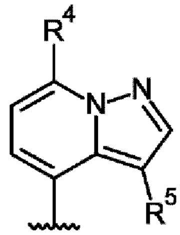

ist; wobei $R^4$ Methyl, Trifluormethyl oder Cyano ist; $R^{4a}$ und $R^{4b}$ gleichzeitig H oder Deuterium sind; $R^5$ H oder Fluor ist;
$R^2$ (4-Fluorpyrrolidin-3-yl)amino; 3,4,4a,5,7,7a-Hexahydro-2H-pyrrolo[3,4-b][1,4]oxazin-6-yl; 3-Amino-3-methylazetidin-1-yl; 3-Aamino-4-methoxypyrrolidin-1-yl; 3-Aminoazetidin-1-yl; 4-Amino-1-piperidinyl; 5-Oxa-2,8-diazaspiro[3.5]nonan-2-yl; 6-Methyl-2,6-diazaspiro[3.3]heptan-2-yl; Morpholin-2-yl; Morpholin-2-ylmethyl oder Piperazin-1-yl ist;
$R^3$ Methyl ist;
A

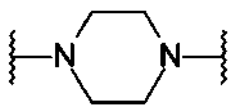

ist;
Q Phenyl, Pyridinyl, Pyrimidinyl oder Methylpyrimidinyl ist;
oder ein pharmazeutisch unbedenkliches Salz davon.

**19.** Verbindung nach Anspruch 1, ausgewählt aus:

5-[(*2R*,*6S*)-2-Methyl-6-[[3-(3-piperazin-1-ylphenyl)pyrrolidin-1-yl]methyl]morpholin-4-yl]chinolin-8-carbonitril;
5-[(*2R*,*6S*)-2-Methyl-6-[[4-[3-(4-piperidyl)phenyl]-1-piperidyl]methyl]morpholin-4-yl]chinolin-8-carbonitril;
5-[(*2R*,*6S*)-2-Methyl-6-[[3-(4-piperazin-1-ylphenyl)azetidin-1-yl]methyl]morpholin-4-yl]chinolin-8-carbonitril;
5-[(*2R*,*6S*)-2-Methyl-6-[[3-(3-piperazin-1-ylphenyl)azetidin-1-yl]methyl]morpholin-4-yl]chinolin-8-carbonitril;
5-[(*2S*,*6R*)-2-[[4-[4-(Aminomethyl)phenyl]piperazin-1-yl]methyl]-6-methylmorpholin-4-yl]chinolin-8-carbonitril;
5-[(*2R*,*6S*)-2-Methyl-6-[[4-(5-piperazin-1-ylpyrazin-2-yl)piperazin-1-yl]methyl]morpholin-4-yl]chinolin-8-carbonitril;
5-[(*2R*,*6S*)-2-Methyl-6-[[4-(5-piperazin-1-yl-3-pyridyl)piperazin-1-yl]methyl]morpholin-4-yl]chinolin-8-carbonitril;
5-[(*2R*,*6S*)-2-Methyl-6-[[4-(5-piperazin-1-yl-2-pyridyl)piperazin-1-yl]methyl]morpholin-4-yl]chinolin-8-carbonitril;
5-[(*2R*,*6S*)-2-Methyl-6-[[4-(4-piperazin-1-yl-2-pyridyl)piperazin-1-yl]methyl]morpholin-4-yl]chinolin-8-carbonitril;
5-[(*2R*,*6S*)-2-Methyl-6-[[4-(6-piperazin-1-yl-2-pyridyl)piperazin-1-yl]methyl]morpholin-4-yl]chinolin-8-carbonitril;
5-[(*2S*,*6R*)-2-[[4-Cyano-4-(4-piperazin-1-ylphenyl)-1-piperidyl]methyl]-6-methylmorpholin-4-yl] chinolin-8-carbonitril;
5-[(*2R*,*6S*)-2-Methyl-6-[[4-(2-piperazin-1-ylpyrimidin-5-yl)piperazin-1-yl]methyl]morpholin-4-yl]chinolin-8-carbonitril;
5-[(*2R*,*6S*)-2-Methyl-6-[[4-(6-piperazin-1-yl-3-pyridyl)piperazin-1-yl]methyl]morpholin-4-yl]chinolin-8-carbonitril;
5-[(*2R*,*6S*)-2-Methyl-6-[[4-(4-piperazin-1-ylpyrimidin-2-yl)piperazin-1-yl]methyl]morpholin-4-yl]chinolin-8-carbonitril;
5-[(*2R*,*6S*)-2-Methyl-6-[[4-(2-piperazin-1-ylpyrimidin-4-yl)piperazin-1-yl]methyl]morpholin-4-yl]chinolin-8-carbonitril;
5-[(*2R*,*6S*)-2-Methyl-6-[[4-(6-piperazin-1-ylpyridazin-3-yl)piperazin-1-yl]methyl]morpholin-4-yl]chinolin-8-carbonitril;
5-[(*2R*,*6S*)-2-Methyl-6-[[4-(5-piperazin-1-ylpyrimidin-2-yl)piperazin-1-yl]methyl]morpholin-4-yl]chinolin-8-carbonitril;
5-[(*2R*,*6S*)-2-Methyl-6-[[4-(6-piperazin-1-ylpyrimidin-4-yl)piperazin-1-yl]methyl]morpholin-4-yl]chinolin-8-carbonitril;
5-[(*2R*,*6S*)-2-Methyl-6-[[4-[4-[(2*R*)-morpholin-2-yl]phenyl]piperazin-1-yl]methyl]morpholin-4-yl]chinolin-8-carbonitril;
5-[(*2R*,*6S*)-2-Methyl-6-[[4-[4-[(2*S*)-morpholin-2-yl]phenyl]piperazin-1-yl]methyl]morpholin-4-yl]chinolin-8-carbonitril;
5-[(*2R*,*6S*)-2-Methyl-6-[[4-[6-(4-methylpiperazin-1-yl)pyrimidin-4-yl]piperazin-1-yl]methyl]morpholin-4-yl]chinolin-8-carbonitril;
5-[(2*S*,6*R*)-2-[[4-[2-(3-Aminoazetidin-1-yl)pyrimidin-4-yl]piperazin-1-yl]methyl]-6-methylmorpholin-4-yl] chinolin-8-carbonitril;
5-[(2*S*,6*R*)-2-[[4-[2-(3-Aminoazetidin-1-yl)pyrimidin-4-yl]piperazin-1-yl]methyl]-6-methylmorpholin-4-yl] chinolin-8-carbonitril;
5-[(2*S*,6*R*)-2-[[4-[2-[[(3*S*,4*R*)-4-Fluorpyrrolidin-3-yl]amino]pyrimidin-4-yl]piperazin-1-yl]methyl]-6-methylmorpholin-4-yl]chinolin-8-carbonitril;
5-[(2*S*,6*R*)-2-[[4-[2-(6-Hydroxy-1,4-diazepan-1-yl)pyrimidin-4-yl]piperazin-1-yl]methyl]-6-methylmorpholin-4-yl]chinolin-8-carbonitril;
5-[(*2S*,*6R*)-2-[[4-[2-*trans*-(3-Amino-4-methoxypyrrolidin-1-yl)pyrimidin-4-yl]piperazin-1-yl]methyl]-6-methylmorpholin-4-yl]chinolin-8-carbonitril;
5-[(2*S*,6*R*)-2-[[4-[2-(4-Amino-1-piperidyl)pyrimidin-4-yl]piperazin-1-yl]methyl]-6-methylmorpholin-4-yl] chinolin-8-carbonitril;
5-[(2*S*,6*R*)-2-[[4-[2-(Azetidin-3-ylamino)pyrimidin-4-yl]piperazin-1-yl]methyl]-6-methylmorpholin-4-yl] chinolin-8-carbonitril;
5-[(2*S*,6*R*)-2-[[4-[2-[(3*R*)-3-(Hydroxymethyl)piperazin-1-yl]pyrimidin-4-yl]piperazin-1-yl]methyl]-6-methylmorpholin-4-yl]chinolin-8-carbonitril;
5-[(2*S*,6*R*)-2-[[4-[2-[2-(Dimethylamino)ethoxy]pyrimidin-4-yl]piperazin-1-yl]methyl]-6-methylmorpholin-4-yl]chinolin-8-carbonitril;
5-[(2*R*,6*S*)-2-Methyl-6-[[4-[2-[4-[(2*S*)-pyrrolidin-2-carbonyl]piperazin-1-yl]pyrimidin-4-yl]piperazin-1-yl]methyl]morpholin-4-yl]chinolin-8-carbonitril;
5-[(2*S*,6*R*)-2-[[4-[2-[4-[2-(Dimethylamino)acetyl]piperazin-1-yl]pyrimidin-4-yl]piperazin-1-yl]methyl]-6-methylmorpholin-4-yl]chinolin-8-carbonitril;
5-[(2*R*,6*S*)-2-Methyl-6-[[4-[6-(4-methylpiperazin-1-yl)-2-pyridyl]piperazin-1-yl]methyl]morpholin-4-yl]chinolin-8-carbonitril;
5-[(2*S*,6*R*)-2-[[4-[2-[4-(Azetidin-2-carbonyl)piperazin-1-yl]pyrimidin-4-yl]piperazin-1-yl]methyl]-6-methylmorpholin-4-yl]chinolin-8-carbonitril;
5-[(2*S*,6*R*)-2-[[4-[6-[[(3*S*,4*S*)-4-Fluorpyrrolidin-3-yl]amino]-2-pyridyl]piperazin-1-yl]methyl]-6-methylmorpholin-4-yl]chinolin-8-carbonitril;
5-[(2*S*,6*R*)-2-[[4-[6-(4-Amino-1-piperidyl)-2-pyridyl]piperazin-1-yl]methyl]-6-methylmorpholin-4-yl] chinolin-8-carbonitril;
5-[(2*S*,6*R*)-2-[[4-[6-[[(3*S*,4*R*)-4-Fluorpyrrolidin-3-yl]amino]-2-pyridyl]piperazin-1-yl]methyl]-6-methylmorpholin-4-yl]chinolin-8-carbonitril;
5-[(*2S*,*6R*)-2-[[4-[6-(4-Amino-1-piperidyl)-2-pyridyl]piperazin-1-yl]methyl]-6-methylmorpholin-4-yl] chinolin-8-carbonitril;
5-[(*2S*,*6R*)-2-[[4-[6-[[(*3R*,*4R*)-4-Methoxypyrrolidin-3-yl]amino]-2-pyridyl]piperazin-1-yl]methyl]-6-methylmorpholin-4-yl]chinolin-8-carbonitril;
5-[(*2S*,*6R*)-2-[[4-[6-(3-Amino-4-fluorpyrrolidin-1-yl)-2-pyridyl]piperazin-1-yl]methyl]-6-methylmorpholin-4-yl]chinolin-8-carbonitril;
5-[(*2S*,*6R*)-2-[[4-[6-(6-Hydroxy-1,4-diazepan-1-yl)-2-pyridyl]piperazin-1-yl]methyl]-6-methylmorpholin-4-yl] chinolin-8-carbonitril;
5-[(*2S*,*6R*)-2-[[4-[6-[(*3R*,*4S*)-3-Amino-4-methoxypyrrolidin-1-yl]-2-pyridyl]piperazin-1-yl]methyl]-6-methylmorpholin-4-yl]chinolin-8-carbonitril;
5-[(*2S*,*6R*)-2-[[4-[6-(3-Amino-4-methoxypyrrolidin-1-yl)-3-pyridyl]piperazin-1-yl]methyl]-6-methylmorpholin-4-yl]chinolin-8-carbonitril;
5-[(*2S*,*6R*)-2-[[4-[6-(3-Amino-4-fluorpyrrolidin-1-yl)-3-pyridyl]piperazin-1-yl]methyl]-6-methylmorpholin-4-yl]chinolin-8-carbonitril;
5-[(*2S*,*6R*)-2-[[4-(5-Fluor-2-piperazin-1-ylpyrimidin-4-yl)piperazin-1-yl]methyl]-6-methylmorpholin-4-yl] chinolin-8-carbonitril;
5-[(*2S*,*6R*)-2-[[4-(5,6-Dimethyl-2-piperazin-1-ylpyrimidin-4-yl)piperazin-1-yl]methyl]-6-methylmorpholin-4-yl]chinolin-8-carbonitril;
5-[(*2R*,*6S*)-2-Methyl-6-[[4-(6-methyl-2-piperazin-1-ylpyrimidin-4-yl)piperazin-1-yl]methyl]morpholin-4-yl]chinolin-8-carbonitril;
5-[(*2S*,*6R*)-2-[[4-(5-Methoxy-2-piperazin-1-ylpyrimidin-4-yl)piperazin-1-yl]methyl]-6-methylmorpholin-4-yl] chinolin-8-carbonitril;
5-[(*2R*,*6S*)-2-Methyl-6-[[4-(2-piperazin-1-ylchinazolin-4-yl)piperazin-1-yl]methyl]morpholin-4-yl]chinolin-8-carbonitril;
5-[(2*S*,6*R*)-2-[[4-(5-Cyano-2-piperazin-1-ylpyrimidin-4-yl)piperazin-1-yl]methyl]-6-methylmorpholin-4-yl] chinolin-8-carbonitril;
5-[(*2S*,*6R*)-2-[[4-(5-Cyano-4-piperazin-1-ylpyrimidin-2-yl)piperazin-1-yl]methyl]-6-methylmorpholin-4-yl] chinolin-8-carbonitril;
5-[(*2S*,*6R*)-2-[[4-[2-[(*3R*,*4R*)-3-Amino-4-methoxypyrrolidin-1-yl]pyrimidin-4-yl]piperazin-1-yl]methyl]-6-methylmorpholin-4-yl]chinolin-8-carbonitril;
5-[(*2S*,*6R*)-2-[[4-[2-[(*3S*,*4S*)-3-Amino-4-methoxypyrrolidin-1-yl]pyrimidin-4-yl]piperazin-1-yl]methyl]-6-methylmorpholin-4-yl]chinolin-8-carbonitril;
5-[(*2S*,*6R*)-2-[[4-[2-[(*3S*,*4R*)-3-Aamino-4-fluorpyrrolidin-1-yl]-6-methylpyrimidin-4-yl]piperazin-1-yl]methyl]-6-methylmorpholin-4-yl]chinolin-8-carbonitril;
5-[(*2S*,*6R*)-2-[[4-[2-[(*3R*,*4S*)-3-Amino-4-fluorpyrrolidin-1-yl]-6-methylpyrimidin-4-yl]piperazin-1-yl]methyl]-6-methylmorpholin-4-yl]chinolin-8-carbonitril;
5-[(*2S*,*6R*)-2-[[4-[2-(6-Amino-1,4-oxazepan-4-yl)-6-methylpyrimidin-4-yl]piperazin-1-yl]methyl]-6-methylmorpholin-4-yl]chinolin-8-carbonitril;
5-[(2*S*,6*R*)-2-[[4-[2-[(4-Cyanopyrrolidin-3-yl)amino]-6-methylpyrimidin-4-yl]piperazin-1-yl]methyl]-6-methylmorpholin-4-yl]chinolin-8-carbonitril;
5-[(2*R*,6*S*)-2-Methyl-6-[[4-[6-methyl-2-[(1*R*,5*R*)-3-oxa-7,9-diazabicyclo[3.3.1]nonan-7-yl]pyrimidin-4-yl]piperazin-1-yl]methyl]morpholin-4-yl]chinolin-8-carbonitril;
5-[(2*R*,6*S*)-2-Methyl-6-[[4-[6-methyl-2-[*trans*-3-amino-4-methoxypyrrolidin-1-yl]pyrimidin-4-yl]piperazin-1-yl]methyl]morpholin-4-yl]chinolin-8-carbonitril;
5-[(*2R*,*6S*)-2-Methyl-6-[[4-[6-methyl-2-[*cis*-3-amino-4-methoxypyrrolidin-1-yl]pyrimidin-4-yl]piperazin-1-yl]methyl]morpholin-4-yl]chinolin-8-carbonitril;
5-[(*2R*,*6S*)-2-Methyl-6-[[4-[6-methyl-2-(5-oxa-2,8-diazaspiro[3.5]nonan-2-yl)pyrimidin-4-yl]piperazin-1-yl]methyl]morpholin-4-yl]chinolin-8-carbonitril;
5-[(*2R*,*6S*)-2-Methyl-6-[[4-(3-morpholin-2-ylphenyl)piperazin-1-yl]methyl]-morpholin-4-yl]chinolin-8-carbonitril;
5-[(2*S*,6*R*)-2-[[4-[2-(4-Amino-3,3-difluorpyrrolidin-1-yl)-6-methylpyrimidin-4-yl]piperazin-1-yl]methyl]-6-methylmorpholin-4-yl]chinolin-8-carbonitril;
8-[(*2R*,*6S*)-2-Methyl-6-[[4-(6-methyl-2-piperazin-1-ylpyrimidin-4-yl)piperazin-1-yl]methyl]morpholin-4-yl]chinoxalin-5-carbonitril;
(*2R*,*6S*)-2-Methyl-6-[[4-(6-methyl-2-piperazin-1-ylpyrimidin-4-yl)piperazin-1-yl]methyl]-4-[8-(trifluormethyl)chinoxalin-5-yl]morpholin;
5-[(*2S*,*6R*)-2-[[4-[2-(6-Hydroxy-1,4-diazepan-1-yl)-6-methylpyrimidin-4-yl]piperazin-1-yl]methyl]-6-methylmorpholin-4-yl]chinolin-8-carbonitril;
3-Fluor-4-[(2*R*,6*S*)-2-methyl-6-[[4-(6-methyl-2-piperazin-1-ylpyrimidin-4-yl)piperazin-1-yl]methyl]morpholin-4-yl]pyrazolo[1,5-a]pyridin-7-carbonitril;
5-[(*2R*,*6S*)-2-Methyl-6-[[4-(4-methyl-6-piperazin-1-ylpyrimidin-2-yl)piperazin-1-yl]methyl]morpholin-4-yl]chinolin-8-carbonitril;
5-[(*2S*,*6R*)-2-[[4-[2-*trans*-3-Amino-4-methoxypyrrolidin-1-yl]-6-methylpyrimidin-4-yl]piperazin-1-yl]methyl]-6-methylmorpholin-4-yl]chinolin-8-carbonitril;
5-[(2*S*,6*R*)-2-[[4-[2-[(3*S*,4*S*)-3-Amino-4-methoxypyrrolidin-1-yl]-6-methylpyrimidin-4-yl]piperazin-1-yl]methyl]-6-methylmorpholin-4-yl]chinolin-8-carbonitril;
5-[(2*S*,6*R*)-2-[[4-[2-[(3*R*,4*R*)-3-Amino-4-methoxypyrrolidin-1-yl]-6-methylpyrimidin-4-yl]piperazin-1-yl]methyl]-6-methylmorpholin-4-yl]chinolin-8-carbonitril;
5-[(2*S*,6*R*)-2-[[4-[2-[[(3*R*,4*R*)-4-Methoxypyrrolidin-3-yl]amino]-6-methylpyrimidin-4-yl]piperazin-1-yl]methyl]-6-methylmorpholin-4-yl]chinolin-8-carbonitril;
5-[(2*S*,6*R*)-2-[[4-[2-[[(3*R*,4*S*)-4-Fluorpyrrolidin-3-yl]amino]-6-methylpyrimidin-4-yl]piperazin-1-yl]methyl]-6-methylmorpholin-4-yl]chinolin-8-carbonitril;
5-[(2*S*,6*R*)-2-[[4-[2-[(3*S*,4*R*)-3-Amino-4-methoxypyrrolidin-1-yl]-6-methylpyrimidin-4-yl]piperazin-1-yl]methyl]-6-methylmorpholin-4-yl]chinolin-8-carbonitril;
5-[(2*S*,6*R*)-2-[[4-[2-[[(3*S*,4*R*)-4-Fluorpyrrolidin-3-yl]amino]-6-methylpyrimidin-4-yl]piperazin-1-yl]methyl]-6-methylmorpholin-4-yl]chinolin-8-carbonitril;

5-[(2*R*,6*S*)-2-Methyl-6-[[4-[6-methyl-2-[*cis*-3-amino-5-fluor-1-piperidyl]pyrimidin-4-yl]piperazin-1-yl]methyl]morpholin-4-yl]chinolin-8-carbonitril;
5-[(2*R*,6*S*)-2-Methyl-6-[[4-[6-methyl-2-[*trans*-3-amino-5-fluor-1-piperidyl]pyrimidin-4-yl]piperazin-1-yl]methyl]morpholin-4-yl]chinolin-8-carbonitril;
5-[(2*S*,6*R*)-2-[[4-[2-[[(3*R*,4*R*)-4-Fluorpyrrolidin-3-yl]amino]-6-methylpyrimidin-4-yl]piperazin-1-yl]methyl]-6-methylmorpholin-4-yl]chinolin-8-carbonitril;
5-[(2*S*,6*R*)-2-[[4-[2-[(3*S*,5*R*)-3-Amino-5-fluor-1-piperidyl]-6-methylpyrimidin-4-yl]piperazin-1-yl]methyl]-6-methylmorpholin-4-yl]chinolin-8-carbonitril;
5-[(2*S*,6*R*)-2-[[4-[2-[(3*S*,4*R*)-4-Amino-3-fluor-1-piperidyl]-6-methylpyrimidin-4-yl]piperazin-1-yl]methyl]-6-methylmorpholin-4-yl]chinolin-8-carbonitril;
5-[(2*S*,6*R*)-2-[[4-[2-[(3*R*,4*R*)-3-Amino-4-fluorpyrrolidin-1-yl]-6-methylpyrimidin-4-yl]piperazin-1-yl]methyl]-6-methylmorpholin-4-yl]chinolin-8-carbonitril;
5-[(2*S*,6*R*)-2-[[4-[2-(5-Amino-3,3-difluor-1-piperidyl)-6-methylpyrimidin-4-yl]piperazin-1-yl]methyl]-6-methylmorpholin-4-yl]chinolin-8-carbonitril;
5-[(2*S*,6*R*)-2-[[4-[2-[3-(1-Hydroxy-1-methylethyl)piperazin-1-yl]-6-methylpyrimidin-4-yl]piperazin-1-yl]methyl]-6-methylmorpholin-4-yl]chinolin-8-carbonitril;
5-[(2*S*,6*R*)-2-[[4-[2-[(*3R*,*4R*)-4-Amino-3-fluor-1-piperidyl]-6-methylpyrimidin-4-yl]piperazin-1-yl]methyl]-6-methylmorpholin-4-yl]chinolin-8-carbonitril;
5-[(*2R*,*6S*)-2-Methyl-6-[[4-[6-methyl-2-[(*3S,4R*)-3-amino-4-fluor-1-piperidyl]-pyrimidin-4-yl]piperazin-1-yl]methyl]morpholin-4-yl]chinolin-8-carbonitril;
5-[(*2A*,*6S*)-2-Methyl-6-[[4-[6-methyl-2-[(*3R*,*4R*)-3-amino-4-fluor-1-piperidyl]pyrimidin-4-yl]piperazin-1-yl]methyl]morpholin-4-yl]chinolin-8-carbonitril;
5-[(*2S*,*6R*)-2-[[4-[2-(Azetidin-3-ylamino)-6-methylpyrimidin-4-yl]piperazin-1-yl]methyl]-6-methylmorpholin-4-yl]chinolin-8-carbonitril;
5-[(*2S*,*6R*)-2-[[4-[2-(4-Amino-3,3-difluor-1-piperidyl)-6-methylpyrimidin-4-yl]piperazin-1-yl]methyl]-6-methylmorpholin-4-yl]chinolin-8-carbonitril;
5-[(*2S*,*6R*)-2-[[4-[2-(3-Aminoazetidin-1-yl)-6-methylpyrimidin-4-yl]piperazin-1-yl]methyl]-6-methylmorpholin-4-yl]chinolin-8-carbonitril;
5-[(*2S*,*6R*)-2-[[4-[2-[(*3R*,*4S*)-3-Amino-4-methoxypyrrolidin-1-yl]-6-methylpyrimidin-4-yl]piperazin-1-yl]methyl]-6-methylmorpholin-4-yl]chinolin-8-carbonitril;
5-[(*2R*,*6S*)-2-Methyl-6-[[4-(2-morpholin-2-ylphenyl)piperazin-1-yl]methyl]morpholin-4-yl]chinolin-8-carbonitril;
5-[*(2S*,*6R*)-2-[[4-[2-[(*3S*,*4S*)-4-Amino-3-methoxy-1-piperidyl]-6-methylpyrimidin-4-yl]piperazin-1-yl]methyl]-6-methylmorpholin-4-yl]chinolin-8-carbonitril;
5-[*(2S*,*6R)*-2-[[4-(6-Methoxy-2-piperazin-1-ylpyrimidin-4-yl)piperazin-1-yl]methyl]-6-methylmorpholin-4-yl] chinolin-8-carbonitril;
5-[*(2S*,*6R)*-2-[[4-[2-[(*3S*,*4R*)-3-Amino-4-methoxypyrrolidin-1-yl]pyrimidin-4-yl]piperazin-1-yl]methyl]-6-methylmorpholin-4-yl]chinolin-8-carbonitril;
5-[*(2S*,*6R)*-2-[[4-[2-[(*3R*,*4S*-3-Amino-4-methoxypyrrolidin-1-yl]pyrimidin-4-yl]piperazin-1-yl]methyl]-6-methylmorpholin-4-yl]chinolin-8-carbonitril;
5-[*(2S*,*6R)*-2-[[4-(5-Fluor-4-piperazin-1-ylpyrimidin-2-yl)piperazin-1-yl]methyl]-6-methylmorpholin-4-yl]chinolin-8-carbonitril;
5-[*(2S*,*6R)*-2-[[4-[2-[(*3S*,*4R*)-3-Amino-4-fluorpyrrolidin-1-yl]pyrimidin-4-yl]piperazin-1-yl]methyl]-6-methylmorpholin-4-yl]chinolin-8-carbonitril;
5-[*(2S*,*6R)*-2-[[4-[2-[(*3R*,*4R*)-3-Amino-4-fluorpyrrolidin-1-yl]pyrimidin-4-yl]piperazin-1-yl]methyl]-6-methylmorpholin-4-yl]chinolin-8-carbonitril;
5-[*(2S*,*6R)*-2-[[4-[4-[(*3S*,*4S*)-3-Amino-4-methoxypyrrolidin-1-yl]pyrimidin-2-yl]piperazin-1-yl]methyl]-6-methylmorpholin-4-yl]chinolin-8-carbonitril;
8-[*(2S*,*6R)*-2-[[4-[2-[(*3R*,*4R*)-3-Amino-4-methoxypyrrolidin-1-yl]pyrimidin-4-yl]piperazin-1-yl]methyl]-6-methylmorpholin-4-yl]chinoxalin-5-carbonitril;
8-[*(2S*,*6R)*-2-[[4-[2-[(*3S*,*4S*)-3-Amino-4-methoxypyrrolidin-1-yl]pyrimidin-4-yl]piperazin-1-yl]methyl]-6-methylmorpholin-4-yl]chinoxalin-5-carbonitril;
5-[*(2S*,*6R)*-2-[[4-[2-[(*3R*,*4S*)-3-Amino-4-fluorpyrrolidin-1-yl]pyrimidin-4-yl]piperazin-1-yl]methyl]-6-methylmorpholin-4-yl]chinolin-8-carbonitril;
5-[(*2R*,*6S*)-2-Methyl-6-[[4-(5-methyl-4-piperazin-1-ylpyrimidin-2-yl)piperazin-1-yl]methyl]morpholin-4-yl]chinolin-8-carbonitril;
5-[*(2S*,*6R)*-2-[[4-[2-[(*4aR*,*7aR*)-3,4,4*a*,5,7,7*a*-Hexahydro-2*H*-pyrrolo[3,4-b][1,4]oxazin-6-yl]pyrimidin-4-yl]piperazin-1-yl]methyl]-6-methylmorpholin-4-yl]chinolin-8-carbonitril;
8-[*(2S*,*6R)*-2-[[4-[4-[(*3S*,*4S*)-3-Amino-4-methoxypyrrolidin-1-yl]-6-methylpyrimidin-2-yl]piperazin-1-yl]methyl]-6-methylmorpholin-4-yl]chinoxalin-5-carbonitril;
8-[(2S,6R)-2-[[4-[4-[(3S,4S)-3-Amino-4-methoxypyrrolidin-1-yl]-6-methylpyrimidin-2-yl]piperazin-1-yl]methyl]-6-methylmorpholin-4-yl]-2,3-dideuteriochinoxalin-5-carbonitril;
8-[(2S,6R)-2-[[4-[2-[(3S,4S)-3-Amino-4-methoxypyrrolidin-1-yl]-6-methylpyrimidin-4-yl]piperazin-1-yl]methyl]-6-methylmorpholin-4-yl]chinoxalin-5-carbonitril;
8-[(2S,6R)-2-[[4-[2-[(3S,4S)-3-Amino-4-fluorpyrrolidin-1-yl]pyrimidin-4-yl]piperazin-1-yl]methyl]-6-methylmorpholin-4-yl]chinoxalin-5-carbonitril;
8-[(2S,6R)-2-[[4-[2-[(3R,4S)-3-Amino-4-fluorpyrrolidin-1-yl]pyrimidin-4-yl]piperazin-1-yl]methyl]-6-methylmorpholin-4-yl]chinoxalin-5-carbonitril;
5-[(2S,6R)-2-[[4-[2-(3-Amino-4-methoxy-1-piperidyl)pyrimidin-4-yl]piperazin-1-yl]methyl]-6-methylmorpholin-4-yl]chinolin-8-carbonitril;
5-[(2S,6R)-2-[[4-[2-[(3S,4S)-3-Amino-4-fluorpyrrolidin-1-yl]pyrimidin-4-yl]piperazin-1-yl]methyl]-6-methylmorpholin-4-yl]chinolin-8-carbonitril;
8-[(2S,6R)-2-[[4-[2-[(3R,4S)-4-Amino-3-fluor-1-piperidyl]pyrimidin-4-yl]piperazin-1-yl]methyl]-6-methylmorpholin-4-yl]chinoxalin-5-carbonitril;
8-[(2S,6R)-2-[[4-[2-(3-Aminoazetidin-1-yl)-6-methylpyrimidin-4-yl]piperazin-1-yl]methyl]-6-methylmorpholin-4-yl]chinoxalin-5-carbonitril;
8-[(2S,6R)-2-[[4-[2-[(3R,4R)-4-Amino-3-fluor-1-piperidyl]pyrimidin-4-yl]piperazin-1-yl]methyl]-6-methylmorpholin-4-yl]chinoxalin-5-carbonitril;
8-[(2R,6S)-2-Methyl-6-[[4-(4-methyl-6-piperazin-1-ylpyrimidin-2-yl)piperazin-1-yl]methyl]morpholin-4-yl]chinoxalin-5-carbonitril;
8-[(2S,6R)-2-[[4-[4-[(3R,4S)-3-Amino-4-fluorpyrrolidin-1-yl]-6-methylpyrimidin-2-yl]piperazin-1-yl]methyl]-6-methylmorpholin-4-yl]chinoxalin-5-carbonitril;
(2R,6S)-2-Methyl-4-(8-methylchinoxalin-5-yl)-6-[[4-(4-piperazin-1-ylpyrimidin-2-yl)piperazin-1-yl]methyl]morpholin;
(2R,6S)-2-Methyl-4-(8-methylchinoxalin-5-yl)-6-[[4-(2-piperazin-1-ylpyrimidin-4-yl)piperazin-1-yl]methyl]morpholin;
8-[(2S,6R)-2-[[4-[4-(3-Aminoazetidin-1-yl)pyrimidin-2-yl]piperazin-1-yl]methyl]-6-methylmorpholin-4-yl]chinoxalin-5-carbonitril;
8-[(2S,6R)-2-[[4-[4-[(3R,4R)-3-Amino-4-fluorpyrrolidin-1-yl]pyrimidin-2-yl]piperazin-1-yl]methyl]-6-methylmorpholin-4-yl]chinoxalin-5-carbonitril;
8-[(2S,6R)-2-[[4-[4-[(3S,4R)-3-Amino-4-methoxypyrrolidin-1-yl]-6-methylpyrimidin-2-yl]piperazin-1-yl]methyl]-6-methylmorpholin-4-yl]chinoxalin-5-carbonitril;
8-[(2S,6R)-2-[[4-[4-[(3R,4R)-3-Amino-4-methoxypyrrolidin-1-yl]-6-methylpyrimidin-2-yl]piperazin-1-yl]methyl]-6-methylmorpholin-4-yl]chinoxalin-5-carbonitril;
8-[(2S,6R)-2-[[4-[4-[(3R,4S)-3-Amino-4-methoxypyrrolidin-1-yl]-6-methylpyrimidin-2-yl]piperazin-1-yl]methyl]-6-methylmorpholin-4-yl]chinoxalin-5-carbonitril;
8-[(2S,6R)-2-[[4-[4-(3-Aminoazetidin-1-yl)-6-methylpyrimidin-2-yl]piperazin-1-yl]methyl]-6-methylmorpholin-4-yl]chinoxalin-5-carbonitril;
8-[(2S,6R)-2-[[4-[4-(3-Aminoazetidin-1-yl)-6-methylpyrimidin-2-yl]piperazin-1-yl]methyl]-6-methylmorpholin-4-yl]-2,3-dideuteriochinoxalin-5-carbonitril;
8-[(2S,6R)-2-[[4-[4-[(3R,4R)-4-Amino-3-hydroxy-3-methylpyrrolidin-1-yl]-6-methylpyrimidin-2-yl]piperazin-1-yl]methyl]-6-methylmorpholin-4-yl]chinoxalin-5-carbonitril,
2-[4-[4-[[(2S,6R)-6-Methyl-4-(8-methylchinoxalin-5-yl)morpholin-2-yl]methyl]piperazin-1-yl]pyrimidin-2-yl]-5-oxa-2,8-diazaspiro[3.5]nonan;
(2R,6S)-2-Methyl-6-[[4-[2-[(3S)-3-methylpiperazin-1-yl]pyrimidin-4-yl]piperazin-1-yl]methyl]-4-(8-methylchinoxalin-5-yl)morpholin;
(2R,6S)-2-Methyl-6-[[4-[2-[(3R)-3-methylpiperazin-1-yl]pyrimidin-4-yl]piperazin-1-yl]methyl]-4-(8-methylchinoxalin-5-yl)morpholin;
(4aR,7aR)-6-[4-[4-[[(2S,6R)-6-Methyl-4-(8-methylchinoxalin-5-yl)morpholin-2-yl]methyl]piperazin-1-yl]pyrimidin-2-yl]-3,4,4a,5,7,7a-hexahydro-2H-pyrrolo[3,4-b][1,4]oxazin,
1-[4-[4-[[(2S,6R)-6-Methyl-4-(8-methylchinoxalin-5-yl)morpholin-2-yl]methyl]piperazin-1-yl]pyrimidin-2-yl]azetidin-3-amin;
5-[(2S,6R)-2-[[4-[4-(3-Amino-3-methylazetidin-1-yl)-6-methoxypyrimidin-2-yl]piperazin-1-yl]methyl]-6-methylmorpholin-4-yl]-2-deuteriochinolin-8-carbonitril;
5-[(2S,6R)-2-[[4-[6-(3-Amino-3-methylazetidin-1-yl)-3-pyridyl]piperazin-1-yl]methyl]-6-methylmorpholin-4-yl]-2-deuteriochinolin-8-carbonitril;
5-[(2S,6R)-2-[[4-[6-(3-Amino-3-methylazetidin-1-yl)-4-methyl-3-pyridyl]piperazin-1-yl]methyl]-6-methylmorpholin-4-yl]-2-deuteriochinolin-8-carbonitril;
5-[*(2S,6R)*-2-[[4-[4-[(*3S*,*4S*)-3-Amino-4-methoxypyrrolidin-1-yl]-6-methylpyrimidin-2-yl]piperazin-1-yl]methyl]-6-methylmorpholin-4-yl]-2-deuteriochinolin-8-carbonitril;
5-[*(2S*,*6R*)-2-[[4-[4-(3-Amino-3-methylazetidin-1-yl)-6-methylpyrimidin-2-yl]piperazin-1-yl]methyl]-6-methylmorpholin-4-yl]-2-deuteriochinolin-8-carbonitril;
5-[*(2S*,*6R)*-2-[[4-[4-(3-Aminoazetidin-1-yl)-6-methylpyrimidin-2-yl]piperazin-1-yl]methyl]-6-methylmorpholin-4-yl]-2-deuteriochinolin-8-carbonitril;
5-[*(2S*,*6R)*-2-[[4-[6-(3-Amino-3-methylazetidin-1-yl)-2-methyl-3-pyridyl]piperazin-1-yl]methyl]-6-methylmorpholin-4-yl]-2-deuteriochinolin-8-carbonitril;
5-[*(2S*,*6R)*-2-[[3-[6-(3-Amino-3-methylazetidin-1-yl)-3-pyridyl]azetidin-1-yl]methyl]-6-methylmorpholin-4-yl]-2-deuteriochinolin-8-carbonitril;
5-[*(2S*,*6R)*-2-[[4-[5-(3-Amino-3-methylazetidin-1-yl)-3-methyl-2-pyridyl]piperazin-1-yl]methyl]-6-methylmorpholin-4-yl]-2-deuteriochinolin-8-carbonitril;
5-[*(2S*,*6R)*-2-[[3-[4-[*(3S*,*4S*)-3-Amino-4-methoxypyrrolidin-1-yl]phenyl]azetidin-1-yl]methyl]-6-methylmorpholin-4-yl]-2-deuteriochinolin-8-carbonitril;
5-[*(2S*,*6R)*-2-[[3-[4-(3-Amino-3-methylazetidin-1-yl)phenyl]azetidin-1-yl]methyl]-6-methylmorpholin-4-yl]-2-deuteriochinolin-8-carbonitril;
5-[*(2S*,*6R)*-2-[[4-[5-[(*3R*,*4R*)-3-Amino-4-methoxypyrrolidin-1-yl]-3-methyl-2-pyridyl]piperazin-1-yl]methyl]-6-methylmorpholin-4-yl]-2-deuteriochinolin-8-carbonitril;
5-[*(2S*,*6R)*-2-[[4-[6-[(*3R*,*4R*)-3-Amino-4-methoxypyrrolidin-1-yl]-3-pyridyl]piperazin-1-yl]methyl]-6-methylmorpholin-4-yl]-2-deuteriochinolin-8-carbonitril;
5-[*(2S*,*6R)*-2-[[4-[4-[(*3R*,*4R*)-3-Amino-4-methoxypyrrolidin-1-yl]-6-methylpyrimidin-2-yl]piperazin-1-yl]methyl]-6-methylmorpholin-4-yl]-2-deuteriochinolin-8-carbonitril;
5-[*(2S*,*6R)*-2-[[3-[6-(3-Amino-3-methylazetidin-1-yl)-4-methyl-3-pyridyl]azetidin-1-yl]methyl]-6-methylmorpholin-4-yl]-2-deuteriochinolin-8-carbonitril;
5-[*(2S*,*6R)*-2-[[3-[6-[(*3R*,*4R*)-3-Amino-4-methoxypyrrolidin-1-yl]-4-methyl-3-pyridyl]azetidin-1-yl]methyl]-6-methylmorpholin-4-yl]-2-deuteriochinolin-8-carbonitril;
5-[*(2S*,*6R)*-2-[[4-[5-(3-Amino-3-methylazetidin-1-yl)-6-methyl-2-pyridyl]piperazin-1-yl]methyl]-6-methylmorpholin-4-yl]-2-deuteriochinolin-8-carbonitril;
4-[*(2S*,*6R)*-2-[[4-[5-(3-Amino-3-methylazetidin-1-yl)-3-methyl-2-pyridyl]piperazin-1-yl]methyl]-6-methylmorpholin-4-yl]-3-fluorpyrazolo[1,5-a]pyridin-7-carbonitril;
5-[*(2S*,*6R)*-2-[[3-[6-[(*3R*,*4R*)-3-Amino-4-methoxypyrrolidin-1-yl]-3-pyridyl]azetidin-1-yl]methyl]-6-methylmorpholin-4-yl]-2-deuteriochinolin-8-carbonitril;
5-[*(2S*,*6R)*-2-[[4-[6-[(*3S*,*4S*)-3-Amino-4-fluorpyrrolidin-1-yl]-3-pyridyl]piperazin-1-yl]methyl]-6-methylmorpholin-4-yl]-2-deuteriochinolin-8-carbonitril;
2-Deuterio-5-[*(2S*,*6R)*-2-[[4-[6-[(1*S*,4*S*)-2,5-diazabicyclo[2.2.1]heptan-2-yl]-3-pyridyl]piperazin-1-yl]methyl]-6-methylmorpholin-4-yl]chinolin-8-carbonitril;
2-Deuterio-5-[(*2R*,*6S*)-2-methyl-6-[[4-[6-(5-oxa-2,8-diazaspiro[3.5]nonan-2-yl)-3-pyridyl]piperazin-1-yl]methyl]morpholin-4-yl]chinolin-8-carbonitril,
2-Deuterio-5-[*(2S*,*6R)*-2-[[4-[6-[(1*R*,4*R*)-2,5-diazabicyclo[2.2.1]heptan-2-yl]-3-pyridyl]piperazin-1-yl]methyl]-6-methylmorpholin-4-yl]chinolin-8-carbonitril;
4-[*(2S*,*6R)*-2-[[4-[6-(3-Amino-3-methylazetidin-1-yl)-3-pyridyl]piperazin-1-yl]methyl]-6-methylmorpholin-4-yl]-3-fluorpyrazolo[1,5-a]pyridin-7-carbonitril;
5-[*(2S*,*6R)*-2-[[4-[6-(3-Amino-3-methylazetidin-1-yl)-2-pyridyl]piperazin-1-yl]methyl]-6-methylmorpholin-4-yl]-2-deuteriochinolin-8-carbonitril;
5-[*(2S*,*6R)*-2-[[3-[4-(3-Amino-3-methylazetidin-1-yl)phenyl]-3-hydroxyazetidin-1-yl]methyl]-6-methylmorpholin-4-yl]-2-deuteriochinolin-8-carbonitril;
4-[*(2S*,*6R)*-2-[[3-[6-(3-Amino-3-methylazetidin-1-yl)-3-pyridyl]azetidin-1-yl]methyl]-6-methylmorpholin-4-yl]pyrazolo[1,5-a]pyridin-7-carbonitril;
4-[*(2S*,*6R)*-2-[[3-[6-(3-Amino-3-methylazetidin-1-yl)-3-pyridyl]azetidin-1-yl]methyl]-6-methylmorpholin-4-yl]-3-fluorpyrazolo[1,5-a]pyridin-7-carbonitril;
5-[*(2S*,*6R)*-2-[[3-[6-(3-Amino-3-methylazetidin-1-yl)-2-pyridyl]azetidin-1-yl]methyl]-6-methylmorpholin-4-yl]-2-deuteriochinolin-8-carbonitril;
4-[*(2S*,*6R)*-2-[[3-[4-(3-Amino-3-methylazetidin-1-yl)phenyl]azetidin-1-yl]methyl]-6-methylmorpholin-4-yl]-3-fluorpyrazolo[1,5-a]pyridin-7-carbonitril;
2-Deuterio-5-[(*2R*,*6S*)-2-methyl-6-[[4-[6-(6-methyl-2,6-diazaspiro[3.3]heptan-2-yl)-3-pyridyl]piperazin-1-yl]methyl]morpholin-4-yl]chinolin-8-carbonitril,
2-Deuterio-5-[*(2S*,*6R)*-2-[[4-[6-[[*(3S*,*4R)*-4-fluorpyrrolidin-3-yl]amino]-3-pyridyl]piperazin-1-yl]methyl]-6-methylmorpholin-4-yl]chinolin-8-carbonitril;
4-[*(2S,6R)*-2-[[3-[4-(3-Amino-3-methylazetidin-1-yl)phenyl]azetidin-1-yl]methyl]-6-methylmorpholin-4-yl]pyrazolo[1,5-a]pyridin-7-carbonitril;
4-[*(2S,6R)*-2-[[3-[6-[(*3R,4R*)-3-Amino-4-methoxypyrrolidin-1-yl]-3-pyridyl]azetidin-1-yl]methyl]-6-methylmorpholin-4-yl]-3-fluorpyrazolo[1,5-a]pyridin-7-carbonitril;
4-[*(2S,6R)*-2-[[3-[6-[(1S,4S)-2,5-Diazabicyclo[2.2.1]heptan-2-yl]-3-pyridyl]azetidin-1-yl]methyl]-6-methylmorpholin-4-yl]-3-fluorpyrazolo[1,5-a]pyridin-7-carbonitril;
4-[*(2S,6R)*-2-[[4-[6-[(*3R,4R*)-3-Amino-4-methoxypyrrolidin-1-yl]-3-pyridyl]piperazin-1-yl]methyl]-6-methylmorpholin-4-yl]-3-fluorpyrazolo[1,5-a]pyridin-7-carbonitril;
3-Fluor-4-[*(2S,6R)*-2-[[4-[6-[[(*3S,4R*)-4-fluorpyrrolidin-3-yl]amino]-3-pyridyl]piperazin-1-yl]methyl]-6-methylmorpholin-4-yl]pyrazolo[1,5-a]pyridin-7-carbonitril;
4-[*(2S,6R)*-2-[[4-[6-[(4*aR*,7*aR*)-3,4,4a,5,7,7*a*-Hexahydro-2*H*-pyrrolo[3,4-b][1,4]oxazin-6-yl]-3-pyridyl]piperazin-1-yl]methyl]-6-methylmorpholin-4-yl]-3-fluorpyrazolo[1,5-a]pyridin-7-carbonitril;
3-Fluor-4-[(2*R*,6*S*)-2-methyl-6-[[4-[6-(5-oxa-2,8-diazaspiro[3.5]nonan-2-yl)-3-pyridyl]piperazin-1-yl]methyl]morpholin-4-yl]pyrazolo[1,5-a]pyridin-7-carbonitril;
3-Fluor-4-[(2*R*,6*S*)-2-methyl-6-[[4-[6-(6-methyl-2,6-diazaspiro[3.3]heptan-2-yl)-3-pyridyl]piperazin-1-yl]methyl]morpholin-4-yl]pyrazolo[1,5-a]pyridin-7-carbonitril;
1-Methyl-4-[(2*R*,6*S*)-2-methyl-6-[[3-(4-piperazin-1-ylphenyl)azetidin-1-yl]methyl]morpholin-4-yl]-1,8-naphthyridin-2-on;
4-[*(2S,6R)*-2-[[3-[4-[(*3R,4R*)-3-Amino-4-methoxypyrrolidin-1-yl]phenyl]azetidin-1-yl]methyl]-6-methylmorpholin-4-yl]-1-methyl-1,8-naphthyridin-2-on;
1-Methyl-4-[(2*R*,6*S*)-2-methyl-6-[[3-[4-(5-oxa-2,8-diazaspiro[3.5]nonan-2-yl)phenyl]azetidin-1-yl]methyl]morpholin-4-yl]-1,8-naphthyridin-2-on;
4-[*(2S,6R)*-2-[[3-[4-[(*3R,4R*)-3-Amino-4-fluorpyrrolidin-1-yl]phenyl]azetidin-1-yl]methyl]-6-methylmorpholin-4-yl]-1-methyl-1,8-naphthyridin-2-on;
4-[*(2S,6R)*-2-[[4-[6-[*(3R,4R)*-4-Amino-3-fluor-1-piperidyl]-3-pyridyl]piperazin-1-yl]methyl]-6-methylmorpholin-4-yl]-3-fluorpyrazolo[1,5-a]pyridin-7-carbonitril;
4-[*(2S,6R)*-2-[[3-[4-(3-Amino-3-methylazetidin-1-yl)phenyl]azetidin-1-yl]methyl]-6-methylmorpholin-4-yl]-1-methyl-1,8-naphthyridin-2-on;
3-Fluor-4-[(2*R*,6*S*)-2-methyl-6-[[4-[4-[(2*R*)-morpholin-2-yl]phenyl]piperazin-1-yl]methyl]morpholin-4-yl]pyrazolo[1,5-a]pyridin-7-carbonitril;
3-Fluor-4-[(2*R*,6*S*)-2-methyl-6-[[4-[6-(5-oxa-2,8-diazaspiro[3.5]nonan-8-yl)-3-pyridyl]piperazin-1-yl]methyl]morpholin-4-yl]pyrazolo[1,5-a]pyridin-7-carbonitril;
4-[*(2S,6R)*-2-[[4-[6-(1,6-Diazaspiro[3.3]heptan-6-yl)-3-pyridyl]piperazin-1-yl]methyl]-6-methylmorpholin-4-yl]-3-fluorpyrazolo[1,5-a]pyridin-7-carbonitril;
1-Methyl-4-[(2*R*,6*S*)-2-methyl-6-[[3-[4-(6-methyl-2,6-diazaspiro[3.3]heptan-2-yl)phenyl]azetidin-1-yl]methyl]morpholin-4-yl]-1,8-naphthyridin-2-on;
4-[(*2S*,*6R*)-2-[[3-[4-[(*4aR*,*7aR*)-3,4,4*a*,5,7,7*a*-Hexahydro-2*H*-pyrrolo[3,4-*b*][1,4]oxazin-6-yl]phenyl] azetidin-1-yl]methyl]-6-methylmorpholin-4-yl]-1-methyl-1, 8-naphthyridin-2-on;
3-Fluor-4-[(2*S*,6*R*)-2-[[4-[2-fluor-4-[(2*S*)-morpholin-2-yl]phenyl]piperazin-1-yl]methyl]-6-methylmorpholin-4-yl]pyrazolo[1,5-a]pyridin-7-carbonitril;
3-Fluor-4-[(2*S*,6*R*)-2-[[4-[3-fluor-4-[(2*R*)-morpholin-2-yl]phenyl]piperazin-1-yl]methyl]-6-methylmorpholin-4-yl]pyrazolo[1,5-a]pyridin-7-carbonitril;
3-Fluor-4-[(2*R*,6*S*)-2-methyl-6-[[4-[4-(2-methylmorpholin-2-yl)phenyl]piperazin-1-yl]methyl]morpholin-4-yl]pyrazolo[1,5-a]pyridin-7-carbonitril; und
3-Fluor-4-[(2*R*,6*S*)-2-methyl-6-[[4-[4-[[(2*R*)-morpholin-2-yl]methyl]phenyl]piperazin-1-yl]methyl]morpholin-4-yl]pyrazolo[1,5-a]pyridin-7-carbonitril;
oder ein pharmazeutisch unbedenkliches Salz davon.

**20.** Verfahren zur Herstellung einer Verbindung nach einem der Ansprüche 1 bis 19, umfassend einen der folgenden Schritte:

a) die Substitution einer Verbindung der Formel (IV)

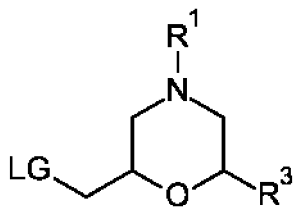

(IV),

mit einer Verbindung der Formel (VIII)

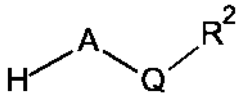

(VIII)

in der Gegenwart einer Base;
b) das Koppeln einer Verbindung der Formel (XIII)

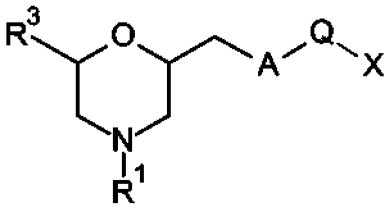

(XIII)

mit einer Verbindung der Formel (VI) in der Gegenwart einer Base oder unter Buchwald-Hartwig-Aminierungsbedingung;

wobei die Base in Schritt a) und b) aus $K_2CO_3$, DIPEA und $Cs_2CO_3$ ausgewählt ist; die Buchwald-Hartwig-Aminierungsbedingung in Schritt b) einen Katalysator und eine Base einschließt, wobei der Katalysator aus tBuXPhos Pd G3, RuPhos Pd G2, BrettPhos Pd G3, XPhos Pd G3, $Pd_2(dba)_3$/BINAP und $Pd_2(dba)_3$/XantPhos ausgewählt ist; die Base $Cs_2CO_3$ oder t-BuONa ist; LG eine Abgangsgruppe ist; $R^1$, $R^2$, $R^3$, A, Q und X wie in einem der Ansprüche 1 bis 18 definiert sind.

**21.** Verbindung oder pharmazeutisch unbedenkliches Salz nach einem der Ansprüche 1 bis 19 zur Verwendung als therapeutisch wirksame Substanz.

**22.** Pharmazeutische Zusammensetzung, umfassend eine Verbindung nach einem der Ansprüche 1 bis 19 und einen therapeutisch inerten Träger.

**23.** Verbindung oder pharmazeutisch unbedenkliches Salz, Enantiomer oder Diastereomer nach einem der Ansprüche 1 bis 19 zur Verwendung bei der Behandlung oder Prophylaxe von systemischem Lupus erythematodes oder Lupusnephritis.

## Revendications

**1.** Composé de formule (I),

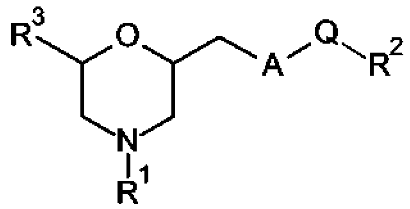

(I),

dans lequel

$R^1$ représente

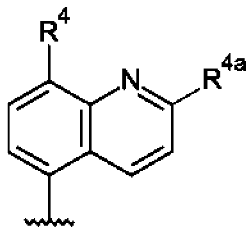

,

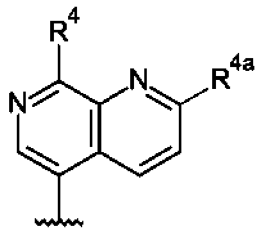

,

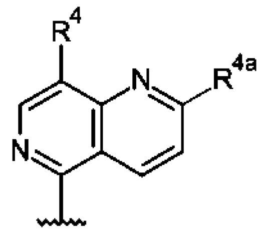

,

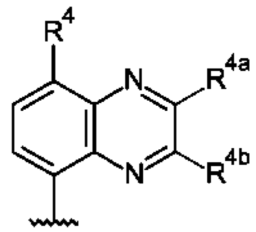

,

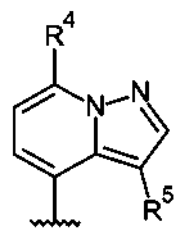

,

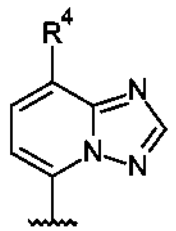

,

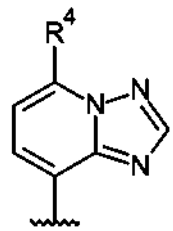

,

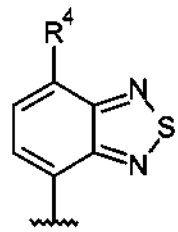

,

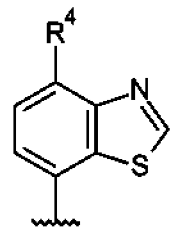

,

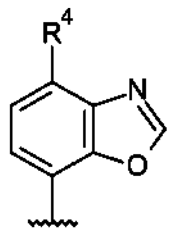

,

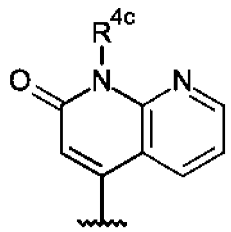

ou

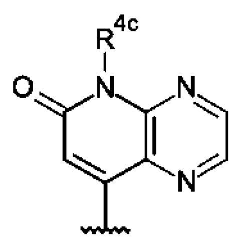

;

dans lequel $R^4$ représente un alkyle en $C_{1-6}$, un alcoxy en $C_{1-6}$, un halogénoalkyle en $C_{1-6}$, un halogène, un nitro ou un cyano ; $R^{4a}$ représente H ou un deutérium ; $R^{4b}$ représente H, un deutérium ou un alkyle en $C_{1-6}$ ; $R^{4c}$ représente un alkyle en $C_{1-6}$ ou un cycloalkyle en $C_{3-7}$ ; $R^5$ représente H ou un halogène ;

$R^2$ représente un ((alkyle en $C_{1-6}$)$_2$amino)alcoxy en $C_{1-6}$ ;

un (alcoxy en $C_{1-6}$-pyrrolidinyl)amino ;
un (cyanopyrrolidinyl)amino ;
un 1,4-diazépanyle substitué par un hydroxy ;
un 1,6-diazaspiro[3.3]heptanyle ;
un 2,5-diazabicyclo[2.2.1]heptanyle ;
un 2,6-diazaspiro[3.3]heptanyle substitué par un alkyle en $C_{1-6}$ ;
un 3,4,4a,5,7,7a-hexahydro-2H-pyrrolo[3,4-b][1,4]oxazinyle ;
un 3-oxa-7,9-diazabicyclo[3.3.1]nonanyle ;
un 5-oxa-2,8-diazaspiro[3.5]nonanyle ;
un aminoalkyle en $C_{1-6}$ ;
un aminooxazépanyle ;
un azétidinyle substitué une ou deux fois par des substituants indépendamment choisis parmi un amino et

un alkyle en $C_{1-6}$ ;
un azétidinylamino ;
un halogénopyrrolidinylamino ;
un morpholinyle non substitué ou substitué par un alkyle en $C_{1-6}$ ;
un morpholinylalkyle en $C_{1-6}$ ;
un pipérazinyle non substitué ou substitué par un alkyle en $C_{1-6}$, un hydroxyalkyle en $C_{1-6}$, un pyrrolidinylcarbonyle, un ((alkyle en $C_{1-6})_2$amino)alkyle en $C_{1-6}$-carbonyle ou un azétidinylcarbonyle ;
un pipéridinyle non substitué ou substitué une ou deux fois par des substituants indépendamment choisis parmi un amino, un halogène et un alcoxy en $C_{1-6}$ ; ou
un pyrrolidinyle substitué une, deux ou trois fois par des substituants indépendamment choisis parmi un amino, un halogène, un hydroxy, un alkyle en $C_{1-6}$ et un alcoxy en $C_{1-6}$ ;

$R^3$ représente un alkyle en $C_{1-6}$ ;
A représente un azétidinyle, un hydroxyazétidinyle, un pipérazinyle, un pyrrolidinyle, un pipéridinyle ou un cyanopipéridinyle ;
Q représente un phényle non substitué ou substitué par un halogène ;

un pyrazinyle ;
un pyridazinyle ;
un pyridinyle non substitué ou substitué par un alkyle en $C_{1-6}$ ;
un pyrimidinyle non substitué ou substitué une ou deux fois par des substituants indépendamment choisis parmi un halogène, un alkyle en $C_{1-6}$, un alcoxy en $C_{1-6}$ et un cyano ; ou
un quinazolinyle ;

ou un sel pharmaceutiquement acceptable de celui-ci.

**2.** Composé selon la revendication 1 de formule (Ia),

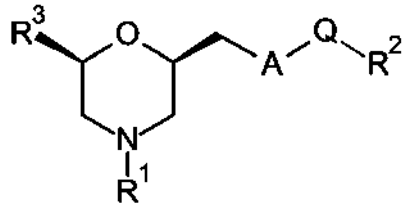

(Ia),

dans lequel

$R^1$ représente

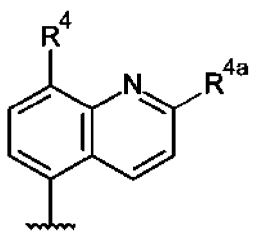
,
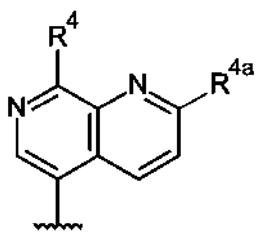
,
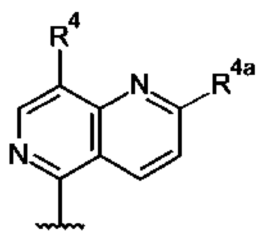
,
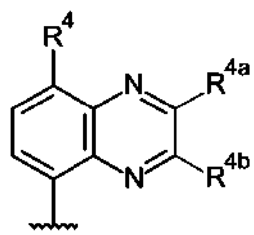
,

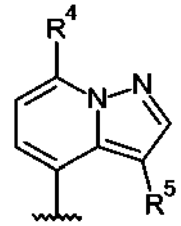
,
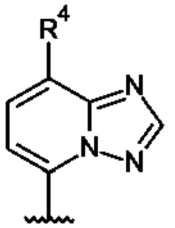
,
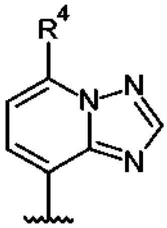
,
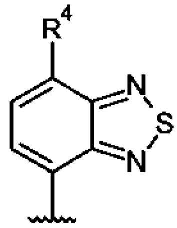
,
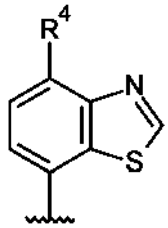
,

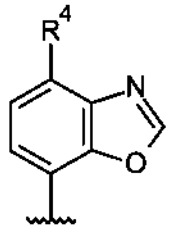
,
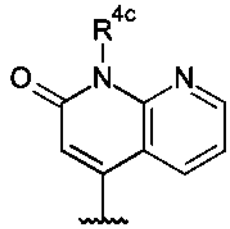
ou
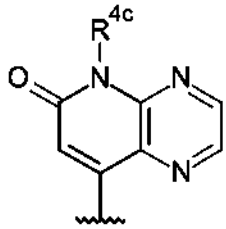
;

dans lequel $R^4$ représente un alkyle en $C_{1-6}$, un alcoxy en $C_{1-6}$, un halogénoalkyle en $C_{1-6}$, un halogène, un nitro ou un cyano ; $R^{4a}$ représente H ou un deutérium ; $R^{4b}$ représente H, un deutérium ou un alkyle en $C_{1-6}$; $R^{4c}$ représente un alkyle en $C_{1-6}$ ou un cycloalkyle en $C_{3-7}$; $R^5$ représente H ou un halogène ;
$R^2$ représente un ((alkyle en $C_{1-6}$)$_2$amino)alcoxy en $C_{1-6}$ ;

un (alcoxy en $C_{1-6}$-pyrrolidinyl)amino ;
un (cyanopyrrolidinyl)amino ;
un 1,4-diazépanyle substitué par un hydroxy ;
un 1,6-diazaspiro[3.3]heptanyle ;
un 2,5-diazabicyclo[2.2.1]heptanyle ;
un 2,6-diazaspiro[3.3]heptanyle substitué par un alkyle en $C_{1-6}$ ;
un 3,4,4a,5,7,7a-hexahydro-2H-pyrrolo[3,4-b][1,4]oxazinyle ;
un 3-oxa-7,9-diazabicyclo[3.3.1]nonanyle ;
un 5-oxa-2,8-diazaspiro[3.5]nonanyle ;
un aminoalkyle en $C_{1-6}$ ;
un aminooxazépanyle ;
un azétidinyle substitué une ou deux fois par des substituants indépendamment choisis parmi un amino et un alkyle en $C_{1-6}$ ;
un azétidinylamino ;
un halogénopyrrolidinylamino ;
un morpholinyle non substitué ou substitué par un alkyle en $C_{1-6}$ ;
un morpholinylalkyle en $C_{1-6}$ ;
un pipérazinyle non substitué ou substitué par un alkyle en $C_{1-6}$, un hydroxyalkyle en $C_{1-6}$, un pyrrolidinylcarbonyle, un ((alkyle en $C_{1-6}$)$_2$amino)alkyle en $C_{1-6}$-carbonyle ou un azétidinylcarbonyle ;
un pipéridinyle non substitué ou substitué une ou deux fois par des substituants indépendamment choisis parmi un amino, un halogène et un alcoxy en $C_{1-6}$ ; ou
un pyrrolidinyle substitué une, deux ou trois fois par des substituants indépendamment choisis parmi un amino, un halogène, un hydroxy, un alkyle en $C_{1-6}$ et un alcoxy en $C_{1-6}$ ;

$R^3$ représente un alkyle en $C_{1-6}$ ;
A représente un azétidinyle, un hydroxyazétidinyle, un pipérazinyle, un pyrrolidinyle, un pipéridinyle ou un cyanopipéridinyle ;
Q représente un phényle non substitué ou substitué par un halogène ;

un pyrazinyle ;
un pyridazinyle ;
un pyridinyle non substitué ou substitué par un alkyle en $C_{1-6}$ ;
un pyrimidinyle non substitué ou substitué une ou deux fois par des substituants indépendamment choisis parmi un halogène, un alkyle en $C_{1-6}$, un alcoxy en $C_{1-6}$ et un cyano ; ou
un quinazolinyle ;

ou un sel pharmaceutiquement acceptable de celui-ci.

**3.** Composé selon la revendication 1 ou 2, ou un sel pharmaceutiquement acceptable de celui-ci, dans lequel $R^1$ représente

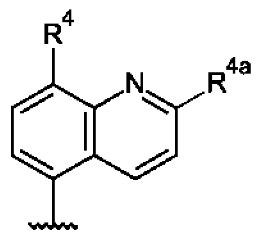 , 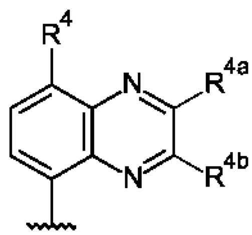 , 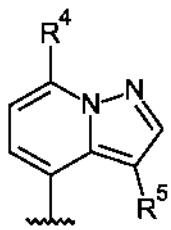

ou

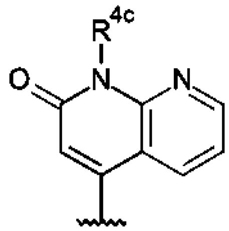 ;

dans lequel $R^4$ représente un alkyle en $C_{1-6}$, un halogénoalkyle en $C_{1-6}$ ou un cyano ; $R^{4a}$ représente H ou un deutérium ; $R^{4b}$ représente H ou un deutérium ; $R^{4c}$ représente un alkyle en $C_{1-6}$ ; $R^5$ représente H ou un halogène.

**4.** Composé selon la revendication 3, ou un sel pharmaceutiquement acceptable de celui-ci, dans lequel $R^4$ représente un méthyle, un trifluorométhyle ou un cyano ; $R^{4a}$ représente H ou un deutérium ; $R^{4b}$ représente H ou un deutérium ; $R^{4c}$ représente un méthyle ; $R^5$ représente H ou un fluor.

**5.** Composé selon la revendication 3, ou un sel pharmaceutiquement acceptable de celui-ci, dans lequel $R^2$ représente un (((alkyle en $C_{1-6}$)$_2$amino)alkyle en $C_{1-6}$-carbonyl)pipérazinyle ; un ((alkyle en $C_{1-6}$)$_2$amino)alcoxy en $C_{1-6}$ ; un (azétidinylcarbonyl)pipérazinyle ; un (alcoxy en $C_{1-6}$-pyrrolidinyl)amino ; un (alkyle en $C_{1-6}$)morpholinyle ; un (cyanopyrrolidinyl)amino ; un (hydroxyalkyle en $C_{1-6}$)pipérazinyle ; un (pyrrolidinylcarbonyl)pipérazinyle ; un 1,6-diazaspiro[3.3]heptanyle ; un 2,5-diazabicyclo[2.2.1]heptanyle ; un 3,4,4a,5,7,7a-hexahydro-2H-pyrrolo[3,4-b][1,4]oxazinyle ; un 3-oxa-7,9-diazabicyclo[3.3.1]nonanyle ; un 5-oxa-2,8-diazaspiro[3.5]nonanyle ; un amino(alcoxy en $C_{1-6}$)pipéridinyle ; un amino(alcoxy en $C_{1-6}$)pyrrolidinyle ; un amino(alkyle en $C_{1-6}$)azétidinyle ; un amino(hydroxy)(alkyle en $C_{1-6}$)pyrrolidinyle ; un aminoazétidinyle ; un aminoalkyle en $C_{1-6}$; un aminohalogénopipéridinyle ; un aminohalogénopyrrolidinyle ; un aminooxazépanyle ; un aminopipéridinyle ; un azétidinylamino ; un alkyle en $C_{1-6}$-2,6-diazaspiro[3.3]heptanyle ; un alkyle en $C_{1-6}$-pipérazinyle ; un halogénopyrrolidinylamino ; un hydroxy-1,4-diazépanyle ; un morpholinyle ; un morpholinylalkyle en $C_{1-6}$ ; un pipérazinyle ou un pipéridinyle.

**6.** Composé selon la revendication 5, ou un sel pharmaceutiquement acceptable de celui-ci, dans lequel $R^2$ représente un (1-hydroxy-1-méthyl-éthyl)pipérazin-1-yle ; un (2-pyrrolidinylcarbonyl)pipérazin-1-yle ; un (4-cyanopyrrolidin-3-yl)amino ; un (4-fluoropyrrolidin-3-yl)amino ; un (4-méthoxypyrrolidin-3-yl)amino ; un (diméthylamino)éthoxy ; un 1,6-diazaspiro[3.3]heptan-6-yle ; un 2,5-diazabicyclo[2.2.1]heptan-2-yle ; un 2-méthylmorpholin-2-yle ; un 2-morpholin-2-yle ; un 3-(hydroxyméthyl)pipérazin-1-yle ; un 3,4,4a,5,7,7a-hexahydro-2H-pyrrolo[3,4-b][1,4]oxazin-6-yle ; un 3-amino-3-méthyl-azétidin-1-yle ; un 3-amino-4-fluoro-1-pipéridinyle ; un 3-amino-4-fluoro-pyrrolidin-1-yle ; un 3-amino-4-méthoxy-1-pipéridinyle ; un 3-amino-4-méthoxy-pyrrolidin-1-yle ; un 3-amino-5-fluoro-1-pipéridinyle ; un 3-aminoazétidin-1-yle ; un 3-méthylpipérazin-1-yle ; un 3-oxa-7,9-diazabicyclo[3.3.1]nonan-7-yle ; un 4-(azétidinyl-2-carbonyl)pipérazin-1-yle ; un 4-[2-(diméthylamino)acétyl]pipérazin-1-yle ; un 4-amino-1-pipéridinyle ; un 4-amino-3,3-difluoro-1-pipéridinyle ; un 4-amino-3,3-difluoro-pyrrolidin-1-yle ; un 4-amino-3-fluoro-1-pipéridinyle ; un 4-amino-3-hydroxy-3-méthyl-pyrrolidin-1-yle ; un 4-amino-3-méthoxy-1-pipéridinyle ; un 4-méthylpipérazin-1-yle ; un 4-pipéridinyle ; un 5-amino-3,3-difluoro-1-pipéridinyle ; un 5-oxa-2,8-diazaspiro[3.5]nonan-2-yle ; un 5-oxa-2,8-diazaspiro[3.5]nonan-8-yle ; un 6-amino-1,4-oxazépan-4-yle ; un 6-hydroxy-1,4-diazépan-1-yle ; un 6-méthyl-2,6-diazaspiro[3.3]heptan-2-yle ; un aminométhyle ; un azétidin-3-ylamino ; un morpholin-2-yle ; un morpholin-2-ylméthyle ou un pipérazin-1-yle.

**7.** Composé selon la revendication 6, ou un sel pharmaceutiquement acceptable de celui-ci, dans lequel A représente

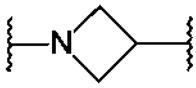 , 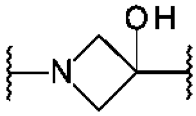 , 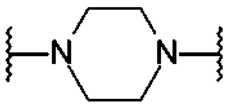 , 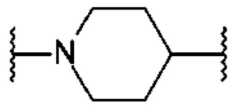 ,

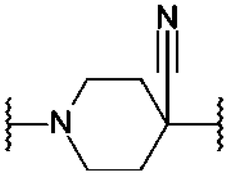 ou 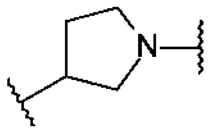 .

8. Composé selon la revendication 7, ou un sel pharmaceutiquement acceptable de celui-ci, dans lequel Q représente un phényle, un fluorophényle, un pyrazinyle, un pyridazinyle, un pyridinyle, un méthylpyridinyle, un pyrimidinyle, un cyanopyrimidinyle, un fluoropyrimidinyle, un méthoxypyrimidinyle, un méthylpyrimidinyle, un diméthylpyrimidinyle ou un quinazolinyle.

9. Composé selon la revendication 8, ou un sel pharmaceutiquement acceptable de celui-ci, dans lequel $R^3$ représente un méthyle.

10. Composé selon la revendication 1 ou 2, ou un sel pharmaceutiquement acceptable de celui-ci, dans lequel $R^1$ représente

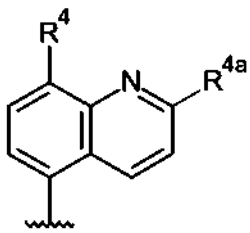 , 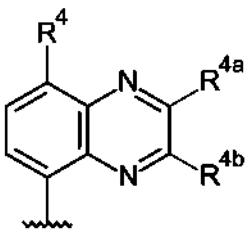 ou 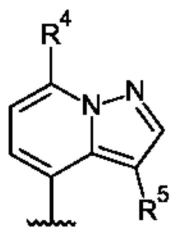 ;

dans lequel $R^4$ représente un alkyle en $C_{1-6}$, un halogénoalkyle en $C_{1-6}$ ou un cyano ; $R^{4a}$ et $R^{4b}$ représentent simultanément H ou un deutérium ; $R^5$ représente H ou un halogène.

11. Composé selon la revendication 10, ou un sel pharmaceutiquement acceptable de celui-ci, dans lequel $R^4$ représente un méthyle, un trifluorométhyle ou un cyano ; $R^{4a}$ et $R^{4b}$ représentent simultanément H ou un deutérium ; $R^5$ représente H ou un fluor.

12. Composé selon la revendication 10, ou un sel pharmaceutiquement acceptable de celui-ci, dans lequel $R^2$ représente un 3,4,4a,5,7,7a-hexahydro-2H-pyrrolo[3,4-b][1,4]oxazinyle ; un 5-oxa-2,8-diazaspiro[3.5]nonanyle ; un amino(alcoxy en $C_{1-6}$)pyrrolidinyle ; un amino(alkyle en $C_{1-6}$)azétidinyle ; un aminoazétidinyle ; un aminopipéridinyle ; un alkyle en $C_{1-6}$-2,6-diazaspiro[3.3]heptanyle ; un halogénopyrrolidinylamino ; un morpholinyle ; un morpholinylalkyle en $C_{1-6}$ ou un pipérazinyle.

13. Composé selon la revendication 12, ou un sel pharmaceutiquement acceptable de celui-ci, dans lequel $R^2$ représente un (4-fluoropyrrolidin-3-yl)amino ; un 3,4,4a,5,7,7a-hexahydro-2H-pyrrolo[3,4-b][1,4]oxazin-6-yle ; un 3-amino-3-méthyl-azétidin-1-yle ; un 3-amino-4-méthoxy-pyrrolidin-1-yle ; un 3-aminoazétidin-1-yle ; un 4-amino-1-pipéridinyle ; un 5-oxa-2,8-diazaspiro[3.5]nonan-2-yle ; un 6-méthyl-2,6-diazaspiro[3.3]heptan-2-yle ; un morpholin-2-yle ; un morpholin-2-ylméthyle ou un pipérazin-1-yle.

14. Composé selon la revendication 12, ou un sel pharmaceutiquement acceptable de celui-ci, dans lequel A représente

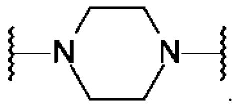

.

**15.** Composé selon la revendication 14, ou un sel pharmaceutiquement acceptable de celui-ci, dans lequel Q représente un phényle, un pyridinyle, un pyrimidinyle ou un alkyle en $C_{1-6}$-pyrimidinyle.

**16.** Composé selon la revendication 15, ou un sel pharmaceutiquement acceptable de celui-ci, dans lequel Q représente un phényle, un pyridinyle, un pyrimidinyle ou un méthylpyrimidinyle.

**17.** Composé selon la revendication 3, dans lequel

$R^1$ représente

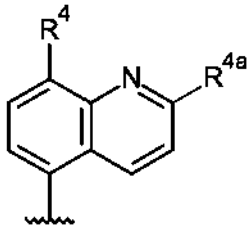

,

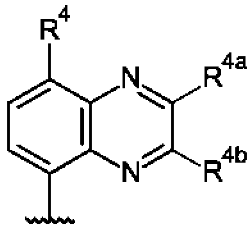

ou

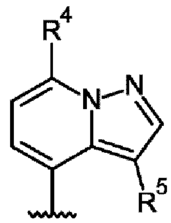

;

dans lequel $R^4$ représente un alkyle en $C_{1-6}$, un halogénoalkyle en $C_{1-6}$ ou un cyano ; $R^{4a}$ et $R^{4b}$ représentent simultanément H ou un deutérium ; $R^5$ représente H ou un halogène ;
$R^2$ représente un 3,4,4a,5,7,7a-hexahydro-2H-pyrrolo[3,4-b][1,4]oxazinyle ; un 5-oxa-2,8-diazaspiro[3.5]nonanyle ; un amino(alcoxy en $C_{1-6}$)pyrrolidinyle ; un amino(alkyle en $C_{1-6}$)azétidinyle ; un aminoazétidinyle ; un aminopipéridinyle ; un alkyle en $C_{1-6}$-2,6-diazaspiro[3.3]heptanyle ; un halogénopyrrolidinylamino ; un morpholinyle ; un morpholinylalkyle en $C_{1-6}$ ou un pipérazinyle ;
$R^3$ représente un alkyle en $C_{1-6}$ ;
A représente

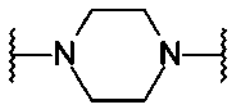

;

Q représente un phényle, un pyridinyle, un pyrimidinyle ou un alkyle en $C_{1-6}$-pyrimidinyle ; ou un sel pharmaceutiquement acceptable de celui-ci.

**18.** Composé selon la revendication 17, dans lequel

$R^1$ représente

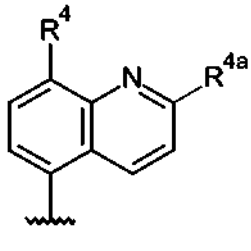

,

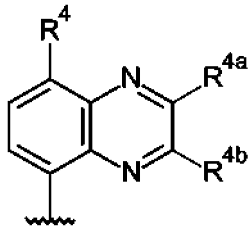

ou

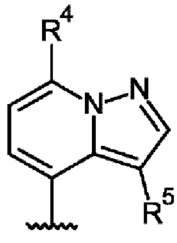

;

dans lequel $R^4$ représente un méthyle, un trifluorométhyle ou un cyano ; $R^{4a}$ et $R^{4b}$ représentent simultanément

H ou un deutérium ; $R^5$ représente H ou un fluor ;
$R^2$ représente un (4-fluoropyrrolidin-3-yl)amino ; un 3,4,4a,5,7,7a-hexahydro-2H-pyrrolo[3,4-b][1,4]oxazin-6-yle ; un 3-amino-3-méthyl-azétidin-1-yle ; un 3-amino-4-méthoxy-pyrrolidin-1-yle ; un 3-aminoazétidin-1-yle ; un 4-amino-1-pipéridinyle ; un 5-oxa-2,8-diazaspiro[3.5]nonan-2-yle ; un 6-méthyl-2,6-diazaspiro[3.3]heptan-2-yle ; un morpholin-2-yle ; un morpholin-2-ylméthyle ou un pipérazin-1-yle ;
$R^3$ représente un méthyle ;
A représente

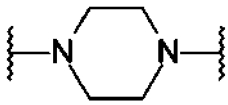
;

Q représente un phényle, un pyridinyle, un pyrimidinyle ou un méthylpyrimidinyle ;
ou un sel pharmaceutiquement acceptable de celui-ci.

**19.** Composé selon la revendication 1 choisi parmi :

le 5-[(*2R*,*6S*)-2-méthyl-6-[[3-(3-pipérazin-1-ylphényl)pyrrolidin-1-yl]méthyl]morpholin-4-yl]quinoléine-8-carbonitrile ;
le 5-[(*2R*,*6S*)-2-méthyl-6-[[4-[3-(4-pipéridyl)phényl]-1-pipéridyl]méthyl]morpholin-4-yl]quinoléine-8-carbonitrile ;
le 5-[(2*R*,6*S*)-2-méthyl-6-[[3-(4-pipérazin-1-ylphényl)azétidin-1-yl]méthyl]morpholin-4-yl]quinoléine-8-carbonitrile ;
le 5-[(*2R*,*6S*)-2-méthyl-6-[[3-(3-pipérazin-1-ylphényl)azétidin-1-yl]méthyl]morpholin-4-yl]quinoléine-8-carbonitrile ;
le 5-[(*2S*,*6R*)-2-[[4-[4-(aminométhyl)phényl]pipérazin-1-yl]méthyl]-6-méthyl-morpholin-4-yl]quinoléine-8-carbonitrile ;
le 5-[(*2R*,*6S*)-2-méthyl-6-[[4-(5-pipérazin-1-ylpyrazin-2-yl)pipérazin-1-yl]méthyl]morpholin-4-yl]quinoléine-8-carbonitrile ;
le 5-[(*2R*,*6S*)-2-méthyl-6-[[4-(5-pipérazin-1-yl-3-pyridyl)pipérazin-1-yl]méthyl]morpholin-4-yl]quinoléine-8-carbonitrile ;
le 5-[(*2R*,*6S*)-2-méthyl-6-[[4-(5-pipérazin-1-yl-2-pyridyl)pipérazin-1-yl]méthyl]morpholin-4-yl]quinoléine-8-carbonitrile ;
le 5-[(*2R*,*6S*)-2-méthyl-6-[[4-(4-pipérazin-1-yl-2-pyridyl)pipérazin-1-yl]méthyl]morpholin-4-yl]quinoléine-8-carbonitrile ;
le 5-[(*2R*,*6S*)-2-méthyl-6-[[4-(6-pipérazin-1-yl-2-pyridyl)pipérazin-1-yl]méthyl]morpholin-4-yl]quinoléine-8-carbonitrile ;
le 5-[(*2S*,*6R*)-2-[[4-cyano-4-(4-pipérazin-1-ylphényl)-1-pipéridyl]méthyl]-6-méthy-morpholin-4-yl]quinoléine-8-carbonitrile ;
le 5-[(*2R*,*6S*)-2-méthyl-6-[[4-(2-pipérazin-1-ylpyrimidin-5-yl)pipérazin-1-yl]méthyl]morpholin-4-yl]quinoléine-8-carbonitrile ;
le 5-[(*2R*,*6S*)-2-méthyl-6-[[4-(6-pipérazin-1-yl-3-pyridyl)pipérazin-1-yl]méthyl]morpholin-4-yl]quinoléine-8-carbonitrile ;
le 5-[(*2R*,*6S*)-2-méthyl-6-[[4-(4-pipérazin-1-ylpyrimidin-2-yl)pipérazin-1-yl]méthyl]morpholin-4-yl]quinoléine-8-carbonitrile ;
le 5-[(*2R*,*6S*)-2-méthyl-6-[[4-(2-pipérazin-1-ylpyrimidin-4-yl)pipérazin-1-yl]méthyl]morpholin-4-yl]quinoléine-8-carbonitrile ;
le 5-[(*2R*,*6S*)-2-méthyl-6-[[4-(6-pipérazin-1-ylpyridazin-3-yl)pipérazin-1-yl]méthyl]morpholin-4-yl]quinoléine-8-carbonitrile ;
le 5-[(*2R*,*6S*-2-méthyl-6-[[4-(5-pipérazin-1-ylpyrimidin-2-yl)pipérazin-1-yl]méthyl]morpholin-4-yl]quinoléine-8-carbonitrile ;
le 5-[(*2R*,*6S*)-2-méthyl-6-[[4-(6-pipérazin-1-ylpyrimidin-4-yl)pipérazin-1-yl]méthyl]morpholin-4-yl]quinoléine-8-carbonitrile ;
le 5-[(*2R*,*6S*)-2-méthyl-6-[[4-[4-[(2*R*)-morpholin-2-yl]phényl]pipérazin-1-yl]méthyl]morpholin-4-yl]quinoléine-8-carbonitrile ;
le 5-[(*2R*,*6S*)-2-méthyl-6-[[4-[4-[(2*S*)-morpholin-2-yl]phényl]pipérazin-1-yl]méthyl]morpholin-4-yl]quinoléine-8-carbonitrile ;
le 5-[(*2R*,*6S*)-2-méthyl-6-[[4-[6-(4-méthylpipérazin-1-yl)pyrimidin-4-yl]pipérazin-1-yl]méthyl]morpholin-4-yl]quinoléine-8-carbonitrile ;
le 5-[(*2S*,*6R*)-2-[[4-[2-(3-aminoazétidin-1-yl)pyrimidin-4-yl]pipérazin-1-yl]méthyl]-6-méthyl-morpholin-4-yl]quinoléine-8-carbonitrile ;
le 5-[(2*S*,6*R*)-2-[[4-[2-(3-aminoazétidin-1-yl)pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[[(3*S*,4*R*)-4-fluoropyrrolidin-3-yl]amino]pyrimidin-4-yl]pipérazin-1-yl]méthyl]-6-méthyl-morpholin-4-yl]quinoléine-8-carbonitrile ;
le 5-[(2*S*,6*R*)-2-[[4-[2-(6-hydroxy-1,4-diazépan-1-yl)pyrimidin-4-yl]pipérazin-1-yl]méthyl]-6-méthyl-morpholin-4-yl]quinoléine-8-carbonitrile ;
le 5-[(*2S*,*6R*)-2-[[4-[2-*trans*-(3-amino-4-méthoxy-pyrrolidin-1-yl)pyrimidin-4-yl]pipérazin-1-yl]méthyl]-6-méthyl-morpholin-4-yl]quinoléine-8-carbonitrile ;
le 5-[(2*S*,6*R*)-2-[[4-[2-(4-amino-1-pipéridyl)pyrimidin-4-yl]pipérazin-1-yl]méthyl]-6-méthyl-morpholin-4-yl]quinoléine-8-carbonitrile ;
le 5-[(2*S*,6*R*)-2-[[4-[2-(azétidin-3-ylamino)pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*R*)-3-(hydroxyméthyl)pipérazin-1-yl]pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[2-(diméthylamino)éthoxy]pyrimidin-4-yl]pipérazin-1-yl]méthyl]-6-méthyl-morpholin-4-yl]quinoléine-8-carbonitrile ;
le 5-[(2*R*,6*S*)-2-méthyl-6-[[4-[2-[4-[(2*S*)-pyrrolidine-2-carbonyl]pipérazin-1-yl]pyrimidin-4-yl]pipérazin-1-yl]méthyl]morpholin-4-yl]quinoléine-8-carbonitrile ;
le 5-[(2*R*,6*S*)-2-[[4-[2-[4-[2-(diméthylamino)acétyl]pipérazin-1-yl]pyrimidin-4-yl]pipérazin-1-yl]méthyl]-6-méthyl-morpholin-4-yl]quinoléine-8-carbonitrile ;
le 5-[(2*S*,6*R*)-2-méthyl-6-[[4-[6-(4-méthylpipérazin-1-yl)-2-pyridyl]pipérazin-1-yl]méthyl]morpholin-4-yl]quinoléine-8-carbonitrile ;
le 5-[(2*R*,6*S*)-2-[[4-[2-[4-(azétidine-2-carbonyl)pipérazin-1-yl]pyrimidin-4-yl]pipérazin-1-yl]méthyl]-6-méthyl-morpholin-4-yl]quinoléine-8-carbonitrile ;
le 5-[(2*S*,6*R*)-2-[[4-[6-[[(3*S*,4*S*)-4-fluoropyrrolidin-3-yl]amino]-2-pyridyl]pipérazin-1-yl]méthyl]-6-méthyl-morpholin-4-yl]quinoléine-8-carbonitrile ;
le 5-[(2*S*,6*R*)-2-[[4-[6-(4-amino-1-pipéridyl)-2-pyridyl]pipérazin-1-yl]méthyl]-6-méthyl-morpholin-4-yl]quinoléine-8-carbonitrile ;
le 5-[(*2S*,*6R*)-2-[[4-[6-[[(*3S*,*4R*)-4-fluoropyrrolidin-3-yl]amino]-2-pyridyl]pipérazin-1-yl]méthyl]-6-méthyl-morpholin-4-yl]quinoléine-8-carbonitrile ;
le 5-[(*2S*,*6R*)-2-[[4-[6-(4-amino-1-pipéridyl)-2-pyridyl]pipérazin-1-yl]méthyl]-6-méthyl-morpholin-4-yl]quinoléine-8-carbonitrile ;
le 5-[(*2S*,*6R*)-2-[[4-[6-[[(*3R*,*4R*)-4-méthoxypyrrolidin-3-yl]amino]-2-pyridyl]pipérazin-1-yl]méthyl]-6-méthyl-morpholin-4-yl]quinoléine-8-carbonitrile ;
le 5-[(*2S*,*6R*)-2-[[4-[6-(3-amino-4-fluoro-pyrrolidin-1-yl)-2-pyridyl]pipérazin-1-yl]méthyl]-6-méthyl-morpholin-4-yl]quinoléine-8-carbonitrile ;
le 5-[(*2S*,*6R*)-2-[[4-[6-(6-hydroxy-1,4-diazépan-1-yl)-2-pyridyl]pipérazin-1-yl]méthyl]-6-méthyl-morpholin-4-yl]quinoléine-8-carbonitrile ;
le 5-[(*2S*,*6R*)-2-[[4-[6-[(*3R*,*4S*)-3-amino-4-méthoxy-pyrrolidin-1-yl]-2-pyridyl]pipérazin-1-yl]méthyl]-6-méthyl-morpholin-4-yl]quinoléine-8-carbonitrile ;
le 5-[(2*S*,*6R*)-2-[[4-[6-(3-amino-4-méthoxy-pyrrolidin-1-yl)-3-pyridyl]pipérazin-1-yl]méthyl]-6-méthyl-morpholin-4-yl]quinoléine-8-carbonitrile ;
le 5-[(2*S*,*6R*)-2-[[4-[6-(3-amino-4-fluoro-pyrrolidin-1-yl)-3-pyridyl]pipérazin-1-yl]méthyl]-6-méthyl-morpholin-4-yl]quinoléine-8-carbonitrile ;
le 5-[(*2S*,*6R*)-2-[[4-(5-fluoro-2-pipérazin-1-yl-pyrimidin-4-yl)pipérazin-1-yl]méthyl]-6-méthyl-morpholin-4-yl]quinoléine-8-carbonitrile ;
le 5-[(*2S*,*6R*)-2-[[4-(5,6-diméthyl-2-pipérazin-1-yl-pyrimidin-4-yl)pipérazin-1-yl]méthyl]-6-méthyl-morpholin-4-yl]quinoléine-8-carbonitrile ;
le 5-[(*2R*,*6S*)-2-méthyl-6-[[4-(6-méthyl-2-pipérazin-1-yl-pyrimidin-4-yl)pipérazin-1-yl]méthyl]morpholin-4-yl]quinoléine-8-carbonitrile ;
le 5-[(*2S*,*6R*)-2-[[4-(5-méthoxy-2-pipérazin-1-yl-pyrimidin-4-yl)pipérazin-1-yl]méthyl]-6-méthyl-morpholin-4-yl]quinoléine-8-carbonitrile ;
le 5-[(*2R*,*6S*)-2-méthyl-6-[[4-(2-pipérazin-1-ylquinazolin-4-yl)pipérazin-1-yl]méthyl]morpholin-4-yl]quinoléine-8-carbonitrile ;
le 5-[(2S,6R)-2-[[4-(5-cyano-2-pipérazin-1-yl-pyrimidin-4-yl)pipérazin-1-yl]méthyl]-6-méthyl-morpholin-4-yl]quinoléine-8-carbonitrile ;
le 5-[(*2S*,*6R*)-2-[[4-(5-cyano-4-pipérazin-1-yl-pyrimidin-2-yl)pipérazin-1-yl]méthyl]-6-méthyl-morpholin-4-yl]quinoléine-8-carbonitrile ;
le 5-[(*2S*,*6R*)-2-[[4-[2-[(*3R*,*4R*)-3-amino-4-méthoxy-pyrrolidin-1-yl]pyrimidin-4-yl]pipérazin-1-yl]méthyl]-6-méthyl-morpholin-4-yl]quinoléine-8-carbonitrile ;
le 5-[(*2S*,*6R*)-2-[[4-[2-[(*3S*,*4S*)-3-amino-4-méthoxy-pyrrolidin-1-yl]pyrimidin-4-yl]pipérazin-1-yl]méthyl]-6-méthyl-morpholin-4-yl]quinoléine-8-carbonitrile ;
le 5-[(*2S*,*6R*)-2-[[4-[2-[(*3S*,*4R*)-3-amino-4-fluoro-pyrrolidin-1-yl]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*R*,4*S*)-3-amino-4-fluoro-pyrrolidin-1-yl]-6-méthyl-pyrimidin-4-yl]pipérazin-1-yl]méthyl]-6-méthyl-morpholin-4-yl]quinoléine-8-carbonitrile ;
le 5-[(*2S*,*6R*)-2-[[4-[2-(6-amino-1,4-oxazépan-4-yl)-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(4-cyanopyrrolidin-3-yl)amino]-6-méthyl-pyrimidin-4-yl]pipérazin-1-yl]méthyl]-6-méthyl-morpholin-4-yl]quinoléine-8-carbonitrile ;
le 5-[(2*R*,6*S*)-2-méthyl-6-[[4-[6-méthyl-2-[(1*R*,5*R*)-3-oxa-7,9-diazabicyclo[3.3.1]nonan-7-yl]pyrimidin-4-yl]pipérazin-1-yl]méthyl]morpholin-4-yl]quinoléine-8-carbonitrile ;
le 5-[(2*R*,6*S*)-2-méthyl-6-[[4-[6-méthyl-2-[trans-3-amino-4-méthoxy-pyrrolidin-1-yl]pyrimidin-4-yl]pipérazin-1-yl]méthyl]morpholin-4-yl]quinoléine-8-carbonitrile ;
le 5-[(2*R*,6*S*)-2-méthyl-6-[[4-[6-méthyl-2-[*cis*-3-amino-4-méthoxy-pyrrolidin-1-yl]pyrimidin-4-yl]pipérazin-1-yl]méthyl]morpholin-4-yl]quinoléine-8-carbonitrile ;
le 5-[(*2R*,*6S*)-2-méthyl-6-[[4-[6-méthyl-2-(5-oxa-2,8-diazaspiro[3.5]nonan-2-yl)pyrimidin-4-yl]pipérazin-1-yl]méthyl]morpholin-4-yl]quinoléine-8-carbonitrile ;
le 5-[(*2R*,*6S*)-2-méthyl-6-[[4-(3-morpholin-2-ylphényl)pipérazin-1-yl]méthyl]-morpholin-4-yl]quinoléine-8-carbonitrile ;
le 5-[(2*S*,6*R*)-2-[[4-[2-(4-amino-3,3-difluoro-pyrrolidin-1-yl)-6-méthyl-pyrimidin-4-yl]pipérazin-1-yl]méthyl]-6-méthyl-morpholin-4-yl]quinoléine-8-carbonitrile ;
le 8-[(*2R*,*6S*)-2-méthyl-6-[[4-(6-méthyl-2-pipérazin-1-yl-pyrimidin-4-yl)pipérazin-1-yl]méthyl]morpholin-4-yl]quinoxaline-5-carbonitrile ;
la (*2R*,*6S*)-2-méthyl-6-[[4-(6-méthyl-2-pipérazin-1-yl-pyrimidin-4-yl)pipérazin-1-yl]méthyl]-4-[8-(trifluorométhyl)quinoxalin-5-yl]morpholine ;
le 5-[(*2S*,*6R*)-2-[[4-[2-(6-hydroxy-1,4-diazépan-1-yl)-6-méthyl-pyrimidin-4-yl]pipérazin-1-yl]méthyl]-6-méthyl-morpholin-4-yl]quinoléine-8-carbonitrile ;
le 3-fluoro-4-[(*2R*,*6S*)-2-méthyl-6-[[4-(6-méthyl-2-pipérazin-1-yl-pyrimidin-4-yl)pipérazin-1-yl]méthyl]morpholin-4-yl]pyrazolo[1,5-a]pyridine-7-carbonitrile ;
le 5-[(2*R*,6*S*)-2-méthyl-6-[[4-(4-méthyl-6-pipérazin-1-yl-pyrimidin-2-yl)pipérazin-1-yl]méthyl]morpholin-4-yl]quinoléine-8-carbonitrile ;
le 5-[(*2S*,*6R*)-2-[[4-[2-*trans*-3-amino-4-méthoxy-pyrrolidin-1-yl]-6-méthyl-pyrimidin-4-yl]pipérazin-1-yl]méthyl]-6-méthyl-morpholin-4-yl]quinoléine-8-carbonitrile ;
le 5-[(2S,6R)-2-[[4-[2-[(3S,4S)-3-amino-4-méthoxy-pyrrolidin-1-yl]-6-méthyl-pyrimidin-4-yl]pipérazin-1-yl]méthyl]-6-méthyl-morpholin-4-yl]quinoléine-8-carbonitrile ;
le 5-[(2S,6R)-2-[[4-[2-[(3R,4R)-3-amino-4-méthoxy-pyrrolidin-1-yl]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[[(3*R*,4*R*)-4-méthoxypyrrolidin-3-yl]amino]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[[(3*R*,4*S*)9-4-fluoropyrrolidin-3-yl]amino]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*S*,4*R*)-3-amino-4-méthoxy-pyrrolidin-1-yl]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[[(3*S*,4*R*)-4-fluoropyrrolidin-3-yl]amino]-6-méthyl-pyrimidin-4-yl]pipérazin-1-yl]méthyl]-6-méthyl-morpholin-4-yl]quinoléine-8-carbonitrile ;
le 5-[(2*R*,6*S*)-2-méthyl-6-[[4-[6-méthyl-2-[*cis*-3-amino-5-fluoro-1-pipéridyl]pyrimidin-4-yl]pipérazin-1-yl]méthyl]morpholin-4-yl]quinoléine-8-carbonitrile ;
le 5-[(2*R*,6*S*)-2-méthyl-6-[[4-[6-méthyl-2-[*trans*-3-amino-5-fluoro-1-pipéridyl]pyrimidin-4-yl]pipérazin-1-yl]méthyl]morpholin-4-yl]quinoléine-8-carbonitrile ;
le 5-[(2*S*,6*R*)-2-[[4-[2-[[(3*R*,4*R*)-4-fluoropyrrolidin-3-yl]amino]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*S*,5*R*)-3-amino-5-fluoro-1-pipéridyl]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*S*,4*R*)-4-amino-3-fluoro-1-pipéridyl]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*R*,4*R*)-3-amino-4-fluoro-pyrrolidin-1-yl]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-(5-amino-3,3-difluoro-1-pipéridyl)-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[3-(1-hydroxy-1-méthyl-éthyl)pipérazin-1-yl]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*R*,4*R*)-4-amino-3-fluoro-1-pipéridyl]-6-méthyl-pyrimidin-4-yl]pipérazin-1-yl]méthyl]-6-méthyl-morpholin-4-yl]quinoléine-8-carbonitrile ;
le 5-[(2*R*,6*S*)-2-méthyl-6-[[4-[6-méthyl-2-[(3*S*,4*R*)-3-amino-4-fluoro-1-pipéridyl]-pyrimidin-4-yl]pipérazin-1-yl]méthyl]morpholin-4-yl]quinoléine-8-carbonitrile ;
le 5-[(2*R*,6*S*)-2-méthyl-6-[[4-[6-méthyl-2-[(3*R*,4*R*)-3-amino-4-fluoro-1-pipéridyl]pyrimidin-4-yl]pipérazin-1-yl]méthyl]morpholin-4-yl]quinoléine-8-carbonitrile ;
le 5-[(2*S*,6*R*)-2-[[4-[2-(azétidin-3-ylamino)-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-(4-amino-3,3-difluoro-1-pipéridyl)-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-(3-aminoazétidin-1-yl)-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*R*,4*S*)-3-amino-4-méthoxy-pyrrolidin-1-yl]-6-méthyl-pyrimidin-4-yl]pipérazin-1-yl]méthyl]-6-méthyl-morpholin-4-yl]quinoléine-8-carbonitrile ;
le 5-[(2*R*,6*S*)-2-méthyl-6-[[4-(2-morpholin-2-ylphényl)pipérazin-1-yl]méthyl]morpholin-4-yl]quinoléine-8-carbonitrile ;
le 5-[(2*S*,6*R*)-2-[[4-[2-[(3*S*,4*S*)-4-amino-3-méthoxy-1-pipéridyl]-6-méthyl-pyrimidin-4-yl]pipérazin-1-yl]méthyl]-6-méthyl-morpholin-4-yl]quinoléine-8-carbonitrile ;
le 5-[(2*S*,6*R*)-2-[[4-(6-méthoxy-2-pipérazin-1-yl-pyrimidin-4-yl)pipérazin-1-yl]méthyl]-6-méthyl-morpholin-4-yl]quinoléine-8-carbonitrile ;
le 5-[(2*S*,6*R*)-2-[[4-[2-[(3*S*,4*R*)-3-amino-4-méthoxy-pyrrolidin-1-yl]pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*R*,4*S*)-3-amino-4-méthoxy-pyrrolidin-1-yl]pyrimidin-4-yl]pipérazin-1-yl]méthyl]-6-méthyl-morpholin-4-yl]quinoléine-8-carbonitrile ;
le 5-[(2*S*,6*R*)-2-[[4-(5-fluoro-4-pipérazin-1-yl-pyrimidin-2-yl)pipérazin-1-yl]méthyl]-6-méthyl-morpholin-4-yl]quinoléine-8-carbonitrile ;
le 5-[(2*S*,6*R*)-2-[[4-[2-[(3*S*,4*R*)-3-amino-4-fluoro-pyrrolidin-1-yl]pyrimidin-4-yl]pipérazin-1-yl]méthyl]-6-méthyl-morpholin-4-yl]quinoléine-8-carbonitrile ;
le 5-[(2*S*,6*R*)-2-[[4-[2-[(3*R*,4*R*)-3-amino-4-fluoro-pyrrolidin-1-yl]pyrimidin-4-yl]pipérazin-1-yl]méthyl]-6-méthyl-morpholin-4-yl]quinoléine-8-carbonitrile ;
le 5-[(2*S*,6*R*)-2-[[4-[4-[(3*S*,4*S*)-3-amino-4-méthoxy-pyrrolidin-1-yl]pyrimidin-2-yl]pipérazin-1-yl]méthyl]-6-méthyl-morpholin-4-yl]quinoléine-8-carbonitrile ;
le 8-[(2*S*,6*R*)-2-[[4-[2-[(3*R*,4*R*)-3-amino-4-méthoxy-pyrrolidin-1-yl]pyrimidin-4-yl]pipérazin-1-yl]méthyl]-6-méthyl-morpholin-4-yl]quinoxaline-5-carbonitrile ;
le 8-[(2*S*,6*R*)-2-[[4-[2-[(3*S*,4*S*)-3-amino-4-méthoxy-pyrrolidin-1-yl]pyrimidin-4-yl]pipérazin-1-yl]méthyl]-6-méthyl-morpholin-4-yl]quinoxaline-5-carbonitrile ;
le 5-[(2*S*,6*R*)-2-[[4-[2-[(3*R*,4*S*)-3-amino-4-fluoro-pyrrolidin-1-yl]pyrimidin-4-yl]pipérazin-1-yl]méthyl]-6-méthyl-morpholin-4-yl]quinoléine-8-carbonitrile ;
le 5-[(2*R*,6*S*)-2-méthyl-6-[[4-(5-méthyl-2-pipérazin-1-yl-pyrimidin-4-yl)pipérazin-1-yl]méthyl]morpholin-4-yl]quinoléine-8-carbonitrile ;
le 5-[*(2S,6R)-2*-[[*4*-[2-[(*4aR,7aR*)-3,4,4*a*,5,7,7*a*-hexahydro-2*H*-pyrrolo[3,4-b][1,4]oxazin-6-yl]pyrimidin-4-yl]pipérazin-1-yl]méthyl]-6-méthyl-morpholin-4-yl]quinoléine-8-carbonitrile ;
le 8-[*(2S,6R)*-2-[[4-[4-[(3*S*,4*S*)-3-amino-4-méthoxy-pyrrolidin-1-yl]-6-méthyl-pyrimidin-2-yl]pipérazin-1-yl]méthyl]-6-méthyl-morpholin-4-yl]quinoxaline-5-carbonitrile ;
le 8-[*(2S,6R)*-2-[[4-[4-[(3*S*,4*S*)-3-amino-4-méthoxy-pyrrolidin-1-yl]-6-méthyl-pyrimidin-2-yl]pipérazin-1-yl]méthyl]-6-méthyl-morpholin-4-yl]-2,3-dideutério-quinoxaline-5-carbonitrile ;
le 8-[*(2S,6R)*-2-[[4-[2-[(3*S*,4*S*)-3-amino-4-méthoxy-pyrrolidin-1-yl]-6-méthyl-pyrimidin-4-yl]pipérazin-1-yl]méthyl]-6-méthyl-morpholin-4-yl]quinoxaline-5-carbonitrile ;
le 8-[*(2S,6R)*-2-[[4-[2-[(*3S,4S*)-3-amino-4-fluoro-pyrrolidin-1-yl]pyrimidin-4-yl]pipérazin-1-yl]méthyl]-6-méthyl-morpholin-4-yl]quinoxaline-5-carbonitrile ;
le 8-[*(2S,6R)*-2-[[4-[2-[(*3R,4S*)-3-amino-4-fluoro-pyrrolidin-1-yl]pyrimidin-4-yl]pipérazin-1-yl]méthyl]-6-méthyl-morpholin-4-yl]quinoxaline-5-carbonitrile ;
le 5-[*(2S,6R)*-2-[[4-[2-(3-amino-4-méthoxy-1-pipéridyl)pyrimidin-4-yl]pipérazin-1-yl]méthyl]-6-méthyl-morpholin-4-yl]quinoléine-8-carbonitrile ;
le 5-[*(2S,6R)*-2-[[4-[2-[*(3S,4S)*-3-amino-4-fluoro-pyrrolidin-1-yl]pyrimidin-4-yl]pipérazin-1-yl]méthyl]-6-méthyl-morpholin-4-yl]quinoléine-8-carbonitrile ;
le 8-[*(2S,6R)*-2-[[4-[2-[*(3R,4S)*-4-amino-3-fluoro-1-pipéridyl]pyrimidin-4-yl]pipérazin-1-yl]méthyl]-6-méthyl-morpholin-4-yl]quinoxaline-5-carbonitrile ;
le 8-[*(2S,6R)*-2-[[4-[2-(3-aminoazétidin-1-yl)-6-méthyl-pyrimidin-4-yl]pipérazin-1-yl]méthyl]-6-méthyl-morpholin-4-yl]quinoxaline-5-carbonitrile ;
le 8-[*(2S,6R)*-2-[[4-[2-[(*3R,4R*)-4-amino-3-fluoro-1-pipéridyl]pyrimidin-4-yl]pipérazin-1-yl]méthyl]-6-méthyl-morpholin-4-yl]quinoxaline-5-carbonitrile ;
le 8-[(*2R,6S*)-2-méthyl-6-[[4-(4-méthyl-6-pipérazin-1-yl-pyrimidin-2-yl)pipérazin-1-yl]méthyl]morpholin-4-yl]quinoxaline-5-carbonitrile ;
le 8-[*(2S,6R)*-2-[[4-[4-[(*3R,4S*)-3-amino-4-fluoro-pyrrolidin-1-yl]-6-méthyl-pyrimidin-2-yl]pipérazin-1-yl]méthyl]-6-méthyl-morpholin-4-yl]quinoxaline-5-carbonitrile ;
la (*2R,6S*)-2-méthyl-4-(8-méthylquinoxalin-5-yl)-6-[[4-(4-pipérazin-1-ylpyrimidin-2-yl)pipérazin-1-yl]méthyl]morpholine ;
la (*2R,6S*)-2-méthyl-4-(8-méthylquinoxalin-5-yl)-6-[[4-(2-pipérazin-1-ylpyrimidin-4-yl)pipérazin-1-yl]méthyl]morpholine ;
le 8-[*(2S,6R)*-2-[[4-[4-(3-aminoazétidin-1-yl)pyrimidin-2-yl]pipérazin-1-yl]méthyl]-6-méthyl-morpholin-4-yl]quinoxaline-5-carbonitrile ;
le 8-[*(2S,6R)*-2-[[4-[4-[(*3R,4R*)-3-amino-4-fluoro-pyrrolidin-1-yl]pyrimidin-2-yl]pipérazin-1-yl]méthyl]-6-méthyl-morpholin-4-yl]quinoxaline-5-carbonitrile ;
le 8-[*(2S,6R)*-2-[[4-[4-[(*3S,4R*)-3-amino-4-méthoxy-pyrrolidin-1-yl]-6-méthyl-pyrimidin-2-yl]pipérazin-1-yl]méthyl]-6-méthyl-morpholin-4-yl]quinoxaline-5-carbonitrile ;
le 8-[*(2S,6R)*-2-[[4-[4-[(*3R,4R*)-3-amino-4-méthoxy-pyrrolidin-1-yl]-6-méthyl-pyrimidin-2-yl]pipérazin-1-yl]méthyl]-6-méthyl-morpholin-4-yl]quinoxaline-5-carbonitrile ;
le 8-[*(2S,6R)*-2-[[4-[4-[(*3R,4S*)-3-amino-4-méthoxy-pyrrolidin-1-yl]-6-méthyl-pyrimidin-2-yl]pipérazin-1-yl]méthyl]-6-méthyl-morpholin-4-yl]quinoxaline-5-carbonitrile ;
le 8-[*(2S,6R)*-2-[[4-[4-(3-aminoazétidin-1-yl)-6-méthyl-pyrimidin-2-yl]pipérazin-1-yl]méthyl]-6-méthyl-morpholin-4-yl]quinoxaline-5-carbonitrile ;
le 8-[*(2S,6R)*-2-[[4-[4-(3-aminoazétidin-1-yl)-6-méthyl-pyrimidin-2-yl]pipérazin-1-yl]méthyl]-6-méthyl-morpholin-4-yl]-2,3-dideutério-quinoxaline-5-carbonitrile ;
le 8-[*(2S,6R)*-2-[[4-[4-[(*3R,4R*)-4-amino-3-hydroxy-3-méthyl-pyrrolidin-1-yl]-6-méthyl-pyrimidin-2-yl]pipérazin-1-yl]méthyl]-6-méthyl-morpholin-4-yl]quinoxaline-5-carbonitrile ;
le 2-[4-[4-[[*(2S,6R)*-6-méthyl-4-(8-méthylquinoxalin-5-yl)morpholin-2-yl]méthyl]pipérazin-1-yl]pyrimidin-2-yl]-5-oxa-2,8-diazaspiro[3.5]nonane ;
la (*2R,6S*)-2-méthyl-6-[[4-[2-[(3S)-3-méthylpipérazin-1-yl]pyrimidin-4-yl]pipérazin-1-yl]méthyl]-4-(8-méthylquinoxalin-5-yl)morpholine ;
la (*2R,6S*)-2-méthyl-6-[[4-[2-[(*3R*)-3-méthylpipérazin-1-yl]pyrimidin-4-yl]pipérazin-1-yl]méthyl]-4-(8-méthylquinoxalin-5-yl)morpholine ;
la (*4aR,7aR*)-6-[4-[4-[[*(2S,6R)*-6-méthyl-4-(8-méthylquinoxalin-5-yl)morpholin-2-yl]méthyl]pipérazin-1-yl]pyrimidin-2-yl]-3,4,4*a*,5,7,7*a*-hexahydro-2*H*-pyrrolo[3,4-b][1,4]oxazine ;
la 1-[4-[4-[[*(2S,6R)*-6-méthyl-4-(8-méthylquinoxalin-5-yl)morpholin-2-yl]méthyl]pipérazin-1-yl]pyrimidin-2-yl]azétidin-3-amine ;
le 5-[*(2S,6R)*-2-[[4-[4-(3-amino-3-méthyl-azétidin-1-yl)-6-méthoxy-pyrimidin-2-yl]pipérazin-1-yl]méthyl]-6-méthyl-morpholin-4-yl]-2-deutério-quinoléine-8-carbonitrile ;
le 5-[*(2S,6R)*-2-[[4-[6-(3-amino-3-méthyl-azétidin-1-yl)-3-pyridyl]pipérazin-1-yl]méthyl]-6-méthyl-morpholin-4-yl]-2-deutério-quinoléine-8-carbonitrile ;
le 5-[*(2S,6R)*-2-[[4-[6-(3-amino-3-méthyl-azétidin-1-yl)-4-méthyl-3-pyridyl]pipérazin-1-yl]méthyl]-6-méthyl-morpholin-4-yl]-2-deutério-quinoléine-8-carbonitrile ;
le 5-[*(2S,6R)*-2-[[4-[4-[(*3S,4S*)-3-amino-4-méthoxy-pyrrolidin-1-yl]-6-méthyl-pyrimidin-2-yl]pipérazin-1-yl]méthyl]-6-méthyl-morpholin-4-yl]-2-deutério-quinoléine-8-carbonitrile ;
le 5-[*(2S,6R)*-2-[[4-[4-(3-amino-3-méthyl-azétidin-1-yl)-6-méthyl-pyrimidin-2-yl]pipérazin-1-yl]méthyl]-6-méthyl-morpholin-4-yl]-2-deutério-quinoléine-8-carbonitrile ;
le 5-[*(2S,6R)*-2-[[4-[4-(3-aminoazétidin-1-yl)-6-méthyl-pyrimidin-2-yl]pipérazin-1-yl]méthyl]-6-méthyl-morpholin-4-yl]-2-deutério-quinoléine-8-carbonitrile ;
le 5-[*(2S,6R)*-2-[[4-[6-(3-amino-3-méthyl-azétidin-1-yl)-2-méthyl-3-pyridyl]pipérazin-1-yl]méthyl]-6-méthyl-morpholin-4-yl]-2-deutério-quinoléine-8-carbonitrile ;
le 5-[(*2S*,*6R*)-2-[[3-[6-(3-amino-3-méthyl-azétidin-1-yl)-3-pyridyl]azétidin-1-yl]méthyl]-6-méthyl-morpholin-4-yl]-2-deutério-quinoléine-8-carbonitrile ;
le 5-[*(2S,6R)*-2-[[4-[5-(3-amino-3-méthyl-azétidin-1-yl)-3-méthyl-2-pyridyl]pipérazin-1-yl]méthyl]-6-méthyl-morpholin-4-yl]-2-deutério-quinoléine-8-carbonitrile ;
le 5-[*(2S,6R)*-2-[[3-[4-[(*3S*,*4S*)-3-amino-4-méthoxy-pyrrolidin-1-yl]phényl]azétidin-1-yl]méthyl]-6-méthyl-morpholin-4-yl]-2-deutério-quinoléine-8-carbonitrile ;
le 5-[*(2S,6R)*-2-[[3-[4-(3-amino-3-méthyl-azétidin-1-yl)phényl]azétidin-1-yl]méthyl]-6-méthyl-morpholin-4-yl]-2-deutério-quinoléine-8-carbonitrile ;
le 5-[*(2S*,*6R*)-2-[[4-[5-[(*3R*,*4R*)-3-amino-4-méthoxy-pyrrolidin-1-yl]-3-méthyl-2-pyridyl]pipérazin-1-yl]méthyl]-6-méthyl-morpholin-4-yl]-2-deutério-quinoléine-8-carbonitrile ;
le 5-[(*2S*,*6R*)-2-[[4-[6-[(*3R*,*4R*)-3-amino-4-méthoxy-pyrrolidin-1-yl]-3-pyridyl]pipérazin-1-yl]méthyl]-6-méthyl-morpholin-4-yl]-2-deutério-quinoléine-8-carbonitrile ;
le 5-[*(2S,6R*)-2-[[4-[4-[*(3R,4R*)-3-amino-4-méthoxy-pyrrolidin-1-yl]-6-méthyl-pyrimidin-2-yl]pipérazin-1-yl]méthyl]-6-méthyl-morpholin-4-yl]-2-deutério-quinoléine-8-carbonitrile ;
le 5-[*(2S,6R)*-2-[[3-[6-(3-amino-3-méthyl-azétidin-1-yl)-4-méthyl-3-pyridyl]azétidin-1-yl]méthyl]-6-méthyl-morpholin-4-yl]-2-deutério-quinoléine-8-carbonitrile ;
le 5-[(*2S*,*6R*)-2-[[3-[6-[(*3R*,*4R*)-3-amino-4-méthoxy-pyrrolidin-1-yl]-4-méthyl-3-pyridyl]azétidin-1-yl]méthyl]-6-méthyl-morpholin-4-yl]-2-deutério-quinoléine-8-carbonitrile ;
le 5-[*(2S,6R)*-2-[[4-[5-(3-amino-3-méthyl-azétidin-1-yl)-6-méthyl-2-pyridyl]pipérazin-1-yl]méthyl]-6-méthyl-morpholin-4-yl]-2-deutério-quinoléine-8-carbonitrile ;
le 4-[*(2S,6R)*-2-[[4-[5-(3-amino-3-méthyl-azétidin-1-yl)-3-méthyl-2-pyridyl]pipérazin-1-yl]méthyl]-6-méthyl-morpholin-4-yl]-3-fluoro-pyrazolo[1,5-a]pyridine-7-carbonitrile ;
le 5-[*(2S,6R)*-2-[[3-[6-[(*3R*,*4R*)-3-amino-4-méthoxy-pyrrolidin-1-yl]-3-pyridyl]azétidin-1-yl]méthyl]-6-méthyl-morpholin-4-yl]-2-deutério-quinoléine-8-carbonitrile ;
le 5-[*(2S,6R)*-2-[[4-[6-[(*3S*,*4S*)-3-amino-4-fluoro-pyrrolidin-1-yl]-3-pyridyl]pipérazin-1-yl]méthyl]-6-méthyl-morpholin-4-yl]-2-deutério-quinoléine-8-carbonitrile ;
le 2-deutério-5-[(*2S*,6R)-2-[[4-[6-[(1S,4S)-2,5-diazabicyclo[2.2.1]heptan-2-yl]-3-pyridyl]pipérazin-1-yl]méthyl]-6-méthyl-morpholin-4-yl]quinoléine-8-carbonitrile ;
le 2-deutério-5-[(*2R*,*6S*)-2-méthyl-6-[[4-[6-(5-oxa-2,8-diazaspiro[3.5]nonan-2-yl)-3-pyridyl]pipérazin-1-yl]méthyl]morpholin-4-yl]quinoléine-8-carbonitrile ;
le 2-deutério-5-[*(2S*,*6R*)-2-[[4-[6-[(1R,4R)-2,5-diazabicyclo[2.2.1]heptan-2-yl]-3-pyridyl]pipérazin-1-yl]méthyl]-6-méthyl-morpholin-4-yl]quinoléine-8-carbonitrile ;
le 4-[(*2S*,*6R*)-2-[[4-[6-(3-amino-3-méthyl-azétidin-1-yl)-3-pyridyl]pipérazin-1-yl]méthyl]-6-méthyl-morpholin-4-yl]-3-fluoro-pyrazolo[1,5-a]pyridine-7-carbonitrile ;
le 5-[*(2S,6R)*-2-[[4-[6-(3-amino-3-méthyl-azétidin-1-yl)-2-pyridyl]pipérazin-1-yl]méthyl]-6-méthyl-morpholin-4-yl]-2-deutério-quinoléine-8-carbonitrile ;
le 5-[(*2S*,*6R*)-2-[[3-[4-(3-amino-3-méthyl-azétidin-1-yl)phényl]-3-hydroxy-azétidin-1-yl]méthyl]-6-méthyl-morpholin-4-yl]-2-deutério-quinoléine-8-carbonitrile ;
le 4-[(*2S*,*6R*)-2-[[3-[6-(3-amino-3-méthyl-azétidin-1-yl)-3-pyridyl]azétidin-1-yl]méthyl]-6-méthyl-morpholin-4-yl]pyrazolo[1,5-a]pyridine-7-carbonitrile ;
le 4-[(*2S*,*6R*)-2-[[3-[6-(3-amino-3-méthyl-azétidin-1-yl)-3-pyridyl]azétidin-1-yl]méthyl]-6-méthyl-morpholin-4-yl]-3-fluoro-pyrazolo[1,5-a]pyridine-7-carbonitrile ;
le 5-[*(2S,6R)*-2-[[3-[6-(3-amino-3-méthyl-azétidin-1-yl)-2-pyridyl]azétidin-1-yl]méthyl]-6-méthyl-morpholin-4-yl]-2-deutério-quinoléine-8-carbonitrile ;
le 4-[*(2S,6R)*-2-[[3-[4-(3-amino-3-méthyl-azétidin-1-yl)phényl]azétidin-1-yl]méthyl]-6-méthyl-morpholin-4-yl]-3-fluoro-pyrazolo[1,5-a]pyridine-7-carbonitrile ;
le 2-deutério-5-[(*2R*,*6S*)-2-méthyl-6-[[4-[6-(6-méthyl-2,6-diazaspiro[3.3]heptan-2-yl)-3-pyridyl]pipérazin-1-yl]méthyl]morpholin-4-yl]quinoléine-8-carbonitrile ;
le 2-deutério-5-[(*2S*,*6R*)-2-[[4-[6-[[(*3S*,*4R*)-4-fluoropyrrolidin-3-yl]amino]-3-pyridyl]pipérazin-1-yl]méthyl]-6-méthyl-morpholin-4-yl]quinoléine-8-carbonitrile ;
le 4-[*(2S,6R)*-2-[[3-[4-(3-amino-3-méthyl-azétidin-1-yl)phényl]azétidin-1-yl]méthyl]-6-méthyl-morpholin-4-yl]pyrazolo[1,5-a]pyridine-7-carbonitrile ;
le 4-[*(2S,6R)*-2-[[3-[6-[(*3R*,*4R*)-3-amino-4-méthoxy-pyrrolidin-1-yl]-3-pyridyl]azétidin-1-yl]méthyl]-6-méthyl-morpholin-4-yl]-3-fluoro-pyrazolo[1,5-a]pyridine-7-carbonitrile ;
le 4-[(*2S*,*6R*)-2-[[3-[6-[(1S,4S)-2,5-diazabicyclo[2.2.1]heptan-2-yl]-3-pyridyl]azétidin-1-yl]méthyl]-6-méthyl-morpholin-4-yl]-3-fluoro-pyrazolo[1,5-a]pyridine-7-carbonitrile ;
le 4-[*(2S,6R)*-2-[[4-[6-[(*3R*,*4R*)-3-amino-4-méthoxy-pyrrolidin-1-yl]-3-pyridyl]pipérazin-1-yl]méthyl]-6-méthyl-morpholin-4-yl]-3-fluoro-pyrazolo[1,5-a]pyridine-7-carbonitrile ;
le 3-fluoro-4-[*(2S,6R)*-2-[[4-[6-[[(*3S,4R*)-4-fluoropyrrolidin-3-yl]amino]-3-pyridyl]pipérazin-1-yl]méthyl]-6-méthyl-morpholin-4-yl]pyrazolo[1,5-a]pyridine-7-carbonitrile ;
le 4-[*(2S,6R)*-2-[[4-[6-[(4*aR*,7*aR*)-3,4,4*a*,5,7,7*a*-hexahydro-2*H*-pyrrolo[3,4-b][1,4]oxazin-6-yl]-3-pyridyl]pipérazin-1-yl]méthyl]-6-méthyl-morpholin-4-yl]-3-fluoro-pyrazolo[1,5-a]pyridine-7-carbonitrile ;
le 3-fluoro-4-[(*2R*,*6S*)-2-méthyl-6-[[4-[6-(5-oxa-2,8-diazaspiro[3.5]nonan-2-yl)-3-pyridyl]pipérazin-1-yl]méthyl]morpholin-4-yl]pyrazolo[1,5-a]pyridine-7-carbonitrile ;
le 3-fluoro-4-[(*2R*,*6S*)-2-méthyl-6-[[4-[6-(6-méthyl-2,6-diazaspiro[3.3]heptan-2-yl)-3-pyridyl]pipérazin-1-yl]méthyl]morpholin-4-yl]pyrazolo[1,5-a]pyridine-7-carbonitrile ;
la 1-méthyl-4-[(*2R*,*6S*)-2-méthyl-6-[[3-(4-pipérazin-1-ylphényl)azétidin-1-yl]méthyl]morpholin-4-yl]-1,8-naphtyridin-2-one ;
la 4-[*(2S,6R)*-2-[[3-[4-[(*3R*,*4R*)-3-amino-4-méthoxy-pyrrolidin-1-yl]phényl]azétidin-1-yl]méthyl]-6-méthyl-morpholin-4-yl]-1-méthyl-1,8-naphtyridin-2-one ;
la 1-méthyl-4-[(*2R*,*6S*)-2-méthyl-6-[[3-[4-(5-oxa-2,8-diazaspiro[3.5]nonan-2-yl)phényl]azétidin-1-yl]méthyl]morpholin-4-yl]-1,8-naphtyridin-2-one ;
la 4-[*(2S,6R)*-2-[[3-[4-[(*3R*,*4R*)-3-amino-4-fluoro-pyrrolidin-1-yl]phényl]azétidin-1-yl]méthyl]-6-méthyl-morpholin-4-yl]-1-méthyl-1,8-naphtyridin-2-one ;
le 4-[*(2S,6R)*-2-[[4-[6-[(*3R*,*4R*)-4-amino-3-fluoro-1-pipéridyl]-3-pyridyl]pipérazin-1-yl]méthyl]-6-méthyl-morpholin-4-yl]-3-fluoro-pyrazolo[1,5-a]pyridine-7-carbonitrile ;
la 4-[*(2S,6R)*-2-[[3-[4-(3-amino-3-méthyl-azétidin-1-yl)phényl]azétidin-1-yl]méthyl]-6-méthyl-morpholin-4-yl]-1-méthyl-1,8-naphtyridin-2-one ;
le 3-fluoro-4-[(*2R*,*6S*-2-méthyl-6-[[4-[4-[(*2R*)-morpholin-2-yl]phényl]pipérazin-1-yl]méthyl]morpholin-4-yl]pyrazolo[1,5-a]pyridine-7-carbonitrile ;
le 3-fluoro-4-[(*2R*,*6S*)-2-méthyl-6-[[4-[6-(5-oxa-2,8-diazaspiro[3.5]nonan-8-yl)-3-pyridyl]pipérazin-1-yl]méthyl]morpholin-4-yl]pyrazolo[1,5-a]pyridine-7-carbonitrile ;
le 4-[*(2S,6R)*-2-[[4-[6-(1,6-diazaspiro[3.3]heptan-6-yl)-3-pyridyl]pipérazin-1-yl]méthyl]-6-méthyl-morpholin-4-yl]-3-fluoro-pyrazolo[1,5-a]pyridine-7-carbonitrile ;
la 1-méthyl-4-[(*2R*,*6S*)-2-méthyl-6-[[3-[4-(6-méthyl-2,6-diazaspiro[3.3]heptan-2-yl)phényl]azétidin-1-yl]méthyl]morpholin-4-yl]-1,8-naphtyridin-2-one ;
la 4-[*(2S,6R)*-2-[[3-[4-[(4*aR*,*7aR*)-3,4,4*a*,5,7,7*a*-hexahydro-2*H*-pyrrolo[3,4-*b*][1,4]oxazin-6-yl]phényl]azétidin-1-yl]méthyl]-6-méthyl-morpholin-4-yl]-1-méthyl-1,8-naphtyridin-2-one ;
le 3-fluoro-4-[(2*S*,6*R*)-2-[[4-[2-fluoro-4-[(2*S*)-morpholin-2-yl]phényl]pipérazin-1-yl]méthyl]-6-méthyl-morpholin-4-yl]pyrazolo[1,5-a]pyridine-7-carbonitrile ;
le 3-fluoro-4-[(2*S*,6*R*)-2-[[4-[3-fluoro-4-[(2*R*)-morpholin-2-yl]phényl]pipérazin-1-yl]méthyl]-6-méthyl-morpholin-4-yl]pyrazolo[1,5-a]pyridine-7-carbonitrile ;
le 3-fluoro-4-[(2*R*,6*S*)-2-méthyl-6-[[4-[4-(2-méthylmorpholin-2-yl)phényl]pipérazin-1-yl]méthyl]morpholin-4-yl]pyrazolo[1,5-a]pyridine-7-carbonitrile ; et
le 3-fluoro-4-[(*2R*,*6S*)-2-méthyl-6-[[4-[4-[[(*2R*)-morpholin-2-yl]méthyl]phényl]pipérazin-1-yl]méthyl]morpholin-4-yl]pyrazolo[1,5-a]pyridine-7-carbonitrile ;
ou un sel pharmaceutiquement acceptable de ceux-ci.

**20.** Procédé de préparation d'un composé selon l'une quelconque des revendications 1 à 19 comprenant l'une quelconque des étapes suivantes :

a) la substitution d'un composé de formule (IV),

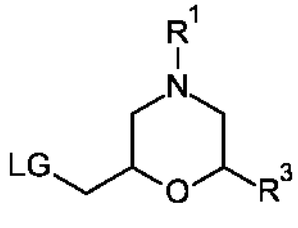

(IV),

avec un composé de formule (VIII),

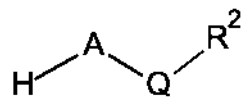

(VIII),

en présence d'une base ;
b) le couplage d'un composé de formule (XIII),

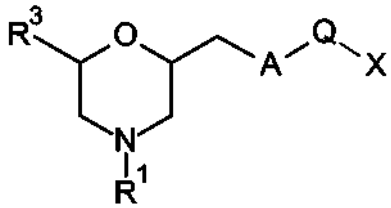

(XIII),

avec un composé de formule (VI) en présence d'une base ou dans une condition d'amination de Buchwald-Hartwig ;

dans lequel la base de l'étape a) et b) est choisie parmi $K_2CO_3$, DIPEA et $Cs_2CO_3$ ; la condition d'amination de Buchwald-Hartwig de l'étape b) comprend un catalyseur et une base, le catalyseur étant choisi parmi tBuXPhos Pd G3, RuPhos Pd G2, BrettPhos Pd G3, XPhos Pd G3, $Pd_2(dba)_3$/BINAP et $Pd_2(dba)_3$/XantPhos ; la base est $Cs_2CO_3$ ou Z-BuONa ; LG représente un groupe partant ; $R^1$, $R^2$, $R^3$, A, Q et X sont tels que définis dans l'une quelconque des revendications 1 à 18.

**21.** Composé ou sel pharmaceutiquement acceptable selon l'une quelconque des revendications 1 à 19 pour une utilisation comme substance thérapeutiquement active.

**22.** Composition pharmaceutique comprenant un composé selon l'une quelconque des revendications 1 à 19 et un véhicule thérapeutiquement inerte.

**23.** Composé ou sel pharmaceutiquement acceptable, énantiomère ou diastéréoisomère selon l'une quelconque des revendications 1 à 19 pour une utilisation dans le traitement ou la prophylaxie du lupus érythémateux disséminé ou de la néphropathie lupique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150105370 A
- US 20170174653 A1
- WO 2012168260 A
- WO 2012126922 A1
- WO 9618615 A1


### Non-patent literature cited in the description


- **NAVARRA, S. V. et al.** *Lancet,* 2011, vol. 377, 721
- **KRIEG, A. M. et al.** *Immunol. Rev.,* 2007, vol. 220, 251
- **JIMÉNEZ-DALMARONI, M. J. et al.** *Autoimmun Rev.,* 2016, vol. 15, 1
- **CHEN, J. Q. et al.** Clinical Reviews. *Allergy & Immunology,* 2016, vol. 50, 1
- *Acc. Chem. Res.,* 1998, vol. 31, 805-818
- *Chem. Rev.,* 2016, vol. 116, 12564-12649
- *Topics in Current Chemistry,* 2002, vol. 219, 131-209
- *CHEMICAL ABSTRACTS,* 1700609-48-8
- *CHEMICAL ABSTRACTS,* 507476-70-2
- *CHEMICAL ABSTRACTS,* 1268520-74-6
- *CHEMICAL ABSTRACTS,* 1360599-43-4
- *CHEMICAL ABSTRACTS,* 2687-25-4
- *CHEMICAL ABSTRACTS,* 1003-99-2
- *CHEMICAL ABSTRACTS,* 7424-91-1
- *CHEMICAL ABSTRACTS,* 72235-36-0
- *CHEMICAL ABSTRACTS,* 38212-30-5
- *CHEMICAL ABSTRACTS,* 38212-33-8
- *CHEMICAL ABSTRACTS,* 1174020-30-4
- *CHEMICAL ABSTRACTS,* 956317-40
- *CHEMICAL ABSTRACTS,* 169448-87-7
- *CHEMICAL ABSTRACTS,* 1400562-12-0
- *CHEMICAL ABSTRACTS,* 956317-40-1
- *CHEMICAL ABSTRACTS,* 1932508-77-4
- *CHEMICAL ABSTRACTS,* 1627185-88-9
- *CHEMICAL ABSTRACTS,* 1033718-91-0
- *CHEMICAL ABSTRACTS,* 1782916-90-8
- *CHEMICAL ABSTRACTS,* 1251010-45-3
- *CHEMICAL ABSTRACTS,* 1434141-95-3
- *CHEMICAL ABSTRACTS,* 1009075-48-2
- *CHEMICAL ABSTRACTS,* 1931911-57-7
- *CHEMICAL ABSTRACTS,* 1363378-08-8
- *CHEMICAL ABSTRACTS,* 1441392-27-3
- *CHEMICAL ABSTRACTS,* 1405128-38-2
- *CHEMICAL ABSTRACTS,* 2097061-04-4
- *CHEMICAL ABSTRACTS,* 1523530-29-1
- *CHEMICAL ABSTRACTS,* 1052713-46-8
- *CHEMICAL ABSTRACTS,* 1263180-22-8
- *CHEMICAL ABSTRACTS,* 907544-19-8
- *CHEMICAL ABSTRACTS,* 2927-71-1
- *CHEMICAL ABSTRACTS,* 1262407-41-9
- *CHEMICAL ABSTRACTS,* 1630815-57-4
- *CHEMICAL ABSTRACTS,* 1251005-61-4
- *CHEMICAL ABSTRACTS,* 169447-70-5
- *CHEMICAL ABSTRACTS,* 170033-47-3
- *CHEMICAL ABSTRACTS,* 113451-59-5
- *CHEMICAL ABSTRACTS,* 134003-84-2
- *CHEMICAL ABSTRACTS,* 1932066-52-8
- *CHEMICAL ABSTRACTS,* 1312566-00-9
- *CHEMICAL ABSTRACTS,* 1251011-05-8
- *CHEMICAL ABSTRACTS,* 1394319-56-2
- *CHEMICAL ABSTRACTS,* 1447831-94-8
- *CHEMICAL ABSTRACTS,* 14400997-81-0
- *CHEMICAL ABSTRACTS,* 179821-59-1
- **REDFERN WS ; CARLSSON L ; DAVIS AS ; LYNCH WG ; MACKENZIE I ; PALETHORPE S ; SIEGL PK ; STRANG I ; SULLIVAN AT ; WALLIS R.** Relationships between preclinical cardiac electrophysiology, clinical QT interval prolongation and torsade de pointes for a broad range of drugs: evidence for a provisional safety margin in drug development. *Cardiovasc. Res.,* 2003, vol. 58, 32-45
- **SANGUINETTI MC ; TRISTANI-FIROUZI M.** hERG potassium channels and cardiac arrhythmia. *Nature,* 2006, vol. 440, 463-469
- **WEBSTER R ; LEISHMAN D ; WALKER D.** Towards a drug concentration effect relationship for QT prolongation and torsades de pointes. *Curr. Opin. Drug Discov. Devel.,* 2002, vol. 5, 116-26